(19) Europäisches Patentamt
European Patent Office
Office européen des brevets

(11) **EP 2 496 258 B1**

(12) **EUROPEAN PATENT SPECIFICATION**

(45) Date of publication and mention
of the grant of the patent:
**02.11.2016 Bulletin 2016/44**

(21) Application number: **10829191.5**

(22) Date of filing: **05.11.2010**

(51) Int Cl.:
***G01N 33/68*** *(2006.01)*

(86) International application number:
**PCT/US2010/055721**

(87) International publication number:
**WO 2011/057138 (12.05.2011 Gazette 2011/19)**

(54) **METHODS FOR DIAGNOSIS AND PROGNOSIS OF RENAL INJURY AND RENAL FAILURE**

VERFAHREN ZUR DIAGNOSE UND PROGNOSE VON NIERENVERLETZUNGEN UND NIERENINSUFFIZIENZ

MÉTHODES DE DIAGNOSTIC ET DE PRONOSTIC DE LÉSION RÉNALE ET D'INSUFFISANCE RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**07.11.2009 US 259155 P
07.11.2009 US 259157 P
07.11.2009 US 259162 P
07.11.2009 US 259147 P
07.11.2009 US 259152 P
20.05.2010 US 346547 P
07.11.2009 US 259136 P
11.12.2009 US 285928 P
03.04.2010 US 320723 P
11.12.2009 US 285929 P
07.11.2009 US 259137 P
07.11.2009 US 259160 P
07.11.2009 US 259159 P
07.11.2009 US 259135 P
07.11.2009 US 259139 P
07.11.2009 US 259153 P
07.11.2009 US 259151 P
20.05.2010 US 346551 P
07.11.2009 US 259148 P
07.11.2009 US 259156 P
07.11.2009 US 259161 P
07.11.2009 US 259145 P**

(43) Date of publication of application:
**12.09.2012 Bulletin 2012/37**

(73) Proprietor: **Astute Medical, Inc.
San Diego, CA 92121 (US)**

(72) Inventors:
• **ANDERBERG, Joseph
Encinitas
CA 92024 (US)**
• **GRAY, Jeff
Solana Beach
CA 92075 (US)**
• **MCPHERSON, Paul
Encinitas
CA 92024 (US)**
• **NAKAMURA, Kevin
Cardiff By The Sea
CA 92007 (US)**
• **KAMPF, James, Patrick
San Diego
CA 92130 (US)**

(74) Representative: **Schiweck, Weinzierl & Koch
European Patent Attorneys
Landsberger Straße 98
80339 München (DE)**

(56) References cited:
**WO-A1-2010/048346     US-A1- 2008 254 485**

• **S G COCA ET AL: "Biomarkers for the diagnosis and risk stratification of acute kidney injury: A systematic review", KIDNEY INTERNATIONAL, vol. 73, no. 9, 19 December 2007 (2007-12-19), pages 1008-1016, XP055027810, ISSN: 0085-2538, DOI: 10.1038/sj.ki.5002729**

**(Cont. next page)**

- **PRASAD DEVARAJAN: "Novel biomarkers for the early prediction of acute kidney injury", CANCER THERAPY, vol. 3, 1 September 2005 (2005-09-01), pages 477-488, XP055029044,**
- **BERAHOVICH ROBERT D ET AL: "Proteolytic activation of alternative CCR1 ligands in inflammation", THE JOURNAL OF IMMUNOLOGY, THE AMERICAN ASSOCIATION OF IMMUNOLOGISTS, US, vol. 174, no. 11, 1 June 2005 (2005-06-01) , pages 7341-7351, XP002435923, ISSN: 0022-1767**
- **KOTA HATTA ET AL: "ORIGINAL ARTICLE: Cytokine Array Comparisons of Plasma from Cycling Fertile Women on Cycle Day 5 and Ovulation", AMERICAN JOURNAL OF REPRODUCTIVE IMMUNOLOGY, vol. 62, no. 3, 1 September 2009 (2009-09-01), pages 158-164, XP055063069, ISSN: 1046-7408, DOI: 10.1111/j.1600-0897.2009.00725.x**
- **BICIK ET AL.: 'Role of Transforming Growth Factor-.beta.2 in, and a Possible Transforming Growth Factor-beta2 Gene Polymorphism as a Marker of, Renal Dysfunction in Essential Hypertension: A Study in Turkish Patients.' CURRENT THERAPEUTIC RESEARCH vol. 44, no. 4, 2005, pages 266 - 278, XP008155197**
- **NISHIYAMA ET AL.: 'Up-Regulation of Galectin-3 in Acute Renal Failure of the Rat.' THE AMERICAN JOURNAL OF PATHOLOGY vol. 157, no. 3, 2000, pages 815 - 823, XP008155236**

**Description**

The present application claims priority to U.S. Provisional Patent Application No. 61/259,135 filed November 7, 2009; U.S. Provisional Patent Application No. 61/259,136 filed November 7, 2009; U.S. Provisional Patent Application No. 61/259,137 filed November 7, 2009; U.S. Provisional Patent Application No. 61/259,139 filed November 7, 2009; U.S. Provisional Patent Application No. 61/259,145 filed November 7, 2009; U.S. Provisional Patent Application No. 61/259,147 filed November 7, 2009; U.S. Provisional Patent Application No. 61/259,148 filed November 7, 2009; U.S. Provisional Patent Application No. 61/259,151 filed November 7, 2009; U.S. Provisional Patent Application No. 61/259,152 filed November 7, 2009; U.S. Provisional Patent Application No. 61/259,153 filed November 7, 2009; U.S. Provisional Patent Application No. 61/259,155 filed November 7, 2009; U.S. Provisional Patent Application No. 61/259,156 filed November 7, 2009; U.S. Provisional Patent Application No. 61/259,157 filed November 7, 2009; U.S. Provisional Patent Application No. 61/259,159 filed November 7, 2009; U.S. Provisional Patent Application No. 61/259,160 filed November 7, 2009; U.S. Provisional Patent Application No. 61/259,161 filed November 7, 2009; U.S. Provisional Patent Application No. 61/259,162 filed November 7, 2009; U.S. Provisional Patent Application No. 61/285,928 filed December 11, 2009; U.S. Provisional Patent Application No. 61/285,929 filed December 11, 2009; U.S. Provisional Patent Application No. 61/320,723 filed April 3, 2010; U.S. Provisional Patent Application No. 61/346,551 filed May 20, 2010; and U.S. Provisional Patent Application No. 61/346,547 filed May 20, 2010.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week) reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, | ACE inhibitors or angiotensin II receptor blockers |

(continued)

| Type | Risk Factors |
|---|---|
| **Prerenal** | |
| especially in patients with bilateral renal artery stenosis) | |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulointerstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI.

However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.

More recently, Mehta et al., Crit. Care 11:R31 (doi:10.1186.cc5713), 2007 , proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006;7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Coca et al. (2008) "Biomarkers for the diagnosis and risk sratification of acute kidney injury: A systematic review" Kidney International 73, 1008-1016 describes different serum and urinary biomarkers used for the diagnosis of established AKI or early AKI or to risk stratify patients with AKI. Devarajan (2005) "Novel biomarkers for the early prediction of acute kidney injury" deals with biomarkers for the prediction of acute kidney injury. Berahovich et al. (2005) "Proteolytic activation of alternative CCR1 ligands in inflammation" The Journal of Immunology, 174: 7341-7351 describes functions of alternative CCR ligands upon inflammation. Nishiyama et al. (2000) "Up-Regulation of Galectin-3 in acute renal failure in rat" American Journal of Pathology Vol. 157, No. 3: 815-823 describes that Galectin-3 expression is upregulated in both, ischemic and toxic types of ARF. Bicik et al. (2005) "Role of transforming growth factor β2 in, and a possible transforming growth factor β2 gene polymorphism as a marker of, renal dysfunction in essential hypertension: a study in turish patients" Current Therapeutic Research, Vol. 66, Number 4, 266-278, suggests that high urinary $TGF\beta_2$ levels and persistent microalbuminuria might predict renal impairment.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the disclosure to provide methods and compositions for evaluating renal function in a subject. As described herein, measurement of one or more biomarkers selected from the group consisting of C-C motif chemokine 23, Transmembrane glycoprotein NMB, Brain-derived neurotrophic factor, Cathepsin S, Transforming growth factor beta-2 (and in certain embodiments its pro-form), Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3 (and in certain embodiments its pro-form), Heparan sulfate, Cadherin-3, Complement C5 (and in certain embodiments, its C5a form), Platelet factor 4, Platelet basic protein, and Stromelysin-2 (each referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers of the present disclosure may be used, individually or in panels comprising a plurality of kidney injury markers, for risk stratification (that is, to identify subjects at risk for a future injury to renal function, for future progression to reduced renal function, for future progression to ARF, for future improvement in renal function, etc.); for diagnosis of existing disease (that is, to identify subjects who have suffered an injury to renal function, who have progressed to reduced renal function, who have progressed to ARF, etc.); for monitoring for deterioration or improvement of renal function; and for predicting a future medical outcome, such as improved or worsening renal function, a decreased or increased mortality risk, a decreased or increased risk that a subject will require renal replacement therapy (i.e., hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, etc. In a first aspect, the present invention relates to a method for evaluating renal status in a subject, comprising: performing one or more assays configured to detect one or more biomarkers, the one or more biomarkers comprising C-C motif chemokine 23, on a body fluid sample obtained from the subject to provide an assay result; and

correlating the assay result(s) to the renal status of the subject.

In some embodiments of the method of the first aspect, the one or more biomarkers further comprise one or more of soluble Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, soluble Cadherin-3, Complement C5, Platelet factor 4, Platelet basic protein, and Stromelysin-2.In a second aspect, the present invention relates to a use of C-C motif chemokine 23, and optionally one or more biomarkers selected from the group consisting of soluble Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, soluble Cadherin-3, Complement C5, Platelet factor 4, Platelet basic protein, and Stromelysin-2, for the evaluation of renal injury, or use of C-C motif chemokine 23, and optionally one or more biomarkers selected from the group consisting of C-C motif chemokine 23, soluble Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, soluble Cadherin-3, Complement C5, Platelet factor 4, Platelet basic protein, and Stromelysin-2, for the evaluation of acute renal injury.

Also disclosed are methods for evaluating renal status in a subject. These methods comprise performing an assay method that is configured to detect one or more biomarkers selected from the group consisting of C-C motif chemokine 23, Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, Cadherin-3, Complement C5, Platelet factor 4, Platelet basic protein, and Stromelysin-2 is/are then correlated to the renal status of the subject. This correlation to renal status may include correlating the assay result(s) to one or more of risk stratification, diagnosis, prognosis, staging, classifying and monitoring of the subject as described herein. Thus, the methods utilize one or more kidney injury markers as disclosed herein for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of C-C motif chemokine 23, Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, Cadherin-3, Complement C5, Platelet factor 4, Platelet basic protein, and Stromelysin-2 is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of C-C motif chemokine 23, Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, Cadherin-3, Complement C5, Platelet factor 4, Platelet basic protein, and Stromelysin-2 is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of C-C motif chemokine 23, Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, Cadherin-3, Complement C5, Platelet factor 4, Platelet basic protein, and Stromelysin-2 is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.),* by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/-5% of a given measurement.

Multiple thresholds may also be used to assess renal status in a subject. For example, a "first" subpopulation which is predisposed to one or more future changes in renal status, the occurrence of an injury, a classification, etc.,

and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, disclosed are also devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.)* as well as molecules that may be indirectly detected by production of a detectable reaction product (*e.g.*, enzymes such as horseradish peroxidase, alkaline phosphatase, *etc.)* or through the use of a specific binding molecule which itself may be detectable (*e.g.*, a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.*, a diffraction grating, electrochemical sensor, etc) for generation of a signal, while in others, a signal is generated by a transducer that is spatially separate from the solid phase antibody (*e.g.*, a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present disclosure relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of C-C motif chemokine 23, Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, Cadherin-3, Complement C5, Platelet factor 4, Platelet basic protein, and Stromelysin-2 or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq 8.8$ μmol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq 26.4$ μmol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "C-C motif chemokine 23" refers to one or more polypeptides present in a biological sample that are derived from the C-C motif chemokine 23 precursor (Swiss-Prot P55773 (SEQ ID NO: 1))

```
          10         20         30         40         50         60
MKVSVAALSC LMLVTALGSQ ARVTKDAETE FMMSKLPLEN PVLLDRFHAT SADCCISYTP

          70         80         90        100        110        120
RSIPCSLLES YFETNSECSK PGVIFLTKKG RRFCANPSDK QVQVCVRMLK LDTRIKTRKN
```

The following domains have been identified in C-C motif chemokine 23:

| Residues | Length | Domain ID |
|----------|--------|-----------|
| 1-21 | 21 | signal peptide |
| 22-120 | 99 | C-C motif chemokine 23 |
| 40-120 | 81 | C-C motif chemokine 23 (19-99) |
| 43-120 | 78 | C-C motif chemokine 23 (22-99) |
| 48-120 | 73 | C-C motif chemokine 23 (27-99) |
| 51-120 | 70 | C-C motif chemokine 23 (30-99) |

As used herein, the term "Glycoprotein (Transmembrane) nmb" refers to one or more polypeptides present in a biological sample that are derived from the Glycoprotein (Transmembrane) nmbprecursor (Swiss-Prot Q14956 (SEQ ID NO: 2))

```
         10          20          30          40          50          60
MECLYYFLGF LLLAARLPLD AAKRFHDVLG NERPSAYMRE HNQLNGWSSD ENDWNEKLYP

         70          80          90         100         110         120
VWKRGDMRWK NSWKGGRVQA VLTSDSPALV GSNITFAVNL IFPRCQKEDA NGNIVYEKNC

        130         140         150         160         170         180
RNEAGLSADP YVYNWTAWSE DSDGENGTGQ SHHNVFPDGK PFPHHPGWRR WNFIYVFHTL

        190         200         210         220         230         240
GQYFQKLGRC SVRVSVNTAN VTLGPQLMEV TVYRRHGRAY VPIAQVKDVY VVTDQIPVFV

        250         260         270         280         290         300
TMFQKNDRNS SDETFLKDLP IMFDVLIHDP SHFLNYSTIN YKWSFGDNTG LFVSTNHTVN

        310         320         330         340         350         360
HTYVLNGTFS LNLTVKAAAP GPCPPPPPPP RPSKPTPSLA TTLKSYDSNT PGPAGDNPLE

        370         380         390         400         410         420
LSRIPDENCQ INRYGHFQAT ITIVEGILEV NIIQMTDVLM PVPWPESSLI DFVVTCQGSI

        430         440         450         460         470         480
PTEVCTIISD PTCEITQNTV CSPVDVDEMC LLTVRRTFNG SGTYCVNLTL GDDTSLALTS

        490         500         510         520         530         540
TLISVPDRDP ASPLRMANSA LISVGCLAIF VTVISLLVYK KHKEYNPIEN SPGNVVRSKG

        550         560         570
LSVFLNRAKA VFFPGNQEKD PLLKNQEFKG VS
```

Most preferably, the Glycoprotein (Transmembrane) nmb assay detects one or more soluble forms of Glycoprotein (Transmembrane) nmb. Glycoprotein (Transmembrane) nmb is a type I membrane protein having a large extracellular domain, most or all of which is present in soluble forms of Glycoprotein (Transmembrane) nmb generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Glycoprotein (Transmembrane) nmb:

| Residues | Length | Domain ID |
|---|---|---|
| 1-21 | 21 | signal peptide |
| 22-572 | 551 | Glycoprotein (Transmembrane) nmb |
| 508-572 | 65 | Cytoplasmic |
| 487-507 | 21 | transmembrane domain |
| 22-486 | 465 | Extracellular |
| 340-351 | 12 | Missing in isoform 2 |

As used herein, the term "Brain-derived neurotrophic factor" refers to one or more polypeptides present in a biological sample that are derived from the Brain-derived neurotrophic factorprecursor (Swiss-Prot P23560 (SEQ ID NO: 3))

```
          10         20         30         40         50         60
     MTILFLTMVI SYFGCMKAAP MKEANIRGQG GLAYPGVRTH GTLESVNGPK AGSRGLTSLA

          70         80         90        100        110        120
     DTFEHVIEEL LDEDQKVRPN EENNKDADLY TSRVMLSSQV PLEPPLLFLL EEYKNYLDAA

         130        140        150        160        170        180
     NMSMRVRRHS DPARRGELSV CDSISEWVTA ADKKTAVDMS GGTVTVLEKV PVSKGQLKQY

         190        200        210        220        230        240
     FYETKCNPMG YTKEGCRGID KRHWNSQCRT TQSYVRALTM DSKKRIGWRF IRIDTSCVCT

     LTIKRGR
```

The following domains have been identified in Brain-derived neurotrophic factor:

| Residues | Length | Domain ID |
|---|---|---|
| 1-21 | 21 | signal peptide |
| 22-120 | 99 | Brain-derived neurotrophic factor |
| 40-120 | 81 | Brain-derived neurotrophic factor (19-99) |
| 43-120 | 78 | Brain-derived neurotrophic factor (22-99) |
| 48-120 | 73 | Brain-derived neurotrophic factor (27-99) |
| 51-120 | 70 | Brain-derived neurotrophic factor (30-99) |

As used herein, the term "Cathepsin S" refers to one or more polypeptides present in a biological sample that are derived from the Cathepsin S precursor (Swiss-Prot P25774 (SEQ ID NO: 4))

```
          10         20         30         40         50         60
     MKRLVCVLLV CSSAVAQLHK DPTLDHHWHL WKKTYGKQYK EKNEEAVRRL IWEKNLKFVM

          70         80         90        100        110        120
     LHNLEHSMGM HSYDLGMNHL GDMTSEEVMS LMSSLRVPSQ WQRNITYKSN PNRILPDSVD

         130        140        150        160        170        180
     WREKGCVTEV KYQGSCGACW AFSAVGALEA QLKLKTGKLV SLSAQNLVDC STEKYGNKGC

         190        200        210        220        230        240
     NGGFMTTAFQ YIIDNKGIDS DASYPYKAMD QKCQYDSKYR AATCSKYTEL PYGREDVLKE

         250        260        270        280        290        300
     AVANKGPVSV GVDARHPSFF LYRSGVYYEP SCTQNVNHGV LVVGYGDLNG KEYWLVKNSW

         310        320        330
     GHNFGEEGYI RMARNKGNHC GIASFPSYPE I
```

The following domains have been identified in Cathepsin S:

| Residues | Length | Domain ID |
|---|---|---|
| 1-16 | 16 | signal peptide |
| 17-114 | 98 | activation peptide |
| 115-331 | 217 | Cathepsin S |

As used herein, the term "Transforming growth factor beta-2" refers to one or more polypeptides present in a biological sample that are derived from the Transforming growth factor beta-2 precursor (Swiss-Prot P61812 (SEQ ID NO: 5))

```
              10         20         30         40         50         60
        MHYCVLSAFL ILHLVTVALS LSTCSTLDMD QFMRKRIEAI RGQILSKLKL TSPPEDYPEP

              70         80         90        100        110        120
        EEVPPEVISI YNSTRDLLQE KASRRAAACE RERSDEEYYA KEVYKIDMPP FFPSENAIPP

             130        140        150        160        170        180
        TFYRPYFRIV RFDVSAMEKN ASNLVKAEFR VFRLQNPKAR VPEQRIELYQ ILKSKDLTSP

             190        200        210        220        230        240
        TQRYIDSKVV KTRAEGEWLS FDVTDAVHEW LHHKDRNLGF KISLHCPCCT FVPSNNYIIP

             250        260        270        280        290        300
        NKSEELEARF AGIDGTSTYT SGDQKTIKST RKKNSGKTPH LLLMLLPSYR LESQQTNRRK

             310        320        330        340        350        360
        KRALDAAYCF RNVQDNCCLR PLYIDFKRDL GWKWIHEPKG YNANFCAGAC PYLWSSDTQH

             370        380        390        400        410
        SRVLSLYNTI NPEASASPCC VSQDLEPLTI LYYIGKTPKI EQLSNMIVKS CKCS
```

Most preferably, the assay detects the pro-form of Transforming growth factor beta-2. The following domains have been identified in Transforming growth factor beta-2:

| Residues | Length | Domain ID |
|---|---|---|
| 1-19 | 19 | signal peptide |
| 20-302 | 283 | propeptide |
| 303-414 | 112 | Transforming growth factor beta-2 |
| 116 | 1 | N → TVCPVVTTPSGSVGSLCSRQ SQVLCGYLD (SEQ ID NO: 6) in isoform B |

As used herein, the term "Urokinase-type plasminogen activator" refers to one or more polypeptides present in a biological sample that are derived from the Urokinase-type plasminogen activator precursor (Swiss-Prot P00749 (SEQ ID NO: 7))

```
          10         20         30         40         50         60
MRALLARLLL CVLVVSDSKG SNELHQVPSN CDCLNGGTCV SNKYFSNIHW CNCPKKFGGQ

          70         80         90        100        110        120
HCEIDKSKTC YEGNGHFYRG KASTDTMGRP CLPWNSATVL QQTYHAHRSD ALQLGLGKHN

         130        140        150        160        170        180
YCRNPDNRRR PWCYVQVGLK PLVQECMVHD CADGKKPSSP PEELKFQCGQ KTLRPRFKII

         190        200        210        220        230        240
GGEFTTIENQ PWFAAIYRRH RGGSVTYVCG GSLISPCWVI SATHCFIDYP KKEDYIVYLG

         250        260        270        280        290        300
RSRLNSNTQG EMKFEVENLI LHKDYSADTL AHHNDIALLK IRSKEGRCAQ PSRTIQTICL

         310        320        330        340        350        360
PSMYNDPQFG TSCEITGFGK ENSTDYLYPE QLKMTVVKLI SHRECQQPHY YGSEVTTKML

         370        380        390        400        410        420
CAADPQWKTD SCQGDSGGPL VCSLQGRMTL TGIVSWGRGC ALKDKPGVYT RVSHFLPWIR

         430
SHTKEENGLA L
```

The following domains have been identified in Urokinase-type plasminogen activator:

| Residues | Length | Domain ID |
|---|---|---|
| 1-20 | 20 | signal peptide |
| 21-431 | 411 | Urokinase-type plasminogen activator |
| 21-177 | 157 | Urokinase-type plasminogen activator long chain A |
| 156-177 | 22 | Urokinase-type plasminogen activator short chain A |
| 179-431 | 253 | Urokinase-type plasminogen activator chain B |

As used herein, the term "Angiopoietin-2" refers to one or more polypeptides present in a biological sample that are derived from the Angiopoietin-2 precursor (Swiss-Prot 015123 (SEQ ID NO: 8))

```
            10          20          30          40          50          60
     MWQIVFFTLS  CDLVLAAAYN  NFRKSMDSIG  KKQYQVQHGS  CSYTFLLPEM  DNCRSSSSPY

            70          80          90         100         110         120
     VSNAVQRDAP  LEYDDSVQRL  QVLENIMENN  TQWLMKLENY  IQDNMKKEMV  EIQQNAVQNQ

           130         140         150         160         170         180
     TAVMIEIGTN  LLNQTAEQTR  KLTDVEAQVL  NQTTRLELQL  LEHSLSTNKL  EKQILDQTSE

           190         200         210         220         230         240
     INKLQDKNSF  LEKKVLAMED  KHIIQLQSIK  EEKDQLQVLV  SKQNSIIEEL  EKKIVTATVN

           250         260         270         280         290         300
     NSVLQKQQHD  LMETVNNLLT  MMSTSNSAKD  PTVAKEEQIS  FRDCAEVFKS  GHTTNGIYTL

           310         320         330         340         350         360
     TFPNSTEEIK  AYCDMEAGGG  GWTIIQRRED  GSVDFQRTWK  EYKVGFGNPS  GEYWLGNEFV

           370         380         390         400         410         420
     SQLTNQQRYV  LKIHLKDWEG  NEAYSLYEHF  YLSSEELNYR  IHLKGLTGTA  GKISSISQPG

           430         440         450         460         470         480
     NDFSTKDGDN  DKCICKCSQM  LTGGWWFDAC  GPSNLNGMYY  PQRQNTNKFN  GIKWYYWKGS

           490
     GYSLKATTMM  IRPADF
```

The following domains have been identified in Angiopoietin-2:

| Residues | Length | Domain ID |
|----------|--------|-----------|
| 1-18 | 18 | signal peptide |
| 19-496 | 478 | Angiopoietin-2 |
| 97 - 148 | 52 | missing in isoform 2 |

As used herein, the term "Matrilysin" refers to one or more polypeptides present in a biological sample that are derived from the Matrilysin precursor (Swiss-Prot P09237 (SEQ ID NO: 9))

```
            10          20          30          40          50          60
     MRLTVLCAVC  LLPGSLALPL  PQEAGGMSEL  QWEQAQDYLK  RFYLYDSETK  NANSLEAKLK

            70          80          90         100         110         120
     EMQKFFGLPI  TGMLNSRVIE  IMQKPRCGVP  DVAEYSLFPN  SPKWTSKVVT  YRIVSYTRDL

           130         140         150         160         170         180
     PHITVDRLVS  KALNMWGKEI  PLHFRKVVWG  TADIMIGFAR  GAHGDSYPFD  GPGNTLAHAF

           190         200         210         220         230         240
     APGTGLGGDA  HFDEDERWTD  GSSLGINFLY  AATHELGHSL  GMGHSSDPNA  VMYPTYGNGD

           250         260
     PQNFKLSQDD  IKGIQKLYGK  RSNSRKK
```

The following domains have been identified in Matrilysin:

| Residues | Length | Domain ID |
|---|---|---|
| 1-17 | 17 | signal peptide |
| 18-94 | 77 | activation peptide |
| 95-267 | 173 | Matrilysin |

As used herein, the term "Carcinoembryonic antigen-related cell adhesion molecule 1" refers to one or more polypeptides present in a biological sample that are derived from the Carcinoembryonic antigen-related cell adhesion molecule 1 precursor (Swiss-Prot P13688 (SEQ ID NO: 10))

```
        10         20         30         40         50         60
MGHLSAPLHR VRVPWQGLLL TASLLTFWNP PTTAQLTTES MPFNVAEGKE VLLLVHNLPQ

        70         80         90        100        110        120
QLFGYSWYKG ERVDGNRQIV GYAIGTQQAT PGPANSGRET IYPNASLLIQ NVTQNDTGFY

       130        140        150        160        170        180
TLQVIKSDLV NEEATGQFHV YPELPKPSIS SNNSNPVEDK DAVAFTCEPE TQDTTYLWWI

       190        200        210        220        230        240
NNQSLPVSPR LQLSNGNRTL TLLSVTRNDT GPYECEIQNP VSANRSDPVT LNVTYGPDTP

       250        260        270        280        290        300
TISPSDTYYR PGANLSLSCY AASNPPAQYS WLINGTFQQS TQELFIPNIT VNNSGSYTCH

       310        320        330        340        350        360
ANNSVTGCNR TTVKTIIVTE LSPVVAKPQI KASKTTVTGD KDSVNLTCST NDTGISIRWF

       370        380        390        400        410        420
FKNQSLPSSE RMKLSQGNTT LSINPVKRED AGTYWCEVFN PISKNQSDPI MLNVNYNALP

       430        440        450        460        470        480
QENGLSPGAI AGIVIGVVAL VALIAVALAC FLHFGKTGRA SDQRDLTEHK PSVSNHTQDH

       490        500        510        520
SNDPPNKMNE VTYSTLNFEA QQPTQPTSAS PSLTATEIIY SEVKKQ
```

Most preferably, the Carcinoembryonic antigen-related cell adhesion molecule 1 assay detects one or more soluble forms of Carcinoembryonic antigen-related cell adhesion molecule 1. Carcinoembryonic antigen-related cell adhesion molecule 1 is a type I membrane protein having a large extracellular domain, most or all of which is present in soluble forms of Carcinoembryonic antigen-related cell adhesion molecule 1 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Carcinoembryonic antigen-related cell adhesion molecule 1:

| Residues | Length | Domain ID |
|---|---|---|
| 1-34 | 34 | Signal peptide |
| 35-526 | 492 | Carcinoembryonic antigen-related cell adhesion molecule 1 |
| 453-526 | 74 | Cytoplasmic |
| 429-452 | 24 | transmembrane domain |
| 35-428 | 394 | Extracellular |

Known variants of this sequence, which are to be considered Carcinoembryonic antigen-related cell adhesion molecule 1 forms for purposes of the present invention have the following features:

| | | |
|---|---|---|
| 143-416 | 274 | Missing in isoform D |
| 320-416 | 97 | Replaced by D (Asp) in isoform C |
| 320-321 | 2 | EL to KG in isoform H |
| 321-416 | 96 | RQNLTMLPRLDSNSWAQAILPSVSQSAEITD (SEQ ID NO: 11) in isoform B |
| 321-351 | 31 | SPVLGEDEAVPGQHHPQHKPCQEGGCWDVLV (SEQ ID NO: 12) in isoform I |
| 322-526 | 175 | Missing in isoform H |
| 416-417 | 2 | YN to CK in isoform G |
| 418-526 | 109 | Missing in isoform G |

As used herein, the term "Creatine Kinase MB" refers to the MB heterodimer formed by Creatine kinase B-type and Creatine kinase M-type. The B chain (Swiss Prot P12277) has the following human precursor sequence (SEQ ID NO: 13):

```
            10          20          30          40          50          60

MPFSNSHNAL  KLRFPAEDEF  PDLSAHNNHM  AKVLTPELYA  ELRAKSTPSG  FTLDDVIQTG

            70          80          90         100         110         120

VDNPGHPYIM  TVGCVAGDEE  SYEVFKDLFD  PIIEDRHGGY  KPSDEHKTDL  NPDNLQGGDD

           130         140         150         160         170         180

LDPNYVLSSR  VRTGRSIRGF  CLPPHCSRGE  RRAIEKLAVE  ALSSLDGDLA  GRYYALKSMT

           190         200         210         220         230         240

EAEQQQLIDD  HFLFDKPVSP  LLLASGMARD  WPDARGIWHN  DNKTFLVWVN  EEDHLRVISM

           250         260         270         280         290         300

QKGGNMKEVF  TRFCTGLTQI  ETLFKSKDYE  FMWNPHLGYI  LTCPSNLGTG  LRAGVHIKLP

           310         320         330         340         350         360

NLGKHEKFSE  VLKRLRLQKR  GTGGVDTAAV  GGVFDVSNAD  RLGFSEVELV  QMVVDGVKLL

           370         380

IEMEQRLEQG  QAIDDLMPAQ  K
```

And the M chain (Swiss Prot P06732) has the following human precursor sequence (SEQ ID NO: 14):

```
            10         20         30         40         50         60
   MPFGNTHNKF KLNYKPEEEY PDLSKHNNHM AKVLTLELYK KLRDKETPSG FTVDDVIQTG

            70         80         90        100        110        120
   VDNPGHPFIM TVGCVAGDEE SYEVFKELFD PIISDRHGGY KPTDKHKTDL NHENLKGGDD

           130        140        150        160        170        180
   LDPNYVLSSR VRTGRSIKGY TLPPHCSRGE RRAVEKLSVE ALNSLTGEFK GKYYPLKSMT

           190        200        210        220        230        240
   EKEQQQLIDD HFLFDKPVSP LLLASGMARD WPDARGIWHN DNKSFLVWVN EEDHLRVISM

           250        260        270        280        290        300
   EKGGNMKEVF RRFCVGLQKI EEIFKKAGHP FMWNQHLGYV LTCPSNLGTG LRGGVHVKLA

           310        320        330        340        350        360
   HLSKHPKFEE ILTRLRLQKR GTGGVDTAAV GSVFDVSNAD RLGSSEVEQV QLVVDGVKLM

           370        380
   VEMEKKLEKG QSIDDMIPAQ K
```

The following domains have been identified in CK B chain:

| Residues | Length | Domain ID |
|----------|--------|-----------|
| 1 | 1 | initiator methionine |
| 2-381 | 380 | Creatine kinase B |

And the following domains have been identified in the CK M chain:

| | | |
|----------|--------|-----------|
| 1-381 | 381 | Creatine kinase M |

As used herein, the term "Insulin" refers to one or more polypeptides present in a biological sample that are derived from the Insulin precursor (Swiss-Prot P01308 (SEQ ID NO: 15))

```
            10         20         30         40         50         60
   MALWMRLLPL LALLALWGPD PAAAFVNQHL CGSHLVEALY LVCGERGFFY TPKTRREAED

            70         80         90        100        110
   LQVGQVELGG GPGAGSLQPL ALEGSLQKRG IVEQCCTSIC SLYQLENYCN
```

The following domains have been identified in Insulin:

| Residues | Length | Domain ID |
|----------|--------|-----------|
| 1-24 | 24 | signal peptide |
| 25-54 | 30 | Insulin B peptide |
| 57-87 | 31 | propeptide (C peptide) |

(continued)

| Residues | Length | Domain ID |
|---|---|---|
| 90-110 | 21 | Insulin A peptide |

As used herein, the term "IgM" refers to an antibody having a molecular mass of about 900 kDa and existing primarily as pentameric or hexameric polymers of immuniglobulins. IgM is the first antibody to appear in response to initial exposure to antigen. IgM possesses high avidity, and is particularly effective at complement activation.

As used herein, the term "IgE" refers to an immunoglobulin molecule which incorporates the IgE-characteristic ε light chain. IgE plays a role in allergy, and is especially associated with type 1 hypersensitivity. IgE elicits an immune response by binding to Fc receptors found on the surface of mast cells and basophils, and are also found on eosinophils, monocytes, macrophages and platelets in humans.

As used herein, the term "Macrophage migration inhibitory factor" refers to one or more polypeptides present in a biological sample that are derived from the Macrophage migration inhibitory factor precursor (Swiss-Prot P 14174 (SEQ ID NO: 16))

```
            10         20         30         40         50         60
    MPMFIVNTNV PRASVPDGFL SELTQQLAQA TGKPPQYIAV HVVPDQLMAF GGSSEPCALC

            70         80         90        100        110
    SLHSIGKIGG AQNRSYSKLL CGLLAERLRI SPDRVYINYY DMNAANVGWN NSTFA
```

The following domains have been identified in Macrophage migration inhibitory factor:

| Residues | Length | Domain ID |
|---|---|---|
| 1 | 1 | initiator methionine |
| 2-115 | 114 | Macrophage migration inhibitory factor |

As used herein, the term "Galectin-3" refers to one or more polypeptides present in a biological sample that are derived from the Galectin-3 precursor (Swiss-Prot P 17931 (SEQ ID NO: 17))

```
            10         20         30         40         50         60
    MADNFSLHDA LSGSGNPNPQ GWPGAWGNQP AGAGGYPGAS YPGAYPGQAP PGAYPGQAPP

            70         80         90        100        110        120
    GAYPGAPGAY PGAPAPGVYP GPPSGPGAYP SSGQPSATGA YPATGPYGAP AGPLIVPYNL

           130        140        150        160        170        180
    PLPGGVVPRM LITILGTVKP NANRIALDFQ RGNDVAFHFN PRFNENNRRV IVCNTKLDNN

           190        200        210        220        230        240
    WGREERQSVF PFESGKPFKI QVLVEPDHFK VAVNDAHLLQ YNHRVKKLNE ISKLGISGDI

           250
    DLTSASYTMI
```

The following domains have been identified in Galectin-3:

| Residues | Length | Domain ID |
|---|---|---|
| 1 | 1 | initiator methionine |
| 115-331 | 217 | Galectin-3 |

As used herein, the term "Transforming growth factor beta-3" refers to one or more polypeptides present in a biological sample that are derived from the Transforming growth factor beta-3 precursor (Swiss-Prot P10600 (SEQ ID

NO: 18))

```
                10          20          30          40          50          60
       MKMHLQRALV  VLALLNFATV  SLSLSTCTTL  DFGHIKKKRV  EAIRGQILSK  LRLTSPPEPT

                70          80          90         100         110         120
       VMTHVPYQVL  ALYNSTRELL  EEMHGEREEG  CTQENTESEY  YAKEIHKFDM  IQGLAEHNEL

               130         140         150         160         170         180
       AVCPKGITSK  VFRFNVSSVE  KNRTNLFRAE  FRVLRVPNPS  SKRNEQRIEL  FQILRPDEHI

               190         200         210         220         230         240
       AKQRYIGGKN  LPTRGTAEWL  SFDVTDTVRE  WLLRRESNLG  LEISIHCPCH  TFQPNGDILE

               250         260         270         280         290         300
       NIHEVMEIKF  KGVDNEDDHG  RGDLGRLKKQ  KDHHNPHLIL  MMIPPHRLDN  PGQGGQRKKR

               310         320         330         340         350         360
       ALDTNYCFRN  LEENCCVRPL  YIDFRQDLGW  KWVHEPKGYY  ANFCSGPCPY  LRSADTTHST

               370         380         390         400         410
       VLGLYNTLNP  EASASPCCVP  QDLEPLTILY  YVGRTPKVEQ  LSNMVVKSCK  CS
```

Most preferably, the assay detects the pro-form of Transforming growth factor beta-3. The following domains have been identified in Transforming growth factor beta-3:

| Residues | Length | Domain ID |
|----------|--------|-----------|
| 1-20 | 20 | signal peptide |
| 21-300 | 280 | propeptide |
| 301-412 | 112 | Transforming growth factor beta-3 |

Heparan Sulfate is biosynthesized as a proteoglycan. HS comprises a domain structure of segregated blocks of repeating β-D-glucuronic acid-(1→4)-N-acetylated β-D-glucosamine disaccharides (NA domains) and blocks of highly sulfated, heparin-like α-L-iduronic acid-(1→4)-N-sulfated β-D-glucosamine disaccharides (NS domains), with a small proportion of mixed sequences of N-acetylated and N-sulfated disaccharides separating the two domains. HS proteoglycans are strategically located on cell surfaces and in the extracellular matrix. There are several families of HS proteoglycans, each with a different core protein. The HS polysaccharide chains of HS proteoglycans are attached to their core proteins via the same β-GlcA-(1,3)-β-Gal-(1,3)-β-Gal-(1,4)-β-Xyl tetrasaccharide linker as in heparin proteoglycan. *See, e.g.,* Rabenstein, Nat. Prod. Rep., 19: 312-331, 2002. As used herein, the term "Heparan Sulfate" refers to both free heparan sulfate chains and the heparan sulfate chains of HS proteoglycan.

As used herein, the term "Cadherin-3" refers to one or more polypeptides present in a biological sample that are derived from the Cadherin-3 precursor (Swiss-Prot P22223 (SEQ ID NO: 19))

```
           10         20         30         40         50         60
    MGLPRGPLAS LLLLQVCWLQ CAASEPCRAV FREAEVTLEA GGAEQEPGQA LGKVFMGCPG

           70         80         90        100        110        120
    QEPALFSTDN DDFTVRNGET VQERRSLKER NPLKIFPSKR ILRRHKRDWV VAPISVPENG

          130        140        150        160        170        180
    KGPFPQRLNQ LKSNKDRDTK IFYSITGPGA DSPPEGVFAV EKETGWLLLN KPLDREEIAK

          190        200        210        220        230        240
    YELFGHAVSE NGASVEDPMN ISIIVTDQND HKPKFTQDTF RGSVLEGVLP GTSVMQVTAT

          250        260        270        280        290        300
    DEDDAIYTYN GVVAYSIHSQ EPKDPHDLMF TIHRSTGTIS VISSGLDREK VPEYTLTIQA

          310        320        330        340        350        360
    TDMDGDGSTT TAVAVVEILD ANDNAPMFDP QKYEAHVPEN AVGHEVQRLT VTDLDAPNSP

          370        380        390        400        410        420
    AWRATYLIMG GDDGDHFTIT THPESNQGIL TTRKGLDFEA KNQHTLYVEV TNEAPFVLKL

          430        440        450        460        470        480
    PTSTATIVVH VEDVNEAPVF VPPSKVVEVQ EGIPTGEPVC VYTAEDPDKE NQKISYRILR

          490        500        510        520        530        540
    DPAGWLAMDP DSGQVTAVGT LDREDEQFVR NNIYEVMVLA MDNGSPPTTG TGTLLLTLID

          550        560        570        580        590        600
    VNDHGPVPEP RQITICNQSP VRQVLNITDK DLSPHTSPFQ AQLTDDSDIY WTAEVNEEGD

          610        620        630        640        650        660
    TVVLSLKKFL KQDTYDVHLS LSDHGNKEQL TVIRATVCDC HGHVETCPGP WKGGFILPVL

          670        680        690        700        710        720
    GAVLALLFLL LVLLLLVRKK RKIKEPLLLP EDDTRDNVFY YGEEGGGEED QDYDITQLHR

          730        740        750        760        770        780
    GLEARPEVVL RNDVAPTIIP TPMYRPRPAN PDEIGNFIIE NLKAANTDPT APPYDTLLVF

          790        800        810        820
    DYEGSGSDAA SLSSLTSSAS DQDQDYDYLN EWGSRFKKLA DMYGGGEDD
```

Most preferably, the Cadherin-3 assay detects one or more soluble forms of Cadherin-3. Cadherin-3 is a type I membrane protein having a large extracellular domain, most or all of which is present in soluble forms of Cadherin-3 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Cadherin-3:

| Residues | Length | Domain ID |
|---|---|---|
| 1-24 | 24 | Signal peptide |
| 25-107 | 83 | Propeptide |
| 108-654 | 722 | Cadherin-3 |
| 678-829 | 152 | Cytoplasmic domain |

(continued)

| Residues | Length | Domain ID |
|----------|--------|-----------|
| 655-677 | 23 | transmembrane domain |
| 108-654 | 547 | Extracellular domain |
| 761-829 | 69 | GRGERGSQRGNGGLQLARGRTRRS (SEQ ID NO: 20) in isoform 2 |

As used herein, the term "Complement C5" refers to one or more polypeptides present in a biological sample that are derived from the Complement C5 precursor (Swiss-Prot P01031 (SEQ ID NO: 21))

```
          10         20         30         40         50         60
MGLLGILCFL IFLGKTWGQE QTYVISAPKI FRVGASENIV IQVYGYTEAF DATISIKSYP

          70         80         90        100        110        120
DKKFSYSSGH VHLSSENKFQ NSAILTIQPK QLPGGQNPVS YVYLEVVSKH FSKSKRMPIT

         130        140        150        160        170        180
YDNGFLFIHT DKPVYTPDQS VKVRVYSLND DLKPAKRETV LTFIDPEGSE VDMVEEIDHI

         190        200        210        220        230        240
GIISFPDFKI PSNPRYGMWT IKAKYKEDFS TTGTAYFEVK EYVLPHFSVS IEPEYNFIGY

         250        260        270        280        290        300
KNFKNFEITI KARYFYNKVV TEADVYITFG IREDLKDDQK EMMQTAMQNT MLINGIAQVT

         310        320        330        340        350        360
FDSETAVKEL SYYSLEDLNN KYLYIAVTVI ESTGGFSEEA EIPGIKYVLS PYKLNLVATP
```

```
          370         380         390         400         410         420
LFLKPGIPYP  IKVQVKDSLD  QLVGGVPVTL  NAQTIDVNQE  TSDLDPSKSV  TRVDDGVASF

          430         440         450         460         470         480
VLNLPSGVTV  LEFNVKTDAP  DLPEENQARE  GYRAIAYSSL  SQSYLYIDWT  DNHKALLVGE

          490         500         510         520         530         540
HLNIIVTPKS  PYIDKITHYN  YLILSKGKII  HFGTREKFSD  ASYQSINIPV  TQNMVPSSRL

          550         560         570         580         590         600
LVYYIVTGEQ  TAELVSDSVW  LNIEEKCGNQ  LQVHLSPDAD  AYSPGQTVSL  NMATGMDSWV

          610         620         630         640         650         660
ALAAVDSAVY  GVQRGAKKPL  ERVFQFLEKS  DLGCGAGGGL  NNANVFHLAG  LTFLTNANAD

          670         680         690         700         710         720
DSQENDEPCK  EILRPRRTLQ  KKIEEIAAKY  KHSVVKKCCY  DGACVNNDET  CEQRAARISL

          730         740         750         760         770         780
GPRCIKAFTE  CCVVASQLRA  NISHKDMQLG  RLHMKTLLPV  SKPEIRSYFP  ESWLWEVHLV

          790         800         810         820         830         840
PRRKQLQFAL  PDSLTTWEIQ  GVGISNTGIC  VADTVKAKVF  KDVFLEMNIP  YSVVRGEQIQ

          850         860         870         880         890         900
LKGTVYNYRT  SGMQFCVKMS  AVEGICTSES  PVIDHQGTKS  SKCVRQKVEG  SSSHLVTFTV

          910         920         930         940         950         960
LPLEIGLHNI  NFSLETWFGK  EILVKTLRVV  PEGVKRESYS  GVTLDPRGIY  GTISRRKEFP

          970         980         990        1000        1010        1020
YRIPLDLVPK  TEIKRILSVK  GLLVCEILSA  VLSQEGINIL  THLPKGSAEA  ELMSVVPVFY

         1030        1040        1050        1060        1070        1080
VFHYLETGNH  WNIFHSDPLI  EKQKLKKKLK  EGMLSIMSYR  NADYSYSVWK  GGSASTWLTA

         1090        1100        1110        1120        1130        1140
FALRVLGQVN  KYVEQNQNSI  CNSLLWLVEN  YQLDNGSFKE  NSQYQPIKLQ  GTLPVEAREN

         1150        1160        1170        1180        1190        1200
SLYLTAFTVI  GIRKAFDICP  LVKIDTALIK  ADNFLLENTL  PAQSTFTLAI  SAYALSLGDK

         1210        1220        1230        1240        1250        1260
THPQFRSIVS  ALKREALVKG  NPPIYRFWKD  NLQHKDSSVP  NTGTARMVET  TAYALLTSLN

         1270        1280        1290        1300        1310        1320
LKDINYVNPV  IKWLSEEQRY  GGGFYSTQDT  INAIEGLTEY  SLLVKQLRLS  MDIDVSYKHK

         1330        1340        1350        1360        1370        1380
GALHNYKMTD  KNFLGRPVEV  LLNDDLIVST  GFGSGLATVH  VTTVVHKTST  SEEVCSFYLK

         1390        1400        1410        1420        1430        1440
IDTQDIEASH  YRGYGNSDYK  RIVACASYKP  SREESSSGSS  HAVMDISLPT  GISANEEDLK

         1450        1460        1470        1480        1490        1500
```

```
ALVEGVDQLF  TDYQIKDGHV  ILQLNSIPSS  DFLCVRFRIF  ELFEVGFLSP  ATFTVYEYHR

     1510        1520        1530        1540        1550        1560
PDKQCTMFYS  TSNIKIQKVC  EGAACKCVEA  DCGQMQEELD  LTISAETRKQ  TACKPEIAYA

     1570        1580        1590        1600        1610        1620
YKVSITSITV  ENVFVKYKAT  LLDIYKTGEA  VAEKDSEITF  IKKVTCTNAE  LVKGRQYLIM

     1630        1640        1650        1660        1670
GKEALQIKYN  FSFRYIYPLD  SLTWIEYWPR  DTTCSSCQAF  LANLDEFAED  IFLNGC
```

Most preferably, the C5 alpha chain, also known as C5a. The following domains have been identified in Complement C5:

| Residues | Length | Domain ID |
|---|---|---|
| 1-18 | 18 | signal peptide |
| 19-673 | 655 | Complement C5 beta chain |
| 674-677 | 4 | Propeptide |
| 678-1676 | 999 | Complement C5 alpha chain |
| 678-751 | 74 | Complement C5a anaphlatoxin |
| 752-1676 | 925 | Complement C5 alpha' chain |

As used herein, the term "Platelet factor 4" refers to one or more polypeptides present in a biological sample that are derived from the Platelet factor 4 precursor (Swiss-Prot P02776 (SEQ ID NO: 22))

```
        10          20          30          40          50          60
MSSAAGFCAS  RPGLLFLGLL  LLPLVVAFAS  AEAEEDGDLQ  CLCVKTTSQV  RPRHITSLEV

        70          80          90         100
IKAGPHCPTA  QLIATLKNGR  KICLDLQAPL  YKKIIKKLLE  S
```

The following domains have been identified in Platelet factor 4:

| Residues | Length | Domain ID |
|---|---|---|
| 1-31 | 31 | Signal peptide |
| 32-101 | 70 | Platelet factor 4 |
| 48-101 | 54 | Platelet factor 4, short form |

As used herein, the term "Platelet basic protein" refers to one or more polypeptides present in a biological sample that are derived from the Platelet basic protein precursor (Swiss-Prot P02775 (SEQ ID NO: 23))

```
        10          20          30          40          50          60
MSLRLDTTPS  CNSARPLHAL  QVLLLLSLLL  TALASSTKGQ  TKRNLAKGKE  ESLDSDLYAE

        70          80          90         100         110         120
LRCMCIKTTS  GIHPKNIQSL  EVIGKGTHCN  QVEVIATLKD  GRKICLDPDA  PRIKKIVQKK


LAGDESAD
```

The following domains have been identified in Platelet basic protein:

| Residues | Length | Domain ID |
|----------|--------|-----------|
| 1-34 | 34 | Signal peptide |
| 35-128 | 94 | Platelet basic protein |
| 44-128 | 85 | Connective tissue-activating peptide III |
| 44-126 | 83 | TC-2 |
| 44-124 | 81 | Connective tissue-activating peptide III(1-81) |
| 48-128 | 81 | Beta-thromboglobulin |
| 55-128 | 74 | Neutrophil-activating peptide 2(74) |
| 56-128 | 73 | Neutrophil-activating peptide 2(73) |
| 59-128 | 70 | Neutrophil-activating peptide 2 |
| 59-126 | 68 | TC-1 |
| 59-124 | 66 | Neutrophil-activating peptide 2(66) |
| 59-121 | 63 | Neutrophil-activating peptide 2(63) |

As used herein, the term "Stromelysin-2" refers to one or more polypeptides present in a biological sample that are derived from the Stromelysin-2 precursor (Swiss-Prot P09238 (SEQ ID NO: 24))

```
           10         20         30         40         50         60
    MMHLAFLVLL CLPVCSAYPL SGAAKEEDSN KDLAQQYLEK YYNLEKDVKQ FRRKDSNLIV

           70         80         90        100        110        120
    KKIQGMQKFL GLEVTGKLDT DTLEVMRKPR CGVPDVGHFS SFPGMPKWRK THLTYRIVNY

          130        140        150        160        170        180
    TPDLPRDAVD SAIEKALKVW EEVTPLTFSR LYEGEADIMI SFAVKEHGDF YSFDGPGHSL

          190        200        210        220        230        240
    AHAYPPGPGL YGDIHFDDDE KWTEDASGTN LFLVAAHELG HSLGLFHSAN TEALMYPLYN

          250        260        270        280        290        300
    SFTELAQFRL SQDDVNGIQS LYGPPPASTE EPLVPTKSVP SGSEMPAKCD PALSFDAIST

          310        320        330        340        350        360
    LRGEYLFFKD RYFWRRSHWN PEPEFHLISA FWPSLPSYLD AAYEVNSRDT VFIFKGNEFW

          370        380        390        400        410        420
    AIRGNEVQAG YPRGIHTLGF PPTIRKIDAA VSDKEKKKTY FFAADKYWRF DENSQSMEQG

          430        440        450        460        470
    FPRLIADDFP GVEPKVDAVL QAFGFFYFFS GSSQFEFDPN ARMVTHILKS NSWLHC
```

The following domains have been identified in the Stromelysin-2 precursor:

| Residue# | Length | Domain description |
|----------|--------|--------------------|
| 1-17 | 17 | Signal peptide |
| 99-476 | 378 | Stromelysin-2 |
| 18-98 | 81 | Activation peptide |

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker disclosed herein, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.*) as well as molecules that may be indirectly detected by production of a detectable reaction product (*e.g.*, enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, kits for the analysis of the described kidney injury markers are disclosed. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker as disclosed herein. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: $r/c = K(n-r)$: where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.*, in sandwich assays) may interfere with one another sterically, *etc.,* assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s). These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Betagalactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q 15848); Alkaline phosphatase (P05186); Aminopeptidase N (P15144); CalbindinD28k (P05937); Cystatin C (P01034); 8 subunit of F1FO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s).

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers described herein may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, and are exemplary.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria
males and females 18 years of age or older;
undergoing a radiographic / angiographic procedure (such as a CT scan or coronary intervention) involving the intravascular administration of contrast media;
expected to be hospitalized for at least 48 hours after contrast administration.
able and willing to provide written informed consent for study participation and to comply with all study procedures.

Exclusion Criteria
renal transplant recipients;
acutely worsening renal function prior to the contrast procedure;
already receiving dialysis (either acute or chronic) or in imminent need of dialysis at enrollment;
expected to undergo a major surgical procedure (such as involving cardiopulmonary bypass) or an additional imaging procedure with contrast media with significant risk for further renal insult within the 48 hrs following contrast administration;
participation in an interventional clinical study with an experimental therapy within the previous 30 days;
known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1 %, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria
males and females 18 years of age or older;
undergoing cardiovascular surgery;
Toronto/Ottawa Predictive Risk Index for Renal Replacement risk score of at least 2 (Wijeysundera et al., JAMA 297: 1801-9, 2007); and
able and willing to provide written informed consent for study participation and to comply with all study procedures.
Exclusion Criteria
known pregnancy;
previous renal transplantation;
acutely worsening renal function prior to enrollment (e.g., any category of RIFLE criteria);
already receiving dialysis (either acute or chronic) or in imminent need of dialysis at enrollment;
currently enrolled in another clinical study or expected to be enrolled in another clinical study within 7 days of cardiac surgery that involves drug infusion or a therapeutic intervention for AKI;
known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria
males and females 18 years of age or older;
Study population 1: approximately 300 patients that have at least one of:

shock (SBP < 90 mmHg and/or need for vasopressor support to maintain MAP > 60 mmHg and/or documented drop in SBP of at least 40 mmHg); and sepsis;

Study population 2: approximately 300 patients that have at least one of:

IV antibiotics ordered in computerized physician order entry (CPOE) within 24 hours of enrollment;

contrast media exposure within 24 hours of enrollment;
increased Intra-Abdominal Pressure with acute decompensated heart failure; and severe trauma as the primary reason for ICU admission and likely to be hospitalized in the ICU for 48 hours after enrollment;
Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria
known pregnancy;
institutionalized individuals;
previous renal transplantation;
known acutely worsening renal function prior to enrollment (e.g., any category of RIFLE criteria);
received dialysis (either acute or chronic) within 5 days prior to enrollment or in imminent need of dialysis at the time of enrollment;
known infection with human immunodeficiency virus (HIV) or a hepatitis virus;
meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards. Concentrations reported below are as follows: C-C motif chemokine 23 ng/mL; Transmembrane glycoprotein NMB (soluble form) ng/mL; Brain-derived neurotrophic factor pg/mL; Cathepsin S ng/mL; Transforming growth factor beta-2 (pro form assay) pg/mL; Urokinase-type plasminogen activator ng/mL; Angiopoietin-2 pg/mL; Matrilysin pg/mL; Carcinoembryonic antigen-related cell adhesion molecule 1 ng/mL; Creatine kinase MB ng/mL; Insulin $\mu$IU; Immunoglobulin M mg/mL; Immunoglobulin E ng/mL; Macrophage migration inhibitory factor pg/mL; Galectin-3 ng/mL; Transforming growth factor beta-3 (pro form assay) pg/mL; Heparan sulfate $\mu$g/mL; Cadherin-3 (soluble form) pg/mL; Complement C5 (C5a assay) ng/mL; Platelet factor 4 ng/mL; Platelet basic protein ng/mL; and Stromelysin-2 ng/mL.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.
Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Brain-derived neurotrophic factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.321 | 0.421 | 0.321 | 0.548 | 0.321 | 0.238 |
| Average | 3.23 | 1.41 | 3.23 | 16.5 | 3.23 | 0.621 |
| Stdev | 33.3 | 3.49 | 33.3 | 100 | 33.3 | 0.965 |
| p(t-test) | | 0.64 | | 0.036 | | 0.61 |
| Min | 1.00E-9 | 1.00E-9 | 1.00E-9 | 1.00E-9 | 1.00E-9 | 1.00E-9 |
| Max | 624 | 27.2 | 624 | 732 | 624 | 5.01 |
| n (Samp) | 361 | 75 | 361 | 91 | 361 | 42 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 191 | 75 | 191 | 91 | 191 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.398 | 0.404 | 0.398 | 0.896 | 0.398 | 0.260 |
| Average | 3.16 | 1.74 | 3.16 | 21.5 | 3.16 | 0.589 |
| Stdev | 32.4 | 2.98 | 32.4 | 120 | 32.4 | 1.04 |
| p(t-test) | | 0.81 | | 0.0077 | | 0.70 |
| Min | 1.00E-9 | 1.00E-9 | 1.00E-9 | 1.00E-9 | 1.00E-9 | 1.00E-9 |
| Max | 624 | 11.7 | 624 | 732 | 624 | 5.01 |
| n (Samp) | 755 | 29 | 755 | 37 | 755 | 23 |
| n (Patient) | 296 | 29 | 296 | 37 | 296 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.768 | 0.383 | 0.506 | 0.383 | 0.403 |
| Average | 1.89 | 1.59 | 1.89 | 18.9 | 1.89 | 0.974 |
| Stdev | 6.96 | 3.59 | 6.96 | 108 | 6.96 | 2.02 |
| p(t-test) | | 0.73 | | 0.0055 | | 0.44 |
| Min | 1.00E-9 | 1.00E-9 | 1.00E-9 | 1.00E-9 | 1.00E-9 | 1.00E-9 |
| Max | 61.7 | 27.2 | 61.7 | 732 | 61.7 | 11.7 |
| n (Samp) | 316 | 65 | 316 | 78 | 316 | 35 |
| n (Patient) | 135 | 65 | 135 | 78 | 135 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.53 | 0.60 | 0.58 | 0.59 | 0.56 | 0.48 | 0.45 | 0.51 |
| SE | 0.037 | 0.055 | 0.040 | 0.034 | 0.050 | 0.037 | 0.048 | 0.063 | 0.052 |
| p | 0.049 | 0.63 | 0.016 | 0.024 | 0.073 | 0.090 | 0.71 | 0.40 | 0.91 |
| nCohort 1 | 361 | 755 | 316 | 361 | 755 | 316 | 361 | 755 | 316 |
| nCohort 2 | 75 | 29 | 65 | 91 | 37 | 78 | 42 | 23 | 35 |
| Cutoff 1 | 0.176 | 0.0967 | 0.219 | 0.0711 | 0.321 | 0.158 | 0.0249 | 0.0852 | 0.0967 |
| Sens 1 | 72% | 76% | 72% | 70% | 70% | 71% | 71% | 74% | 71% |
| Spec 1 | 39% | 29% | 40% | 33% | 44% | 34% | 26% | 27% | 32% |
| Cutoff 2 | 0.0356 | 0.0249 | 0.0852 | 0 | 0.0356 | 0 | 0 | 0.0249 | 0 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 81% | 83% | 80% | 100% | 81% | 100% | 100% | 83% | 100% |
| Spec 2 | 28% | 20% | 31% | 0% | 22% | 0% | 0% | 20% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.708 | 0.838 | 0.742 | 0.708 | 0.838 | 0.742 | 0.708 | 0.838 | 0.742 |
| Sens 4 | 45% | 38% | 51% | 49% | 57% | 46% | 31% | 17% | 37% |
| Spec 4 | 71% | 70% | 70% | 71% | 70% | 70% | 71% | 70% | 70% |
| Cutoff 5 | 1.03 | 1.26 | 1.06 | 1.03 | 1.26 | 1.06 | 1.03 | 1.26 | 1.06 |
| Sens 5 | 33% | 28% | 38% | 38% | 32% | 37% | 17% | 9% | 23% |
| Spec 5 | 80% | 80% | 80% | 80% | 80% | 80% | 80% | 80% | 80% |
| Cutoff 6 | 2.23 | 2.55 | 2.23 | 2.23 | 2.55 | 2.23 | 2.23 | 2.55 | 2.23 |
| Sens 6 | 15% | 24% | 17% | 18% | 16% | 21% | 5% | 4% | 9% |
| Spec 6 | 90% | 90% | 90% | 90% | 90% | 90% | 90% | 90% | 90% |
| OR Quart 2 | 1.4 | 1.0 | 1.3 | 0.94 | 0.086 | 0.74 | 0.80 | 1.5 | 0.56 |
| p Value | 0.35 | 1.0 | 0.53 | 0.86 | 0.020 | 0.43 | 0.64 | 0.52 | 0.29 |
| 95% CI of | 0.68 | 0.34 | 0.57 | 0.47 | 0.011 | 0.35 | 0.32 | 0.42 | 0.20 |
| OR Quart2 | 3.0 | 2.9 | 2.9 | 1.9 | 0.67 | 1.6 | 2.0 | 5.5 | 1.6 |
| OR Quart 3 | 1.1 | 0.71 | 1.0 | 0.82 | 0.72 | 0.64 | 1.0 | 1.8 | 0.99 |
| p Value | 0.84 | 0.56 | 1.0 | 0.59 | 0.48 | 0.25 | 1.0 | 0.36 | 0.98 |
| 95% CI of | 0.50 | 0.22 | 0.42 | 0.41 | 0.28 | 0.29 | 0.41 | 0.51 | 0.39 |
| OR Quart3 | 2.4 | 2.3 | 2.4 | 1.7 | 1.8 | 1.4 | 2.4 | 6.2 | 2.5 |
| OR Quart 4 | 2.2 | 1.5 | 2.6 | 2.1 | 1.6 | 1.9 | 1.0 | 1.5 | 0.88 |
| p Value | 0.026 | 0.46 | 0.014 | 0.021 | 0.24 | 0.056 | 0.98 | 0.52 | 0.79 |
| 95% CI of | 1.1 | 0.54 | 1.2 | 1.1 | 0.73 | 0.98 | 0.42 | 0.42 | 0.34 |
| OR Quart4 | 4.5 | 3.9 | 5.5 | 3.9 | 3.5 | 3.7 | 2.5 | 5.5 | 2.3 |

**Creatine Kinase-MB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00871 | 0.00728 | 0.00871 | 0.0113 | 0.00871 | 0.00872 |
| Average | 0.0121 | 0.0144 | 0.0121 | 0.0344 | 0.0121 | 0.0107 |
| Stdev | 0.0157 | 0.0221 | 0.0157 | 0.163 | 0.0157 | 0.00985 |
| p(t-test) | | 0.38 | | 0.032 | | 0.67 |
| Min | 0.00168 | 0.00168 | 0.00168 | 0.00168 | 0.00168 | 0.00168 |
| Max | 0.105 | 0.117 | 0.105 | 1.24 | 0.105 | 0.0423 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 255 | 48 | 255 | 57 | 255 | 27 |
| n (Patient) | 103 | 48 | 103 | 57 | 103 | 27 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00871 | 0.00448 | 0.00871 | 0.0127 | 0.00871 | 0.0101 |
| Average | 0.0160 | 0.00800 | 0.0160 | 0.0157 | 0.0160 | 0.0104 |
| Stdev | 0.0609 | 0.00844 | 0.0609 | 0.0149 | 0.0609 | 0.00764 |
| p(t-test) | | 0.60 | | 0.98 | | 0.74 |
| Min | 0.00168 | 0.00168 | 0.00168 | 0.00168 | 0.00168 | 0.00168 |
| Max | 1.24 | 0.0325 | 1.24 | 0.0697 | 1.24 | 0.0294 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00864 | 0.00976 | 0.00864 | 0.0110 | 0.00864 | 0.00872 |
| Average | 0.0126 | 0.0154 | 0.0126 | 0.0358 | 0.0126 | 0.0106 |
| Stdev | 0.0166 | 0.0223 | 0.0166 | 0.172 | 0.0166 | 0.00971 |
| p(t-test) | | 0.32 | | 0.050 | | 0.57 |
| Min | 0.00168 | 0.00168 | 0.00168 | 0.00168 | 0.00168 | 0.00168 |
| Max | 0.105 | 0.117 | 0.105 | 1.24 | 0.105 | 0.0423 |
| n (Samp) | 218 | 46 | 218 | 51 | 218 | 25 |
| n (Patient) | 87 | 46 | 87 | 51 | 87 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.42 | 0.51 | 0.56 | 0.61 | 0.54 | 0.51 | 0.52 | 0.50 |
| SE | 0.046 | 0.076 | 0.047 | 0.043 | 0.067 | 0.046 | 0.059 | 0.082 | 0.061 |
| p | 0.79 | 0.31 | 0.80 | 0.18 | 0.10 | 0.35 | 0.92 | 0.79 | 0.98 |
| nCohort 1 | 255 | 447 | 218 | 255 | 447 | 218 | 255 | 447 | 218 |
| nCohort 2 | 48 | 16 | 46 | 57 | 21 | 51 | 27 | 13 | 25 |
| Cutoff 1 | 0 | 0 | 0 | 0 | 0.00987 | 0 | 0.00647 | 0.00168 | 0 |
| Sens 1 | 100% | 100% | 100% | 100% | 71% | 100% | 70% | 77% | 100% |
| Spec 1 | 0% | 0% | 0% | 0% | 54% | 0% | 43% | 39% | 0% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.0136 | 0.0144 | 0.0138 | 0.0136 | 0.0144 | 0.0138 | 0.0136 | 0.0144 | 0.0138 |
| Sens 4 | 33% | 12% | 37% | 40% | 43% | 37% | 22% | 31% | 24% |
| Spec 4 | 70% | 72% | 70% | 70% | 72% | 70% | 70% | 72% | 70% |
| Cutoff 5 | 0.0159 | 0.0181 | 0.0163 | 0.0159 | 0.0181 | 0.0163 | 0.0159 | 0.0181 | 0.0163 |
| Sens 5 | 25% | 6% | 28% | 32% | 38% | 27% | 15% | 8% | 16% |
| Spec 5 | 81% | 82% | 80% | 81% | 82% | 80% | 81% | 82% | 80% |
| Cutoff 6 | 0.0245 | 0.0287 | 0.0287 | 0.0245 | 0.0287 | 0.0287 | 0.0245 | 0.0287 | 0.0287 |
| Sens 6 | 19% | 6% | 15% | 14% | 5% | 8% | 11% | 8% | 4% |
| Spec 6 | 91% | 90% | 91% | 91% | 90% | 91% | 91% | 90% | 91% |
| OR Quart 2 | 0.45 | 5.2 | 0.12 | 0.26 | 0.19 | 0.31 | 13 | >6.3 | 1.8 |
| p Value | 0.11 | 0.14 | 0.0010 | 0.0069 | 0.14 | 0.024 | 0.017 | <0.090 | 0.36 |
| 95% CI of | 0.17 | 0.60 | 0.033 | 0.097 | 0.022 | 0.11 | 1.6 | >0.75 | 0.50 |
| OR Quart2 | 1.2 | 45 | 0.42 | 0.69 | 1.7 | 0.86 | 100 | na | 6.6 |
| OR Quart 3 | 1.5 | 2.0 | 0.44 | 0.56 | 1.4 | 0.77 | 10 | >6.3 | 2.1 |
| p Value | 0.33 | 0.57 | 0.062 | 0.16 | 0.56 | 0.53 | 0.030 | <0.090 | 0.24 |
| 95% CI of | 0.68 | 0.18 | 0.19 | 0.25 | 0.44 | 0.34 | 1.3 | >0.75 | 0.60 |
| OR Quart3 | 3.2 | 23 | 1.0 | 1.3 | 4.6 | 1.8 | 83 | na | 7.4 |
| OR Quart 4 | 0.53 | 8.6 | 0.67 | 1.1 | 1.6 | 0.98 | 6.4 | >1.0 | 1.5 |
| p Value | 0.18 | 0.044 | 0.32 | 0.85 | 0.40 | 0.96 | 0.091 | <1.00 | 0.53 |
| 95% CI of | 0.21 | 1.1 | 0.30 | 0.52 | 0.52 | 0.44 | 0.75 | >0.062 | 0.41 |
| OR Quart4 | 1.3 | 70 | 1.5 | 2.2 | 5.2 | 2.2 | 54 | na | 5.7 |

**Immunoglobulin E**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0560 | 0.424 | 0.0560 | 0.0560 | 0.0560 | 0.0560 |
| Average | 0.854 | 2.10 | 0.854 | 2.90 | 0.854 | 1.39 |
| Stdev | 1.58 | 2.59 | 1.58 | 7.91 | 1.58 | 2.22 |
| p(t-test) | | 1.3E-5 | | 1.6E-4 | | 0.11 |
| Min | 0.0560 | 0.0560 | 0.0560 | 0.0560 | 0.0560 | 0.0560 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 8.45 | 7.62 | 8.45 | 58.4 | 8.45 | 7.23 |
| n (Samp) | 255 | 48 | 255 | 57 | 255 | 27 |
| n (Patient) | 103 | 48 | 103 | 57 | 103 | 27 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0560 | 0.106 | 0.0560 | 0.747 | 0.0560 | 0.0560 |
| Average | 1.29 | 1.14 | 1.29 | 1.87 | 1.29 | 0.986 |
| Stdev | 3.33 | 2.05 | 3.33 | 2.51 | 3.33 | 2.12 |
| p(t-test) | | 0.86 | | 0.43 | | 0.74 |
| Min | 0.0560 | 0.0560 | 0.0560 | 0.0560 | 0.0560 | 0.0560 |
| Max | 58.4 | 6.97 | 58.4 | 8.64 | 58.4 | 7.08 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0560 | 0.0970 | 0.0560 | 0.0560 | 0.0560 | 0.0560 |
| Average | 0.861 | 2.01 | 0.861 | 2.66 | 0.861 | 1.43 |
| Stdev | 1.59 | 2.59 | 1.59 | 8.29 | 1.59 | 2.23 |
| p(t-test) | | 1.1E-4 | | 0.0030 | | 0.10 |
| Min | 0.0560 | 0.0560 | 0.0560 | 0.0560 | 0.0560 | 0.0560 |
| Max | 9.65 | 7.62 | 9.65 | 58.4 | 9.65 | 7.23 |
| n (Samp) | 218 | 46 | 218 | 51 | 218 | 25 |
| n (Patient) | 87 | 46 | 87 | 51 | 87 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.51 | 0.59 | 0.59 | 0.58 | 0.56 | 0.55 | 0.43 | 0.55 |
| SE | 0.046 | 0.074 | 0.048 | 0.043 | 0.067 | 0.046 | 0.060 | 0.084 | 0.062 |
| p | 0.0073 | 0.86 | 0.051 | 0.034 | 0.21 | 0.22 | 0.43 | 0.43 | 0.47 |
| nCohort 1 | 255 | 447 | 218 | 255 | 447 | 218 | 255 | 447 | 218 |
| nCohort 2 | 48 | 16 | 46 | 57 | 21 | 51 | 27 | 13 | 25 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 1 | 0% | 0% | 0% | 0% | 0% | 0% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.499 | 0.784 | 0.473 | 0.499 | 0.784 | 0.473 | 0.499 | 0.784 | 0.473 |
| Sens 4 | 50% | 31% | 46% | 46% | 48% | 39% | 41% | 23% | 40% |
| Spec 4 | 70% | 70% | 71% | 70% | 70% | 71% | 70% | 70% | 71% |
| Cutoff 5 | 1.16 | 2.34 | 1.37 | 1.16 | 2.34 | 1.37 | 1.16 | 2.34 | 1.37 |
| Sens 5 | 42% | 12% | 39% | 40% | 33% | 33% | 37% | 15% | 32% |
| Spec 5 | 80% | 80% | 80% | 80% | 80% | 80% | 80% | 80% | 80% |
| Cutoff 6 | 3.24 | 4.59 | 3.40 | 3.24 | 4.59 | 3.40 | 3.24 | 4.59 | 3.40 |
| Sens 6 | 29% | 12% | 28% | 30% | 14% | 22% | 15% | 8% | 16% |
| Spec 6 | 90% | 90% | 90% | 90% | 90% | 90% | 90% | 90% | 90% |
| OR Quart 2 | >18 | >8.5 | 0.16 | 0.31 | 0 | 0.48 | 1.5 | 1.0 | 0.98 |
| p Value | <0.0054 | <0.045 | 0.0016 | 0.0096 | na | 0.093 | 0.43 | 1.0 | 0.97 |
| 95% CI of | >2.4 | >1.0 | 0.051 | 0.13 | na | 0.20 | 0.52 | 0.14 | 0.32 |
| OR Quart2 | na | na | 0.50 | 0.75 | na | 1.1 | 4.6 | 7.2 | 3.0 |
| OR Quart 3 | >14 | >4.1 | 0.20 | 0.15 | 0.42 | 0.22 | 0.31 | 0 | 0.26 |
| p Value | <0.012 | <0.21 | 0.0031 | 8.1E-4 | 0.16 | 0.0050 | 0.16 | na | 0.099 |
| 95% CI of | >1.8 | >0.45 | 0.071 | 0.048 | 0.13 | 0.076 | 0.061 | na | 0.051 |
| OR Quart3 | na | na | 0.58 | 0.45 | 1.4 | 0.63 | 1.6 | na | 1.3 |
| OR Quart 4 | >29 | >4.1 | 0.93 | 1.2 | 0.88 | 0.98 | 1.7 | 4.8 | 1.3 |
| p Value | <0.0012 | <0.21 | 0.85 | 0.60 | 0.80 | 0.96 | 0.31 | 0.048 | 0.62 |
| 95% CI of | >3.7 | >0.45 | 0.43 | 0.60 | 0.33 | 0.46 | 0.60 | 1.0 | 0.45 |
| OR Quart4 | na | na | 2.0 | 2.4 | 2.4 | 2.1 | 5.1 | 23 | 3.8 |

**Immunoglobulin M**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.48E-5 | 9.38E-5 | 9.48E-5 | 0.000145 | 9.48E-5 | 6.82E-5 |
| Average | 0.000504 | 0.000218 | 0.000504 | 0.000685 | 0.000504 | 0.000208 |
| Stdev | 0.00147 | 0.000450 | 0.00147 | 0.00253 | 0.00147 | 0.000240 |
| p(t-test) | | 0.18 | | 0.47 | | 0.30 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 3.78E-8 | 8.56E-6 | 3.78E-8 | 3.78E-8 | 3.78E-8 | 3.78E-8 |
| Max | 0.0120 | 0.00293 | 0.0120 | 0.0186 | 0.0120 | 0.000848 |
| n (Samp) | 255 | 48 | 255 | 57 | 255 | 27 |
| n (Patient) | 103 | 48 | 103 | 57 | 103 | 27 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.48E-5 | 8.78E-5 | 9.48E-5 | 0.000320 | 9.48E-5 | 0.000110 |
| Average | 0.000466 | 0.000511 | 0.000466 | 0.000653 | 0.000466 | 0.000227 |
| Stdev | 0.00155 | 0.000800 | 0.00155 | 0.00117 | 0.00155 | 0.000257 |
| p(t-test) | | 0.91 | | 0.58 | | 0.58 |
| Min | 3.78E-8 | 1.74E-5 | 3.78E-8 | 3.78E-8 | 3.78E-8 | 3.78E-8 |
| Max | 0.0186 | 0.00293 | 0.0186 | 0.00464 | 0.0186 | 0.000848 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.54E-5 | 0.000113 | 9.54E-5 | 0.000143 | 9.54E-5 | 5.27E-5 |
| Average | 0.000426 | 0.000182 | 0.000426 | 0.000608 | 0.000426 | 0.000304 |
| Stdev | 0.00130 | 0.000212 | 0.00130 | 0.00259 | 0.00130 | 0.000595 |
| p(t-test) | | 0.21 | | 0.47 | | 0.64 |
| Min | 3.78E-8 | 8.56E-6 | 3.78E-8 | 3.78E-8 | 3.78E-8 | 1.15E-5 |
| Max | 0.0120 | 0.00109 | 0.0120 | 0.0186 | 0.0120 | 0.00293 |
| n (Samp) | 218 | 46 | 218 | 51 | 218 | 25 |
| n (Patient) | 87 | 46 | 87 | 51 | 87 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.55 | 0.53 | 0.55 | 0.63 | 0.56 | 0.51 | 0.56 | 0.52 |
| SE | 0.046 | 0.075 | 0.047 | 0.043 | 0.067 | 0.046 | 0.059 | 0.083 | 0.062 |
| p | 0.89 | 0.47 | 0.53 | 0.25 | 0.057 | 0.16 | 0.90 | 0.50 | 0.70 |
| nCohort 1 | 255 | 447 | 218 | 255 | 447 | 218 | 255 | 447 | 218 |
| nCohort 2 | 48 | 16 | 46 | 57 | 21 | 51 | 27 | 13 | 25 |
| Cutoff 1 | 4.38E-5 | 3.77E-5 | 5.73E-5 | 4.60E-5 | 8.31E-5 | 4.71E-5 | 4.83E-5 | 8.08E-5 | 4.60E-5 |
| Sens 1 | 71% | 75% | 72% | 70% | 71% | 71% | 70% | 77% | 72% |
| Spec 1 | 33% | 30% | 40% | 36% | 47% | 39% | 36% | 47% | 39% |
| Cutoff 2 | 2.18E-5 | 3.48E-5 | 3.47E-5 | 3.55E-5 | 3.55E-5 | 3.96E-5 | 3.25E-5 | 5.91E-5 | 3.69E-5 |
| Sens 2 | 83% | 81% | 80% | 81% | 81% | 80% | 85% | 85% | 80% |
| Spec 2 | 22% | 29% | 29% | 30% | 30% | 32% | 28% | 42% | 32% |
| Cutoff 3 | 1.88E-5 | 1.93E-5 | 2.10E-5 | 1.88E-5 | 2.41E-5 | 2.14E-5 | 1.74E-5 | 3.77E-5 | 2.56E-5 |
| Sens 3 | 92% | 94% | 91% | 91% | 90% | 90% | 93% | 92% | 92% |
| Spec 3 | 20% | 18% | 20% | 20% | 22% | 22% | 20% | 30% | 26% |
| Cutoff 4 | 0.000227 | 0.000227 | 0.000238 | 0.000227 | 0.000227 | 0.000238 | 0.000227 | 0.000227 | 0.000238 |
| Sens 4 | 23% | 44% | 24% | 39% | 57% | 37% | 37% | 31% | 36% |
| Spec 4 | 70% | 70% | 70% | 70% | 70% | 70% | 70% | 70% | 70% |
| Cutoff 5 | 0.000447 | 0.000418 | 0.000429 | 0.000447 | 0.000418 | 0.000429 | 0.000447 | 0.000418 | 0.000429 |
| Sens 5 | 6% | 31% | 9% | 21% | 33% | 22% | 22% | 23% | 24% |
| Spec 5 | 80% | 80% | 80% | 80% | 80% | 80% | 80% | 80% | 80% |
| Cutoff 6 | 0.000994 | 0.000755 | 0.000708 | 0.000994 | 0.000755 | 0.000708 | 0.000994 | 0.000755 | 0.000708 |
| Sens 6 | 4% | 25% | 4% | 9% | 14% | 10% | 0% | 8% | 8% |
| Spec 6 | 90% | 90% | 90% | 90% | 90% | 90% | 90% | 90% | 90% |
| OR Quart 2 | 1.8 | 1.7 | 1.6 | 2.1 | 1.3 | 2.7 | 3.4 | 4.1 | 7.9 |
| p Value | 0.19 | 0.48 | 0.33 | 0.090 | 0.70 | 0.044 | 0.045 | 0.21 | 0.0086 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.75 | 0.39 | 0.61 | 0.89 | 0.29 | 1.0 | 1.0 | 0.45 | 1.7 |
| OR Quart2 | 4.5 | 7.2 | 4.2 | 5.1 | 6.1 | 7.0 | 11 | 37 | 37 |
| OR Quart 3 | 1.5 | 0.65 | 2.5 | 1.8 | 1.3 | 2.1 | 1.0 | 5.2 | 1.5 |
| p Value | 0.36 | 0.65 | 0.050 | 0.19 | 0.70 | 0.15 | 1.0 | 0.14 | 0.66 |
| 95% CI of | 0.61 | 0.11 | 1.0 | 0.75 | 0.29 | 0.77 | 0.24 | 0.60 | 0.24 |
| OR Quart3 | 3.8 | 4.0 | 6.3 | 4.5 | 6.1 | 5.6 | 4.2 | 45 | 9.3 |
| OR Quart 4 | 1.3 | 2.0 | 1.1 | 2.0 | 3.6 | 2.4 | 1.8 | 3.1 | 3.8 |
| p Value | 0.61 | 0.32 | 0.80 | 0.13 | 0.059 | 0.073 | 0.36 | 0.34 | 0.11 |
| 95% CI of | 0.50 | 0.50 | 0.41 | 0.82 | 0.95 | 0.92 | 0.50 | 0.31 | 0.75 |
| OR Quart4 | 3.3 | 8.3 | 3.2 | 4.8 | 13 | 6.4 | 6.5 | 30 | 19 |

**Insulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 84.5 | 208 | 84.5 | 555 | nd | nd |
| Average | 1210 | 2750 | 1210 | 3810 | nd | nd |
| Stdev | 3710 | 4060 | 3710 | 6050 | nd | nd |
| p(t-test) | | 0.13 | | 0.031 | nd | nd |
| Min | 9.87 | 0.118 | 9.87 | 1.00E-9 | nd | nd |
| Max | 18900 | 13500 | 18900 | 22400 | nd | nd |
| n (Samp) | 53 | 19 | 53 | 19 | nd | nd |
| n (Patient) | 41 | 19 | 41 | 19 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 91.1 | 328 | 91.1 | 360 | nd | nd |
| Average | 956 | 2920 | 956 | 4880 | nd | nd |
| Stdev | 2980 | 4200 | 2980 | 12000 | nd | nd |
| p(t-test) | | 0.052 | | 0.042 | nd | nd |
| Min | 9.87 | 9.87 | 9.87 | 27.1 | nd | nd |
| Max | 18700 | 13500 | 18700 | 53000 | nd | nd |
| n (Samp) | 45 | 15 | 45 | 20 | nd | nd |
| n (Patient) | 34 | 15 | 34 | 20 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.60 | 0.71 | nd | 0.68 | nd | nd | nd |
| SE | 0.078 | nd | 0.087 | 0.074 | nd | 0.075 | nd | nd | nd |
| p | 0.25 | nd | 0.25 | 0.0055 | nd | 0.018 | nd | nd | nd |
| nCohort 1 | 53 | nd | 45 | 53 | nd | 45 | nd | nd | nd |
| nCohort 2 | 19 | nd | 15 | 19 | nd | 20 | nd | nd | nd |
| Cutoff 1 | 69.3 | nd | 69.3 | 107 | nd | 102 | nd | nd | nd |
| Sens 1 | 74% | nd | 73% | 74% | nd | 75% | nd | nd | nd |
| Spec 1 | 45% | nd | 40% | 57% | nd | 53% | nd | nd | nd |
| Cutoff 2 | 16.1 | nd | 30.7 | 74.3 | nd | 86.9 | nd | nd | nd |
| Sens 2 | 84% | nd | 80% | 84% | nd | 80% | nd | nd | nd |
| Spec 2 | 8% | nd | 20% | 49% | nd | 49% | nd | nd | nd |
| Cutoff 3 | 0.118 | nd | 12.6 | 23.8 | nd | 65.1 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | 95% | nd | 93% | 95% | nd | 90% | nd | nd | nd |
| Spec 3 | 0% | nd | 7% | 13% | nd | 36% | nd | nd | nd |
| Cutoff 4 | 320 | nd | 338 | 320 | nd | 338 | nd | nd | nd |
| Sens 4 | 47% | nd | 47% | 58% | nd | 50% | nd | nd | nd |
| Spec 4 | 72% | nd | 71% | 72% | nd | 71% | nd | nd | nd |
| Cutoff 5 | 590 | nd | 539 | 590 | nd | 539 | nd | nd | nd |
| Sens 5 | 42% | nd | 47% | 47% | nd | 45% | nd | nd | nd |
| Spec 5 | 81% | nd | 80% | 81% | nd | 80% | nd | nd | nd |
| Cutoff 6 | 1560 | nd | 1490 | 1560 | nd | 1490 | nd | nd | nd |
| Sens 6 | 42% | nd | 47% | 42% | nd | 35% | nd | nd | nd |
| Spec 6 | 91% | nd | 91% | 91% | nd | 91% | nd | nd | nd |
| OR Quart 2 | 0.32 | nd | 0.69 | 2.3 | nd | 4.2 | nd | nd | nd |
| p Value | 0.22 | nd | 0.67 | 0.38 | nd | 0.12 | nd | nd | nd |
| 95% CI of | 0.054 | nd | 0.12 | 0.36 | nd | 0.70 | nd | nd | nd |
| OR Quart2 | 2.0 | nd | 3.8 | 14 | nd | 25 | nd | nd | nd |
| OR Quart 3 | 0.74 | nd | 0.20 | 3.1 | nd | 2.3 | nd | nd | nd |
| p Value | 0.70 | nd | 0.17 | 0.22 | nd | 0.37 | nd | nd | nd |
| 95% CI of | 0.16 | nd | 0.019 | 0.51 | nd | 0.36 | nd | nd | nd |
| OR Quart3 | 3.4 | nd | 2.0 | 19 | nd | 15 | nd | nd | nd |
| OR Quart 4 | 2.1 | nd | 2.4 | 6.4 | nd | 6.2 | nd | nd | nd |
| p Value | 0.30 | nd | 0.26 | 0.036 | nd | 0.042 | nd | nd | nd |
| 95% CI of | 0.52 | nd | 0.52 | 1.1 | nd | 1.1 | nd | nd | nd |
| OR Quart4 | 8.3 | nd | 11 | 36 | nd | 36 | nd | nd | nd |

**Macrophage migration inhibitory factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 14900 | 11400 | 14900 | 16700 | 14900 | 12400 |
| Average | 20300 | 19800 | 20300 | 19900 | 20300 | 15000 |
| Stdev | 18800 | 17600 | 18800 | 17500 | 18800 | 12500 |
| p(t-test) |  | 0.86 |  | 0.88 |  | 0.17 |
| Min | 380 | 272 | 380 | 50.5 | 380 | 325 |
| Max | 107000 | 50000 | 107000 | 62800 | 107000 | 49800 |
| n (Samp) | 117 | 47 | 117 | 53 | 117 | 26 |
| n (Patient) | 95 | 47 | 95 | 53 | 95 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14000 | 4240 | 14000 | 7430 | 14000 | 12600 |
| Average | 20400 | 10100 | 20400 | 12700 | 20400 | 18200 |
| Stdev | 18500 | 12500 | 18500 | 12900 | 18500 | 15900 |
| p(t-test) | | 0.042 | | 0.079 | | 0.68 |
| Min | 220 | 1100 | 220 | 50.5 | 220 | 2400 |
| Max | 107000 | 39600 | 107000 | 50000 | 107000 | 50000 |
| n (Samp) | 257 | 14 | 257 | 19 | 257 | 13 |
| n (Patient) | 154 | 14 | 154 | 19 | 154 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14600 | 14700 | 14600 | 16700 | 14600 | 13600 |
| Average | 20900 | 21000 | 20900 | 20700 | 20900 | 16600 |
| Stdev | 19800 | 17100 | 19800 | 17700 | 19800 | 13800 |
| p(t-test) | | 0.97 | | 0.96 | | 0.32 |
| Min | 380 | 272 | 380 | 676 | 380 | 325 |
| Max | 107000 | 50000 | 107000 | 62800 | 107000 | 49800 |
| n (Samp) | 106 | 44 | 106 | 48 | 106 | 23 |
| n (Patient) | 82 | 44 | 82 | 48 | 82 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.31 | 0.53 | 0.50 | 0.38 | 0.51 | 0.44 | 0.49 | 0.45 |
| SE | 0.050 | 0.080 | 0.052 | 0.048 | 0.071 | 0.051 | 0.064 | 0.083 | 0.068 |
| p | 0.85 | 0.019 | 0.61 | 0.97 | 0.086 | 0.77 | 0.32 | 0.89 | 0.49 |
| nCohort 1 | 117 | 257 | 106 | 117 | 257 | 106 | 117 | 257 | 106 |
| nCohort 2 | 47 | 14 | 44 | 53 | 19 | 48 | 26 | 13 | 23 |
| Cutoff 1 | 6110 | 2930 | 7370 | 6720 | 3600 | 7040 | 6680 | 6870 | 3800 |
| Sens 1 | 70% | 71% | 70% | 72% | 74% | 71% | 73% | 77% | 74% |
| Spec 1 | 26% | 16% | 34% | 27% | 18% | 32% | 27% | 29% | 23% |
| Cutoff 2 | 3370 | 1510 | 4990 | 2770 | 1830 | 4910 | 3800 | 4210 | 2320 |
| Sens 2 | 81% | 86% | 82% | 81% | 84% | 81% | 81% | 85% | 83% |
| Spec 2 | 15% | 9% | 28% | 15% | 11% | 27% | 19% | 22% | 13% |
| Cutoff 3 | 1950 | 1330 | 2770 | 1330 | 590 | 1330 | 2040 | 3370 | 1010 |
| Sens 3 | 91% | 93% | 91% | 91% | 95% | 92% | 92% | 92% | 91% |
| Spec 3 | 12% | 9% | 14% | 8% | 3% | 6% | 13% | 17% | 6% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 4 | 24200 | 27400 | 28500 | 24200 | 27400 | 28500 | 24200 | 27400 | 28500 |
| Sens 4 | 38% | 14% | 32% | 30% | 11% | 27% | 23% | 23% | 22% |
| Spec 4 | 70% | 70% | 71% | 70% | 70% | 71% | 70% | 70% | 71% |
| Cutoff 5 | 36100 | 36200 | 38000 | 36100 | 36200 | 38000 | 36100 | 36200 | 38000 |
| Sens 5 | 19% | 7% | 18% | 19% | 5% | 19% | 8% | 15% | 9% |
| Spec 5 | 80% | 80% | 80% | 80% | 80% | 80% | 80% | 80% | 80% |
| Cutoff 6 | 50000 | 50000 | 50000 | 50000 | 50000 | 50000 | 50000 | 50000 | 50000 |
| Sens 6 | 0% | 0% | 0% | 2% | 0% | 2% | 0% | 0% | 0% |
| Spec 6 | 97% | 98% | 97% | 97% | 98% | 97% | 97% | 98% | 97% |
| OR Quart 2 | 0.61 | 0.49 | 2.1 | 1.0 | 3.2 | 1.8 | 1.9 | 1.0 | 3.3 |
| p Value | 0.32 | 0.57 | 0.15 | 0.94 | 0.16 | 0.24 | 0.33 | 0.99 | 0.099 |
| 95% CI of | 0.23 | 0.044 | 0.76 | 0.42 | 0.62 | 0.67 | 0.51 | 0.20 | 0.80 |
| OR Quart2 | 1.6 | 5.6 | 5.9 | 2.6 | 16 | 4.9 | 7.3 | 5.2 | 14 |
| OR Quart 3 | 0.89 | 2.1 | 1.5 | 0.80 | 2.1 | 1.7 | 2.3 | 1.4 | 1.9 |
| p Value | 0.81 | 0.41 | 0.43 | 0.64 | 0.41 | 0.31 | 0.21 | 0.70 | 0.43 |
| 95% CI of | 0.35 | 0.36 | 0.53 | 0.32 | 0.36 | 0.61 | 0.62 | 0.29 | 0.40 |
| OR Quart3 | 2.3 | 12 | 4.4 | 2.0 | 12 | 4.6 | 8.4 | 6.3 | 8.5 |
| OR Quart 4 | 1.0 | 3.8 | 1.5 | 0.93 | 3.8 | 1.4 | 2.0 | 1.0 | 2.8 |
| p Value | 1.0 | 0.10 | 0.47 | 0.87 | 0.11 | 0.49 | 0.31 | 0.99 | 0.16 |
| 95% CI of | 0.39 | 0.77 | 0.52 | 0.37 | 0.76 | 0.52 | 0.53 | 0.20 | 0.65 |
| OR Quart4 | 2.5 | 19 | 4.2 | 2.3 | 19 | 3.9 | 7.6 | 5.2 | 12 |

**Matrilysin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 10000 | 14100 | 10000 | 16400 | 10000 | 12000 |
| Average | 18300 | 28300 | 18300 | 32500 | 18300 | 20300 |
| Stdev | 26400 | 49000 | 26400 | 59000 | 26400 | 25500 |
| p(t-test) |  | 0.012 |  | 6.8E-4 |  | 0.63 |
| Min | 1.00E-9 | 181 | 1.00E-9 | 1.00E-9 | 1.00E-9 | 183 |
| Max | 261000 | 408000 | 261000 | 406000 | 261000 | 150000 |
| n (Samp) | 360 | 75 | 360 | 91 | 360 | 43 |
| n (Patient) | 191 | 75 | 191 | 91 | 191 | 43 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12700 | 14100 | 12700 | 26000 | 12700 | 11600 |
| Average | 21000 | 37000 | 21000 | 42300 | 21000 | 22100 |
| Stdev | 33100 | 75000 | 33100 | 68700 | 33100 | 22000 |
| p(t-test) | | 0.017 | | 3.9E-4 | | 0.87 |
| Min | 1.00E-9 | 181 | 1.00E-9 | 1.00E-9 | 1.00E-9 | 401 |
| Max | 485000 | 408000 | 485000 | 406000 | 485000 | 77500 |
| n (Samp) | 756 | 29 | 756 | 37 | 756 | 23 |
| n (Patient) | 297 | 29 | 297 | 37 | 297 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10500 | 22800 | 10500 | 21300 | 10500 | 14400 |
| Average | 20100 | 33700 | 20100 | 37200 | 20100 | 22200 |
| Stdev | 29100 | 52600 | 29100 | 63500 | 29100 | 26000 |
| p(t-test) | | 0.0036 | | 4.8E-4 | | 0.68 |
| Min | 1.00E-9 | 1760 | 1.00E-9 | 114 | 1.00E-9 | 183 |
| Max | 261000 | 408000 | 261000 | 406000 | 261000 | 150000 |
| n (Samp) | 314 | 65 | 314 | 78 | 314 | 36 |
| n (Patient) | 135 | 65 | 135 | 78 | 135 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.58 | 0.67 | 0.62 | 0.64 | 0.65 | 0.55 | 0.52 | 0.59 |
| SE | 0.037 | 0.056 | 0.039 | 0.034 | 0.050 | 0.037 | 0.048 | 0.062 | 0.052 |
| p | 5.8E-4 | 0.17 | 1.1E-5 | 2.9E-4 | 0.0064 | 5.9E-5 | 0.27 | 0.72 | 0.10 |
| nCohort 1 | 360 | 756 | 314 | 360 | 756 | 314 | 360 | 756 | 314 |
| nCohort 2 | 75 | 29 | 65 | 91 | 37 | 78 | 43 | 23 | 36 |
| Cutoff 1 | 8780 | 7950 | 11800 | 9310 | 12100 | 12300 | 7930 | 4760 | 8850 |
| Sens 1 | 71% | 72% | 71% | 70% | 70% | 71% | 72% | 74% | 72% |
| Spec 1 | 45% | 35% | 54% | 48% | 48% | 55% | 44% | 24% | 46% |
| Cutoff 2 | 7700 | 6490 | 9950 | 5620 | 7120 | 6910 | 4520 | 3100 | 7930 |
| Sens 2 | 80% | 83% | 80% | 80% | 81% | 81% | 81% | 83% | 81% |
| Spec 2 | 43% | 31% | 49% | 36% | 33% | 39% | 30% | 16% | 42% |
| Cutoff 3 | 3750 | 1480 | 6390 | 2600 | 2560 | 4310 | 815 | 1270 | 2710 |
| Sens 3 | 91% | 93% | 91% | 90% | 92% | 91% | 91% | 91% | 92% |
| Spec 3 | 26% | 8% | 37% | 19% | 13% | 26% | 9% | 8% | 18% |
| Cutoff 4 | 19300 | 21400 | 20600 | 19300 | 21400 | 20600 | 19300 | 21400 | 20600 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 41% | 45% | 51% | 45% | 54% | 51% | 35% | 43% | 42% |
| Spec 4 | 70% | 70% | 70% | 70% | 70% | 70% | 70% | 70% | 70% |
| Cutoff 5 | 25600 | 28300 | 27800 | 25600 | 28300 | 27800 | 25600 | 28300 | 27800 |
| Sens 5 | 37% | 34% | 38% | 38% | 46% | 44% | 26% | 35% | 25% |
| Spec 5 | 80% | 80% | 80% | 80% | 80% | 80% | 80% | 80% | 80% |
| Cutoff 6 | 40100 | 43000 | 46100 | 40100 | 43000 | 46100 | 40100 | 43000 | 46100 |
| Sens 6 | 21% | 24% | 20% | 18% | 30% | 14% | 16% | 17% | 8% |
| Spec 6 | 90% | 90% | 90% | 90% | 90% | 90% | 90% | 90% | 90% |
| OR Quart 2 | 2.0 | 2.0 | 3.1 | 1.4 | 1.2 | 1.8 | 1.1 | 0.70 | 2.4 |
| p Value | 0.10 | 0.25 | 0.039 | 0.35 | 0.78 | 0.20 | 0.82 | 0.55 | 0.17 |
| 95% CI of | 0.86 | 0.60 | 1.1 | 0.67 | 0.39 | 0.74 | 0.42 | 0.22 | 0.70 |
| OR Quart2 | 4.8 | 6.9 | 8.9 | 3.1 | 3.6 | 4.3 | 3.0 | 2.3 | 8.0 |
| OR Quart 3 | 2.5 | 1.3 | 4.2 | 2.1 | 0.83 | 2.5 | 1.7 | 0.28 | 2.7 |
| p Value | 0.034 | 0.74 | 0.0070 | 0.054 | 0.76 | 0.031 | 0.26 | 0.11 | 0.11 |
| 95% CI of | 1.1 | 0.33 | 1.5 | 0.99 | 0.25 | 1.1 | 0.67 | 0.057 | 0.81 |
| OR Quart3 | 5.7 | 4.8 | 12 | 4.3 | 2.8 | 5.9 | 4.3 | 1.3 | 8.9 |
| OR Quart 4 | 4.0 | 3.1 | 7.4 | 3.6 | 3.4 | 5.3 | 1.7 | 1.3 | 3.6 |
| p Value | 7.4E-4 | 0.053 | 8.8E-5 | 3.8E-4 | 0.011 | 5.0E-5 | 0.26 | 0.62 | 0.031 |
| 95% CI of | 1.8 | 0.99 | 2.7 | 1.8 | 1.3 | 2.4 | 0.67 | 0.47 | 1.1 |
| OR Quart4 | 8.9 | 9.8 | 20 | 7.2 | 8.6 | 12 | 4.3 | 3.5 | 12 |

**Transforming growth factor beta-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.72 | 9.56 | 1.72 | 15.0 | 1.72 | 1.00E-9 |
| Average | 19.8 | 24.9 | 19.8 | 26.8 | 19.8 | 15.1 |
| Stdev | 43.5 | 36.1 | 43.5 | 32.7 | 43.5 | 25.2 |
| p(t-test) | | 0.47 | | 0.30 | | 0.60 |
| Min | 1.00E-9 | 1.00E-9 | 1.00E-9 | 1.00E-9 | 1.00E-9 | 1.00E-9 |
| Max | 364 | 171 | 364 | 130 | 364 | 109 |
| n (Samp) | 122 | 47 | 122 | 51 | 122 | 26 |
| n (Patient) | 99 | 47 | 99 | 51 | 99 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.13 | 1.00E-9 | 4.13 | 18.4 | 4.13 | 1.00E-9 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 21.9 | 18.7 | 21.9 | 23.0 | 21.9 | 9.83 |
| Stdev | 40.1 | 33.5 | 40.1 | 26.4 | 40.1 | 13.6 |
| p(t-test) | | 0.77 | | 0.91 | | 0.28 |
| Min | 1.00E-9 | 1.00E-9 | 1.00E-9 | 1.00E-9 | 1.00E-9 | 1.00E-9 |
| Max | 364 | 120 | 364 | 85.4 | 364 | 36.4 |
| n (Samp) | 262 | 14 | 262 | 19 | 262 | 13 |
| n (Patient) | 160 | 14 | 160 | 19 | 160 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.72 | 15.6 | 1.72 | 17.0 | 1.72 | 1.00E-9 |
| Average | 18.6 | 26.2 | 18.6 | 27.8 | 18.6 | 19.3 |
| Stdev | 43.6 | 36.6 | 43.6 | 33.3 | 43.6 | 30.4 |
| p(t-test) | | 0.30 | | 0.20 | | 0.94 |
| Min | 1.00E-9 | 1.00E-9 | 1.00E-9 | 1.00E-9 | 1.00E-9 | 1.00E-9 |
| Max | 364 | 171 | 364 | 130 | 364 | 109 |
| n (Samp) | 111 | 44 | 111 | 47 | 111 | 23 |
| n (Patient) | 86 | 44 | 86 | 47 | 86 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.45 | 0.61 | 0.62 | 0.54 | 0.63 | 0.48 | 0.40 | 0.50 |
| SE | 0.050 | 0.081 | 0.052 | 0.048 | 0.070 | 0.050 | 0.063 | 0.085 | 0.066 |
| p | 0.080 | 0.51 | 0.030 | 0.013 | 0.54 | 0.0081 | 0.74 | 0.26 | 0.96 |
| nCohort 1 | 122 | 262 | 111 | 122 | 262 | 111 | 122 | 262 | 111 |
| nCohort 2 | 47 | 14 | 44 | 51 | 19 | 47 | 26 | 13 | 23 |
| Cutoff 1 | 0 | 0 | 1.72 | 1.40 | 0 | 3.86 | 0 | 0 | 0 |
| Sens 1 | 100% | 100% | 70% | 71% | 100% | 70% | 100% | 100% | 100% |
| Spec 1 | 0% | 0% | 51% | 49% | 0% | 54% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 4 | 15.0 | 22.9 | 12.5 | 15.0 | 22.9 | 12.5 | 15.0 | 22.9 | 12.5 |
| Sens 4 | 47% | 36% | 52% | 49% | 47% | 60% | 38% | 31% | 39% |
| Spec 4 | 70% | 70% | 70% | 70% | 70% | 70% | 70% | 70% | 70% |
| Cutoff 5 | 34.7 | 36.1 | 26.6 | 34.7 | 36.1 | 26.6 | 34.7 | 36.1 | 26.6 |
| Sens 5 | 26% | 21% | 32% | 29% | 32% | 34% | 15% | 8% | 26% |
| Spec 5 | 80% | 80% | 80% | 80% | 80% | 80% | 80% | 80% | 80% |
| Cutoff 6 | 58.4 | 63.6 | 44.3 | 58.4 | 63.6 | 44.3 | 58.4 | 63.6 | 44.3 |
| Sens 6 | 13% | 7% | 20% | 16% | 11% | 21% | 8% | 0% | 13% |
| Spec 6 | 90% | 90% | 90% | 90% | 90% | 90% | 90% | 90% | 90% |
| OR Quart 2 p Value | 0.15 | 0.49 | 1.3 | 1.2 | 0 | 10.0 | 0.67 | 1.5 | 0.53 |
| 95% CI of OR Quart2 | 0.0061 | 0.41 | 0.62 | 0.79 | na | 0.0040 | 0.53 | 0.65 | 0.35 |
|  | 0.040 | 0.086 | 0.44 | 0.40 | na | 2.1 | 0.19 | 0.25 | 0.14 |
|  | 0.59 | 2.7 | 4.0 | 3.4 | na | 48 | 2.3 | 9.4 | 2.0 |
| OR Quart 3 p Value | 1.1 | 1.5 | 2.5 | 3.1 | 0.60 | 10 | 1.4 | 3.8 | 0 |
| 95% CI of OR Quart3 | 0.82 | 0.51 | 0.090 | 0.022 | 0.39 | 0.0034 | 0.57 | 0.11 | na |
|  | 0.45 | 0.42 | 0.87 | 1.2 | 0.18 | 2.2 | 0.45 | 0.76 | na |
|  | 2.7 | 5.7 | 7.1 | 8.4 | 1.9 | 50 | 4.2 | 19 | na |
| OR Quart 4 p Value | 1.1 | 0.49 | 2.5 | 2.5 | 0.72 | 14 | 0.67 | 0.50 | 2.2 |
| 95% CI of OR Quart4 | 0.88 | 0.41 | 0.090 | 0.068 | 0.56 | 9.8E-4 | 0.53 | 0.58 | 0.16 |
|  | 0.44 | 0.086 | 0.87 | 0.94 | 0.23 | 2.9 | 0.19 | 0.044 | 0.74 |
|  | 2.6 | 2.7 | 7.1 | 6.7 | 2.2 | 65 | 2.3 | 5.6 | 6.6 |

**Transforming growth factor beta-3**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.611 | 1.42 | 0.611 | 1.00 | 0.611 | 0.558 |
| Average | 7.16 | 10.8 | 7.16 | 10.1 | 7.16 | 9.33 |
| Stdev | 23.6 | 35.6 | 23.6 | 29.2 | 23.6 | 23.1 |
| p(t-test) |  | 0.44 |  | 0.48 |  | 0.67 |
| Min | 1.00E-9 | 1.00E-9 | 1.00E-9 | 1.00E-9 | 1.00E-9 | 1.00E-9 |
| Max | 218 | 241 | 218 | 196 | 218 | 102 |
| n (Samp) | 122 | 47 | 122 | 51 | 122 | 26 |
| n (Patient) | 99 | 47 | 99 | 51 | 99 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.611 | 0.850 | 0.611 | 0.603 | 0.611 | 0.0594 |
| Average | 7.40 | 4.79 | 7.40 | 15.8 | 7.40 | 9.81 |
| Stdev | 24.2 | 9.36 | 24.2 | 45.1 | 24.2 | 28.1 |
| p(t-test) | | 0.69 | | 0.17 | | 0.73 |
| Min | 1.00E-9 | 1.00E-9 | 1.00E-9 | 1.00E-9 | 1.00E-9 | 1.00E-9 |
| Max | 241 | 33.3 | 241 | 196 | 241 | 102 |
| n (Samp) | 262 | 14 | 262 | 19 | 262 | 13 |
| n (Patient) | 160 | 14 | 160 | 19 | 160 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.611 | 1.36 | 0.611 | 1.00 | 0.611 | 0.558 |
| Average | 7.51 | 10.3 | 7.51 | 5.59 | 7.51 | 9.23 |
| Stdev | 25.5 | 36.7 | 25.5 | 11.2 | 25.5 | 19.9 |
| p(t-test) | | 0.58 | | 0.62 | | 0.76 |
| Min | 1.00E-9 | 1.00E-9 | 1.00E-9 | 1.00E-9 | 1.00E-9 | 1.00E-9 |
| Max | 218 | 241 | 218 | 59.9 | 218 | 74.6 |
| n (Samp) | 111 | 44 | 111 | 47 | 111 | 23 |
| n (Patient) | 86 | 44 | 86 | 47 | 86 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.53 | 0.55 | 0.52 | 0.49 | 0.54 | 0.44 | 0.42 | 0.49 |
| SE | 0.050 | 0.080 | 0.052 | 0.049 | 0.069 | 0.051 | 0.064 | 0.085 | 0.067 |
| p | 0.46 | 0.73 | 0.38 | 0.63 | 0.90 | 0.44 | 0.36 | 0.35 | 0.91 |
| nCohort 1 | 122 | 262 | 111 | 122 | 262 | 111 | 122 | 262 | 111 |
| nCohort 2 | 47 | 14 | 44 | 51 | 19 | 47 | 26 | 13 | 23 |
| Cutoff 1 | 0 | 0.558 | 1.00E-9 | 0.258 | 0 | 0.553 | 0 | 0 | 1.00E-9 |
| Sens 1 | 100% | 71% | 70% | 71% | 100% | 70% | 100% | 100% | 74% |
| Spec 1 | 0% | 40% | 31% | 30% | 0% | 36% | 0% | 0% | 31% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 2.30 | 2.30 | 2.23 | 2.30 | 2.30 | 2.23 | 2.30 | 2.30 | 2.23 |
| Sens 4 | 38% | 29% | 41% | 33% | 26% | 32% | 27% | 31% | 26% |
| Spec 4 | 75% | 73% | 70% | 75% | 73% | 70% | 75% | 73% | 70% |
| Cutoff 5 | 3.49 | 4.28 | 3.44 | 3.49 | 4.28 | 3.44 | 3.49 | 4.28 | 3.44 |
| Sens 5 | 32% | 21% | 30% | 27% | 26% | 26% | 23% | 15% | 26% |
| Spec 5 | 80% | 81% | 80% | 80% | 81% | 80% | 80% | 81% | 80% |
| Cutoff 6 | 14.9 | 14.9 | 14.1 | 14.9 | 14.9 | 14.1 | 14.9 | 14.9 | 14.1 |
| Sens 6 | 15% | 14% | 16% | 16% | 21% | 13% | 15% | 15% | 17% |
| Spec 6 | 90% | 90% | 91% | 90% | 90% | 91% | 90% | 90% | 91% |
| OR Quart 2 p Value | 0.77 | 6.5 | 0.74 | 1.0 | 0.39 | 1.4 | 0.24 | 0 | 0.47 |
| 95% CI of OR Quart2 | 0.61 | 0.088 | 0.56 | 1.0 | 0.27 | 0.49 | 0.094 | na | 0.31 |
| | 0.28 | 0.76 | 0.26 | 0.39 | 0.073 | 0.52 | 0.047 | na | 0.11 |
| | 2.1 | 55 | 2.0 | 2.6 | 2.1 | 3.9 | 1.3 | na | 2.0 |
| OR Quart 3 p Value | 0.88 | 3.1 | 0.85 | 0.89 | 1.5 | 1.7 | 1.2 | 0.74 | 1.7 |
| 95% CI of OR Quart3 | 0.80 | 0.33 | 0.74 | 0.81 | 0.53 | 0.32 | 0.77 | 0.70 | 0.38 |
| | 0.33 | 0.31 | 0.31 | 0.34 | 0.44 | 0.61 | 0.38 | 0.16 | 0.52 |
| | 2.4 | 30 | 2.3 | 2.3 | 4.9 | 4.5 | 3.7 | 3.4 | 5.4 |
| OR Quart 4 p Value | 1.8 | 4.2 | 1.4 | 1.5 | 1.0 | 1.6 | 1.4 | 1.6 | 0.83 |
| 95% CI of OR Quart4 | 0.19 | 0.21 | 0.52 | 0.40 | 0.98 | 0.35 | 0.57 | 0.50 | 0.78 |
| | 0.73 | 0.46 | 0.53 | 0.60 | 0.28 | 0.59 | 0.45 | 0.42 | 0.23 |
| | 4.6 | 38 | 3.6 | 3.7 | 3.7 | 4.3 | 4.2 | 5.8 | 3.0 |

**Heparan Sulfate**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.77 | 5.16 | 2.77 | 4.40 | nd | nd |
| Average | 4.14 | 5.66 | 4.14 | 6.66 | nd | nd |
| Stdev | 3.75 | 4.88 | 3.75 | 7.67 | nd | nd |
| p(t-test) | | 0.074 | | 0.027 | nd | nd |
| Min | 0.121 | 0.00869 | 0.121 | 0.838 | nd | nd |
| Max | 16.3 | 16.8 | 16.3 | 36.7 | nd | nd |
| n (Samp) | 86 | 32 | 86 | 24 | nd | nd |
| n (Patient) | 69 | 32 | 69 | 24 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.64 | 1.40 | 3.64 | 4.97 | nd | nd |
| Average | 5.00 | 2.37 | 5.00 | 10.0 | nd | nd |
| Stdev | 4.24 | 2.47 | 4.24 | 13.7 | nd | nd |
| p(t-test) | | 0.054 | | 0.014 | nd | nd |
| Min | 0.121 | 0.00869 | 0.121 | 0.838 | nd | nd |
| Max | 18.3 | 6.72 | 18.3 | 36.7 | nd | nd |
| n (Samp) | 152 | 10 | 152 | 6 | nd | nd |
| n (Patient) | 114 | 10 | 114 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.50 | 5.94 | 2.50 | 4.93 | nd | nd |
| Average | 3.75 | 6.33 | 3.75 | 5.82 | nd | nd |
| Stdev | 3.41 | 5.09 | 3.41 | 4.22 | nd | nd |
| p(t-test) | | 0.0042 | | 0.016 | nd | nd |
| Min | 0.121 | 0.187 | 0.121 | 0.970 | nd | nd |
| Max | 14.5 | 16.8 | 14.5 | 18.3 | nd | nd |
| n (Samp) | 77 | 26 | 77 | 24 | nd | nd |
| n (Patient) | 61 | 26 | 61 | 24 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | 0.29 | 0.65 | 0.62 | 0.54 | 0.69 | nd | nd | nd |
| SE | 0.061 | 0.094 | 0.065 | 0.067 | 0.12 | 0.066 | nd | nd | nd |
| p | 0.18 | 0.024 | 0.022 | 0.076 | 0.74 | 0.0045 | nd | nd | nd |
| nCohort 1 | 86 | 152 | 77 | 86 | 152 | 77 | nd | nd | nd |
| nCohort 2 | 32 | 10 | 26 | 24 | 6 | 24 | nd | nd | nd |
| Cutoff 1 | 2.14 | 1.12 | 3.02 | 2.86 | 1.22 | 3.39 | nd | nd | nd |
| Sens 1 | 72% | 70% | 73% | 71% | 83% | 71% | nd | nd | nd |
| Spec 1 | 40% | 14% | 58% | 53% | 16% | 64% | nd | nd | nd |
| Cutoff 2 | 1.12 | 0.288 | 1.07 | 1.46 | 1.22 | 2.09 | nd | nd | nd |
| Sens 2 | 81% | 80% | 81% | 83% | 83% | 83% | nd | nd | nd |
| Spec 2 | 14% | 3% | 16% | 27% | 16% | 40% | nd | nd | nd |
| Cutoff 3 | 0.787 | 0 | 0.555 | 1.22 | 0.796 | 1.46 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | 91% | 100% | 92% | 92% | 100% | 92% | nd | nd | nd |
| Spec 3 | 7% | 0% | 6% | 16% | 8% | 31% | nd | nd | nd |
| Cutoff 4 | 4.45 | 6.28 | 3.93 | 4.45 | 6.28 | 3.93 | nd | nd | nd |
| Sens 4 | 53% | 10% | 62% | 50% | 50% | 58% | nd | nd | nd |
| Spec 4 | 71% | 70% | 70% | 71% | 70% | 70% | nd | nd | nd |
| Cutoff 5 | 7.09 | 8.09 | 5.34 | 7.09 | 8.09 | 5.34 | nd | nd | nd |
| Sens 5 | 25% | 0% | 54% | 33% | 33% | 42% | nd | nd | nd |
| Spec 5 | 80% | 80% | 81% | 80% | 80% | 81% | nd | nd | nd |
| Cutoff 6 | 9.22 | 12.6 | 9.18 | 9.22 | 12.6 | 9.18 | nd | nd | nd |
| Sens 6 | 16% | 0% | 19% | 17% | 17% | 17% | nd | nd | nd |
| Spec 6 | 91% | 90% | 91% | 91% | 90% | 91% | nd | nd | nd |
| OR Quart 2 | 0.40 | >3.3 | 0.13 | 0.96 | 0.47 | 2.2 | nd | nd | nd |
| p Value | 0.18 | <0.31 | 0.066 | 0.96 | 0.55 | 0.39 | nd | nd | nd |
| 95% CI of | 0.11 | >0.33 | 0.014 | 0.21 | 0.041 | 0.36 | nd | nd | nd |
| OR Quart2 | 1.5 | na | 1.1 | 4.3 | 5.5 | 13 | nd | nd | nd |
| OR Quart 3 | 1.0 | >1.0 | 0.95 | 1.6 | 0 | 5.4 | nd | nd | nd |
| p Value | 1.0 | <0.99 | 0.94 | 0.49 | na | 0.048 | nd | nd | nd |
| 95% CI of | 0.32 | >0.062 | 0.26 | 0.41 | na | 1.0 | nd | nd | nd |
| OR Quart3 | 3.2 | na | 3.5 | 6.6 | na | 29 | nd | nd | nd |
| OR Quart 4 | 1.8 | >7.2 | 3.2 | 3.2 | 1.5 | 7.2 | nd | nd | nd |
| p Value | 0.32 | <0.073 | 0.059 | 0.083 | 0.67 | 0.019 | nd | nd | nd |
| 95% CI of | 0.59 | >0.83 | 0.96 | 0.86 | 0.24 | 1.4 | nd | nd | nd |
| OR Quart4 | 5.2 | na | 10 | 12 | 9.5 | 37 | nd | nd | nd |

**Transmembrane glycoprotein NMB**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.72 | 2.51 | 1.72 | 2.34 | 1.72 | 2.19 |
| Average | 1.97 | 2.62 | 1.97 | 2.98 | 1.97 | 2.01 |
| Stdev | 1.28 | 1.31 | 1.28 | 3.50 | 1.28 | 0.999 |
| p(t-test) |  | 0.0032 |  | 0.0050 |  | 0.89 |
| Min | 0.203 | 0.545 | 0.203 | 0.339 | 0.203 | 0.257 |
| Max | 6.99 | 5.60 | 6.99 | 25.3 | 6.99 | 3.91 |
| n (Samp) | 131 | 47 | 131 | 50 | 131 | 26 |
| n (Patient) | 102 | 47 | 102 | 50 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.93 | 2.08 | 1.93 | 2.57 | 1.93 | 2.66 |
| Average | 2.18 | 2.49 | 2.18 | 3.29 | 2.18 | 2.72 |
| Stdev | 1.87 | 1.66 | 1.87 | 2.17 | 1.87 | 1.26 |
| p(t-test) | | 0.54 | | 0.014 | | 0.31 |
| Min | 0.135 | 0.545 | 0.135 | 0.340 | 0.135 | 0.849 |
| Max | 25.3 | 5.60 | 25.3 | 8.02 | 25.3 | 5.18 |
| n (Samp) | 270 | 14 | 270 | 19 | 270 | 13 |
| n (Patient) | 163 | 14 | 163 | 19 | 163 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.61 | 2.56 | 1.61 | 2.48 | 1.61 | 2.21 |
| Average | 1.93 | 2.75 | 1.93 | 3.12 | 1.93 | 2.08 |
| Stdev | 1.25 | 1.24 | 1.25 | 3.60 | 1.25 | 1.06 |
| p(t-test) | | 2.7E-4 | | 0.0021 | | 0.61 |
| Min | 0.203 | 0.744 | 0.203 | 0.339 | 0.203 | 0.257 |
| Max | 6.99 | 5.60 | 6.99 | 25.3 | 6.99 | 3.91 |
| n (Samp) | 118 | 44 | 118 | 46 | 118 | 23 |
| n (Patient) | 87 | 44 | 87 | 46 | 87 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.55 | 0.70 | 0.64 | 0.66 | 0.68 | 0.54 | 0.65 | 0.56 |
| SE | 0.048 | 0.081 | 0.049 | 0.048 | 0.070 | 0.049 | 0.063 | 0.084 | 0.067 |
| p | 0.0012 | 0.51 | 2.9E-5 | 0.0026 | 0.021 | 2.6E-4 | 0.51 | 0.074 | 0.35 |
| nCohort 1 | 131 | 270 | 118 | 131 | 270 | 118 | 131 | 270 | 118 |
| nCohort 2 | 47 | 14 | 44 | 50 | 19 | 46 | 26 | 13 | 23 |
| Cutoff 1 | 1.60 | 1.60 | 1.96 | 1.77 | 1.72 | 1.78 | 1.16 | 1.54 | 1.11 |
| Sens 1 | 70% | 71% | 70% | 70% | 74% | 72% | 73% | 77% | 74% |
| Spec 1 | 47% | 42% | 60% | 50% | 45% | 54% | 31% | 40% | 30% |
| Cutoff 2 | 1.42 | 0.757 | 1.53 | 1.52 | 1.53 | 1.65 | 1.03 | 1.53 | 1.03 |
| Sens 2 | 81% | 86% | 82% | 80% | 84% | 80% | 81% | 85% | 83% |
| Spec 2 | 40% | 11% | 47% | 46% | 40% | 52% | 28% | 40% | 27% |
| Cutoff 3 | 0.880 | 0.547 | 1.33 | 0.955 | 0.955 | 1.10 | 0.777 | 1.29 | 0.777 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 91% | 93% | 91% | 90% | 95% | 91% | 92% | 92% | 91% |
| Spec 3 | 20% | 8% | 36% | 22% | 17% | 29% | 15% | 29% | 14% |
| Cutoff 4 | 2.30 | 2.51 | 2.25 | 2.30 | 2.51 | 2.25 | 2.30 | 2.51 | 2.25 |
| Sens 4 | 57% | 43% | 64% | 54% | 53% | 57% | 35% | 62% | 43% |
| Spec 4 | 70% | 70% | 70% | 70% | 70% | 70% | 70% | 70% | 70% |
| Cutoff 5 | 2.82 | 3.05 | 2.70 | 2.82 | 3.05 | 2.70 | 2.82 | 3.05 | 2.70 |
| Sens 5 | 45% | 36% | 45% | 36% | 37% | 41% | 19% | 31% | 30% |
| Spec 5 | 80% | 80% | 81% | 80% | 80% | 81% | 80% | 80% | 81% |
| Cutoff 6 | 3.87 | 3.91 | 3.74 | 3.87 | 3.91 | 3.74 | 3.87 | 3.91 | 3.74 |
| Sens 6 | 19% | 29% | 20% | 16% | 37% | 24% | 4% | 23% | 9% |
| Spec 6 | 90% | 90% | 91% | 90% | 90% | 91% | 90% | 90% | 91% |
| OR Quart 2 | 2.0 | 0.74 | 2.2 | 2.6 | 2.1 | 1.6 | 1.2 | 3.0 | 1.3 |
| p Value | 0.20 | 0.70 | 0.24 | 0.076 | 0.41 | 0.39 | 0.75 | 0.34 | 0.72 |
| 95% CI of | 0.68 | 0.16 | 0.60 | 0.90 | 0.37 | 0.53 | 0.34 | 0.31 | 0.32 |
| OR Quart2 | 6.1 | 3.4 | 7.9 | 7.7 | 12 | 5.1 | 4.4 | 30 | 5.3 |
| OR Quart 3 | 1.6 | 0.49 | 3.9 | 2.4 | 2.1 | 2.4 | 1.5 | 4.1 | 1.6 |
| p Value | 0.40 | 0.41 | 0.032 | 0.12 | 0.41 | 0.12 | 0.53 | 0.21 | 0.50 |
| 95% CI of | 0.53 | 0.086 | 1.1 | 0.80 | 0.37 | 0.81 | 0.43 | 0.45 | 0.41 |
| OR Quart3 | 5.0 | 2.7 | 13 | 7.0 | 12 | 7.2 | 5.2 | 38 | 6.3 |
| OR Quart 4 | 5.5 | 1.3 | 8.6 | 4.6 | 4.9 | 5.0 | 1.7 | 5.2 | 2.2 |
| p Value | 0.0013 | 0.73 | 4.5E-4 | 0.0042 | 0.047 | 0.0028 | 0.39 | 0.14 | 0.23 |
| 95% CI of | 2.0 | 0.33 | 2.6 | 1.6 | 1.0 | 1.7 | 0.50 | 0.59 | 0.60 |
| OR Quart4 | 16 | 4.9 | 28 | 13 | 24 | 15 | 5.7 | 46 | 8.2 |

**Cadherin-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.8 | 22.6 | 14.8 | 12.0 | 14.8 | 12.6 |
| Average | 27.5 | 37.1 | 27.5 | 28.4 | 27.5 | 20.4 |
| Stdev | 26.6 | 43.7 | 26.6 | 37.1 | 26.6 | 19.7 |
| p(t-test) | | 0.078 | | 0.86 | | 0.20 |
| Min | 0.690 | 1.11 | 0.690 | 0.877 | 0.690 | 1.77 |
| Max | 126 | 212 | 126 | 164 | 126 | 68.4 |
| n (Samp) | 131 | 47 | 131 | 50 | 131 | 26 |
| n (Patient) | 102 | 47 | 102 | 50 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.8 | 8.87 | 14.8 | 9.15 | 14.8 | 9.73 |
| Average | 29.5 | 18.4 | 29.5 | 11.7 | 29.5 | 20.4 |
| Stdev | 31.9 | 20.5 | 31.9 | 10.9 | 31.9 | 32.4 |
| p(t-test) | | 0.20 | | 0.016 | | 0.32 |
| Min | 0.690 | 1.32 | 0.690 | 0.877 | 0.690 | 1.77 |
| Max | 212 | 62.0 | 212 | 40.4 | 212 | 119 |
| n (Samp) | 270 | 14 | 270 | 19 | 270 | 13 |
| n (Patient) | 163 | 14 | 163 | 19 | 163 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.2 | 25.2 | 14.2 | 13.2 | 14.2 | 15.2 |
| Average | 26.9 | 40.1 | 26.9 | 32.4 | 26.9 | 23.4 |
| Stdev | 27.0 | 45.5 | 27.0 | 39.0 | 27.0 | 20.2 |
| p(t-test) | | 0.025 | | 0.30 | | 0.56 |
| Min | 0.690 | 1.11 | 0.690 | 1.21 | 0.690 | 2.34 |
| Max | 126 | 212 | 126 | 164 | 126 | 68.4 |
| n (Samp) | 118 | 44 | 118 | 46 | 118 | 23 |
| n (Patient) | 87 | 44 | 87 | 46 | 87 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.38 | 0.56 | 0.44 | 0.31 | 0.49 | 0.41 | 0.36 | 0.46 |
| SE | 0.050 | 0.082 | 0.052 | 0.049 | 0.069 | 0.050 | 0.063 | 0.085 | 0.067 |
| p | 0.51 | 0.14 | 0.22 | 0.21 | 0.0051 | 0.83 | 0.15 | 0.10 | 0.53 |
| nCohort 1 | 131 | 270 | 118 | 131 | 270 | 118 | 131 | 270 | 118 |
| nCohort 2 | 47 | 14 | 44 | 50 | 19 | 46 | 26 | 13 | 23 |
| Cutoff 1 | 8.99 | 5.78 | 11.5 | 6.10 | 3.24 | 6.69 | 4.44 | 4.44 | 5.29 |
| Sens 1 | 70% | 71% | 70% | 70% | 74% | 72% | 73% | 77% | 74% |
| Spec 1 | 31% | 21% | 45% | 17% | 8% | 18% | 8% | 14% | 13% |
| Cutoff 2 | 6.69 | 4.62 | 5.78 | 5.22 | 1.96 | 5.78 | 4.00 | 2.34 | 4.00 |
| Sens 2 | 81% | 86% | 82% | 80% | 84% | 80% | 81% | 85% | 83% |
| Spec 2 | 18% | 15% | 16% | 12% | 3% | 16% | 7% | 4% | 7% |
| Cutoff 3 | 2.61 | 2.59 | 2.38 | 2.83 | 0.877 | 2.83 | 3.34 | 1.77 | 3.75 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 91% | 93% | 91% | 90% | 95% | 91% | 92% | 92% | 91% |
| Spec 3 | 3% | 5% | 1% | 4% | 0% | 2% | 5% | 3% | 4% |
| Cutoff 4 | 35.4 | 38.0 | 35.1 | 35.4 | 38.0 | 35.1 | 35.4 | 38.0 | 35.1 |
| Sens 4 | 38% | 29% | 39% | 26% | 5% | 30% | 27% | 15% | 35% |
| Spec 4 | 70% | 70% | 70% | 70% | 70% | 70% | 70% | 70% | 70% |
| Cutoff 5 | 48.0 | 48.4 | 48.1 | 48.0 | 48.4 | 48.1 | 48.0 | 48.4 | 48.1 |
| Sens 5 | 28% | 14% | 30% | 18% | 0% | 24% | 15% | 8% | 17% |
| Spec 5 | 80% | 80% | 81% | 80% | 80% | 81% | 80% | 80% | 81% |
| Cutoff 6 | 66.5 | 71.2 | 69.3 | 66.5 | 71.2 | 69.3 | 66.5 | 71.2 | 69.3 |
| Sens 6 | 17% | 0% | 20% | 14% | 0% | 20% | 4% | 8% | 0% |
| Spec 6 | 90% | 90% | 91% | 90% | 90% | 91% | 90% | 90% | 91% |
| OR Quart 2 | 0.67 | 1.0 | 0.45 | 0.81 | >6.6 | 0.61 | 1.0 | 0.49 | 1.2 |
| p Value | 0.42 | 1.0 | 0.16 | 0.67 | <0.083 | 0.32 | 0.96 | 0.57 | 0.72 |
| 95% CI of | 0.25 | 0.14 | 0.15 | 0.31 | >0.78 | 0.23 | 0.30 | 0.044 | 0.37 |
| OR Quart2 | 1.8 | 7.3 | 1.4 | 2.1 | na | 1.6 | 3.5 | 5.6 | 4.2 |
| OR Quart 3 | 1.1 | 2.6 | 1.4 | 0.92 | >5.4 | 0.69 | 0.65 | 2.6 | 0.15 |
| p Value | 0.81 | 0.26 | 0.47 | 0.86 | <0.13 | 0.46 | 0.53 | 0.26 | 0.084 |
| 95% CI of | 0.44 | 0.49 | 0.55 | 0.36 | >0.62 | 0.26 | 0.17 | 0.49 | 0.017 |
| OR Quart3 | 2.8 | 14 | 3.7 | 2.4 | na | 1.8 | 2.5 | 14 | 1.3 |
| OR Quart 4 | 1.1 | 2.6 | 1.2 | 1.7 | >9.1 | 1.1 | 2.0 | 2.7 | 1.7 |
| p Value | 0.87 | 0.26 | 0.68 | 0.23 | <0.040 | 0.81 | 0.24 | 0.25 | 0.35 |
| 95% CI of | 0.43 | 0.49 | 0.47 | 0.70 | >1.1 | 0.44 | 0.63 | 0.50 | 0.54 |
| OR Quart4 | 2.7 | 14 | 3.2 | 4.2 | na | 2.8 | 6.0 | 14 | 5.5 |

**Cathepsin S**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.96 | 3.19 | 1.96 | 2.23 | nd | nd |
| Average | 3.07 | 2.94 | 3.07 | 4.97 | nd | nd |
| Stdev | 4.98 | 2.13 | 4.98 | 8.69 | nd | nd |
| p(t-test) | | 0.91 | | 0.23 | nd | nd |
| Min | 1.00E-9 | 0.200 | 1.00E-9 | 1.00E-9 | nd | nd |
| Max | 37.1 | 7.08 | 37.1 | 39.3 | nd | nd |
| n (Samp) | 62 | 19 | 62 | 19 | nd | nd |
| n (Patient) | 50 | 19 | 50 | 19 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.80 | 1.91 | 1.80 | 2.36 | nd | nd |
| Average | 2.95 | 2.97 | 2.95 | 4.99 | nd | nd |
| Stdev | 5.23 | 2.29 | 5.23 | 8.40 | nd | nd |
| p(t-test) | | 0.99 | | 0.22 | nd | nd |
| Min | 1.00E-9 | 0.200 | 1.00E-9 | 0.370 | nd | nd |
| Max | 37.1 | 7.08 | 37.1 | 39.3 | nd | nd |
| n (Samp) | 52 | 15 | 52 | 20 | nd | nd |
| n (Patient) | 41 | 15 | 41 | 20 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.59 | 0.59 | nd | 0.63 | nd | nd | nd |
| SE | 0.077 | nd | 0.086 | 0.077 | nd | 0.076 | nd | nd | nd |
| p | 0.31 | nd | 0.28 | 0.23 | nd | 0.079 | nd | nd | nd |
| nCohort 1 | 62 | nd | 52 | 62 | nd | 52 | nd | nd | nd |
| nCohort 2 | 19 | nd | 15 | 19 | nd | 20 | nd | nd | nd |
| Cutoff 1 | 1.23 | nd | 1.23 | 1.10 | nd | 1.63 | nd | nd | nd |
| Sens 1 | 74% | nd | 73% | 74% | nd | 70% | nd | nd | nd |
| Spec 1 | 42% | nd | 44% | 40% | nd | 48% | nd | nd | nd |
| Cutoff 2 | 0.885 | nd | 1.03 | 0.827 | nd | 1.08 | nd | nd | nd |
| Sens 2 | 84% | nd | 80% | 84% | nd | 80% | nd | nd | nd |
| Spec 2 | 35% | nd | 38% | 34% | nd | 42% | nd | nd | nd |
| Cutoff 3 | 0.324 | nd | 0.478 | 0.324 | nd | 0.827 | nd | nd | nd |
| Sens 3 | 95% | nd | 93% | 95% | nd | 90% | nd | nd | nd |
| Spec 3 | 13% | nd | 21% | 13% | nd | 33% | nd | nd | nd |
| Cutoff 4 | 3.43 | nd | 2.93 | 3.43 | nd | 2.93 | nd | nd | nd |
| Sens 4 | 47% | nd | 47% | 37% | nd | 45% | nd | nd | nd |
| Spec 4 | 71% | nd | 71% | 71% | nd | 71% | nd | nd | nd |
| Cutoff 5 | 4.16 | nd | 4.00 | 4.16 | nd | 4.00 | nd | nd | nd |
| Sens 5 | 21% | nd | 33% | 32% | nd | 35% | nd | nd | nd |
| Spec 5 | 81% | nd | 81% | 81% | nd | 81% | nd | nd | nd |
| Cutoff 6 | 6.61 | nd | 6.42 | 6.61 | nd | 6.42 | nd | nd | nd |
| Sens 6 | 11% | nd | 13% | 26% | nd | 15% | nd | nd | nd |
| Spec 6 | 90% | nd | 90% | 90% | nd | 90% | nd | nd | nd |
| OR Quart 2 | 2.4 | nd | 2.9 | 3.9 | nd | 4.0 | nd | nd | nd |
| p Value | 0.26 | nd | 0.25 | 0.13 | nd | 0.12 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.51 | nd | 0.48 | 0.67 | nd | 0.68 | nd | nd | nd |
| OR Quart2 | 12 | nd | 18 | 22 | nd | 23 | nd | nd | nd |
| OR Quart 3 | 1.4 | nd | 1.5 | 3.0 | nd | 3.1 | nd | nd | nd |
| p Value | 0.68 | nd | 0.68 | 0.23 | nd | 0.22 | nd | nd | nd |
| 95% CI of | 0.27 | nd | 0.22 | 0.51 | nd | 0.51 | nd | nd | nd |
| OR Quart3 | 7.3 | nd | 10 | 18 | nd | 19 | nd | nd | nd |
| OR Quart 4 | 2.3 | nd | 2.9 | 3.6 | nd | 5.1 | nd | nd | nd |
| p Value | 0.30 | nd | 0.25 | 0.15 | nd | 0.068 | nd | nd | nd |
| 95% CI of | 0.48 | nd | 0.48 | 0.63 | nd | 0.89 | nd | nd | nd |
| OR Quart4 | 11 | nd | 18 | 21 | nd | 29 | nd | nd | nd |

**Glectin-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.06 | 0.899 | 1.06 | 1.06 | nd | nd |
| Average | 1.05 | 1.00 | 1.05 | 1.24 | nd | nd |
| Stdev | 0.435 | 0.476 | 0.435 | 1.32 | nd | nd |
| p(t-test) | | 0.66 | | 0.33 | nd | nd |
| Min | 0.253 | 0.278 | 0.253 | 0.0847 | nd | nd |
| Max | 2.52 | 1.90 | 2.52 | 6.42 | nd | nd |
| n (Samp) | 62 | 19 | 62 | 19 | nd | nd |
| n (Patient) | 50 | 19 | 50 | 19 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.03 | 0.955 | 1.03 | 1.08 | nd | nd |
| Average | 1.03 | 1.01 | 1.03 | 1.26 | nd | nd |
| Stdev | 0.427 | 0.441 | 0.427 | 1.28 | nd | nd |
| p(t-test) | | 0.85 | | 0.25 | nd | nd |
| Min | 0.205 | 0.417 | 0.205 | 0.262 | nd | nd |
| Max | 2.52 | 1.76 | 2.52 | 6.42 | nd | nd |
| n (Samp) | 52 | 15 | 52 | 20 | nd | nd |
| n (Patient) | 41 | 15 | 41 | 20 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | nd | 0.47 | 0.47 | nd | 0.50 | nd | nd | nd |
| SE | 0.077 | nd | 0.086 | 0.077 | nd | 0.077 | nd | nd | nd |
| p | 0.49 | nd | 0.69 | 0.72 | nd | 0.98 | nd | nd | nd |
| nCohort 1 | 62 | nd | 52 | 62 | nd | 52 | nd | nd | nd |
| nCohort 2 | 19 | nd | 15 | 19 | nd | 20 | nd | nd | nd |
| Cutoff 1 | 0.629 | nd | 0.629 | 0.743 | nd | 0.835 | nd | nd | nd |
| Sens 1 | 74% | nd | 73% | 74% | nd | 70% | nd | nd | nd |
| Spec 1 | 21% | nd | 19% | 24% | nd | 27% | nd | nd | nd |
| Cutoff 2 | 0.547 | nd | 0.600 | 0.629 | nd | 0.639 | nd | nd | nd |
| Sens 2 | 84% | nd | 80% | 84% | nd | 80% | nd | nd | nd |
| Spec 2 | 16% | nd | 17% | 21% | nd | 19% | nd | nd | nd |
| Cutoff 3 | 0.279 | nd | 0.480 | 0.279 | nd | 0.480 | nd | nd | nd |
| Sens 3 | 95% | nd | 93% | 95% | nd | 90% | nd | nd | nd |
| Spec 3 | 5% | nd | 13% | 5% | nd | 13% | nd | nd | nd |
| Cutoff 4 | 1.23 | nd | 1.22 | 1.23 | nd | 1.22 | nd | nd | nd |
| Sens 4 | 26% | nd | 27% | 26% | nd | 30% | nd | nd | nd |
| Spec 4 | 71% | nd | 71% | 71% | nd | 71% | nd | nd | nd |
| Cutoff 5 | 1.42 | nd | 1.34 | 1.42 | nd | 1.34 | nd | nd | nd |
| Sens 5 | 21% | nd | 27% | 11% | nd | 10% | nd | nd | nd |
| Spec 5 | 81% | nd | 81% | 81% | nd | 81% | nd | nd | nd |
| Cutoff 6 | 1.54 | nd | 1.50 | 1.54 | nd | 1.50 | nd | nd | nd |
| Sens 6 | 21% | nd | 20% | 11% | nd | 10% | nd | nd | nd |
| Spec 6 | 90% | nd | 90% | 90% | nd | 90% | nd | nd | nd |
| OR Quart 2 | 0.36 | nd | 0.70 | 1.1 | nd | 0.77 | nd | nd | nd |
| p Value | 0.25 | nd | 0.67 | 0.93 | nd | 0.72 | nd | nd | nd |
| 95% CI of | 0.060 | nd | 0.13 | 0.26 | nd | 0.19 | nd | nd | nd |
| OR Quart2 | 2.1 | nd | 3.7 | 4.4 | nd | 3.2 | nd | nd | nd |
| OR Quart 3 | 1.4 | nd | 0.70 | 0.80 | nd | 0.40 | nd | nd | nd |
| p Value | 0.66 | nd | 0.67 | 0.77 | nd | 0.26 | nd | nd | nd |
| 95% CI of | 0.34 | nd | 0.13 | 0.18 | nd | 0.082 | nd | nd | nd |
| OR Quart3 | 5.5 | nd | 3.7 | 3.5 | nd | 1.9 | nd | nd | nd |
| OR Quart 4 | 1.4 | nd | 1.5 | 1.1 | nd | 1.0 | nd | nd | nd |
| p Value | 0.66 | nd | 0.62 | 0.93 | nd | 1.0 | nd | nd | nd |
| 95% CI of | 0.34 | nd | 0.32 | 0.26 | nd | 0.25 | nd | nd | nd |
| OR Quart4 | 5.5 | nd | 6.9 | 4.4 | nd | 4.0 | nd | nd | nd |

**C-C motif chemokine 23**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00253 | 0.00573 | 0.00253 | 0.00530 | nd | nd |
| Average | 0.226 | 0.0131 | 0.226 | 0.106 | nd | nd |
| Stdev | 1.73 | 0.0203 | 1.73 | 0.416 | nd | nd |
| p(t-test) | | 0.60 | | 0.77 | nd | nd |
| Min | 1.00E-9 | 8.99E-5 | 1.00E-9 | 0.000283 | nd | nd |
| Max | 13.7 | 0.0748 | 13.7 | 1.82 | nd | nd |
| n (Samp) | 62 | 19 | 62 | 19 | nd | nd |
| n (Patient) | 50 | 19 | 50 | 19 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00271 | 0.00389 | 0.00271 | 0.00633 | nd | nd |
| Average | 0.268 | 0.0141 | 0.268 | 0.107 | nd | nd |
| Stdev | 1.89 | 0.0227 | 1.89 | 0.405 | nd | nd |
| p(t-test) | | 0.61 | | 0.71 | nd | nd |
| Min | 1.00E-9 | 8.99E-5 | 1.00E-9 | 0.000283 | nd | nd |
| Max | 13.7 | 0.0748 | 13.7 | 1.82 | nd | nd |
| n (Samp) | 52 | 15 | 52 | 20 | nd | nd |
| n (Patient) | 41 | 15 | 41 | 20 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.58 | 0.63 | nd | 0.66 | nd | nd | nd |
| SE | 0.077 | nd | 0.086 | 0.076 | nd | 0.075 | nd | nd | nd |
| p | 0.18 | nd | 0.37 | 0.089 | nd | 0.028 | nd | nd | nd |
| nCohort 1 | 62 | nd | 52 | 62 | nd | 52 | nd | nd | nd |
| nCohort 2 | 19 | nd | 15 | 19 | nd | 20 | nd | nd | nd |
| Cutoff 1 | 0.00146 | nd | 0.00159 | 0.00146 | nd | 0.00298 | nd | nd | nd |
| Sens 1 | 74% | nd | 73% | 74% | nd | 70% | nd | nd | nd |
| Spec 1 | 42% | nd | 40% | 42% | nd | 52% | nd | nd | nd |
| Cutoff 2 | 0.000468 | nd | 0.000524 | 0.000615 | nd | 0.00147 | nd | nd | nd |
| Sens 2 | 84% | nd | 80% | 84% | nd | 80% | nd | nd | nd |
| Spec 2 | 23% | nd | 25% | 29% | nd | 38% | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 0.000158 | nd | 0.000158 | 0.000305 | nd | 0.000615 | nd | nd | nd |
| Sens 3 | 95% | nd | 93% | 95% | nd | 90% | nd | nd | nd |
| Spec 3 | 19% | nd | 17% | 21% | nd | 27% | nd | nd | nd |
| Cutoff 4 | 0.00623 | nd | 0.00623 | 0.00623 | nd | 0.00623 | nd | nd | nd |
| Sens 4 | 47% | nd | 40% | 42% | nd | 50% | nd | nd | nd |
| Spec 4 | 71% | nd | 71% | 71% | nd | 71% | nd | nd | nd |
| Cutoff 5 | 0.0102 | nd | 0.00974 | 0.0102 | nd | 0.00974 | nd | nd | nd |
| Sens 5 | 37% | nd | 33% | 37% | nd | 40% | nd | nd | nd |
| Spec 5 | 81% | nd | 81% | 81% | nd | 81% | nd | nd | nd |
| Cutoff 6 | 0.0166 | nd | 0.0166 | 0.0166 | nd | 0.0166 | nd | nd | nd |
| Sens 6 | 16% | nd | 20% | 26% | nd | 25% | nd | nd | nd |
| Spec 6 | 90% | nd | 90% | 90% | nd | 90% | nd | nd | nd |
| OR Quart 2 | 1.0 | nd | 1.3 | 1.4 | nd | 1.4 | nd | nd | nd |
| p Value | 1.0 | nd | 0.74 | 0.68 | nd | 0.67 | nd | nd | nd |
| 95% CI of | 0.21 | nd | 0.25 | 0.27 | nd | 0.27 | nd | nd | nd |
| OR Quart2 | 4.7 | nd | 7.2 | 7.3 | nd | 7.5 | nd | nd | nd |
| OR Quart 3 | 1.0 | nd | 0.58 | 1.9 | nd | 1.9 | nd | nd | nd |
| p Value | 1.0 | nd | 0.58 | 0.43 | nd | 0.43 | nd | nd | nd |
| 95% CI of | 0.21 | nd | 0.083 | 0.38 | nd | 0.38 | nd | nd | nd |
| OR Quart3 | 4.7 | nd | 4.0 | 9.3 | nd | 9.6 | nd | nd | nd |
| OR Quart 4 p Value 95% CI of OR Quart4 | 2.0 | nd | 2.4 | 2.8 | nd | 4.0 | nd | nd | nd |
| | 0.34 | nd | 0.29 | 0.18 | nd | 0.080 | nd | nd | nd |
| | 0.48 | nd | 0.48 | 0.62 | nd | 0.85 | nd | nd | nd |
| | 8.3 | nd | 12 | 13 | nd | 19 | nd | nd | nd |

**Carcinoembryonic antigen-related cell adhesion molecule 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.09 | 7.59 | 6.09 | 6.66 | 6.09 | 6.45 |
| Average | 8.23 | 10.8 | 8.23 | 12.9 | 8.23 | 7.87 |
| Stdev | 6.90 | 12.1 | 6.90 | 23.6 | 6.90 | 6.61 |
| p(t-test) | | 0.075 | | 0.043 | | 0.81 |
| Min | 0.605 | 0.879 | 0.605 | 0.426 | 0.605 | 0.704 |
| Max | 32.3 | 65.1 | 32.3 | 155 | 32.3 | 29.9 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 131 | 47 | 131 | 50 | 131 | 26 |
| n (Patient) | 102 | 47 | 102 | 50 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.78 | 3.89 | 6.78 | 6.08 | 6.78 | 7.27 |
| Average | 9.95 | 6.47 | 9.95 | 12.8 | 9.95 | 10.9 |
| Stdev | 12.9 | 6.02 | 12.9 | 17.6 | 12.9 | 10.6 |
| p(t-test) | | 0.32 | | 0.36 | | 0.79 |
| Min | 0.426 | 0.879 | 0.426 | 0.565 | 0.426 | 1.70 |
| Max | 155 | 18.8 | 155 | 76.0 | 155 | 41.9 |
| n (Samp) | 270 | 14 | 270 | 19 | 270 | 13 |
| n (Patient) | 163 | 14 | 163 | 19 | 163 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.08 | 7.90 | 6.08 | 8.03 | 6.08 | 6.17 |
| Average | 7.47 | 11.3 | 7.47 | 12.7 | 7.47 | 8.02 |
| Stdev | 6.04 | 12.3 | 6.04 | 22.9 | 6.04 | 7.42 |
| p(t-test) | | 0.0081 | | 0.022 | | 0.70 |
| Min | 0.605 | 1.09 | 0.605 | 0.426 | 0.605 | 0.704 |
| Max | 31.5 | 65.1 | 31.5 | 155 | 31.5 | 29.9 |
| n (Samp) | 118 | 44 | 118 | 46 | 118 | 23 |
| n (Patient) | 87 | 44 | 87 | 46 | 87 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.37 | 0.61 | 0.55 | 0.51 | 0.59 | 0.49 | 0.54 | 0.49 |
| SE | 0.050 | 0.082 | 0.051 | 0.049 | 0.069 | 0.051 | 0.062 | 0.084 | 0.066 |
| p | 0.23 | 0.10 | 0.038 | 0.29 | 0.85 | 0.066 | 0.91 | 0.59 | 0.84 |
| nCohort 1 | 131 | 270 | 118 | 131 | 270 | 118 | 131 | 270 | 118 |
| nCohort 2 | 47 | 14 | 44 | 50 | 19 | 46 | 26 | 13 | 23 |
| Cutoff 1 | 5.13 | 1.67 | 5.16 | 4.77 | 4.92 | 4.52 | 4.30 | 5.22 | 4.04 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 70% | 71% | 70% | 70% | 74% | 72% | 73% | 77% | 74% |
| Spec 1 | 41% | 7% | 42% | 38% | 35% | 36% | 31% | 37% | 30% |
| Cutoff 2 | 4.04 | 1.25 | 4.35 | 4.04 | 3.76 | 4.04 | 4.04 | 4.14 | 1.43 |
| Sens 2 | 81% | 86% | 82% | 80% | 84% | 80% | 81% | 85% | 83% |
| Spec 2 | 27% | 3% | 33% | 27% | 21% | 30% | 27% | 26% | 5% |
| Cutoff 3 | 1.53 | 1.15 | 2.24 | 3.23 | 1.87 | 2.83 | 1.43 | 2.90 | 1.15 |
| Sens 3 | 91% | 93% | 91% | 90% | 95% | 91% | 92% | 92% | 91% |
| Spec 3 | 6% | 3% | 15% | 21% | 9% | 23% | 5% | 18% | 3% |
| Cutoff 4 | 9.41 | 10.4 | 8.28 | 9.41 | 10.4 | 8.28 | 9.41 | 10.4 | 8.28 |
| Sens 4 | 43% | 29% | 48% | 38% | 37% | 48% | 19% | 31% | 30% |
| Spec 4 | 70% | 70% | 70% | 70% | 70% | 70% | 70% | 70% | 70% |
| Cutoff 5 | 12.0 | 13.3 | 11.5 | 12.0 | 13.3 | 11.5 | 12.0 | 13.3 | 11.5 |
| Sens 5 | 26% | 14% | 27% | 24% | 32% | 30% | 15% | 23% | 22% |
| Spec 5 | 80% | 80% | 81% | 80% | 80% | 81% | 80% | 80% | 81% |
| Cutoff 6 | 17.5 | 19.4 | 13.7 | 17.5 | 19.4 | 13.7 | 17.5 | 19.4 | 13.7 |
| Sens 6 | 15% | 0% | 20% | 10% | 11% | 20% | 12% | 15% | 17% |
| Spec 6 | 90% | 90% | 91% | 90% | 90% | 91% | 90% | 90% | 91% |
| OR Quart 2 | 1.1 | 0.66 | 1.3 | 1.4 | 2.1 | 1.7 | 2.1 | 1.5 | 1.6 |
| p Value | 0.84 | 0.65 | 0.62 | 0.47 | 0.24 | 0.31 | 0.22 | 0.66 | 0.49 |
| 95% CI of | 0.40 | 0.11 | 0.44 | 0.55 | 0.61 | 0.61 | 0.63 | 0.24 | 0.44 |
| OR Quart2 | 3.1 | 4.1 | 4.0 | 3.7 | 7.4 | 4.8 | 7.0 | 9.3 | 5.4 |
| OR Quart 3 | 2.0 | 0.66 | 2.3 | 1.4 | 0.24 | 1.5 | 1.5 | 2.6 | 1.3 |
| p Value | 0.15 | 0.65 | 0.13 | 0.47 | 0.21 | 0.43 | 0.50 | 0.27 | 0.71 |
| 95% CI of | 0.77 | 0.11 | 0.79 | 0.55 | 0.026 | 0.54 | 0.44 | 0.48 | 0.35 |
| OR Quart3 | 5.3 | 4.1 | 6.5 | 3.7 | 2.2 | 4.3 | 5.3 | 14 | 4.7 |
| OR Quart 4 | 1.6 | 2.5 | 2.7 | 1.5 | 1.5 | 2.4 | 1.0 | 1.5 | 1.0 |
| p Value | 0.36 | 0.20 | 0.058 | 0.38 | 0.53 | 0.089 | 0.97 | 0.66 | 0.96 |
| 95% CI of | 0.60 | 0.61 | 0.97 | 0.60 | 0.41 | 0.88 | 0.27 | 0.24 | 0.27 |
| OR Quart4 | 4.2 | 10 | 7.6 | 3.9 | 5.6 | 6.5 | 3.9 | 9.3 | 3.9 |

**Platelet factor 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.18 | 4.09 | 2.18 | 4.71 | 2.18 | 3.45 |
| Average | 5.72 | 6.12 | 5.72 | 7.14 | 5.72 | 4.04 |
| Stdev | 10.2 | 6.91 | 10.2 | 8.22 | 10.2 | 3.90 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.80 | | 0.39 | | 0.41 |
| Min | 1.00E-9 | 1.00E-9 | 1.00E-9 | 1.00E-9 | 1.00E-9 | 1.00E-9 |
| Max | 81.1 | 28.4 | 81.1 | 34.9 | 81.1 | 14.1 |
| n (Samp) | 130 | 47 | 130 | 49 | 130 | 26 |
| n (Patient) | 102 | 47 | 102 | 49 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.94 | 3.97 | 2.94 | 3.04 | 2.94 | 3.32 |
| Average | 5.83 | 5.18 | 5.83 | 7.40 | 5.83 | 3.96 |
| Stdev | 9.66 | 6.20 | 9.66 | 9.39 | 9.66 | 4.42 |
| p(t-test) | | 0.81 | | 0.49 | | 0.49 |
| Min | 1.00E-9 | 1.00E-9 | 1.00E-9 | 1.00E-9 | 1.00E-9 | 1.00E-9 |
| Max | 84.2 | 21.4 | 84.2 | 34.9 | 84.2 | 15.5 |
| n (Samp) | 268 | 14 | 268 | 19 | 268 | 13 |
| n (Patient) | 162 | 14 | 162 | 19 | 162 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.80 | 4.51 | 2.80 | 4.62 | 2.80 | 4.06 |
| Average | 6.01 | 8.13 | 6.01 | 7.19 | 6.01 | 5.39 |
| Stdev | 10.5 | 13.1 | 10.5 | 7.87 | 10.5 | 6.05 |
| p(t-test) | | 0.29 | | 0.50 | | 0.79 |
| Min | 1.00E-9 | 1.00E-9 | 1.00E-9 | 1.00E-9 | 1.00E-9 | 1.00E-9 |
| Max | 81.1 | 79.7 | 81.1 | 32.1 | 81.1 | 25.8 |
| n (Samp) | 117 | 44 | 117 | 45 | 117 | 23 |
| n (Patient) | 87 | 44 | 87 | 45 | 87 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.49 | 0.59 | 0.60 | 0.55 | 0.59 | 0.51 | 0.46 | 0.54 |
| SE | 0.050 | 0.080 | 0.052 | 0.049 | 0.070 | 0.051 | 0.062 | 0.084 | 0.067 |
| p | 0.15 | 0.91 | 0.079 | 0.045 | 0.45 | 0.065 | 0.92 | 0.65 | 0.56 |
| nCohort 1 | 130 | 268 | 117 | 130 | 268 | 117 | 130 | 268 | 117 |
| nCohort 2 | 47 | 14 | 44 | 49 | 19 | 45 | 26 | 13 | 23 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 1 | 1.39 | 0.595 | 1.61 | 2.48 | 1.39 | 2.47 | 0.422 | 0.297 | 0.990 |
| Sens 1 | 70% | 71% | 70% | 71% | 74% | 71% | 73% | 77% | 74% |
| Spec 1 | 45% | 26% | 43% | 52% | 40% | 48% | 26% | 18% | 36% |
| Cutoff 2 | 0.509 | 0 | 1.10 | 0.464 | 0.0365 | 0.692 | 0.0355 | 0 | 0.422 |
| Sens 2 | 81% | 100% | 82% | 82% | 84% | 80% | 81% | 100% | 83% |
| Spec 2 | 27% | 0% | 38% | 27% | 13% | 32% | 14% | 0% | 24% |
| Cutoff 3 | 0 | 0 | 0.225 | 0 | 0 | 0.286 | 0 | 0 | 0.0355 |
| Sens 3 | 100% | 100% | 91% | 100% | 100% | 91% | 100% | 100% | 91% |
| Spec 3 | 0% | 0% | 16% | 0% | 0% | 16% | 0% | 0% | 11% |
| Cutoff 4 | 5.33 | 5.92 | 5.56 | 5.33 | 5.92 | 5.56 | 5.33 | 5.92 | 5.56 |
| Sens 4 | 40% | 29% | 45% | 41% | 42% | 40% | 35% | 23% | 30% |
| Spec 4 | 70% | 70% | 70% | 70% | 70% | 70% | 70% | 70% | 70% |
| Cutoff 5 | 8.33 | 8.93 | 8.20 | 8.33 | 8.93 | 8.20 | 8.33 | 8.93 | 8.20 |
| Sens 5 | 26% | 21% | 27% | 27% | 26% | 31% | 15% | 8% | 22% |
| Spec 5 | 80% | 80% | 80% | 80% | 80% | 80% | 80% | 80% | 80% |
| Cutoff 6 | 14.9 | 14.2 | 15.5 | 14.9 | 14.2 | 15.5 | 14.9 | 14.2 | 15.5 |
| Sens 6 | 11% | 7% | 14% | 10% | 16% | 9% | 0% | 8% | 4% |
| Spec 6 | 90% | 90% | 91% | 90% | 90% | 91% | 90% | 90% | 91% |
| OR Quart 2 | 1.1 | 1.0 | 1.8 | 0.84 | 0.99 | 0.71 | 0.38 | 2.7 | 0.29 |
| p Value | 0.80 | 0.98 | 0.29 | 0.75 | 0.98 | 0.54 | 0.19 | 0.25 | 0.15 |
| 95% CI of | 0.41 | 0.24 | 0.61 | 0.29 | 0.24 | 0.24 | 0.091 | 0.50 | 0.055 |
| OR Quart2 | 3.2 | 4.2 | 5.2 | 2.4 | 4.1 | 2.1 | 1.6 | 14 | 1.6 |
| OR Quart 3 | 2.0 | 0.49 | 2.5 | 2.1 | 0.99 | 1.9 | 1.6 | 1.0 | 1.7 |
| p Value | 0.15 | 0.41 | 0.080 | 0.12 | 0.98 | 0.22 | 0.41 | 0.99 | 0.38 |
| 95% CI of | 0.77 | 0.086 | 0.89 | 0.83 | 0.24 | 0.69 | 0.53 | 0.14 | 0.52 |
| OR Quart3 | 5.3 | 2.7 | 7.2 | 5.6 | 4.1 | 5.0 | 4.7 | 7.4 | 5.3 |
| OR Quart 4 | 1.6 | 1.0 | 2.0 | 2.1 | 1.8 | 2.0 | 0.83 | 2.1 | 1.0 |
| p Value | 0.36 | 0.98 | 0.22 | 0.12 | 0.36 | 0.17 | 0.76 | 0.40 | 1.0 |
| 95% CI of | 0.60 | 0.24 | 0.68 | 0.83 | 0.50 | 0.75 | 0.25 | 0.37 | 0.29 |
| OR Quart4 | 4.2 | 4.2 | 5.6 | 5.6 | 6.5 | 5.3 | 2.7 | 12 | 3.5 |

**Inulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.898 | 1.94 | 0.898 | 1.42 | 0.898 | 1.50 |
| Average | 6.54 | 19.4 | 6.54 | 32.9 | 6.54 | 23.1 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 20.9 | 42.0 | 20.9 | 101 | 20.9 | 56.9 |
| p(t-test) | | 0.0014 | | 1.6E-4 | | 0.0021 |
| Min | 0.00344 | 0.00344 | 0.00344 | 0.0393 | 0.00344 | 0.00344 |
| Max | 228 | 192 | 228 | 533 | 228 | 214 |
| n (Samp) | 255 | 48 | 255 | 57 | 255 | 27 |
| n (Patient) | 103 | 48 | 103 | 57 | 103 | 27 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.973 | 1.27 | 0.973 | 1.62 | 0.973 | 1.16 |
| Average | 16.8 | 17.3 | 16.8 | 14.5 | 16.8 | 21.9 |
| Stdev | 102 | 33.0 | 102 | 29.1 | 102 | 59.0 |
| p(t-test) | | 0.99 | | 0.92 | | 0.86 |
| Min | 0.00344 | 0.00344 | 0.00344 | 0.151 | 0.00344 | 0.116 |
| Max | 1750 | 98.1 | 1750 | 121 | 1750 | 214 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.06 | 2.34 | 1.06 | 1.37 | 1.06 | 1.47 |
| Average | 7.65 | 19.6 | 7.65 | 56.9 | 7.65 | 19.3 |
| Stdev | 23.0 | 42.2 | 23.0 | 193 | 23.0 | 45.6 |
| p(t-test) | | 0.0073 | | 2.9E-4 | | 0.036 |
| Min | 0.00344 | 0.101 | 0.00344 | 0.0393 | 0.00344 | 0.00344 |
| Max | 214 | 192 | 214 | 1190 | 214 | 209 |
| n (Samp) | 218 | 46 | 218 | 51 | 218 | 25 |
| n (Patient) | 87 | 46 | 87 | 51 | 87 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.50 | 0.62 | 0.61 | 0.59 | 0.60 | 0.57 | 0.57 | 0.55 |
| SE | 0.046 | 0.074 | 0.048 | 0.043 | 0.067 | 0.046 | 0.060 | 0.084 | 0.062 |
| p | 0.0078 | 0.98 | 0.011 | 0.011 | 0.16 | 0.031 | 0.25 | 0.42 | 0.42 |
| nCohort 1 | 255 | 447 | 218 | 255 | 447 | 218 | 255 | 447 | 218 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 48 | 16 | 46 | 57 | 21 | 51 | 27 | 13 | 25 |
| Cutoff 1 | 0.898 | 0.330 | 0.897 | 0.917 | 0.908 | 0.950 | 0.655 | 0.733 | 0.648 |
| Sens 1 | 71% | 75% | 72% | 70% | 71% | 71% | 70% | 77% | 72% |
| Spec 1 | 50% | 17% | 45% | 51% | 47% | 46% | 35% | 37% | 30% |
| Cutoff 2 | 0.604 | 0.128 | 0.751 | 0.544 | 0.507 | 0.762 | 0.558 | 0.558 | 0.604 |
| Sens 2 | 81% | 81% | 80% | 81% | 81% | 80% | 81% | 85% | 80% |
| Spec 2 | 32% | 8% | 34% | 29% | 24% | 35% | 30% | 27% | 27% |
| Cutoff 3 | 0.128 | 0.0381 | 0.334 | 0.276 | 0.329 | 0.336 | 0.101 | 0.530 | 0.228 |
| Sens 3 | 92% | 94% | 91% | 91% | 90% | 90% | 93% | 92% | 92% |
| Spec 3 | 9% | 5% | 14% | 15% | 16% | 15% | 9% | 25% | 10% |
| Cutoff 4 | 2.02 | 2.63 | 2.59 | 2.02 | 2.63 | 2.59 | 2.02 | 2.63 | 2.59 |
| Sens 4 | 50% | 31% | 48% | 39% | 38% | 35% | 37% | 38% | 32% |
| Spec 4 | 70% | 70% | 70% | 70% | 70% | 70% | 70% | 70% | 70% |
| Cutoff 5 | 3.90 | 6.93 | 5.69 | 3.90 | 6.93 | 5.69 | 3.90 | 6.93 | 5.69 |
| Sens 5 | 42% | 31% | 39% | 33% | 29% | 33% | 33% | 23% | 28% |
| Spec 5 | 80% | 80% | 80% | 80% | 80% | 80% | 80% | 80% | 80% |
| Cutoff 6 | 17.8 | 22.7 | 19.3 | 17.8 | 22.7 | 19.3 | 17.8 | 22.7 | 19.3 |
| Sens 6 | 17% | 19% | 17% | 21% | 24% | 20% | 19% | 15% | 20% |
| Spec 6 | 90% | 90% | 90% | 90% | 90% | 90% | 90% | 90% | 90% |
| OR Quart 2 | 1.3 | 0.16 | 1.7 | 0.90 | 0.39 | 1.4 | 1.4 | 4.1 | 1.5 |
| p Value | 0.64 | 0.090 | 0.31 | 0.81 | 0.27 | 0.46 | 0.56 | 0.21 | 0.53 |
| 95% CI of | 0.47 | 0.019 | 0.61 | 0.36 | 0.074 | 0.54 | 0.43 | 0.45 | 0.41 |
| OR Quart2 | 3.4 | 1.3 | 4.7 | 2.2 | 2.0 | 3.9 | 4.7 | 37 | 5.7 |
| OR Quart 3 | 1.3 | 0.65 | 1.3 | 1.7 | 1.4 | 1.9 | 1.2 | 3.1 | 1.8 |
| p Value | 0.64 | 0.51 | 0.59 | 0.21 | 0.56 | 0.17 | 0.75 | 0.34 | 0.36 |
| 95% CI of | 0.47 | 0.18 | 0.46 | 0.74 | 0.44 | 0.76 | 0.35 | 0.31 | 0.50 |
| OR Quart3 | 3.4 | 2.4 | 3.8 | 3.9 | 4.6 | 5.0 | 4.2 | 30 | 6.6 |
| OR Quart 4 | 3.0 | 0.82 | 3.4 | 2.0 | 1.4 | 2.7 | 1.9 | 5.2 | 2.1 |
| p Value | 0.016 | 0.75 | 0.011 | 0.11 | 0.56 | 0.036 | 0.28 | 0.14 | 0.24 |
| 95% CI of | 1.2 | 0.24 | 1.3 | 0.86 | 0.44 | 1.1 | 0.60 | 0.60 | 0.60 |
| OR Quart4 | 7.3 | 2.8 | 8.8 | 4.5 | 4.6 | 6.6 | 5.9 | 45 | 7.4 |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Angiopoietin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.11 | 3.75 | 7.11 | 5.43 | 7.11 | 4.97 |
| Average | 20.3 | 108 | 20.3 | 216 | 20.3 | 7.45 |
| Stdev | 79.6 | 562 | 79.6 | 1350 | 79.6 | 7.30 |
| p(t-test) | | 4.2E-4 | | 1.9E-4 | | 0.39 |
| Min | 1.00E-9 | 1.00E-9 | 1.00E-9 | 1.00E-9 | 1.00E-9 | 1.00E-9 |
| Max | 1730 | 3430 | 1730 | 9360 | 1730 | 23.4 |
| n (Samp) | 691 | 37 | 691 | 48 | 691 | 28 |
| n (Patient) | 280 | 37 | 280 | 48 | 280 | 28 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.53 | 2.66 | 6.53 | 3.42 | 6.53 | 5.10 |
| Average | 28.9 | 386 | 28.9 | 46.8 | 28.9 | 9.78 |
| Stdev | 319 | 1140 | 319 | 138 | 319 | 18.2 |
| p(t-test) | | 0.0015 | | 0.84 | | 0.83 |
| Min | 1.00E-9 | 0.318 | 1.00E-9 | 0.511 | 1.00E-9 | 1.00E-9 |
| Max | 9360 | 3430 | 9360 | 504 | 9360 | 68.3 |
| n (Samp) | 900 | 9 | 900 | 13 | 900 | 13 |
| n (Patient) | 336 | 9 | 336 | 13 | 336 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.03 | 3.65 | 7.03 | 5.03 | 7.03 | 4.98 |
| Average | 21.6 | 114 | 21.6 | 240 | 21.6 | 8.12 |
| Stdev | 86.3 | 578 | 86.3 | 1430 | 86.3 | 7.43 |
| p(t-test) | | 9.9E-4 | | 3.0E-4 | | 0.43 |
| Min | 1.00E-9 | 1.00E-9 | 1.00E-9 | 1.00E-9 | 1.00E-9 | 1.00E-9 |
| Max | 1730 | 3430 | 1730 | 9360 | 1730 | 23.4 |
| n (Samp) | 579 | 35 | 579 | 43 | 579 | 25 |
| n (Patient) | 206 | 35 | 206 | 43 | 206 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | 0.40 | 0.42 | 0.43 | 0.42 | 0.42 | 0.40 | 0.38 | 0.43 |
| SE | 0.050 | 0.10 | 0.052 | 0.044 | 0.084 | 0.047 | 0.058 | 0.084 | 0.061 |
| p | 0.18 | 0.30 | 0.14 | 0.11 | 0.32 | 0.078 | 0.078 | 0.15 | 0.23 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 691 | 900 | 579 | 691 | 900 | 579 | 691 | 900 | 579 |
| nCohort 2 | 37 | 9 | 35 | 48 | 13 | 43 | 28 | 13 | 25 |
| Cutoff 1 | 2.71 | 2.09 | 2.53 | 2.49 | 2.65 | 2.19 | 2.77 | 1.73 | 3.66 |
| Sens 1 | 70% | 78% | 71% | 71% | 77% | 72% | 71% | 77% | 72% |
| Spec 1 | 22% | 18% | 19% | 19% | 23% | 16% | 22% | 12% | 30% |
| Cutoff 2 | 1.89 | 1.39 | 1.74 | 1.81 | 0.666 | 1.51 | 1.84 | 0.976 | 2.62 |
| Sens 2 | 81% | 89% | 80% | 81% | 85% | 81% | 82% | 85% | 80% |
| Spec 2 | 13% | 10% | 12% | 12% | 5% | 10% | 13% | 7% | 20% |
| Cutoff 3 | 0.727 | 0.317 | 0.707 | 0.666 | 0.517 | 0.666 | 0.340 | 0.485 | 1.84 |
| Sens 3 | 92% | 100% | 91% | 92% | 92% | 91% | 93% | 92% | 92% |
| Spec 3 | 4% | 2% | 5% | 4% | 3% | 5% | 2% | 3% | 13% |
| Cutoff 4 | 13.8 | 13.1 | 14.6 | 13.8 | 13.1 | 14.6 | 13.8 | 13.1 | 14.6 |
| Sens 4 | 24% | 22% | 23% | 23% | 15% | 26% | 21% | 15% | 24% |
| Spec 4 | 70% | 70% | 70% | 70% | 70% | 70% | 70% | 70% | 70% |
| Cutoff 5 | 20.0 | 19.2 | 21.2 | 20.0 | 19.2 | 21.2 | 20.0 | 19.2 | 21.2 |
| Sens 5 | 24% | 22% | 23% | 12% | 15% | 14% | 14% | 8% | 12% |
| Spec 5 | 80% | 80% | 80% | 80% | 80% | 80% | 80% | 80% | 80% |
| Cutoff 6 | 36.2 | 35.1 | 38.5 | 36.2 | 35.1 | 38.5 | 36.2 | 35.1 | 38.5 |
| Sens 6 | 19% | 11% | 20% | 8% | 15% | 9% | 0% | 8% | 0% |
| Spec 6 | 90% | 90% | 90% | 90% | 90% | 90% | 90% | 90% | 90% |
| OR Quart 2 | 0.54 | 0.50 | 0.62 | 1.2 | 1.5 | 1.1 | 1.0 | 1.0 | 1.0 |
| p Value | 0.28 | 0.57 | 0.41 | 0.65 | 0.65 | 0.79 | 1.0 | 1.00 | 1.0 |
| 95% CI of | 0.18 | 0.045 | 0.20 | 0.50 | 0.25 | 0.43 | 0.28 | 0.14 | 0.28 |
| OR Quart2 | 1.7 | 5.6 | 1.9 | 3.1 | 9.1 | 3.0 | 3.5 | 7.2 | 3.5 |
| OR Quart 3 | 1.0 | 0.50 | 1.1 | 1.1 | 2.0 | 1.0 | 1.8 | 2.0 | 1.6 |
| p Value | 1.0 | 0.57 | 0.80 | 0.81 | 0.42 | 1.0 | 0.28 | 0.42 | 0.40 |
| 95% CI of | 0.39 | 0.045 | 0.43 | 0.44 | 0.37 | 0.37 | 0.61 | 0.37 | 0.52 |
| OR Quart3 | 2.6 | 5.6 | 3.0 | 2.8 | 11 | 2.7 | 5.6 | 11 | 5.1 |
| OR Quart 4 | 1.6 | 2.5 | 1.7 | 2.1 | 2.0 | 2.4 | 1.9 | 2.5 | 1.4 |
| p Value | 0.29 | 0.27 | 0.26 | 0.075 | 0.42 | 0.044 | 0.28 | 0.27 | 0.56 |
| 95% CI of | 0.68 | 0.49 | 0.68 | 0.93 | 0.37 | 1.0 | 0.61 | 0.49 | 0.44 |
| OR Quart4 | 3.8 | 13 | 4.2 | 4.9 | 11 | 5.8 | 5.6 | 13 | 4.6 |

**Brain-derived neurotrophic factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 1.03 | 0.383 | 0.886 | 0.383 | 0.443 |
| Average | 2.43 | 2.34 | 2.43 | 30.0 | 2.43 | 0.851 |
| Stdev | 24.3 | 4.80 | 24.3 | 137 | 24.3 | 0.905 |
| p(t-test) | | 0.98 | | 1.1E-5 | | 0.73 |
| Min | 1.00E-9 | 1.00E-9 | 1.00E-9 | 1.00E-9 | 1.00E-9 | 0.0469 |
| Max | 624 | 27.2 | 624 | 732 | 624 | 3.18 |
| n (Samp) | 691 | 37 | 691 | 48 | 691 | 28 |
| n (Patient) | 280 | 37 | 280 | 48 | 280 | 28 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.398 | 2.02 | 0.398 | 1.69 | 0.398 | 1.03 |
| Average | 3.71 | 3.60 | 3.71 | 6.31 | 3.71 | 1.96 |
| Stdev | 38.4 | 3.21 | 38.4 | 10.6 | 38.4 | 2.35 |
| p(t-test) | | 0.99 | | 0.81 | | 0.87 |
| Min | 1.00E-9 | 0.0280 | 1.00E-9 | 1.00E-9 | 1.00E-9 | 0.0249 |
| Max | 732 | 8.94 | 732 | 37.3 | 732 | 8.33 |
| n (Samp) | 899 | 9 | 899 | 13 | 899 | 13 |
| n (Patient) | 336 | 9 | 336 | 13 | 336 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.403 | 0.756 | 0.403 | 0.838 | 0.403 | 0.547 |
| Average | 1.70 | 2.35 | 1.70 | 33.0 | 1.70 | 0.970 |
| Stdev | 5.80 | 4.93 | 5.80 | 145 | 5.80 | 0.914 |
| p(t-test) | | 0.52 | | 2.7E-7 | | 0.53 |
| Min | 1.00E-9 | 1.00E-9 | 1.00E-9 | 1.00E-9 | 1.00E-9 | 0.0483 |
| Max | 61.7 | 27.2 | 61.7 | 732 | 61.7 | 3.18 |
| n (Samp) | 579 | 35 | 579 | 43 | 579 | 25 |
| n (Patient) | 206 | 35 | 206 | 43 | 206 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.80 | 0.59 | 0.67 | 0.77 | 0.64 | 0.59 | 0.65 | 0.61 |
| SE | 0.050 | 0.090 | 0.052 | 0.044 | 0.078 | 0.047 | 0.058 | 0.083 | 0.061 |
| p | 0.013 | 9.3E-4 | 0.070 | 7.1E-5 | 6.2E-4 | 0.0020 | 0.12 | 0.073 | 0.064 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 691 | 899 | 579 | 691 | 899 | 579 | 691 | 899 | 579 |
| nCohort 2 | 37 | 9 | 35 | 48 | 13 | 43 | 28 | 13 | 25 |
| Cutoff 1 | 0.215 | 1.54 | 0.215 | 0.404 | 0.838 | 0.393 | 0.181 | 0.0984 | 0.249 |
| Sens 1 | 73% | 78% | 71% | 71% | 77% | 72% | 75% | 77% | 72% |
| Spec 1 | 39% | 83% | 36% | 54% | 70% | 48% | 38% | 31% | 41% |
| Cutoff 2 | 0.0656 | 0.524 | 0.0656 | 0.321 | 0.610 | 0.215 | 0.0984 | 0.0852 | 0.181 |
| Sens 2 | 81% | 89% | 80% | 81% | 85% | 84% | 82% | 85% | 84% |
| Spec 2 | 29% | 57% | 26% | 46% | 63% | 36% | 33% | 28% | 35% |
| Cutoff 3 | 0 | 0.0249 | 0 | 0.0356 | 0.404 | 0.0356 | 0.0711 | 0.0466 | 0.0967 |
| Sens 3 | 100% | 100% | 100% | 92% | 92% | 93% | 93% | 92% | 92% |
| Spec 3 | 0% | 21% | 0% | 26% | 52% | 23% | 30% | 25% | 30% |
| Cutoff 4 | 0.768 | 0.853 | 0.838 | 0.768 | 0.853 | 0.838 | 0.768 | 0.853 | 0.838 |
| Sens 4 | 51% | 78% | 49% | 54% | 69% | 47% | 43% | 62% | 48% |
| Spec 4 | 70% | 70% | 70% | 70% | 70% | 70% | 70% | 70% | 70% |
| Cutoff 5 | 1.13 | 1.21 | 1.19 | 1.13 | 1.21 | 1.19 | 1.13 | 1.21 | 1.19 |
| Sens 5 | 46% | 78% | 40% | 44% | 54% | 42% | 29% | 46% | 36% |
| Spec 5 | 80% | 80% | 80% | 80% | 80% | 80% | 80% | 80% | 80% |
| Cutoff 6 | 2.50 | 2.64 | 2.64 | 2.50 | 2.64 | 2.64 | 2.50 | 2.64 | 2.64 |
| Sens 6 | 22% | 44% | 23% | 23% | 31% | 21% | 11% | 38% | 8% |
| Spec 6 | 90% | 90% | 90% | 90% | 90% | 90% | 90% | 90% | 90% |
| OR Quart 2 | 1.8 | 0 | 1.1 | 1.8 | 0 | 1.3 | >14 | 1.5 | 8.4 |
| p Value | 0.28 | na | 0.80 | 0.29 | na | 0.59 | <0.012 | 0.66 | 0.046 |
| 95% CI of | 0.61 | na | 0.40 | 0.60 | na | 0.45 | >1.8 | 0.25 | 1.0 |
| OR Quart2 | 5.6 | na | 3.2 | 5.6 | na | 4.0 | na | 9.1 | 68 |
| OR Quart 3 | 0.80 | 1.0 | 0.70 | 2.5 | 4.1 | 1.7 | >5.1 | 1.0 | 5.1 |
| p Value | 0.74 | 1.0 | 0.56 | 0.094 | 0.21 | 0.31 | <0.14 | 1.0 | 0.14 |
| 95% CI of | 0.21 | 0.062 | 0.22 | 0.86 | 0.45 | 0.61 | >0.59 | 0.14 | 0.59 |
| OR Quart3 | 3.0 | 16 | 2.3 | 7.2 | 37 | 4.8 | na | 7.2 | 45 |
| OR Quart 4 | 4.1 | 7.2 | 2.3 | 4.8 | 8.3 | 3.4 | >11 | 3.1 | 12 |
| p Value | 0.0058 | 0.066 | 0.086 | 0.0019 | 0.047 | 0.010 | <0.026 | 0.17 | 0.019 |
| 95% CI of | 1.5 | 0.88 | 0.89 | 1.8 | 1.0 | 1.3 | >1.3 | 0.61 | 1.5 |
| OR Quart4 | 11 | 59 | 5.7 | 13 | 67 | 8.9 | na | 15 | 92 |

**Creatine Kinase-MB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00872 | 0.0115 | 0.00872 | 0.0101 | 0.00872 | 0.0101 |
| Average | 0.0127 | 0.0203 | 0.0127 | 0.0537 | 0.0127 | 0.0122 |
| Stdev | 0.0168 | 0.0316 | 0.0168 | 0.214 | 0.0168 | 0.0142 |
| p(t-test) | | 0.040 | | 1.5E-4 | | 0.91 |
| Min | 0.00168 | 0.00168 | 0.00168 | 0.00168 | 0.00168 | 0.00168 |
| Max | 0.128 | 0.117 | 0.128 | 1.24 | 0.128 | 0.0612 |
| n (Samp) | 421 | 25 | 421 | 33 | 421 | 17 |
| n (Patient) | 165 | 25 | 165 | 33 | 165 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.00871 | 0.00634 | 0.00871 | 0.0101 |
| Average | nd | nd | 0.0155 | 0.0159 | 0.0155 | 0.00944 |
| Stdev | nd | nd | 0.0571 | 0.0236 | 0.0571 | 0.00572 |
| p(t-test) | nd | nd | | 0.98 | | 0.78 |
| Min | nd | nd | 0.00168 | 0.00168 | 0.00168 | 0.00168 |
| Max | nd | nd | 1.24 | 0.0697 | 1.24 | 0.0153 |
| n (Samp) | nd | nd | 511 | 8 | 511 | 7 |
| n (Patient) | nd | nd | 198 | 8 | 198 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00946 | 0.0101 | 0.00946 | 0.0101 | 0.00946 | 0.0119 |
| Average | 0.0132 | 0.0197 | 0.0132 | 0.0579 | 0.0132 | 0.0130 |
| Stdev | 0.0177 | 0.0319 | 0.0177 | 0.228 | 0.0177 | 0.0139 |
| p(t-test) | | 0.096 | | 3.3E-4 | | 0.97 |
| Min | 0.00168 | 0.00168 | 0.00168 | 0.00168 | 0.00168 | 0.00168 |
| Max | 0.128 | 0.117 | 0.128 | 1.24 | 0.128 | 0.0612 |
| n (Samp) | 357 | 25 | 357 | 29 | 357 | 17 |
| n (Patient) | 135 | 25 | 135 | 29 | 135 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | nd | 0.51 | 0.53 | 0.47 | 0.53 | 0.52 | 0.51 | 0.54 |
| SE | 0.061 | nd | 0.060 | 0.053 | 0.10 | 0.057 | 0.072 | 0.11 | 0.073 |
| p | 0.43 | nd | 0.81 | 0.57 | 0.78 | 0.55 | 0.78 | 0.94 | 0.55 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 421 | nd | 357 | 421 | 511 | 357 | 421 | 511 | 357 |
| nCohort 2 | 25 | nd | 25 | 33 | 8 | 29 | 17 | 7 | 17 |
| Cutoff 1 | 0 | nd | 0 | 0 | 0 | 0 | 0 | 0.00987 | 0.00719 |
| Sens 1 | 100% | nd | 100% | 100% | 100% | 100% | 100% | 71% | 71% |
| Spec 1 | 0% | nd | 0% | 0% | 0% | 0% | 0% | 54% | 40% |
| Cutoff 2 | 0 | nd | 0 | 0 | 0 | 0 | 0 | 0 | 0 |
| Sens 2 | 100% | nd | 100% | 100% | 100% | 100% | 100% | 100% | 100% |
| Spec 2 | 0% | nd | 0% | 0% | 0% | 0% | 0% | 0% | 0% |
| Cutoff 3 | 0 | nd | 0 | 0 | 0 | 0 | 0 | 0 | 0 |
| Sens 3 | 100% | nd | 100% | 100% | 100% | 100% | 100% | 100% | 100% |
| Spec 3 | 0% | nd | 0% | 0% | 0% | 0% | 0% | 0% | 0% |
| Cutoff 4 | 0.0139 | nd | 0.0144 | 0.0139 | 0.0144 | 0.0144 | 0.0139 | 0.0144 | 0.0144 |
| Sens 4 | 36% | nd | 36% | 39% | 25% | 34% | 41% | 29% | 41% |
| Spec 4 | 70% | nd | 72% | 70% | 72% | 72% | 70% | 72% | 72% |
| Cutoff 5 | 0.0177 | nd | 0.0181 | 0.0177 | 0.0181 | 0.0181 | 0.0177 | 0.0181 | 0.0181 |
| Sens 5 | 24% | nd | 24% | 30% | 25% | 28% | 18% | 0% | 12% |
| Spec 5 | 80% | nd | 81% | 80% | 81% | 81% | 80% | 81% | 81% |
| Cutoff 6 | 0.0287 | nd | 0.0287 | 0.0287 | 0.0287 | 0.0287 | 0.0287 | 0.0287 | 0.0287 |
| Sens 6 | 12% | nd | 12% | 18% | 12% | 17% | 6% | 0% | 6% |
| Spec 6 | 91% | nd | 91% | 91% | 90% | 91% | 91% | 90% | 91% |
| OR Quart 2 | 2.4 | nd | 0.18 | 2.3 | 1.0 | 0.37 | >8.5 | >2.0 | 0.38 |
| p Value | 0.21 | nd | 0.030 | 0.13 | 1.0 | 0.10 | <0.045 | <0.57 | 0.26 |
| 95% CI of | 0.60 | nd | 0.039 | 0.77 | 0.14 | 0.11 | >1.1 | >0.18 | 0.072 |
| OR Quart2 | 9.5 | nd | 0.85 | 6.9 | 7.2 | 1.2 | na | na | 2.0 |
| OR Quart 3 | 2.8 | nd | 0.57 | 1.4 | 2.0 | 0.68 | >5.2 | >5.2 | 1.2 |
| p Value | 0.14 | nd | 0.30 | 0.55 | 0.42 | 0.45 | <0.13 | <0.13 | 0.76 |
| 95% CI of | 0.72 | nd | 0.20 | 0.44 | 0.37 | 0.25 | >0.60 | >0.60 | 0.36 |
| OR Quart3 | 11 | nd | 1.6 | 4.6 | 11 | 1.9 | na | na | 4.1 |
| OR Quart 4 | 2.4 | nd | 0.67 | 2.1 | 0 | 0.77 | >4.1 | >0 | 0.78 |
| p Value | 0.21 | nd | 0.43 | 0.20 | na | 0.61 | <0.21 | <na | 0.72 |
| 95% CI of | 0.60 | nd | 0.24 | 0.69 | na | 0.29 | >0.45 | >na | 0.20 |
| OR Quart4 | 9.5 | nd | 1.8 | 6.3 | na | 2.1 | na | na | 3.0 |

**Immunoglobulin M**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8.17E-5 | 0.000143 | 8.17E-5 | 0.000232 | 8.17E-5 | 0.000152 |
| Average | 0.000396 | 0.000187 | 0.000396 | 0.00137 | 0.000396 | 0.000246 |
| Stdev | 0.00119 | 0.000161 | 0.00119 | 0.00370 | 0.00119 | 0.000256 |
| p(t-test) | | 0.38 | | 3.8E-4 | | 0.60 |
| Min | 3.78E-8 | 8.56E-6 | 3.78E-8 | 3.78E-8 | 3.78E-8 | 3.78E-8 |
| Max | 0.0120 | 0.000683 | 0.0120 | 0.0186 | 0.0120 | 0.000755 |
| n (Samp) | 421 | 25 | 421 | 33 | 421 | 17 |
| n (Patient) | 165 | 25 | 165 | 33 | 165 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 9.54E-5 | 0.000274 | 9.54E-5 | 0.000468 |
| Average | nd | nd | 0.000448 | 0.000838 | 0.000448 | 0.000460 |
| Stdev | nd | nd | 0.00147 | 0.00157 | 0.00147 | 0.000363 |
| p(t-test) | nd | nd | | 0.46 | | 0.98 |
| Min | nd | nd | 3.78E-8 | 3.78E-8 | 3.78E-8 | 3.78E-8 |
| Max | nd | nd | 0.0186 | 0.00464 | 0.0186 | 0.000996 |
| n (Samp) | nd | nd | 511 | 8 | 511 | 7 |
| n (Patient) | nd | nd | 198 | 8 | 198 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8.01E-5 | 0.000143 | 8.01E-5 | 0.000232 | 8.01E-5 | 0.000190 |
| Average | 0.000343 | 0.000180 | 0.000343 | 0.00136 | 0.000343 | 0.000247 |
| Stdev | 0.00105 | 0.000157 | 0.00105 | 0.00389 | 0.00105 | 0.000244 |
| p(t-test) | | 0.44 | | 3.4E-4 | | 0.71 |
| Min | 3.78E-8 | 8.56E-6 | 3.78E-8 | 3.78E-8 | 3.78E-8 | 6.81E-6 |
| Max | 0.0120 | 0.000683 | 0.0120 | 0.0186 | 0.0120 | 0.000755 |
| n (Samp) | 357 | 25 | 357 | 29 | 357 | 17 |
| n (Patient) | 135 | 25 | 135 | 29 | 135 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.59 | 0.66 | 0.62 | 0.67 | 0.53 | 0.66 | 0.57 |
| SE | 0.061 | nd | 0.062 | 0.053 | 0.11 | 0.057 | 0.073 | 0.11 | 0.074 |
| p | 0.14 | nd | 0.16 | 0.0029 | 0.27 | 0.0030 | 0.69 | 0.16 | 0.37 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 421 | nd | 357 | 421 | 511 | 357 | 421 | 511 | 357 |
| nCohort 2 | 25 | nd | 25 | 33 | 8 | 29 | 17 | 7 | 17 |
| Cutoff 1 | 7.08E-5 | nd | 7.08E-5 | 0.000106 | 5.56E-5 | 0.000106 | 3.25E-5 | 0.000434 | 3.77E-5 |
| Sens 1 | 76% | nd | 76% | 73% | 75% | 72% | 71% | 71% | 71% |
| Spec 1 | 47% | nd | 47% | 56% | 39% | 56% | 28% | 81% | 32% |
| Cutoff 2 | 5.76E-5 | nd | 5.73E-5 | 5.27E-5 | 5.24E-5 | 5.91E-5 | 1.88E-5 | 3.77E-5 | 2.44E-5 |
| Sens 2 | 80% | nd | 80% | 82% | 88% | 83% | 82% | 86% | 82% |
| Spec 2 | 43% | nd | 42% | 41% | 38% | 44% | 18% | 30% | 24% |
| Cutoff 3 | 3.77E-5 | nd | 3.77E-5 | 3.47E-5 | 0 | 3.47E-5 | 6.27E-6 | 0 | 1.23E-5 |
| Sens 3 | 92% | nd | 92% | 91% | 100% | 93% | 94% | 100% | 94% |
| Spec 3 | 32% | nd | 32% | 29% | 0% | 29% | 6% | 0% | 11% |
| Cutoff 4 | 0.000211 | nd | 0.000216 | 0.000211 | 0.000227 | 0.000216 | 0.000211 | 0.000227 | 0.000216 |
| Sens 4 | 28% | nd | 24% | 55% | 50% | 52% | 41% | 71% | 47% |
| Spec 4 | 70% | nd | 70% | 70% | 70% | 70% | 70% | 70% | 70% |
| Cutoff 5 | 0.000362 | nd | 0.000343 | 0.000362 | 0.000405 | 0.000343 | 0.000362 | 0.000405 | 0.000343 |
| Sens 5 | 16% | nd | 12% | 39% | 25% | 41% | 35% | 71% | 29% |
| Spec 5 | 80% | nd | 80% | 80% | 80% | 80% | 80% | 80% | 80% |
| Cutoff 6 | 0.000751 | nd | 0.000676 | 0.000751 | 0.000753 | 0.000676 | 0.000751 | 0.000753 | 0.000676 |
| Sens 6 | 0% | nd | 4% | 24% | 25% | 21% | 6% | 29% | 6% |
| Spec 6 | 90% | nd | 90% | 90% | 90% | 90% | 90% | 90% | 90% |
| OR Quart 2 | 7.3 | nd | 7.4 | 2.0 | 2.0 | 2.6 | 0.39 | 0.99 | 0.48 |
| p Value | 0.064 | nd | 0.064 | 0.32 | 0.57 | 0.27 | 0.26 | 1.00 | 0.41 |
| 95% CI of | 0.89 | nd | 0.89 | 0.50 | 0.18 | 0.48 | 0.073 | 0.061 | 0.086 |
| OR Quart2 | 61 | nd | 61 | 8.4 | 22 | 13 | 2.0 | 16 | 2.7 |
| OR Quart 3 | 13 | nd | 15 | 3.2 | 0.99 | 4.9 | 0.79 | 0 | 1.5 |
| p Value | 0.014 | nd | 0.0100 | 0.090 | 1.00 | 0.047 | 0.73 | na | 0.52 |
| 95% CI of | 1.7 | nd | 1.9 | 0.84 | 0.061 | 1.0 | 0.21 | na | 0.42 |
| OR Quart3 | 100 | nd | 120 | 12 | 16 | 23 | 3.0 | na | 5.6 |
| OR Quart 4 | 5.1 | nd | 4.1 | 5.6 | 4.1 | 7.3 | 1.2 | 5.1 | 1.2 |
| p Value | 0.14 | nd | 0.21 | 0.0081 | 0.21 | 0.010 | 0.77 | 0.14 | 0.75 |
| 95% CI of | 0.59 | nd | 0.45 | 1.6 | 0.45 | 1.6 | 0.36 | 0.59 | 0.32 |
| OR Quart4 | 45 | nd | 37 | 20 | 37 | 33 | 4.1 | 44 | 4.8 |

**Insulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 208 | 107 | nd | nd |
| Average | nd | nd | 2500 | 4270 | nd | nd |
| Stdev | nd | nd | 6500 | 13600 | nd | nd |
| p(t-test) | nd | nd | | 0.41 | nd | nd |
| Min | nd | nd | 1.00E-9 | 20.3 | nd | nd |
| Max | nd | nd | 50000 | 53000 | nd | nd |
| n (Samp) | nd | nd | 97 | 15 | nd | nd |
| n (Patient) | nd | nd | 74 | 15 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 214 | 98.0 | nd | nd |
| Average | nd | nd | 2370 | 808 | nd | nd |
| Stdev | nd | nd | 6410 | 2080 | nd | nd |
| p(t-test) | nd | nd | | 0.37 | nd | nd |
| Min | nd | nd | 1.00E-9 | 20.3 | nd | nd |
| Max | nd | nd | 50000 | 7810 | nd | nd |
| n (Samp) | nd | nd | 82 | 14 | nd | nd |
| n (Patient) | nd | nd | 62 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.43 | nd | 0.39 | nd | nd | nd |
| SE | nd | nd | nd | 0.082 | nd | 0.086 | nd | nd | nd |
| p | nd | nd | nd | 0.38 | nd | 0.21 | nd | nd | nd |
| nCohort 1 | nd | nd | nd | 97 | nd | 82 | nd | nd | nd |
| nCohort 2 | nd | nd | nd | 15 | nd | 14 | nd | nd | nd |
| Cutoff 1 | nd | nd | nd | 27.1 | nd | 30.7 | nd | nd | nd |
| Sens 1 | nd | nd | nd | 73% | nd | 71% | nd | nd | nd |
| Spec 1 | nd | nd | nd | 13% | nd | 16% | nd | nd | nd |
| Cutoff 2 | nd | nd | nd | 23.8 | nd | 26.2 | nd | nd | nd |
| Sens 2 | nd | nd | nd | 87% | nd | 86% | nd | nd | nd |
| Spec 2 | nd | nd | nd | 11% | nd | 13% | nd | nd | nd |
| Cutoff 3 | nd | nd | nd | 20.3 | nd | 20.3 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | nd | nd | nd | 93% | nd | 93% | nd | nd | nd |
| Spec 3 | nd | nd | nd | 9% | nd | 10% | nd | nd | nd |
| Cutoff 4 | nd | nd | nd | 592 | nd | 665 | nd | nd | nd |
| Sens 4 | nd | nd | nd | 20% | nd | 21% | nd | nd | nd |
| Spec 4 | nd | nd | nd | 70% | nd | 71% | nd | nd | nd |
| Cutoff 5 | nd | nd | nd | 2710 | nd | 2710 | nd | nd | nd |
| Sens 5 | nd | nd | nd | 13% | nd | 7% | nd | nd | nd |
| Spec 5 | nd | nd | nd | 80% | nd | 80% | nd | nd | nd |
| Cutoff 6 | nd | nd | nd | 8120 | nd | 6570 | nd | nd | nd |
| Sens 6 | nd | nd | nd | 7% | nd | 7% | nd | nd | nd |
| Spec 6 | nd | nd | nd | 91% | nd | 90% | nd | nd | nd |
| OR Quart 2 | nd | nd | nd | 0.64 | nd | 1.0 | nd | nd | nd |
| p Value | nd | nd | nd | 0.64 | nd | 1.0 | nd | nd | nd |
| 95% CI of | nd | nd | nd | 0.099 | nd | 0.13 | nd | nd | nd |
| OR Quart2 | nd | nd | nd | 4.2 | nd | 7.7 | nd | nd | nd |
| OR Quart 3 | nd | nd | nd | 1.8 | nd | 2.9 | nd | nd | nd |
| p Value | nd | nd | nd | 0.45 | nd | 0.23 | nd | nd | nd |
| 95% CI of | nd | nd | nd | 0.39 | nd | 0.50 | nd | nd | nd |
| OR Quart3 | nd | nd | nd | 8.4 | nd | 17 | nd | nd | nd |
| OR Quart 4 | nd | nd | nd | 1.8 | nd | 2.9 | nd | nd | nd |
| p Value | nd | nd | nd | 0.45 | nd | 0.23 | nd | nd | nd |
| 95% CI of | nd | nd | nd | 0.39 | nd | 0.50 | nd | nd | nd |
| OR Quart4 | nd | nd | nd | 8.4 | nd | 17 | nd | nd | nd |

**Macrophage migration inhibitory factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14300 | 14600 | 14300 | 17400 | 14300 | 7990 |
| Average | 20200 | 19400 | 20200 | 20200 | 20200 | 12600 |
| Stdev | 18400 | 16700 | 18400 | 17000 | 18400 | 14200 |
| p(t-test) | | 0.86 | | 0.99 | | 0.097 |
| Min | 50.5 | 220 | 50.5 | 989 | 50.5 | 412 |
| Max | 107000 | 50000 | 107000 | 51300 | 107000 | 50000 |
| n (Samp) | 240 | 22 | 240 | 32 | 240 | 17 |
| n (Patient) | 154 | 22 | 154 | 32 | 154 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 13800 | 17600 | 13800 | 12000 |
| Average | nd | nd | 19900 | 20300 | 19900 | 15000 |
| Stdev | nd | nd | 18200 | 16400 | 18200 | 13700 |
| p(t-test) | nd | nd | | 0.95 | | 0.49 |
| Min | nd | nd | 50.5 | 1330 | 50.5 | 3640 |
| Max | nd | nd | 107000 | 50000 | 107000 | 43100 |
| n (Samp) | nd | nd | 308 | 6 | 308 | 7 |
| n (Patient) | nd | nd | 181 | 6 | 181 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14200 | 14600 | 14200 | 16700 | 14200 | 9000 |
| Average | 20600 | 19500 | 20600 | 19600 | 20600 | 16200 |
| Stdev | 19100 | 16600 | 19100 | 16800 | 19100 | 17000 |
| p(t-test) | | 0.79 | | 0.78 | | 0.37 |
| Min | 50.5 | 220 | 50.5 | 989 | 50.5 | 412 |
| Max | 107000 | 50000 | 107000 | 51300 | 107000 | 50000 |
| n (Samp) | 209 | 22 | 209 | 29 | 209 | 16 |
| n (Patient) | 128 | 22 | 128 | 29 | 128 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | nd | 0.51 | 0.51 | 0.54 | 0.50 | 0.37 | 0.45 | 0.43 |
| SE | 0.065 | nd | 0.065 | 0.055 | 0.12 | 0.057 | 0.075 | 0.11 | 0.077 |
| p | 0.89 | nd | 0.86 | 0.85 | 0.73 | 0.95 | 0.091 | 0.67 | 0.39 |
| nCohort 1 | 240 | nd | 209 | 240 | 308 | 209 | 240 | 308 | 209 |
| nCohort 2 | 22 | nd | 22 | 32 | 6 | 29 | 17 | 7 | 16 |
| Cutoff 1 | 7370 | nd | 7460 | 6940 | 11400 | 5100 | 3600 | 6940 | 3370 |
| Sens 1 | 73% | nd | 73% | 72% | 83% | 72% | 71% | 71% | 75% |
| Spec 1 | 33% | nd | 34% | 31% | 44% | 27% | 19% | 31% | 19% |
| Cutoff 2 | 4790 | nd | 7060 | 2210 | 11400 | 2210 | 2780 | 4210 | 2780 |
| Sens 2 | 82% | nd | 82% | 81% | 83% | 83% | 82% | 86% | 81% |
| Spec 2 | 24% | nd | 34% | 13% | 44% | 13% | 16% | 22% | 16% |
| Cutoff 3 | 3960 | nd | 3960 | 1330 | 1330 | 1330 | 1010 | 3600 | 1010 |
| Sens 3 | 91% | nd | 91% | 91% | 100% | 93% | 94% | 100% | 94% |
| Spec 3 | 21% | nd | 22% | 8% | 8% | 7% | 5% | 19% | 6% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 4 | 26200 | nd | 28500 | 26200 | 26700 | 28500 | 26200 | 26700 | 28500 |
| Sens 4 | 27% | nd | 18% | 31% | 17% | 31% | 18% | 14% | 25% |
| Spec 4 | 70% | nd | 70% | 70% | 70% | 70% | 70% | 70% | 70% |
| Cutoff 5 | 34800 | nd | 37700 | 34800 | 36100 | 37700 | 34800 | 36100 | 37700 |
| Sens 5 | 18% | nd | 18% | 25% | 17% | 14% | 12% | 14% | 19% |
| Spec 5 | 80% | nd | 80% | 80% | 80% | 80% | 80% | 80% | 80% |
| Cutoff 6 | 50000 | nd | 50000 | 50000 | 50000 | 50000 | 50000 | 50000 | 50000 |
| Sens 6 | 0% | nd | 0% | 3% | 0% | 3% | 0% | 0% | 0% |
| Spec 6 | 98% | nd | 98% | 98% | 98% | 98% | 98% | 98% | 98% |
| OR Quart 2 | 1.2 | nd | 1.8 | 0.60 | 0.99 | 1.5 | 1.5 | 2.0 | 1.0 |
| p Value | 0.77 | nd | 0.36 | 0.39 | 0.99 | 0.41 | 0.64 | 0.57 | 0.98 |
| 95% CI of | 0.35 | nd | 0.50 | 0.18 | 0.061 | 0.55 | 0.25 | 0.18 | 0.20 |
| OR Quart2 | 4.1 | nd | 6.6 | 1.9 | 16 | 4.4 | 9.6 | 23 | 5.3 |
| OR Quart 3 | 1.4 | nd | 1.8 | 1.4 | 3.1 | 0.69 | 2.7 | 2.0 | 1.8 |
| p Value | 0.55 | nd | 0.36 | 0.46 | 0.33 | 0.54 | 0.25 | 0.57 | 0.45 |
| 95% CI of | 0.43 | nd | 0.50 | 0.54 | 0.31 | 0.21 | 0.50 | 0.18 | 0.40 |
| OR Quart3 | 4.8 | nd | 6.6 | 3.9 | 30 | 2.3 | 14 | 23 | 7.8 |
| OR Quart 4 | 0.77 | nd | 0.98 | 1.0 | 0.99 | 1.0 | 3.9 | 2.1 | 1.8 |
| p Value | 0.71 | nd | 0.98 | 1.0 | 0.99 | 0.97 | 0.100 | 0.56 | 0.45 |
| 95% CI of | 0.20 | nd | 0.23 | 0.35 | 0.061 | 0.33 | 0.77 | 0.18 | 0.40 |
| OR Quart4 | 3.0 | nd | 4.1 | 2.8 | 16 | 3.1 | 19 | 23 | 7.8 |

**Matrilysin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 11900 | 21400 | 11900 | 21200 | 11900 | 17400 |
| Average | 20000 | 40900 | 20000 | 52500 | 20000 | 24500 |
| Stdev | 25400 | 72600 | 25400 | 80800 | 25400 | 23900 |
| p(t-test) |  | 3.2E-5 |  | 1.9E-11 |  | 0.37 |
| Min | 1.00E-9 | 181 | 1.00E-9 | 674 | 1.00E-9 | 401 |
| Max | 261000 | 408000 | 261000 | 406000 | 261000 | 103000 |
| n (Samp) | 691 | 37 | 691 | 48 | 691 | 27 |
| n (Patient) | 281 | 37 | 281 | 48 | 281 | 27 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12800 | 33800 | 12800 | 37500 | 12800 | 22600 |
| Average | 21800 | 92300 | 21800 | 69000 | 21800 | 28300 |
| Stdev | 32500 | 128000 | 32500 | 106000 | 32500 | 25900 |
| p(t-test) | | 1.5E-9 | | 1.1E-6 | | 0.49 |
| Min | 1.00E-9 | 5500 | 1.00E-9 | 1280 | 1.00E-9 | 401 |
| Max | 485000 | 408000 | 485000 | 406000 | 485000 | 72700 |
| n (Samp) | 900 | 9 | 900 | 13 | 900 | 12 |
| n (Patient) | 337 | 9 | 337 | 13 | 337 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12500 | 21600 | 12500 | 21600 | 12500 | 22000 |
| Average | 21400 | 40800 | 21400 | 53800 | 21400 | 30600 |
| Stdev | 26700 | 73400 | 26700 | 84400 | 26700 | 28000 |
| p(t-test) | | 3.9E-4 | | 2.6E-9 | | 0.092 |
| Min | 1.00E-9 | 181 | 1.00E-9 | 674 | 1.00E-9 | 530 |
| Max | 261000 | 408000 | 261000 | 406000 | 261000 | 110000 |
| n (Samp) | 578 | 35 | 578 | 43 | 578 | 25 |
| n (Patient) | 207 | 35 | 207 | 43 | 207 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.79 | 0.64 | 0.68 | 0.72 | 0.68 | 0.58 | 0.57 | 0.64 |
| SE | 0.050 | 0.091 | 0.052 | 0.044 | 0.081 | 0.046 | 0.059 | 0.087 | 0.061 |
| p | 0.0031 | 0.0014 | 0.0071 | 4.2E-5 | 0.0080 | 1.3E-4 | 0.16 | 0.41 | 0.019 |
| nCohort 1 | 691 | 900 | 578 | 691 | 900 | 578 | 691 | 900 | 578 |
| nCohort 2 | 37 | 9 | 35 | 48 | 13 | 43 | 27 | 12 | 25 |
| Cutoff 1 | 13000 | 26400 | 13000 | 14600 | 13600 | 14700 | 9060 | 8260 | 13100 |
| Sens 1 | 70% | 78% | 71% | 71% | 77% | 72% | 70% | 75% | 72% |
| Spec 1 | 53% | 75% | 51% | 57% | 52% | 55% | 42% | 36% | 52% |
| Cutoff 2 | 11700 | 17400 | 11800 | 10100 | 11200 | 10400 | 5680 | 4760 | 9170 |
| Sens 2 | 81% | 89% | 80% | 81% | 85% | 81% | 81% | 83% | 80% |
| Spec 2 | 50% | 60% | 48% | 45% | 45% | 43% | 30% | 24% | 40% |
| Cutoff 3 | 5620 | 5460 | 7260 | 3190 | 2710 | 6910 | 1270 | 1270 | 5680 |
| Sens 3 | 92% | 100% | 91% | 92% | 92% | 91% | 93% | 92% | 92% |
| Spec 3 | 30% | 27% | 34% | 17% | 14% | 32% | 8% | 8% | 27% |
| Cutoff 4 | 21900 | 22600 | 23500 | 21900 | 22600 | 23500 | 21900 | 22600 | 23500 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 46% | 78% | 43% | 48% | 69% | 49% | 44% | 50% | 44% |
| Spec 4 | 70% | 70% | 70% | 70% | 70% | 70% | 70% | 70% | 70% |
| Cutoff 5 | 29500 | 30800 | 33200 | 29500 | 30800 | 33200 | 29500 | 30800 | 33200 |
| Sens 5 | 30% | 56% | 31% | 46% | 62% | 42% | 33% | 50% | 36% |
| Spec 5 | 80% | 80% | 80% | 80% | 80% | 80% | 80% | 80% | 80% |
| Cutoff 6 | 46000 | 49400 | 50800 | 46000 | 49400 | 50800 | 46000 | 49400 | 50800 |
| Sens 6 | 16% | 44% | 14% | 33% | 46% | 28% | 15% | 17% | 12% |
| Spec 6 | 90% | 90% | 90% | 90% | 90% | 90% | 90% | 90% | 90% |
| OR Quart 2 | 4.7 | >1.0 | 4.2 | 0.99 | 0.50 | 1.8 | 1.5 | 1.0 | 2.0 |
| p Value | 0.050 | <1.00 | 0.074 | 0.99 | 0.57 | 0.36 | 0.53 | 1.0 | 0.42 |
| 95% CI of | 1.00 | >0.062 | 0.87 | 0.31 | 0.045 | 0.51 | 0.42 | 0.20 | 0.36 |
| OR Quart2 | 22 | na | 20 | 3.1 | 5.5 | 6.2 | 5.4 | 5.0 | 11 |
| OR Quart 3 | 6.9 | >2.0 | 7.0 | 2.4 | 1.0 | 3.2 | 2.0 | 0 | 4.7 |
| p Value | 0.012 | <0.57 | 0.011 | 0.076 | 1.0 | 0.050 | 0.25 | na | 0.051 |
| 95% CI of | 1.5 | >0.18 | 1.6 | 0.91 | 0.14 | 1.00 | 0.61 | na | 1.00 |
| OR Quart3 | 31 | na | 32 | 6.5 | 7.2 | 10 | 6.9 | na | 22 |
| OR Quart 4 | 6.9 | >6.1 | 6.4 | 4.0 | 4.1 | 5.6 | 2.3 | 2.0 | 5.2 |
| p Value | 0.012 | <0.094 | 0.016 | 0.0034 | 0.077 | 0.0022 | 0.17 | 0.32 | 0.034 |
| 95% CI of | 1.5 | >0.73 | 1.4 | 1.6 | 0.86 | 1.9 | 0.70 | 0.50 | 1.1 |
| OR Quart4 | 31 | na | 29 | 10 | 19 | 17 | 7.6 | 8.2 | 24 |

**Transforming growth factor beta-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.86 | 7.13 | 3.86 | 19.6 | 3.86 | 1.00E-9 |
| Average | 19.9 | 16.6 | 19.9 | 30.6 | 19.9 | 19.5 |
| Stdev | 38.5 | 19.6 | 38.5 | 36.9 | 38.5 | 32.8 |
| p(t-test) | | 0.69 | | 0.14 | | 0.97 |
| Min | 1.00E-9 | 1.00E-9 | 1.00E-9 | 1.00E-9 | 1.00E-9 | 1.00E-9 |
| Max | 364 | 59.3 | 364 | 171 | 364 | 109 |
| n (Samp) | 249 | 22 | 249 | 32 | 249 | 16 |
| n (Patient) | 162 | 22 | 162 | 32 | 162 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 4.40 | 25.1 | 4.40 | 22.8 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | nd | nd | 20.8 | 24.0 | 20.8 | 30.6 |
| Stdev | nd | nd | 37.7 | 16.7 | 37.7 | 43.0 |
| p(t-test) | nd | nd | | 0.83 | | 0.50 |
| Min | nd | nd | 1.00E-9 | 1.00E-9 | 1.00E-9 | 1.00E-9 |
| Max | nd | nd | 364 | 45.5 | 364 | 120 |
| n (Samp) | nd | nd | 315 | 6 | 315 | 7 |
| n (Patient) | nd | nd | 189 | 6 | 189 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.40 | 14.2 | 4.40 | 16.1 | 4.40 | 1.00E-9 |
| Average | 19.7 | 18.5 | 19.7 | 30.4 | 19.7 | 20.5 |
| Stdev | 39.6 | 19.4 | 39.6 | 38.6 | 39.6 | 33.5 |
| p(t-test) | | 0.89 | | 0.17 | | 0.94 |
| Min | 1.00E-9 | 1.00E-9 | 1.00E-9 | 1.00E-9 | 1.00E-9 | 1.00E-9 |
| Max | 364 | 59.3 | 364 | 171 | 364 | 109 |
| n (Samp) | 217 | 22 | 217 | 29 | 217 | 15 |
| n (Patient) | 135 | 22 | 135 | 29 | 135 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | nd | 0.56 | 0.65 | 0.66 | 0.63 | 0.47 | 0.56 | 0.48 |
| SE | 0.065 | nd | 0.066 | 0.055 | 0.12 | 0.058 | 0.076 | 0.11 | 0.078 |
| p | 0.84 | nd | 0.39 | 0.0074 | 0.20 | 0.021 | 0.65 | 0.61 | 0.79 |
| nCohort 1 | 249 | nd | 217 | 249 | 315 | 217 | 249 | 315 | 217 |
| nCohort 2 | 22 | nd | 22 | 32 | 6 | 29 | 16 | 7 | 15 |
| Cutoff 1 | 0 | nd | 0 | 2.83 | 10.6 | 2.63 | 0 | 0 | 0 |
| Sens 1 | 100% | nd | 100% | 72% | 83% | 72% | 100% | 100% | 100% |
| Spec 1 | 0% | nd | 0% | 49% | 59% | 48% | 0% | 0% | 0% |
| Cutoff 2 | 0 | nd | 0 | 1.72 | 10.6 | 0 | 0 | 0 | 0 |
| Sens 2 | 100% | nd | 100% | 81% | 83% | 100% | 100% | 100% | 100% |
| Spec 2 | 0% | nd | 0% | 47% | 59% | 0% | 0% | 0% | 0% |
| Cutoff 3 | 0 | nd | 0 | 0 | 0 | 0 | 0 | 0 | 0 |
| Sens 3 | 100% | nd | 100% | 100% | 100% | 100% | 100% | 100% | 100% |
| Spec 3 | 0% | nd | 0% | 0% | 0% | 0% | 0% | 0% | 0% |
| Cutoff 4 | 18.9 | nd | 17.7 | 18.9 | 22.9 | 17.7 | 18.9 | 22.9 | 17.7 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 41% | nd | 45% | 50% | 67% | 48% | 31% | 43% | 40% |
| Spec 4 | 70% | nd | 70% | 70% | 70% | 70% | 70% | 70% | 70% |
| Cutoff 5 | 29.7 | nd | 27.7 | 29.7 | 35.5 | 27.7 | 29.7 | 35.5 | 27.7 |
| Sens 5 | 27% | nd | 36% | 41% | 33% | 38% | 19% | 29% | 20% |
| Spec 5 | 80% | nd | 80% | 80% | 80% | 80% | 80% | 80% | 80% |
| Cutoff 6 | 58.4 | nd | 48.5 | 58.4 | 59.8 | 48.5 | 58.4 | 59.8 | 48.5 |
| Sens 6 | 5% | nd | 9% | 16% | 0% | 21% | 12% | 14% | 13% |
| Spec 6 | 90% | nd | 90% | 90% | 90% | 90% | 90% | 90% | 90% |
| OR Quart 2 | 0.085 | nd | 0.11 | >13 | 0 | 2.9 | 0.75 | 0 | 1.4 |
| p Value | 0.021 | nd | 0.039 | <0.015 | na | 0.13 | 0.71 | na | 0.70 |
| 95% CI of | 0.011 | nd | 0.013 | >1.6 | na | 0.72 | 0.16 | na | 0.29 |
| OR Quart2 | 0.68 | nd | 0.89 | na | na | 11 | 3.5 | na | 6.4 |
| OR Quart 3 | 0.36 | nd | 0.46 | >9.0 | 2.0 | 2.5 | 0 | 0.32 | 1.7 |
| p Value | 0.095 | nd | 0.22 | <0.041 | 0.57 | 0.20 | na | 0.33 | 0.47 |
| 95% CI of | 0.11 | nd | 0.13 | >1.1 | 0.18 | 0.62 | na | 0.033 | 0.39 |
| OR Quart3 | 1.2 | nd | 1.6 | na | 23 | 10 | na | 3.2 | 7.6 |
| OR Quart 4 | 0.65 | nd | 1.1 | >16 | 3.0 | 4.2 | 2.5 | 0.99 | 1.0 |
| p Value | 0.42 | nd | 0.82 | <0.0089 | 0.34 | 0.035 | 0.15 | 0.99 | 1.0 |
| 95% CI of | 0.23 | nd | 0.40 | >2.0 | 0.31 | 1.1 | 0.73 | 0.19 | 0.19 |
| OR Quart4 | 1.8 | nd | 3.1 | na | 30 | 16 | 8.5 | 5.0 | 5.2 |

**Transforming growth factor beta-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.611 | 0.558 | 0.611 | 0.610 | 0.611 | 1.21 |
| Average | 6.89 | 6.48 | 6.89 | 13.8 | 6.89 | 14.4 |
| Stdev | 22.2 | 21.6 | 22.2 | 45.0 | 22.2 | 25.2 |
| p(t-test) | | 0.93 | | 0.15 | | 0.20 |
| Min | 1.00E-9 | 1.00E-9 | 1.00E-9 | 1.00E-9 | 1.00E-9 | 1.00E-9 |
| Max | 218 | 100 | 218 | 241 | 218 | 74.6 |
| n (Samp) | 249 | 22 | 249 | 32 | 249 | 16 |
| n (Patient) | 162 | 22 | 162 | 32 | 162 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.611 | 0.610 | 0.611 | 0.603 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | nd | nd | 8.40 | 4.59 | 8.40 | 1.90 |
| Stdev | nd | nd | 26.2 | 9.42 | 26.2 | 4.22 |
| p(t-test) | nd | nd | | 0.72 | | 0.51 |
| Min | nd | nd | 1.00E-9 | 1.00E-9 | 1.00E-9 | 1.00E-9 |
| Max | nd | nd | 241 | 23.7 | 241 | 11.4 |
| n (Samp) | nd | nd | 315 | 6 | 315 | 7 |
| n (Patient) | nd | nd | 189 | 6 | 189 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.610 | 0.558 | 0.610 | 0.610 | 0.610 | 1.64 |
| Average | 5.82 | 6.50 | 5.82 | 14.4 | 5.82 | 15.5 |
| Stdev | 19.3 | 21.6 | 19.3 | 47.2 | 19.3 | 25.6 |
| p(t-test) | | 0.88 | | 0.076 | | 0.068 |
| Min | 1.00E-9 | 1.00E-9 | 1.00E-9 | 1.00E-9 | 1.00E-9 | 1.00E-9 |
| Max | 218 | 100 | 218 | 241 | 218 | 74.6 |
| n (Samp) | 217 | 22 | 217 | 29 | 217 | 15 |
| n (Patient) | 135 | 22 | 135 | 29 | 135 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.41 | nd | 0.44 | 0.52 | 0.47 | 0.53 | 0.57 | 0.38 | 0.64 |
| SE | 0.066 | nd | 0.066 | 0.055 | 0.12 | 0.058 | 0.077 | 0.11 | 0.079 |
| p | 0.17 | nd | 0.35 | 0.75 | 0.82 | 0.63 | 0.39 | 0.31 | 0.080 |
| nCohort 1 | 249 | nd | 217 | 249 | 315 | 217 | 249 | 315 | 217 |
| nCohort 2 | 22 | nd | 22 | 32 | 6 | 29 | 16 | 7 | 15 |
| Cutoff 1 | 0 | nd | 0 | 0.258 | 0 | 0.258 | 0.553 | 1.00E-9 | 0.558 |
| Sens 1 | 100% | nd | 100% | 72% | 100% | 72% | 75% | 71% | 73% |
| Spec 1 | 0% | nd | 0% | 33% | 0% | 35% | 35% | 30% | 43% |
| Cutoff 2 | 0 | nd | 0 | 0 | 0 | 0 | 1.00E-9 | 0 | 0.553 |
| Sens 2 | 100% | nd | 100% | 100% | 100% | 100% | 81% | 100% | 87% |
| Spec 2 | 0% | nd | 0% | 0% | 0% | 0% | 31% | 0% | 38% |
| Cutoff 3 | 0 | nd | 0 | 0 | 0 | 0 | 0 | 0 | 0 |
| Sens 3 | 100% | nd | 100% | 100% | 100% | 100% | 100% | 100% | 100% |
| Spec 3 | 0% | nd | 0% | 0% | 0% | 0% | 0% | 0% | 0% |
| Cutoff 4 | 2.30 | nd | 2.23 | 2.30 | 2.30 | 2.23 | 2.30 | 2.30 | 2.23 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 4 | 23% | nd | 23% | 31% | 33% | 31% | 31% | 14% | 40% |
| Spec 4 | 73% | nd | 71% | 73% | 71% | 71% | 73% | 71% | 71% |
| Cutoff 5 | 4.28 | nd | 3.33 | 4.28 | 5.91 | 3.33 | 4.28 | 5.91 | 3.33 |
| Sens 5 | 14% | nd | 23% | 28% | 17% | 28% | 31% | 14% | 33% |
| Spec 5 | 81% | nd | 80% | 81% | 80% | 80% | 81% | 80% | 80% |
| Cutoff 6 | 15.7 | nd | 14.1 | 15.7 | 18.6 | 14.1 | 15.7 | 18.6 | 14.1 |
| Sens 6 | 9% | nd | 9% | 12% | 17% | 10% | 25% | 0% | 27% |
| Spec 6 | 90% | nd | 91% | 90% | 90% | 91% | 90% | 90% | 91% |
| OR Quart 2 | 0.38 | nd | 0.38 | 2.0 | 1.0 | 1.6 | 6.5 | 0 | 1.5 |
| p Value | 0.26 | nd | 0.26 | 0.20 | 0.99 | 0.43 | 0.087 | na | 0.65 |
| 95% CI of | 0.071 | nd | 0.071 | 0.69 | 0.062 | 0.52 | 0.76 | na | 0.25 |
| OR Quart2 | 2.0 | nd | 2.0 | 5.7 | 16 | 4.7 | 56 | na | 9.5 |
| OR Quart 3 | 2.4 | nd | 1.2 | 1.0 | 4.2 | 1.0 | 4.2 | 4.2 | 2.1 |
| p Value | 0.12 | nd | 0.75 | 1.0 | 0.20 | 1.0 | 0.21 | 0.21 | 0.41 |
| 95% CI of | 0.80 | nd | 0.35 | 0.31 | 0.46 | 0.30 | 0.46 | 0.45 | 0.36 |
| OR Quart3 | 7.4 | nd | 4.2 | 3.3 | 39 | 3.3 | 39 | 38 | 12 |
| OR Quart 4 | 0.80 | nd | 2.0 | 1.5 | 0 | 1.4 | 5.2 | 2.1 | 3.2 |
| p Value | 0.75 | nd | 0.25 | 0.43 | na | 0.59 | 0.14 | 0.56 | 0.16 |
| 95% CI of | 0.21 | nd | 0.62 | 0.52 | na | 0.44 | 0.60 | 0.18 | 0.62 |
| OR Quart4 | 3.1 | nd | 6.3 | 4.6 | na | 4.2 | 46 | 23 | 17 |

**Heparan Sulfate**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 3.22 | 6.78 | nd | nd |
| Average | nd | nd | 4.55 | 7.80 | nd | nd |
| Stdev | nd | nd | 3.96 | 8.10 | nd | nd |
| p(t-test) | nd | nd |  | 0.0029 | nd | nd |
| Min | nd | nd | 0.121 | 0.00869 | nd | nd |
| Max | nd | nd | 16.8 | 36.7 | nd | nd |
| n (Samp) | nd | nd | 155 | 21 | nd | nd |
| n (Patient) | nd | nd | 117 | 21 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 3.05 | 5.22 | nd | nd |
| Average | nd | nd | 4.37 | 6.61 | nd | nd |
| Stdev | nd | nd | 3.93 | 4.70 | nd | nd |
| p(t-test) | nd | nd | | 0.026 | nd | nd |
| Min | nd | nd | 0.00869 | 0.796 | nd | nd |
| Max | nd | nd | 16.8 | 18.3 | nd | nd |
| n (Samp) | nd | nd | 132 | 19 | nd | nd |
| n (Patient) | nd | nd | 99 | 19 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.63 | nd | 0.66 | nd | nd | nd |
| SE | nd | nd | nd | 0.069 | nd | 0.072 | nd | nd | nd |
| p | nd | nd | nd | 0.052 | nd | 0.028 | nd | nd | nd |
| nCohort 1 | nd | nd | nd | 155 | nd | 132 | nd | nd | nd |
| nCohort 2 | nd | nd | nd | 21 | nd | 19 | nd | nd | nd |
| Cutoff 1 | nd | nd | nd | 4.09 | nd | 4.09 | nd | nd | nd |
| Sens 1 | nd | nd | nd | 71% | nd | 74% | nd | nd | nd |
| Spec 1 | nd | nd | nd | 59% | nd | 63% | nd | nd | nd |
| Cutoff 2 | nd | nd | nd | 1.86 | nd | 1.83 | nd | nd | nd |
| Sens 2 | nd | nd | nd | 81% | nd | 84% | nd | nd | nd |
| Spec 2 | nd | nd | nd | 29% | nd | 31% | nd | nd | nd |
| Cutoff 3 | nd | nd | nd | 0.879 | nd | 0.879 | nd | nd | nd |
| Sens 3 | nd | nd | nd | 90% | nd | 95% | nd | nd | nd |
| Spec 3 | nd | nd | nd | 9% | nd | 11% | nd | nd | nd |
| Cutoff 4 | nd | nd | nd | 5.34 | nd | 5.32 | nd | nd | nd |
| Sens 4 | nd | nd | nd | 52% | nd | 47% | nd | nd | nd |
| Spec 4 | nd | nd | nd | 70% | nd | 70% | nd | nd | nd |
| Cutoff 5 | nd | nd | nd | 7.30 | nd | 6.83 | nd | nd | nd |
| Sens 5 | nd | nd | nd | 38% | nd | 37% | nd | nd | nd |
| Spec 5 | nd | nd | nd | 80% | nd | 80% | nd | nd | nd |
| Cutoff 6 | nd | nd | nd | 10.3 | nd | 9.32 | nd | nd | nd |
| Sens 6 | nd | nd | nd | 24% | nd | 26% | nd | nd | nd |
| Spec 6 | nd | nd | nd | 90% | nd | 90% | nd | nd | nd |
| OR Quart 2 | nd | nd | nd | 0.23 | nd | 0.31 | nd | nd | nd |
| p Value | nd | nd | nd | 0.20 | nd | 0.32 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | nd | nd | nd | 0.025 | nd | 0.030 | nd | nd | nd |
| OR Quart2 | nd | nd | nd | 2.2 | nd | 3.1 | nd | nd | nd |
| OR Quart 3 | nd | nd | nd | 1.9 | nd | 2.1 | nd | nd | nd |
| p Value | nd | nd | nd | 0.34 | nd | 0.31 | nd | nd | nd |
| 95% CI of | nd | nd | nd | 0.51 | nd | 0.49 | nd | nd | nd |
| OR Quart3 | nd | nd | nd | 7.0 | nd | 9.2 | nd | nd | nd |
| OR Quart 4 | nd | nd | nd | 2.6 | nd | 3.5 | nd | nd | nd |
| p Value | nd | nd | nd | 0.14 | nd | 0.078 | nd | nd | nd |
| 95% CI of | nd | nd | nd | 0.73 | nd | 0.87 | nd | nd | nd |
| OR Quart4 | nd | nd | nd | 9.1 | nd | 14 | nd | nd | nd |

**Transmembrane glycoprotein NMB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.82 | 2.06 | 1.82 | 2.54 | 1.82 | 2.30 |
| Average | 2.08 | 2.31 | 2.08 | 3.70 | 2.08 | 2.03 |
| Stdev | 1.29 | 1.27 | 1.29 | 4.22 | 1.29 | 1.11 |
| p(t-test) | | 0.42 | | 4.7E-6 | | 0.86 |
| Min | 0.203 | 0.181 | 0.203 | 0.533 | 0.203 | 0.135 |
| Max | 8.02 | 5.34 | 8.02 | 25.3 | 8.02 | 3.89 |
| n (Samp) | 257 | 22 | 257 | 32 | 257 | 16 |
| n (Patient) | 164 | 22 | 164 | 32 | 164 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.92 | 3.30 | 1.92 | 2.96 |
| Average | nd | nd | 2.21 | 3.49 | 2.21 | 3.44 |
| Stdev | nd | nd | 1.82 | 1.34 | 1.82 | 2.10 |
| p(t-test) | nd | nd | | 0.087 | | 0.078 |
| Min | nd | nd | 0.135 | 2.23 | 0.135 | 0.849 |
| Max | nd | nd | 25.3 | 5.44 | 25.3 | 7.41 |
| n (Samp) | nd | nd | 323 | 6 | 323 | 7 |
| n (Patient) | nd | nd | 191 | 6 | 191 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.89 | 2.06 | 1.89 | 2.55 | 1.89 | 2.29 |
| Average | 2.12 | 2.40 | 2.12 | 3.68 | 2.12 | 2.19 |
| Stdev | 1.29 | 1.45 | 1.29 | 4.44 | 1.29 | 1.27 |
| p(t-test) | | 0.33 | | 5.6E-5 | | 0.83 |
| Min | 0.203 | 0.181 | 0.203 | 0.533 | 0.203 | 0.135 |
| Max | 8.02 | 6.40 | 8.02 | 25.3 | 8.02 | 4.69 |
| n (Samp) | 223 | 22 | 223 | 29 | 223 | 15 |
| n (Patient) | 136 | 22 | 136 | 29 | 136 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | nd | 0.56 | 0.70 | 0.78 | 0.67 | 0.52 | 0.71 | 0.53 |
| SE | 0.066 | nd | 0.066 | 0.054 | 0.11 | 0.058 | 0.075 | 0.11 | 0.078 |
| p | 0.33 | nd | 0.35 | 1.8E-4 | 0.011 | 0.0036 | 0.84 | 0.064 | 0.68 |
| nCohort 1 | 257 | nd | 223 | 257 | 323 | 223 | 257 | 323 | 223 |
| nCohort 2 | 22 | nd | 22 | 32 | 6 | 29 | 16 | 7 | 15 |
| Cutoff 1 | 1.56 | nd | 1.56 | 2.17 | 2.28 | 1.78 | 1.11 | 2.65 | 1.19 |
| Sens 1 | 73% | nd | 73% | 72% | 83% | 72% | 75% | 71% | 73% |
| Spec 1 | 42% | nd | 41% | 60% | 62% | 47% | 25% | 72% | 26% |
| Cutoff 2 | 1.39 | nd | 1.39 | 1.74 | 2.28 | 1.51 | 0.845 | 1.94 | 1.11 |
| Sens 2 | 82% | nd | 82% | 81% | 83% | 83% | 81% | 86% | 80% |
| Spec 2 | 33% | nd | 31% | 47% | 62% | 39% | 16% | 51% | 24% |
| Cutoff 3 | 1.06 | nd | 1.06 | 1.47 | 2.22 | 1.03 | 0.659 | 0.845 | 0.665 |
| Sens 3 | 91% | nd | 91% | 91% | 100% | 93% | 94% | 100% | 93% |
| Spec 3 | 24% | nd | 22% | 38% | 59% | 22% | 11% | 14% | 9% |
| Cutoff 4 | 2.46 | nd | 2.50 | 2.46 | 2.55 | 2.50 | 2.46 | 2.55 | 2.50 |
| Sens 4 | 36% | nd | 36% | 59% | 50% | 59% | 38% | 71% | 33% |
| Spec 4 | 70% | nd | 70% | 70% | 70% | 70% | 70% | 70% | 70% |
| Cutoff 5 | 3.05 | nd | 3.07 | 3.05 | 3.12 | 3.07 | 3.05 | 3.12 | 3.07 |
| Sens 5 | 23% | nd | 23% | 44% | 50% | 45% | 12% | 43% | 20% |
| Spec 5 | 80% | nd | 80% | 80% | 80% | 80% | 80% | 80% | 80% |
| Cutoff 6 | 4.06 | nd | 4.00 | 4.06 | 4.12 | 4.00 | 4.06 | 4.12 | 4.00 |
| Sens 6 | 14% | nd | 14% | 28% | 33% | 28% | 0% | 29% | 7% |
| Spec 6 | 90% | nd | 90% | 90% | 90% | 90% | 90% | 90% | 90% |
| OR Quart 2 | 2.4 | nd | 2.5 | 1.7 | >0 | 1.3 | 0.48 | 0 | 0.23 |
| p Value | 0.21 | nd | 0.20 | 0.47 | <na | 0.73 | 0.41 | na | 0.20 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.61 | nd | 0.62 | 0.39 | >na | 0.33 | 0.086 | na | 0.025 |
| OR Quart2 | 9.9 | nd | 10 | 7.5 | na | 5.0 | 2.7 | na | 2.1 |
| OR Quart 3 | 2.1 | nd | 2.1 | 3.3 | >3.1 | 1.8 | 1.5 | 2.0 | 1.9 |
| p Value | 0.32 | nd | 0.31 | 0.084 | <0.33 | 0.35 | 0.51 | 0.57 | 0.35 |
| 95% CI of | 0.49 | nd | 0.50 | 0.85 | >0.32 | 0.51 | 0.42 | 0.18 | 0.51 |
| OR Quart3 | 8.6 | nd | 8.9 | 13 | na | 6.6 | 5.8 | 23 | 6.7 |
| OR Quart 4 | 2.1 | nd | 2.1 | 5.9 | >3.1 | 3.8 | 0.98 | 4.1 | 0.72 |
| p Value | 0.32 | nd | 0.32 | 0.0067 | <0.34 | 0.026 | 0.98 | 0.21 | 0.68 |
| 95% CI of | 0.49 | nd | 0.49 | 1.6 | >0.31 | 1.2 | 0.24 | 0.45 | 0.15 |
| OR Quart4 | 8.6 | nd | 8.7 | 22 | na | 13 | 4.1 | 38 | 3.4 |

**Cadherin-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.3 | 22.2 | 14.3 | 13.1 | 14.3 | 6.17 |
| Average | 27.1 | 37.1 | 27.1 | 25.0 | 27.1 | 18.9 |
| Stdev | 27.6 | 46.9 | 27.6 | 33.3 | 27.6 | 28.1 |
| p(t-test) | | 0.13 | | 0.70 | | 0.26 |
| Min | 0.690 | 1.11 | 0.690 | 1.72 | 0.690 | 1.80 |
| Max | 180 | 212 | 180 | 164 | 180 | 110 |
| n (Samp) | 257 | 22 | 257 | 32 | 257 | 16 |
| n (Patient) | 164 | 22 | 164 | 32 | 164 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 14.5 | 11.8 | 14.5 | 2.37 |
| Average | nd | nd | 28.6 | 14.6 | 28.6 | 9.54 |
| Stdev | nd | nd | 31.2 | 7.89 | 31.2 | 10.8 |
| p(t-test) | nd | nd | | 0.28 | | 0.11 |
| Min | nd | nd | 0.690 | 8.86 | 0.690 | 1.32 |
| Max | nd | nd | 212 | 29.6 | 212 | 27.3 |
| n (Samp) | nd | nd | 323 | 6 | 323 | 7 |
| n (Patient) | nd | nd | 191 | 6 | 191 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.5 | 19.4 | 14.5 | 11.0 | 14.5 | 6.83 |
| Average | 27.7 | 35.5 | 27.7 | 25.1 | 27.7 | 20.7 |
| Stdev | 28.6 | 47.5 | 28.6 | 35.2 | 28.6 | 29.1 |
| p(t-test) | | 0.26 | | 0.66 | | 0.36 |
| Min | 0.690 | 1.11 | 0.690 | 1.72 | 0.690 | 2.21 |
| Max | 180 | 212 | 180 | 164 | 180 | 110 |
| n (Samp) | 223 | 22 | 223 | 29 | 223 | 15 |
| n (Patient) | 136 | 22 | 136 | 29 | 136 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.51 | 0.45 | 0.45 | 0.42 | 0.34 | 0.24 | 0.37 |
| SE | 0.065 | nd | 0.065 | 0.055 | 0.12 | 0.058 | 0.076 | 0.11 | 0.079 |
| p | 0.50 | nd | 0.86 | 0.36 | 0.67 | 0.16 | 0.041 | 0.017 | 0.090 |
| nCohort 1 | 257 | nd | 223 | 257 | 323 | 223 | 257 | 323 | 223 |
| nCohort 2 | 22 | nd | 22 | 32 | 6 | 29 | 16 | 7 | 15 |
| Cutoff 1 | 13.5 | nd | 9.08 | 7.44 | 9.43 | 5.13 | 4.14 | 1.96 | 4.31 |
| Sens 1 | 73% | nd | 73% | 72% | 83% | 72% | 75% | 71% | 73% |
| Spec 1 | 48% | nd | 35% | 25% | 35% | 15% | 11% | 3% | 11% |
| Cutoff 2 | 3.97 | nd | 3.34 | 5.13 | 9.43 | 3.20 | 4.00 | 1.71 | 4.09 |
| Sens 2 | 82% | nd | 82% | 81% | 83% | 83% | 81% | 86% | 80% |
| Spec 2 | 9% | nd | 7% | 15% | 35% | 6% | 9% | 2% | 11% |
| Cutoff 3 | 1.96 | nd | 1.95 | 2.53 | 8.79 | 2.13 | 1.96 | 1.27 | 3.75 |
| Sens 3 | 91% | nd | 91% | 91% | 100% | 93% | 94% | 100% | 93% |
| Spec 3 | 3% | nd | 3% | 4% | 33% | 3% | 3% | 2% | 9% |
| Cutoff 4 | 34.1 | nd | 36.1 | 34.1 | 36.2 | 36.1 | 34.1 | 36.2 | 36.1 |
| Sens 4 | 41% | nd | 36% | 22% | 0% | 24% | 19% | 0% | 27% |
| Spec 4 | 70% | nd | 70% | 70% | 70% | 70% | 70% | 70% | 70% |
| Cutoff 5 | 48.0 | nd | 48.4 | 48.0 | 48.2 | 48.4 | 48.0 | 48.2 | 48.4 |
| Sens 5 | 23% | nd | 18% | 12% | 0% | 14% | 12% | 0% | 13% |
| Spec 5 | 80% | nd | 80% | 80% | 80% | 80% | 80% | 80% | 80% |
| Cutoff 6 | 68.4 | nd | 69.4 | 68.4 | 69.4 | 69.4 | 68.4 | 69.4 | 69.4 |
| Sens 6 | 9% | nd | 9% | 9% | 0% | 10% | 6% | 0% | 7% |
| Spec 6 | 90% | nd | 90% | 90% | 90% | 90% | 90% | 90% | 90% |
| OR Quart 2 | 0.57 | nd | 0.47 | 1.2 | >2.1 | 1.8 | 1.5 | >2.1 | 1.6 |
| p Value | 0.46 | nd | 0.31 | 0.75 | <0.55 | 0.35 | 0.64 | <0.55 | 0.64 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.13 | nd | 0.11 | 0.38 | >0.18 | 0.51 | 0.25 | >0.18 | 0.25 |
| OR Quart2 | 2.5 | nd | 2.0 | 3.8 | na | 6.6 | 9.6 | na | 9.7 |
| OR Quart 3 | 1.4 | nd | 1.0 | 1.8 | >4.3 | 1.8 | 1.0 | >1.0 | 1.0 |
| p Value | 0.56 | nd | 1.0 | 0.28 | <0.20 | 0.35 | 0.99 | <0.99 | 1.0 |
| 95% CI of | 0.43 | nd | 0.30 | 0.62 | >0.47 | 0.51 | 0.14 | >0.062 | 0.14 |
| OR Quart3 | 4.7 | nd | 3.3 | 5.2 | na | 6.6 | 7.4 | na | 7.3 |
| OR Quart 4 | 1.4 | nd | 1.2 | 1.6 | >0 | 3.1 | 5.1 | >4.3 | 4.5 |
| p Value | 0.56 | nd | 0.79 | 0.40 | <na | 0.064 | 0.042 | <0.20 | 0.063 |
| 95% CI of | 0.43 | nd | 0.37 | 0.54 | >na | 0.94 | 1.1 | >0.47 | 0.92 |
| OR Quart4 | 4.7 | nd | 3.7 | 4.7 | na | 10 | 25 | na | 22 |

**Stromelysin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.154 | 0.196 | 0.154 | 0.0447 | 0.154 | 0.0653 |
| Average | 0.771 | 0.859 | 0.771 | 0.736 | 0.771 | 0.579 |
| Stdev | 5.89 | 1.92 | 5.89 | 1.77 | 5.89 | 1.24 |
| p(t-test) | | 0.95 | | 0.97 | | 0.90 |
| Min | 1.00E-9 | 1.00E-9 | 1.00E-9 | 1.00E-9 | 1.00E-9 | 1.00E-9 |
| Max | 93.1 | 7.20 | 93.1 | 7.26 | 93.1 | 4.68 |
| n (Samp) | 256 | 22 | 256 | 32 | 256 | 16 |
| n (Patient) | 163 | 22 | 163 | 32 | 163 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.151 | 0.119 | 0.151 | 1.00E-9 |
| Average | nd | nd | 0.799 | 0.131 | 0.799 | 0.302 |
| Stdev | nd | nd | 5.32 | 0.129 | 5.32 | 0.680 |
| p(t-test) | nd | nd | | 0.76 | | 0.81 |
| Min | nd | nd | 1.00E-9 | 1.00E-9 | 1.00E-9 | 1.00E-9 |
| Max | nd | nd | 93.1 | 0.311 | 93.1 | 1.82 |
| n (Samp) | nd | nd | 322 | 6 | 322 | 7 |
| n (Patient) | nd | nd | 190 | 6 | 190 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.152 | 0.123 | 0.152 | 0.00845 | 0.152 | 0.0957 |
| Average | 0.854 | 0.841 | 0.854 | 0.791 | 0.854 | 0.510 |
| Stdev | 6.32 | 1.93 | 6.32 | 1.85 | 6.32 | 1.23 |
| p(t-test) | | 0.99 | | 0.96 | | 0.83 |
| Min | 1.00E-9 | 1.00E-9 | 1.00E-9 | 1.00E-9 | 1.00E-9 | 1.00E-9 |
| Max | 93.1 | 7.20 | 93.1 | 7.26 | 93.1 | 4.68 |
| n (Samp) | 222 | 22 | 222 | 29 | 222 | 15 |
| n (Patient) | 135 | 22 | 135 | 29 | 135 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.46 | 0.40 | 0.40 | 0.39 | 0.41 | 0.30 | 0.41 |
| SE | 0.064 | nd | 0.066 | 0.056 | 0.12 | 0.058 | 0.077 | 0.11 | 0.080 |
| p | 0.94 | nd | 0.52 | 0.065 | 0.40 | 0.053 | 0.25 | 0.072 | 0.27 |
| nCohort 1 | 256 | nd | 222 | 256 | 322 | 222 | 256 | 322 | 222 |
| nCohort 2 | 22 | nd | 22 | 32 | 6 | 29 | 16 | 7 | 15 |
| Cutoff 1 | 0 | nd | 0 | 0 | 0 | 0 | 0 | 0 | 0 |
| Sens 1 | 100% | nd | 100% | 100% | 100% | 100% | 100% | 100% | 100% |
| Spec 1 | 0% | nd | 0% | 0% | 0% | 0% | 0% | 0% | 0% |
| Cutoff 2 | 0 | nd | 0 | 0 | 0 | 0 | 0 | 0 | 0 |
| Sens 2 | 100% | nd | 100% | 100% | 100% | 100% | 100% | 100% | 100% |
| Spec 2 | 0% | nd | 0% | 0% | 0% | 0% | 0% | 0% | 0% |
| Cutoff 3 | 0 | nd | 0 | 0 | 0 | 0 | 0 | 0 | 0 |
| Sens 3 | 100% | nd | 100% | 100% | 100% | 100% | 100% | 100% | 100% |
| Spec 3 | 0% | nd | 0% | 0% | 0% | 0% | 0% | 0% | 0% |
| Cutoff 4 | 0.325 | nd | 0.345 | 0.325 | 0.342 | 0.345 | 0.325 | 0.342 | 0.345 |
| Sens 4 | 41% | nd | 36% | 25% | 0% | 24% | 19% | 14% | 13% |
| Spec 4 | 70% | nd | 70% | 70% | 70% | 70% | 70% | 70% | 70% |
| Cutoff 5 | 0.475 | nd | 0.557 | 0.475 | 0.546 | 0.557 | 0.475 | 0.546 | 0.557 |
| Sens 5 | 27% | nd | 27% | 19% | 0% | 21% | 19% | 14% | 13% |
| Spec 5 | 80% | nd | 80% | 80% | 80% | 80% | 80% | 80% | 80% |
| Cutoff 6 | 0.861 | nd | 0.909 | 0.861 | 1.08 | 0.909 | 0.861 | 1.08 | 0.909 |
| Sens 6 | 18% | nd | 18% | 16% | 0% | 17% | 19% | 14% | 13% |
| Spec 6 | 90% | nd | 90% | 90% | 90% | 90% | 90% | 90% | 90% |
| OR Quart 2 | 0.11 | nd | 0.54 | 0.69 | >3.1 | 0.40 | 1.0 | 1.0 | 2.7 |
| p Value | 0.040 | nd | 0.35 | 0.55 | <0.33 | 0.20 | 1.0 | 0.99 | 0.25 |
| 95% CI of | 0.013 | nd | 0.15 | 0.21 | >0.32 | 0.099 | 0.19 | 0.062 | 0.50 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 0.91 | nd | 2.0 | 2.3 | na | 1.6 | 5.1 | 16 | 14 |
| OR Quart 3 | 0.60 | nd | 0.13 | 0.69 | >1.0 | 0.54 | 1.0 | 0 | 1.0 |
| p Value | 0.39 | nd | 0.059 | 0.55 | <0.99 | 0.35 | 1.0 | na | 0.99 |
| 95% CI of | 0.18 | nd | 0.015 | 0.21 | >0.062 | 0.15 | 0.19 | na | 0.14 |
| OR Quart3 | 1.9 | nd | 1.1 | 2.3 | na | 2.0 | 5.1 | na | 7.5 |
| OR Quart 4 | 0.98 | nd | 1.5 | 2.4 | >2.0 | 2.6 | 2.5 | 5.3 | 3.3 |
| p Value | 0.98 | nd | 0.43 | 0.070 | <0.56 | 0.060 | 0.20 | 0.13 | 0.16 |
| 95% CI of | 0.35 | nd | 0.54 | 0.93 | >0.18 | 0.96 | 0.62 | 0.61 | 0.63 |
| OR Quart4 | 2.8 | nd | 4.3 | 6.4 | na | 6.8 | 10 | 47 | 17 |

**Cathepsin S**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2.23 | 4.00 | nd | nd |
| Average | nd | nd | 2.99 | 6.14 | nd | nd |
| Stdev | nd | nd | 4.05 | 9.41 | nd | nd |
| p(t-test) | nd | nd | | 0.024 | nd | nd |
| Min | nd | nd | 1.00E-9 | 0.992 | nd | nd |
| Max | nd | nd | 37.1 | 39.3 | nd | nd |
| n (Samp) | nd | nd | 106 | 15 | nd | nd |
| n (Patient) | nd | nd | 83 | 15 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2.13 | 3.13 | nd | nd |
| Average | nd | nd | 2.91 | 5.84 | nd | nd |
| Stdev | nd | nd | 4.23 | 9.83 | nd | nd |
| pet-test) | nd | nd | | 0.056 | nd | nd |
| Min | nd | nd | 1.00E-9 | 0.992 | nd | nd |
| Max | nd | nd | 37.1 | 39.3 | nd | nd |
| n (Samp) | nd | nd | 89 | 14 | nd | nd |
| n (Patient) | nd | nd | 69 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | nd | nd | nd | 3.43 | nd | 3.17 | nd | nd | nd |
| Sens 4 | nd | nd | nd | 60% | nd | 50% | nd | nd | nd |
| Spec 4 | nd | nd | nd | 71% | nd | 71% | nd | nd | nd |
| Cutoff 5 | nd | nd | nd | 4.09 | nd | 4.01 | nd | nd | nd |
| Sens 5 | nd | nd | nd | 47% | nd | 36% | nd | nd | nd |
| Spec 5 | nd | nd | nd | 80% | nd | 81% | nd | nd | nd |
| Cutoff 6 | nd | nd | nd | 6.69 | nd | 6.63 | nd | nd | nd |
| Sens 6 | nd | nd | nd | 13% | nd | 14% | nd | nd | nd |
| Spec 6 | nd | nd | nd | 91% | nd | 91% | nd | nd | nd |
| OR Quart 2 | nd | nd | nd | >6.0 | nd | >7.5 | nd | nd | nd |
| p Value | nd | nd | nd | <0.11 | nd | <0.072 | nd | nd | nd |
| 95% CI of | nd | nd | nd | >0.66 | nd | >0.83 | nd | nd | nd |
| OR Quart2 | nd | nd | nd | na | nd | na | nd | nd | nd |
| OR Quart 3 | nd | nd | nd | >2.1 | nd | >1.0 | nd | nd | nd |
| p Value | nd | nd | nd | <0.54 | nd | <1.0 | nd | nd | nd |
| 95% CI of | nd | nd | nd | >0.18 | nd | >0.059 | nd | nd | nd |
| OR Quart3 | nd | nd | nd | na | nd | na | nd | nd | nd |
| OR Quart 4 | nd | nd | nd | >10 | nd | >9.2 | nd | nd | nd |
| p Value | nd | nd | nd | <0.032 | nd | <0.046 | nd | nd | nd |
| 95% CI of | nd | nd | nd | >1.2 | nd | >1.0 | nd | nd | nd |
| OR Quart4 | nd | nd | nd | na | nd | na | nd | nd | nd |

**Urokinase-type plasminogen activator**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 18.9 | 4.87 | nd | nd |
| Average | nd | nd | 26.8 | 16.8 | nd | nd |
| Stdev | nd | nd | 27.5 | 26.0 | nd | nd |
| p(t-test) | nd | nd | | 0.19 | nd | nd |
| Min | nd | nd | 0.125 | 1.24 | nd | nd |
| Max | nd | nd | 119 | 78.3 | nd | nd |
| n (Samp) | nd | nd | 106 | 15 | nd | nd |
| n (Patient) | nd | nd | 83 | 15 | nd | nd |

# EP 2 496 258 B1

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 17.5 | 5.62 | nd | nd |
| Average | nd | nd | 25.8 | 18.7 | nd | nd |
| Stdev | nd | nd | 27.9 | 26.4 | nd | nd |
| p(t-test) | nd | nd | | 0.37 | nd | nd |
| Min | nd | nd | 0.308 | 1.24 | nd | nd |
| Max | nd | nd | 119 | 78.3 | nd | nd |
| n (Samp) | nd | nd | 89 | 14 | nd | nd |
| n (Patient) | nd | nd | 69 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.34 | nd | 0.39 | nd | nd | nd |
| SE | nd | nd | nd | 0.081 | nd | 0.085 | nd | nd | nd |
| p | nd | nd | nd | 0.053 | nd | 0.20 | nd | nd | nd |
| nCohort 1 | nd | nd | nd | 106 | nd | 89 | nd | nd | nd |
| nCohort 2 | nd | nd | nd | 15 | nd | 14 | nd | nd | nd |
| Cutoff 1 | nd | nd | nd | 2.44 | nd | 2.99 | nd | nd | nd |
| Sens 1 | nd | nd | nd | 73% | nd | 71% | nd | nd | nd |
| Spec 1 | nd | nd | nd | 16% | nd | 20% | nd | nd | nd |
| Cutoff 2 | nd | nd | nd | 1.97 | nd | 1.87 | nd | nd | nd |
| Sens 2 | nd | nd | nd | 80% | nd | 86% | nd | nd | nd |
| Spec 2 | nd | nd | nd | 11% | nd | 15% | nd | nd | nd |
| Cutoff 3 | nd | nd | nd | 1.82 | nd | 1.82 | nd | nd | nd |
| Sens 3 | nd | nd | nd | 93% | nd | 93% | nd | nd | nd |
| Spec 3 | nd | nd | nd | 9% | nd | 12% | nd | nd | nd |
| Cutoff 4 | nd | nd | nd | 31.8 | nd | 31.8 | nd | nd | nd |
| Sens 4 | nd | nd | nd | 20% | nd | 21% | nd | nd | nd |
| Spec 4 | nd | nd | nd | 71% | nd | 71% | nd | nd | nd |
| Cutoff 5 | nd | nd | nd | 45.9 | nd | 45.9 | nd | nd | nd |
| Sens 5 | nd | nd | nd | 20% | nd | 21% | nd | nd | nd |
| Spec 5 | nd | nd | nd | 80% | nd | 81% | nd | nd | nd |
| Cutoff 6 | nd | nd | nd | 61.5 | nd | 59.9 | nd | nd | nd |
| Sens 6 | nd | nd | nd | 13% | nd | 14% | nd | nd | nd |
| Spec 6 | nd | nd | nd | 91% | nd | 91% | nd | nd | nd |
| OR Quart 2 | nd | nd | nd | 0 | nd | 0 | nd | nd | nd |
| p Value | nd | nd | nd | na | nd | na | nd | nd | nd |

102

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | nd | nd | nd | na | nd | na | nd | nd | nd |
| OR Quart2 | nd | nd | nd | na | nd | na | nd | nd | nd |
| OR Quart 3 | nd | nd | nd | 1.9 | nd | 2.3 | nd | nd | nd |
| p Value | nd | nd | nd | 0.42 | nd | 0.28 | nd | nd | nd |
| 95% CI of | nd | nd | nd | 0.40 | nd | 0.51 | nd | nd | nd |
| OR Quart3 | nd | nd | nd | 8.6 | nd | 10 | nd | nd | nd |
| OR Quart 4 | nd | nd | nd | 2.8 | nd | 1.9 | nd | nd | nd |
| p Value | nd | nd | nd | 0.16 | nd | 0.41 | nd | nd | nd |
| 95% CI of | nd | nd | nd | 0.66 | nd | 0.41 | nd | nd | nd |
| OR Quart4 | nd | nd | nd | 12 | nd | 9.0 | nd | nd | nd |

**C-C motif chemokine 23**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.00304 | 0.0115 | nd | nd |
| Average | nd | nd | 0.136 | 0.142 | nd | nd |
| Stdev | nd | nd | 1.33 | 0.466 | nd | nd |
| p(t-test) | nd | nd |  | 0.99 | nd | nd |
| Min | nd | nd | 1.00E-9 | 1.00E-9 | nd | nd |
| Max | nd | nd | 13.7 | 1.82 | nd | nd |
| n (Samp) | nd | nd | 106 | 15 | nd | nd |
| n (Patient) | nd | nd | 83 | 15 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.00306 | 0.00981 | nd | nd |
| Average | nd | nd | 0.161 | 0.144 | nd | nd |
| Stdev | nd | nd | 1.45 | 0.484 | nd | nd |
| p(t-test) | nd | nd |  | 0.97 | nd | nd |
| Min | nd | nd | 1.00E-9 | 1.00E-9 | nd | nd |
| Max | nd | nd | 13.7 | 1.82 | nd | nd |
| n (Samp) | nd | nd | 89 | 14 | nd | nd |
| n (Patient) | nd | nd | 69 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.67 | nd | 0.68 | nd | nd | nd |
| SE | nd | nd | nd | 0.081 | nd | 0.083 | nd | nd | nd |
| p | nd | nd | nd | 0.037 | nd | 0.031 | nd | nd | nd |
| nCohort 1 | nd | nd | nd | 106 | nd | 89 | nd | nd | nd |
| nCohort 2 | nd | nd | nd | 15 | nd | 14 | nd | nd | nd |
| Cutoff 1 | nd | nd | nd | 0.00306 | nd | 0.00573 | nd | nd | nd |
| Sens 1 | nd | nd | nd | 73% | nd | 71% | nd | nd | nd |
| Spec 1 | nd | nd | nd | 51% | nd | 65% | nd | nd | nd |
| Cutoff 2 | nd | nd | nd | 0.00221 | nd | 0.00221 | nd | nd | nd |
| Sens 2 | nd | nd | nd | 80% | nd | 86% | nd | nd | nd |
| Spec 2 | nd | nd | nd | 43% | nd | 43% | nd | nd | nd |
| Cutoff 3 | nd | nd | nd | 5.03E-5 | nd | 1.00E-9 | nd | nd | nd |
| Sens 3 | nd | nd | nd | 93% | nd | 93% | nd | nd | nd |
| Spec 3 | nd | nd | nd | 9% | nd | 7% | nd | nd | nd |
| Cutoff 4 | nd | nd | nd | 0.00670 | nd | 0.00670 | nd | nd | nd |
| Sens 4 | nd | nd | nd | 67% | nd | 64% | nd | nd | nd |
| Spec 4 | nd | nd | nd | 71% | nd | 71% | nd | nd | nd |
| Cutoff 5 | nd | nd | nd | 0.0127 | nd | 0.0133 | nd | nd | nd |
| Sens 5 | nd | nd | nd | 47% | nd | 43% | nd | nd | nd |
| Spec 5 | nd | nd | nd | 80% | nd | 81% | nd | nd | nd |
| Cutoff 6 | nd | nd | nd | 0.0187 | nd | 0.0168 | nd | nd | nd |
| Sens 6 | nd | nd | nd | 33% | nd | 36% | nd | nd | nd |
| Spec 6 | nd | nd | nd | 91% | nd | 91% | nd | nd | nd |
| OR Quart 2 | nd | nd | nd | 0.64 | nd | 0.96 | nd | nd | nd |
| p Value | nd | nd | nd | 0.64 | nd | 0.97 | nd | nd | nd |
| 95% CI of | nd | nd | nd | 0.100 | nd | 0.12 | nd | nd | nd |
| OR Quart2 | nd | nd | nd | 4.2 | nd | 7.4 | nd | nd | nd |
| OR Quart 3 | nd | nd | nd | 0.64 | nd | 1.5 | nd | nd | nd |
| p Value | nd | nd | nd | 0.64 | nd | 0.67 | nd | nd | nd |
| 95% CI of | nd | nd | nd | 0.100 | nd | 0.23 | nd | nd | nd |
| OR Quart3 | nd | nd | nd | 4.2 | nd | 9.8 | nd | nd | nd |
| OR Quart 4 | nd | nd | nd | 3.1 | nd | 4.2 | nd | nd | nd |
| p Value | nd | nd | nd | 0.12 | nd | 0.093 | nd | nd | nd |
| 95% CI of | nd | nd | nd | 0.74 | nd | 0.79 | nd | nd | nd |
| OR Quart4 | nd | nd | nd | 13 | nd | 23 | nd | nd | nd |

**Carcinoembryonic antigen-related cell adhesion molecule 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.02 | 7.93 | 6.02 | 11.9 | 6.02 | 8.41 |
| Average | 8.42 | 11.1 | 8.42 | 19.9 | 8.42 | 9.30 |
| Stdev | 9.13 | 8.77 | 9.13 | 29.1 | 9.13 | 6.65 |
| p(t-test) | | 0.18 | | 3.1 E-6 | | 0.71 |
| Min | 0.426 | 0.920 | 0.426 | 1.75 | 0.426 | 1.70 |
| Max | 86.1 | 33.9 | 86.1 | 155 | 86.1 | 22.1 |
| n (Samp) | 257 | 22 | 257 | 32 | 257 | 16 |
| n (Patient) | 164 | 22 | 164 | 32 | 164 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 6.73 | 9.28 | 6.73 | 9.81 |
| Average | nd | nd | 9.58 | 19.9 | 9.58 | 11.9 |
| Stdev | nd | nd | 12.2 | 28.2 | 12.2 | 12.6 |
| p(t-test) | nd | nd | | 0.047 | | 0.62 |
| Min | nd | nd | 0.426 | 1.75 | 0.426 | 1.70 |
| Max | nd | nd | 155 | 76.0 | 155 | 38.0 |
| n (Samp) | nd | nd | 323 | 6 | 323 | 7 |
| n (Patient) | nd | nd | 191 | 6 | 191 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.09 | 7.93 | 6.09 | 12.1 | 6.09 | 8.57 |
| Average | 8.30 | 11.5 | 8.30 | 19.9 | 8.30 | 10.6 |
| Stdev | 9.29 | 9.27 | 9.29 | 28.7 | 9.29 | 6.74 |
| p(t-test) | | 0.13 | | 8.6E-6 | | 0.35 |
| Min | 0.426 | 0.774 | 0.426 | 1.42 | 0.426 | 1.92 |
| Max | 86.1 | 33.9 | 86.1 | 155 | 86.1 | 22.1 |
| n (Samp) | 223 | 22 | 223 | 29 | 223 | 15 |
| n (Patient) | 136 | 22 | 136 | 29 | 136 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | nd | 0.62 | 0.69 | 0.58 | 0.71 | 0.57 | 0.54 | 0.64 |
| SE | 0.066 | nd | 0.066 | 0.054 | 0.12 | 0.056 | 0.077 | 0.11 | 0.079 |
| p | 0.072 | nd | 0.068 | 3.4E-4 | 0.51 | 1.6E-4 | 0.33 | 0.71 | 0.079 |
| nCohort 1 | 257 | nd | 223 | 257 | 323 | 223 | 257 | 323 | 223 |
| nCohort 2 | 22 | nd | 22 | 32 | 6 | 29 | 16 | 7 | 15 |
| Cutoff 1 | 5.66 | nd | 5.66 | 6.57 | 4.82 | 6.58 | 5.49 | 5.13 | 6.09 |
| Sens 1 | 73% | nd | 73% | 72% | 83% | 72% | 75% | 71% | 73% |
| Spec 1 | 47% | nd | 46% | 54% | 35% | 54% | 46% | 38% | 50% |
| Cutoff 2 | 5.13 | nd | 5.13 | 4.92 | 4.82 | 5.16 | 2.24 | 1.87 | 5.53 |
| Sens 2 | 82% | nd | 82% | 81% | 83% | 83% | 81% | 86% | 80% |
| Spec 2 | 42% | nd | 41% | 40% | 35% | 42% | 14% | 10% | 44% |
| Cutoff 3 | 1.81 | nd | 1.81 | 2.83 | 1.67 | 2.62 | 1.87 | 1.67 | 2.24 |
| Sens 3 | 91% | nd | 91% | 91% | 100% | 93% | 94% | 100% | 93% |
| Spec 3 | 11% | nd | 11% | 19% | 8% | 18% | 11% | 8% | 15% |
| Cutoff 4 | 8.97 | nd | 8.98 | 8.97 | 10.1 | 8.98 | 8.97 | 10.1 | 8.98 |
| Sens 4 | 45% | nd | 45% | 59% | 50% | 62% | 44% | 43% | 47% |
| Spec 4 | 70% | nd | 70% | 70% | 70% | 70% | 70% | 70% | 70% |
| Cutoff 5 | 11.6 | nd | 11.3 | 11.6 | 12.6 | 11.3 | 11.6 | 12.6 | 11.3 |
| Sens 5 | 36% | nd | 36% | 53% | 50% | 55% | 25% | 29% | 33% |
| Spec 5 | 80% | nd | 80% | 80% | 80% | 80% | 80% | 80% | 80% |
| Cutoff 6 | 16.8 | nd | 15.1 | 16.8 | 18.3 | 15.1 | 16.8 | 18.3 | 15.1 |
| Sens 6 | 18% | nd | 27% | 31% | 17% | 41% | 25% | 14% | 33% |
| Spec 6 | 90% | nd | 90% | 90% | 90% | 90% | 90% | 90% | 90% |
| OR Quart 2 | 0.98 | nd | 1.7 | 1.3 | 2.0 | 0.74 | 0.24 | 0.49 | 2.0 |
| p Value | 0.98 | nd | 0.47 | 0.73 | 0.57 | 0.70 | 0.21 | 0.56 | 0.42 |
| 95% CI of | 0.24 | nd | 0.39 | 0.33 | 0.18 | 0.16 | 0.026 | 0.043 | 0.36 |
| OR Quart2 | 4.1 | nd | 7.6 | 4.9 | 23 | 3.4 | 2.2 | 5.5 | 12 |
| OR Quart 3 | 1.5 | nd | 2.1 | 1.3 | 0 | 1.3 | 1.5 | 1.0 | 2.1 |
| p Value | 0.53 | nd | 0.31 | 0.73 | na | 0.73 | 0.51 | 1.0 | 0.41 |
| 95% CI of | 0.41 | nd | 0.50 | 0.33 | na | 0.33 | 0.42 | 0.14 | 0.36 |
| OR Quart3 | 5.7 | nd | 8.9 | 4.9 | na | 5.0 | 5.8 | 7.3 | 12 |
| OR Quart 4 | 2.1 | nd | 2.9 | 5.6 | 3.0 | 5.5 | 1.2 | 0.99 | 2.6 |
| p Value | 0.25 | nd | 0.13 | 0.0032 | 0.34 | 0.0040 | 0.75 | 0.99 | 0.27 |
| 95% CI of | 0.60 | nd | 0.72 | 1.8 | 0.31 | 1.7 | 0.32 | 0.14 | 0.48 |
| OR Quart4 | 7.3 | nd | 11 | 17 | 30 | 17 | 4.9 | 7.2 | 14 |

**Insulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.06 | 0.981 | 1.06 | 0.660 | 1.06 | 0.950 |
| Average | 19.0 | 6.73 | 19.0 | 38.3 | 19.0 | 1.90 |
| Stdev | 105 | 20.7 | 105 | 207 | 105 | 3.46 |
| p(t-test) | | 0.56 | | 0.36 | | 0.50 |
| Min | 0.00344 | 0.101 | 0.00344 | 0.0393 | 0.00344 | 0.00344 |
| Max | 1750 | 105 | 1750 | 1190 | 1750 | 14.3 |
| n (Samp) | 421 | 25 | 421 | 33 | 421 | 17 |
| n (Patient) | 165 | 25 | 165 | 33 | 165 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.981 | 1.91 | 0.981 | 0.917 |
| Average | nd | nd | 16.6 | 154 | 16.6 | 1.48 |
| Stdev | nd | nd | 95.8 | 419 | 95.8 | 1.72 |
| p(t-test) | nd | nd | | 3.3E-4 | | 0.68 |
| Min | nd | nd | 0.00344 | 0.283 | 0.00344 | 0.116 |
| Max | nd | nd | 1750 | 1190 | 1750 | 5.16 |
| n (Samp) | nd | nd | 511 | 8 | 511 | 7 |
| n (Patient) | nd | nd | 198 | 8 | 198 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.23 | 0.849 | 1.23 | 0.660 | 1.23 | 0.960 |
| Average | 20.9 | 5.35 | 20.9 | 5.60 | 20.9 | 72.2 |
| Stdev | 113 | 15.8 | 113 | 19.4 | 113 | 288 |
| p(t-test) | | 0.49 | | 0.47 | | 0.10 |
| Min | 0.00344 | 0.101 | 0.00344 | 0.0393 | 0.00344 | 0.00344 |
| Max | 1750 | 80.0 | 1750 | 105 | 1750 | 1190 |
| n (Samp) | 357 | 25 | 357 | 29 | 357 | 17 |
| n (Patient) | 135 | 25 | 135 | 29 | 135 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | nd | 0.44 | 0.40 | 0.59 | 0.37 | 0.39 | 0.44 | 0.42 |
| SE | 0.060 | nd | 0.061 | 0.054 | 0.11 | 0.057 | 0.074 | 0.11 | 0.074 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.75 | nd | 0.30 | 0.073 | 0.40 | 0.026 | 0.13 | 0.61 | 0.27 |
| nCohort 1 | 421 | nd | 357 | 421 | 511 | 357 | 421 | 511 | 357 |
| nCohort 2 | 25 | nd | 25 | 33 | 8 | 29 | 17 | 7 | 17 |
| Cutoff 1 | 0.620 | nd | 0.620 | 0.435 | 0.713 | 0.489 | 0.336 | 0.898 | 0.661 |
| Sens 1 | 72% | nd | 72% | 73% | 75% | 72% | 71% | 71% | 71% |
| Spec 1 | 29% | nd | 25% | 19% | 36% | 18% | 16% | 46% | 29% |
| Cutoff 2 | 0.467 | nd | 0.467 | 0.329 | 0.329 | 0.329 | 0.101 | 0.348 | 0.245 |
| Sens 2 | 80% | nd | 80% | 82% | 88% | 83% | 88% | 86% | 82% |
| Spec 2 | 19% | nd | 18% | 14% | 16% | 12% | 7% | 17% | 11% |
| Cutoff 3 | 0.129 | nd | 0.116 | 0.219 | 0.277 | 0.173 | 0.0381 | 0.101 | 0.0381 |
| Sens 3 | 92% | nd | 92% | 91% | 100% | 93% | 94% | 100% | 94% |
| Spec 3 | 8% | nd | 6% | 10% | 14% | 7% | 5% | 7% | 4% |
| Cutoff 4 | 2.78 | nd | 3.44 | 2.78 | 2.76 | 3.44 | 2.78 | 2.76 | 3.44 |
| Sens 4 | 36% | nd | 24% | 21% | 38% | 17% | 18% | 14% | 18% |
| Spec 4 | 70% | nd | 70% | 70% | 70% | 70% | 70% | 70% | 70% |
| Cutoff 5 | 8.45 | nd | 8.97 | 8.45 | 7.71 | 8.97 | 8.45 | 7.71 | 8.97 |
| Sens 5 | 12% | nd | 8% | 18% | 38% | 14% | 6% | 0% | 18% |
| Spec 5 | 80% | nd | 80% | 80% | 80% | 80% | 80% | 80% | 80% |
| Cutoff 6 | 28.0 | nd | 29.0 | 28.0 | 27.1 | 29.0 | 28.0 | 27.1 | 29.0 |
| Sens 6 | 4% | nd | 4% | 3% | 12% | 3% | 0% | 0% | 6% |
| Spec 6 | 90% | nd | 90% | 90% | 90% | 90% | 90% | 90% | 90% |
| OR Quart 2 | 0.83 | nd | 1.6 | 0.83 | 0.49 | 0.59 | 2.1 | 2.0 | 1.0 |
| p Value | 0.77 | nd | 0.51 | 0.77 | 0.56 | 0.48 | 0.41 | 0.56 | 0.99 |
| 95% CI of | 0.25 | nd | 0.42 | 0.25 | 0.044 | 0.14 | 0.37 | 0.18 | 0.20 |
| OR Quart2 | 2.8 | nd | 5.7 | 2.8 | 5.5 | 2.6 | 11 | 23 | 5.1 |
| OR Quart 3 | 1.4 | nd | 2.4 | 1.5 | 0.99 | 2.1 | 2.6 | 2.0 | 2.1 |
| p Value | 0.58 | nd | 0.16 | 0.43 | 0.99 | 0.19 | 0.27 | 0.57 | 0.31 |
| 95% CI of | 0.46 | nd | 0.71 | 0.53 | 0.14 | 0.70 | 0.49 | 0.18 | 0.50 |
| OR Quart3 | 4.1 | nd | 8.0 | 4.5 | 7.2 | 6.4 | 14 | 23 | 8.5 |
| OR Quart 4 | 1.0 | nd | 1.6 | 2.3 | 1.5 | 2.4 | 3.1 | 2.0 | 1.7 |
| p Value | 0.99 | nd | 0.51 | 0.097 | 0.66 | 0.12 | 0.17 | 0.56 | 0.47 |
| 95% CI of | 0.32 | nd | 0.42 | 0.86 | 0.25 | 0.79 | 0.62 | 0.18 | 0.40 |
| OR Quart4 | 3.2 | nd | 5.7 | 6.4 | 9.1 | 7.1 | 16 | 23 | 7.4 |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Angopoietin-2**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 5.44 | 6.54 | 5.17 | 7.13 | 5.85 | 6.15 |
| Average | 12.1 | 12.7 | 15.5 | 8.07 | 13.0 | 12.3 |
| Stdev | 19.6 | 18.5 | 28.5 | 6.57 | 24.9 | 20.8 |
| p(t-test) | | 0.88 | | 0.42 | | 0.91 |
| Min | 1.00E-9 | 1.00E-9 | 1.32 | 0.525 | 1.00E-9 | 1.00E-9 |
| Max | 148 | 102 | 148 | 19.6 | 185 | 102 |
| n (Samp) | 78 | 33 | 30 | 10 | 61 | 24 |
| n (Patient) | 78 | 33 | 30 | 10 | 61 | 24 |

| | At Enrollment | | |
|---|---|---|---|
| | sCr or UO | sCr only | UO only |
| AUC | 0.54 | 0.47 | 0.50 |
| SE | 0.061 | 0.11 | 0.070 |
| p | 0.51 | 0.78 | 0.99 |
| nCohort 1 | 78 | 30 | 61 |
| nCohort 2 | 33 | 10 | 24 |
| Cutoff 1 | 2.90 | 2.90 | 2.46 |
| Sens 1 | 73% | 70% | 71% |
| Spec 1 | 36% | 23% | 36% |
| Cutoff 2 | 2.32 | 2.25 | 1.73 |
| Sens 2 | 82% | 80% | 83% |
| Spec 2 | 28% | 13% | 21% |
| Cutoff 3 | 1.43 | 1.62 | 1.00 |
| Sens 3 | 91% | 90% | 92% |
| Spec 3 | 17% | 10% | 16% |
| Cutoff 4 | 12.0 | 10.9 | 12.9 |
| Sens 4 | 33% | 30% | 29% |
| Spec 4 | 71% | 70% | 70% |
| Cutoff 5 | 15.8 | 14.6 | 15.9 |
| Sens 5 | 27% | 20% | 25% |
| Spec 5 | 81% | 80% | 80% |
| Cutoff 6 | 31.7 | 38.5 | 29.6 |
| Sens 6 | 6% | 0% | 4% |
| Spec 6 | 91% | 90% | 90% |
| OR Quart 2 | 1.4 | 1.0 | 1.6 |

(continued)

|  | At Enrollment | | |
|---|---|---|---|
|  | sCr or UO | sCr only | UO only |
| p Value | 0.59 | 1.0 | 0.50 |
| 95% CI of | 0.41 | 0.15 | 0.41 |
| OR Quart2 | 4.8 | 6.8 | 6.2 |
| OR Quart 3 | 1.7 | 0.26 | 1.0 |
| p Value | 0.41 | 0.28 | 1.0 |
| 95% CI of | 0.50 | 0.022 | 0.24 |
| OR Quart3 | 5.5 | 3.1 | 4.1 |
| OR Quart 4 | 1.9 | 1.0 | 1.5 |
| p Value | 0.27 | 1.0 | 0.56 |
| 95% CI of | 0.59 | 0.15 | 0.39 |
| OR Quart4 | 6.4 | 6.8 | 5.7 |

**Immunoglobulin E**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.609 | 0.0560 | nd | nd | 0.695 | 0.0560 |
| Average | 1.85 | 1.58 | nd | nd | 1.98 | 1.16 |
| Stdev | 2.42 | 2.47 | nd | nd | 2.52 | 2.21 |
| p(t-test) |  | 0.65 | nd | nd |  | 0.26 |
| Min | 0.0560 | 0.0560 | nd | nd | 0.0560 | 0.0560 |
| Max | 8.64 | 7.36 | nd | nd | 8.64 | 6.46 |
| n (Samp) | 52 | 23 | nd | nd | 43 | 16 |
| n (Patient) | 52 | 23 | nd | nd | 43 | 16 |

|  | At Enrollment | | |
|---|---|---|---|
|  | sCr or UO | sCr only | UO only |
| AUC | 0.43 | nd | 0.39 |
| SE | 0.073 | nd | 0.085 |
| p | 0.36 | nd | 0.19 |
| nCohort 1 | 52 | nd | 43 |
| nCohort 2 | 23 | nd | 16 |
| Cutoff 1 | 0 | nd | 0 |
| Sens 1 | 100% | nd | 100% |
| Spec 1 | 0% | nd | 0% |
| Cutoff 2 | 0 | nd | 0 |
| Sens 2 | 100% | nd | 100% |

(continued)

|  | At Enrollment | | |
|---|---|---|---|
|  | sCr or UO | sCr only | UO only |
| Spec 2 | 0% | nd | 0% |
| Cutoff 3 | 0 | nd | 0 |
| Sens 3 | 100% | nd | 100% |
| Spec 3 | 0% | nd | 0% |
| Cutoff 4 | 2.39 | nd | 3.01 |
| Sens 4 | 26% | nd | 19% |
| Spec 4 | 71% | nd | 72% |
| Cutoff 5 | 3.99 | nd | 4.59 |
| Sens 5 | 22% | nd | 12% |
| Spec 5 | 81% | nd | 81% |
| Cutoff 6 | 5.97 | nd | 6.25 |
| Sens 6 | 9% | nd | 6% |
| Spec 6 | 90% | nd | 93% |
| OR Quart 2 | 0.41 | nd | 0.62 |
| p Value | 0.26 | nd | 0.63 |
| 95% CI of | 0.085 | nd | 0.087 |
| OR Quart2 | 1.9 | nd | 4.3 |
| OR Quart 3 | 0.77 | nd | 2.7 |
| p Value | 0.72 | nd | 0.24 |
| 95% CI of | 0.19 | nd | 0.52 |
| OR Quart3 | 3.2 | nd | 14 |
| OR Quart 4 | 2.2 | nd | 2.2 |
| p Value | 0.26 | nd | 0.35 |
| 95% CI of | 0.57 | nd | 0.42 |
| OR Quart4 | 8.3 | nd | 12 |

**Immunoglobulin M**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 8.20E-5 | 0.000175 | nd | nd | 9.71E-5 | 0.000182 |
| Average | 0.000239 | 0.000765 | nd | nd | 0.000213 | 0.000978 |
| Stdev | 0.000467 | 0.00229 | nd | nd | 0.000290 | 0.00274 |
| p(t-test) |  | 0.12 | nd | nd |  | 0.072 |
| Min | 7.02E-6 | 3.78E-8 | nd | nd | 8.56E-6 | 3.78E-8 |
| Max | 0.00293 | 0.0111 | nd | nd | 0.00118 | 0.0111 |
| n (Samp) | 52 | 23 | nd | nd | 43 | 16 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| n (Patient) | 52 | 23 | nd | nd | 43 | 16 |

|  | At Enrollment | | |
|---|---|---|---|
|  | sCr or UO | sCr only | UO only |
| AUC | 0.62 | nd | 0.63 |
| SE | 0.072 | nd | 0.085 |
| p | 0.10 | nd | 0.14 |
| nCohort 1 | 52 | nd | 43 |
| nCohort 2 | 23 | nd | 16 |
| Cutoff 1 | 5.15E-5 | nd | 8.07E-5 |
| Sens 1 | 74% | nd | 75% |
| Spec 1 | 42% | nd | 44% |
| Cutoff 2 | 3.65E-5 | nd | 5.15E-5 |
| Sens 2 | 83% | nd | 81% |
| Spec 2 | 33% | nd | 35% |
| Cutoff 3 | 2.64E-5 | nd | 2.64E-5 |
| Sens 3 | 91% | nd | 94% |
| Spec 3 | 29% | nd | 26% |
| Cutoff 4 | 0.000215 | nd | 0.000223 |
| Sens 4 | 43% | nd | 44% |
| Spec 4 | 71% | nd | 72% |
| Cutoff 5 | 0.000302 | nd | 0.000319 |
| Sens 5 | 35% | nd | 38% |
| Spec 5 | 81% | nd | 81% |
| Cutoff 6 | 0.000434 | nd | 0.000451 |
| Sens 6 | 26% | nd | 19% |
| Spec 6 | 90% | nd | 91% |
| OR Quart 2 | 1.8 | nd | 1.5 |
| p Value | 0.48 | nd | 0.69 |
| 95% CI of | 0.36 | nd | 0.21 |
| OR Quart2 | 8.9 | nd | 11 |
| OR Quart 3 | 2.9 | nd | 3.0 |
| p Value | 0.18 | nd | 0.24 |
| 95% CI of | 0.62 | nd | 0.48 |
| OR Quart3 | 14 | nd | 19 |
| OR Quart 4 | 3.6 | nd | 4.0 |

(continued)

|  | At Enrollment | | |
|---|---|---|---|
|  | sCr or UO | sCr only | UO only |
| p Value | 0.100 | nd | 0.14 |
| 95% CI of | 0.78 | nd | 0.65 |
| OR Quart4 | 17 | nd | 25 |

**Macrophage migration inhibitory factor**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 9690 | 16700 | nd | nd | 15400 | 11400 |
| Average | 19200 | 21400 | nd | nd | 21600 | 20600 |
| Stdev | 18000 | 18300 | nd | nd | 18400 | 17700 |
| p(t-test) |  | 0.62 | nd | nd |  | 0.85 |
| Min | 272 | 989 | nd | nd | 272 | 989 |
| Max | 50000 | 51300 | nd | nd | 50000 | 51300 |
| n (Samp) | 50 | 24 | nd | nd | 41 | 17 |
| n (Patient) | 50 | 24 | nd | nd | 41 | 17 |

|  | At Enrollment | | |
|---|---|---|---|
|  | sCr or UO | sCr only | UO only |
| AUC | 0.54 | nd | 0.50 |
| SE | 0.073 | nd | 0.084 |
| p | 0.57 | nd | 0.98 |
| nCohort 1 | 50 | nd | 41 |
| nCohort 2 | 24 | nd | 17 |
| Cutoff 1 | 5750 | nd | 7020 |
| Sens 1 | 71% | nd | 71% |
| Spec 1 | 36% | nd | 34% |
| Cutoff 2 | 4640 | nd | 4820 |
| Sens 2 | 83% | nd | 82% |
| Spec 2 | 30% | nd | 22% |
| Cutoff 3 | 1510 | nd | 1220 |
| Sens 3 | 92% | nd | 94% |
| Spec 3 | 6% | nd | 5% |
| Cutoff 4 | 28800 | nd | 31800 |
| Sens 4 | 38% | nd | 24% |
| Spec 4 | 70% | nd | 71% |
| Cutoff 5 | 34600 | nd | 50000 |

(continued)

|  | At Enrollment | | |
|---|---|---|---|
|  | sCr or UO | sCr only | UO only |
| Sens 5 | 21% | nd | 6% |
| Spec 5 | 80% | nd | 100% |
| Cutoff 6 | 50000 | nd | 50000 |
| Sens 6 | 4% | nd | 6% |
| Spec 6 | 100% | nd | 100% |
| OR Quart 2 | 2.0 | nd | 1.1 |
| p Value | 0.33 | nd | 0.91 |
| 95% CI of | 0.48 | nd | 0.22 |
| OR Quart2 | 8.7 | nd | 5.6 |
| OR Quart 3 | 2.2 | nd | 1.0 |
| p Value | 0.28 | nd | 1.0 |
| 95% CI of | 0.52 | nd | 0.20 |
| OR Quart3 | 9.6 | nd | 5.0 |
| OR Quart 4 | 1.6 | nd | 1.5 |
| p Value | 0.52 | nd | 0.60 |
| 95% CI of | 0.37 | nd | 0.31 |
| OR Quart4 | 7.0 | nd | 7.4 |

**Matrilysin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 14100 | 18500 | 13000 | 37600 | 17700 | 19800 |
| Average | 21900 | 35700 | 23300 | 40100 | 25100 | 37200 |
| Stdev | 21100 | 42200 | 25500 | 30700 | 24500 | 44000 |
| p(t-test) |  | 0.023 |  | 0.094 |  | 0.11 |
| Min | 181 | 548 | 181 | 548 | 1030 | 3820 |
| Max | 115000 | 212000 | 117000 | 91900 | 115000 | 212000 |
| n (Samp) | 79 | 33 | 30 | 10 | 62 | 24 |
| n (Patient) | 79 | 33 | 30 | 10 | 62 | 24 |

|  | At Enrollment | | |
|---|---|---|---|
|  | sCr or UO | sCr only | UO only |
| AUC | 0.60 | 0.68 | 0.59 |
| SE | 0.060 | 0.10 | 0.070 |
| p | 0.091 | 0.090 | 0.19 |
| nCohort 1 | 79 | 30 | 62 |

(continued)

|  | At Enrollment | | |
|---|---|---|---|
|  | sCr or UO | sCr only | UO only |
| nCohort 2 | 33 | 10 | 24 |
| Cutoff 1 | 10200 | 14100 | 13600 |
| Sens 1 | 73% | 70% | 71% |
| Spec 1 | 39% | 57% | 42% |
| Cutoff 2 | 8810 | 8810 | 9680 |
| Sens 2 | 82% | 80% | 83% |
| Spec 2 | 38% | 47% | 32% |
| Cutoff 3 | 4310 | 8160 | 4570 |
| Sens 3 | 91% | 90% | 92% |
| Spec 3 | 18% | 47% | 15% |
| Cutoff 4 | 27900 | 27900 | 30700 |
| Sens 4 | 39% | 60% | 42% |
| Spec 4 | 71% | 70% | 71% |
| Cutoff 5 | 36500 | 38400 | 42300 |
| Sens 5 | 33% | 50% | 33% |
| Spec 5 | 81% | 80% | 81% |
| Cutoff 6 | 51000 | 49400 | 51800 |
| Sens 6 | 24% | 30% | 25% |
| Spec 6 | 91% | 90% | 90% |
| OR Quart 2 | 2.2 | 2.2 | 3.4 |
| p Value | 0.22 | 0.54 | 0.11 |
| 95% CI of | 0.62 | 0.17 | 0.77 |
| OR Quart2 | 7.6 | 30 | 15 |
| OR Quart 3 | 1.8 | 2.2 | 1.9 |
| p Value | 0.35 | 0.54 | 0.44 |
| 95% CI of | 0.52 | 0.17 | 0.39 |
| OR Quart3 | 6.5 | 30 | 9.1 |
| OR Quart 4 | 3.0 | 9.0 | 3.4 |
| p Value | 0.082 | 0.074 | 0.11 |
| 95% CI of | 0.87 | 0.81 | 0.77 |
| OR Quart4 | 10 | 100 | 15 |

**Cadherin-3**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 19.7 | 11.0 | nd | nd | 22.5 | 9.68 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Average | 30.4 | 18.7 | nd | nd | 32.5 | 21.0 |
| Stdev | 32.6 | 23.2 | nd | nd | 34.2 | 27.6 |
| p(t-test) |  | 0.13 | nd | nd |  | 0.23 |
| Min | 1.16 | 2.53 | nd | nd | 1.16 | 2.29 |
| Max | 180 | 110 | nd | nd | 180 | 110 |
| n (Samp) | 53 | 23 | nd | nd | 43 | 16 |
| n (Patient) | 53 | 23 | nd | nd | 43 | 16 |

|  | At Enrollment | | |
|---|---|---|---|
|  | sCr or UO | sCr only | UO only |
| AUC | 0.38 | nd | 0.36 |
| SE | 0.072 | nd | 0.084 |
| p | 0.11 | nd | 0.092 |
| nCohort 1 | 53 | nd | 43 |
| nCohort 2 | 23 | nd | 16 |
| Cutoff 1 | 6.69 | nd | 3.71 |
| Sens 1 | 74% | nd | 75% |
| Spec 1 | 23% | nd | 9% |
| Cutoff 2 | 3.97 | nd | 2.82 |
| Sens 2 | 83% | nd | 81% |
| Spec 2 | 11% | nd | 7% |
| Cutoff 3 | 2.82 | nd | 2.38 |
| Sens 3 | 91% | nd | 94% |
| Spec 3 | 8% | nd | 5% |
| Cutoff 4 | 38.3 | nd | 41.0 |
| Sens 4 | 9% | nd | 6% |
| Spec 4 | 72% | nd | 72% |
| Cutoff 5 | 53.6 | nd | 54.9 |
| Sens 5 | 4% | nd | 6% |
| Spec 5 | 81% | nd | 81% |
| Cutoff 6 | 72.1 | nd | 73.4 |
| Sens 6 | 4% | nd | 6% |
| Spec 6 | 91% | nd | 91% |
| OR Quart 2 | 2.5 | nd | 2.4 |
| p Value | 0.26 | nd | 0.37 |
| 95% CI of | 0.51 | nd | 0.36 |

(continued)

|  | At Enrollment | | |
|---|---|---|---|
|  | sCr or UO | sCr only | UO only |
| OR Quart2 | 12 | nd | 15 |
| OR Quart 3 | 3.1 | nd | 1.6 |
| p Value | 0.15 | nd | 0.63 |
| 95% CI of | 0.66 | nd | 0.23 |
| OR Quart3 | 15 | nd | 11 |
| OR Quart 4 | 3.1 | nd | 6.5 |
| p Value | 0.15 | nd | 0.044 |
| 95% CI of | 0.66 | nd | 1.1 |
| OR Quart4 | 15 | nd | 40 |

**Carcinoembryonic antigen-related cell adhesion molecule 1**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 7.27 | 10.8 | nd | nd | 7.27 | 12.5 |
| Average | 10.3 | 12.2 | nd | nd | 11.4 | 12.9 |
| Stdev | 11.8 | 9.10 | nd | nd | 12.7 | 7.12 |
| p(t-test) |  | 0.50 | nd | nd |  | 0.67 |
| Min | 1.09 | 0.879 | nd | nd | 1.09 | 1.42 |
| Max | 65.1 | 37.9 | nd | nd | 65.1 | 27.5 |
| n (Samp) | 53 | 23 | nd | nd | 43 | 16 |
| n (Patient) | 53 | 23 | nd | nd | 43 | 16 |

|  | At Enrollment | | |
|---|---|---|---|
|  | sCr or UO | sCr only | UO only |
| AUC | 0.61 | nd | 0.65 |
| SE | 0.072 | nd | 0.084 |
| p | 0.14 | nd | 0.072 |
| nCohort 1 | 53 | nd | 43 |
| nCohort 2 | 23 | nd | 16 |
| Cutoff 1 | 5.64 | nd | 8.32 |
| Sens 1 | 74% | nd | 75% |
| Spec 1 | 38% | nd | 56% |
| Cutoff 2 | 4.40 | nd | 7.88 |
| Sens 2 | 83% | nd | 81% |
| Spec 2 | 28% | nd | 56% |
| Cutoff 3 | 1.67 | nd | 1.67 |

(continued)

|  | At Enrollment | | |
|--|--|--|--|
|  | sCr or UO | sCr only | UO only |
| Sens 3 | 91% | nd | 94% |
| Spec 3 | 9% | nd | 5% |
| Cutoff 4 | 10.2 | nd | 11.2 |
| Sens 4 | 52% | nd | 56% |
| Spec 4 | 72% | nd | 72% |
| Cutoff 5 | 12.0 | nd | 16.6 |
| Sens 5 | 43% | nd | 25% |
| Spec 5 | 81% | nd | 81% |
| Cutoff 6 | 19.6 | nd | 27.0 |
| Sens 6 | 17% | nd | 6% |
| Spec 6 | 91% | nd | 91% |
| OR Quart 2 | 1.0 | nd | 1.5 |
| p Value | 1.0 | nd | 0.69 |
| 95% CI of | 0.21 | nd | 0.21 |
| OR Quart2 | 4.8 | nd | 11 |
| OR Quart 3 | 1.3 | nd | 3.0 |
| p Value | 0.70 | nd | 0.24 |
| 95% CI of | 0.30 | nd | 0.48 |
| OR Quart3 | 6.0 | nd | 19 |
| OR Quart 4 | 4.2 | nd | 4.0 |
| p Value | 0.049 | nd | 0.14 |
| 95% CI of | 1.0 | nd | 0.65 |
| OR Quart4 | 17 | nd | 25 |

**Platelet factor 4**

|  | sCr or UO | | sCr only | | UO only | |
|--|--|--|--|--|--|--|
|  | Cohort 1 | Cohort 2 | Cohort 1 | Cohort 2 | Cohort 1 | Cohort 2 |
| Median | 4.33 | 1.47 | nd | nd | 4.67 | 2.13 |
| Average | 5.89 | 3.97 | nd | nd | 6.02 | 2.46 |
| Stdev | 6.35 | 6.39 | nd | nd | 6.60 | 2.60 |
| p(t-test) |  | 0.23 | nd | nd |  | 0.041 |
| Min | 1.00E-9 | 1.00E-9 | nd | nd | 1.00E-9 | 1.00E-9 |
| Max | 28.4 | 25.8 | nd | nd | 28.4 | 9.60 |
| n (Samp) | 53 | 23 | nd | nd | 43 | 16 |
| n (Patient) | 53 | 23 | nd | nd | 43 | 16 |

|  | At Enrollment | | |
|---|---|---|---|
|  | sCr or UO | sCr only | UO only |
| AUC | 0.39 | nd | 0.34 |
| SE | 0.072 | nd | 0.084 |
| p | 0.12 | nd | 0.054 |
| nCohort 1 | 53 | nd | 43 |
| nCohort 2 | 23 | nd | 16 |
| Cutoff 1 | 0.228 | nd | 0.228 |
| Sens 1 | 74% | nd | 75% |
| Spec 1 | 21% | nd | 21% |
| Cutoff 2 | 0.134 | nd | 0.134 |
| Sens 2 | 83% | nd | 81% |
| Spec 2 | 19% | nd | 19% |
| Cutoff 3 | 0 | nd | 0 |
| Sens 3 | 100% | nd | 100% |
| Spec 3 | 0% | nd | 0% |
| Cutoff 4 | 7.31 | nd | 7.27 |
| Sens 4 | 17% | nd | 6% |
| Spec 4 | 72% | nd | 72% |
| Cutoff 5 | 11.3 | nd | 10.0 |
| Sens 5 | 13% | nd | 0% |
| Spec 5 | 81% | nd | 81% |
| Cutoff 6 | 14.2 | nd | 17.2 |
| Sens 6 | 13% | nd | 0% |
| Spec 6 | 91% | nd | 91% |
| OR Quart 2 | 0.70 | nd | 3.5 |
| p Value | 0.68 | nd | 0.30 |
| 95% CI of | 0.13 | nd | 0.32 |
| OR Quart2 | 3.7 | nd | 38 |
| OR Quart 3 | 2.7 | nd | 12 |
| p Value | 0.17 | nd | 0.030 |
| 95% CI of | 0.65 | nd | 1.3 |
| OR Quart3 | 11 | nd | 120 |
| OR Quart 4 | 2.7 | nd | 7.8 |
| p Value | 0.17 | nd | 0.081 |
| 95% CI of | 0.65 | nd | 0.78 |
| OR Quart4 | 11 | nd | 78 |

**Insulin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1.22 | 0.605 | nd | nd | 2.16 | 0.730 |
| Average | 17.7 | 53.7 | nd | nd | 18.1 | 9.10 |
| Stdev | 40.8 | 248 | nd | nd | 41.7 | 25.9 |
| p(t-test) | | 0.31 | nd | nd | | 0.42 |
| Min | 0.00344 | 0.116 | nd | nd | 0.0393 | 0.116 |
| Max | 192 | 1190 | nd | nd | 192 | 105 |
| n (Samp) | 52 | 23 | nd | nd | 43 | 16 |
| n (Patient) | 52 | 23 | nd | nd | 43 | 16 |

| | At Enrollment | | |
|---|---|---|---|
| | sCr or UO | sCr only | UO only |
| AUC | 0.39 | nd | 0.37 |
| SE | 0.072 | nd | 0.085 |
| p | 0.11 | nd | 0.14 |
| nCohort 1 | 52 | nd | 43 |
| nCohort 2 | 23 | nd | 16 |
| Cutoff 1 | 0.397 | nd | 0.397 |
| Sens 1 | 74% | nd | 75% |
| Spec 1 | 19% | nd | 16% |
| Cutoff 2 | 0.377 | nd | 0.335 |
| Sens 2 | 83% | nd | 81% |
| Spec 2 | 19% | nd | 14% |
| Cutoff 3 | 0.190 | nd | 0.116 |
| Sens 3 | 91% | nd | 94% |
| Spec 3 | 13% | nd | 7% |
| Cutoff 4 | 5.65 | nd | 7.99 |
| Sens 4 | 13% | nd | 19% |
| Spec 4 | 71% | nd | 72% |
| Cutoff 5 | 11.4 | nd | 15.8 |
| Sens 5 | 13% | nd | 6% |
| Spec 5 | 81% | nd | 81% |
| Cutoff 6 | 79.7 | nd | 43.7 |
| Sens 6 | 4% | nd | 6% |
| Spec 6 | 90% | nd | 91% |
| OR Quart 2 | 2.5 | nd | 1.0 |
| p Value | 0.26 | nd | 1.0 |

(continued)

|  | At Enrollment | | |
|---|---|---|---|
|  | sCr or UO | sCr only | UO only |
| 95% CI of OR Quart2 | 0.51 | nd | 0.17 |
|  | 12 | nd | 6.0 |
| OR Quart 3 | 3.1 | nd | 1.5 |
| p Value | 0.15 | nd | 0.67 |
| 95% CI of OR Quart3 | 0.66 | nd | 0.26 |
|  | 15 | nd | 8.0 |
| OR Quart 4 | 3.4 | nd | 3.0 |
| p Value | 0.12 | nd | 0.19 |
| 95% CI of OR Quart4 | 0.72 | nd | 0.58 |
|  | 16 | nd | 16 |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Angiopoietin-2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 8.99 | 13.5 | 8.99 | 13.5 | 8.99 | 10.5 |
| Average | 30.4 | 626 | 30.4 | 487 | 30.4 | 18.4 |
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Stdev | 135 | 2130 | 135 | 2040 | 135 | 19.0 |
| p(t-test) |  | 1.5E-4 |  | 0.0022 |  | 0.77 |
| Min | 1.00E-9 | 0.978 | 1.00E-9 | 0.461 | 1.00E-9 | 5.22 |
| Max | 1730 | 9360 | 1730 | 9360 | 1730 | 68.3 |
| n (Samp) | 191 | 21 | 191 | 21 | 191 | 11 |
| n (Patient) | 191 | 21 | 191 | 21 | 191 | 11 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 9.27 | 9.65 | 9.27 | 9.65 | 9.27 | 7.47 |
| Average | 60.0 | 330 | 60.0 | 63.5 | 60.0 | 10.8 |
| Stdev | 553 | 1030 | 553 | 148 | 553 | 14.1 |
| p(t-test) |  | 0.13 |  | 0.98 |  | 0.83 |
| Min | 1.00E-9 | 0.978 | 1.00E-9 | 0.461 | 1.00E-9 | 0.505 |
| Max | 9360 | 3430 | 9360 | 504 | 9360 | 38.4 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 296 | 11 | 296 | 11 | 296 | 6 |
| n (Patient) | 296 | 11 | 296 | 11 | 296 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8.99 | 14.7 | 8.99 | 14.7 | 8.99 | 13.5 |
| Average | 38.2 | 867 | 38.2 | 672 | 38.2 | 20.7 |
| Stdev | 159 | 2510 | 159 | 2410 | 159 | 20.4 |
| p(t-test) | | 1.6E-4 | | 0.0025 | | 0.74 |
| Min | 1.00E-9 | 1.15 | 1.00E-9 | 0.505 | 1.00E-9 | 5.22 |
| Max | 1730 | 9360 | 1730 | 9360 | 1730 | 68.3 |
| n (Samp) | 135 | 15 | 135 | 15 | 135 | 9 |
| n (Patient) | 135 | 15 | 135 | 15 | 135 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.52 | 0.59 | 0.58 | 0.52 | 0.58 | 0.62 | 0.39 | 0.64 |
| SE | 0.068 | 0.090 | 0.081 | 0.068 | 0.090 | 0.081 | 0.093 | 0.12 | 0.10 |
| p | 0.19 | 0.82 | 0.27 | 0.21 | 0.85 | 0.30 | 0.19 | 0.36 | 0.19 |
| nCohort 1 | 191 | 296 | 135 | 191 | 296 | 135 | 191 | 296 | 135 |
| nCohort 2 | 21 | 11 | 15 | 21 | 11 | 15 | 11 | 6 | 9 |
| Cutoff 1 | 6.53 | 5.22 | 6.53 | 6.53 | 5.22 | 6.53 | 9.51 | 0.644 | 9.51 |
| Sens 1 | 71% | 73% | 73% | 71% | 73% | 73% | 73% | 83% | 78% |
| Spec 1 | 43% | 35% | 45% | 43% | 35% | 45% | 54% | 2% | 56% |
| Cutoff 2 | 5.22 | 1.39 | 5.22 | 5.22 | 1.39 | 5.22 | 6.53 | 0.644 | 6.53 |
| Sens 2 | 81% | 82% | 80% | 81% | 82% | 80% | 82% | 83% | 89% |
| Spec 2 | 38% | 6% | 37% | 38% | 6% | 37% | 43% | 2% | 45% |
| Cutoff 3 | 1.39 | 1.00 | 1.39 | 0.644 | 1.00 | 0.644 | 5.22 | 0.0274 | 5.22 |
| Sens 3 | 90% | 91% | 93% | 90% | 91% | 93% | 91% | 100% | 100% |
| Spec 3 | 5% | 5% | 6% | 3% | 5% | 3% | 39% | 1% | 37% |
| Cutoff 4 | 16.6 | 17.3 | 18.1 | 16.6 | 17.3 | 18.1 | 16.6 | 17.3 | 18.1 |
| Sens 4 | 38% | 36% | 33% | 38% | 36% | 33% | 27% | 17% | 33% |
| Spec 4 | 70% | 70% | 70% | 70% | 70% | 70% | 70% | 70% | 70% |
| Cutoff 5 | 22.6 | 27.9 | 26.3 | 22.6 | 27.9 | 26.3 | 22.6 | 27.9 | 26.3 |
| Sens 5 | 29% | 36% | 27% | 29% | 36% | 27% | 18% | 17% | 22% |
| Spec 5 | 80% | 80% | 80% | 80% | 80% | 80% | 80% | 80% | 80% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 44.6 | 50.6 | 57.2 | 44.6 | 50.6 | 57.2 | 44.6 | 50.6 | 57.2 |
| Sens 6 | 24% | 27% | 20% | 24% | 27% | 20% | 9% | 0% | 11% |
| Spec 6 | 90% | 90% | 90% | 90% | 90% | 90% | 90% | 90% | 90% |
| OR Quart 2 | 1.0 | 0.65 | 0.63 | 1.0 | 0.65 | 0.63 | >3.1 | 2.1 | >2.1 |
| p Value | 1.0 | 0.64 | 0.62 | 1.0 | 0.64 | 0.62 | <0.33 | 0.56 | <0.55 |
| 95% CI of | 0.24 | 0.11 | 0.099 | 0.24 | 0.11 | 0.099 | >0.31 | 0.18 | >0.18 |
| OR Quart2 | 4.2 | 4.0 | 4.0 | 4.2 | 4.0 | 4.0 | na | 23 | na |
| OR Quart 3 | 1.6 | 0.65 | 2.2 | 1.6 | 0.65 | 2.2 | >5.6 | 1.0 | >5.8 |
| p Value | 0.51 | 0.64 | 0.29 | 0.51 | 0.64 | 0.29 | <0.12 | 1.0 | <0.12 |
| 95% CI of | 0.41 | 0.11 | 0.50 | 0.41 | 0.11 | 0.50 | >0.63 | 0.061 | >0.64 |
| OR Quart3 | 5.9 | 4.0 | 9.5 | 5.9 | 4.0 | 9.5 | na | 16 | na |
| OR Quart 4 | 1.9 | 1.3 | 1.3 | 1.9 | 1.3 | 1.3 | >3.1 | 2.1 | >2.1 |
| p Value | 0.35 | 0.71 | 0.72 | 0.35 | 0.71 | 0.72 | <0.33 | 0.56 | <0.55 |
| 95% CI of | 0.51 | 0.29 | 0.28 | 0.51 | 0.29 | 0.28 | >0.31 | 0.18 | >0.18 |
| OR Quart4 | 6.8 | 6.2 | 6.4 | 6.8 | 6.2 | 6.4 | na | 23 | na |

**Brain-derived neurotrophic factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.403 | 1.52 | 0.403 | 1.52 | 0.403 | 1.38 |
| Average | 5.25 | 67.1 | 5.25 | 66.7 | 5.25 | 2.01 |
| Stdev | 45.6 | 204 | 45.6 | 204 | 45.6 | 2.26 |
| p(t-test) | | 5.3E-4 | | 5.8E-4 | | 0.81 |
| Min | 1.00E-9 | 0.158 | 1.00E-9 | 0.158 | 1.00E-9 | 0.158 |
| Max | 624 | 732 | 624 | 732 | 624 | 8.33 |
| n (Samp) | 191 | 21 | 191 | 21 | 191 | 11 |
| n (Patient) | 191 | 21 | 191 | 21 | 191 | 11 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.695 | 2.82 | 0.695 | 1.45 | 0.695 | 2.14 |
| Average | 6.64 | 3.09 | 6.64 | 2.15 | 6.64 | 1.97 |
| Stdev | 51.5 | 2.49 | 51.5 | 2.08 | 51.5 | 1.22 |
| p(t-test) | | 0.82 | | 0.77 | | 0.82 |
| Min | 1.00E-9 | 0.243 | 1.00E-9 | 0.243 | 1.00E-9 | 0.421 |
| Max | 624 | 7.60 | 624 | 7.60 | 624 | 3.36 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 296 | 11 | 296 | 11 | 296 | 6 |
| n (Patient) | 296 | 11 | 296 | 11 | 296 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.547 | 2.82 | 0.547 | 2.82 | 0.547 | 1.38 |
| Average | 2.84 | 93.2 | 2.84 | 92.8 | 2.84 | 2.04 |
| Stdev | 9.37 | 238 | 9.37 | 238 | 9.37 | 2.47 |
| p(t-test) | | 1.4E-5 | | 1.5E-5 | | 0.80 |
| Min | 1.00E-9 | 0.158 | 1.00E-9 | 0.158 | 1.00E-9 | 0.158 |
| Max | 61.7 | 732 | 61.7 | 732 | 61.7 | 8.33 |
| n (Samp) | 135 | 15 | 135 | 15 | 135 | 9 |
| n (Patient) | 135 | 15 | 135 | 15 | 135 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.78 | 0.75 | 0.77 | 0.78 | 0.68 | 0.77 | 0.75 | 0.70 | 0.70 |
| SE | 0.061 | 0.086 | 0.073 | 0.062 | 0.090 | 0.074 | 0.087 | 0.12 | 0.100 |
| p | 3.4E-6 | 0.0038 | 2.0E-4 | 7.1E-6 | 0.042 | 3.0E-4 | 0.0038 | 0.096 | 0.041 |
| nCohort 1 | 191 | 296 | 135 | 191 | 296 | 135 | 191 | 296 | 135 |
| nCohort 2 | 21 | 11 | 15 | 21 | 11 | 15 | 11 | 6 | 9 |
| Cutoff 1 | 1.05 | 1.19 | 1.16 | 1.05 | 1.05 | 1.16 | 0.838 | 0.838 | 0.834 |
| Sens 1 | 71% | 73% | 73% | 71% | 73% | 73% | 82% | 83% | 78% |
| Spec 1 | 75% | 67% | 73% | 75% | 64% | 73% | 68% | 57% | 61% |
| Cutoff 2 | 0.608 | 1.05 | 0.608 | 0.608 | 0.838 | 0.608 | 0.838 | 0.838 | 0.608 |
| Sens 2 | 81% | 82% | 80% | 81% | 82% | 80% | 82% | 83% | 89% |
| Spec 2 | 61% | 64% | 53% | 61% | 57% | 53% | 68% | 57% | 53% |
| Cutoff 3 | 0.403 | 0.838 | 0.403 | 0.403 | 0.404 | 0.403 | 0.608 | 0.404 | 0.124 |
| Sens 3 | 90% | 91% | 93% | 90% | 91% | 93% | 91% | 100% | 100% |
| Spec 3 | 51% | 57% | 44% | 51% | 40% | 44% | 61% | 40% | 30% |
| Cutoff 4 | 0.907 | 1.36 | 1.05 | 0.907 | 1.36 | 1.05 | 0.907 | 1.36 | 1.05 |
| Sens 4 | 71% | 64% | 73% | 71% | 55% | 73% | 64% | 67% | 67% |
| Spec 4 | 70% | 70% | 70% | 70% | 70% | 70% | 70% | 70% | 70% |
| Cutoff 5 | 1.36 | 2.17 | 1.54 | 1.36 | 2.17 | 1.54 | 1.36 | 2.17 | 1.54 |
| Sens 5 | 57% | 55% | 53% | 57% | 36% | 53% | 55% | 50% | 22% |
| Spec 5 | 80% | 80% | 80% | 80% | 80% | 80% | 80% | 80% | 80% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 3.40 | 4.75 | 4.60 | 3.40 | 4.75 | 4.60 | 3.40 | 4.75 | 4.60 |
| Sens 6 | 33% | 27% | 33% | 29% | 9% | 27% | 9% | 0% | 11% |
| Spec 6 | 90% | 90% | 90% | 90% | 90% | 90% | 90% | 90% | 90% |
| OR Quart 2 | >3.2 | >1.0 | >3.2 | >3.2 | >2.0 | >3.2 | >1.0 | >1.0 | >1.0 |
| p Value | <0.32 | <1.0 | <0.33 | <0.32 | <0.57 | <0.33 | <1.0 | <1.0 | <0.98 |
| 95% CI of | >0.32 | >0.061 | >0.31 | >0.32 | >0.18 | >0.31 | >0.061 | >0.061 | >0.062 |
| OR Quart2 | na | na | na | na | na | na | na | na | na |
| OR Quart 3 | >6.8 | >4.2 | >3.3 | >6.8 | >5.3 | >3.3 | >3.2 | >2.1 | >4.5 |
| p Value | <0.082 | <0.21 | <0.32 | <0.082 | <0.13 | <0.32 | <0.32 | <0.56 | <0.19 |
| 95% CI of | >0.79 | >0.45 | >0.32 | >0.79 | >0.60 | >0.32 | >0.32 | >0.18 | >0.48 |
| OR Quart3 | na | na | na | na | na | na | na | na | na |
| OR Quart 4 | >16 | >6.4 | >11 | >16 | >4.2 | >11 | >8.0 | >3.1 | >4.5 |
| p Value | <0.0098 | <0.089 | <0.024 | <0.0098 | <0.21 | <0.024 | <0.057 | <0.33 | <0.19 |
| 95% CI of | >1.9 | >0.75 | >1.4 | >1.9 | >0.45 | >1.4 | >0.94 | >0.31 | >0.48 |
| OR Quart4 | na | na | na | na | na | na | na | na | na |

**Creatine Kinase-MB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0149 | 0.0128 | 0.0149 | 0.0113 | 0.0149 | 0.0107 |
| Average | 0.0205 | 0.0886 | 0.0205 | 0.0930 | 0.0205 | 0.00904 |
| Stdev | 0.0210 | 0.307 | 0.0210 | 0.317 | 0.0210 | 0.00640 |
| p(t-test) | | 0.025 | | 0.021 | | 0.13 |
| Min | 0.00168 | 0.00168 | 0.00168 | 0.00168 | 0.00168 | 0.00168 |
| Max | 0.105 | 1.24 | 0.105 | 1.24 | 0.105 | 0.0153 |
| n (Samp) | 103 | 16 | 103 | 15 | 103 | 8 |
| n (Patient) | 103 | 16 | 103 | 15 | 103 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0153 | 0.0106 | 0.0153 | 0.0101 | nd | nd |
| Average | 0.0301 | 0.00839 | 0.0301 | 0.00785 | nd | nd |
| Stdev | 0.0967 | 0.00582 | 0.0967 | 0.00537 | nd | nd |
| p(t-test) | | 0.53 | | 0.52 | nd | nd |
| Min | 0.00168 | 0.00168 | 0.00168 | 0.00168 | nd | nd |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 1.24 | 0.0153 | 1.24 | 0.0153 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0149 | 0.0153 | 0.0149 | 0.0153 | 0.0149 | 0.0133 |
| Average | 0.0215 | 0.138 | 0.0215 | 0.151 | 0.0215 | 0.0101 |
| Stdev | 0.0223 | 0.387 | 0.0223 | 0.408 | 0.0223 | 0.00670 |
| p(t-test) | | 0.0050 | | 0.0029 | | 0.22 |
| Min | 0.00168 | 0.00168 | 0.00168 | 0.00168 | 0.00168 | 0.00168 |
| Max | 0.105 | 1.24 | 0.105 | 1.24 | 0.105 | 0.0153 |
| n (Samp) | 87 | 10 | 87 | 9 | 87 | 6 |
| n (Patient) | 87 | 10 | 87 | 9 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.42 | 0.25 | 0.54 | 0.39 | 0.22 | 0.52 | 0.30 | nd | 0.34 |
| SE | 0.080 | 0.10 | 0.098 | 0.082 | 0.099 | 0.10 | 0.11 | nd | 0.13 |
| p | 0.33 | 0.013 | 0.72 | 0.18 | 0.0050 | 0.84 | 0.064 | nd | 0.20 |
| nCohort 1 | 103 | 170 | 87 | 103 | 170 | 87 | 103 | nd | 87 |
| nCohort 2 | 16 | 8 | 10 | 15 | 8 | 9 | 8 | nd | 6 |
| Cutoff 1 | 0.00942 | 0 | 0.0149 | 0.00942 | 0 | 0.0101 | 0 | nd | 0 |
| Sens 1 | 75% | 100% | 70% | 73% | 100% | 78% | 100% | nd | 100% |
| Spec 1 | 25% | 0% | 51% | 25% | 0% | 25% | 0% | nd | 0% |
| Cutoff 2 | 0 | 0 | 0.0101 | 0 | 0 | 0.00820 | 0 | nd | 0 |
| Sens 2 | 100% | 100% | 80% | 100% | 100% | 89% | 100% | nd | 100% |
| Spec 2 | 0% | 0% | 25% | 0% | 0% | 24% | 0% | nd | 0% |
| Cutoff 3 | 0 | 0 | 0.00820 | 0 | 0 | 0 | 0 | nd | 0 |
| Sens 3 | 100% | 100% | 90% | 100% | 100% | 100% | 100% | nd | 100% |
| Spec 3 | 0% | 0% | 24% | 0% | 0% | 0% | 0% | nd | 0% |
| Cutoff 4 | 0.0198 | 0.0206 | 0.0198 | 0.0198 | 0.0206 | 0.0198 | 0.0198 | nd | 0.0198 |
| Sens 4 | 12% | 0% | 20% | 13% | 0% | 22% | 0% | nd | 0% |
| Spec 4 | 71% | 70% | 70% | 71% | 70% | 70% | 71% | nd | 70% |
| Cutoff 5 | 0.0290 | 0.0308 | 0.0292 | 0.0290 | 0.0308 | 0.0292 | 0.0290 | nd | 0.0292 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 12% | 0% | 20% | 13% | 0% | 22% | 0% | nd | 0% |
| Spec 5 | 81% | 81% | 80% | 81% | 81% | 80% | 81% | nd | 80% |
| Cutoff 6 | 0.0435 | 0.0570 | 0.0570 | 0.0435 | 0.0570 | 0.0570 | 0.0435 | nd | 0.0570 |
| Sens 6 | 6% | 0% | 10% | 7% | 0% | 11% | 0% | nd | 0% |
| Spec 6 | 90% | 91% | 92% | 90% | 91% | 92% | 90% | nd | 92% |
| OR Quart 2 | 2.8 | >1.0 | 0.48 | 2.2 | >1.0 | 0.48 | >3.4 | nd | >3.6 |
| p Value | 0.24 | <0.97 | 0.56 | 0.37 | <0.97 | 0.56 | <0.31 | nd | <0.28 |
| 95% CI of | 0.50 | >0.063 | 0.040 | 0.38 | >0.063 | 0.040 | >0.33 | nd | >0.35 |
| OR Quart2 | 16 | na | 5.7 | 13 | na | 5.7 | na | nd | na |
| OR Quart 3 | 2.8 | >4.4 | 2.9 | 2.8 | >3.2 | 2.2 | >2.2 | nd | >1.1 |
| p Value | 0.24 | <0.19 | 0.23 | 0.24 | <0.32 | 0.39 | <0.54 | nd | <0.95 |
| 95% CI of | 0.50 | >0.47 | 0.50 | 0.50 | >0.32 | 0.36 | >0.18 | nd | >0.064 |
| OR Quart3 | 16 | na | 17 | 16 | na | 13 | na | nd | na |
| OR Quart 4 | 2.2 | >3.3 | 0.96 | 2.2 | >4.5 | 1.0 | >3.5 | nd | >2.3 |
| p Value | 0.37 | <0.31 | 0.97 | 0.37 | <0.19 | 1.0 | <0.29 | nd | <0.51 |
| 95% CI of | 0.38 | >0.33 | 0.12 | 0.38 | >0.48 | 0.13 | >0.34 | nd | >0.19 |
| OR Quart4 | 13 | na | 7.4 | 13 | na | 7.7 | na | nd | na |

**Immunoglobulin M**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000147 | 0.000752 | 0.000147 | 0.000592 | 0.000147 | 0.000558 |
| Average | 0.000816 | 0.00284 | 0.000816 | 0.00284 | 0.000816 | 0.00214 |
| Stdev | 0.00204 | 0.00526 | 0.00204 | 0.00547 | 0.00204 | 0.00437 |
| p(t-test) | | 0.0057 | | 0.0075 | | 0.12 |
| Min | 3.78E-8 | 1.38E-5 | 3.78E-8 | 1.38E-5 | 3.78E-8 | 0.000177 |
| Max | 0.0120 | 0.0186 | 0.0120 | 0.0186 | 0.0120 | 0.0129 |
| n (Samp) | 103 | 16 | 103 | 15 | 103 | 8 |
| n (Patient) | 103 | 16 | 103 | 15 | 103 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000172 | 0.000920 | 0.000172 | 0.000920 | nd | nd |
| Average | 0.000866 | 0.00276 | 0.000866 | 0.00268 | nd | nd |
| Stdev | 0.00233 | 0.00434 | 0.00233 | 0.00438 | nd | nd |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.033 | | 0.042 | nd | nd |
| Min | 3.78E-8 | 1.38E-5 | 3.78E-8 | 1.38E-5 | nd | nd |
| Max | 0.0186 | 0.0129 | 0.0186 | 0.0129 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000143 | 0.000696 | 0.000143 | 0.000592 | 0.000143 | 0.000530 |
| Average | 0.000678 | 0.00257 | 0.000678 | 0.00261 | 0.000678 | 0.000610 |
| Stdev | 0.00175 | 0.00566 | 0.00175 | 0.00601 | 0.00175 | 0.000441 |
| p(t-test) | | 0.021 | | 0.025 | | 0.92 |
| Min | 3.78E-8 | 0.000192 | 3.78E-8 | 0.000177 | 3.78E-8 | 0.000177 |
| Max | 0.0120 | 0.0186 | 0.0120 | 0.0186 | 0.0120 | 0.00138 |
| n (Samp) | 87 | 10 | 87 | 9 | 87 | 6 |
| n (Patient) | 87 | 10 | 87 | 9 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.76 | 0.78 | 0.73 | 0.71 | 0.75 | 0.77 | nd | 0.73 |
| SE | 0.073 | 0.10 | 0.090 | 0.078 | 0.10 | 0.097 | 0.10 | nd | 0.12 |
| p | 3.1E-4 | 0.0087 | 0.0020 | 0.0037 | 0.043 | 0.0084 | 0.0083 | nd | 0.059 |
| nCohort 1 | 103 | 170 | 87 | 103 | 170 | 87 | 103 | nd | 87 |
| nCohort 2 | 16 | 8 | 10 | 15 | 8 | 9 | 8 | nd | 6 |
| Cutoff 1 | 0.000447 | 0.000692 | 0.000447 | 0.000251 | 0.000320 | 0.000251 | 0.000447 | nd | 0.000223 |
| Sens 1 | 75% | 75% | 70% | 73% | 75% | 78% | 75% | nd | 83% |
| Spec 1 | 73% | 81% | 72% | 59% | 59% | 56% | 73% | nd | 55% |
| Cutoff 2 | 0.000273 | 0.000481 | 0.000273 | 0.000212 | 0.000222 | 0.000177 | 0.000223 | nd | 0.000223 |
| Sens 2 | 81% | 88% | 80% | 80% | 88% | 89% | 88% | nd | 83% |
| Spec 2 | 60% | 72% | 57% | 57% | 52% | 54% | 58% | nd | 55% |
| Cutoff 3 | 0.000191 | 1.31E-5 | 0.000192 | 0.000173 | 1.31E-5 | 0.000173 | 0.000173 | nd | 0.000173 |
| Sens 3 | 94% | 100% | 90% | 93% | 100% | 100% | 100% | nd | 100% |
| Spec 3 | 56% | 5% | 54% | 55% | 5% | 54% | 55% | nd | 54% |
| Cutoff 4 | 0.000441 | 0.000442 | 0.000441 | 0.000441 | 0.000442 | 0.000441 | 0.000441 | nd | 0.000441 |
| Sens 4 | 75% | 88% | 70% | 60% | 62% | 67% | 75% | nd | 67% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 4 | 71% | 70% | 70% | 71% | 70% | 70% | 71% | nd | 70% |
| Cutoff 5 | 0.000691 | 0.000691 | 0.000674 | 0.000691 | 0.000691 | 0.000674 | 0.000691 | nd | 0.000674 |
| Sens 5 | 56% | 75% | 50% | 47% | 62% | 44% | 38% | nd | 33% |
| Spec 5 | 81% | 80% | 80% | 81% | 80% | 80% | 81% | nd | 80% |
| Cutoff 6 | 0.00174 | 0.00150 | 0.00123 | 0.00174 | 0.00150 | 0.00123 | 0.00174 | nd | 0.00123 |
| Sens 6 | 19% | 25% | 40% | 20% | 25% | 22% | 12% | nd | 17% |
| Spec 6 | 90% | 90% | 91% | 90% | 90% | 91% | 90% | nd | 91% |
| OR Quart 2 | 0 | 0 | >1.0 | 0.97 | 0 | >1.0 | >0 | nd | >0 |
| p Value | na | na | <0.98 | 0.98 | na | <0.98 | <na | nd | <na |
| 95% CI of | na | na | >0.062 | 0.058 | na | >0.062 | >na | nd | >na |
| OR Quart2 | na | na | na | 16 | na | na | na | nd | na |
| OR Quart 3 | 7.0 | 1.0 | >3.4 | 7.3 | 2.0 | >3.4 | >4.5 | nd | >3.4 |
| p Value | 0.081 | 1.0 | <0.30 | 0.075 | 0.56 | <0.30 | <0.19 | nd | <0.30 |
| 95% CI of | 0.79 | 0.061 | >0.33 | 0.82 | 0.18 | >0.33 | >0.47 | nd | >0.33 |
| OR Quart3 | 62 | 17 | na | 65 | 23 | na | na | nd | na |
| OR Quart 4 | 12 | 6.6 | >7.6 | 8.5 | 5.4 | >6.3 | >4.5 | nd | >3.3 |
| p Value | 0.023 | 0.087 | <0.071 | 0.053 | 0.13 | <0.11 | <0.19 | nd | <0.32 |
| 95% CI of | 1.4 | 0.76 | >0.84 | 0.98 | 0.60 | >0.68 | >0.47 | nd | >0.32 |
| OR Quart4 | 100 | 57 | na | 74 | 48 | na | na | nd | na |

**Matrilysin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 11000 | 43100 | 11000 | 42700 | 11000 | 37800 |
| Average | 19600 | 83500 | 19600 | 73600 | 19600 | 34700 |
| Stdev | 27900 | 108000 | 27900 | 108000 | 27900 | 20000 |
| p(t-test) |  | 5.2E-10 |  | 1.0E-7 |  | 0.079 |
| Min | 1.00E-9 | 8300 | 1.00E-9 | 1840 | 1.00E-9 | 8300 |
| Max | 261000 | 408000 | 261000 | 406000 | 261000 | 72700 |
| n (Samp) | 191 | 21 | 191 | 21 | 191 | 11 |
| n (Patient) | 191 | 21 | 191 | 21 | 191 | 11 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 18300 | 48400 | 18300 | 37500 | 18300 | 36500 |
| Average | 27800 | 92500 | 27800 | 80300 | 27800 | 39800 |
| Stdev | 44500 | 114000 | 44500 | 114000 | 44500 | 19900 |
| p(t-test) | | 1.8E-5 | | 4.7E-4 | | 0.51 |
| Min | 1.00E-9 | 9150 | 1.00E-9 | 2040 | 1.00E-9 | 12600 |
| Max | 485000 | 408000 | 485000 | 406000 | 485000 | 72700 |
| n (Samp) | 297 | 11 | 297 | 11 | 297 | 6 |
| n (Patient) | 297 | 11 | 297 | 11 | 297 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13800 | 48400 | 13800 | 43100 | 13800 | 37800 |
| Average | 23500 | 96000 | 23500 | 86400 | 23500 | 30200 |
| Stdev | 33600 | 125000 | 33600 | 125000 | 33600 | 17300 |
| p(t-test) | | 3.5E-7 | | 8.6E-6 | | 0.56 |
| Min | 1.00E-9 | 8300 | 1.00E-9 | 1840 | 1.00E-9 | 8300 |
| Max | 261000 | 408000 | 261000 | 406000 | 261000 | 49700 |
| n (Samp) | 135 | 15 | 135 | 15 | 135 | 9 |
| n (Patient) | 135 | 15 | 135 | 15 | 135 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.79 | 0.79 | 0.78 | 0.75 | 0.75 | 0.76 | 0.75 | 0.69 |
| SE | 0.057 | 0.082 | 0.071 | 0.062 | 0.087 | 0.075 | 0.085 | 0.12 | 0.10 |
| D | 2.0E-8 | 3.5E-4 | 3.4E-5 | 7.1E-6 | 0.0047 | 7.5E-4 | 0.0020 | 0.034 | 0.056 |
| nCohort 1 | 191 | 297 | 135 | 191 | 297 | 135 | 191 | 297 | 135 |
| nCohort 2 | 21 | 11 | 15 | 21 | 11 | 15 | 11 | 6 | 9 |
| Cutoff 1 | 30100 | 36900 | 30100 | 30100 | 31800 | 30100 | 17100 | 31800 | 12600 |
| Sens 1 | 71% | 73% | 73% | 71% | 73% | 73% | 73% | 83% | 78% |
| Spec 1 | 82% | 78% | 77% | 82% | 72% | 77% | 61% | 72% | 48% |
| Cutoff 2 | 12600 | 28300 | 12600 | 12600 | 28300 | 12600 | 12600 | 31800 | 11200 |
| Sens 2 | 81% | 82% | 80% | 81% | 82% | 80% | 82% | 83% | 89% |
| Spec 2 | 52% | 69% | 48% | 52% | 69% | 48% | 52% | 72% | 47% |
| Cutoff 3 | 11200 | 12600 | 11200 | 8090 | 12600 | 8160 | 11200 | 12600 | 8160 |
| Sens 3 | 90% | 91% | 93% | 90% | 91% | 93% | 91% | 100% | 100% |
| Spec 3 | 51% | 40% | 47% | 40% | 40% | 37% | 51% | 40% | 37% |
| Cutoff 4 | 22400 | 29600 | 23600 | 22400 | 29600 | 23600 | 22400 | 29600 | 23600 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 4 | 76% | 73% | 73% | 76% | 73% | 73% | 64% | 83% | 56% |
| Spec 4 | 70% | 70% | 70% | 70% | 70% | 70% | 70% | 70% | 70% |
| Cutoff 5 | 27800 | 38200 | 35300 | 27800 | 38200 | 35300 | 27800 | 38200 | 35300 |
| Sens 5 | 76% | 55% | 67% | 76% | 45% | 67% | 64% | 33% | 56% |
| Spec 5 | 80% | 80% | 80% | 80% | 80% | 80% | 80% | 80% | 80% |
| Cutoff 6 | 41100 | 53600 | 50000 | 41100 | 53600 | 50000 | 41100 | 53600 | 50000 |
| Sens 6 | 57% | 45% | 40% | 52% | 36% | 27% | 45% | 17% | 0% |
| Spec 6 | 90% | 90% | 90% | 90% | 90% | 90% | 90% | 90% | 90% |
| OR Quart 2 | >5.5 | >2.1 | >4.4 | 1.5 | 1.0 | 3.1 | >2.0 | >1.0 | >3.3 |
| p Value | <0.12 | <0.56 | <0.20 | 0.65 | 1.0 | 0.34 | <0.57 | <1.0 | <0.31 |
| 95% CI of | >0.62 | >0.18 | >0.46 | 0.25 | 0.061 | 0.31 | >0.18 | >0.061 | >0.32 |
| OR Quart2 | na | na | na | 9.5 | 16 | 31 | na | na | na |
| OR Quart 3 | >0 | >1.0 | >1.0 | 0 | 2.0 | 1.0 | >2.1 | >1.0 | >1.0 |
| p Value | <na | <0.99 | <0.98 | na | 0.57 | 1.0 | <0.55 | <1.0 | <0.98 |
| 95% CI of | >na | >0.062 | >0.062 | na | 0.18 | 0.060 | >0.18 | >0.061 | >0.062 |
| OR Quart3 | na | na | na | na | 23 | 17 | na | na | na |
| OR Quart 4 | >23 | >8.9 | >13 | 11 | 7.6 | 13 | >8.0 | >4.2 | >5.8 |
| p Value | <0.0029 | <0.041 | <0.017 | 0.0021 | 0.061 | 0.018 | <0.057 | <0.21 | <0.12 |
| 95% CI of | >2.9 | >1.1 | >1.6 | 2.4 | 0.91 | 1.6 | >0.94 | >0.45 | >0.64 |
| OR Quart4 | na | na | na | 51 | 63 | 110 | na | na | na |

**Transforming growth factor beta-2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.72 | 35.0 | 1.72 | 25.1 | 1.72 | 26.6 |
| Average | 22.7 | 40.7 | 22.7 | 39.0 | 22.7 | 48.4 |
| Stdev | 47.1 | 38.3 | 47.1 | 38.7 | 47.1 | 47.3 |
| p(t-test) |  | 0.21 |  | 0.25 |  | 0.17 |
| Min | 1.00E-9 | 1.00E-9 | 1.00E-9 | 1.00E-9 | 1.00E-9 | 1.00E-9 |
| Max | 364 | 120 | 364 | 120 | 364 | 120 |
| n (Samp) | 99 | 12 | 99 | 12 | 99 | 7 |
| n (Patient) | 99 | 12 | 99 | 12 | 99 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.64 | 43.9 | 9.64 | 36.1 | nd | nd |
| Average | 29.1 | 46.7 | 29.1 | 43.5 | nd | nd |
| Stdev | 47.8 | 39.9 | 47.8 | 41.1 | nd | nd |
| p(t-test) | | 0.37 | | 0.47 | nd | nd |
| Min | 1.00E-9 | 1.00E-9 | 1.00E-9 | 1.00E-9 | nd | nd |
| Max | 364 | 120 | 364 | 120 | nd | nd |
| n (Samp) | 160 | 6 | 160 | 6 | nd | nd |
| n (Patient) | 160 | 6 | 160 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.62 | 33.4 | 3.62 | 33.4 | 3.62 | 33.6 |
| Average | 22.9 | 46.6 | 22.9 | 46.6 | 22.9 | 52.1 |
| Stdev | 48.6 | 44.8 | 48.6 | 44.8 | 48.6 | 50.7 |
| p(t-test) | | 0.19 | | 0.19 | | 0.16 |
| Min | 1.00E-9 | 1.00E-9 | 1.00E-9 | 1.00E-9 | 1.00E-9 | 1.00E-9 |
| Max | 364 | 120 | 364 | 120 | 364 | 120 |
| n (Samp) | 86 | 8 | 86 | 8 | 86 | 6 |
| n (Patient) | 86 | 8 | 86 | 8 | 86 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.70 | 0.73 | 0.70 | 0.67 | 0.73 | 0.73 | nd | 0.72 |
| SE | 0.087 | 0.12 | 0.10 | 0.088 | 0.12 | 0.10 | 0.11 | nd | 0.12 |
| p | 0.015 | 0.11 | 0.029 | 0.023 | 0.17 | 0.029 | 0.043 | nd | 0.070 |
| nCohort 1 | 99 | 160 | 86 | 99 | 160 | 86 | 99 | nd | 86 |
| nCohort 2 | 12 | 6 | 8 | 12 | 6 | 8 | 7 | nd | 6 |
| Cutoff 1 | 15.6 | 26.6 | 15.6 | 15.6 | 21.7 | 15.6 | 20.6 | nd | 15.6 |
| Sens 1 | 75% | 83% | 75% | 75% | 83% | 75% | 71% | nd | 83% |
| Spec 1 | 67% | 68% | 66% | 67% | 61% | 66% | 70% | nd | 66% |
| Cutoff 2 | 14.5 | 26.6 | 10.6 | 14.5 | 21.7 | 10.6 | 15.6 | nd | 15.6 |
| Sens 2 | 83% | 83% | 88% | 83% | 83% | 88% | 86% | nd | 83% |
| Spec 2 | 65% | 68% | 64% | 65% | 61% | 64% | 67% | nd | 66% |
| Cutoff 3 | 0 | 0 | 0 | 0 | 0 | 0 | 0 | nd | 0 |
| Sens 3 | 100% | 100% | 100% | 100% | 100% | 100% | 100% | nd | 100% |
| Spec 3 | 0% | 0% | 0% | 0% | 0% | 0% | 0% | nd | 0% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 4 | 23.1 | 29.7 | 23.1 | 23.1 | 29.7 | 23.1 | 23.1 | nd | 23.1 |
| Sens 4 | 58% | 67% | 50% | 50% | 50% | 50% | 57% | nd | 50% |
| Spec 4 | 71% | 70% | 71% | 71% | 70% | 71% | 71% | nd | 71% |
| Cutoff 5 | 37.1 | 45.5 | 36.1 | 37.1 | 45.5 | 36.1 | 37.1 | nd | 36.1 |
| Sens 5 | 50% | 33% | 50% | 42% | 33% | 50% | 43% | nd | 50% |
| Spec 5 | 81% | 80% | 80% | 81% | 80% | 80% | 81% | nd | 80% |
| Cutoff 6 | 65.6 | 94.0 | 58.4 | 65.6 | 94.0 | 58.4 | 65.6 | nd | 58.4 |
| Sens 6 | 17% | 17% | 25% | 17% | 17% | 25% | 29% | nd | 33% |
| Spec 6 | 91% | 90% | 91% | 91% | 90% | 91% | 91% | nd | 91% |
| OR Quart 2 | >2.1 | >1.0 | 0 | >2.1 | >1.0 | 0 | >1.0 | nd | >1.0 |
| p Value | <0.56 | <1.0 | na | <0.56 | <1.0 | na | <1.0 | nd | <0.98 |
| 95% CI of | >0.18 | >0.060 | na | >0.18 | >0.060 | na | >0.059 | nd | >0.062 |
| OR Quart2 | na | na | na | na | na | na | na | nd | na |
| OR Quart 3 | >4.5 | >1.0 | 3.3 | >5.9 | >2.1 | 3.3 | >3.4 | nd | >2.2 |
| p Value | <0.19 | <0.99 | 0.32 | <0.12 | <0.55 | 0.32 | <0.30 | nd | <0.53 |
| 95% CI of | >0.47 | >0.062 | 0.32 | >0.64 | >0.18 | 0.32 | >0.33 | nd | >0.18 |
| OR Quart3 | na | na | 34 | na | na | 34 | na | nd | na |
| OR Quart 4 | >7.4 | >4.3 | 4.4 | >5.9 | >3.2 | 4.4 | >3.2 | nd | >3.4 |
| p Value | <0.074 | <0.20 | 0.20 | <0.12 | <0.33 | 0.20 | <0.32 | nd | <0.30 |
| 95% CI of | >0.82 | >0.46 | 0.45 | >0.64 | >0.31 | 0.45 | >0.32 | nd | >0.33 |
| OR Quart4 | na | na | 43 | na | na | 43 | na | nd | na |

**Heparan Sulfate**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.02 | 6.93 | 3.02 | 6.93 | nd | nd |
| Average | 4.59 | 8.15 | 4.59 | 8.15 | nd | nd |
| Stdev | 3.97 | 5.42 | 3.97 | 5.42 | nd | nd |
| p(t-test) |  | 0.018 |  | 0.018 | nd | nd |
| Min | 0.121 | 0.970 | 0.121 | 0.970 | nd | nd |
| Max | 16.3 | 18.3 | 16.3 | 18.3 | nd | nd |
| n (Samp) | 69 | 9 | 69 | 9 | nd | nd |
| n (Patient) | 69 | 9 | 69 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.81 | 8.29 | 2.81 | 8.29 | nd | nd |
| Average | 4.26 | 8.62 | 4.26 | 8.62 | nd | nd |
| Stdev | 3.61 | 6.30 | 3.61 | 6.30 | nd | nd |
| p(t-test) | | 0.011 | | 0.011 | nd | nd |
| Min | 0.121 | 0.970 | 0.121 | 0.970 | nd | nd |
| Max | 14.5 | 18.3 | 14.5 | 18.3 | nd | nd |
| n (Samp) | 61 | 6 | 61 | 6 | nd | nd |
| n (Patient) | 61 | 6 | 61 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | nd | 0.73 | 0.71 | nd | 0.73 | nd | nd | nd |
| SE | 0.10 | nd | 0.12 | 0.10 | nd | 0.12 | nd | nd | nd |
| p | 0.037 | nd | 0.055 | 0.037 | nd | 0.055 | nd | nd | nd |
| nCohort 1 | 69 | nd | 61 | 69 | nd | 61 | nd | nd | nd |
| nCohort 2 | 9 | nd | 6 | 9 | nd | 6 | nd | nd | nd |
| Cutoff 1 | 4.06 | nd | 4.06 | 4.06 | nd | 4.06 | nd | nd | nd |
| Sens 1 | 78% | nd | 83% | 78% | nd | 83% | nd | nd | nd |
| Spec 1 | 61% | nd | 66% | 61% | nd | 66% | nd | nd | nd |
| Cutoff 2 | 3.02 | nd | 4.06 | 3.02 | nd | 4.06 | nd | nd | nd |
| Sens 2 | 89% | nd | 83% | 89% | nd | 83% | nd | nd | nd |
| Spec 2 | 51% | nd | 66% | 51% | nd | 66% | nd | nd | nd |
| Cutoff 3 | 0.953 | nd | 0.953 | 0.953 | nd | 0.953 | nd | nd | nd |
| Sens 3 | 100% | nd | 100% | 100% | nd | 100% | nd | nd | nd |
| Spec 3 | 9% | nd | 10% | 9% | nd | 10% | nd | nd | nd |
| Cutoff 4 | 4.88 | nd | 4.88 | 4.88 | nd | 4.88 | nd | nd | nd |
| Sens 4 | 67% | nd | 67% | 67% | nd | 67% | nd | nd | nd |
| Spec 4 | 71% | nd | 70% | 71% | nd | 70% | nd | nd | nd |
| Cutoff 5 | 7.80 | nd | 6.72 | 7.80 | nd | 6.72 | nd | nd | nd |
| Sens 5 | 44% | nd | 50% | 44% | nd | 50% | nd | nd | nd |
| Spec 5 | 81% | nd | 80% | 81% | nd | 80% | nd | nd | nd |
| Cutoff 6 | 11.4 | nd | 9.22 | 11.4 | nd | 9.22 | nd | nd | nd |
| Sens 6 | 22% | nd | 50% | 22% | nd | 50% | nd | nd | nd |
| Spec 6 | 91% | nd | 90% | 91% | nd | 90% | nd | nd | nd |
| OR Quart 2 | 0.95 | nd | 0 | 0.95 | nd | 0 | nd | nd | nd |
| p Value | 0.97 | nd | na | 0.97 | nd | na | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.055 | nd | na | 0.055 | nd | na | nd | nd | nd |
| OR Quart2 | 16 | nd | na | 16 | nd | na | nd | nd | nd |
| OR Quart 3 | 3.4 | nd | 2.0 | 3.4 | nd | 2.0 | nd | nd | nd |
| p Value | 0.31 | nd | 0.59 | 0.31 | nd | 0.59 | nd | nd | nd |
| 95% CI of | 0.32 | nd | 0.16 | 0.32 | nd | 0.16 | nd | nd | nd |
| OR Quart3 | 36 | nd | 24 | 36 | nd | 24 | nd | nd | nd |
| OR Quart 4 | 4.5 | nd | 3.2 | 4.5 | nd | 3.2 | nd | nd | nd |
| p Value | 0.20 | nd | 0.34 | 0.20 | nd | 0.34 | nd | nd | nd |
| 95% CI of | 0.45 | nd | 0.30 | 0.45 | nd | 0.30 | nd | nd | nd |
| OR Quart4 | 45 | nd | 35 | 45 | nd | 35 | nd | nd | nd |

**Transmembrane glycoprotein NMB**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.95 | 4.02 | 1.95 | 4.02 | 1.95 | 3.87 |
| Average | 2.05 | 5.77 | 2.05 | 5.76 | 2.05 | 3.76 |
| Stdev | 1.30 | 6.37 | 1.30 | 6.37 | 1.30 | 1.19 |
| p(t-test) |  | 9.2E-7 |  | 9.6E-7 |  | 9.9E-4 |
| Min | 0.203 | 1.66 | 0.203 | 1.66 | 0.203 | 2.13 |
| Max | 6.99 | 25.3 | 6.99 | 25.3 | 6.99 | 5.92 |
| n (Samp) | 102 | 12 | 102 | 12 | 102 | 7 |
| n (Patient) | 102 | 12 | 102 | 12 | 102 | 7 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.20 | 4.13 | 2.20 | 4.13 | nd | nd |
| Average | 2.44 | 4.26 | 2.44 | 4.25 | nd | nd |
| Stdev | 2.22 | 2.17 | 2.22 | 2.18 | nd | nd |
| p(t-test) |  | 0.051 |  | 0.052 | nd | nd |
| Min | 0.203 | 1.66 | 0.203 | 1.66 | nd | nd |
| Max | 25.3 | 7.41 | 25.3 | 7.41 | nd | nd |
| n (Samp) | 163 | 6 | 163 | 6 | nd | nd |
| n (Patient) | 163 | 6 | 163 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.82 | 4.40 | 1.82 | 4.40 | 1.82 | 3.75 |
| Average | 2.00 | 7.12 | 2.00 | 7.12 | 2.00 | 3.74 |
| Stdev | 1.26 | 7.53 | 1.26 | 7.53 | 1.26 | 1.31 |
| p(t-test) | | 9.7E-8 | | 9.7E-8 | | 0.0016 |
| Min | 0.203 | 2.13 | 0.203 | 2.13 | 0.203 | 2.13 |
| Max | 6.99 | 25.3 | 6.99 | 25.3 | 6.99 | 5.92 |
| n (Samp) | 87 | 8 | 87 | 8 | 87 | 6 |
| n (Patient) | 87 | 8 | 87 | 8 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.84 | 0.77 | 0.91 | 0.84 | 0.77 | 0.91 | 0.84 | nd | 0.85 |
| SE | 0.073 | 0.11 | 0.071 | 0.073 | 0.11 | 0.071 | 0.094 | nd | 0.100 |
| p | 2.2E-6 | 0.016 | 7.8E-9 | 3.7E-6 | 0.019 | 7.8E-9 | 2.5E-4 | nd | 4.0E-4 |
| nCohort 1 | 102 | 163 | 87 | 102 | 163 | 87 | 102 | nd | 87 |
| nCohort 2 | 12 | 6 | 8 | 12 | 6 | 8 | 7 | nd | 6 |
| Cutoff 1 | 3.35 | 2.28 | 3.87 | 3.35 | 2.22 | 3.87 | 3.46 | nd | 2.70 |
| Sens 1 | 75% | 83% | 75% | 75% | 83% | 75% | 71% | nd | 83% |
| Spec 1 | 87% | 55% | 91% | 87% | 52% | 91% | 88% | nd | 79% |
| Cutoff 2 | 2.28 | 2.28 | 3.35 | 2.21 | 2.22 | 3.35 | 2.60 | nd | 2.70 |
| Sens 2 | 83% | 83% | 88% | 83% | 83% | 88% | 86% | nd | 83% |
| Spec 2 | 67% | 55% | 89% | 62% | 52% | 89% | 76% | nd | 79% |
| Cutoff 3 | 2.11 | 1.60 | 2.11 | 2.11 | 1.60 | 2.11 | 2.11 | nd | 2.11 |
| Sens 3 | 92% | 100% | 100% | 92% | 100% | 100% | 100% | nd | 100% |
| Spec 3 | 60% | 36% | 61% | 60% | 36% | 61% | 60% | nd | 61% |
| Cutoff 4 | 2.36 | 2.88 | 2.32 | 2.36 | 2.88 | 2.32 | 2.36 | nd | 2.32 |
| Sens 4 | 75% | 67% | 88% | 75% | 67% | 88% | 86% | nd | 83% |
| Spec 4 | 71% | 71% | 70% | 71% | 71% | 70% | 71% | nd | 70% |
| Cutoff 5 | 2.88 | 3.34 | 2.80 | 2.88 | 3.34 | 2.80 | 2.88 | nd | 2.80 |
| Sens 5 | 75% | 67% | 88% | 75% | 67% | 88% | 71% | nd | 67% |
| Spec 5 | 80% | 80% | 80% | 80% | 80% | 80% | 80% | nd | 80% |
| Cutoff 6 | 4.07 | 4.26 | 3.87 | 4.07 | 4.26 | 3.87 | 4.07 | nd | 3.87 |
| Sens 6 | 50% | 50% | 75% | 50% | 50% | 75% | 29% | nd | 50% |
| Spec 6 | 90% | 90% | 91% | 90% | 90% | 91% | 90% | nd | 91% |
| OR Quart 2 | >1.0 | >1.0 | >0 | >1.0 | >1.0 | >0 | >0 | nd | >0 |
| p Value | <1.0 | <0.99 | <na | <1.0 | <0.99 | <na | <na | nd | <na |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | >0.060 | >0.062 | >na | >0.060 | >0.062 | >na | >na | nd | >na |
| OR Quart2 | na | na | na | na | na | na | na | nd | na |
| OR Quart 3 | >2.2 | >1.0 | >1.0 | >2.2 | >1.0 | >1.0 | >2.2 | nd | >1.0 |
| p Value | <0.54 | <0.99 | <1.0 | <0.54 | <0.99 | <1.0 | <0.54 | nd | <0.98 |
| 95% CI of | >0.18 | >0.062 | >0.059 | >0.18 | >0.062 | >0.059 | >0.18 | nd | >0.062 |
| OR Quart3 | na | na | na | na | na | na | na | nd | na |
| OR Quart 4 | >13 | >4.3 | >9.5 | >13 | >4.3 | >9.5 | >5.9 | nd | >6.1 |
| p Value | <0.021 | <0.20 | <0.044 | <0.021 | <0.20 | <0.044 | <0.12 | nd | <0.11 |
| 95% CI of | >1.5 | >0.46 | >1.1 | >1.5 | >0.46 | >1.1 | >0.64 | nd | >0.65 |
| OR Quart4 | na | na | na | na | na | na | na | nd | na |

**Cadherin-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 18.7 | 9.61 | 18.7 | 9.61 | 18.7 | 8.99 |
| Average | 28.3 | 18.4 | 28.3 | 18.4 | 28.3 | 15.1 |
| Stdev | 26.4 | 24.6 | 26.4 | 24.6 | 26.4 | 16.2 |
| p(t-test) | | 0.22 | | 0.22 | | 0.20 |
| Min | 0.690 | 1.03 | 0.690 | 1.03 | 0.690 | 2.37 |
| Max | 126 | 85.2 | 126 | 85.2 | 126 | 48.8 |
| n (Samp) | 102 | 12 | 102 | 12 | 102 | 7 |
| n (Patient) | 102 | 12 | 102 | 12 | 102 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.0 | 8.92 | 21.0 | 8.92 | nd | nd |
| Average | 32.8 | 6.71 | 32.8 | 6.71 | nd | nd |
| Stdev | 33.4 | 4.00 | 33.4 | 4.00 | nd | nd |
| p(t-test) | | 0.059 | | 0.059 | nd | nd |
| Min | 0.690 | 1.03 | 0.690 | 1.03 | nd | nd |
| Max | 212 | 9.80 | 212 | 9.80 | nd | nd |
| n (Samp) | 163 | 6 | 163 | 6 | nd | nd |
| n (Patient) | 163 | 6 | 163 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 16.9 | 11.4 | 16.9 | 11.4 | 16.9 | 11.1 |
| Average | 28.1 | 24.0 | 28.1 | 24.0 | 28.1 | 16.5 |
| Stdev | 26.8 | 28.9 | 26.8 | 28.9 | 26.8 | 17.3 |
| p(t-test) | | 0.68 | | 0.68 | | 0.30 |
| Min | 0.690 | 2.13 | 0.690 | 2.13 | 0.690 | 2.37 |
| Max | 126 | 85.2 | 126 | 85.2 | 126 | 48.8 |
| n (Samp) | 87 | 8 | 87 | 8 | 87 | 6 |
| n (Patient) | 87 | 8 | 87 | 8 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.36 | 0.19 | 0.43 | 0.36 | 0.19 | 0.43 | 0.33 | nd | 0.36 |
| SE | 0.090 | 0.11 | 0.11 | 0.090 | 0.11 | 0.11 | 0.12 | nd | 0.13 |
| p | 0.12 | 0.0044 | 0.50 | 0.12 | 0.0044 | 0.50 | 0.15 | nd | 0.25 |
| nCohort 1 | 102 | 163 | 87 | 102 | 163 | 87 | 102 | nd | 87 |
| nCohort 2 | 12 | 6 | 8 | 12 | 6 | 8 | 7 | nd | 6 |
| Cutoff 1 | 8.79 | 1.96 | 8.79 | 8.79 | 1.96 | 8.79 | 6.69 | nd | 3.34 |
| Sens 1 | 75% | 83% | 75% | 75% | 83% | 75% | 71% | nd | 83% |
| Spec 1 | 27% | 3% | 29% | 27% | 3% | 29% | 19% | nd | 3% |
| Cutoff 2 | 2.13 | 1.96 | 2.13 | 2.13 | 1.96 | 2.13 | 3.34 | nd | 3.34 |
| Sens 2 | 83% | 83% | 88% | 83% | 83% | 88% | 86% | nd | 83% |
| Spec 2 | 4% | 3% | 1% | 4% | 3% | 1% | 6% | nd | 3% |
| Cutoff 3 | 1.96 | 0.690 | 0.690 | 1.96 | 0.690 | 0.690 | 1.96 | nd | 0.690 |
| Sens 3 | 92% | 100% | 100% | 92% | 100% | 100% | 100% | nd | 100% |
| Spec 3 | 4% | 1% | 1% | 4% | 1% | 1% | 4% | nd | 1% |
| Cutoff 4 | 36.1 | 44.8 | 35.4 | 36.1 | 44.8 | 35.4 | 36.1 | nd | 35.4 |
| Sens 4 | 17% | 0% | 25% | 17% | 0% | 25% | 14% | nd | 17% |
| Spec 4 | 71% | 71% | 70% | 71% | 71% | 70% | 71% | nd | 70% |
| Cutoff 5 | 48.1 | 53.5 | 48.4 | 48.1 | 53.5 | 48.4 | 48.1 | nd | 48.4 |
| Sens 5 | 17% | 0% | 25% | 17% | 0% | 25% | 14% | nd | 17% |
| Spec 5 | 80% | 80% | 80% | 80% | 80% | 80% | 80% | nd | 80% |
| Cutoff 6 | 63.2 | 73.9 | 69.3 | 63.2 | 73.9 | 69.3 | 63.2 | nd | 69.3 |
| Sens 6 | 8% | 0% | 12% | 8% | 0% | 12% | 0% | nd | 0% |
| Spec 6 | 90% | 90% | 91% | 90% | 90% | 91% | 90% | nd | 91% |
| OR Quart 2 | 0.50 | >0 | 0.48 | 0.50 | >0 | 0.48 | 1.0 | nd | 1.0 |
| p Value | 0.58 | <na | 0.56 | 0.58 | <na | 0.56 | 0.98 | nd | 0.98 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.043 | >na | 0.040 | 0.043 | >na | 0.040 | 0.062 | nd | 0.062 |
| OR Quart2 | 5.8 | na | 5.7 | 5.8 | na | 5.7 | 17 | nd | 18 |
| OR Quart 3 | 3.5 | >4.5 | 1.6 | 3.5 | >4.5 | 1.6 | 2.2 | nd | 2.2 |
| p Value | 0.15 | <0.19 | 0.64 | 0.15 | <0.19 | 0.64 | 0.54 | nd | 0.53 |
| 95% CI of | 0.65 | >0.48 | 0.24 | 0.65 | >0.48 | 0.24 | 0.18 | nd | 0.18 |
| OR Quart3 | 19 | na | 10 | 19 | na | 10 | 25 | nd | 26 |
| OR Quart 4 | 1.6 | >2.1 | 1.0 | 1.6 | >2.1 | 1.0 | 3.4 | nd | 2.2 |
| p Value | 0.61 | <0.54 | 0.96 | 0.61 | <0.54 | 0.96 | 0.31 | nd | 0.53 |
| 95% CI of | 0.25 | >0.19 | 0.13 | 0.25 | >0.19 | 0.13 | 0.33 | nd | 0.18 |
| OR Quart4 | 11 | na | 8.1 | 11 | na | 8.1 | 35 | nd | 26 |

**Stromelysin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.181 | 0.0268 | 0.181 | 0.0268 | 0.181 | 0.0348 |
| Average | 1.28 | 0.680 | 1.28 | 0.680 | 1.28 | 0.0790 |
| Stdev | 9.21 | 2.08 | 9.21 | 2.08 | 9.21 | 0.0900 |
| p(t-test) | | 0.82 | | 0.82 | | 0.73 |
| Min | 1.00E-9 | 1.00E-9 | 1.00E-9 | 1.00E-9 | 1.00E-9 | 1.00E-9 |
| Max | 93.1 | 7.26 | 93.1 | 7.26 | 93.1 | 0.231 |
| n (Samp) | 102 | 12 | 102 | 12 | 102 | 7 |
| n (Patient) | 102 | 12 | 102 | 12 | 102 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.185 | 0.00938 | 0.185 | 0.00938 | nd | nd |
| Average | 1.09 | 0.0935 | 1.09 | 0.0935 | nd | nd |
| Stdev | 7.36 | 0.140 | 7.36 | 0.140 | nd | nd |
| p(t-test) | | 0.74 | | 0.74 | nd | nd |
| Min | 1.00E-9 | 1.00E-9 | 1.00E-9 | 1.00E-9 | nd | nd |
| Max | 93.1 | 0.311 | 93.1 | 0.311 | nd | nd |
| n (Samp) | 162 | 6 | 162 | 6 | nd | nd |
| n (Patient) | 162 | 6 | 162 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.174 | 0.0718 | 0.174 | 0.0718 | 0.174 | 0.0718 |
| Average | 1.44 | 0.980 | 1.44 | 0.980 | 1.44 | 0.0890 |
| Stdev | 9.97 | 2.54 | 9.97 | 2.54 | 9.97 | 0.0942 |
| p(t-test) | | 0.90 | | 0.90 | | 0.74 |
| Min | 1.00E-9 | 1.00E-9 | 1.00E-9 | 1.00E-9 | 1.00E-9 | 1.00E-9 |
| Max | 93.1 | 7.26 | 93.1 | 7.26 | 93.1 | 0.231 |
| n (Samp) | 87 | 8 | 87 | 8 | 87 | 6 |
| n (Patient) | 87 | 8 | 87 | 8 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | 0.26 | 0.36 | 0.32 | 0.26 | 0.36 | 0.27 | nd | 0.30 |
| SE | 0.089 | 0.12 | 0.11 | 0.089 | 0.12 | 0.11 | 0.11 | nd | 0.12 |
| p | 0.038 | 0.046 | 0.22 | 0.038 | 0.046 | 0.22 | 0.038 | nd | 0.098 |
| nCohort 1 | 102 | 162 | 87 | 102 | 162 | 87 | 102 | nd | 87 |
| nCohort 2 | 12 | 6 | 8 | 12 | 6 | 8 | 7 | nd | 6 |
| Cutoff 1 | 0 | 0 | 0 | 0 | 0 | 0 | 0.0134 | nd | 0 |
| Sens 1 | 100% | 100% | 100% | 100% | 100% | 100% | 71% | nd | 100% |
| Spec 1 | 0% | 0% | 0% | 0% | 0% | 0% | 17% | nd | 0% |
| Cutoff 2 | 0 | 0 | 0 | 0 | 0 | 0 | 0 | nd | 0 |
| Sens 2 | 100% | 100% | 100% | 100% | 100% | 100% | 100% | nd | 100% |
| Spec 2 | 0% | 0% | 0% | 0% | 0% | 0% | 0% | nd | 0% |
| Cutoff 3 | 0 | 0 | 0 | 0 | 0 | 0 | 0 | nd | 0 |
| Sens 3 | 100% | 100% | 100% | 100% | 100% | 100% | 100% | nd | 100% |
| Spec 3 | 0% | 0% | 0% | 0% | 0% | 0% | 0% | nd | 0% |
| Cutoff 4 | 0.348 | 0.363 | 0.345 | 0.348 | 0.363 | 0.345 | 0.348 | nd | 0.345 |
| Sens 4 | 8% | 0% | 12% | 8% | 0% | 12% | 0% | nd | 0% |
| Spec 4 | 71% | 70% | 70% | 71% | 70% | 70% | 71% | nd | 70% |
| Cutoff 5 | 0.499 | 0.564 | 0.546 | 0.499 | 0.564 | 0.546 | 0.499 | nd | 0.546 |
| Sens 5 | 8% | 0% | 12% | 8% | 0% | 12% | 0% | nd | 0% |
| Spec 5 | 80% | 80% | 80% | 80% | 80% | 80% | 80% | nd | 80% |
| Cutoff 6 | 0.729 | 1.01 | 0.813 | 0.729 | 1.01 | 0.813 | 0.729 | nd | 0.813 |
| Sens 6 | 8% | 0% | 12% | 8% | 0% | 12% | 0% | nd | 0% |
| Spec 6 | 90% | 90% | 91% | 90% | 90% | 91% | 90% | nd | 91% |
| OR Quart 2 | 3.4 | >2.1 | 2.1 | 3.4 | >2.1 | 2.1 | >1.1 | nd | >1.1 |
| p Value | 0.31 | <0.55 | 0.56 | 0.31 | <0.55 | 0.56 | <0.96 | nd | <0.95 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.33 | >0.18 | 0.18 | 0.33 | >0.18 | 0.18 | >0.064 | nd | >0.064 |
| OR Quart2 | 34 | na | 25 | 34 | na | 25 | na | nd | na |
| OR Quart 3 | 1.0 | >0 | 1.0 | 1.0 | >0 | 1.0 | >2.2 | nd | >2.3 |
| p Value | 1.0 | <na | 1.0 | 1.0 | <na | 1.0 | <0.52 | nd | <0.51 |
| 95% CI of | 0.060 | >na | 0.059 | 0.060 | >na | 0.059 | >0.19 | nd | >0.19 |
| OR Quart3 | 17 | na | 17 | 17 | na | 17 | na | nd | na |
| OR Quart 4 | 9.3 | >4.4 | 4.8 | 9.3 | >4.4 | 4.8 | >4.9 | nd | >3.6 |
| p Value | 0.044 | <0.19 | 0.17 | 0.044 | <0.19 | 0.17 | <0.17 | nd | <0.28 |
| 95% CI of | 1.1 | >0.47 | 0.50 | 1.1 | >0.47 | 0.50 | >0.51 | nd | >0.35 |
| OR Quart4 | 82 | na | 47 | 82 | na | 47 | na | nd | na |

**Cathepsin S**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.11 | 5.95 | 2.11 | 5.95 | nd | nd |
| Average | 3.30 | 8.61 | 3.30 | 8.61 | nd | nd |
| Stdev | 5.43 | 12.6 | 5.43 | 12.6 | nd | nd |
| p(t-test) |  | 0.044 |  | 0.044 | nd | nd |
| Min | 1.00E-9 | 1.12 | 1.00E-9 | 1.12 | nd | nd |
| Max | 37.1 | 39.3 | 37.1 | 39.3 | nd | nd |
| n (Samp) | 50 | 8 | 50 | 8 | nd | nd |
| n (Patient) | 50 | 8 | 50 | 8 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.72 | nd | nd | 0.72 | nd | nd | nd | nd | nd |
| SE | 0.11 | nd | nd | 0.11 | nd | nd | nd | nd | nd |
| p | 0.041 | nd | nd | 0.041 | nd | nd | nd | nd | nd |
| nCohort 1 | 50 | nd | nd | 50 | nd | nd | nd | nd | nd |
| nCohort 2 | 8 | nd | nd | 8 | nd | nd | nd | nd | nd |
| Cutoff 1 | 2.31 | nd | nd | 2.31 | nd | nd | nd | nd | nd |
| Sens 1 | 75% | nd | nd | 75% | nd | nd | nd | nd | nd |
| Spec 1 | 60% | nd | nd | 60% | nd | nd | nd | nd | nd |
| Cutoff 2 | 1.52 | nd | nd | 1.52 | nd | nd | nd | nd | nd |
| Sens 2 | 88% | nd | nd | 88% | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 44% | nd | nd | 44% | nd | nd | nd | nd | nd |
| Cutoff 3 | 1.08 | nd | nd | 1.08 | nd | nd | nd | nd | nd |
| Sens 3 | 100% | nd | nd | 100% | nd | nd | nd | nd | nd |
| Spec 3 | 36% | nd | nd | 36% | nd | nd | nd | nd | nd |
| Cutoff 4 | 3.10 | nd | nd | 3.10 | nd | nd | nd | nd | nd |
| Sens 4 | 62% | nd | nd | 62% | nd | nd | nd | nd | nd |
| Spec 4 | 70% | nd | nd | 70% | nd | nd | nd | nd | nd |
| Cutoff 5 | 4.16 | nd | nd | 4.16 | nd | nd | nd | nd | nd |
| Sens 5 | 62% | nd | nd | 62% | nd | nd | nd | nd | nd |
| Spec 5 | 80% | nd | nd | 80% | nd | nd | nd | nd | nd |
| Cutoff 6 | 6.61 | nd | nd | 6.61 | nd | nd | nd | nd | nd |
| Sens 6 | 12% | nd | nd | 12% | nd | nd | nd | nd | nd |
| Spec 6 | 90% | nd | nd | 90% | nd | nd | nd | nd | nd |
| OR Quart 2 | >2.2 | nd | nd | >2.2 | nd | nd | nd | nd | nd |
| p Value | <0.55 | nd | nd | <0.55 | nd | nd | nd | nd | nd |
| 95% CI of | >0.17 | nd | nd | >0.17 | nd | nd | nd | nd | nd |
| OR Quart2 | na | nd | nd | na | nd | nd | nd | nd | nd |
| OR Quart 3 | >1.1 | nd | nd | >1.1 | nd | nd | nd | nd | nd |
| p Value | <0.96 | nd | nd | <0.96 | nd | nd | nd | nd | nd |
| 95% CI of | >0.061 | nd | nd | >0.061 | nd | nd | nd | nd | nd |
| OR Quart3 | na | nd | nd | na | nd | nd | nd | nd | nd |
| OR Quart 4 | >7.0 | nd | nd | >7.0 | nd | nd | nd | nd | nd |
| p Value | <0.097 | nd | nd | <0.097 | nd | nd | nd | nd | nd |
| 95% CI of | >0.71 | nd | nd | >0.71 | nd | nd | nd | nd | nd |
| OR Quart4 | na | nd | nd | na | nd | nd | nd | nd | nd |

**Urokinase-type plasminogen activator**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 19.9 | 3.85 | 19.9 | 3.85 | nd | nd |
| Average | 28.4 | 12.7 | 28.4 | 12.7 | nd | nd |
| Stdev | 27.7 | 26.5 | 27.7 | 26.5 | nd | nd |
| p(t-test) | | 0.14 | | 0.14 | nd | nd |
| Min | 0.308 | 1.24 | 0.308 | 1.24 | nd | nd |
| Max | 119 | 78.3 | 119 | 78.3 | nd | nd |
| n (Samp) | 50 | 8 | 50 | 8 | nd | nd |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 50 | 8 | 50 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.24 | nd | nd | 0.24 | nd | nd | nd | nd | nd |
| SE | 0.10 | nd | nd | 0.10 | nd | nd | nd | nd | nd |
| p | 0.012 | nd | nd | 0.012 | nd | nd | nd | nd | nd |
| nCohort 1 | 50 | nd | nd | 50 | nd | nd | nd | nd | nd |
| nCohort 2 | 8 | nd | nd | 8 | nd | nd | nd | nd | nd |
| Cutoff 1 | 1.97 | nd | nd | 1.97 | nd | nd | nd | nd | nd |
| Sens 1 | 75% | nd | nd | 75% | nd | nd | nd | nd | nd |
| Spec 1 | 10% | nd | nd | 10% | nd | nd | nd | nd | nd |
| Cutoff 2 | 1.82 | nd | nd | 1.82 | nd | nd | nd | nd | nd |
| Sens 2 | 88% | nd | nd | 88% | nd | nd | nd | nd | nd |
| Spec 2 | 10% | nd | nd | 10% | nd | nd | nd | nd | nd |
| Cutoff 3 | 0.670 | nd | nd | 0.670 | nd | nd | nd | nd | nd |
| Sens 3 | 100% | nd | nd | 100% | nd | nd | nd | nd | nd |
| Spec 3 | 6% | nd | nd | 6% | nd | nd | nd | nd | nd |
| Cutoff 4 | 26.6 | nd | nd | 26.6 | nd | nd | nd | nd | nd |
| Sens 4 | 12% | nd | nd | 12% | nd | nd | nd | nd | nd |
| Spec 4 | 70% | nd | nd | 70% | nd | nd | nd | nd | nd |
| Cutoff 5 | 48.9 | nd | nd | 48.9 | nd | nd | nd | nd | nd |
| Sens 5 | 12% | nd | nd | 12% | nd | nd | nd | nd | nd |
| Spec 5 | 80% | nd | nd | 80% | nd | nd | nd | nd | nd |
| Cutoff 6 | 64.4 | nd | nd | 64.4 | nd | nd | nd | nd | nd |
| Sens 6 | 12% | nd | nd | 12% | nd | nd | nd | nd | nd |
| Spec 6 | 90% | nd | nd | 90% | nd | nd | nd | nd | nd |
| OR Quart 2 | 0 | nd | nd | 0 | nd | nd | nd | nd | nd |
| p Value | na | nd | nd | na | nd | nd | nd | nd | nd |
| 95% CI of | na | nd | nd | na | nd | nd | nd | nd | nd |
| OR Quart2 | na | nd | nd | na | nd | nd | nd | nd | nd |
| OR Quart 3 | 3.5 | nd | nd | 3.5 | nd | nd | nd | nd | nd |
| p Value | 0.30 | nd | nd | 0.30 | nd | nd | nd | nd | nd |
| 95% CI of | 0.32 | nd | nd | 0.32 | nd | nd | nd | nd | nd |
| OR Quart3 | 38 | nd | nd | 38 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | 5.6 | nd | nd | 5.6 | nd | nd | nd | nd | nd |
| p Value | 0.15 | nd | nd | 0.15 | nd | nd | nd | nd | nd |
| 95% CI of | 0.54 | nd | nd | 0.54 | nd | nd | nd | nd | nd |
| OR Quart4 | 58 | nd | nd | 58 | nd | nd | nd | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00300 | 0.0293 | 0.00300 | 0.0293 | nd | nd |
| Average | 0.279 | 0.257 | 0.279 | 0.257 | nd | nd |
| Stdev | 1.93 | 0.634 | 1.93 | 0.634 | nd | nd |
| p(t-test) | | 0.97 | | 0.97 | nd | nd |
| Min | 1.00E-9 | 0.000202 | 1.00E-9 | 0.000202 | nd | nd |
| Max | 13.7 | 1.82 | 13.7 | 1.82 | nd | nd |
| n (Samp) | 50 | 8 | 50 | 8 | nd | nd |
| n (Patient) | 50 | 8 | 50 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | nd | nd | 0.76 | nd | nd | nd | nd | nd |
| SE | 0.10 | nd | nd | 0.10 | nd | nd | nd | nd | nd |
| p | 0.011 | nd | nd | 0.011 | nd | nd | nd | nd | nd |
| nCohort 1 | 50 | nd | nd | 50 | nd | nd | nd | nd | nd |
| nCohort 2 | 8 | nd | nd | 8 | nd | nd | nd | nd | nd |
| Cutoff 1 | 0.00654 | nd | nd | 0.00654 | nd | nd | nd | nd | nd |
| Sens 1 | 75% | nd | nd | 75% | nd | nd | nd | nd | nd |
| Spec 1 | 72% | nd | nd | 72% | nd | nd | nd | nd | nd |
| Cutoff 2 | 0.00261 | nd | nd | 0.00261 | nd | nd | nd | nd | nd |
| Sens 2 | 88% | nd | nd | 88% | nd | nd | nd | nd | nd |
| Spec 2 | 50% | nd | nd | 50% | nd | nd | nd | nd | nd |
| Cutoff 3 | 0.000158 | nd | nd | 0.000158 | nd | nd | nd | nd | nd |
| Sens 3 | 100% | nd | nd | 100% | nd | nd | nd | nd | nd |
| Spec 3 | 16% | nd | nd | 16% | nd | nd | nd | nd | nd |
| Cutoff 4 | 0.00646 | nd | nd | 0.00646 | nd | nd | nd | nd | nd |
| Sens 4 | 75% | nd | nd | 75% | nd | nd | nd | nd | nd |
| Spec 4 | 70% | nd | nd | 70% | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | 0.0104 | nd | nd | 0.0104 | nd | nd | nd | nd | nd |
| Sens 5 | 62% | nd | nd | 62% | nd | nd | nd | nd | nd |
| Spec 5 | 80% | nd | nd | 80% | nd | nd | nd | nd | nd |
| Cutoff 6 | 0.0166 | nd | nd | 0.0166 | nd | nd | nd | nd | nd |
| Sens 6 | 50% | nd | nd | 50% | nd | nd | nd | nd | nd |
| Spec 6 | 90% | nd | nd | 90% | nd | nd | nd | nd | nd |
| OR Quart 2 | 0.93 | nd | nd | 0.93 | nd | nd | nd | nd | nd |
| p Value | 0.96 | nd | nd | 0.96 | nd | nd | nd | nd | nd |
| 95% CI of | 0.053 | nd | nd | 0.053 | nd | nd | nd | nd | nd |
| OR Quart2 | 16 | nd | nd | 16 | nd | nd | nd | nd | nd |
| OR Quart 3 | 1.0 | nd | nd | 1.0 | nd | nd | nd | nd | nd |
| p Value | 1.0 | nd | nd | 1.0 | nd | nd | nd | nd | nd |
| 95% CI of | 0.056 | nd | nd | 0.056 | nd | nd | nd | nd | nd |
| OR Quart3 | 18 | nd | nd | 18 | nd | nd | nd | nd | nd |
| OR Quart 4 | 6.5 | nd | nd | 6.5 | nd | nd | nd | nd | nd |
| p Value | 0.11 | nd | nd | 0.11 | nd | nd | nd | nd | nd |
| 95% CI of | 0.65 | nd | nd | 0.65 | nd | nd | nd | nd | nd |
| OR Quart4 | 65 | nd | nd | 65 | nd | nd | nd | nd | nd |

**Complement C5a**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 3.94 | 1.00E-9 | 3.94 | 1.00E-9 | 2.15 |
| Average | 2.00 | 5.34 | 2.00 | 5.34 | 2.00 | 3.01 |
| Stdev | 5.91 | 5.42 | 5.91 | 5.42 | 5.91 | 3.41 |
| p(t-test) | | 0.089 | | 0.089 | | 0.66 |
| Min | 1.00E-9 | 1.00E-9 | 1.00E-9 | 1.00E-9 | 1.00E-9 | 1.00E-9 |
| Max | 50.8 | 13.7 | 50.8 | 13.7 | 50.8 | 9.29 |
| n (Samp) | 102 | 10 | 102 | 10 | 102 | 7 |
| n (Patient) | 102 | 10 | 102 | 10 | 102 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 2.96 | 1.00E-9 | 2.96 | 1.00E-9 | 1.37 |
| Average | 2.73 | 2.55 | 2.73 | 2.55 | 2.73 | 1.97 |
| Stdev | 6.74 | 2.22 | 6.74 | 2.22 | 6.74 | 2.19 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.95 | | 0.95 | | 0.79 |
| Min | 1.00E-9 | 1.00E-9 | 1.00E-9 | 1.00E-9 | 1.00E-9 | 1.00E-9 |
| Max | 50.8 | 5.29 | 50.8 | 5.29 | 50.8 | 5.29 |
| n (Samp) | 87 | 6 | 87 | 6 | 87 | 6 |
| n (Patient) | 87 | 6 | 87 | 6 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | nd | 0.63 | 0.72 | nd | 0.63 | 0.68 | nd | 0.59 |
| SE | 0.095 | nd | 0.13 | 0.095 | nd | 0.13 | 0.11 | nd | 0.13 |
| p | 0.021 | nd | 0.32 | 0.021 | nd | 0.32 | 0.11 | nd | 0.47 |
| nCohort 1 | 102 | nd | 87 | 102 | nd | 87 | 102 | nd | 87 |
| nCohort 2 | 10 | nd | 6 | 10 | nd | 6 | 7 | nd | 6 |
| Cutoff 1 | 1.98 | nd | 0 | 1.98 | nd | 0 | 0.573 | nd | 0 |
| Sens 1 | 70% | nd | 100% | 70% | nd | 100% | 71% | nd | 100% |
| Spec 1 | 81% | nd | 0% | 81% | nd | 0% | 68% | nd | 0% |
| Cutoff 2 | 0 | nd | 0 | 0 | nd | 0 | 0 | nd | 0 |
| Sens 2 | 100% | nd | 100% | 100% | nd | 100% | 100% | nd | 100% |
| Spec 2 | 0% | nd | 0% | 0% | nd | 0% | 0% | nd | 0% |
| Cutoff 3 | 0 | nd | 0 | 0 | nd | 0 | 0 | nd | 0 |
| Sens 3 | 100% | nd | 100% | 100% | nd | 100% | 100% | nd | 100% |
| Spec 3 | 0% | nd | 0% | 0% | nd | 0% | 0% | nd | 0% |
| Cutoff 4 | 0.791 | nd | 1.42 | 0.791 | nd | 1.42 | 0.791 | nd | 1.42 |
| Sens 4 | 70% | nd | 67% | 70% | nd | 67% | 57% | nd | 50% |
| Spec 4 | 71% | nd | 70% | 71% | nd | 70% | 71% | nd | 70% |
| Cutoff 5 | 1.77 | nd | 3.13 | 1.77 | nd | 3.13 | 1.77 | nd | 3.13 |
| Sens 5 | 70% | nd | 50% | 70% | nd | 50% | 57% | nd | 33% |
| Spec 5 | 80% | nd | 80% | 80% | nd | 80% | 80% | nd | 80% |
| Cutoff 6 | 6.22 | nd | 7.79 | 6.22 | nd | 7.79 | 6.22 | nd | 7.79 |
| Sens 6 | 30% | nd | 0% | 30% | nd | 0% | 14% | nd | 0% |
| Spec 6 | 90% | nd | 91% | 90% | nd | 91% | 90% | nd | 91% |
| OR Quart 2 | 0 | nd | >2.2 | 0 | nd | >2.2 | 0 | nd | >2.2 |
| p Value | na | nd | <0.53 | na | nd | <0.53 | na | nd | <0.53 |
| 95% CI of | na | nd | >0.18 | na | nd | >0.18 | na | nd | >0.18 |
| OR Quart2 | na | nd | na | na | nd | na | na | nd | na |
| OR Quart 3 | 0 | nd | >1.0 | 0 | nd | >1.0 | 0.48 | nd | >1.0 |

(continued)

|  | 0hr prior to AKI stage | | | 24hr prior to AKI stage | | | 48hr prior to AKI stage | | |
| --- | --- | --- | --- | --- | --- | --- | --- | --- | --- |
|  | sCr or UO | sCr only | UO only | sCr or UO | sCr only | UO only | sCr or UO | sCr only | UO only |
| p Value | na | nd | <0.98 | na | nd | <0.98 | 0.56 | nd | <0.98 |
| 95% CI of | na | nd | >0.062 | na | nd | >0.062 | 0.041 | nd | >0.062 |
| OR Quart3 | na | nd | na | na | nd | na | 5.6 | nd | na |
| OR Quart 4 | 2.8 | nd | >3.3 | 2.8 | nd | >3.3 | 2.1 | nd | >3.3 |
| p Value | 0.17 | nd | <0.32 | 0.17 | nd | <0.32 | 0.42 | nd | <0.32 |
| 95% CI of | 0.64 | nd | >0.32 | 0.64 | nd | >0.32 | 0.35 | nd | >0.32 |
| OR Quart4 | 12 | nd | na | 12 | nd | na | 12 | nd | na |

**Carcinoembryonic antigen-related cell adhesion molecule 1**

| sCr or UO | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 6.67 | 16.1 | 6.67 | 16.1 | 6.67 | 14.2 |
| Average | 8.84 | 27.8 | 8.84 | 27.8 | 8.84 | 16.1 |
| Stdev | 7.13 | 42.2 | 7.13 | 42.2 | 7.13 | 13.5 |
| p(t-test) |  | 5.7E-5 |  | 5.7E-5 |  | 0.016 |
| Min | 0.605 | 3.63 | 0.605 | 3.63 | 0.605 | 3.63 |
| Max | 32.3 | 155 | 32.3 | 155 | 32.3 | 41.9 |
| n (Samp) | 102 | 12 | 102 | 12 | 102 | 7 |
| n (Patient) | 102 | 12 | 102 | 12 | 102 | 7 |

| sCr only | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 7.88 | 9.56 | 7.88 | 9.56 | nd | nd |
| Average | 11.8 | 13.9 | 11.8 | 13.9 | nd | nd |
| Stdev | 15.7 | 13.3 | 15.7 | 13.3 | nd | nd |
| p(t-test) |  | 0.75 |  | 0.75 | nd | nd |
| Min | 0.605 | 3.63 | 0.605 | 3.63 | nd | nd |
| Max | 155 | 38.0 | 155 | 38.0 | nd | nd |
| n (Samp) | 163 | 6 | 163 | 6 | nd | nd |
| n (Patient) | 163 | 6 | 163 | 6 | nd | nd |

| UO only | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 6.77 | 20.7 | 6.77 | 20.7 | 6.77 | 16.1 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 8.24 | 37.9 | 8.24 | 37.9 | 8.24 | 18.2 |
| Stdev | 6.64 | 49.4 | 6.64 | 49.4 | 6.64 | 13.5 |
| p(t-test) | | 6.2E-7 | | 6.2E-7 | | 0.0014 |
| Min | 0.605 | 5.68 | 0.605 | 5.68 | 0.605 | 5.68 |
| Max | 31.5 | 155 | 31.5 | 155 | 31.5 | 41.9 |
| n (Samp) | 87 | 8 | 87 | 8 | 87 | 6 |
| n (Patient) | 87 | 8 | 87 | 8 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.54 | 0.83 | 0.69 | 0.54 | 0.83 | 0.68 | nd | 0.77 |
| SE | 0.088 | 0.12 | 0.092 | 0.088 | 0.12 | 0.092 | 0.11 | nd | 0.12 |
| p | 0.030 | 0.73 | 3.5E-4 | 0.030 | 0.73 | 3.5E-4 | 0.11 | nd | 0.019 |
| nCohort 1 | 102 | 163 | 87 | 102 | 163 | 87 | 102 | nd | 87 |
| nCohort 2 | 12 | 6 | 8 | 12 | 6 | 8 | 7 | nd | 6 |
| Cutoff 1 | 5.66 | 3.94 | 13.8 | 5.66 | 3.94 | 13.8 | 6.05 | nd | 6.05 |
| Sens 1 | 75% | 83% | 75% | 75% | 83% | 75% | 71% | nd | 83% |
| Spec 1 | 42% | 20% | 90% | 42% | 20% | 90% | 46% | nd | 45% |
| Cutoff 2 | 4.82 | 3.94 | 6.05 | 4.82 | 3.94 | 6.05 | 5.66 | nd | 6.05 |
| Sens 2 | 83% | 83% | 88% | 83% | 83% | 88% | 86% | nd | 83% |
| Spec 2 | 36% | 20% | 45% | 36% | 20% | 45% | 42% | nd | 45% |
| Cutoff 3 | 3.94 | 3.31 | 5.66 | 3.94 | 3.31 | 5.66 | 3.31 | nd | 5.66 |
| Sens 3 | 92% | 100% | 100% | 92% | 100% | 100% | 100% | nd | 100% |
| Spec 3 | 25% | 15% | 41% | 25% | 15% | 41% | 21% | nd | 41% |
| Cutoff 4 | 10.2 | 12.1 | 9.54 | 10.2 | 12.1 | 9.54 | 10.2 | nd | 9.54 |
| Sens 4 | 58% | 50% | 75% | 58% | 50% | 75% | 57% | nd | 67% |
| Spec 4 | 71% | 71% | 70% | 71% | 71% | 70% | 71% | nd | 70% |
| Cutoff 5 | 13.5 | 15.6 | 12.0 | 13.5 | 15.6 | 12.0 | 13.5 | nd | 12.0 |
| Sens 5 | 58% | 33% | 75% | 58% | 33% | 75% | 57% | nd | 67% |
| Spec 5 | 80% | 80% | 80% | 80% | 80% | 80% | 80% | nd | 80% |
| Cutoff 6 | 17.8 | 23.1 | 15.8 | 17.8 | 23.1 | 15.8 | 17.8 | nd | 15.8 |
| Sens 6 | 50% | 17% | 62% | 50% | 17% | 62% | 43% | nd | 50% |
| Spec 6 | 90% | 90% | 91% | 90% | 90% | 91% | 90% | nd | 91% |
| OR Quart 2 | 1.5 | 0.49 | >2.1 | 1.5 | 0.49 | >2.1 | 2.1 | nd | >2.2 |
| p Value | 0.67 | 0.56 | <0.56 | 0.67 | 0.56 | <0.56 | 0.56 | nd | <0.53 |
| 95% CI of | 0.23 | 0.043 | >0.18 | 0.23 | 0.043 | >0.18 | 0.18 | nd | >0.18 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 9.7 | 5.6 | na | 9.7 | 5.6 | na | 24 | nd | na |
| OR Quart 3 | 0 | 0 | >0 | 0 | 0 | >0 | 0 | nd | >0 |
| p Value | na | na | <na | na | na | <na | na | nd | <na |
| 95% CI of | na | na | >na | na | na | >na | na | nd | >na |
| OR Quart3 | na | na | na | na | na | na | na | nd | na |
| OR Quart 4 | 4.1 | 1.5 | >7.7 | 4.1 | 1.5 | >7.7 | 4.3 | nd | >4.6 |
| p Value | 0.096 | 0.67 | <0.070 | 0.096 | 0.67 | <0.070 | 0.20 | nd | <0.19 |
| 95% CI of | 0.78 | 0.24 | >0.85 | 0.78 | 0.24 | >0.85 | 0.45 | nd | >0.47 |
| OR Quart4 | 22 | 9.5 | na | 22 | 9.5 | na | 42 | nd | na |

**Isulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.55 | 1.34 | 1.55 | 1.44 | 1.55 | 1.12 |
| Average | 12.0 | 98.5 | 12.0 | 102 | 12.0 | 38.5 |
| Stdev | 30.7 | 299 | 30.7 | 309 | 30.7 | 97.8 |
| p(t-test) |  | 0.0044 |  | 0.0041 |  | 0.065 |
| Min | 0.00344 | 0.175 | 0.00344 | 0.175 | 0.00344 | 0.0393 |
| Max | 228 | 1190 | 228 | 1190 | 228 | 280 |
| n (Samp) | 103 | 16 | 103 | 15 | 103 | 8 |
| n (Patient) | 103 | 16 | 103 | 15 | 103 | 8 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.82 | 12.0 | 1.82 | 12.0 | nd | nd |
| Average | 33.2 | 196 | 33.2 | 190 | nd | nd |
| Stdev | 161 | 413 | 161 | 415 | nd | nd |
| p(t-test) |  | 0.012 |  | 0.016 | nd | nd |
| Min | 0.00344 | 1.12 | 0.00344 | 1.12 | nd | nd |
| Max | 1750 | 1190 | 1750 | 1190 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.08 | 1.12 | 2.08 | 1.05 | 2.08 | 1.04 |
| Average | 13.3 | 1.45 | 13.3 | 1.49 | 13.3 | 1.51 |
| Stdev | 31.9 | 1.34 | 31.9 | 1.40 | 31.9 | 1.78 |
| p(t-test) | | 0.24 | | 0.27 | | 0.37 |
| Min | 0.00344 | 0.175 | 0.00344 | 0.175 | 0.00344 | 0.0393 |
| Max | 214 | 5.04 | 214 | 5.04 | 214 | 5.04 |
| n (Samp) | 87 | 10 | 87 | 9 | 87 | 6 |
| n (Patient) | 87 | 10 | 87 | 9 | 87 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.71 | 0.32 | 0.53 | 0.70 | 0.33 | 0.48 | nd | 0.30 |
| SE | 0.079 | 0.11 | 0.097 | 0.081 | 0.11 | 0.10 | 0.11 | nd | 0.12 |
| p | 0.75 | 0.047 | 0.062 | 0.69 | 0.063 | 0.092 | 0.86 | nd | 0.11 |
| nCohort 1 | 103 | 170 | 87 | 103 | 170 | 87 | 103 | nd | 87 |
| nCohort 2 | 16 | 8 | 10 | 15 | 8 | 9 | 8 | nd | 6 |
| Cutoff 1 | 1.02 | 1.88 | 0.994 | 1.02 | 1.88 | 0.892 | 0.994 | nd | 0.687 |
| Sens 1 | 75% | 75% | 70% | 73% | 75% | 78% | 75% | nd | 83% |
| Spec 1 | 35% | 51% | 30% | 35% | 51% | 28% | 34% | nd | 17% |
| Cutoff 2 | 0.994 | 1.22 | 0.743 | 0.994 | 1.22 | 0.743 | 0.687 | nd | 0.687 |
| Sens 2 | 81% | 88% | 80% | 80% | 88% | 89% | 88% | nd | 83% |
| Spec 2 | 34% | 40% | 20% | 34% | 40% | 20% | 21% | nd | 17% |
| Cutoff 3 | 0.710 | 1.07 | 0.687 | 0.743 | 1.07 | 0.0381 | 0.0381 | nd | 0.0381 |
| Sens 3 | 94% | 100% | 90% | 93% | 100% | 100% | 100% | nd | 100% |
| Spec 3 | 22% | 36% | 17% | 23% | 36% | 3% | 4% | nd | 3% |
| Cutoff 4 | 5.48 | 6.99 | 8.45 | 5.48 | 6.99 | 8.45 | 5.48 | nd | 8.45 |
| Sends 4 | 25% | 50% | 0% | 27% | 50% | 0% | 25% | nd | 0% |
| Spec 4 | 71% | 70% | 70% | 71% | 70% | 70% | 71% | nd | 70% |
| Cutoff 5 | 13.9 | 13.8 | 17.8 | 13.9 | 13.8 | 17.8 | 13.9 | nd | 17.8 |
| Sens 5 | 25% | 50% | 0% | 27% | 50% | 0% | 25% | nd | 0% |
| Spec 5 | 81% | 80% | 80% | 81% | 80% | 80% | 81% | nd | 80% |
| Cutoff 6 | 33.7 | 38.4 | 35.9 | 33.7 | 38.4 | 35.9 | 33.7 | nd | 35.9 |
| Sens 6 | 19% | 38% | 0% | 13% | 25% | 0% | 12% | nd | 0% |
| Spec 6 | 90% | 90% | 91% | 90% | 90% | 91% | 90% | nd | 91% |
| OR Quart 2 | 2.2 | >2.0 | >1.1 | 3.4 | >2.0 | >1.0 | 0.48 | nd | >1.1 |
| p Value | 0.31 | <0.56 | <0.95 | 0.16 | <0.56 | <0.98 | 0.56 | nd | <0.95 |
| 95% CI of | 0.49 | >0.18 | >0.064 | 0.62 | >0.18 | >0.062 | 0.041 | nd | >0.064 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 9.6 | na | na | 18 | na | na | 5.6 | nd | na |
| OR Quart 3 | 0.96 | >2.1 | >8.3 | 1.6 | >2.1 | >8.0 | 1.6 | nd | >3.6 |
| p Value | 0.97 | <0.55 | <0.059 | 0.64 | <0.55 | <0.064 | 0.64 | nd | <0.28 |
| 95% CI of | 0.18 | >0.18 | >0.92 | 0.24 | >0.18 | >0.88 | 0.24 | nd | >0.35 |
| OR Quart3 | 5.2 | na | na | 10 | na | na | 10 | nd | na |
| OR Quart 4 | 1.3 | >4.3 | >3.6 | 2.1 | >4.3 | >2.2 | 1.0 | nd | >2.3 |
| p Value | 0.72 | <0.20 | <0.29 | 0.42 | <0.20 | <0.54 | 0.97 | nd | <0.51 |
| 95% CI of | 0.27 | >0.46 | >0.35 | 0.35 | >0.46 | >0.18 | 0.14 | nd | >0.19 |
| OR Quart4 | 6.6 | na | na | 12 | na | na | 8.0 | nd | na |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Brain-derived neurotrophic factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1940 | 2150 | 1940 | 1590 | 1940 | 1890 |
| Average | 2580 | 2730 | 2580 | 3430 | 2580 | 2670 |
| Stdev | 2290 | 2840 | 2290 | 5740 | 2290 | 2160 |
| p(t-test) | | 0.76 | | 0.24 | | 0.85 |
| Min | 24.3 | 1.00E-9 | 24.3 | 16.9 | 24.3 | 116 |
| Max | 10700 | 13100 | 10700 | 33100 | 10700 | 7960 |
| n (Samp) | 82 | 37 | 82 | 45 | 82 | 26 |
| n (Patient) | 76 | 37 | 76 | 45 | 76 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1690 | 2520 | 1690 | 3370 | 1690 | 2020 |
| Average | 2530 | 2740 | 2530 | 5620 | 2530 | 2840 |
| Stdev | 2710 | 2630 | 2710 | 8440 | 2710 | 2410 |
| p(t-test) | | 0.80 | | 0.0011 | | 0.74 |
| Min | 1.00E-9 | 302 | 1.00E-9 | 283 | 1.00E-9 | 591 |
| Max | 19100 | 9730 | 19100 | 33100 | 19100 | 7960 |
| n (Samp) | 196 | 12 | 196 | 14 | 196 | 9 |
| n (Patient) | 131 | 12 | 131 | 14 | 131 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1680 | 1640 | 1680 | 1560 | 1680 | 1910 |
| Average | 2100 | 2540 | 2100 | 2660 | 2100 | 3170 |
| Stdev | 1630 | 2820 | 1630 | 3590 | 1630 | 2740 |
| p(t-test) | | 0.32 | | 0.25 | | 0.026 |
| Min | 24.3 | 1.00E-9 | 24.3 | 16.9 | 24.3 | 116 |
| Max | 7310 | 13100 | 7310 | 19100 | 7310 | 9730 |
| n (Samp) | 76 | 29 | 76 | 43 | 76 | 21 |
| n (Patient) | 64 | 29 | 64 | 43 | 64 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.54 | 0.49 | 0.48 | 0.66 | 0.48 | 0.52 | 0.57 | 0.59 |
| SE | 0.058 | 0.088 | 0.063 | 0.054 | 0.082 | 0.055 | 0.066 | 0.10 | 0.072 |
| p | 0.75 | 0.66 | 0.87 | 0.64 | 0.058 | 0.67 | 0.75 | 0.48 | 0.21 |
| nCohort 1 | 82 | 196 | 76 | 82 | 196 | 76 | 82 | 196 | 76 |
| nCohort 2 | 37 | 12 | 29 | 45 | 14 | 43 | 26 | 9 | 21 |
| Cutoff 1 | 880 | 956 | 671 | 763 | 1710 | 789 | 1010 | 1040 | 1220 |
| Sens 1 | 70% | 75% | 72% | 71% | 71% | 72% | 73% | 78% | 71% |
| Spec 1 | 24% | 34% | 20% | 23% | 52% | 24% | 27% | 36% | 38% |
| Cutoff 2 | 542 | 880 | 329 | 632 | 1170 | 599 | 748 | 806 | 682 |
| Sens 2 | 81% | 83% | 83% | 80% | 86% | 81% | 81% | 89% | 81% |
| Spec 2 | 17% | 32% | 11% | 21% | 37% | 17% | 23% | 29% | 21% |
| Cutoff 3 | 259 | 542 | 118 | 296 | 657 | 296 | 542 | 542 | 542 |
| Sens 3 | 92% | 92% | 93% | 91% | 93% | 91% | 92% | 100% | 90% |
| Spec 3 | 7% | 20% | 4% | 9% | 23% | 9% | 17% | 20% | 16% |
| Cutoff 4 | 3080 | 3060 | 2950 | 3080 | 3060 | 2950 | 3080 | 3060 | 2950 |
| Sens 4 | 30% | 25% | 28% | 31% | 50% | 26% | 38% | 44% | 43% |
| Spec 4 | 71% | 70% | 71% | 71% | 70% | 71% | 71% | 70% | 71% |
| Cutoff 5 | 3590 | 3760 | 3170 | 3590 | 3760 | 3170 | 3590 | 3760 | 3170 |
| Sens 5 | 22% | 17% | 28% | 29% | 36% | 21% | 31% | 33% | 43% |
| Spec 5 | 80% | 80% | 80% | 80% | 80% | 80% | 80% | 80% | 80% |
| Cutoff 6 | 5550 | 5710 | 4240 | 5550 | 5710 | 4240 | 5550 | 5710 | 4240 |
| Sens 6 | 14% | 8% | 21% | 13% | 21% | 19% | 12% | 11% | 33% |
| Spec 6 | 90% | 90% | 91% | 90% | 90% | 91% | 90% | 90% | 91% |
| OR Quart 2 | 0.86 | 1.5 | 0.71 | 0.49 | 0.98 | 1.2 | 1.0 | 3.1 | 0.27 |
| p Value | 0.78 | 0.65 | 0.59 | 0.19 | 0.98 | 0.79 | 1.0 | 0.33 | 0.14 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.29 | 0.25 | 0.21 | 0.17 | 0.13 | 0.40 | 0.30 | 0.31 | 0.049 |
| OR Quart2 | 2.5 | 9.6 | 2.4 | 1.4 | 7.2 | 3.3 | 3.4 | 31 | 1.5 |
| OR Quart 3 | 0.73 | 2.7 | 0.71 | 0.88 | 1.5 | 1.0 | 0.36 | 1.0 | 0.60 |
| p Value | 0.57 | 0.26 | 0.59 | 0.80 | 0.65 | 1.0 | 0.17 | 1.0 | 0.48 |
| 95% CI of | 0.24 | 0.49 | 0.21 | 0.32 | 0.25 | 0.34 | 0.082 | 0.061 | 0.15 |
| OR Quart3 | 2.2 | 14 | 2.4 | 2.4 | 9.6 | 2.9 | 1.6 | 16 | 2.5 |
| OR Quart 4 | 1.1 | 1.0 | 1.3 | 0.92 | 3.8 | 1.4 | 1.4 | 4.2 | 1.7 |
| p Value | 0.93 | 1.0 | 0.70 | 0.88 | 0.11 | 0.52 | 0.55 | 0.21 | 0.41 |
| 95% CI of | 0.36 | 0.14 | 0.40 | 0.34 | 0.75 | 0.49 | 0.44 | 0.45 | 0.49 |
| OR Quart4 | 3.1 | 7.4 | 4.0 | 2.5 | 19 | 4.1 | 4.6 | 39 | 5.8 |

**Creatine Kinase-MB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.239 | 0.297 | 0.239 | 0.295 | 0.239 | 0.200 |
| Average | 0.645 | 0.513 | 0.645 | 0.628 | 0.645 | 0.470 |
| Stdev | 2.27 | 0.886 | 2.27 | 0.731 | 2.27 | 0.527 |
| p(t-test) | | 0.68 | | 0.95 | | 0.70 |
| Min | 0.00420 | 0.00420 | 0.00420 | 0.0278 | 0.00420 | 0.00420 |
| Max | 32.5 | 6.14 | 32.5 | 3.05 | 32.5 | 2.13 |
| n (Samp) | 263 | 51 | 263 | 56 | 263 | 26 |
| n (Patient) | 111 | 51 | 111 | 56 | 111 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.260 | 0.308 | 0.260 | 0.383 | 0.260 | 0.482 |
| Average | 0.638 | 0.805 | 0.638 | 0.755 | 0.638 | 0.636 |
| Stdev | 1.82 | 1.99 | 1.82 | 1.40 | 1.82 | 0.535 |
| p(t-test) | | 0.70 | | 0.77 | | 1.00 |
| Min | 0.00420 | 0.00420 | 0.00420 | 0.0278 | 0.00420 | 0.00420 |
| Max | 32.5 | 8.69 | 32.5 | 6.59 | 32.5 | 1.68 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.244 | 0.329 | 0.244 | 0.295 | 0.244 | 0.208 |
| Average | 0.780 | 0.562 | 0.780 | 0.663 | 0.780 | 0.456 |
| Stdev | 2.50 | 0.910 | 2.50 | 0.755 | 2.50 | 0.500 |
| p(t-test) | | 0.54 | | 0.74 | | 0.54 |
| Min | 0.00420 | 0.00420 | 0.00420 | 0.0597 | 0.00420 | 0.0721 |
| Max | 32.5 | 6.14 | 32.5 | 3.05 | 32.5 | 2.13 |
| n (Samp) | 221 | 50 | 221 | 52 | 221 | 23 |
| n (Patient) | 91 | 50 | 91 | 52 | 91 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.53 | 0.55 | 0.59 | 0.55 | 0.58 | 0.54 | 0.61 | 0.53 |
| SE | 0.045 | 0.071 | 0.046 | 0.043 | 0.066 | 0.045 | 0.060 | 0.084 | 0.064 |
| p | 0.26 | 0.67 | 0.32 | 0.041 | 0.47 | 0.064 | 0.56 | 0.19 | 0.63 |
| nCohort 1 | 263 | 466 | 221 | 263 | 466 | 221 | 263 | 466 | 221 |
| nCohort 2 | 51 | 18 | 50 | 56 | 21 | 52 | 26 | 13 | 23 |
| Cutoff 1 | 0.192 | 0.192 | 0.192 | 0.175 | 0.175 | 0.175 | 0.135 | 0.120 | 0.144 |
| Sens 1 | 71% | 72% | 70% | 73% | 71% | 73% | 73% | 85% | 74% |
| Spec 1 | 44% | 41% | 44% | 40% | 36% | 39% | 32% | 22% | 33% |
| Cutoff 2 | 0.120 | 0.166 | 0.120 | 0.131 | 0.0842 | 0.131 | 0.120 | 0.120 | 0.120 |
| Sens 2 | 80% | 83% | 82% | 80% | 81% | 81% | 81% | 85% | 83% |
| Spec 2 | 27% | 34% | 27% | 30% | 12% | 29% | 27% | 22% | 27% |
| Cutoff 3 | 0.0716 | 0.0141 | 0.0716 | 0.0910 | 0.0594 | 0.105 | 0.107 | 0.113 | 0.105 |
| Sens 3 | 90% | 94% | 92% | 91% | 90% | 90% | 92% | 92% | 96% |
| Spec 3 | 10% | 2% | 10% | 15% | 4% | 22% | 24% | 21% | 22% |
| Cutoff 4 | 0.401 | 0.458 | 0.463 | 0.401 | 0.458 | 0.463 | 0.401 | 0.458 | 0.463 |
| Sens 4 | 37% | 28% | 34% | 38% | 43% | 40% | 38% | 54% | 35% |
| Spec 4 | 71% | 70% | 70% | 71% | 70% | 70% | 71% | 70% | 70% |
| Cutoff 5 | 0.538 | 0.602 | 0.625 | 0.538 | 0.602 | 0.625 | 0.538 | 0.602 | 0.625 |
| Sens 5 | 27% | 17% | 22% | 32% | 33% | 33% | 27% | 46% | 17% |
| Spec 5 | 80% | 80% | 80% | 80% | 80% | 80% | 80% | 80% | 80% |
| Cutoff 6 | 1.01 | 1.26 | 1.41 | 1.01 | 1.26 | 1.41 | 1.01 | 1.26 | 1.41 |
| Sens 6 | 10% | 6% | 8% | 23% | 10% | 12% | 19% | 8% | 9% |
| Spec 6 | 90% | 90% | 90% | 90% | 90% | 90% | 90% | 90% | 90% |
| OR Quart 2 | 1.1 | 1.7 | 1.1 | 1.4 | 0.16 | 2.7 | 1.9 | 0 | 2.2 |
| p Value | 0.84 | 0.48 | 0.83 | 0.50 | 0.090 | 0.045 | 0.27 | na | 0.23 |
| 95% CI of | 0.44 | 0.40 | 0.42 | 0.54 | 0.019 | 1.0 | 0.61 | na | 0.61 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 2.8 | 7.3 | 2.9 | 3.5 | 1.3 | 7.0 | 6.0 | na | 7.6 |
| OR Quart3 | 1.5 | 1.7 | 2.1 | 1.8 | 1.2 | 1.7 | 0.79 | 0.74 | 1.0 |
| p Value | 0.38 | 0.48 | 0.093 | 0.20 | 0.79 | 0.32 | 0.73 | 0.69 | 1.0 |
| 95% CI of | 0.62 | 0.40 | 0.88 | 0.74 | 0.38 | 0.61 | 0.20 | 0.16 | 0.24 |
| OR Quart3 | 3.6 | 7.3 | 5.2 | 4.4 | 3.6 | 4.6 | 3.1 | 3.4 | 4.2 |
| OR Quart4 | 1.7 | 1.7 | 1.7 | 2.6 | 1.2 | 3.1 | 1.6 | 1.5 | 1.8 |
| p Value | 0.21 | 0.48 | 0.27 | 0.030 | 0.79 | 0.020 | 0.40 | 0.53 | 0.35 |
| 95% CI of | 0.73 | 0.40 | 0.67 | 1.1 | 0.38 | 1.2 | 0.51 | 0.42 | 0.51 |
| OR Quart4 | 4.1 | 7.3 | 4.2 | 6.1 | 3.6 | 7.9 | 5.3 | 5.5 | 6.7 |

**Imunoglobulin E**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 30.6 | 54.0 | 30.6 | 59.2 | 30.6 | 52.4 |
| Average | 130 | 110 | 130 | 105 | 130 | 112 |
| Stdev | 313 | 186 | 313 | 143 | 313 | 163 |
| p(t-test) | | 0.67 | | 0.57 | | 0.77 |
| Min | 0.140 | 0.140 | 0.140 | 0.586 | 0.140 | 0.492 |
| Max | 1940 | 1170 | 1940 | 752 | 1940 | 570 |
| n (Samp) | 263 | 51 | 263 | 56 | 263 | 26 |
| n (Patient) | 111 | 51 | 111 | 56 | 111 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 45.7 | 25.4 | 45.7 | 26.4 | 45.7 | 23.0 |
| Average | 132 | 95.4 | 132 | 98.0 | 132 | 116 |
| Stdev | 276 | 188 | 276 | 154 | 276 | 169 |
| p(t-test) | | 0.57 | | 0.57 | | 0.84 |
| Min | 0.140 | 0.140 | 0.140 | 2.16 | 0.140 | 0.492 |
| Max | 1940 | 767 | 1940 | 559 | 1940 | 474 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.6 | 59.2 | 31.6 | 58.0 | 31.6 | 42.6 |
| Average | 113 | 107 | 113 | 95.3 | 113 | 90.0 |
| Stdev | 258 | 183 | 258 | 136 | 258 | 153 |
| p(t-test) | | 0.86 | | 0.62 | | 0.67 |
| Min | 0.972 | 0.586 | 0.972 | 0.586 | 0.972 | 0.140 |
| Max | 1940 | 1170 | 1940 | 752 | 1940 | 570 |
| n (Samp) | 221 | 50 | 221 | 52 | 221 | 23 |
| n (Patient) | 91 | 50 | 91 | 52 | 91 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.38 | 0.56 | 0.57 | 0.44 | 0.55 | 0.56 | 0.44 | 0.52 |
| SE | 0.045 | 0.072 | 0.046 | 0.043 | 0.066 | 0.045 | 0.061 | 0.083 | 0.064 |
| p | 0.24 | 0.090 | 0.16 | 0.088 | 0.34 | 0.32 | 0.33 | 0.51 | 0.81 |
| nCohort 1 | 263 | 466 | 221 | 263 | 466 | 221 | 263 | 466 | 221 |
| nCohort 2 | 51 | 18 | 50 | 56 | 21 | 52 | 26 | 13 | 23 |
| Cutoff 1 | 21.5 | 8.92 | 25.3 | 23.2 | 12.5 | 22.1 | 21.5 | 13.4 | 21.5 |
| Sens 1 | 71% | 72% | 70% | 71% | 71% | 71% | 73% | 77% | 74% |
| Spec 1 | 39% | 10% | 46% | 44% | 16% | 40% | 39% | 18% | 39% |
| Cutoff 2 | 13.4 | 4.01 | 16.2 | 17.2 | 7.68 | 14.3 | 19.3 | 4.82 | 15.4 |
| Sens 2 | 80% | 83% | 80% | 80% | 81% | 81% | 81% | 85% | 83% |
| Spec 2 | 24% | 4% | 28% | 32% | 8% | 25% | 36% | 5% | 27% |
| Cutoff 3 | 8.45 | 1.22 | 12.6 | 11.5 | 6.36 | 11.5 | 9.75 | 1.22 | 9.75 |
| Sens 3 | 90% | 94% | 90% | 91% | 90% | 90% | 92% | 92% | 91% |
| Spec 3 | 14% | 2% | 21% | 21% | 7% | 19% | 16% | 2% | 17% |
| Cutoff 4 | 79.9 | 90.3 | 83.0 | 79.9 | 90.3 | 83.0 | 79.9 | 90.3 | 83.0 |
| Sens 4 | 39% | 22% | 34% | 36% | 29% | 31% | 31% | 31% | 17% |
| Spec 4 | 70% | 70% | 70% | 70% | 70% | 70% | 70% | 70% | 70% |
| Cutoff 5 | 119 | 127 | 124 | 119 | 127 | 124 | 119 | 127 | 124 |
| Sens 5 | 25% | 22% | 16% | 25% | 24% | 21% | 19% | 31% | 9% |
| Spec 5 | 81% | 80% | 80% | 81% | 80% | 80% | 81% | 80% | 80% |
| Cutoff 6 | 283 | 314 | 268 | 283 | 314 | 268 | 283 | 314 | 268 |
| Sens 6 | 12% | 11% | 10% | 9% | 10% | 8% | 12% | 15% | 9% |
| Spec 6 | 90% | 90% | 90% | 90% | 90% | 90% | 90% | 90% | 90% |
| OR Quart 2 | 0.80 | 0.74 | 1.1 | 1.6 | 0.79 | 0.79 | 1.5 | 0.24 | 1.6 |
| p Value | 0.62 | 0.70 | 0.83 | 0.28 | 0.73 | 0.63 | 0.51 | 0.21 | 0.51 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.32 | 0.16 | 0.42 | 0.67 | 0.21 | 0.30 | 0.42 | 0.027 | 0.42 |
| OR Quart2 | 2.0 | 3.4 | 2.9 | 4.1 | 3.0 | 2.1 | 5.7 | 2.2 | 5.8 |
| OR Quart 3 | 1.1 | 0.74 | 2.3 | 2.1 | 1.0 | 2.0 | 2.4 | 1.0 | 2.5 |
| p Value | 0.83 | 0.70 | 0.062 | 0.097 | 1.0 | 0.10 | 0.16 | 1.0 | 0.15 |
| 95% CI of | 0.47 | 0.16 | 0.96 | 0.87 | 0.28 | 0.87 | 0.71 | 0.24 | 0.72 |
| OR Quart3 | 2.6 | 3.4 | 5.6 | 5.0 | 3.5 | 4.6 | 8.3 | 4.1 | 8.5 |
| OR Quart 4 | 1.4 | 2.1 | 1.5 | 1.9 | 1.4 | 1.2 | 1.8 | 1.0 | 1.0 |
| p Value | 0.43 | 0.25 | 0.37 | 0.14 | 0.55 | 0.68 | 0.36 | 0.99 | 1.0 |
| 95% CI of | 0.61 | 0.61 | 0.60 | 0.80 | 0.44 | 0.50 | 0.50 | 0.25 | 0.24 |
| OR Quart4 | 3.2 | 7.1 | 3.8 | 4.7 | 4.7 | 2.9 | 6.4 | 4.1 | 4.2 |

**Immunoglobulin M**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.629 | 0.492 | 0.629 | 0.503 | 0.629 | 0.461 |
| Average | 0.846 | 0.713 | 0.846 | 0.762 | 0.846 | 0.600 |
| Stdev | 0.702 | 0.647 | 0.702 | 0.739 | 0.702 | 0.430 |
| p(t-test) |  | 0.21 |  | 0.42 |  | 0.081 |
| Min | 0.0493 | 0.132 | 0.0493 | 0.0967 | 0.0493 | 0.0968 |
| Max | 3.81 | 3.09 | 3.81 | 3.51 | 3.81 | 1.79 |
| n (Samp) | 263 | 51 | 263 | 56 | 263 | 26 |
| n (Patient) | 111 | 51 | 111 | 56 | ill | 26 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.544 | 0.692 | 0.544 | 0.856 | 0.544 | 0.659 |
| Average | 0.773 | 0.881 | 0.773 | 1.04 | 0.773 | 0.860 |
| Stdev | 0.687 | 0.616 | 0.687 | 0.762 | 0.687 | 0.746 |
| p(t-test) |  | 0.51 |  | 0.083 |  | 0.65 |
| Min | 0.0493 | 0.185 | 0.0493 | 0.142 | 0.0493 | 0.0968 |
| Max | 4.82 | 2.23 | 4.82 | 2.70 | 4.82 | 2.89 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.561 | 0.500 | 0.561 | 0.463 | 0.561 | 0.460 |
| Average | 0.786 | 0.744 | 0.786 | 0.752 | 0.786 | 0.670 |
| Stdev | 0.657 | 0.687 | 0.657 | 0.766 | 0.657 | 0.556 |
| p(t-test) | | 0.69 | | 0.75 | | 0.42 |
| Min | 0.0815 | 0.132 | 0.0815 | 0.0967 | 0.0815 | 0.132 |
| Max | 3.75 | 3.09 | 3.75 | 3.51 | 3.75 | 2.23 |
| n (Samp) | 221 | 50 | 221 | 52 | 221 | 23 |
| n (Patient) | 91 | 50 | 91 | 52 | 91 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | 0.57 | 0.47 | 0.44 | 0.62 | 0.46 | 0.41 | 0.55 | 0.45 |
| SE | 0.045 | 0.072 | 0.046 | 0.043 | 0.067 | 0.045 | 0.061 | 0.083 | 0.065 |
| p | 0.14 | 0.30 | 0.54 | 0.17 | 0.063 | 0.36 | 0.13 | 0.55 | 0.45 |
| nCohort 1 | 263 | 466 | 221 | 263 | 466 | 221 | 263 | 466 | 221 |
| nCohort 2 | 51 | 18 | 50 | 56 | 21 | 52 | 26 | 13 | 23 |
| Cutoff 1 | 0.350 | 0.475 | 0.350 | 0.310 | 0.548 | 0.312 | 0.349 | 0.365 | 0.324 |
| Sens 1 | 71% | 72% | 72% | 71% | 71% | 71% | 73% | 77% | 74% |
| Spec 1 | 25% | 41% | 28% | 21% | 50% | 24% | 25% | 29% | 25% |
| Cutoff 2 | 0.248 | 0.312 | 0.273 | 0.248 | 0.477 | 0.273 | 0.318 | 0.312 | 0.295 |
| Sens 2 | 84% | 83% | 80% | 80% | 81% | 81% | 81% | 85% | 83% |
| Spec 2 | 17% | 23% | 21% | 17% | 41% | 21% | 22% | 23% | 23% |
| Cutoff 3 | 0.229 | 0.184 | 0.229 | 0.202 | 0.202 | 0.229 | 0.202 | 0.229 | 0.215 |
| Sens 3 | 90% | 100% | 94% | 91% | 90% | 92% | 92% | 92% | 91% |
| Spec 3 | 13% | 7% | 14% | 11% | 10% | 14% | 11% | 12% | 12% |
| Cutoff 4 | 0.977 | 0.845 | 0.850 | 0.977 | 0.845 | 0.850 | 0.977 | 0.845 | 0.850 |
| Sens 4 | 22% | 39% | 30% | 25% | 52% | 29% | 19% | 31% | 22% |
| Spec 4 | 70% | 70% | 70% | 70% | 70% | 70% | 70% | 70% | 70% |
| Cutoff 5 | 1.29 | 1.10 | 1.26 | 1.29 | 1.10 | 1.26 | 1.29 | 1.10 | 1.26 |
| Sens 5 | 14% | 33% | 16% | 18% | 29% | 15% | 8% | 23% | 13% |
| Spec 5 | 80% | 80% | 81% | 80% | 80% | 81% | 80% | 80% | 81% |
| Cutoff 6 | 1.82 | 1.70 | 1.71 | 1.82 | 1.70 | 1.71 | 1.82 | 1.70 | 1.71 |
| Sens 6 | 6% | 17% | 8% | 7% | 14% | 8% | 0% | 15% | 13% |
| Spec 6 | 90% | 90% | 90% | 90% | 90% | 90% | 90% | 90% | 90% |
| OR Quart 2 | 1.1 | 0.49 | 1.0 | 1.1 | 1.3 | 0.72 | 2.1 | 0.32 | 0.79 |
| p Value | 0.78 | 0.42 | 1.0 | 0.82 | 0.71 | 0.50 | 0.30 | 0.33 | 0.73 |
| 95% CI of | 0.44 | 0.088 | 0.40 | 0.46 | 0.29 | 0.28 | 0.51 | 0.033 | 0.20 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 3.0 | 2.7 | 2.5 | 2.7 | 6.1 | 1.9 | 8.8 | 3.2 | 3.1 |
| OR Quart3 | 2.1 | 1.5 | 1.3 | 1.4 | 1.7 | 1.3 | 3.8 | 1.7 | 1.7 |
| p Value | 0.091 | 0.52 | 0.51 | 0.39 | 0.48 | 0.49 | 0.052 | 0.48 | 0.38 |
| 95% CI of | 0.89 | 0.42 | 0.56 | 0.62 | 0.39 | 0.58 | 0.99 | 0.39 | 0.52 |
| OR Quart3 | 5.1 | 5.5 | 3.2 | 3.4 | 7.2 | 3.1 | 14 | 7.2 | 5.5 |
| OR Quart4 | 1.9 | 1.5 | 1.4 | 1.9 | 3.1 | 1.5 | 2.5 | 1.3 | 1.2 |
| p Value | 0.18 | 0.52 | 0.48 | 0.14 | 0.093 | 0.37 | 0.20 | 0.71 | 0.75 |
| 95% CI of | 0.76 | 0.42 | 0.57 | 0.81 | 0.83 | 0.63 | 0.62 | 0.29 | 0.35 |
| OR Quart4 | 4.5 | 5.5 | 3.3 | 4.2 | 12 | 3.4 | 10 | 6.1 | 4.2 |

**Insulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 181 | 291 | 181 | 164 | 181 | 196 |
| Average | 402 | 597 | 402 | 263 | 402 | 399 |
| Stdev | 876 | 581 | 876 | 249 | 876 | 636 |
| p(t-test) |  | 0.41 |  | 0.45 |  | 0.99 |
| Min | 30.2 | 84.2 | 30.2 | 27.6 | 30.2 | 73.3 |
| Max | 6430 | 2010 | 6430 | 909 | 6430 | 1960 |
| n (Samp) | 56 | 16 | 56 | 24 | 56 | 8 |
| n (Patient) | 55 | 16 | 55 | 24 | 55 | 8 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 177 | 361 | 177 | 152 | 177 | 161 |
| Average | 418 | 600 | 418 | 228 | 418 | 373 |
| Stdev | 916 | 549 | 916 | 235 | 916 | 600 |
| p(t-test) |  | 0.49 |  | 0.33 |  | 0.89 |
| Min | 30.2 | 164 | 30.2 | 27.6 | 30.2 | 73.3 |
| Max | 6430 | 2010 | 6430 | 909 | 6430 | 1960 |
| n (Samp) | 51 | 13 | 51 | 23 | 51 | 9 |
| n (Patient) | 49 | 13 | 49 | 23 | 49 | 9 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.69 | nd | 0.73 | 0.45 | nd | 0.42 | 0.48 | nd | 0.49 |
| SE | 0.080 | nd | 0.085 | 0.071 | nd | 0.073 | 0.11 | nd | 0.11 |
| p | 0.019 | nd | 0.0063 | 0.49 | nd | 0.26 | 0.88 | nd | 0.93 |
| nCohort 1 | 56 | nd | 51 | 56 | nd | 51 | 56 | nd | 51 |
| nCohort 2 | 16 | nd | 13 | 24 | nd | 23 | 8 | nd | 9 |
| Cutoff 1 | 207 | nd | 239 | 112 | nd | 84.2 | 139 | nd | 139 |
| Sens 1 | 75% | nd | 77% | 71% | nd | 74% | 75% | nd | 78% |
| Spec 1 | 55% | nd | 63% | 29% | nd | 20% | 39% | nd | 43% |
| Cutoff 2 | 179 | nd | 207 | 64.2 | nd | 64.2 | 73.3 | nd | 75.1 |
| Sens 2 | 81% | nd | 85% | 83% | nd | 83% | 88% | nd | 89% |
| Spec 2 | 50% | nd | 57% | 9% | nd | 10% | 11% | nd | 12% |
| Cutoff 3 | 153 | nd | 177 | 33.9 | nd | 33.9 | 72.0 | nd | 64.2 |
| Sens 3 | 94% | nd | 92% | 92% | nd | 91% | 100% | nd | 100% |
| Spec 3 | 43% | nd | 51% | 4% | nd | 4% | 11% | nd | 10% |
| Cutoff 4 | 317 | nd | 362 | 317 | nd | 362 | 317 | nd | 362 |
| Sens 4 | 44% | nd | 46% | 29% | nd | 17% | 12% | nd | 11% |
| Spec 4 | 71% | nd | 71% | 71% | nd | 71% | 71% | nd | 71% |
| Cutoff 5 | 415 | nd | 457 | 415 | nd | 457 | 415 | nd | 457 |
| Sens 5 | 38% | nd | 38% | 25% | nd | 17% | 12% | nd | 11% |
| Spec 5 | 80% | nd | 80% | 80% | nd | 80% | 80% | nd | 80% |
| Cutoff 6 | 756 | nd | 756 | 756 | nd | 756 | 756 | nd | 756 |
| Sens 6 | 31% | nd | 31% | 4% | nd | 4% | 12% | nd | 11% |
| Spec 6 | 91% | nd | 90% | 91% | nd | 90% | 91% | nd | 90% |
| OR Quart 2 | 4.9 | nd | >3.7 | 0.58 | nd | 1.9 | 3.5 | nd | 3.5 |
| p Value | 0.18 | nd | <0.28 | 0.47 | nd | 0.40 | 0.31 | nd | 0.30 |
| 95% CI of | 0.49 | nd | >0.34 | 0.14 | nd | 0.43 | 0.32 | nd | 0.32 |
| OR Quart2 | 49 | nd | na | 2.5 | nd | 8.2 | 37 | nd | 38 |
| OR Quart 3 | 6.5 | nd | >7.3 | 1.3 | nd | 1.3 | 2.1 | nd | 3.5 |
| p Value | 0.10 | nd | <0.088 | 0.74 | nd | 0.70 | 0.55 | nd | 0.30 |
| 95% CI of | 0.68 | nd | >0.74 | 0.33 | nd | 0.30 | 0.17 | nd | 0.32 |
| OR Quart3 | 63 | nd | na | 4.7 | nd | 6.0 | 26 | nd | 38 |
| OR Quart 4 | 8.5 | nd | >7.3 | 1.3 | nd | 3.0 | 2.1 | nd | 2.2 |
| p Value | 0.061 | nd | <0.088 | 0.74 | nd | 0.14 | 0.55 | nd | 0.55 |
| 95% CI of | 0.90 | nd | >0.74 | 0.33 | nd | 0.71 | 0.17 | nd | 0.17 |
| OR Quart4 | 80 | nd | na | 4.7 | nd | 13 | 26 | nd | 27 |

**Macrophage migration inhibitory factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 758 | 973 | 758 | 879 | 758 | 823 |
| Average | 885 | 1410 | 885 | 924 | 885 | 815 |
| Stdev | 516 | 2110 | 516 | 491 | 516 | 343 |
| p(t-test) | | 0.017 | | 0.66 | | 0.52 |
| Min | 127 | 212 | 127 | 264 | 127 | 246 |
| Max | 3190 | 14200 | 3190 | 2460 | 3190 | 1740 |
| n (Samp) | 106 | 45 | 106 | 49 | 106 | 25 |
| n (Patient) | 98 | 45 | 98 | 49 | 98 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 782 | 1070 | 782 | 933 | 782 | 911 |
| Average | 921 | 2340 | 921 | 1030 | 921 | 831 |
| Stdev | 645 | 3690 | 645 | 561 | 645 | 252 |
| p(t-test) | | 1.6E-6 | | 0.51 | | 0.64 |
| Min | 79.0 | 338 | 79.0 | 337 | 79.0 | 417 |
| Max | 4780 | 14200 | 4780 | 2310 | 4780 | 1200 |
| n (Samp) | 246 | 13 | 246 | 16 | 246 | 11 |
| n (Patient) | 160 | 13 | 160 | 16 | 160 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 771 | 907 | 771 | 930 | 771 | 823 |
| Average | 992 | 1110 | 992 | 987 | 992 | 843 |
| Stdev | 1430 | 855 | 1430 | 551 | 1430 | 396 |
| p(t-test) | | 0.64 | | 0.98 | | 0.64 |
| Min | 81.9 | 212 | 81.9 | 264 | 81.9 | 246 |
| Max | 14200 | 4060 | 14200 | 2470 | 14200 | 1740 |
| n (Samp) | 100 | 39 | 100 | 43 | 100 | 21 |
| n (Patient) | 86 | 39 | 86 | 43 | 86 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.71 | 0.56 | 0.54 | 0.58 | 0.57 | 0.51 | 0.53 | 0.52 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| SE | 0.052 | 0.083 | 0.055 | 0.050 | 0.077 | 0.053 | 0.065 | 0.091 | 0.070 |
| p | 0.073 | 0.013 | 0.28 | 0.46 | 0.30 | 0.18 | 0.87 | 0.73 | 0.77 |
| nCohort 1 | 106 | 246 | 100 | 106 | 246 | 100 | 106 | 246 | 100 |
| nCohort 2 | 45 | 13 | 39 | 49 | 16 | 43 | 25 | 11 | 21 |
| Cutoff 1 | 676 | 877 | 649 | 641 | 632 | 663 | 605 | 621 | 605 |
| Sens 1 | 71% | 77% | 72% | 71% | 75% | 72% | 72% | 73% | 71% |
| Spec 1 | 35% | 61% | 33% | 31% | 34% | 34% | 28% | 33% | 28% |
| Cutoff 2 | 497 | 830 | 476 | 521 | 621 | 605 | 492 | 592 | 408 |
| Sens 2 | 80% | 85% | 82% | 82% | 81% | 81% | 80% | 82% | 81% |
| Spec 2 | 18% | 57% | 18% | 22% | 33% | 28% | 18% | 30% | 14% |
| Cutoff 3 | 356 | 533 | 337 | 337 | 435 | 356 | 386 | 533 | 386 |
| Sens 3 | 91% | 92% | 92% | 92% | 94% | 91% | 92% | 91% | 90% |
| Spec 3 | 8% | 27% | 10% | 7% | 17% | 12% | 10% | 27% | 14% |
| Cutoff 4 | 970 | 987 | 970 | 970 | 987 | 970 | 970 | 987 | 970 |
| Sens 4 | 51% | 62% | 44% | 37% | 44% | 40% | 28% | 36% | 29% |
| Spec 4 | 71% | 70% | 70% | 71% | 70% | 70% | 71% | 70% | 70% |
| Cutoff 5 | 1120 | 1160 | 1100 | 1120 | 1160 | 1100 | 1120 | 1160 | 1100 |
| Sens 5 | 38% | 46% | 33% | 22% | 38% | 23% | 12% | 9% | 24% |
| Spec 5 | 80% | 80% | 80% | 80% | 80% | 80% | 80% | 80% | 80% |
| Cutoff 6 | 1550 | 1630 | 1440 | 1550 | 1630 | 1440 | 1550 | 1630 | 1440 |
| Sens 6 | 20% | 31% | 21% | 14% | 12% | 19% | 4% | 0% | 10% |
| Spec 6 | 91% | 90% | 90% | 91% | 90% | 90% | 91% | 90% | 90% |
| OR Quart 2 | 0.25 | 0.98 | 0.35 | 1.1 | 2.6 | 0.96 | 0.36 | 4.2 | 0.29 |
| p Value | 0.027 | 0.99 | 0.082 | 0.85 | 0.27 | 0.95 | 0.16 | 0.21 | 0.15 |
| 95% CI of | 0.071 | 0.060 | 0.11 | 0.40 | 0.48 | 0.32 | 0.084 | 0.46 | 0.053 |
| OR Quart2 | 0.85 | 16 | 1.1 | 3.0 | 14 | 2.9 | 1.5 | 39 | 1.5 |
| OR Quart 3 | 0.96 | 5.2 | 0.84 | 1.8 | 1.5 | 2.4 | 1.3 | 4.2 | 1.2 |
| p Value | 0.94 | 0.14 | 0.73 | 0.26 | 0.65 | 0.094 | 0.61 | 0.21 | 0.75 |
| 95% CI of | 0.36 | 0.60 | 0.30 | 0.66 | 0.25 | 0.86 | 0.43 | 0.46 | 0.36 |
| OR Quart3 | 2.5 | 46 | 2.3 | 4.6 | 9.4 | 6.8 | 4.2 | 39 | 4.2 |
| OR Quart 4 | 1.7 | 6.4 | 1.2 | 1.4 | 3.1 | 1.7 | 0.79 | 2.0 | 0.96 |
| p Value | 0.28 | 0.090 | 0.68 | 0.50 | 0.17 | 0.33 | 0.71 | 0.58 | 0.95 |
| 95% CI of | 0.66 | 0.75 | 0.46 | 0.52 | 0.61 | 0.59 | 0.23 | 0.18 | 0.27 |
| OR Quart4 | 4.3 | 55 | 3.3 | 3.7 | 16 | 4.8 | 2.7 | 23 | 3.4 |

**Transforming growth factor beta-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.369 | 1.00E-9 | 0.369 | 0.738 | nd | nd |
| Average | 2.02 | 0.465 | 2.02 | 0.683 | nd | nd |
| Stdev | 5.28 | 0.899 | 5.28 | 0.835 | nd | nd |
| p(t-test) | | 0.39 | | 0.39 | nd | nd |
| Min | 1.00E-9 | 1.00E-9 | 1.00E-9 | 1.00E-9 | nd | nd |
| Max | 27.1 | 2.71 | 27.1 | 2.71 | nd | nd |
| n (Samp) | 28 | 9 | 28 | 12 | nd | nd |
| n (Patient) | 28 | 9 | 28 | 12 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.738 | 1.00E-9 | 0.738 | 0.738 | nd | nd |
| Average | 2.58 | 5.39 | 2.58 | 0.678 | nd | nd |
| Stdev | 5.52 | 13.9 | 5.52 | 0.876 | nd | nd |
| p(t-test) | | 0.40 | | 0.27 | nd | nd |
| Min | 1.00E-9 | 1.00E-9 | 1.00E-9 | 1.00E-9 | nd | nd |
| Max | 27.1 | 39.7 | 27.1 | 2.71 | nd | nd |
| n (Samp) | 26 | 8 | 26 | 11 | nd | nd |
| n (Patient) | 26 | 8 | 26 | 11 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.40 | nd | 0.41 | 0.51 | nd | 0.41 | nd | nd | nd |
| SE | 0.11 | nd | 0.12 | 0.10 | nd | 0.11 | nd | nd | nd |
| p | 0.38 | nd | 0.43 | 0.95 | nd | 0.42 | nd | nd | nd |
| nCohort 1 | 28 | nd | 26 | 28 | nd | 26 | nd | nd | nd |
| nCohort 2 | 9 | nd | 8 | 12 | nd | 11 | nd | nd | nd |
| Cutoff 1 | 0 | nd | 0 | 0 | nd | 0 | nd | nd | nd |
| Sens 1 | 100% | nd | 100% | 100% | nd | 100% | nd | nd | nd |
| Spec 1 | 0% | nd | 0% | 0% | nd | 0% | nd | nd | nd |
| Cutoff 2 | 0 | nd | 0 | 0 | nd | 0 | nd | nd | nd |
| Sens 2 | 100% | nd | 100% | 100% | nd | 100% | nd | nd | nd |
| Spec 2 | 0% | nd | 0% | 0% | nd | 0% | nd | nd | nd |
| Cutoff 3 | 0 | nd | 0 | 0 | nd | 0 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 100% | nd | 100% | 100% | nd | 100% | nd | nd | nd |
| Spec 3 | 0% | nd | 0% | 0% | nd | 0% | nd | nd | nd |
| Cutoff 4 | 0.738 | nd | 2.71 | 0.738 | nd | 2.71 | nd | nd | nd |
| Sens 4 | 11% | nd | 12% | 17% | nd | 0% | nd | nd | nd |
| Spec 4 | 79% | nd | 81% | 79% | nd | 81% | nd | nd | nd |
| Cutoff 5 | 2.71 | nd | 2.71 | 2.71 | nd | 2.71 | nd | nd | nd |
| Sens 5 | 0% | nd | 12% | 0% | nd | 0% | nd | nd | nd |
| Spec 5 | 86% | nd | 81% | 86% | nd | 81% | nd | nd | nd |
| Cutoff 6 | 5.82 | nd | 7.21 | 5.82 | nd | 7.21 | nd | nd | nd |
| Sens 6 | 0% | nd | 12% | 0% | nd | 0% | nd | nd | nd |
| Spec 6 | 93% | nd | 92% | 93% | nd | 92% | nd | nd | nd |
| OR Quart 2 | 4.5 | nd | 0.50 | 1.0 | nd | 2.0 | nd | nd | nd |
| p Value | 0.24 | nd | 0.60 | 1.0 | nd | 0.51 | nd | nd | nd |
| 95% CI of | 0.37 | nd | 0.036 | 0.15 | nd | 0.25 | nd | nd | nd |
| OR Quart2 | 54 | nd | 6.9 | 6.8 | nd | 16 | nd | nd | nd |
| OR Quart 3 | 4.5 | nd | 4.4 | 1.6 | nd | 2.0 | nd | nd | nd |
| p Value | 0.24 | nd | 0.16 | 0.64 | nd | 0.51 | nd | nd | nd |
| 95% CI of | 0.37 | nd | 0.56 | 0.24 | nd | 0.25 | nd | nd | nd |
| OR Quart3 | 54 | nd | 34 | 9.9 | nd | 16 | nd | nd | nd |
| OR Quart 4 | 2.6 | nd | 0 | 0.58 | nd | 2.0 | nd | nd | nd |
| p Value | 0.48 | nd | na | 0.61 | nd | 0.51 | nd | nd | nd |
| 95% CI of | 0.19 | nd | na | 0.075 | nd | 0.25 | nd | nd | nd |
| OR Quart4 | 34 | nd | na | 4.6 | nd | 16 | nd | nd | nd |

**Transforming growth factor beta-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.774 | 0.607 | 0.774 | 0.607 | nd | nd |
| Average | 1.45 | 0.722 | 1.45 | 0.546 | nd | nd |
| Stdev | 1.91 | 0.458 | 1.91 | 0.606 | nd | nd |
| p(t-test) | | 0.27 | | 0.12 | nd | nd |
| Min | 1.00E-9 | 1.00E-9 | 1.00E-9 | 1.00E-9 | nd | nd |
| Max | 7.69 | 1.27 | 7.69 | 1.91 | nd | nd |
| n (Samp) | 28 | 9 | 28 | 12 | nd | nd |
| n (Patient) | 28 | 9 | 28 | 12 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.10 | 0.607 | 1.10 | 0.607 | nd | nd |
| Average | 1.53 | 5.43 | 1.53 | 0.595 | nd | nd |
| Stdev | 1.94 | 13.5 | 1.94 | 0.610 | nd | nd |
| p(t-test) | | 0.15 | | 0.13 | nd | nd |
| Min | 1.00E-9 | 1.00E-9 | 1.00E-9 | 1.00E-9 | nd | nd |
| Max | 7.69 | 38.8 | 7.69 | 1.91 | nd | nd |
| n (Samp) | 26 | 8 | 26 | 11 | nd | nd |
| n (Patient) | 26 | 8 | 26 | 11 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.42 | nd | 0.44 | 0.32 | nd | 0.31 | nd | nd | nd |
| SE | 0.11 | nd | 0.12 | 0.098 | nd | 0.10 | nd | nd | nd |
| p | 0.46 | nd | 0.59 | 0.072 | nd | 0.067 | nd | nd | nd |
| nCohort 1 | 28 | nd | 26 | 28 | nd | 26 | nd | nd | nd |
| nCohort 2 | 9 | nd | 8 | 12 | nd | 11 | nd | nd | nd |
| Cutoff 1 | 0.263 | nd | 0.263 | 0 | nd | 0 | nd | nd | nd |
| Sens 1 | 78% | nd | 75% | 100% | nd | 100% | nd | nd | nd |
| Spec 1 | 21% | nd | 19% | 0% | nd | 0% | nd | nd | nd |
| Cutoff 2 | 1.00E-9 | nd | 1.00E-9 | 0 | nd | 0 | nd | nd | nd |
| Sens 2 | 89% | nd | 88% | 100% | nd | 100% | nd | nd | nd |
| Spec 2 | 18% | nd | 15% | 0% | nd | 0% | nd | nd | nd |
| Cutoff 3 | 0 | nd | 0 | 0 | nd | 0 | nd | nd | Nd |
| Sens 3 | 100% | nd | 100% | 100% | nd | 100% | nd | nd | Nd |
| Spec 3 | 0% | nd | 0% | 0% | nd | 0% | nd | nd | Nd |
| Cutoff 4 | 1.27 | nd | 1.27 | 1.27 | nd | 1.27 | nd | nd | Nd |
| Sens 4 | 0% | nd | 12% | 8% | nd | 9% | nd | nd | Nd |
| Spec 4 | 79% | nd | 81% | 79% | nd | 81% | nd | nd | nd |
| Cutoff 5 | 1.91 | nd | 1.27 | 1.91 | nd | 1.27 | nd | nd | nd |
| Sens 5 | 0% | nd | 12% | 0% | nd | 9% | nd | nd | nd |
| Spec 5 | 82% | nd | 81% | 82% | nd | 81% | nd | nd | nd |
| Cutoff 6 | 4.70 | nd | 4.70 | 4.70 | nd | 4.70 | nd | nd | nd |
| Sens 6 | 0% | nd | 12% | 0% | nd | 0% | nd | nd | nd |
| Spec 6 | 93% | nd | 92% | 93% | nd | 92% | nd | nd | nd |
| OR Quart 2 | >5.0 | nd | 2.7 | 3.9 | nd | 0.50 | nd | nd | nd |
| p Value | <0.20 | nd | 0.46 | 0.28 | nd | 0.60 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | >0.42 | nd | 0.19 | 0.33 | nd | 0.037 | nd | nd | nd |
| OR Quart2 | na | nd | 37 | 46 | nd | 6.7 | nd | nd | nd |
| OR Quart 3 | >5.0 | nd | 4.0 | 3.9 | nd | 3.2 | nd | nd | nd |
| p Value | <0.20 | nd | 0.28 | 0.28 | nd | 0.26 | nd | nd | nd |
| 95% CI of | >0.42 | nd | 0.33 | 0.33 | nd | 0.42 | nd | nd | nd |
| OR Quart3 | na | nd | 49 | 46 | nd | 24 | nd | nd | nd |
| OR Quart 4 | >5.0 | nd | 2.7 | 9.0 | nd | 3.2 | nd | nd | nd |
| p Value | <0.20 | nd | 0.46 | 0.074 | nd | 0.26 | nd | nd | nd |
| 95% CI of | >0.42 | nd | 0.19 | 0.81 | nd | 0.42 | nd | nd | nd |
| OR Quart4 | na | nd | 37 | 100 | nd | 24 | nd | nd | nd |

**Transmembrane glycoprotein NMB**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 15.0 | 13.9 | 15.0 | 13.2 | 15.0 | 14.1 |
| Average | 16.7 | 15.6 | 16.7 | 16.2 | 16.7 | 14.9 |
| Stdev | 6.72 | 7.76 | 6.72 | 8.09 | 6.72 | 4.57 |
| p(t-test) |  | 0.61 |  | 0.78 |  | 0.47 |
| Min | 7.70 | 6.34 | 7.70 | 6.41 | 7.70 | 8.10 |
| Max | 41.3 | 33.2 | 41.3 | 41.2 | 41.3 | 23.0 |
| n (Samp) | 53 | 16 | 53 | 24 | 53 | 8 |
| n (Patient) | 52 | 16 | 52 | 24 | 52 | 8 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 14.6 | 13.6 | 14.6 | 14.6 | 14.6 | 13.1 |
| Average | 16.5 | 16.2 | 16.5 | 16.7 | 16.5 | 14.8 |
| Stdev | 6.49 | 8.49 | 6.49 | 8.24 | 6.49 | 4.93 |
| p(t-test) |  | 0.92 |  | 0.90 |  | 0.52 |
| Min | 7.85 | 6.34 | 7.85 | 6.41 | 7.85 | 8.10 |
| Max | 41.3 | 33.2 | 41.3 | 41.2 | 41.3 | 23.0 |
| n (Samp) | 48 | 14 | 48 | 24 | 48 | 7 |
| n (Patient) | 44 | 14 | 44 | 24 | 44 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | nd | 0.44 | 0.44 | nd | 0.46 | 0.45 | nd | 0.44 |
| SE | 0.084 | nd | 0.089 | 0.072 | nd | 0.073 | 0.11 | nd | 0.12 |
| p | 0.40 | nd | 0.52 | 0.41 | nd | 0.62 | 0.64 | nd | 0.64 |
| nCohort 1 | 53 | nd | 48 | 53 | nd | 48 | 53 | nd | 48 |
| nCohort 2 | 16 | nd | 14 | 24 | nd | 24 | 8 | nd | 7 |
| Cutoff 1 | 10.3 | nd | 10.3 | 11.0 | nd | 11.0 | 12.2 | nd | 12.4 |
| Sens 1 | 75% | nd | 71% | 71% | nd | 71% | 75% | nd | 71% |
| Spec 1 | 15% | nd | 10% | 19% | nd | 15% | 30% | nd | 29% |
| Cutoff 2 | 10.00 | nd | 9.09 | 10.8 | nd | 10.8 | 11.7 | nd | 11.7 |
| Sens 2 | 81% | nd | 86% | 83% | nd | 83% | 88% | nd | 86% |
| Spec 2 | 13% | nd | 8% | 17% | nd | 12% | 23% | nd | 19% |
| Cutoff 3 | 6.34 | nd | 6.34 | 9.09 | nd | 9.09 | 8.02 | nd | 8.02 |
| Sens 3 | 94% | nd | 93% | 92% | nd | 92% | 100% | nd | 100% |
| Spec 3 | 0% | nd | 0% | 11% | nd | 8% | 6% | nd | 4% |
| Cutoff 4 | 19.4 | nd | 18.0 | 19.4 | nd | 18.0 | 19.4 | nd | 18.0 |
| Sens 4 | 25% | nd | 36% | 21% | nd | 25% | 12% | nd | 29% |
| Spec 4 | 72% | nd | 71% | 72% | nd | 71% | 72% | nd | 71% |
| Cutoff 5 | 21.4 | nd | 20.7 | 21.4 | nd | 20.7 | 21.4 | nd | 20.7 |
| Sens 5 | 19% | nd | 29% | 21% | nd | 25% | 12% | nd | 14% |
| Spec 5 | 81% | nd | 81% | 81% | nd | 81% | 81% | nd | 81% |
| Cutoff 6 | 25.3 | nd | 25.3 | 25.3 | nd | 25.3 | 25.3 | nd | 25.3 |
| Sens 6 | 12% | nd | 14% | 17% | nd | 17% | 0% | nd | 0% |
| Spec 6 | 91% | nd | 92% | 91% | nd | 92% | 91% | nd | 92% |
| OR Quart 2 | 2.1 | nd | 0.75 | 1.4 | nd | 1.0 | 3.8 | nd | 2.2 |
| p Value | 0.37 | nd | 0.74 | 0.65 | nd | 1.0 | 0.28 | nd | 0.55 |
| 95% CI of | 0.41 | nd | 0.14 | 0.34 | nd | 0.25 | 0.34 | nd | 0.17 |
| OR Quart2 | 11 | nd | 4.1 | 5.6 | nd | 4.0 | 41 | nd | 27 |
| OR Quart 3 | 0.67 | nd | 0 | 1.1 | nd | 0.40 | 2.3 | nd | 2.2 |
| p Value | 0.68 | nd | na | 0.93 | nd | 0.26 | 0.51 | nd | 0.55 |
| 95% CI of | 0.097 | nd | na | 0.25 | nd | 0.082 | 0.19 | nd | 0.17 |
| OR Quart3 | 4.6 | nd | na | 4.5 | nd | 1.9 | 28 | nd | 27 |
| OR Quart 4 | 2.7 | nd | 2.6 | 2.2 | nd | 2.0 | 2.3 | nd | 2.4 |
| p Value | 0.22 | nd | 0.21 | 0.26 | nd | 0.31 | 0.51 | nd | 0.51 |
| 95% CI of | 0.56 | nd | 0.57 | 0.56 | nd | 0.52 | 0.19 | nd | 0.19 |
| OR Quart4 | 13 | nd | 12 | 8.5 | nd | 7.7 | 28 | nd | 30 |

**Cadherin-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.6 | 16.4 | 21.6 | 18.8 | 21.6 | 19.0 |
| Average | 24.9 | 19.2 | 24.9 | 26.6 | 24.9 | 29.2 |
| Stdev | 13.4 | 9.80 | 13.4 | 26.9 | 13.4 | 24.4 |
| p(t-test) | | 0.12 | | 0.71 | | 0.45 |
| Min | 7.60 | 6.92 | 7.60 | 6.67 | 7.60 | 6.13 |
| Max | 69.3 | 40.0 | 69.3 | 135 | 69.3 | 78.5 |
| n (Samp) | 53 | 16 | 53 | 24 | 53 | 8 |
| n (Patient) | 52 | 16 | 52 | 24 | 52 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.5 | 16.4 | 22.5 | 18.8 | 22.5 | 16.5 |
| Average | 25.4 | 19.9 | 25.4 | 26.4 | 25.4 | 29.0 |
| Stdev | 12.8 | 9.76 | 12.8 | 26.9 | 12.8 | 26.3 |
| p(t-test) | | 0.14 | | 0.83 | | 0.56 |
| Min | 9.76 | 7.52 | 9.76 | 6.67 | 9.76 | 6.13 |
| Max | 69.3 | 39.1 | 69.3 | 135 | 69.3 | 78.5 |
| n (Samp) | 48 | 14 | 48 | 24 | 48 | 7 |
| n (Patient) | 44 | 14 | 44 | 24 | 44 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.38 | nd | 0.37 | 0.44 | nd | 0.42 | 0.50 | nd | 0.44 |
| SE | 0.083 | nd | 0.089 | 0.072 | nd | 0.073 | 0.11 | nd | 0.12 |
| p | 0.15 | nd | 0.14 | 0.41 | nd | 0.25 | 0.97 | nd | 0.64 |
| nCohort 1 | 53 | nd | 48 | 53 | nd | 48 | 53 | nd | 48 |
| nCohort 2 | 16 | nd | 14 | 24 | nd | 24 | 8 | nd | 7 |
| Cutoff 1 | 11.4 | nd | 12.6 | 13.1 | nd | 13.1 | 14.9 | nd | 14.9 |
| Sens 1 | 75% | nd | 71% | 71% | nd | 71% | 75% | nd | 71% |
| Spec 1 | 13% | nd | 17% | 21% | nd | 17% | 28% | nd | 23% |
| Cutoff 2 | 10.9 | nd | 10.9 | 10.9 | nd | 10.9 | 12.6 | nd | 12.6 |
| Sens 2 | 81% | nd | 86% | 83% | nd | 83% | 88% | nd | 86% |
| Spec 2 | 13% | nd | 8% | 13% | nd | 8% | 21% | nd | 17% |
| Cutoff 3 | 6.92 | nd | 9.76 | 9.21 | nd | 9.21 | 0 | nd | 0 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 94% | nd | 93% | 92% | nd | 92% | 100% | nd | 100% |
| Spec 3 | 0% | nd | 2% | 2% | nd | 0% | 0% | nd | 0% |
| Cutoff 4 | 30.0 | nd | 30.8 | 30.0 | nd | 30.8 | 30.0 | nd | 30.8 |
| Sens 4 | 19% | nd | 14% | 21% | nd | 21% | 38% | nd | 29% |
| Spec 4 | 72% | nd | 71% | 72% | nd | 71% | 72% | nd | 71% |
| Cutoff 5 | 34.5 | nd | 36.1 | 34.5 | nd | 36.1 | 34.5 | nd | 36.1 |
| Sens 5 | 6% | nd | 7% | 17% | nd | 17% | 25% | nd | 29% |
| Spec 5 | 81% | nd | 81% | 81% | nd | 81% | 81% | nd | 81% |
| Cutoff 6 | 42.2 | nd | 42.2 | 42.2 | nd | 42.2 | 42.2 | nd | 42.2 |
| Sens 6 | 0% | nd | 0% | 12% | nd | 12% | 25% | nd | 29% |
| Spec 6 | 91% | nd | 92% | 91% | nd | 92% | 91% | nd | 92% |
| OR Quart 2 | 1.5 | nd | 2.5 | 1.4 | nd | 1.3 | 1.1 | nd | 0.46 |
| p Value | 0.61 | nd | 0.33 | 0.65 | nd | 0.72 | 0.94 | nd | 0.55 |
| 95% CI of | 0.29 | nd | 0.39 | 0.34 | nd | 0.31 | 0.13 | nd | 0.037 |
| OR Quart2 | 8.2 | nd | 17 | 5.6 | nd | 5.4 | 8.8 | nd | 5.8 |
| OR Quart 3 | 1.1 | nd | 1.6 | 1.1 | nd | 1.0 | 1.1 | nd | 1.0 |
| p Value | 0.94 | nd | 0.63 | 0.93 | nd | 1.0 | 0.94 | nd | 1.0 |
| 95% CI of | 0.18 | nd | 0.23 | 0.25 | nd | 0.23 | 0.13 | nd | 0.12 |
| OR Quart3 | 6.2 | nd | 11 | 4.5 | nd | 4.3 | 8.8 | nd | 8.3 |
| OR Quart 4 | 2.7 | nd | 3.5 | 2.2 | nd | 2.1 | 1.1 | nd | 1.1 |
| p Value | 0.22 | nd | 0.18 | 0.26 | nd | 0.30 | 0.94 | nd | 0.94 |
| 95% CI of | 0.56 | nd | 0.56 | 0.56 | nd | 0.52 | 0.13 | nd | 0.13 |
| OR Quart4 | 13 | nd | 22 | 8.5 | nd | 8.3 | 8.8 | nd | 9.1 |

**Cathepsin S**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.26 | 7.38 | 7.26 | 7.12 | 7.26 | 8.38 |
| Average | 7.64 | 7.90 | 7.64 | 8.44 | 7.64 | 8.82 |
| Stdev | 2.89 | 3.10 | 2.89 | 3.31 | 2.89 | 2.30 |
| p(t-test) | | 0.76 | | 0.29 | | 0.28 |
| Min | 4.02 | 3.54 | 4.02 | 4.40 | 4.02 | 5.64 |
| Max | 22.1 | 15.2 | 22.1 | 18.8 | 22.1 | 12.8 |
| n (Samp) | 53 | 16 | 53 | 24 | 53 | 8 |
| n (Patient) | 52 | 16 | 52 | 24 | 52 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.52 | 7.61 | 7.52 | 7.12 | 7.52 | 8.32 |
| Average | 7.91 | 7.95 | 7.91 | 8.00 | 7.91 | 8.87 |
| Stdev | 2.82 | 3.27 | 2.82 | 2.48 | 2.82 | 2.48 |
| p(t-test) | | 0.96 | | 0.89 | | 0.40 |
| Min | 4.02 | 3.54 | 4.02 | 4.40 | 4.02 | 5.64 |
| Max | 22.1 | 15.2 | 22.1 | 13.0 | 22.1 | 12.8 |
| n (Samp) | 48 | 14 | 48 | 24 | 48 | 7 |
| n (Patient) | 44 | 14 | 44 | 24 | 44 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | nd | 0.49 | 0.55 | nd | 0.49 | 0.70 | nd | 0.65 |
| SE | 0.083 | nd | 0.089 | 0.072 | nd | 0.073 | 0.11 | nd | 0.12 |
| p | 0.83 | nd | 0.88 | 0.46 | nd | 0.93 | 0.066 | nd | 0.21 |
| nCohort 1 | 53 | nd | 48 | 53 | nd | 48 | 53 | nd | 48 |
| nCohort 2 | 16 | nd | 14 | 24 | nd | 24 | 8 | nd | 7 |
| Cutoff 1 | 6.07 | nd | 6.07 | 6.56 | nd | 6.51 | 7.91 | nd | 7.91 |
| Sens 1 | 75% | nd | 71% | 71% | nd | 71% | 75% | nd | 71% |
| Spec 1 | 26% | nd | 23% | 38% | nd | 27% | 70% | nd | 62% |
| Cutoff 2 | 5.85 | nd | 5.48 | 6.07 | nd | 6.07 | 7.31 | nd | 7.31 |
| Sens 2 | 81% | nd | 86% | 83% | nd | 83% | 88% | nd | 86% |
| Spec 2 | 23% | nd | 15% | 26% | nd | 23% | 53% | nd | 44% |
| Cutoff 3 | 3.54 | nd | 3.54 | 5.02 | nd | 5.02 | 5.26 | nd | 5.48 |
| Sens 3 | 94% | nd | 93% | 92% | nd | 92% | 100% | nd | 100% |
| Spec 3 | 0% | nd | 0% | 15% | nd | 10% | 17% | nd | 15% |
| Cutoff 4 | 8.01 | nd | 8.27 | 8.01 | nd | 8.27 | 8.01 | nd | 8.27 |
| Sens 4 | 38% | nd | 36% | 33% | nd | 29% | 62% | nd | 57% |
| Spec 4 | 72% | nd | 71% | 72% | nd | 71% | 72% | nd | 71% |
| Cutoff 5 | 9.32 | nd | 9.73 | 9.32 | nd | 9.73 | 9.32 | nd | 9.73 |
| Sens 5 | 12% | nd | 14% | 33% | nd | 25% | 25% | nd | 29% |
| Spec 5 | 81% | nd | 81% | 81% | nd | 81% | 81% | nd | 81% |
| Cutoff 6 | 10.2 | nd | 10.4 | 10.2 | nd | 10.4 | 10.2 | nd | 10.4 |
| Sens 6 | 12% | nd | 14% | 29% | nd | 25% | 25% | nd | 29% |
| Spec 6 | 91% | nd | 92% | 91% | nd | 92% | 91% | nd | 92% |
| OR Quart 2 | 0.70 | nd | 0.75 | 1.6 | nd | 0.31 | 1.0 | nd | 0.92 |
| p Value | 0.67 | nd | 0.74 | 0.49 | nd | 0.15 | 1.0 | nd | 0.96 |

(continued)

| | Ohr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.13 | nd | 0.14 | 0.41 | nd | 0.066 | 0.057 | nd | 0.052 |
| OR Quart2 | 3.7 | nd | 4.1 | 6.5 | nd | 1.5 | 18 | nd | 16 |
| OR Quart 3 | 0.70 | nd | 0.69 | 0.75 | nd | 1.3 | 2.2 | nd | 3.3 |
| p Value | 0.67 | nd | 0.67 | 0.70 | nd | 0.74 | 0.55 | nd | 0.33 |
| 95% CI of | 0.13 | nd | 0.13 | 0.17 | nd | 0.33 | 0.17 | nd | 0.29 |
| OR Quart3 | 3.7 | nd | 3.8 | 3.4 | nd | 4.7 | 27 | nd | 36 |
| OR Quart 4 | 1.6 | nd | 1.1 | 1.9 | nd | 0.79 | 4.7 | nd | 2.0 |
| p Value | 0.52 | nd | 0.92 | 0.37 | nd | 0.73 | 0.19 | nd | 0.59 |
| 95% CI of | 0.37 | nd | 0.22 | 0.48 | nd | 0.20 | 0.46 | nd | 0.16 |
| OR Quart4 | 7.2 | nd | 5.5 | 7.3 | nd | 3.1 | 48 | nd | 25 |

**Galectin-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.57 | 3.02 | 2.57 | 3.28 | 2.57 | 3.75 |
| Average | 2.67 | 3.14 | 2.67 | 3.17 | 2.67 | 3.86 |
| Stdev | 1.21 | 1.45 | 1.21 | 1.53 | 1.21 | 1.75 |
| p(t-test) | | 0.20 | | 0.12 | | 0.018 |
| Min | 0.691 | 0.986 | 0.691 | 1.21 | 0.691 | 1.46 |
| Max | 5.62 | 6.11 | 5.62 | 7.71 | 5.62 | 6.59 |
| n (Samp) | 53 | 16 | 53 | 24 | 53 | 8 |
| n (Patient) | 52 | 16 | 52 | 24 | 52 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.70 | 3.32 | 2.70 | 3.28 | 2.70 | 4.18 |
| Average | 2.70 | 3.26 | 2.70 | 3.17 | 2.70 | 3.93 |
| Stdev | 1.31 | 1.47 | 1.31 | 1.53 | 1.31 | 1.88 |
| p(t-test) | | 0.18 | | 0.18 | | 0.032 |
| Min | 0.589 | 0.986 | 0.589 | 1.21 | 0.589 | 1.46 |
| Max | 5.62 | 6.11 | 5.62 | 7.71 | 5.62 | 6.59 |
| n (Samp) | 48 | 14 | 48 | 24 | 48 | 7 |
| n (Patient) | 44 | 14 | 44 | 24 | 44 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.61 | 0.59 | nd | 0.60 | 0.70 | nd | 0.68 |
| SE | 0.084 | nd | 0.089 | 0.072 | nd | 0.073 | 0.11 | nd | 0.12 |
| p | 0.24 | nd | 0.21 | 0.19 | nd | 0.19 | 0.074 | nd | 0.12 |
| nCohort 1 | 53 | nd | 48 | 53 | nd | 48 | 53 | nd | 48 |
| nCohort 2 | 16 | nd | 14 | 24 | nd | 24 | 8 | nd | 7 |
| Cutoff 1 | 2.16 | nd | 2.40 | 2.16 | nd | 2.16 | 2.43 | nd | 2.43 |
| Sens 1 | 75% | nd | 71% | 71% | nd | 71% | 75% | nd | 71% |
| Spec 1 | 36% | nd | 42% | 36% | nd | 33% | 45% | nd | 42% |
| Cutoff 2 | 1.65 | nd | 1.65 | 1.52 | nd | 1.52 | 2.40 | nd | 2.40 |
| Sens 2 | 81% | nd | 86% | 83% | nd | 83% | 88% | nd | 86% |
| Spec 2 | 25% | nd | 29% | 23% | nd | 27% | 45% | nd | 42% |
| Cutoff 3 | 1.13 | nd | 1.14 | 1.33 | nd | 1.33 | 1.26 | nd | 1.26 |
| Sens 3 | 94% | nd | 93% | 92% | nd | 92% | 100% | nd | 100% |
| Spec 3 | 15% | nd | 19% | 19% | nd | 23% | 19% | nd | 23% |
| Cutoff 4 | 3.41 | nd | 3.41 | 3.41 | nd | 3.41 | 3.41 | nd | 3.41 |
| Sens 4 | 44% | nd | 50% | 42% | nd | 42% | 50% | nd | 57% |
| Spec 4 | 72% | nd | 71% | 72% | nd | 71% | 72% | nd | 71% |
| Cutoff 5 | 3.70 | nd | 3.77 | 3.70 | nd | 3.77 | 3.70 | nd | 3.77 |
| Sens 5 | 38% | nd | 43% | 33% | nd | 29% | 50% | nd | 57% |
| Spec 5 | 81% | nd | 81% | 81% | nd | 81% | 81% | nd | 81% |
| Cutoff 6 | 4.35 | nd | 4.40 | 4.35 | nd | 4.40 | 4.35 | nd | 4.40 |
| Sens 6 | 19% | nd | 14% | 17% | nd | 17% | 38% | nd | 43% |
| Spec 6 | 91% | nd | 92% | 91% | nd | 92% | 91% | nd | 92% |
| OR Quart 2 | 0.43 | nd | 1.5 | 0.41 | nd | 1.0 | 2.2 | nd | 2.0 |
| p Value | 0.38 | nd | 0.68 | 0.26 | nd | 1.0 | 0.55 | nd | 0.59 |
| 95% CI of | 0.068 | nd | 0.21 | 0.085 | nd | 0.23 | 0.17 | nd | 0.16 |
| OR Quart2 | 2.8 | nd | 11 | 1.9 | nd | 4.3 | 27 | nd | 25 |
| OR Quart 3 | 1.0 | nd | 1.6 | 1.3 | nd | 1.3 | 1.0 | nd | 0 |
| p Value | 1.0 | nd | 0.63 | 0.73 | nd | 0.72 | 1.0 | nd | na |
| 95% CI of | 0.20 | nd | 0.23 | 0.33 | nd | 0.31 | 0.057 | nd | na |
| OR Quart3 | 4.9 | nd | 11 | 4.8 | nd | 5.4 | 18 | nd | na |
| OR Quart 4 | 1.6 | nd | 3.9 | 1.4 | nd | 2.1 | 4.7 | nd | 4.8 |
| p Value | 0.52 | nd | 0.14 | 0.58 | nd | 0.30 | 0.19 | nd | 0.19 |
| 95% CI of | 0.37 | nd | 0.64 | 0.39 | nd | 0.52 | 0.46 | nd | 0.46 |
| OR Quart4 | 7.2 | nd | 24 | 5.4 | nd | 8.3 | 48 | nd | 50 |

**C-C motif chemokine 23**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.47 | 1.96 | 1.47 | 2.28 | 1.47 | 2.42 |
| Average | 1.69 | 2.56 | 1.69 | 2.51 | 1.69 | 2.74 |
| Stdev | 1.13 | 2.66 | 1.13 | 2.36 | 1.13 | 2.10 |
| p(t-test) | | 0.059 | | 0.042 | | 0.034 |
| Min | 0.00620 | 0.00425 | 0.00620 | 0.00626 | 0.00620 | 0.0116 |
| Max | 4.53 | 10.3 | 4.53 | 10.3 | 4.53 | 6.24 |
| n (Samp) | 55 | 16 | 55 | 24 | 55 | 8 |
| n (Patient) | 54 | 16 | 54 | 24 | 54 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.76 | 1.94 | 1.76 | 2.28 | 1.76 | 2.00 |
| Average | 1.94 | 2.38 | 1.94 | 2.58 | 1.94 | 2.41 |
| Stdev | 1.22 | 2.79 | 1.22 | 2.41 | 1.22 | 2.03 |
| p(t-test) | | 0.39 | | 0.13 | | 0.39 |
| Min | 0.00620 | 0.00425 | 0.00620 | 0.00626 | 0.00620 | 0.0116 |
| Max | 5.09 | 10.3 | 5.09 | 10.3 | 5.09 | 6.24 |
| n (Samp) | 49 | 14 | 49 | 24 | 49 | 7 |
| n (Patient) | 45 | 14 | 45 | 24 | 45 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | nd | 0.47 | 0.59 | nd | 0.55 | 0.65 | nd | 0.55 |
| SE | 0.084 | nd | 0.089 | 0.071 | nd | 0.073 | 0.11 | nd | 0.12 |
| p | 0.45 | nd | 0.69 | 0.23 | nd | 0.47 | 0.19 | nd | 0.69 |
| nCohort 1 | 55 | nd | 49 | 55 | nd | 49 | 55 | nd | 49 |
| nCohort 2 | 16 | nd | 14 | 24 | nd | 24 | 8 | nd | 7 |
| Cutoff 1 | 0.815 | nd | 0.815 | 1.02 | nd | 0.941 | 1.40 | nd | 1.40 |
| Sens 1 | 75% | nd | 71% | 71% | nd | 71% | 75% | nd | 71% |
| Spec 1 | 31% | nd | 20% | 36% | nd | 22% | 49% | nd | 41% |
| Cutoff 2 | 0.682 | nd | 0.00876 | 0.449 | nd | 0.449 | 0.978 | nd | 0.941 |
| Sens 2 | 81% | nd | 86% | 83% | nd | 83% | 88% | nd | 86% |
| Spec 2 | 18% | nd | 4% | 13% | nd | 12% | 35% | nd | 22% |
| Cutoff 3 | 0.00425 | nd | 0.00425 | 0.0152 | nd | 0.0152 | 0.00620 | nd | 0.00876 |
| Sens 3 | 94% | nd | 93% | 92% | nd | 92% | 100% | nd | 100% |
| Spec 3 | 0% | nd | 0% | 2% | nd | 4% | 2% | nd | 4% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 2.29 | nd | 2.46 | 2.29 | nd | 2.46 | 2.29 | nd | 2.46 |
| Sens 4 | 44% | nd | 21% | 50% | nd | 46% | 50% | nd | 43% |
| Spec 4 | 71% | nd | 71% | 71% | nd | 71% | 71% | nd | 71% |
| Cutoff 5 | 2.58 | nd | 2.94 | 2.58 | nd | 2.94 | 2.58 | nd | 2.94 |
| Sens 5 | 31% | nd | 21% | 46% | nd | 38% | 50% | nd | 29% |
| Spec 5 | 80% | nd | 82% | 80% | nd | 82% | 80% | nd | 82% |
| Cutoff 6 | 3.32 | nd | 3.85 | 3.32 | nd | 3.85 | 3.32 | nd | 3.85 |
| Sens 6 | 31% | nd | 14% | 29% | nd | 25% | 38% | nd | 14% |
| Spec 6 | 91% | nd | 92% | 91% | nd | 92% | 91% | nd | 92% |
| OR Quart 2 | 0.41 | nd | 1.4 | 0.72 | nd | 0.45 | 2.0 | nd | 0.46 |
| p Value | 0.34 | nd | 0.67 | 0.65 | nd | 0.28 | 0.59 | nd | 0.55 |
| 95% CI of | 0.064 | nd | 0.27 | 0.18 | nd | 0.10 | 0.16 | nd | 0.037 |
| OR Quart2 | 2.6 | nd | 7.8 | 2.9 | nd | 1.9 | 25 | nd | 5.8 |
| OR Quart 3 | 1.2 | nd | 0.62 | 0.38 | nd | 0.45 | 0.93 | nd | 0.46 |
| p Value | 0.77 | nd | 0.63 | 0.23 | nd | 0.28 | 0.96 | nd | 0.55 |
| 95% CI of | 0.27 | nd | 0.089 | 0.080 | nd | 0.10 | 0.053 | nd | 0.037 |
| OR Quart3 | 5.7 | nd | 4.3 | 1.8 | nd | 1.9 | 16 | nd | 5.8 |
| OR Quart 4 | 1.2 | nd | 2.2 | 2.2 | nd | 1.4 | 4.7 | nd | 1.6 |
| p Value | 0.77 | nd | 0.36 | 0.25 | nd | 0.60 | 0.19 | nd | 0.62 |
| 95% CI of | 0.27 | nd | 0.42 | 0.59 | nd | 0.38 | 0.46 | nd | 0.23 |
| OR Quart4 | 5.7 | nd | 11 | 8.0 | nd | 5.2 | 48 | nd | 12 |

**Insulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.81 | 3.19 | 2.81 | 2.54 | 2.81 | 2.55 |
| Average | 5.55 | 7.03 | 5.55 | 5.77 | 5.55 | 5.39 |
| Stdev | 8.27 | 10.6 | 8.27 | 7.31 | 8.27 | 10.3 |
| p(t-test) | | 0.26 | | 0.85 | | 0.93 |
| Min | 0.00859 | 0.00859 | 0.00859 | 0.00859 | 0.00859 | 0.00859 |
| Max | 87.2 | 51.7 | 87.2 | 39.2 | 87.2 | 41.9 |
| n (Samp) | 263 | 51 | 263 | 56 | 263 | 26 |
| n (Patient) | 111 | 51 | 111 | 56 | 111 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.81 | 2.18 | 2.81 | 3.65 | 2.81 | 3.26 |
| Average | 5.82 | 5.53 | 5.82 | 11.3 | 5.82 | 6.75 |
| Stdev | 10.7 | 6.81 | 10.7 | 25.6 | 10.7 | 11.3 |
| p(t-test) | | 0.91 | | 0.038 | | 0.76 |
| Min | 0.00859 | 0.253 | 0.00859 | 0.583 | 0.00859 | 0.483 |
| Max | 162 | 19.8 | 162 | 120 | 162 | 41.9 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.93 | 3.72 | 2.93 | 2.23 | 2.93 | 1.74 |
| Average | 5.96 | 7.15 | 5.96 | 5.69 | 5.96 | 3.69 |
| Stdev | 8.86 | 10.6 | 8.86 | 9.65 | 8.86 | 7.68 |
| p(t-test) | | 0.41 | | 0.85 | | 0.24 |
| Min | 0.00859 | 0.00859 | 0.00859 | 0.00859 | 0.00859 | 0.00859 |
| Max | 87.2 | 51.7 | 87.2 | 53.1 | 87.2 | 38.1 |
| n (Samp) | 221 | 50 | 221 | 52 | 221 | 23 |
| n (Patient) | 91 | 50 | 91 | 52 | 91 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.45 | 0.51 | 0.50 | 0.56 | 0.44 | 0.44 | 0.51 | 0.36 |
| SE | 0.044 | 0.071 | 0.045 | 0.043 | 0.066 | 0.045 | 0.061 | 0.082 | 0.065 |
| p | 0.80 | 0.52 | 0.81 | 1.00 | 0.37 | 0.19 | 0.33 | 0.92 | 0.029 |
| nCohort 1 | 263 | 466 | 221 | 263 | 466 | 221 | 263 | 466 | 221 |
| nCohort 2 | 51 | 18 | 50 | 56 | 21 | 52 | 26 | 13 | 23 |
| Cutoff 1 | 1.41 | 0.554 | 1.41 | 1.58 | 2.00 | 0.976 | 1.37 | 1.55 | 0.871 |
| Sens 1 | 71% | 72% | 70% | 71% | 71% | 71% | 73% | 77% | 74% |
| Spec 1 | 27% | 9% | 25% | 31% | 38% | 17% | 26% | 29% | 15% |
| Cutoff 2 | 1.03 | 0.389 | 1.07 | 0.784 | 1.19 | 0.777 | 0.871 | 1.40 | 0.647 |
| Sens 2 | 80% | 83% | 80% | 80% | 81% | 81% | 81% | 85% | 83% |
| Spec 2 | 20% | 6% | 21% | 13% | 22% | 13% | 15% | 26% | 10% |
| Cutoff 3 | 0.328 | 0.253 | 0.179 | 0.583 | 0.611 | 0.328 | 0.481 | 0.658 | 0.481 |
| Sens 3 | 90% | 94% | 90% | 91% | 90% | 90% | 92% | 92% | 91% |
| Spec 3 | 6% | 5% | 4% | 9% | 9% | 6% | 7% | 10% | 7% |
| Cutoff 4 | 4.85 | 5.02 | 5.19 | 4.85 | 5.02 | 5.19 | 4.85 | 5.02 | 5.19 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 4 | 33% | 28% | 36% | 36% | 43% | 31% | 15% | 23% | 9% |
| Spec 4 | 70% | 70% | 70% | 70% | 70% | 70% | 70% | 70% | 70% |
| Cutoff 5 | 7.49 | 7.78 | 9.23 | 7.49 | 7.78 | 9.23 | 7.49 | 7.78 | 9.23 |
| Sens 5 | 27% | 28% | 26% | 29% | 29% | 15% | 8% | 15% | 4% |
| Spec 5 | 80% | 80% | 80% | 80% | 80% | 80% | 80% | 80% | 80% |
| Cutoff 6 | 13.9 | 13.7 | 14.5 | 13.9 | 13.7 | 14.5 | 13.9 | 13.7 | 14.5 |
| Sens 6 | 14% | 17% | 12% | 12% | 19% | 8% | 8% | 15% | 4% |
| Spec 6 | 90% | 90% | 90% | 90% | 90% | 90% | 90% | 90% | 90% |
| OR Quart2 | 0.90 | 0.59 | 0.80 | 0.38 | 0.79 | 0.72 | 2.2 | 1.5 | 2.6 |
| p Value | 0.80 | 0.48 | 0.62 | 0.036 | 0.73 | 0.50 | 0.23 | 0.66 | 0.26 |
| 95% CI of | 0.38 | 0.14 | 0.33 | 0.16 | 0.21 | 0.28 | 0.62 | 0.25 | 0.49 |
| OR Quart2 | 2.1 | 2.5 | 1.9 | 0.94 | 3.0 | 1.9 | 7.5 | 9.1 | 14 |
| OR Quart3 | 0.82 | 0.79 | 0.89 | 0.86 | 0.99 | 1.3 | 1.9 | 3.1 | 4.5 |
| p Value | 0.66 | 0.73 | 0.79 | 0.70 | 0.99 | 0.49 | 0.34 | 0.17 | 0.066 |
| 95% CI of | 0.34 | 0.21 | 0.37 | 0.40 | 0.28 | 0.58 | 0.52 | 0.61 | 0.91 |
| OR Quart3 | 2.0 | 3.0 | 2.1 | 1.8 | 3.5 | 3.1 | 6.6 | 16 | 22 |
| OR Quart4 | 1.2 | 1.2 | 1.1 | 0.74 | 1.4 | 1.5 | 1.9 | 0.99 | 4.5 |
| p Value | 0.70 | 0.76 | 0.86 | 0.45 | 0.57 | 0.37 | 0.34 | 0.99 | 0.066 |
| 95% CI of | 0.52 | 0.36 | 0.46 | 0.34 | 0.44 | 0.63 | 0.52 | 0.14 | 0.91 |
| OR Quart4 | 2.7 | 4.1 | 2.5 | 1.6 | 4.6 | 3.4 | 6.6 | 7.2 | 22 |

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Angiopoietin-2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1500 | 2080 | 1500 | 2150 | 1500 | 1890 |
| Average | 1990 | 2440 | 1990 | 4050 | 1990 | 2020 |
| Stdev | 1570 | 1960 | 1570 | 8490 | 1570 | 921 |
| p(t-test) |  | 0.46 |  | 0.0036 |  | 0.93 |
| Min | 219 | 569 | 219 | 792 | 219 | 708 |
| Max | 10500 | 6410 | 10500 | 39900 | 10500 | 3530 |
| n (Samp) | 189 | 7 | 189 | 20 | 189 | 15 |
| n (Patient) | 129 | 7 | 129 | 20 | 129 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1620 | 2410 | 1620 | 2410 |
| Average | nd | nd | 2110 | 4890 | 2110 | 2310 |
| Stdev | nd | nd | 1660 | 9110 | 1660 | 839 |
| p(t-test) | nd | nd | | 7.0E-4 | | 0.68 |
| Min | nd | nd | 219 | 1190 | 219 | 1100 |
| Max | nd | nd | 10500 | 39900 | 10500 | 3530 |
| n (Samp) | nd | nd | 163 | 17 | 163 | 12 |
| n (Patient) | nd | nd | 107 | 17 | 107 | 12 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | nd | 0.65 | nd | 0.72 | 0.58 | nd | 0.66 |
| SE | 0.12 | nd | nd | 0.069 | nd | 0.072 | 0.080 | nd | 0.089 |
| p | 0.44 | nd | nd | 0.030 | nd | 0.0022 | 0.31 | nd | 0.079 |
| nCohort 1 | 189 | nd | nd | 189 | nd | 163 | 189 | nd | 163 |
| nCohort 2 | 7 | nd | nd | 20 | nd | 17 | 15 | nd | 12 |
| Cutoff 1 | 1970 | nd | nd | 1530 | nd | 2010 | 1410 | nd | 1840 |
| Sens 1 | 71% | nd | nd | 70% | nd | 71% | 73% | nd | 75% |
| Spec 1 | 70% | nd | nd | 52% | nd | 69% | 44% | nd | 63% |
| Cutoff 2 | 624 | nd | nd | 1450 | nd | 1530 | 1150 | nd | 1410 |
| Sens 2 | 86% | nd | nd | 80% | nd | 82% | 80% | nd | 83% |
| Spec 2 | 2% | nd | nd | 46% | nd | 48% | 27% | nd | 40% |
| Cutoff 3 | 512 | nd | nd | 1190 | nd | 1370 | 832 | nd | 1150 |
| Sens 3 | 100% | nd | nd | 90% | nd | 94% | 93% | nd | 92% |
| Spec 3 | 1% | nd | nd | 28% | nd | 39% | 7% | nd | 24% |
| Cutoff 4 | 1970 | nd | nd | 1970 | nd | 2090 | 1970 | nd | 2090 |
| Sens 4 | 71% | nd | nd | 60% | nd | 65% | 47% | nd | 50% |
| Spec 4 | 70% | nd | nd | 70% | nd | 71% | 70% | nd | 71% |
| Cutoff 5 | 2430 | nd | nd | 2430 | nd | 2480 | 2430 | nd | 2480 |
| Sens 5 | 43% | nd | nd | 35% | nd | 47% | 40% | nd | 50% |
| Spec 5 | 80% | nd | nd | 80% | nd | 80% | 80% | nd | 80% |
| Cutoff 6 | 3500 | nd | nd | 3500 | nd | 3850 | 3500 | nd | 3850 |
| Sens 6 | 14% | nd | nd | 10% | nd | 18% | 7% | nd | 0% |
| Spec 6 | 90% | nd | nd | 90% | nd | 90% | 90% | nd | 90% |
| OR Quart 2 | 0 | nd | nd | 2.1 | nd | 3.1 | 1.0 | nd | 0.48 |
| p Value | na | nd | nd | 0.41 | nd | 0.33 | 1.0 | nd | 0.55 |
| 95% CI of | na | nd | nd | 0.36 | nd | 0.31 | 0.19 | nd | 0.042 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | na | nd | nd | 12 | nd | 31 | 5.2 | nd | 5.5 |
| OR Quart 3 | 1.0 | nd | nd | 2.1 | nd | 4.3 | 1.0 | nd | 1.5 |
| p Value | 1.0 | nd | nd | 0.41 | nd | 0.20 | 1.0 | nd | 0.67 |
| 95% CI of | 0.14 | nd | nd | 0.36 | nd | 0.46 | 0.19 | nd | 0.24 |
| OR Quart3 | 7.4 | nd | nd | 12 | nd | 40 | 5.2 | nd | 9.5 |
| OR Quart 4 | 1.5 | nd | nd | 5.8 | nd | 11 | 2.1 | nd | 3.2 |
| p Value | 0.65 | nd | nd | 0.028 | nd | 0.026 | 0.30 | nd | 0.17 |
| 95% CI of | 0.24 | nd | nd | 1.2 | nd | 1.3 | 0.50 | nd | 0.62 |
| OR Quart4 | 9.6 | nd | nd | 28 | nd | 91 | 9.0 | nd | 17 |

**Brain-derived neurotrophic factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1930 | 813 | 1930 | 1270 | 1930 | 1630 |
| Average | 2790 | 1410 | 2790 | 3100 | 2790 | 2060 |
| Stdev | 2820 | 1800 | 2820 | 6970 | 2820 | 1590 |
| p(t-test) |  | 0.20 |  | 0.70 |  | 0.31 |
| Min | 1.00E-9 | 349 | 1.00E-9 | 283 | 1.00E-9 | 118 |
| Max | 19100 | 5440 | 19100 | 33100 | 19100 | 5570 |
| n (Samp) | 196 | 7 | 196 | 21 | 196 | 16 |
| n (Patient) | 131 | 7 | 131 | 21 | 131 | 16 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1760 | 1250 | 1760 | 1080 |
| Average | nd | nd | 2530 | 1720 | 2530 | 1860 |
| Stdev | nd | nd | 2740 | 1640 | 2740 | 1640 |
| p(t-test) | nd | nd |  | 0.22 |  | 0.38 |
| Min | nd | nd | 1.00E-9 | 283 | 1.00E-9 | 118 |
| Max | nd | nd | 19100 | 6290 | 19100 | 5570 |
| n (Samp) | nd | nd | 170 | 18 | 170 | 13 |
| n (Patient) | nd | nd | 109 | 18 | 109 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | nd | nd | 0.42 | nd | 0.42 | 0.46 | nd | 0.45 |
| SE | 0.11 | nd | nd | 0.068 | nd | 0.074 | 0.077 | nd | 0.085 |
| p | 0.12 | nd | nd | 0.22 | nd | 0.26 | 0.58 | nd | 0.57 |
| nCohort 1 | 196 | nd | nd | 196 | nd | 170 | 196 | nd | 170 |
| nCohort 2 | 7 | nd | nd | 21 | nd | 18 | 16 | nd | 13 |
| Cutoff 1 | 792 | nd | nd | 792 | nd | 542 | 909 | nd | 828 |
| Sens 1 | 71% | nd | nd | 71% | nd | 72% | 75% | nd | 77% |
| Spec 1 | 26% | nd | nd | 26% | nd | 19% | 29% | nd | 29% |
| Cutoff 2 | 393 | nd | nd | 542 | nd | 393 | 828 | nd | 542 |
| Sens 2 | 86% | nd | nd | 81% | nd | 83% | 81% | nd | 85% |
| Spec 2 | 14% | nd | nd | 17% | nd | 15% | 27% | nd | 19% |
| Cutoff 3 | 330 | nd | nd | 302 | nd | 296 | 259 | nd | 259 |
| Sens 3 | 100% | nd | nd | 90% | nd | 94% | 94% | nd | 92% |
| Spec 3 | 12% | nd | nd | 11% | nd | 12% | 9% | nd | 11% |
| Cutoff 4 | 3210 | nd | nd | 3210 | nd | 2950 | 3210 | nd | 2950 |
| Sens 4 | 14% | nd | nd | 19% | nd | 17% | 25% | nd | 31% |
| Spec 4 | 71% | nd | nd | 71% | nd | 70% | 71% | nd | 70% |
| Cutoff 5 | 4350 | nd | nd | 4350 | nd | 3710 | 4350 | nd | 3710 |
| Sens 5 | 14% | nd | nd | 5% | nd | 11% | 6% | nd | 15% |
| Spec 5 | 80% | nd | nd | 80% | nd | 80% | 80% | nd | 80% |
| Cutoff 6 | 6170 | nd | nd | 6170 | nd | 5790 | 6170 | nd | 5790 |
| Sens 6 | 0% | nd | nd | 5% | nd | 6% | 0% | nd | 0% |
| Spec 6 | 90% | nd | nd | 90% | nd | 90% | 90% | nd | 90% |
| OR Quart 2 | 0 | nd | nd | 0.75 | nd | 1.4 | 1.4 | nd | 0.65 |
| p Value | na | nd | nd | 0.72 | nd | 0.70 | 0.70 | nd | 0.65 |
| 95% CI of | na | nd | nd | 0.16 | nd | 0.29 | 0.29 | nd | 0.10 |
| OR Quart2 | na | nd | nd | 3.5 | nd | 6.5 | 6.4 | nd | 4.1 |
| OR Quart 3 | 4.3 | nd | nd | 2.2 | nd | 1.7 | 2.1 | nd | 1.7 |
| p Value | 0.20 | nd | nd | 0.22 | nd | 0.46 | 0.30 | nd | 0.46 |
| 95% CI of | 0.46 | nd | nd | 0.63 | nd | 0.39 | 0.50 | nd | 0.39 |
| OR Quart3 | 39 | nd | nd | 7.9 | nd | 7.8 | 9.0 | nd | 7.8 |
| OR Quart 4 | 2.1 | nd | nd | 1.6 | nd | 2.1 | 1.0 | nd | 1.0 |
| p Value | 0.55 | nd | nd | 0.49 | nd | 0.30 | 1.0 | nd | 0.98 |
| 95% CI of | 0.18 | nd | nd | 0.42 | nd | 0.50 | 0.19 | nd | 0.20 |
| OR Quart4 | 24 | nd | nd | 6.0 | nd | 9.1 | 5.2 | nd | 5.4 |

**Creatine Kinase-MB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.263 | 0.329 | 0.263 | 0.361 | 0.263 | 0.294 |
| Average | 0.654 | 0.602 | 0.654 | 0.630 | 0.654 | 0.450 |
| Stdev | 1.87 | 1.09 | 1.87 | 0.739 | 1.87 | 0.400 |
| p(t-test) | | 0.89 | | 0.94 | | 0.65 |
| Min | 0.00420 | 0.00420 | 0.00420 | 0.0597 | 0.00420 | 0.0721 |
| Max | 32.5 | 5.63 | 32.5 | 2.81 | 32.5 | 1.26 |
| n (Samp) | 437 | 26 | 437 | 33 | 437 | 17 |
| n (Patient) | 174 | 26 | 174 | 33 | 174 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.264 | 0.550 | 0.264 | 0.392 | 0.264 | 1.20 |
| Average | 0.632 | 0.713 | 0.632 | 0.790 | 0.632 | 1.95 |
| Stdev | 1.73 | 0.618 | 1.73 | 0.908 | 1.73 | 3.03 |
| p(t-test) | | 0.91 | | 0.78 | | 0.048 |
| Min | 0.00420 | 0.143 | 0.00420 | 0.0597 | 0.00420 | 0.0849 |
| Max | 32.5 | 1.73 | 32.5 | 2.75 | 32.5 | 8.69 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.272 | 0.359 | 0.272 | 0.397 | 0.272 | 0.294 |
| Average | 0.722 | 0.627 | 0.722 | 0.628 | 0.722 | 0.482 |
| Stdev | 2.04 | 1.10 | 2.04 | 0.732 | 2.04 | 0.468 |
| p(t-test) | | 0.82 | | 0.80 | | 0.63 |
| Min | 0.00420 | 0.00420 | 0.00420 | 0.0597 | 0.00420 | 0.0721 |
| Max | 32.5 | 5.63 | 32.5 | 2.81 | 32.5 | 1.68 |
| n (Samp) | 362 | 26 | 362 | 31 | 362 | 17 |
| n (Patient) | 140 | 26 | 140 | 31 | 140 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.65 | 0.52 | 0.55 | 0.57 | 0.55 | 0.53 | 0.70 | 0.52 |
| SE | 0.059 | 0.12 | 0.059 | 0.053 | 0.10 | 0.055 | 0.072 | 0.11 | 0.072 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.77 | 0.21 | 0.76 | 0.31 | 0.51 | 0.36 | 0.72 | 0.080 | 0.82 |
| nCohort 1 | 437 | 535 | 362 | 437 | 535 | 362 | 437 | 535 | 362 |
| nCohort 2 | 26 | 6 | 26 | 33 | 9 | 31 | 17 | 7 | 17 |
| Cutoff 1 | 0.136 | 0.166 | 0.136 | 0.132 | 0.0911 | 0.244 | 0.131 | 0.602 | 0.141 |
| Sens 1 | 73% | 83% | 73% | 73% | 78% | 71% | 71% | 71% | 71% |
| Spec 1 | 26% | 33% | 25% | 26% | 13% | 46% | 25% | 79% | 25% |
| Cutoff 2 | 0.114 | 0.166 | 0.114 | 0.0910 | 0.0790 | 0.112 | 0.120 | 0.120 | 0.124 |
| Sens 2 | 81% | 83% | 81% | 85% | 89% | 81% | 88% | 86% | 82% |
| Spec 2 | 21% | 33% | 19% | 13% | 11% | 18% | 22% | 22% | 23% |
| Cutoff 3 | 0.0740 | 0.141 | 0.0740 | 0.0790 | 0.0594 | 0.0842 | 0.108 | 0.0842 | 0.108 |
| Sens 3 | 92% | 100% | 92% | 91% | 100% | 90% | 94% | 100% | 94% |
| Spec 3 | 10% | 27% | 9% | 11% | 5% | 11% | 19% | 12% | 17% |
| Cutoff 4 | 0.472 | 0.472 | 0.498 | 0.472 | 0.472 | 0.498 | 0.472 | 0.472 | 0.498 |
| Sens 4 | 35% | 50% | 35% | 36% | 44% | 39% | 41% | 71% | 35% |
| Spec 4 | 70% | 70% | 70% | 70% | 70% | 70% | 70% | 70% | 70% |
| Cutoff 5 | 0.625 | 0.625 | 0.645 | 0.625 | 0.625 | 0.645 | 0.625 | 0.625 | 0.645 |
| Sens 5 | 19% | 50% | 15% | 33% | 44% | 32% | 24% | 57% | 29% |
| Spec 5 | 80% | 80% | 80% | 80% | 80% | 80% | 80% | 80% | 80% |
| Cutoff 6 | 1.23 | 1.23 | 1.42 | 1.23 | 1.23 | 1.42 | 1.23 | 1.23 | 1.42 |
| Sens 6 | 12% | 17% | 8% | 15% | 22% | 10% | 6% | 43% | 6% |
| Spec 6 | 90% | 90% | 90% | 90% | 90% | 90% | 90% | 90% | 90% |
| OR Quart 2 | 0.55 | >2.0 | 0.55 | 0.31 | 0 | 0.31 | 0.58 | 0 | 0.58 |
| p Value | 0.35 | <0.57 | 0.36 | 0.088 | na | 0.088 | 0.47 | na | 0.47 |
| 95% CI of | 0.16 | >0.18 | 0.16 | 0.083 | na | 0.082 | 0.14 | na | 0.13 |
| OR Quart2 | 1.9 | na | 2.0 | 1.2 | na | 1.2 | 2.5 | na | 2.5 |
| OR Quart 3 | 1.1 | >1.0 | 1.2 | 1.0 | 0.66 | 1.0 | 0.59 | 0 | 0.78 |
| p Value | 0.80 | <1.00 | 0.79 | 1.0 | 0.65 | 1.0 | 0.48 | na | 0.72 |
| 95% CI of | 0.40 | >0.062 | 0.40 | 0.38 | 0.11 | 0.38 | 0.14 | na | 0.20 |
| OR Quart3 | 3.3 | na | 3.3 | 2.6 | 4.0 | 2.6 | 2.5 | na | 3.0 |
| OR Quart 4 | 0.99 | >3.0 | 1.0 | 1.4 | 1.3 | 1.1 | 1.2 | 2.5 | 0.99 |
| p Value | 0.99 | <0.34 | 1.0 | 0.51 | 0.70 | 0.83 | 0.77 | 0.27 | 0.99 |
| 95% CI of | 0.34 | >0.31 | 0.34 | 0.55 | 0.29 | 0.43 | 0.36 | 0.48 | 0.28 |
| OR Quart4 | 2.9 | na | 3.0 | 3.4 | 6.1 | 2.9 | 4.1 | 13 | 3.5 |

**Immunoglobulin M**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.534 | 0.613 | 0.534 | 0.683 | 0.534 | 0.682 |
| Average | 0.764 | 0.744 | 0.764 | 0.837 | 0.764 | 0.822 |
| Stdev | 0.676 | 0.599 | 0.676 | 0.743 | 0.676 | 0.745 |
| p(t-test) | | 0.88 | | 0.55 | | 0.73 |
| Min | 0.0493 | 0.142 | 0.0493 | 0.142 | 0.0493 | 0.0968 |
| Max | 4.82 | 2.85 | 4.82 | 3.51 | 4.82 | 3.12 |
| n (Samp) | 437 | 26 | 437 | 33 | 437 | 17 |
| n (Patient) | 174 | 26 | 174 | 33 | 174 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.541 | 0.667 | 0.541 | 0.774 | 0.541 | 1.01 |
| Average | 0.769 | 0.868 | 0.769 | 0.973 | 0.769 | 1.04 |
| Stdev | 0.684 | 0.565 | 0.684 | 0.758 | 0.684 | 0.952 |
| p(t-test) | | 0.73 | | 0.38 | | 0.31 |
| Min | 0.0493 | 0.240 | 0.0493 | 0.142 | 0.0493 | 0.0968 |
| Max | 4.82 | 1.85 | 4.82 | 2.66 | 4.82 | 2.89 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.508 | 0.524 | 0.508 | 0.614 | 0.508 | 0.682 |
| Average | 0.735 | 0.733 | 0.735 | 0.806 | 0.735 | 0.823 |
| Stdev | 0.657 | 0.601 | 0.657 | 0.754 | 0.657 | 0.728 |
| p(t-test) | | 0.99 | | 0.56 | | 0.59 |
| Min | 0.0815 | 0.142 | 0.0815 | 0.142 | 0.0815 | 0.231 |
| Max | 4.82 | 2.85 | 4.82 | 3.51 | 4.82 | 3.12 |
| n (Samp) | 362 | 26 | 362 | 31 | 362 | 17 |
| n (Patient) | 140 | 26 | 140 | 31 | 140 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.61 | 0.52 | 0.54 | 0.62 | 0.54 | 0.52 | 0.57 | 0.55 |
| SE | 0.059 | 0.12 | 0.059 | 0.053 | 0.10 | 0.055 | 0.072 | 0.11 | 0.073 |
| p | 0.82 | 0.37 | 0.78 | 0.42 | 0.25 | 0.49 | 0.80 | 0.56 | 0.51 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 437 | 535 | 362 | 437 | 535 | 362 | 437 | 535 | 362 |
| nCohort 2 | 26 | 6 | 26 | 33 | 9 | 31 | 17 | 7 | 17 |
| Cutoff 1 | 0.313 | 0.613 | 0.346 | 0.395 | 0.522 | 0.346 | 0.353 | 0.497 | 0.387 |
| Sens 1 | 73% | 83% | 73% | 73% | 78% | 71% | 71% | 71% | 71% |
| Spec 1 | 22% | 56% | 28% | 31% | 47% | 28% | 27% | 44% | 32% |
| Cutoff 2 | 0.260 | 0.613 | 0.272 | 0.290 | 0.428 | 0.290 | 0.231 | 0.229 | 0.273 |
| Sens 2 | 81% | 83% | 81% | 82% | 89% | 81% | 82% | 86% | 82% |
| Spec 2 | 18% | 56% | 19% | 20% | 36% | 22% | 12% | 11% | 20% |
| Cutoff 3 | 0.229 | 0.238 | 0.229 | 0.273 | 0.141 | 0.273 | 0.229 | 0.0967 | 0.231 |
| Sens 3 | 96% | 100% | 96% | 91% | 100% | 90% | 94% | 100% | 94% |
| Spec 3 | 12% | 14% | 13% | 19% | 3% | 20% | 12% | 1% | 13% |
| Cutoff 4 | 0.794 | 0.831 | 0.746 | 0.794 | 0.831 | 0.746 | 0.794 | 0.831 | 0.746 |
| Sens 4 | 35% | 33% | 38% | 45% | 44% | 42% | 41% | 57% | 35% |
| Spec 4 | 70% | 70% | 70% | 70% | 70% | 70% | 70% | 70% | 70% |
| Cutoff 5 | 1.11 | 1.09 | 1.06 | 1.11 | 1.09 | 1.06 | 1.11 | 1.09 | 1.06 |
| Sens 5 | 15% | 33% | 19% | 18% | 22% | 19% | 12% | 29% | 24% |
| Spec 5 | 81% | 80% | 80% | 81% | 80% | 80% | 81% | 80% | 80% |
| Cutoff 6 | 1.72 | 1.70 | 1.66 | 1.72 | 1.70 | 1.66 | 1.72 | 1.70 | 1.66 |
| Sens 6 | 8% | 17% | 8% | 6% | 11% | 6% | 12% | 14% | 12% |
| Spec 6 | 90% | 90% | 90% | 90% | 90% | 90% | 90% | 90% | 90% |
| OR Quart 2 | 0.48 | 0 | 0.70 | 0.53 | 2.0 | 0.42 | 0.39 | 0.49 | 0.73 |
| p Value | 0.24 | na | 0.55 | 0.27 | 0.57 | 0.16 | 0.26 | 0.57 | 0.69 |
| 95% CI of | 0.14 | na | 0.21 | 0.17 | 0.18 | 0.13 | 0.073 | 0.044 | 0.16 |
| OR Quart2 | 1.6 | na | 2.3 | 1.6 | 22 | 1.4 | 2.0 | 5.5 | 3.4 |
| OR Quart 3 | 0.86 | 3.0 | 0.85 | 1.0 | 3.0 | 0.76 | 0.59 | 0 | 0.99 |
| p Value | 0.78 | 0.34 | 0.77 | 1.0 | 0.34 | 0.60 | 0.48 | na | 0.99 |
| 95% CI of | 0.30 | 0.31 | 0.27 | 0.38 | 0.31 | 0.27 | 0.14 | na | 0.24 |
| OR Quart3 | 2.5 | 30 | 2.6 | 2.6 | 30 | 2.1 | 2.5 | na | 4.1 |
| OR Quart 4 | 0.86 | 2.0 | 1.2 | 1.1 | 3.0 | 1.2 | 1.4 | 2.0 | 1.5 |
| p Value | 0.78 | 0.57 | 0.79 | 0.83 | 0.34 | 0.65 | 0.57 | 0.42 | 0.53 |
| 95% CI of | 0.30 | 0.18 | 0.40 | 0.43 | 0.31 | 0.49 | 0.43 | 0.36 | 0.41 |
| OR Quart4 | 2.5 | 22 | 3.3 | 2.8 | 30 | 3.1 | 4.6 | 11 | 5.6 |

**Insulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 236 | 153 | nd | nd |
| Average | nd | nd | 491 | 213 | nd | nd |
| Stdev | nd | nd | 1040 | 197 | nd | nd |
| p(t-test) | nd | nd | | 0.36 | nd | nd |
| Min | nd | nd | 27.6 | 33.9 | nd | nd |
| Max | nd | nd | 8650 | 608 | nd | nd |
| n (Samp) | nd | nd | 112 | 12 | nd | nd |
| n (Patient) | nd | nd | 94 | 12 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 231 | 134 | nd | nd |
| Average | nd | nd | 505 | 138 | nd | nd |
| Stdev | nd | nd | 1100 | 99.5 | nd | nd |
| p(t-test) | nd | nd | | 0.30 | nd | nd |
| Min | nd | nd | 27.6 | 33.9 | nd | nd |
| Max | nd | nd | 8650 | 365 | nd | nd |
| n (Samp) | nd | nd | 99 | 10 | nd | nd |
| n (Patient) | nd | nd | 80 | 10 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.36 | nd | 0.28 | nd | nd | nd |
| SE | nd | nd | nd | 0.090 | nd | 0.094 | nd | nd | nd |
| p | nd | nd | nd | 0.11 | nd | 0.017 | nd | nd | nd |
| nCohort 1 | nd | nd | nd | 112 | nd | 99 | nd | nd | nd |
| nCohort 2 | nd | nd | nd | 12 | nd | 10 | nd | nd | nd |
| Cutoff 1 | nd | nd | nd | 64.2 | nd | 64.2 | nd | nd | nd |
| Sens 1 | nd | nd | nd | 83% | nd | 80% | nd | nd | nd |
| Spec 1 | nd | nd | nd | 7% | nd | 7% | nd | nd | nd |
| Cutoff 2 | nd | nd | nd | 64.2 | nd | 64.2 | nd | nd | nd |
| Sens 2 | nd | nd | nd | 83% | nd | 80% | nd | nd | nd |
| Spec 2 | nd | nd | nd | 7% | nd | 7% | nd | nd | nd |
| Cutoff 3 | nd | nd | nd | 37.8 | nd | 37.8 | nd | nd | nd |
| Sens 3 | nd | nd | nd | 92% | nd | 90% | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | nd | nd | nd | 4% | nd | 4% | nd | nd | nd |
| Cutoff 4 | nd | nd | nd | 389 | nd | 392 | nd | nd | nd |
| Sens 4 | nd | nd | nd | 17% | nd | 0% | nd | nd | nd |
| Spec 4 | nd | nd | nd | 71% | nd | 71% | nd | nd | nd |
| Cutoff 5 | nd | nd | nd | 601 | nd | 625 | nd | nd | nd |
| Sens 5 | nd | nd | nd | 8% | nd | 0% | nd | nd | nd |
| Spec 5 | nd | nd | nd | 80% | nd | 81% | nd | nd | nd |
| Cutoff 6 | nd | nd | nd | 909 | nd | 946 | nd | nd | nd |
| Sens 6 | nd | nd | nd | 0% | nd | 0% | nd | nd | nd |
| Spec 6 | nd | nd | nd | 90% | nd | 91% | nd | nd | nd |
| OR Quart 2 | nd | nd | nd | 0.48 | nd | >2.2 | nd | nd | nd |
| p Value | nd | nd | nd | 0.56 | nd | <0.52 | nd | nd | nd |
| 95% CI of | nd | nd | nd | 0.042 | nd | >0.19 | nd | nd | nd |
| OR Quart2 | nd | nd | nd | 5.6 | nd | na | nd | nd | nd |
| OR Quart 3 | nd | nd | nd | 2.8 | nd | >4.9 | nd | nd | nd |
| p Value | nd | nd | nd | 0.24 | nd | <0.17 | nd | nd | nd |
| 95% CI of | nd | nd | nd | 0.50 | nd | >0.51 | nd | nd | nd |
| OR Quart3 | nd | nd | nd | 16 | nd | na | nd | nd | nd |
| OR Quart 4 | nd | nd | nd | 2.1 | nd | >4.9 | nd | nd | nd |
| p Value | nd | nd | nd | 0.40 | nd | <0.17 | nd | nd | nd |
| 95% CI of | nd | nd | nd | 0.36 | nd | >0.51 | nd | nd | nd |
| OR Quart4 | nd | nd | nd | 13 | nd | na | nd | nd | nd |

**Macrophage migration inhibitory factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 804 | 705 | 804 | 938 | 804 | 1040 |
| Average | 969 | 1140 | 969 | 1020 | 969 | 1020 |
| Stdev | 1040 | 1010 | 1040 | 655 | 1040 | 445 |
| p(t-test) | | 0.50 | | 0.80 | | 0.86 |
| Min | 81.9 | 79.0 | 81.9 | 289 | 81.9 | 246 |
| Max | 14200 | 3610 | 14200 | 2960 | 14200 | 1740 |
| n (Samp) | 231 | 18 | 231 | 26 | 231 | 15 |
| n (Patient) | 159 | 18 | 159 | 26 | 159 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 805 | 705 | 805 | 896 | 805 | 1060 |
| Average | 966 | 974 | 966 | 994 | 966 | 1200 |
| Stdev | 1100 | 809 | 1100 | 664 | 1100 | 832 |
| p(t-test) | | 0.98 | | 0.90 | | 0.43 |
| Min | 81.9 | 79.0 | 81.9 | 289 | 81.9 | 246 |
| Max | 14200 | 2900 | 14200 | 2960 | 14200 | 3610 |
| n (Samp) | 202 | 18 | 202 | 26 | 202 | 14 |
| n (Patient) | 134 | 18 | 134 | 26 | 134 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.47 | 0.54 | nd | 0.53 | 0.61 | nd | 0.63 |
| SE | 0.071 | nd | 0.072 | 0.061 | nd | 0.061 | 0.080 | nd | 0.082 |
| p | 0.94 | nd | 0.65 | 0.47 | nd | 0.65 | 0.17 | nd | 0.11 |
| nCohort 1 | 231 | nd | 202 | 231 | nd | 202 | 231 | nd | 202 |
| nCohort 2 | 18 | nd | 18 | 26 | nd | 26 | 15 | nd | 14 |
| Cutoff 1 | 498 | nd | 472 | 628 | nd | 621 | 823 | nd | 823 |
| Sens 1 | 72% | nd | 72% | 73% | nd | 73% | 73% | nd | 71% |
| Spec 1 | 21% | nd | 20% | 31% | nd | 32% | 54% | nd | 53% |
| Cutoff 2 | 444 | nd | 401 | 614 | nd | 551 | 669 | nd | 492 |
| Sens 2 | 83% | nd | 83% | 81% | nd | 81% | 80% | nd | 86% |
| Spec 2 | 16% | nd | 16% | 30% | nd | 26% | 35% | nd | 22% |
| Cutoff 3 | 258 | nd | 254 | 389 | nd | 389 | 356 | nd | 356 |
| Sens 3 | 94% | nd | 94% | 92% | nd | 92% | 93% | nd | 93% |
| Spec 3 | 4% | nd | 4% | 13% | nd | 15% | 10% | nd | 12% |
| Cutoff 4 | 1010 | nd | 1000 | 1010 | nd | 1000 | 1010 | nd | 1000 |
| Sens 4 | 39% | nd | 33% | 27% | nd | 23% | 53% | nd | 57% |
| Spec 4 | 70% | nd | 70% | 70% | nd | 70% | 70% | nd | 70% |
| Cutoff 5 | 1170 | nd | 1140 | 1170 | nd | 1140 | 1170 | nd | 1140 |
| Sens 5 | 33% | nd | 28% | 23% | nd | 19% | 33% | nd | 43% |
| Spec 5 | 80% | nd | 80% | 80% | nd | 80% | 80% | nd | 80% |
| Cutoff 6 | 1610 | nd | 1560 | 1610 | nd | 1560 | 1610 | nd | 1560 |
| Sens 6 | 22% | nd | 22% | 12% | nd | 12% | 7% | nd | 14% |
| Spec 6 | 90% | nd | 90% | 90% | nd | 90% | 90% | nd | 90% |
| OR Quart 2 | 0.16 | nd | 0.38 | 1.0 | nd | 1.2 | 0.32 | nd | 0.32 |
| p Value | 0.090 | nd | 0.26 | 1.0 | nd | 0.75 | 0.33 | nd | 0.33 |
| 95% CI of | 0.018 | nd | 0.070 | 0.27 | nd | 0.35 | 0.032 | nd | 0.032 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 1.3 | nd | 2.0 | 3.6 | nd | 4.3 | 3.1 | nd | 3.2 |
| OR Quart 3 | 0.83 | nd | 0.78 | 2.2 | nd | 2.2 | 2.1 | nd | 1.4 |
| p Value | 0.77 | nd | 0.73 | 0.18 | nd | 0.17 | 0.31 | nd | 0.70 |
| 95% CI of | 0.24 | nd | 0.20 | 0.70 | nd | 0.71 | 0.50 | nd | 0.29 |
| OR Quart3 | 2.9 | nd | 3.1 | 6.8 | nd | 6.9 | 8.9 | nd | 6.4 |
| OR Quart 4 | 1.0 | nd | 1.5 | 1.2 | nd | 1.0 | 1.7 | nd | 2.1 |
| p Value | 0.98 | nd | 0.54 | 0.77 | nd | 1.0 | 0.48 | nd | 0.31 |
| 95% CI of | 0.31 | nd | 0.43 | 0.35 | nd | 0.27 | 0.39 | nd | 0.50 |
| OR Quart4 | 3.3 | nd | 4.9 | 4.1 | nd | 3.7 | 7.4 | nd | 9.0 |

**Transforming growth factor beta-2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.738 | 1.00E-9 | nd | nd |
| Average | nd | nd | 2.13 | 0.422 | nd | nd |
| Stdev | nd | nd | 6.38 | 0.735 | nd | nd |
| p(t-test) | nd | nd |  | 0.52 | nd | nd |
| Min | nd | nd | 1.00E-9 | 1.00E-9 | nd | nd |
| Max | nd | nd | 39.7 | 1.80 | nd | nd |
| n (Samp) | nd | nd | 57 | 6 | nd | nd |
| n (Patient) | nd | nd | 52 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.40 | nd | nd | nd | nd | nd |
| SE | nd | nd | nd | 0.13 | nd | nd | nd | nd | nd |
| p | nd | nd | nd | 0.44 | nd | nd | nd | nd | nd |
| nCohort 1 | nd | nd | nd | 57 | nd | nd | nd | nd | nd |
| nCohort 2 | nd | nd | nd | 6 | nd | nd | nd | nd | nd |
| Cutoff 1 | nd | nd | nd | 0 | nd | nd | nd | nd | nd |
| Sens 1 | nd | nd | nd | 100% | nd | nd | nd | nd | nd |
| Spec 1 | nd | nd | nd | 0% | nd | nd | nd | nd | nd |
| Cutoff 2 | nd | nd | nd | 0 | nd | nd | nd | nd | nd |
| Sens 2 | nd | nd | nd | 100% | nd | nd | nd | nd | nd |
| Spec 2 | nd | nd | nd | 0% | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | nd | nd | nd | 0 | nd | nd | nd | nd | nd |
| Sens 3 | nd | nd | nd | 100% | nd | nd | nd | nd | nd |
| Spec 3 | nd | nd | nd | 0% | nd | nd | nd | nd | nd |
| Cutoff 4 | nd | nd | nd | 0.738 | nd | nd | nd | nd | nd |
| Sens 4 | nd | nd | nd | 17% | nd | nd | nd | nd | nd |
| Spec 4 | nd | nd | nd | 79% | nd | nd | nd | nd | nd |
| Cutoff 5 | nd | nd | nd | 2.71 | nd | nd | nd | nd | nd |
| Sens 5 | nd | nd | nd | 0% | nd | nd | nd | nd | nd |
| Spec 5 | nd | nd | nd | 89% | nd | nd | nd | nd | nd |
| Cutoff 6 | nd | nd | nd | 5.09 | nd | nd | nd | nd | nd |
| Sens 6 | nd | nd | nd | 0% | nd | nd | nd | nd | nd |
| Spec 6 | nd | nd | nd | 91% | nd | nd | nd | nd | nd |
| OR Quart 2 | nd | nd | nd | 1.0 | nd | nd | nd | nd | nd |
| p Value | nd | nd | nd | 1.0 | nd | nd | nd | nd | nd |
| 95% CI of | nd | nd | nd | 0.057 | nd | nd | nd | nd | nd |
| OR Quart2 | nd | nd | nd | 18 | nd | nd | nd | nd | nd |
| OR Quart 3 | nd | nd | nd | 5.0 | nd | nd | nd | nd | nd |
| p Value | nd | nd | nd | 0.17 | nd | nd | nd | nd | nd |
| 95% CI of | nd | nd | nd | 0.49 | nd | nd | nd | nd | nd |
| OR Quart3 | nd | nd | nd | 51 | nd | nd | nd | nd | nd |
| OR Quart 4 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart4 | nd | nd | nd | na | nd | nd | nd | nd | nd |

**Transforming growth factor beta-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.607 | 0.607 | nd | nd |
| Average | nd | nd | 1.77 | 0.515 | nd | nd |
| Stdev | nd | nd | 5.19 | 0.474 | nd | nd |
| p(t-test) | nd | nd | | 0.56 | nd | nd |
| Min | nd | nd | 1.00E-9 | 1.00E-9 | nd | nd |
| Max | nd | nd | 38.8 | 1.27 | nd | nd |
| n (Samp) | nd | nd | 57 | 6 | nd | nd |
| n (Patient) | nd | nd | 52 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.34 | nd | nd | nd | nd | nd |
| SE | nd | nd | nd | 0.13 | nd | nd | nd | nd | nd |
| p | nd | nd | nd | 0.20 | nd | nd | nd | nd | nd |
| nCohort 1 | nd | nd | nd | 57 | nd | nd | nd | nd | nd |
| nCohort 2 | nd | nd | nd | 6 | nd | nd | nd | nd | nd |
| Cutoff 1 | nd | nd | nd | 0 | nd | nd | nd | nd | nd |
| Sens 1 | nd | nd | nd | 100% | nd | nd | nd | nd | nd |
| Spec 1 | nd | nd | nd | 0% | nd | nd | nd | nd | nd |
| Cutoff 2 | nd | nd | nd | 0 | nd | nd | nd | nd | nd |
| Sens 2 | nd | nd | nd | 100% | nd | nd | nd | nd | nd |
| Spec 2 | nd | nd | nd | 0% | nd | nd | nd | nd | nd |
| Cutoff 3 | nd | nd | nd | 0 | nd | nd | nd | nd | nd |
| Sens 3 | nd | nd | nd | 100% | nd | nd | nd | nd | nd |
| Spec 3 | nd | nd | nd | 0% | nd | nd | nd | nd | nd |
| Cutoff 4 | nd | nd | nd | 1.27 | nd | nd | nd | nd | nd |
| Sens 4 | nd | nd | nd | 0% | nd | nd | nd | nd | nd |
| Spec 4 | nd | nd | nd | 82% | nd | nd | nd | nd | nd |
| Cutoff 5 | nd | nd | nd | 1.27 | nd | nd | nd | nd | nd |
| Sens 5 | nd | nd | nd | 0% | nd | nd | nd | nd | nd |
| Spec 5 | nd | nd | nd | 82% | nd | nd | nd | nd | nd |
| Cutoff 6 | nd | nd | nd | 2.23 | nd | nd | nd | nd | nd |
| Sens 6 | nd | nd | nd | 0% | nd | nd | nd | nd | nd |
| Spec 6 | nd | nd | nd | 91% | nd | nd | nd | nd | nd |
| OR Quart 2 | nd | nd | nd | 1.0 | nd | nd | nd | nd | nd |
| p Value | nd | nd | nd | 1.0 | nd | nd | nd | nd | nd |
| 95% CI of | nd | nd | nd | 0.057 | nd | nd | nd | nd | nd |
| OR Quart2 | nd | nd | nd | 18 | nd | nd | nd | nd | nd |
| OR Quart 3 | nd | nd | nd | 1.0 | nd | nd | nd | nd | nd |
| p Value | nd | nd | nd | 1.0 | nd | nd | nd | nd | nd |
| 95% CI of | nd | nd | nd | 0.057 | nd | nd | nd | nd | nd |
| OR Quart3 | nd | nd | nd | 18 | nd | nd | nd | nd | nd |
| OR Quart 4 | nd | nd | nd | 3.8 | nd | nd | nd | nd | nd |
| p Value | nd | nd | nd | 0.28 | nd | nd | nd | nd | nd |
| 95% CI of | nd | nd | nd | 0.34 | nd | nd | nd | nd | nd |
| OR Quart4 | nd | nd | nd | 41 | nd | nd | nd | nd | nd |

**Transmembrane glycoprotein NMB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 15.4 | 19.2 | nd | nd |
| Average | nd | nd | 16.7 | 20.3 | nd | nd |
| Stdev | nd | nd | 6.80 | 8.16 | nd | nd |
| p(t-test) | nd | nd | | 0.13 | nd | nd |
| Min | nd | nd | 6.34 | 9.17 | nd | nd |
| Max | nd | nd | 41.3 | 33.1 | nd | nd |
| n (Samp) | nd | nd | 111 | 9 | nd | nd |
| n (Patient) | nd | nd | 90 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 15.0 | 19.2 | nd | nd |
| Average | nd | nd | 16.7 | 20.3 | nd | nd |
| Stdev | nd | nd | 6.93 | 8.16 | nd | nd |
| p(t-test) | nd | nd | | 0.15 | nd | nd |
| Min | nd | nd | 6.34 | 9.17 | nd | nd |
| Max | nd | nd | 41.3 | 33.1 | nd | nd |
| n (Samp) | nd | nd | 98 | 9 | nd | nd |
| n (Patient) | nd | nd | 76 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.63 | nd | 0.63 | nd | nd | nd |
| SE | nd | nd | nd | 0.10 | nd | 0.10 | nd | nd | nd |
| p | nd | nd | nd | 0.20 | nd | 0.21 | nd | nd | nd |
| nCohort 1 | nd | nd | nd | 111 | nd | 98 | nd | nd | nd |
| nCohort 2 | nd | nd | nd | 9 | nd | 9 | nd | nd | nd |
| Cutoff 1 | nd | nd | nd | 15.0 | nd | 15.0 | nd | nd | nd |
| Sens 1 | nd | nd | nd | 78% | nd | 78% | nd | nd | nd |
| Spec 1 | nd | nd | nd | 49% | nd | 50% | nd | nd | nd |
| Cutoff 2 | nd | nd | nd | 10.3 | nd | 10.3 | nd | nd | nd |
| Sens 2 | nd | nd | nd | 89% | nd | 89% | nd | nd | nd |
| Spec 2 | nd | nd | nd | 14% | nd | 13% | nd | nd | nd |
| Cutoff 3 | nd | nd | nd | 9.09 | nd | 9.09 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | nd | nd | nd | 100% | nd | 100% | nd | nd | nd |
| Spec 3 | nd | nd | nd | 10% | nd | 9% | nd | nd | nd |
| Cutoff 4 | nd | nd | nd | 18.8 | nd | 18.9 | nd | nd | nd |
| Sens 4 | nd | nd | nd | 56% | nd | 56% | nd | nd | nd |
| Spec 4 | nd | nd | nd | 70% | nd | 70% | nd | nd | nd |
| Cutoff 5 | nd | nd | nd | 21.8 | nd | 22.3 | nd | nd | nd |
| Sens 5 | nd | nd | nd | 44% | nd | 44% | nd | nd | nd |
| Spec 5 | nd | nd | nd | 80% | nd | 81% | nd | nd | nd |
| Cutoff 6 | nd | nd | nd | 25.7 | nd | 27.9 | nd | nd | nd |
| Sens 6 | nd | nd | nd | 33% | nd | 11% | nd | nd | nd |
| Spec 6 | nd | nd | nd | 90% | nd | 91% | nd | nd | nd |
| OR Quart 2 | nd | nd | nd | 0.48 | nd | 0.46 | nd | nd | nd |
| p Value | nd | nd | nd | 0.56 | nd | 0.54 | nd | nd | nd |
| 95% CI of | nd | nd | nd | 0.041 | nd | 0.039 | nd | nd | nd |
| OR Quart2 | nd | nd | nd | 5.6 | nd | 5.4 | nd | nd | nd |
| OR Quart 3 | nd | nd | nd | 1.0 | nd | 0.96 | nd | nd | nd |
| p Value | nd | nd | nd | 1.0 | nd | 0.97 | nd | nd | nd |
| 95% CI of | nd | nd | nd | 0.13 | nd | 0.13 | nd | nd | nd |
| OR Quart3 | nd | nd | nd | 7.6 | nd | 7.4 | nd | nd | nd |
| OR Quart 4 | nd | nd | nd | 2.2 | nd | 2.1 | nd | nd | nd |
| p Value | nd | nd | nd | 0.40 | nd | 0.42 | nd | nd | nd |
| 95% CI of | nd | nd | nd | 0.36 | nd | 0.35 | nd | nd | nd |
| OR Quart4 | nd | nd | nd | 13 | nd | 13 | nd | nd | nd |

**Cadherin-3**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 18.6 | 21.9 | nd | nd |
| Average | nd | nd | 23.5 | 37.0 | nd | nd |
| Stdev | nd | nd | 15.8 | 39.0 | nd | nd |
| p(t-test) | nd | nd |  | 0.036 | nd | nd |
| Min | nd | nd | 1.18 | 6.53 | nd | nd |
| Max | nd | nd | 89.0 | 135 | nd | nd |
| n (Samp) | nd | nd | 111 | 9 | nd | nd |
| n (Patient) | nd | nd | 90 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 18.8 | 21.9 | nd | nd |
| Average | nd | nd | 24.0 | 37.0 | nd | nd |
| Stdev | nd | nd | 16.1 | 39.0 | nd | nd |
| p(t-test) | nd | nd | | 0.051 | nd | nd |
| Min | nd | nd | 1.18 | 6.53 | nd | nd |
| Max | nd | nd | 89.0 | 135 | nd | nd |
| n (Samp) | nd | nd | 98 | 9 | nd | nd |
| n (Patient) | nd | nd | 76 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.60 | nd | 0.60 | nd | nd | nd |
| SE | nd | nd | nd | 0.10 | nd | 0.10 | nd | nd | nd |
| p | nd | nd | nd | 0.32 | nd | 0.36 | nd | nd | nd |
| nCohort 1 | nd | nd | nd | 111 | nd | 98 | nd | nd | nd |
| nCohort 2 | nd | nd | nd | 9 | nd | 9 | nd | nd | nd |
| Cutoff 1 | nd | nd | nd | 14.7 | nd | 14.7 | nd | nd | nd |
| Sens 1 | nd | nd | nd | 78% | nd | 78% | nd | nd | nd |
| Spec 1 | nd | nd | nd | 35% | nd | 35% | nd | nd | nd |
| Cutoff 2 | nd | nd | nd | 14.2 | nd | 14.2 | nd | nd | nd |
| Sens 2 | nd | nd | nd | 89% | nd | 89% | nd | nd | nd |
| Spec 2 | nd | nd | nd | 32% | nd | 32% | nd | nd | nd |
| Cutoff 3 | nd | nd | nd | 6.13 | nd | 6.13 | nd | nd | nd |
| Sens 3 | nd | nd | nd | 100% | nd | 100% | nd | nd | nd |
| Spec 3 | nd | nd | nd | 2% | nd | 2% | nd | nd | nd |
| Cutoff 4 | nd | nd | nd | 26.9 | nd | 27.8 | nd | nd | nd |
| Sens 4 | nd | nd | nd | 44% | nd | 44% | nd | nd | nd |
| Spec 4 | nd | nd | nd | 70% | nd | 70% | nd | nd | nd |
| Cutoff 5 | nd | nd | nd | 32.7 | nd | 34.1 | nd | nd | nd |
| Sens 5 | nd | nd | nd | 44% | nd | 44% | nd | nd | nd |
| Spec 5 | nd | nd | nd | 80% | nd | 81% | nd | nd | nd |
| Cutoff 6 | nd | nd | nd | 42.2 | nd | 44.7 | nd | nd | nd |
| Sens 6 | nd | nd | nd | 22% | nd | 11% | nd | nd | nd |
| Spec 6 | nd | nd | nd | 90% | nd | 91% | nd | nd | nd |
| OR Quart 2 | nd | nd | nd | 3.2 | nd | 3.1 | nd | nd | nd |
| p Value | nd | nd | nd | 0.32 | nd | 0.34 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | nd | nd | nd | 0.32 | nd | 0.30 | nd | nd | nd |
| OR Quart2 | nd | nd | nd | 33 | nd | 32 | nd | nd | nd |
| OR Quart 3 | nd | nd | nd | 1.0 | nd | 0.96 | nd | nd | nd |
| p Value | nd | nd | nd | 1.0 | nd | 0.98 | nd | nd | nd |
| 95% CI of | nd | nd | nd | 0.060 | nd | 0.057 | nd | nd | nd |
| OR Quart3 | nd | nd | nd | 17 | nd | 16 | nd | nd | nd |
| OR Quart 4 | nd | nd | nd | 4.5 | nd | 4.3 | nd | nd | nd |
| p Value | nd | nd | nd | 0.19 | nd | 0.20 | nd | nd | nd |
| 95% CI of | nd | nd | nd | 0.47 | nd | 0.45 | nd | nd | nd |
| OR Quart4 | nd | nd | nd | 43 | nd | 42 | nd | nd | nd |

**Cathepsin S**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 7.44 | 8.12 | nd | nd |
| Average | nd | nd | 7.86 | 8.03 | nd | nd |
| Stdev | nd | nd | 2.73 | 2.44 | nd | nd |
| p(t-test) | nd | nd | | 0.86 | nd | nd |
| Min | nd | nd | 3.54 | 3.11 | nd | nd |
| Max | nd | nd | 22.1 | 11.6 | nd | nd |
| n (Samp) | nd | nd | 111 | 9 | nd | nd |
| n (Patient) | nd | nd | 90 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 7.57 | 8.12 | nd | nd |
| Average | nd | nd | 8.15 | 8.03 | nd | nd |
| Stdev | nd | nd | 2.96 | 2.44 | nd | nd |
| p(t-test) | nd | nd | | 0.91 | nd | nd |
| Min | nd | nd | 3.54 | 3.11 | nd | nd |
| Max | nd | nd | 22.1 | 11.6 | nd | nd |
| n (Samp) | nd | nd | 98 | 9 | nd | nd |
| n (Patient) | nd | nd | 76 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.59 | nd | 0.55 | nd | nd | nd |
| SE | nd | nd | nd | 0.10 | nd | 0.10 | nd | nd | nd |
| p | nd | nd | nd | 0.39 | nd | 0.60 | nd | nd | nd |
| nCohort 1 | nd | nd | nd | 111 | nd | 98 | nd | nd | nd |
| nCohort 2 | nd | nd | nd | 9 | nd | 9 | nd | nd | nd |
| Cutoff 1 | nd | nd | nd | 7.02 | nd | 7.02 | nd | nd | nd |
| Sens 1 | nd | nd | nd | 78% | nd | 78% | nd | nd | nd |
| Spec 1 | nd | nd | nd | 41% | nd | 35% | nd | nd | nd |
| Cutoff 2 | nd | nd | nd | 6.56 | nd | 6.51 | nd | nd | nd |
| Sens 2 | nd | nd | nd | 89% | nd | 89% | nd | nd | nd |
| Spec 2 | nd | nd | nd | 35% | nd | 30% | nd | nd | nd |
| Cutoff 3 | nd | nd | nd | 0 | nd | 0 | nd | nd | nd |
| Sens 3 | nd | nd | nd | 100% | nd | 100% | nd | nd | nd |
| Spec 3 | nd | nd | nd | 0% | nd | 0% | nd | nd | nd |
| Cutoff 4 | nd | nd | nd | 8.32 | nd | 8.56 | nd | nd | nd |
| Sens 4 | nd | nd | nd | 44% | nd | 33% | nd | nd | nd |
| Spec 4 | nd | nd | nd | 70% | nd | 70% | nd | nd | nd |
| Cutoff 5 | nd | nd | nd | 9.32 | nd | 9.78 | nd | nd | nd |
| Sens 5 | nd | nd | nd | 22% | nd | 22% | nd | nd | nd |
| Spec 5 | nd | nd | nd | 80% | nd | 81% | nd | nd | nd |
| Cutoff 6 | nd | nd | nd | 11.5 | nd | 11.7 | nd | nd | nd |
| Sens 6 | nd | nd | nd | 11% | nd | 0% | nd | nd | nd |
| Spec 6 | nd | nd | nd | 90% | nd | 91% | nd | nd | nd |
| OR Quart 2 | nd | nd | nd | 2.1 | nd | 2.0 | nd | nd | nd |
| p Value | nd | nd | nd | 0.56 | nd | 0.58 | nd | nd | nd |
| 95% CI of | nd | nd | nd | 0.18 | nd | 0.17 | nd | nd | nd |
| OR Quart2 | nd | nd | nd | 24 | nd | 23 | nd | nd | nd |
| OR Quart 3 | nd | nd | nd | 4.5 | nd | 4.3 | nd | nd | nd |
| p Value | nd | nd | nd | 0.19 | nd | 0.20 | nd | nd | nd |
| 95% CI of | nd | nd | nd | 0.47 | nd | 0.45 | nd | nd | nd |
| OR Quart3 | nd | nd | nd | 43 | nd | 42 | nd | nd | nd |
| OR Quart 4 | nd | nd | nd | 2.1 | nd | 2.0 | nd | nd | nd |
| p Value | nd | nd | nd | 0.56 | nd | 0.58 | nd | nd | nd |
| 95% CI of | nd | nd | nd | 0.18 | nd | 0.17 | nd | nd | nd |
| OR Quart4 | nd | nd | nd | 24 | nd | 23 | nd | nd | nd |

**Urokinase-type plasminogen activator**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.0702 | 0.0728 | nd | nd |
| Average | nd | nd | 0.0824 | 0.102 | nd | nd |
| Stdev | nd | nd | 0.0518 | 0.122 | nd | nd |
| p(t-test) | nd | nd | | 0.35 | nd | nd |
| Min | nd | nd | 0.0172 | 0.0248 | nd | nd |
| Max | nd | nd | 0.297 | 0.422 | nd | nd |
| n (Samp) | nd | nd | 111 | 9 | nd | nd |
| n (Patient) | nd | nd | 90 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.0717 | 0.0728 | nd | nd |
| Average | nd | nd | 0.0853 | 0.102 | nd | nd |
| Stdev | nd | nd | 0.0541 | 0.122 | nd | nd |
| p(t-test) | nd | nd | | 0.44 | nd | nd |
| Min | nd | nd | 0.0172 | 0.0248 | nd | nd |
| Max | nd | nd | 0.297 | 0.422 | nd | nd |
| n (Samp) | nd | nd | 98 | 9 | nd | nd |
| n (Patient) | nd | nd | 76 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.48 | nd | 0.47 | nd | nd | nd |
| SE | nd | nd | nd | 0.10 | nd | 0.10 | nd | nd | nd |
| p | nd | nd | nd | 0.85 | nd | 0.74 | nd | nd | nd |
| nCohort 1 | nd | nd | nd | 111 | nd | 98 | nd | nd | nd |
| nCohort 2 | nd | nd | nd | 9 | nd | 9 | nd | nd | nd |
| Cutoff 1 | nd | nd | nd | 0.0474 | nd | 0.0474 | nd | nd | nd |
| Sens 1 | nd | nd | nd | 78% | nd | 78% | nd | nd | nd |
| Spec 1 | nd | nd | nd | 25% | nd | 24% | nd | nd | nd |
| Cutoff 2 | nd | nd | nd | 0.0447 | nd | 0.0442 | nd | nd | nd |
| Sens 2 | nd | nd | nd | 89% | nd | 89% | nd | nd | nd |
| Spec 2 | nd | nd | nd | 20% | nd | 18% | nd | nd | nd |
| Cutoff 3 | nd | nd | nd | 0.0240 | nd | 0.0213 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | nd | nd | nd | 100% | nd | 100% | nd | nd | nd |
| Spec 3 | nd | nd | nd | 3% | nd | 2% | nd | nd | nd |
| Cutoff 4 | nd | nd | nd | 0.0892 | nd | 0.0934 | nd | nd | nd |
| Sens 4 | nd | nd | nd | 11% | nd | 11% | nd | nd | nd |
| Spec 4 | nd | nd | nd | 70% | nd | 70% | nd | nd | nd |
| Cutoff 5 | nd | nd | nd | 0.110 | nd | 0.116 | nd | nd | nd |
| Sens 5 | nd | nd | nd | 11% | nd | 11% | nd | nd | nd |
| Spec 5 | nd | nd | nd | 80% | nd | 81% | nd | nd | nd |
| Cutoff 6 | nd | nd | nd | 0.154 | nd | 0.161 | nd | nd | nd |
| Sens 6 | nd | nd | nd | 11% | nd | 11% | nd | nd | nd |
| Spec 6 | nd | nd | nd | 90% | nd | 91% | nd | nd | nd |
| OR Quart 2 | nd | nd | nd | 4.5 | nd | 4.5 | nd | nd | nd |
| p Value | nd | nd | nd | 0.19 | nd | 0.19 | nd | nd | nd |
| 95% CI of | nd | nd | nd | 0.47 | nd | 0.47 | nd | nd | nd |
| OR Quart2 | nd | nd | nd | 43 | nd | 43 | nd | nd | nd |
| OR Quart 3 | nd | nd | nd | 2.1 | nd | 2.1 | nd | nd | nd |
| p Value | nd | nd | nd | 0.56 | nd | 0.56 | nd | nd | nd |
| 95% CI of | nd | nd | nd | 0.18 | nd | 0.18 | nd | nd | nd |
| OR Quart3 | nd | nd | nd | 24 | nd | 24 | nd | nd | nd |
| OR Quart 4 | nd | nd | nd | 2.1 | nd | 2.2 | nd | nd | nd |
| p Value | nd | nd | nd | 0.56 | nd | 0.54 | nd | nd | nd |
| 95% CI of | nd | nd | nd | 0.18 | nd | 0.18 | nd | nd | nd |
| OR Quart4 | nd | nd | nd | 24 | nd | 25 | nd | nd | nd |

**C-C motif chemokine 23**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1.63 | 2.88 | nd | nd |
| Average | nd | nd | 2.01 | 3.00 | nd | nd |
| Stdev | nd | nd | 1.84 | 3.11 | nd | nd |
| p(t-test) | nd | nd |  | 0.15 | nd | nd |
| Min | nd | nd | 0.00425 | 0.00165 | nd | nd |
| Max | nd | nd | 10.3 | 10.3 | nd | nd |
| n (Samp) | nd | nd | 113 | 9 | nd | nd |
| n (Patient) | nd | nd | 92 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.73 | 2.88 | nd | nd |
| Average | nd | nd | 2.14 | 3.00 | nd | nd |
| Stdev | nd | nd | 1.89 | 3.11 | nd | nd |
| p(t-test) | nd | nd | | 0.22 | nd | nd |
| Min | nd | nd | 0.00425 | 0.00165 | nd | nd |
| Max | nd | nd | 10.3 | 10.3 | nd | nd |
| n (Samp) | nd | nd | 99 | 9 | nd | nd |
| n (Patient) | nd | nd | 77 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.60 | nd | 0.59 | nd | nd | nd |
| SE | nd | nd | nd | 0.10 | nd | 0.10 | nd | nd | nd |
| p | nd | nd | nd | 0.34 | nd | 0.40 | nd | nd | nd |
| nCohort 1 | nd | nd | nd | 113 | nd | 99 | nd | nd | nd |
| nCohort 2 | nd | nd | nd | 9 | nd | 9 | nd | nd | nd |
| Cutoff 1 | nd | nd | nd | 1.50 | nd | 1.50 | nd | nd | nd |
| Sens 1 | nd | nd | nd | 78% | nd | 78% | nd | nd | nd |
| Spec 1 | nd | nd | nd | 50% | nd | 46% | nd | nd | nd |
| Cutoff 2 | nd | nd | nd | 0.0186 | nd | 0.0186 | nd | nd | nd |
| Sens 2 | nd | nd | nd | 89% | nd | 89% | nd | nd | nd |
| Spec 2 | nd | nd | nd | 7% | nd | 8% | nd | nd | nd |
| Cutoff 3 | nd | nd | nd | 0 | nd | 0 | nd | nd | nd |
| Sens 3 | nd | nd | nd | 100% | nd | 100% | nd | nd | nd |
| Spec 3 | nd | nd | nd | 0% | nd | 0% | nd | nd | nd |
| Cutoff 4 | nd | nd | nd | 2.31 | nd | 2.38 | nd | nd | nd |
| Sens 4 | nd | nd | nd | 56% | nd | 56% | nd | nd | nd |
| Spec 4 | nd | nd | nd | 71% | nd | 71% | nd | nd | nd |
| Cutoff 5 | nd | nd | nd | 2.94 | nd | 3.32 | nd | nd | nd |
| Sens 5 | nd | nd | nd | 44% | nd | 33% | nd | nd | nd |
| Spec 5 | nd | nd | nd | 81% | nd | 81% | nd | nd | nd |
| Cutoff 6 | nd | nd | nd | 4.21 | nd | 4.48 | nd | nd | nd |
| Sens 6 | nd | nd | nd | 11% | nd | 11% | nd | nd | nd |
| Spec 6 | nd | nd | nd | 90% | nd | 91% | nd | nd | nd |
| OR Quart 2 | nd | nd | nd | 0.97 | nd | 1.0 | nd | nd | nd |
| p Value | nd | nd | nd | 0.97 | nd | 1.0 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | nd | nd | nd | 0.13 | nd | 0.13 | nd | nd | nd |
| OR Quart2 | nd | nd | nd | 7.3 | nd | 7.7 | nd | nd | nd |
| OR Quart 3 | nd | nd | nd | 0 | nd | 0.48 | nd | nd | nd |
| p Value | nd | nd | nd | na | nd | 0.56 | nd | nd | nd |
| 95% CI of | nd | nd | nd | na | nd | 0.041 | nd | nd | nd |
| OR Quart3 | nd | nd | nd | na | nd | 5.6 | nd | nd | nd |
| OR Quart 4 | nd | nd | nd | 2.7 | nd | 2.2 | nd | nd | nd |
| p Value | nd | nd | nd | 0.26 | nd | 0.40 | nd | nd | nd |
| 95% CI of | nd | nd | nd | 0.48 | nd | 0.36 | nd | nd | nd |
| OR Quart4 | nd | nd | nd | 15 | nd | 13 | nd | nd | nd |

**Insulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.10 | 1.48 | 3.10 | 2.20 | 3.10 | 2.89 |
| Average | 6.53 | 3.78 | 6.53 | 6.31 | 6.53 | 3.04 |
| Stdev | 12.4 | 6.17 | 12.4 | 11.5 | 12.4 | 2.65 |
| p(t-test) | | 0.26 | | 0.92 | | 0.25 |
| Min | 0.00859 | 0.00859 | 0.00859 | 0.00859 | 0.00859 | 0.00859 |
| Max | 162 | 23.8 | 162 | 53.1 | 162 | 10.1 |
| n (Samp) | 437 | 26 | 437 | 33 | 437 | 17 |
| n (Patient) | 174 | 26 | 174 | 33 | 174 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.81 | 6.38 | 2.81 | 5.95 | 2.81 | 1.57 |
| Average | 6.01 | 7.73 | 6.01 | 12.0 | 6.01 | 4.04 |
| Stdev | 11.5 | 7.71 | 11.5 | 16.7 | 11.5 | 5.73 |
| p(t-test) | | 0.72 | | 0.12 | | 0.65 |
| Min | 0.00859 | 0.00859 | 0.00859 | 0.00859 | 0.00859 | 0.00859 |
| Max | 162 | 18.9 | 162 | 53.1 | 162 | 16.1 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.08 | 1.39 | 3.08 | 2.05 | 3.08 | 1.89 |
| Average | 6.69 | 3.23 | 6.69 | 4.29 | 6.69 | 5.71 |
| Stdev | 13.2 | 5.36 | 13.2 | 7.84 | 13.2 | 12.5 |
| p(t-test) | | 0.19 | | 0.32 | | 0.76 |
| Min | 0.00859 | 0.00859 | 0.00859 | 0.00859 | 0.00859 | 0.00859 |
| Max | 162 | 23.8 | 162 | 39.2 | 162 | 53.1 |
| n (Samp) | 362 | 26 | 362 | 31 | 362 | 17 |
| n (Patient) | 140 | 26 | 140 | 31 | 140 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.33 | 0.56 | 0.31 | 0.41 | 0.58 | 0.37 | 0.40 | 0.39 | 0.40 |
| SE | 0.059 | 0.12 | 0.059 | 0.054 | 0.10 | 0.056 | 0.074 | 0.11 | 0.074 |
| p | 0.0034 | 0.62 | 0.0013 | 0.11 | 0.41 | 0.017 | 0.19 | 0.34 | 0.19 |
| nCohort 1 | 437 | 535 | 362 | 437 | 535 | 362 | 437 | 535 | 362 |
| nCohort 2 | 26 | 6 | 26 | 33 | 9 | 31 | 17 | 7 | 17 |
| Cutoff 1 | 0.554 | 1.59 | 0.554 | 0.871 | 0.611 | 0.976 | 1.55 | 0.583 | 1.59 |
| Sens 1 | 73% | 83% | 73% | 73% | 78% | 71% | 71% | 71% | 71% |
| Spec 1 | 8% | 31% | 8% | 14% | 10% | 16% | 27% | 10% | 28% |
| Cutoff 2 | 0.331 | 1.59 | 0.331 | 0.583 | 0.554 | 0.583 | 0.777 | 0.495 | 0.777 |
| Sens 2 | 81% | 83% | 81% | 82% | 89% | 81% | 82% | 86% | 82% |
| Spec 2 | 6% | 31% | 6% | 8% | 10% | 8% | 12% | 9% | 12% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 5.55 | 5.08 | 5.61 | 5.55 | 5.08 | 5.61 | 5.55 | 5.08 | 5.61 |
| Sens 4 | 19% | 50% | 15% | 24% | 56% | 16% | 18% | 29% | 18% |
| Spec 4 | 70% | 70% | 70% | 70% | 70% | 70% | 70% | 70% | 70% |
| Cutoff 5 | 9.24 | 8.18 | 8.65 | 9.24 | 8.18 | 8.65 | 9.24 | 8.18 | 8.65 |
| Sens 5 | 15% | 50% | 12% | 18% | 44% | 10% | 6% | 14% | 12% |
| Spec 5 | 80% | 80% | 80% | 80% | 80% | 80% | 80% | 80% | 80% |
| Cutoff 6 | 14.7 | 13.9 | 14.5 | 14.7 | 13.9 | 14.5 | 14.7 | 13.9 | 14.5 |
| Sens 6 | 8% | 17% | 4% | 12% | 33% | 10% | 0% | 14% | 6% |
| Spec 6 | 90% | 90% | 90% | 90% | 90% | 90% | 90% | 90% | 90% |
| OR Quart 2 | 0.49 | 2.0 | 1.0 | 1.4 | 0 | 2.8 | 7.5 | 2.0 | 2.6 |
| p Value | 0.42 | 0.57 | 1.0 | 0.57 | na | 0.13 | 0.062 | 0.57 | 0.26 |
| 95% CI of | 0.088 | 0.18 | 0.20 | 0.46 | na | 0.73 | 0.90 | 0.18 | 0.49 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 2.7 | 22 | 5.1 | 4.1 | na | 11 | 62 | 23 | 14 |
| OR Quart3 | 2.4 | 0 | 2.4 | 1.4 | 0.66 | 2.8 | 4.1 | 1.0 | 2.6 |
| p Value | 0.16 | na | 0.21 | 0.58 | 0.65 | 0.13 | 0.21 | 1.0 | 0.26 |
| 95% CI of | 0.70 | na | 0.61 | 0.46 | 0.11 | 0.73 | 0.45 | 0.062 | 0.49 |
| OR Quart3 | 7.9 | na | 9.7 | 4.0 | 4.0 | 11 | 37 | 16 | 14 |
| OR Quart4 | 3.0 | 3.0 | 4.8 | 1.9 | 1.3 | 4.5 | 5.2 | 3.1 | 2.6 |
| p Value | 0.070 | 0.34 | 0.016 | 0.21 | 0.70 | 0.024 | 0.13 | 0.33 | 0.26 |
| 95% CI of | 0.91 | 0.31 | 1.3 | 0.69 | 0.29 | 1.2 | 0.60 | 0.32 | 0.49 |
| OR Quart4 | 9.6 | 29 | 18 | 5.4 | 6.1 | 16 | 46 | 30 | 14 |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Angiopoietin-2**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1530 | 1950 | nd | nd | 1610 | 2410 |
| Average | 2220 | 2150 | nd | nd | 2130 | 2770 |
| Stdev | 1970 | 804 | nd | nd | 1650 | 1580 |
| p(t-test) | | 0.91 | nd | nd | | 0.30 |
| Min | 219 | 1100 | nd | nd | 219 | 1100 |
| Max | 10500 | 3530 | nd | nd | 6710 | 6410 |
| n (Samp) | 41 | 12 | nd | nd | 31 | 9 |
| n (Patient) | 41 | 12 | nd | nd | 31 | 9 |

| | At Enrollment | | |
|---|---|---|---|
| | sCr or UO | sCr only | UO only |
| AUC | 0.63 | nd | 0.69 |
| SE | 0.096 | nd | 0.11 |
| p | 0.18 | nd | 0.081 |
| nCohort 1 | 41 | nd | 31 |
| nCohort 2 | 12 | nd | 9 |
| Cutoff 1 | 1470 | nd | 1740 |
| Sens 1 | 75% | nd | 78% |
| Spec 1 | 49% | nd | 68% |
| Cutoff 2 | 1310 | nd | 1410 |
| Sens 2 | 83% | nd | 89% |

(continued)

|  | At Enrollment | | |
|---|---|---|---|
|  | sCr or UO | sCr only | UO only |
| Spec 2 | 39% | nd | 45% |
| Cutoff 3 | 1280 | nd | 1040 |
| Sens 3 | 92% | nd | 100% |
| Spec 3 | 37% | nd | 16% |
| Cutoff 4 | 2000 | nd | 2050 |
| Sens 4 | 50% | nd | 56% |
| Spec 4 | 71% | nd | 71% |
| Cutoff 5 | 2480 | nd | 2670 |
| Sens 5 | 33% | nd | 44% |
| Spec 5 | 80% | nd | 81% |
| Cutoff 6 | 5140 | nd | 5140 |
| Sens 6 | 0% | nd | 11% |
| Spec 6 | 90% | nd | 90% |
| OR Quart 2 | 3.6 | nd | 1.0 |
| p Value | 0.30 | nd | 1.0 |
| 95% CI of | 0.32 | nd | 0.054 |
| OR Quart2 | 40 | nd | 19 |
| OR Quart 3 | 5.3 | nd | 3.9 |
| p Value | 0.16 | nd | 0.28 |
| 95% CI of | 0.51 | nd | 0.33 |
| OR Quart3 | 56 | nd | 46 |
| OR Quart 4 | 4.8 | nd | 6.0 |
| p Value | 0.19 | nd | 0.15 |
| 95% CI of | 0.46 | nd | 0.53 |
| OR Quart4 | 50 | nd | 68 |

**Immunoglobulin E**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 66.8 | 22.8 | nd | nd | 69.1 | 23.0 |
| Average | 163 | 51.3 | nd | nd | 175 | 51.4 |
| Stdev | 338 | 80.6 | nd | nd | 365 | 89.0 |
| p(t-test) |  | 0.13 | nd | nd |  | 0.19 |
| Min | 0.140 | 0.140 | nd | nd | 6.48 | 2.11 |
| Max | 1940 | 368 | nd | nd | 1940 | 368 |
| n (Samp) | 54 | 22 | nd | nd | 45 | 16 |

(continued)

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| n (Patient) | 54 | 22 | nd | nd | 45 | 16 |

| | At Enrollment | | |
|---|---|---|---|
| | sCr or UO | sCr only | UO only |
| AUC | 0.34 | nd | 0.29 |
| SE | 0.072 | nd | 0.080 |
| p | 0.029 | nd | 0.0086 |
| nCohort 1 | 54 | nd | 45 |
| nCohort 2 | 22 | nd | 16 |
| Cutoff 1 | 9.20 | nd | 9.20 |
| Sens 1 | 73% | nd | 75% |
| Spec 1 | 13% | nd | 7% |
| Cutoff 2 | 4.01 | nd | 8.09 |
| Sens 2 | 82% | nd | 81% |
| Spec 2 | 6% | nd | 4% |
| Cutoff 3 | 1.52 | nd | 2.11 |
| Sens 3 | 95% | nd | 94% |
| Spec 3 | 4% | nd | 0% |
| Cutoff 4 | 122 | nd | 122 |
| Sens 4 | 14% | nd | 6% |
| Spec 4 | 70% | nd | 71% |
| Cutoff 5 | 190 | nd | 146 |
| Sens 5 | 5% | nd | 6% |
| Spec 5 | 81% | nd | 80% |
| Cutoff 6 | 292 | nd | 292 |
| Sens 6 | 5% | nd | 6% |
| Spec 6 | 91% | nd | 91% |
| OR Quart 2 | 1.0 | nd | 3.8 |
| p Value | 1.0 | nd | 0.28 |
| 95% CI of | 0.17 | nd | 0.34 |
| OR Quart2 | 5.7 | nd | 41 |
| OR Quart 3 | 4.8 | nd | 13 |
| p Value | 0.044 | nd | 0.026 |
| 95% CI of | 1.0 | nd | 1.4 |
| OR Quart3 | 22 | nd | 130 |
| OR Quart 4 | 3.1 | nd | 7.5 |

(continued)

|  | At Enrollment | | |
|---|---|---|---|
|  | sCr or UO | sCr only | UO only |
| p Value | 0.15 | nd | 0.085 |
| 95% CI of | 0.66 | nd | 0.76 |
| OR Quart4 | 15 | nd | 74 |

**Immunoglobulin M**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.480 | 0.678 | nd | nd | 0.497 | 0.638 |
| Average | 0.656 | 0.857 | nd | nd | 0.724 | 0.810 |
| Stdev | 0.713 | 0.721 | nd | nd | 0.802 | 0.715 |
| p(t-test) |  | 0.27 | nd | nd |  | 0.71 |
| Min | 0.132 | 0.107 | nd | nd | 0.132 | 0.107 |
| Max | 4.82 | 3.09 | nd | nd | 4.82 | 3.09 |
| n (Samp) | 54 | 22 | nd | nd | 45 | 16 |
| n (Patient) | 54 | 22 | nd | nd | 45 | 16 |

|  | At Enrollment | | |
|---|---|---|---|
|  | sCr or UO | sCr only | UO only |
| AUC | 0.63 | nd | 0.58 |
| SE | 0.073 | nd | 0.085 |
| p | 0.080 | nd | 0.36 |
| nCohort 1 | 54 | nd | 45 |
| nCohort 2 | 22 | nd | 16 |
| Cutoff 1 | 0.482 | nd | 0.482 |
| Sens 1 | 73% | nd | 75% |
| Spec 1 | 52% | nd | 44% |
| Cutoff 2 | 0.365 | nd | 0.365 |
| Sens 2 | 82% | nd | 81% |
| Spec 2 | 33% | nd | 31% |
| Cutoff 3 | 0.200 | nd | 0.132 |
| Sens 3 | 91% | nd | 94% |
| Spec 3 | 11% | nd | 2% |
| Cutoff 4 | 0.613 | nd | 0.734 |
| Sens 4 | 59% | nd | 38% |
| Spec 4 | 70% | nd | 71% |
| Cutoff 5 | 0.773 | nd | 0.881 |

(continued)

|  | At Enrollment | | |
|---|---|---|---|
|  | sCr or UO | sCr only | UO only |
| Sens 5 | 36% | nd | 31% |
| Spec 5 | 81% | nd | 82% |
| Cutoff 6 | 1.28 | nd | 1.40 |
| Sens 6 | 14% | nd | 12% |
| Spec 6 | 91% | nd | 91% |
| OR Quart 2 | 0.70 | nd | 1.0 |
| p Value | 0.68 | nd | 1.0 |
| 95% CI of | 0.13 | nd | 0.17 |
| OR Quart2 | 3.7 | nd | 6.0 |
| OR Quart 3 | 2.2 | nd | 2.0 |
| p Value | 0.29 | nd | 0.41 |
| 95% CI of | 0.52 | nd | 0.38 |
| OR Quart3 | 9.3 | nd | 11 |
| OR Quart 4 | 2.7 | nd | 1.8 |
| p Value | 0.17 | nd | 0.48 |
| 95% CI of | 0.65 | nd | 0.35 |
| OR Quart4 | 11 | nd | 9.5 |

**Macrophage migration inhibitory factor**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 989 | 874 | nd | nd | 951 | 670 |
| Average | 1360 | 972 | nd | nd | 1140 | 868 |
| Stdev | 2010 | 713 | nd | nd | 821 | 688 |
| p(t-test) |  | 0.41 | nd | nd |  | 0.28 |
| Min | 81.9 | 289 | nd | nd | 212 | 289 |
| Max | 14200 | 2960 | nd | nd | 4060 | 2960 |
| n (Samp) | 50 | 19 | nd | nd | 40 | 14 |
| n (Patient) | 50 | 19 | nd | nd | 40 | 14 |

|  | At Enrollment | | |
|---|---|---|---|
|  | sCr or UO | sCr only | UO only |
| AUC | 0.39 | nd | 0.35 |
| SE | 0.079 | nd | 0.089 |
| p | 0.18 | nd | 0.090 |
| nCohort 1 | 50 | nd | 40 |

(continued)

|  | At Enrollment | | |
|---|---|---|---|
|  | sCr or UO | sCr only | UO only |
| nCohort 2 | 19 | nd | 14 |
| Cutoff 1 | 481 | nd | 481 |
| Sens 1 | 74% | nd | 71% |
| Spec 1 | 18% | nd | 20% |
| Cutoff 2 | 401 | nd | 401 |
| Sens 2 | 84% | nd | 86% |
| Spec 2 | 12% | nd | 12% |
| Cutoff 3 | 289 | nd | 289 |
| Sens 3 | 95% | nd | 93% |
| Spec 3 | 6% | nd | 5% |
| Cutoff 4 | 1190 | nd | 1160 |
| Sens 4 | 21% | nd | 14% |
| Spec 4 | 70% | nd | 70% |
| Cutoff 5 | 1380 | nd | 1380 |
| Sens 5 | 21% | nd | 14% |
| Spec 5 | 80% | nd | 80% |
| Cutoff 6 | 1800 | nd | 1800 |
| Sens 6 | 11% | nd | 7% |
| Spec 6 | 90% | nd | 90% |
| OR Quart 2 | 0.75 | nd | 1.1 |
| p Value | 0.74 | nd | 0.94 |
| 95% CI of | 0.14 | nd | 0.13 |
| OR Quart2 | 4.0 | nd | 9.1 |
| OR Quart 3 | 1.9 | nd | 3.3 |
| p Value | 0.40 | nd | 0.20 |
| 95% CI of | 0.43 | nd | 0.52 |
| OR Quart3 | 8.5 | nd | 21 |
| OR Quart 4 | 1.9 | nd | 3.8 |
| p Value | 0.40 | nd | 0.17 |
| 95% CI of | 0.43 | nd | 0.58 |
| OR Quart4 | 8.5 | nd | 24 |

**Insulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 4.68 | 2.55 | nd | nd | 4.89 | 2.55 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Average | 10.3 | 5.92 | nd | nd | 11.2 | 4.58 |
| Stdev | 18.5 | 11.7 | nd | nd | 19.9 | 6.81 |
| p(t-test) |  | 0.31 | nd | nd |  | 0.20 |
| Min | 0.00859 | 0.00859 | nd | nd | 0.00859 | 0.00859 |
| Max | 120 | 53.1 | nd | nd | 120 | 24.1 |
| n (Samp) | 54 | 22 | nd | nd | 45 | 16 |
| n (Patient) | 54 | 22 | nd | nd | 45 | 16 |

|  | At Enrollment | | |
|---|---|---|---|
|  | sCr or UO | sCr only | UO only |
| AUC | 0.36 | nd | 0.29 |
| SE | 0.073 | nd | 0.080 |
| p | 0.055 | nd | 0.0086 |
| nCohort 1 | 54 | nd | 45 |
| nCohort 2 | 22 | nd | 16 |
| Cutoff 1 | 1.10 | nd | 1.10 |
| Sens 1 | 73% | nd | 75% |
| Spec 1 | 20% | nd | 16% |
| Cutoff 2 | 0.708 | nd | 0.708 |
| Sens 2 | 82% | nd | 81% |
| Spec 2 | 13% | nd | 4% |
| Cutoff 3 | 0.392 | nd | 0.00859 |
| Sens 3 | 91% | nd | 94% |
| Spec 3 | 13% | nd | 4% |
| Cutoff 4 | 9.35 | nd | 9.34 |
| Sens 4 | 9% | nd | 12% |
| Spec 4 | 70% | nd | 71% |
| Cutoff 5 | 12.9 | nd | 12.0 |
| Sens 5 | 9% | nd | 12% |
| Spec 5 | 81% | nd | 80% |
| Cutoff 6 | 19.8 | nd | 26.5 |
| Sens 6 | 9% | nd | 0% |
| Spec 6 | 91% | nd | 91% |
| OR Quart 2 | 3.0 | nd | 0.50 |
| p Value | 0.22 | nd | 0.59 |
| 95% CI of | 0.51 | nd | 0.041 |

(continued)

| | At Enrollment | | |
|---|---|---|---|
| | sCr or UO | sCr only | UO only |
| OR Quart2 | 18 | nd | 6.2 |
| OR Quart 3 | 5.0 | nd | 4.7 |
| p Value | 0.071 | nd | 0.095 |
| 95% CI of | 0.87 | nd | 0.77 |
| OR Quart3 | 28 | nd | 28 |
| OR Quart 4 | 6.2 | nd | 6.1 |
| p Value | 0.038 | nd | 0.048 |
| 95% CI of | 1.1 | nd | 1.0 |
| OR Quart4 | 35 | nd | 37 |

Table 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Angiopoietin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1480 | 3240 | 1480 | 2770 | nd | nd |
| Average | 1820 | 7760 | 1820 | 7310 | nd | nd |
| Stdev | 1010 | 13100 | 1010 | 13200 | nd | nd |
| p(t-test) | | 1.4E-4 | | 4.5E-4 | nd | nd |
| Min | 575 | 1290 | 575 | 1290 | nd | nd |
| Max | 4810 | 39900 | 4810 | 39900 | nd | nd |
| n (Samp) | 74 | 8 | 74 | 8 | nd | nd |
| n (Patient) | 74 | 8 | 74 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1710 | 3240 | 1710 | 3240 | nd | nd |
| Average | 2060 | 9040 | 2060 | 9040 | nd | nd |
| Stdev | 1440 | 15200 | 1440 | 15200 | nd | nd |
| p(t-test) | | 4.2E-4 | | 4.2E-4 | nd | nd |
| Min | 575 | 1290 | 575 | 1290 | nd | nd |
| Max | 10500 | 39900 | 10500 | 39900 | nd | nd |
| n (Samp) | 62 | 6 | 62 | 6 | nd | nd |
| n (Patient) | 62 | 6 | 62 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.78 | nd | 0.76 | 0.76 | nd | 0.76 | nd | nd | nd |
| SE | 0.100 | nd | 0.12 | 0.10 | nd | 0.12 | nd | nd | nd |
| p | 0.0052 | nd | 0.029 | 0.012 | nd | 0.029 | nd | nd | nd |
| nCohort 1 | 74 | nd | 62 | 74 | nd | 62 | nd | nd | nd |
| nCohort 2 | 8 | nd | 6 | 8 | nd | 6 | nd | nd | nd |
| Cutoff 1 | 1780 | nd | 1890 | 1780 | nd | 1890 | nd | nd | nd |
| Sens 1 | 75% | nd | 83% | 75% | nd | 83% | nd | nd | nd |
| Spec 1 | 64% | nd | 56% | 64% | nd | 56% | nd | nd | nd |
| Cutoff 2 | 1450 | nd | 1890 | 1450 | nd | 1890 | nd | nd | nd |
| Sens 2 | 88% | nd | 83% | 88% | nd | 83% | nd | nd | nd |
| Spec 2 | 49% | nd | 56% | 49% | nd | 56% | nd | nd | nd |
| Cutoff 3 | 1280 | nd | 1280 | 1280 | nd | 1280 | nd | nd | nd |
| Sens 3 | 100% | nd | 100% | 100% | nd | 100% | nd | nd | nd |
| Spec 3 | 38% | nd | 29% | 38% | nd | 29% | nd | nd | nd |
| Cutoff 4 | 2130 | nd | 2150 | 2130 | nd | 2150 | nd | nd | nd |
| Sens 4 | 62% | nd | 67% | 62% | nd | 67% | nd | nd | nd |
| Spec 4 | 70% | nd | 71% | 70% | nd | 71% | nd | nd | nd |
| Cutoff 5 | 2550 | nd | 2660 | 2550 | nd | 2660 | nd | nd | nd |
| Sens 5 | 62% | nd | 67% | 62% | nd | 67% | nd | nd | nd |
| Spec 5 | 81% | nd | 81% | 81% | nd | 81% | nd | nd | nd |
| Cutoff 6 | 3360 | nd | 3360 | 3360 | nd | 3360 | nd | nd | nd |
| Sens 6 | 50% | nd | 50% | 38% | nd | 50% | nd | nd | nd |
| Spec 6 | 91% | nd | 90% | 91% | nd | 90% | nd | nd | nd |
| OR Quart 2 | >2.1 | nd | >1.1 | >2.1 | nd | >1.1 | nd | nd | nd |
| p Value | <0.56 | nd | <0.97 | <0.56 | nd | <0.97 | nd | nd | nd |
| 95% CI of | >0.18 | nd | >0.061 | >0.18 | nd | >0.061 | nd | nd | nd |
| OR Quart2 | na | nd | na | na | nd | na | nd | nd | nd |
| OR Quart 3 | >1.1 | nd | >1.1 | >1.1 | nd | >1.1 | nd | nd | nd |
| p Value | <0.97 | nd | <0.97 | <0.97 | nd | <0.97 | nd | nd | nd |
| 95% CI of | >0.061 | nd | >0.061 | >0.061 | nd | >0.061 | nd | nd | nd |
| OR Quart3 | na | nd | na | na | nd | na | nd | nd | nd |
| OR Quart 4 | >6.2 | nd | >5.2 | >6.2 | nd | >5.2 | nd | nd | nd |
| p Value | <0.11 | nd | <0.16 | <0.11 | nd | <0.16 | nd | nd | nd |
| 95% CI of | >0.66 | nd | >0.52 | >0.66 | nd | >0.52 | nd | nd | nd |
| OR Quart4 | na | nd | na | na | nd | na | nd | nd | nd |

**Brain-derived neurotrophic factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2080 | 1580 | 2080 | 1580 | nd | nd |
| Average | 2630 | 2650 | 2630 | 2650 | nd | nd |
| Stdev | 2350 | 2210 | 2350 | 2210 | nd | nd |
| p(t-test) | | 0.98 | | 0.98 | nd | nd |
| Min | 24.3 | 468 | 24.3 | 468 | nd | nd |
| Max | 10700 | 6290 | 10700 | 6290 | nd | nd |
| n (Samp) | 76 | 8 | 76 | 8 | nd | nd |
| n (Patient) | 76 | 8 | 76 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1840 | 2650 | 1840 | 2650 | nd | nd |
| Average | 2210 | 3160 | 2210 | 3160 | nd | nd |
| Stdev | 1710 | 2370 | 1710 | 2370 | nd | nd |
| p(t-test) | | 0.21 | | 0.21 | nd | nd |
| Min | 24.3 | 468 | 24.3 | 468 | nd | nd |
| Max | 7310 | 6290 | 7310 | 6290 | nd | nd |
| n (Samp) | 64 | 6 | 64 | 6 | nd | nd |
| n (Patient) | 64 | 6 | 64 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | nd | 0.63 | 0.51 | nd | 0.63 | nd | nd | nd |
| SE | 0.11 | nd | 0.13 | 0.11 | nd | 0.13 | nd | nd | nd |
| p | 0.91 | nd | 0.32 | 0.91 | nd | 0.32 | nd | nd | nd |
| nCohort 1 | 76 | nd | 64 | 76 | nd | 64 | nd | nd | nd |
| nCohort 2 | 8 | nd | 6 | 8 | nd | 6 | nd | nd | nd |
| Cutoff 1 | 1150 | nd | 1250 | 1150 | nd | 1250 | nd | nd | nd |
| Sens 1 | 75% | nd | 83% | 75% | nd | 83% | nd | nd | nd |
| Spec 1 | 29% | nd | 34% | 29% | nd | 34% | nd | nd | nd |
| Cutoff 2 | 1010 | nd | 1250 | 1010 | nd | 1250 | nd | nd | nd |
| Sens 2 | 88% | nd | 83% | 88% | nd | 83% | nd | nd | nd |
| Spec 2 | 28% | nd | 34% | 28% | nd | 34% | nd | nd | nd |
| Cutoff 3 | 449 | nd | 428 | 449 | nd | 428 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 100% | nd | 100% | 100% | nd | 100% | nd | nd | nd |
| Spec 3 | 16% | nd | 14% | 16% | nd | 14% | nd | nd | nd |
| Cutoff 4 | 3100 | nd | 3010 | 3100 | nd | 3010 | nd | nd | nd |
| Sens 4 | 38% | nd | 50% | 38% | nd | 50% | nd | nd | nd |
| Spec 4 | 71% | nd | 70% | 71% | nd | 70% | nd | nd | nd |
| Cutoff 5 | 3590 | nd | 3210 | 3590 | nd | 3210 | nd | nd | nd |
| Sens 5 | 25% | nd | 50% | 25% | nd | 50% | nd | nd | nd |
| Spec 5 | 80% | nd | 83% | 80% | nd | 83% | nd | nd | nd |
| Cutoff 6 | 5710 | nd | 4810 | 5710 | nd | 4810 | nd | nd | nd |
| Sens 6 | 12% | nd | 33% | 12% | nd | 33% | nd | nd | nd |
| Spec 6 | 91% | nd | 91% | 91% | nd | 91% | nd | nd | nd |
| OR Quart 2 | 4.7 | nd | 2.0 | 4.7 | nd | 2.0 | nd | nd | nd |
| p Value | 0.18 | nd | 0.59 | 0.18 | nd | 0.59 | nd | nd | nd |
| 95% CI of | 0.48 | nd | 0.16 | 0.48 | nd | 0.16 | nd | nd | nd |
| OR Quart2 | 46 | nd | 24 | 46 | nd | 24 | nd | nd | nd |
| OR Quart 3 | 0 | nd | 0 | 0 | nd | 0 | nd | nd | nd |
| p Value | na | nd | na | na | nd | na | nd | nd | nd |
| 95% CI of | na | nd | na | na | nd | na | nd | nd | nd |
| OR Quart3 | na | nd | na | na | nd | na | nd | nd | nd |
| OR Quart 4 | 3.3 | nd | 3.2 | 3.3 | nd | 3.2 | nd | nd | nd |
| p Value | 0.32 | nd | 0.34 | 0.32 | nd | 0.34 | nd | nd | nd |
| 95% CI of | 0.32 | nd | 0.30 | 0.32 | nd | 0.30 | nd | nd | nd |
| OR Quart4 | 35 | nd | 34 | 35 | nd | 34 | nd | nd | nd |

**Creatine Kinase-MB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.370 | 1.28 | 0.370 | 0.631 | 0.370 | 0.631 |
| Average | 0.986 | 1.76 | 0.986 | 1.57 | 0.986 | 1.81 |
| Stdev | 3.23 | 2.11 | 3.23 | 2.17 | 3.23 | 2.90 |
| p(t-test) | | 0.36 | | 0.49 | | 0.49 |
| Min | 0.00420 | 0.0646 | 0.00420 | 0.0646 | 0.00420 | 0.0646 |
| Max | 32.5 | 8.69 | 32.5 | 8.69 | 32.5 | 8.69 |
| n (Samp) | 111 | 16 | 111 | 16 | 111 | 8 |
| n (Patient) | 111 | 16 | 111 | 16 | 111 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.401 | 0.850 | 0.401 | 0.850 | nd | nd |
| Average | 1.03 | 1.92 | 1.03 | 1.90 | nd | nd |
| Stdev | 2.69 | 2.87 | 2.69 | 2.87 | nd | nd |
| p(t-test) | | 0.36 | | 0.37 | nd | nd |
| Min | 0.00420 | 0.0646 | 0.00420 | 0.0646 | nd | nd |
| Max | 32.5 | 8.69 | 32.5 | 8.69 | nd | nd |
| n (Samp) | 180 | 8 | 180 | 8 | nd | nd |
| n (Patient) | 180 | 8 | 180 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.392 | 1.73 | 0.392 | 0.537 | 0.392 | 0.939 |
| Average | 1.23 | 2.18 | 1.23 | 1.90 | 1.23 | 2.28 |
| Stdev | 3.60 | 2.52 | 3.60 | 2.64 | 3.60 | 3.27 |
| p(t-test) | | 0.42 | | 0.57 | | 0.49 |
| Min | 0.00420 | 0.111 | 0.00420 | 0.0849 | 0.00420 | 0.0721 |
| Max | 32.5 | 8.69 | 32.5 | 8.69 | 32.5 | 8.69 |
| n (Samp) | 91 | 10 | 91 | 10 | 91 | 6 |
| n (Patient) | 91 | 10 | 91 | 10 | 91 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.63 | 0.72 | 0.63 | 0.61 | 0.62 | 0.63 | nd | 0.66 |
| SE | 0.076 | 0.11 | 0.095 | 0.079 | 0.11 | 0.099 | 0.11 | nd | 0.13 |
| p | 0.0067 | 0.23 | 0.020 | 0.093 | 0.29 | 0.23 | 0.25 | nd | 0.21 |
| nCohort 1 | 111 | 180 | 91 | 111 | 180 | 91 | 111 | nd | 91 |
| nCohort 2 | 16 | 8 | 10 | 16 | 8 | 10 | 8 | nd | 6 |
| Cutoff 1 | 0.416 | 0.392 | 0.528 | 0.405 | 0.392 | 0.416 | 0.416 | nd | 0.416 |
| Sens 1 | 75% | 75% | 70% | 75% | 75% | 70% | 75% | nd | 83% |
| Spec 1 | 58% | 49% | 63% | 57% | 49% | 54% | 58% | nd | 54% |
| Cutoff 2 | 0.392 | 0.162 | 0.416 | 0.0849 | 0.0778 | 0.405 | 0.0716 | nd | 0.416 |
| Sens 2 | 81% | 88% | 80% | 81% | 88% | 80% | 88% | nd | 83% |
| Spec 2 | 54% | 18% | 54% | 9% | 6% | 53% | 7% | nd | 54% |
| Cutoff 3 | 0.0983 | 0.0597 | 0.392 | 0.0778 | 0.0597 | 0.0849 | 0.0597 | nd | 0.0716 |
| Sens 3 | 94% | 100% | 90% | 94% | 100% | 90% | 100% | nd | 100% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 3 | 12% | 5% | 51% | 8% | 5% | 10% | 6% | nd | 8% |
| Cutoff 4 | 0.542 | 0.630 | 0.629 | 0.542 | 0.630 | 0.629 | 0.542 | nd | 0.629 |
| Sens 4 | 62% | 62% | 60% | 50% | 62% | 40% | 50% | nd | 50% |
| Spec 4 | 70% | 70% | 70% | 70% | 70% | 70% | 70% | nd | 70% |
| Cutoff 5 | 0.657 | 1.10 | 1.13 | 0.657 | 1.10 | 1.13 | 0.657 | nd | 1.13 |
| Sens 5 | 62% | 38% | 60% | 50% | 38% | 40% | 50% | nd | 50% |
| Spec 5 | 80% | 81% | 80% | 80% | 81% | 80% | 80% | nd | 80% |
| Cutoff 6 | 1.41 | 2.46 | 2.75 | 1.41 | 2.46 | 2.75 | 1.41 | nd | 2.75 |
| Sens 6 | 50% | 25% | 20% | 38% | 25% | 20% | 25% | nd | 17% |
| Spec 6 | 90% | 90% | 90% | 90% | 90% | 90% | 90% | nd | 90% |
| OR Quart 2 | 0 | 0 | 1.0 | 0 | 0 | 0 | 0 | nd | 0 |
| p Value | na | na | 1.0 | na | na | na | na | nd | na |
| 95% CI of | na | na | 0.059 | na | na | na | na | nd | na |
| OR Quart2 | na | na | 17 | na | na | na | na | nd | na |
| OR Quart 3 | 0.97 | 1.0 | 2.1 | 0.96 | 1.0 | 2.2 | 0.96 | nd | 2.1 |
| p Value | 0.97 | 1.0 | 0.56 | 0.96 | 1.0 | 0.39 | 0.97 | nd | 0.56 |
| 95% CI of | 0.18 | 0.13 | 0.18 | 0.22 | 0.13 | 0.36 | 0.13 | nd | 0.18 |
| OR Quart3 | 5.2 | 7.4 | 25 | 4.3 | 7.4 | 13 | 7.3 | nd | 25 |
| OR Quart 4 | 4.2 | 2.1 | 7.2 | 2.2 | 2.1 | 2.1 | 2.1 | nd | 3.1 |
| p Value | 0.044 | 0.41 | 0.078 | 0.23 | 0.41 | 0.42 | 0.42 | nd | 0.34 |
| 95% CI of | 1.0 | 0.36 | 0.80 | 0.60 | 0.36 | 0.35 | 0.35 | nd | 0.30 |
| OR Quart4 | 17 | 12 | 65 | 8.4 | 12 | 13 | 12 | nd | 32 |

**Immunoglobulin M**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.656 | 0.943 | 0.656 | 0.938 | 0.656 | 1.06 |
| Average | 0.886 | 1.09 | 0.886 | 1.04 | 0.886 | 1.40 |
| Stdev | 0.755 | 0.792 | 0.755 | 0.824 | 0.755 | 0.943 |
| p(t-test) |  | 0.32 |  | 0.45 |  | 0.069 |
| Min | 0.0493 | 0.173 | 0.0493 | 0.0919 | 0.0493 | 0.586 |
| Max | 3.81 | 3.51 | 3.81 | 3.51 | 3.81 | 3.51 |
| n (Samp) | 111 | 16 | 111 | 16 | 111 | 8 |
| n (Patient) | 111 | 16 | 111 | 16 | 111 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.636 | 1.11 | 0.636 | 1.04 | nd | nd |
| Average | 0.852 | 1.12 | 0.852 | 1.07 | nd | nd |
| Stdev | 0.761 | 0.505 | 0.761 | 0.544 | nd | nd |
| p(t-test) | | 0.32 | | 0.42 | nd | nd |
| Min | 0.0493 | 0.557 | 0.0493 | 0.430 | nd | nd |
| Max | 4.82 | 1.79 | 4.82 | 1.79 | nd | nd |
| n (Samp) | 180 | 8 | 180 | 8 | nd | nd |
| n (Patient) | 180 | 8 | 180 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.614 | 0.943 | 0.614 | 0.943 | 0.614 | 1.06 |
| Average | 0.815 | 1.11 | 0.815 | 1.07 | 0.815 | 1.47 |
| Stdev | 0.660 | 0.933 | 0.660 | 0.965 | 0.660 | 1.04 |
| p(t-test) | | 0.21 | | 0.27 | | 0.025 |
| Min | 0.132 | 0.173 | 0.132 | 0.0919 | 0.132 | 0.804 |
| Max | 3.75 | 3.51 | 3.75 | 3.51 | 3.75 | 3.51 |
| n (Samp) | 91 | 10 | 91 | 10 | 91 | 6 |
| n (Patient) | 91 | 10 | 91 | 10 | 91 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.70 | 0.62 | 0.58 | 0.66 | 0.59 | 0.73 | nd | 0.78 |
| SE | 0.079 | 0.11 | 0.099 | 0.079 | 0.11 | 0.099 | 0.10 | nd | 0.11 |
| p | 0.16 | 0.061 | 0.21 | 0.33 | 0.14 | 0.38 | 0.030 | nd | 0.016 |
| nCohort 1 | 111 | 180 | 91 | 111 | 180 | 91 | 111 | nd | 91 |
| nCohort 2 | 16 | 8 | 10 | 16 | 8 | 10 | 8 | nd | 6 |
| Cutoff 1 | 0.578 | 0.613 | 0.799 | 0.460 | 0.578 | 0.799 | 0.804 | nd | 0.804 |
| Sens 1 | 75% | 75% | 70% | 75% | 75% | 70% | 75% | nd | 83% |
| Spec 1 | 44% | 48% | 64% | 34% | 46% | 64% | 60% | nd | 64% |
| Cutoff 2 | 0.575 | 0.578 | 0.570 | 0.422 | 0.466 | 0.331 | 0.799 | nd | 0.804 |
| Sens 2 | 81% | 88% | 80% | 81% | 88% | 80% | 88% | nd | 83% |
| Spec 2 | 42% | 46% | 46% | 33% | 33% | 21% | 60% | nd | 64% |
| Cutoff 3 | 0.370 | 0.555 | 0.370 | 0.313 | 0.422 | 0.290 | 0.578 | nd | 0.799 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | 94% | 100% | 90% | 94% | 100% | 90% | 100% | nd | 100% |
| Spec 3 | 26% | 42% | 26% | 20% | 32% | 20% | 44% | nd | 64% |
| Cutoff 4 | 0.998 | 0.887 | 0.850 | 0.998 | 0.887 | 0.850 | 0.998 | nd | 0.850 |
| Sens 4 | 50% | 62% | 60% | 50% | 62% | 60% | 62% | nd | 67% |
| Spec 4 | 70% | 70% | 70% | 70% | 70% | 70% | 70% | nd | 70% |
| Cutoff 5 | 1.28 | 1.13 | 1.26 | 1.28 | 1.13 | 1.26 | 1.28 | nd | 1.26 |
| Sens 5 | 25% | 50% | 20% | 25% | 38% | 20% | 38% | nd | 33% |
| Spec 5 | 81% | 80% | 81% | 81% | 80% | 81% | 81% | nd | 81% |
| Cutoff 6 | 1.82 | 1.77 | 1.77 | 1.82 | 1.77 | 1.77 | 1.82 | nd | 1.77 |
| Sens 6 | 6% | 12% | 10% | 6% | 12% | 10% | 12% | nd | 17% |
| Spec 6 | 90% | 90% | 90% | 90% | 90% | 90% | 90% | nd | 90% |
| OR Quart 2 | 2.1 | >3.2 | 2.1 | 0.97 | >3.2 | 0 | >1.0 | nd | >0 |
| p Value | 0.42 | <0.32 | 0.56 | 0.97 | <0.32 | na | <1.0 | nd | <na |
| 95% CI of | 0.35 | >0.32 | 0.18 | 0.18 | >0.32 | na | >0.060 | nd | >na |
| OR Quart2 | 12 | na | 25 | 5.2 | na | na | na | nd | na |
| OR Quart 3 | 2.7 | >1.0 | 4.6 | 2.2 | >2.1 | 1.4 | >4.5 | nd | >3.4 |
| p Value | 0.26 | <0.99 | 0.19 | 0.31 | <0.55 | 0.68 | <0.19 | nd | <0.30 |
| 95% CI of | 0.48 | >0.062 | 0.47 | 0.49 | >0.18 | 0.28 | >0.47 | nd | >0.33 |
| OR Quart3 | 15 | na | 44 | 9.5 | na | 7.0 | na | nd | na |
| OR Quart 4 | 2.7 | >4.4 | 3.1 | 1.3 | >3.2 | 0.96 | >3.2 | nd | >3.3 |
| p Value | 0.26 | <0.19 | 0.34 | 0.72 | <0.32 | 0.96 | <0.32 | nd | <0.32 |
| 95% CI of | 0.48 | >0.47 | 0.30 | 0.27 | >0.32 | 0.17 | >0.32 | nd | >0.32 |
| OR Quart4 | 15 | na | 32 | 6.5 | na | 5.3 | na | nd | na |

**Insulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 4.17 | 4.44 | 4.17 | 4.44 | 4.17 | 4.44 |
| Average | 8.54 | 38.1 | 8.54 | 21.5 | 8.54 | 27.9 |
| Stdev | 11.4 | 83.8 | 11.4 | 49.3 | 11.4 | 68.8 |
| p(t-test) |  | 5.1E-4 |  | 0.018 |  | 0.0098 |
| Min | 0.00859 | 0.593 | 0.00859 | 0.00859 | 0.00859 | 0.00859 |
| Max | 87.2 | 293 | 87.2 | 198 | 87.2 | 198 |
| n (Samp) | 111 | 16 | 111 | 16 | 111 | 8 |
| n (Patient) | 111 | 16 | 111 | 16 | 111 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.66 | 9.36 | 4.66 | 6.93 | nd | nd |
| Average | 9.20 | 13.4 | 9.20 | 12.8 | nd | nd |
| Stdev | 15.5 | 17.0 | 15.5 | 17.2 | nd | nd |
| p(t-test) | | 0.45 | | 0.52 | nd | nd |
| Min | 0.00859 | 0.593 | 0.00859 | 0.593 | nd | nd |
| Max | 162 | 53.1 | 162 | 53.1 | nd | nd |
| n (Samp) | 180 | 8 | 180 | 8 | nd | nd |
| n (Patient) | 180 | 8 | 180 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.38 | 2.02 | 4.38 | 1.93 | 4.38 | 2.26 |
| Average | 9.38 | 50.8 | 9.38 | 24.7 | 9.38 | 34.9 |
| Stdev | 12.4 | 105 | 12.4 | 61.7 | 12.4 | 79.9 |
| p(t-test) | | 3.9E-4 | | 0.039 | | 0.0070 |
| Min | 0.00859 | 0.593 | 0.00859 | 0.00859 | 0.00859 | 0.00859 |
| Max | 87.2 | 293 | 87.2 | 198 | 87.2 | 198 |
| n (Samp) | 91 | 10 | 91 | 10 | 91 | 6 |
| n (Patient) | 91 | 10 | 91 | 10 | 91 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.58 | 0.39 | 0.48 | 0.56 | 0.37 | 0.44 | nd | 0.38 |
| SE | 0.078 | 0.11 | 0.099 | 0.078 | 0.11 | 0.099 | 0.11 | nd | 0.13 |
| p | 0.99 | 0.47 | 0.26 | 0.75 | 0.61 | 0.18 | 0.61 | nd | 0.33 |
| nCohort 1 | 111 | 180 | 91 | 111 | 180 | 91 | 111 | nd | 91 |
| nCohort 2 | 16 | 8 | 10 | 16 | 8 | 10 | 8 | nd | 6 |
| Cutoff 1 | 1.34 | 5.66 | 1.33 | 0.976 | 5.66 | 0.976 | 1.34 | nd | 0.976 |
| Sens 1 | 75% | 75% | 70% | 75% | 75% | 70% | 75% | nd | 83% |
| Spec 1 | 15% | 56% | 16% | 10% | 56% | 12% | 15% | nd | 12% |
| Cutoff 2 | 0.976 | 0.593 | 0.976 | 0.837 | 0.593 | 0.837 | 0.976 | nd | 0.976 |
| Sens 2 | 81% | 88% | 80% | 81% | 88% | 80% | 88% | nd | 83% |
| Spec 2 | 10% | 3% | 12% | 8% | 3% | 10% | 10% | nd | 12% |
| Cutoff 3 | 0.593 | 0.565 | 0.837 | 0.509 | 0.565 | 0.509 | 0 | nd | 0 |
| Sens 3 | 94% | 100% | 90% | 94% | 100% | 90% | 100% | nd | 100% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 5% | 3% | 10% | 5% | 3% | 4% | 0% | nd | 0% |
| Cutoff 4 | 9.98 | 9.98 | 11.3 | 9.98 | 9.98 | 11.3 | 9.98 | nd | 11.3 |
| Sens 4 | 38% | 50% | 20% | 31% | 38% | 20% | 12% | nd | 17% |
| Spec 4 | 70% | 70% | 70% | 70% | 70% | 70% | 70% | nd | 70% |
| Cutoff 5 | 13.7 | 12.7 | 14.5 | 13.7 | 12.7 | 14.5 | 13.7 | nd | 14.5 |
| Sens 5 | 25% | 25% | 20% | 25% | 25% | 20% | 12% | nd | 17% |
| Spec 5 | 80% | 80% | 81% | 80% | 80% | 81% | 80% | nd | 81% |
| Cutoff 6 | 24.3 | 19.6 | 24.7 | 24.3 | 19.6 | 24.7 | 24.3 | nd | 24.7 |
| Sens 6 | 19% | 12% | 20% | 19% | 12% | 20% | 12% | nd | 17% |
| Spec 6 | 90% | 90% | 90% | 90% | 90% | 90% | 90% | nd | 90% |
| OR Quart 2 | 1.0 | 0 | 0.50 | 1.0 | 0 | 0.50 | 3.2 | nd | 1.0 |
| p Value | 1.0 | na | 0.58 | 1.0 | na | 0.58 | 0.32 | nd | 0.98 |
| 95% CI of | 0.23 | na | 0.042 | 0.23 | na | 0.042 | 0.32 | nd | 0.062 |
| OR Quart2 | 4.4 | na | 5.9 | 4.4 | na | 5.9 | 33 | nd | 18 |
| OR Quart 3 | 0.47 | 2.1 | 1.0 | 0.47 | 2.1 | 1.0 | 1.0 | nd | 1.0 |
| p Value | 0.40 | 0.41 | 0.97 | 0.40 | 0.41 | 0.97 | 1.0 | nd | 0.98 |
| 95% CI of | 0.079 | 0.36 | 0.14 | 0.079 | 0.36 | 0.14 | 0.060 | nd | 0.062 |
| OR Quart3 | 2.8 | 12 | 8.0 | 2.8 | 12 | 8.0 | 17 | nd | 18 |
| OR Quart 4 | 1.7 | 1.0 | 3.0 | 1.7 | 1.0 | 3.0 | 3.3 | nd | 3.4 |
| p Value | 0.46 | 1.0 | 0.22 | 0.46 | 1.0 | 0.22 | 0.31 | nd | 0.30 |
| 95% CI of | 0.42 | 0.13 | 0.52 | 0.42 | 0.13 | 0.52 | 0.33 | nd | 0.33 |
| OR Quart4 | 6.6 | 7.4 | 17 | 6.6 | 7.4 | 17 | 34 | nd | 36 |

Table 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Angiopoietin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.38 | 3.15 | 6.38 | 8.81 | 6.38 | 4.88 |
| Average | 17.9 | 280 | 17.9 | 624 | 17.9 | 5.85 |
| Stdev | 69.2 | 947 | 69.2 | 2330 | 69.2 | 5.55 |
| p(t-test) | | 3.5E-13 | | 4.4E-15 | | 0.64 |
| Min | 1.00E-9 | 1.00E-9 | 1.00E-9 | 0.505 | 1.00E-9 | 1.00E-9 |
| Max | 1730 | 3430 | 1730 | 9360 | 1730 | 15.2 |
| n (Samp) | 933 | 13 | 933 | 16 | 933 | 7 |
| n (Patient) | 344 | 13 | 344 | 16 | 344 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.34 | 2.11 | nd | nd | nd | nd |
| Average | 27.4 | 500 | nd | nd | nd | nd |
| Stdev | 308 | 1290 | nd | nd | nd | nd |
| p(t-test) | | 1.2E-4 | nd | nd | nd | nd |
| Min | 1.00E-9 | 0.318 | nd | nd | nd | nd |
| Max | 9360 | 3430 | nd | nd | nd | nd |
| n (Samp) | 970 | 7 | nd | nd | nd | nd |
| n (Patient) | 354 | 7 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.32 | 3.15 | 6.32 | 11.6 | nd | nd |
| Average | 18.9 | 439 | 18.9 | 712 | nd | nd |
| Stdev | 74.3 | 1210 | 74.3 | 2490 | nd | nd |
| p(t-test) | | 1.1E-17 | | 6.9E-15 | nd | nd |
| Min | 1.00E-9 | 1.00E-9 | 1.00E-9 | 0.505 | nd | nd |
| Max | 1730 | 3430 | 1730 | 9360 | nd | nd |
| n (Samp) | 798 | 8 | 798 | 14 | nd | nd |
| n (Patient) | 261 | 8 | 261 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | Cr only | UO only | sCr or UO | sCr only | UO only | sCr or UO | sCr only | UO only |
| AUC | 0.42 | 0.36 | 0.39 | 0.54 | nd | 0.58 | 0.37 | nd | nd |
| SE | 0.083 | 0.11 | 0.11 | 0.074 | nd | 0.080 | 0.11 | nd | nd |
| p | 0.33 | 0.21 | 0.31 | 0.57 | nd | 0.35 | 0.25 | nd | nd |
| nCohort 1 | 933 | 970 | 798 | 933 | nd | 798 | 933 | nd | nd |
| nCohort 2 | 13 | 7 | 8 | 16 | nd | 14 | 7 | nd | nd |
| Cutoff 1 | 1.89 | 1.39 | 1.89 | 3.43 | nd | 6.54 | 1.84 | nd | nd |
| Sens 1 | 77% | 71% | 75% | 75% | nd | 71% | 71% | nd | nd |
| Spec 1 | 15% | 10% | 15% | 30% | nd | 52% | 15% | nd | nd |
| Cutoff 2 | 1.39 | 0.384 | 1.39 | 2.45 | nd | 2.42 | 0.976 | nd | nd |
| Sens 2 | 85% | 86% | 88% | 81% | nd | 86% | 86% | nd | nd |
| Spec 2 | 10% | 3% | 11% | 21% | nd | 21% | 8% | nd | nd |
| Cutoff 3 | 0.384 | 0.317 | 0 | 0.517 | nd | 0.666 | 0 | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | Cr only | UO only | sCr or UO | sCr only | UO only | sCr or UO | sCr only | UO only |
| Sens 3 | 92% | 100% | 100% | 94% | nd | 93% | 100% | nd | nd |
| Spec 3 | 3% | 2% | 0% | 4% | nd | 5% | 0% | nd | nd |
| Cutoff 4 | 12.9 | 12.7 | 13.3 | 12.9 | nd | 13.3 | 12.9 | nd | nd |
| Sens 4 | 31% | 29% | 25% | 44% | nd | 50% | 14% | nd | nd |
| Spec 4 | 70% | 70% | 70% | 70% | nd | 70% | 70% | nd | nd |
| Cutoff 5 | 19.2 | 18.7 | 19.7 | 19.2 | nd | 19.7 | 19.2 | nd | nd |
| Sens 5 | 31% | 29% | 25% | 19% | nd | 14% | 0% | nd | nd |
| Spec 5 | 80% | 80% | 80% | 80% | nd | 80% | 80% | nd | nd |
| Cutoff 6 | 34.9 | 34.0 | 36.0 | 34.9 | nd | 36.0 | 34.9 | nd | nd |
| Sens 6 | 31% | 29% | 25% | 12% | nd | 14% | 0% | nd | nd |
| Spec 6 | 90% | 90% | 90% | 90% | nd | 90% | 90% | nd | nd |
| OR Quart 2 | 0 | 0 | 0 | 0.25 | nd | 0.33 | >3.0 | nd | nd |
| p Value | na | na | na | 0.21 | nd | 0.34 | <0.34 | nd | nd |
| 95% CI of | na | na | na | 0.027 | nd | 0.034 | >0.31 | nd | nd |
| OR Quart2 | na | na | na | 2.2 | nd | 3.2 | na | nd | nd |
| OR Quart 3 | 0.75 | 0 | 1.5 | 1.5 | nd | 1.7 | >1.0 | nd | nd |
| p Value | 0.70 | na | 0.65 | 0.53 | nd | 0.48 | <1.00 | nd | nd |
| 95% CI of | 0.17 | na | 0.25 | 0.42 | nd | 0.40 | >0.062 | nd | nd |
| OR Quart3 | 3.4 | na | 9.1 | 5.4 | nd | 7.1 | na | nd | nd |
| OR Quart 4 | 1.5 | 2.5 | 1.5 | 1.2 | nd | 1.7 | >3.0 | nd | nd |
| p Value | 0.52 | 0.27 | 0.65 | 0.74 | nd | 0.48 | <0.34 | nd | nd |
| | sCr or UO | sCr only | UO only | sCr or UO | sCr only | UO only | sCr or UO | sCr only | UO only |
| 95% CI of | 0.42 | 0.49 | 0.25 | 0.33 | nd | 0.40 | >0.31 | nd | nd |
| OR Quart4 | 5.5 | 13 | 9.2 | 4.7 | nd | 7.1 | na | nd | nd |

**Brain-derived neurotrophic factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.397 | 1.05 | 0.397 | 1.45 | 0.397 | 0.869 |
| Average | 2.47 | 2.42 | 2.47 | 86.7 | 2.47 | 0.739 |
| Stdev | 22.4 | 3.46 | 22.4 | 232 | 22.4 | 0.589 |
| p(t-test) | | 0.99 | | 4.5E-19 | | 0.84 |
| Min | 1.00E-9 | 1.00E-9 | 1.00E-9 | 0.158 | 1.00E-9 | 0.0469 |
| Max | 624 | 9.49 | 624 | 732 | 624 | 1.52 |
| n (Samp) | 932 | 13 | 932 | 16 | 932 | 7 |
| n (Patient) | 344 | 13 | 344 | 16 | 344 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.403 | 1.21 | nd | nd | nd | nd |
| Average | 3.88 | 2.53 | nd | nd | nd | nd |
| Stdev | 37.8 | 3.06 | nd | nd | nd | nd |
| p(t-test) | | 0.92 | nd | nd | nd | nd |
| Min | 1.00E-9 | 0.0280 | nd | nd | nd | nd |
| Max | 732 | 7.21 | nd | nd | nd | nd |
| n (Samp) | 969 | 7 | nd | nd | nd | nd |
| n (Patient) | 354 | 7 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.403 | 0.703 | 0.403 | 2.28 | nd | nd |
| Average | 1.98 | 3.31 | 1.98 | 99.0 | nd | nd |
| Stdev | 9.97 | 4.23 | 9.97 | 246 | nd | nd |
| p(t-test) | | 0.70 | | 2.8E-26 | nd | nd |
| Min | 1.00E-9 | 1.00E-9 | 1.00E-9 | 0.158 | nd | nd |
| Max | 237 | 9.49 | 237 | 732 | nd | nd |
| n (Samp) | 797 | 8 | 797 | 14 | nd | nd |
| n (Patient) | 261 | 8 | 261 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.67 | 0.57 | 0.76 | nd | 0.75 | 0.59 | nd | nd |
| SE | 0.084 | 0.11 | 0.11 | 0.071 | nd | 0.076 | 0.11 | nd | nd |
| p | 0.16 | 0.12 | 0.53 | 2.8E-4 | nd | 0.0010 | 0.45 | nd | nd |
| nCohort 1 | 932 | 969 | 797 | 932 | nd | 797 | 932 | nd | nd |
| nCohort 2 | 13 | 7 | 8 | 16 | nd | 14 | 7 | nd | nd |
| Cutoff 1 | 0.215 | 0.524 | 0.0360 | 0.610 | nd | 0.610 | 0.359 | nd | nd |
| Sens 1 | 77% | 71% | 75% | 75% | nd | 71% | 71% | nd | nd |
| Spec 1 | 37% | 56% | 23% | 63% | nd | 61% | 48% | nd | nd |
| Cutoff 2 | 0.0360 | 0.235 | 0.0249 | 0.547 | nd | 0.404 | 0.0967 | nd | nd |
| Sens 2 | 85% | 86% | 88% | 81% | nd | 86% | 86% | nd | nd |
| Spec 2 | 25% | 40% | 19% | 59% | nd | 51% | 30% | nd | nd |
| Cutoff 3 | 0.0249 | 0.0249 | 0 | 0.215 | nd | 0.215 | 0.0466 | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 92% | 100% | 100% | 94% | nd | 93% | 100% | nd | nd |
| Spec 3 | 21% | 20% | 0% | 37% | nd | 35% | 25% | nd | nd |
| Cutoff 4 | 0.853 | 0.885 | 0.885 | 0.853 | nd | 0.885 | 0.853 | nd | nd |
| Sens 4 | 54% | 57% | 50% | 56% | nd | 57% | 57% | nd | nd |
| Spec 4 | 70% | 70% | 71% | 70% | nd | 71% | 70% | nd | nd |
| Cutoff 5 | 1.26 | 1.37 | 1.36 | 1.26 | nd | 1.36 | 1.26 | nd | nd |
| Sens 5 | 31% | 43% | 38% | 50% | nd | 50% | 29% | nd | nd |
| Spec 5 | 80% | 80% | 80% | 80% | nd | 80% | 80% | nd | nd |
| Cutoff 6 | 2.56 | 3.07 | 2.75 | 2.56 | nd | 2.75 | 2.56 | nd | nd |
| Sens 6 | 23% | 29% | 38% | 44% | nd | 50% | 0% | nd | nd |
| Spec 6 | 90% | 90% | 90% | 90% | nd | 90% | 90% | nd | nd |
| OR Quart 2 | 0.66 | 1.0 | 0.33 | >2.0 | nd | >2.0 | >3.0 | nd | nd |
| p Value | 0.66 | 1.0 | 0.34 | <0.57 | nd | <0.57 | <0.34 | nd | nd |
| 95% CI of | 0.11 | 0.062 | 0.034 | >0.18 | nd | >0.18 | >0.31 | nd | nd |
| OR Quart2 | 4.0 | 16 | 3.2 | na | nd | na | na | nd | nd |
| OR Quart 3 | 0.33 | 1.0 | 0 | >5.1 | nd | >4.1 | >2.0 | nd | nd |
| p Value | 0.34 | 1.0 | na | <0.14 | nd | <0.21 | <0.57 | nd | nd |
| 95% CI of | 0.034 | 0.062 | na | >0.59 | nd | >0.45 | >0.18 | nd | nd |
| OR Quart3 | 3.2 | 16 | na | na | nd | na | na | nd | nd |
| OR Quart 4 | 2.4 | 4.0 | 1.3 | >9.4 | nd | >8.3 | >2.0 | nd | nd |
| p Value | 0.22 | 0.21 | 0.71 | <0.035 | nd | <0.047 | <0.57 | nd | nd |
| 95% CI of | 0.60 | 0.45 | 0.29 | >1.2 | nd | >1.0 | >0.18 | nd | nd |
| OR Quart4 | 9.3 | 36 | 6.0 | na | nd | na | na | nd | nd |

**Immunoglobulin E**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0560 | 0.0560 | 0.0560 | 0.138 | nd | nd |
| Average | 1.24 | 0.138 | 1.24 | 6.26 | nd | nd |
| Stdev | 2.04 | 0.160 | 2.04 | 17.4 | nd | nd |
| p(t-test) | | 0.088 | | 1.6E-7 | nd | nd |
| Min | 0.0560 | 0.0560 | 0.0560 | 0.0560 | nd | nd |
| Max | 9.65 | 0.523 | 9.65 | 58.4 | nd | nd |
| n (Samp) | 534 | 10 | 534 | 11 | nd | nd |
| n (Patient) | 204 | 10 | 204 | 11 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0560 | 0.0560 | 0.0560 | 0.0970 | nd | nd |
| Average | 1.18 | 0.106 | 1.18 | 8.31 | nd | nd |
| Stdev | 1.95 | 0.101 | 1.95 | 20.3 | nd | nd |
| p(t-test) | | 0.15 | | 6.4E-10 | nd | nd |
| Min | 0.0560 | 0.0560 | 0.0560 | 0.0560 | nd | nd |
| Max | 9.65 | 0.324 | 9.65 | 58.4 | nd | nd |
| n (Samp) | 454 | 7 | 454 | 8 | nd | nd |
| n (Patient) | 168 | 7 | 168 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.36 | nd | 0.36 | 0.55 | nd | 0.55 | nd | nd | nd |
| SE | 0.096 | nd | 0.11 | 0.090 | nd | 0.11 | nd | nd | nd |
| p | 0.16 | nd | 0.22 | 0.57 | nd | 0.64 | nd | nd | nd |
| nCohort 1 | 534 | nd | 454 | 534 | nd | 454 | nd | nd | nd |
| nCohort 2 | 10 | nd | 7 | 11 | nd | 8 | nd | nd | nd |
| Cutoff 1 | 0 | nd | 0 | 0 | nd | 0 | nd | nd | nd |
| Sens 1 | 100% | nd | 100% | 100% | nd | 100% | nd | nd | nd |
| Spec 1 | 0% | nd | 0% | 0% | nd | 0% | nd | nd | nd |
| Cutoff 2 | 0 | nd | 0 | 0 | nd | 0 | nd | nd | nd |
| Sens 2 | 100% | nd | 100% | 100% | nd | 100% | nd | nd | nd |
| Spec 2 | 0% | nd | 0% | 0% | nd | 0% | nd | nd | nd |
| Cutoff 3 | 0 | nd | 0 | 0 | nd | 0 | nd | nd | nd |
| Sens 3 | 100% | nd | 100% | 100% | nd | 100% | nd | nd | nd |
| Spec 3 | 0% | nd | 0% | 0% | nd | 0% | nd | nd | nd |
| Cutoff 4 | 0.834 | nd | 0.827 | 0.834 | nd | 0.827 | nd | nd | nd |
| Sens 4 | 0% | nd | 0% | 45% | nd | 38% | nd | nd | nd |
| Spec 4 | 70% | nd | 70% | 70% | nd | 70% | nd | nd | nd |
| Cutoff 5 | 2.51 | nd | 2.33 | 2.51 | nd | 2.33 | nd | nd | nd |
| Sens 5 | 0% | nd | 0% | 27% | nd | 38% | nd | nd | nd |
| Spec 5 | 80% | nd | 80% | 80% | nd | 80% | nd | nd | nd |
| Cutoff 6 | 4.59 | nd | 4.51 | 4.59 | nd | 4.51 | nd | nd | nd |
| Sens 6 | 0% | nd | 0% | 18% | nd | 25% | nd | nd | nd |
| Spec 6 | 90% | nd | 90% | 90% | nd | 90% | nd | nd | nd |
| OR Quart 2 | >7.4 | nd | >2.1 | >5.2 | nd | >4.1 | nd | nd | nd |
| p Value | <0.063 | nd | <0.56 | <0.14 | nd | <0.21 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | >0.90 | nd | >0.18 | >0.60 | nd | >0.45 | nd | nd | nd |
| OR Quart2 | na | nd | na | na | nd | na | nd | nd | nd |
| OR Quart 3 | >3.1 | nd | >5.3 | >3.1 | nd | >1.0 | nd | nd | nd |
| p Value | <0.33 | nd | <0.13 | <0.33 | nd | <1.00 | nd | nd | nd |
| 95% CI of | >0.32 | nd | >0.61 | >0.32 | nd | >0.062 | nd | nd | nd |
| OR Quart3 | na | nd | na | na | nd | na | nd | nd | nd |
| OR Quart 4 | >0 | nd | >0 | >3.0 | nd | >3.1 | nd | nd | nd |
| p Value | <na | nd | <na | <0.34 | nd | <0.34 | nd | nd | nd |
| 95% CI of | >na | nd | >na | >0.31 | nd | >0.31 | nd | nd | nd |
| OR Quart4 | na | nd | na | na | nd | na | nd | nd | nd |

**Immunoglobulin M**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.51E-5 | 0.000221 | 9.51E-5 | 0.000374 | nd | nd |
| Average | 0.000447 | 0.000231 | 0.000447 | 0.00246 | nd | nd |
| Stdev | 0.00135 | 0.000144 | 0.00135 | 0.00551 | nd | nd |
| p(t-test) | | 0.61 | | 1.8E-5 | nd | nd |
| Min | 3.78E-8 | 1.38E-5 | 3.78E-8 | 5.94E-5 | nd | nd |
| Max | 0.0131 | 0.000481 | 0.0131 | 0.0186 | nd | nd |
| n (Samp) | 534 | 10 | 534 | 11 | nd | nd |
| n (Patient) | 204 | 10 | 204 | 11 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.72E-5 | 0.000201 | 9.72E-5 | 0.000317 | nd | nd |
| Average | 0.000399 | 0.000223 | 0.000399 | 0.00265 | nd | nd |
| Stdev | 0.00128 | 0.000137 | 0.00128 | 0.00645 | nd | nd |
| p(t-test) | | 0.72 | | 3.0E-5 | nd | nd |
| Min | 3.78E-8 | 3.45E-5 | 3.78E-8 | 5.94E-5 | nd | nd |
| Max | 0.0131 | 0.000481 | 0.0131 | 0.0186 | nd | nd |
| n (Samp) | 454 | 7 | 454 | 8 | nd | nd |
| n (Patient) | 168 | 7 | 168 | 8 | nd | nd |

| | | 0hr prior to AKI stage | | | 24hr prior to AKI stage | | | 48hr prior to AKI stage | | |
|---|---|---|---|---|---|---|---|---|---|---|
| | | sCr or UO | sCr only | UO only | sCr or UO | sCr only | UO only | sCr or UO | sCr only | UO only |
| | AUC | 0.62 | nd | 0.64 | 0.77 | nd | 0.74 | nd | nd | nd |
| | SE | 0.096 | nd | 0.11 | 0.084 | nd | 0.10 | nd | nd | nd |
| | p | 0.22 | nd | 0.22 | 0.0014 | nd | 0.020 | nd | nd | nd |
| | nCohort 1 | 534 | nd | 454 | 534 | nd | 454 | nd | nd | nd |
| | nCohort 2 | 10 | nd | 7 | 11 | nd | 8 | nd | nd | nd |
| | Cutoff 1 | 0.000199 | nd | 0.000191 | 0.000222 | nd | 0.000154 | nd | nd | nd |
| | Sens 1 | 70% | nd | 71% | 73% | nd | 75% | nd | nd | nd |
| | Spec 1 | 67% | nd | 66% | 69% | nd | 62% | nd | nd | nd |
| | Cutoff 2 | 0.000152 | nd | 0.000152 | 0.000154 | nd | 0.000107 | nd | nd | nd |
| | Sens 2 | 80% | nd | 86% | 82% | nd | 88% | nd | nd | nd |
| | Spec 2 | 62% | nd | 62% | 62% | nd | 53% | nd | nd | nd |
| | Cutoff 3 | 3.36E-5 | nd | 3.36E-5 | 0.000107 | nd | 5.91E-5 | nd | nd | nd |
| | Sens 3 | 90% | nd | 100% | 91% | nd | 100% | nd | nd | nd |
| | Spec 3 | 27% | nd | 26% | 53% | nd | 41% | nd | nd | nd |
| | Cutoff 4 | 0.000223 | nd | 0.000227 | 0.000223 | nd | 0.000227 | nd | nd | nd |
| | Sens 4 | 50% | nd | 29% | 64% | nd | 62% | nd | nd | nd |
| | Spec 4 | 70% | nd | 70% | 70% | nd | 70% | nd | nd | nd |
| | Cutoff 5 | 0.000419 | nd | 0.000398 | 0.000419 | nd | 0.000398 | nd | nd | nd |
| | Sens 5 | 10% | nd | 14% | 45% | nd | 38% | nd | nd | nd |
| | Spec 5 | 80% | nd | 80% | 80% | nd | 80% | nd | nd | nd |
| | Cutoff 6 | 0.000828 | nd | 0.000691 | 0.000828 | nd | 0.000691 | nd | nd | nd |
| | Sens 6 | 0% | nd | 0% | 36% | nd | 25% | nd | nd | nd |
| | Spec 6 | 90% | nd | 90% | 90% | nd | 90% | nd | nd | nd |
| | OR Quart 2 | 1.0 | nd | >1.0 | >1.0 | nd | >1.0 | nd | nd | nd |
| | p Value | 1.0 | nd | <1.00 | <1.00 | nd | <1.0 | nd | nd | nd |
| | 95% CI of | 0.062 | nd | >0.062 | >0.062 | nd | >0.062 | nd | nd | nd |
| | OR Quart2 | 16 | nd | na | na | nd | na | nd | nd | nd |
| | OR Quart 3 | 5.2 | nd | >5.2 | >4.1 | nd | >3.1 | nd | nd | nd |
| | p Value | 0.14 | nd | <0.13 | <0.21 | nd | <0.33 | nd | nd | nd |
| | 95% CI of | 0.59 | nd | >0.60 | >0.45 | nd | >0.32 | nd | nd | nd |
| | OR Quart3 | 45 | nd | na | na | nd | na | nd | nd | nd |
| | OR Quart 4 | 3.0 | nd | >1.0 | >6.2 | nd | >4.1 | nd | nd | nd |
| | p Value | 0.34 | nd | <1.0 | <0.092 | nd | <0.21 | nd | nd | nd |
| | 95% CI of | 0.31 | nd | >0.062 | >0.74 | nd | >0.45 | nd | nd | nd |
| | OR Quart4 | 30 | nd | na | na | nd | na | nd | nd | nd |

**Matrilysin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12800 | 29500 | 12800 | 39500 | 12800 | 17400 |
| Average | 20900 | 63500 | 20900 | 84700 | 20900 | 27400 |
| Stdev | 25800 | 109000 | 25800 | 122000 | 25800 | 25000 |
| p(t-test) | | 1.1E-7 | | 9.6E-17 | | 0.51 |
| Min | 1.00E-9 | 2040 | 1.00E-9 | 1840 | 1.00E-9 | 4600 |
| Max | 261000 | 408000 | 261000 | 406000 | 261000 | 72700 |
| n (Samp) | 932 | 13 | 932 | 16 | 932 | 7 |
| n (Patient) | 345 | 13 | 345 | 16 | 345 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13100 | 33800 | nd | nd | nd | nd |
| Average | 22300 | 109000 | nd | nd | nd | nd |
| Stdev | 32000 | 142000 | nd | nd | nd | nd |
| p(t-test) | | 2.7E-11 | nd | nd | nd | nd |
| Min | 1.00E-9 | 2040 | nd | nd | nd | nd |
| Max | 485000 | 408000 | nd | nd | nd | nd |
| n (Samp) | 969 | 7 | nd | nd | nd | nd |
| n (Patient) | 355 | 7 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13600 | 22100 | 13600 | 34100 | nd | nd |
| Average | 22100 | 76700 | 22100 | 86500 | nd | nd |
| Stdev | 26900 | 137000 | 26900 | 131000 | nd | nd |
| p(t-test) | | 2.8E-7 | | 8.3E-14 | nd | nd |
| Min | 1.00E-9 | 5630 | 1.00E-9 | 1840 | nd | nd |
| Max | 261000 | 408000 | 261000 | 406000 | nd | nd |
| n (Samp) | 797 | 8 | 797 | 14 | nd | nd |
| n (Patient) | 262 | 8 | 262 | 14 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.68 | 0.78 | 0.65 | 0.75 | nd | 0.71 | 0.60 | nd | nd |
| SE | 0.083 | 0.10 | 0.11 | 0.071 | nd | 0.079 | 0.11 | nd | nd |
| p | 0.031 | 0.0085 | 0.17 | 4.2E-4 | nd | 0.0074 | 0.37 | nd | nd |
| nCohort 1 | 932 | 969 | 797 | 932 | nd | 797 | 932 | nd | nd |
| nCohort 2 | 13 | 7 | 8 | 16 | nd | 14 | 7 | nd | nd |
| Cutoff 1 | 11900 | 29300 | 11900 | 20800 | nd | 20800 | 8540 | nd | nd |
| Sens 1 | 77% | 71% | 75% | 75% | nd | 71% | 71% | nd | nd |
| Spec 1 | 47% | 77% | 45% | 66% | nd | 65% | 36% | nd | nd |
| Cutoff 2 | 8190 | 28400 | 8190 | 11800 | nd | 9300 | 7560 | nd | nd |
| Sens 2 | 85% | 86% | 88% | 81% | nd | 86% | 86% | nd | nd |
| Spec 2 | 36% | 77% | 34% | 47% | nd | 38% | 33% | nd | nd |
| Cutoff 3 | 5620 | 2010 | 5620 | 6910 | nd | 6910 | 4580 | nd | nd |
| Sens 3 | 92% | 100% | 100% | 94% | nd | 93% | 100% | nd | nd |
| Spec 3 | 27% | 11% | 25% | 31% | nd | 29% | 23% | nd | nd |
| Cutoff 4 | 22800 | 23400 | 23700 | 22800 | nd | 23700 | 22800 | nd | nd |
| Sens 4 | 62% | 86% | 50% | 69% | nd | 64% | 43% | nd | nd |
| Spec 4 | 70% | 70% | 70% | 70% | nd | 70% | 70% | nd | nd |
| Cutoff 5 | 30800 | 33300 | 33500 | 30800 | nd | 33500 | 30800 | nd | nd |
| Sens 5 | 46% | 57% | 38% | 69% | nd | 50% | 43% | nd | nd |
| Spec 5 | 80% | 80% | 80% | 80% | nd | 80% | 80% | nd | nd |
| Cutoff 6 | 50000 | 50800 | 51100 | 50000 | nd | 51100 | 50000 | nd | nd |
| Sens 6 | 23% | 43% | 25% | 38% | nd | 29% | 14% | nd | nd |
| Spec 6 | 90% | 90% | 90% | 90% | nd | 90% | 90% | nd | nd |
| OR Quart 2 | 3.0 | 0 | 2.0 | 3.0 | nd | 3.0 | 2.0 | nd | nd |
| p Value | 0.34 | na | 0.57 | 0.34 | nd | 0.34 | 0.57 | nd | nd |
| 95% CI of | 0.31 | na | 0.18 | 0.31 | nd | 0.31 | 0.18 | nd | nd |
| OR Quart2 | 29 | na | 22 | 29 | nd | 29 | 22 | nd | nd |
| OR Quart 3 | 1.0 | 0 | 1.0 | 1.0 | nd | 1.00 | 1.00 | nd | nd |
| p Value | 1.0 | na | 1.0 | 1.0 | nd | 1.00 | 1.00 | nd | nd |
| 95% CI of | 0.062 | na | 0.062 | 0.062 | nd | 0.062 | 0.062 | nd | nd |
| OR Quart3 | 16 | na | 16 | 16 | nd | 16 | 16 | nd | nd |
| OR Quart 4 | 8.2 | 6.1 | 4.0 | 11 | nd | 9.3 | 3.0 | nd | nd |
| p Value | 0.048 | 0.094 | 0.21 | 0.020 | nd | 0.035 | 0.34 | nd | nd |
| 95% CI of | 1.0 | 0.73 | 0.45 | 1.5 | nd | 1.2 | 0.31 | nd | nd |
| OR Quart4 | 66 | 51 | 36 | 90 | nd | 74 | 29 | nd | nd |

**Transforming growth factor beta-2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.19 | 1.00E-9 | 3.19 | 19.6 | nd | nd |
| Average | 19.6 | 13.4 | 19.6 | 27.5 | nd | nd |
| Stdev | 36.7 | 18.0 | 36.7 | 22.8 | nd | nd |
| p(t-test) |  | 0.66 |  | 0.50 | nd | nd |
| Min | 1.00E-9 | 1.00E-9 | 1.00E-9 | 1.00E-9 | nd | nd |
| Max | 364 | 44.2 | 364 | 69.4 | nd | nd |
| n (Samp) | 332 | 7 | 332 | 10 | nd | nd |
| n (Patient) | 193 | 7 | 193 | 10 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 4.40 | 15.5 | nd | nd |
| Average | nd | nd | 20.2 | 25.8 | nd | nd |
| Stdev | nd | nd | 37.8 | 24.8 | nd | nd |
| p(t-test) | nd | nd |  | 0.68 | nd | nd |
| Min | nd | nd | 1.00E-9 | 1.00E-9 | nd | nd |
| Max | nd | nd | 364 | 69.4 | nd | nd |
| n (Samp) | nd | nd | 290 | 8 | nd | nd |
| n (Patient) | nd | nd | 162 | 8 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.46 | nd | nd | 0.67 | nd | 0.63 | nd | nd | nd |
| SE | 0.11 | nd | nd | 0.095 | nd | 0.11 | nd | nd | nd |
| p | 0.71 | nd | nd | 0.079 | nd | 0.23 | nd | nd | nd |
| nCohort 1 | 332 | nd | nd | 332 | nd | 290 | nd | nd | nd |
| nCohort 2 | 7 | nd | nd | 10 | nd | 8 | nd | nd | nd |
| Cutoff 1 | 0 | nd | nd | 14.5 | nd | 14.4 | nd | nd | nd |
| Sens 1 | 100% | nd | nd | 80% | nd | 75% | nd | nd | nd |
| Spec 1 | 0% | nd | nd | 65% | nd | 64% | nd | nd | nd |
| Cutoff 2 | 0 | nd | nd | 14.5 | nd | 0 | nd | nd | nd |
| Sens 2 | 100% | nd | nd | 80% | nd | 100% | nd | nd | nd |
| Spec 2 | 0% | nd | nd | 65% | nd | 0% | nd | nd | nd |
| Cutoff 3 | 0 | nd | nd | 0 | nd | 0 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 100% | nd | nd | 100% | nd | 100% | nd | nd | nd |
| Spec 3 | 0% | nd | nd | 0% | nd | 0% | nd | nd | nd |
| Cutoff 4 | 20.1 | nd | nd | 20.1 | nd | 20.7 | nd | nd | nd |
| Sens 4 | 43% | nd | nd | 50% | nd | 38% | nd | nd | nd |
| Spec 4 | 70% | nd | nd | 70% | nd | 70% | nd | nd | nd |
| Cutoff 5 | 33.5 | nd | nd | 33.5 | nd | 34.7 | nd | nd | nd |
| Sens 5 | 14% | nd | nd | 40% | nd | 38% | nd | nd | nd |
| Spec 5 | 80% | nd | nd | 80% | nd | 80% | nd | nd | nd |
| Cutoff 6 | 57.9 | nd | nd | 57.9 | nd | 55.8 | nd | nd | nd |
| Sens 6 | 0% | nd | nd | 10% | nd | 12% | nd | nd | nd |
| Spec 6 | 90% | nd | nd | 90% | nd | 90% | nd | nd | nd |
| OR Quart 2 | 0.49 | nd | nd | >2.0 | nd | 0.99 | nd | nd | nd |
| p Value | 0.57 | nd | nd | <0.57 | nd | 0.99 | nd | nd | nd |
| 95% CI of | 0.044 | nd | nd | >0.18 | nd | 0.061 | nd | nd | nd |
| OR Quart2 | 5.6 | nd | nd | na | nd | 16 | nd | nd | nd |
| OR Quart 3 | 0 | nd | nd | >4.2 | nd | 3.1 | nd | nd | nd |
| p Value | na | nd | nd | <0.20 | nd | 0.33 | nd | nd | nd |
| 95% CI of | na | nd | nd | >0.46 | nd | 0.31 | nd | nd | nd |
| OR Quart3 | na | nd | nd | na | nd | 30 | nd | nd | nd |
| OR Quart 4 | 2.1 | nd | nd | >4.1 | nd | 3.0 | nd | nd | nd |
| p Value | 0.41 | nd | nd | <0.21 | nd | 0.34 | nd | nd | nd |
| 95% CI of | 0.37 | nd | nd | >0.45 | nd | 0.31 | nd | nd | nd |
| OR Quart4 | 12 | nd | nd | na | nd | 30 | nd | nd | nd |

**Transforming growth factor beta-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.611 | 1.00E-9 | 0.611 | 0.861 | nd | nd |
| Average | 7.96 | 0.259 | 7.96 | 3.60 | nd | nd |
| Stdev | 25.5 | 0.323 | 25.5 | 4.71 | nd | nd |
| p(t-test) | | 0.43 | | 0.59 | nd | nd |
| Min | 1.00E-9 | 1.00E-9 | 1.00E-9 | 1.00E-9 | nd | nd |
| Max | 241 | 0.610 | 241 | 12.4 | nd | nd |
| n (Samp) | 332 | 7 | 332 | 10 | nd | nd |
| n (Patient) | 193 | 7 | 193 | 10 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.610 | 0.861 | nd | nd |
| Average | nd | nd | 7.34 | 4.09 | nd | nd |
| Stdev | nd | nd | 24.5 | 5.18 | nd | nd |
| p(t-test) | nd | nd | | 0.71 | nd | nd |
| Min | nd | nd | 1.00E-9 | 1.00E-9 | nd | nd |
| Max | nd | nd | 241 | 12.4 | nd | nd |
| n (Samp) | nd | nd | 290 | 8 | nd | nd |
| n (Patient) | nd | nd | 162 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.28 | nd | nd | 0.59 | nd | 0.60 | nd | nd | nd |
| SE | 0.11 | nd | nd | 0.096 | nd | 0.11 | nd | nd | nd |
| p | 0.046 | nd | nd | 0.36 | nd | 0.37 | nd | nd | nd |
| nCohort 1 | 332 | nd | nd | 332 | nd | 290 | nd | nd | nd |
| nCohort 2 | 7 | nd | nd | 10 | nd | 8 | nd | nd | nd |
| Cutoff 1 | 0 | nd | nd | 0.603 | nd | 0.603 | nd | nd | nd |
| Sens 1 | 100% | nd | nd | 90% | nd | 88% | nd | nd | nd |
| Spec 1 | 0% | nd | nd | 44% | nd | 46% | nd | nd | nd |
| Cutoff 2 | 0 | nd | nd | 0.603 | nd | 0.603 | nd | nd | nd |
| Sens 2 | 100% | nd | nd | 90% | nd | 88% | nd | nd | nd |
| Spec 2 | 0% | nd | nd | 44% | nd | 46% | nd | nd | nd |
| Cutoff 3 | 0 | nd | nd | 0.603 | nd | 0 | nd | nd | nd |
| Sens 3 | 100% | nd | nd | 90% | nd | 100% | nd | nd | nd |
| Spec 3 | 0% | nd | nd | 44% | nd | 0% | nd | nd | nd |
| Cutoff 4 | 2.30 | nd | nd | 2.30 | nd | 2.23 | nd | nd | nd |
| Sens 4 | 0% | nd | nd | 40% | nd | 38% | nd | nd | nd |
| Spec 4 | 73% | nd | nd | 73% | nd | 70% | nd | nd | nd |
| Cutoff 5 | 4.28 | nd | nd | 4.28 | nd | 3.49 | nd | nd | nd |
| Sens 5 | 0% | nd | nd | 30% | nd | 38% | nd | nd | nd |
| Spec 5 | 80% | nd | nd | 80% | nd | 80% | nd | nd | nd |
| Cutoff 6 | 17.8 | nd | nd | 17.8 | nd | 14.4 | nd | nd | nd |
| Sens 6 | 0% | nd | nd | 0% | nd | 0% | nd | nd | nd |
| Spec 6 | 90% | nd | nd | 90% | nd | 90% | nd | nd | nd |
| OR Quart 2 | >0 | nd | nd | 4.1 | nd | 3.0 | nd | nd | nd |
| p Value | <na | nd | nd | 0.21 | nd | 0.34 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | >na | nd | nd | 0.45 | nd | 0.31 | nd | nd | nd |
| OR Quart 2 | na | nd | nd | 37 | nd | 30 | nd | nd | nd |
| OR Quart 3 | >5.3 | nd | nd | 2.0 | nd | 1.0 | nd | nd | nd |
| p Value | <0.13 | nd | nd | 0.57 | nd | 1.0 | nd | nd | nd |
| 95% CI of | >0.61 | nd | nd | 0.18 | nd | 0.061 | nd | nd | nd |
| OR Quart3 | na | nd | nd | 23 | nd | 16 | nd | nd | nd |
| OR Quart 4 | >2.1 | nd | nd | 3.0 | nd | 3.0 | nd | nd | nd |
| p Value | <0.55 | nd | nd | 0.34 | nd | 0.34 | nd | nd | nd |
| 95% CI of | >0.18 | nd | nd | 0.31 | nd | 0.31 | nd | nd | nd |
| OR Quart4 | na | nd | nd | 30 | nd | 30 | nd | nd | nd |

**Transmembrane glycoprotein NMB**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.93 | 1.94 | 1.93 | 3.60 | nd | nd |
| Average | 2.16 | 2.28 | 2.16 | 5.72 | nd | nd |
| Stdev | 1.31 | 1.28 | 1.31 | 7.09 | nd | nd |
| p(t-test) | | 0.80 | | 3.9E-10 | nd | nd |
| Min | 0.135 | 0.744 | 0.135 | 1.77 | nd | nd |
| Max | 8.02 | 4.40 | 8.02 | 25.3 | nd | nd |
| n (Samp) | 340 | 7 | 340 | 10 | nd | nd |
| n (Patient) | 195 | 7 | 195 | 10 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.96 | 3.85 | nd | nd |
| Average | nd | nd | 2.20 | 6.56 | nd | nd |
| Stdev | nd | nd | 1.31 | 7.78 | nd | nd |
| p(t-test) | nd | nd | | 2.6E-11 | nd | nd |
| Min | nd | nd | 0.135 | 1.77 | nd | nd |
| Max | nd | nd | 8.02 | 25.3 | nd | nd |
| n (Samp) | nd | nd | 296 | 8 | nd | nd |
| n (Patient) | nd | nd | 163 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | nd | nd | 0.78 | nd | 0.81 | nd | nd | nd |
| SE | 0.11 | nd | nd | 0.087 | nd | 0.093 | nd | nd | nd |
| p | 0.76 | nd | nd | 0.0011 | nd | 7.6E-4 | nd | nd | nd |
| nCohort 1 | 340 | nd | nd | 340 | nd | 296 | nd | nd | nd |
| nCohort 2 | 7 | nd | nd | 10 | nd | 8 | nd | nd | nd |
| Cutoff 1 | 1.65 | nd | nd | 2.51 | nd | 3.44 | nd | nd | nd |
| Sens 1 | 71% | nd | nd | 70% | nd | 75% | nd | nd | nd |
| Spec 1 | 43% | nd | nd | 70% | nd | 84% | nd | nd | nd |
| Cutoff 2 | 1.25 | nd | nd | 2.22 | nd | 2.11 | nd | nd | nd |
| Sens 2 | 86% | nd | nd | 80% | nd | 88% | nd | nd | nd |
| Spec 2 | 26% | nd | nd | 58% | nd | 53% | nd | nd | nd |
| Cutoff 3 | 0.740 | nd | nd | 2.11 | nd | 1.76 | nd | nd | nd |
| Sens 3 | 100% | nd | nd | 90% | nd | 100% | nd | nd | nd |
| Spec 3 | 10% | nd | nd | 56% | nd | 45% | nd | nd | nd |
| Cutoff 4 | 2.53 | nd | nd | 2.53 | nd | 2.60 | nd | nd | nd |
| Sens 4 | 29% | nd | nd | 60% | nd | 75% | nd | nd | nd |
| Spec 4 | 70% | nd | nd | 70% | nd | 70% | nd | nd | nd |
| Cutoff 5 | 3.12 | nd | nd | 3.12 | nd | 3.19 | nd | nd | nd |
| Sens 5 | 29% | nd | nd | 60% | nd | 75% | nd | nd | nd |
| Spec 5 | 80% | nd | nd | 80% | nd | 80% | nd | nd | nd |
| Cutoff 6 | 4.12 | nd | nd | 4.12 | nd | 4.12 | nd | nd | nd |
| Sens 6 | 14% | nd | nd | 30% | nd | 38% | nd | nd | nd |
| Spec 6 | 90% | nd | nd | 90% | nd | 90% | nd | nd | nd |
| OR Quart 2 | 2.0 | nd | nd | >1.0 | nd | >1.0 | nd | nd | nd |
| p Value | 0.57 | nd | nd | <1.0 | nd | <0.99 | nd | nd | nd |
| 95% CI of | 0.18 | nd | nd | >0.062 | nd | >0.062 | nd | nd | nd |
| OR Quart2 | 22 | nd | nd | na | nd | na | nd | nd | nd |
| OR Quart 3 | 2.0 | nd | nd | >3.1 | nd | >1.0 | nd | nd | nd |
| p Value | 0.57 | nd | nd | <0.33 | nd | <0.99 | nd | nd | nd |
| 95% CI of | 0.18 | nd | nd | >0.32 | nd | >0.062 | nd | nd | nd |
| OR Quart3 | 22 | nd | nd | na | nd | na | nd | nd | nd |
| OR Quart 4 | 2.0 | nd | nd | >6.4 | nd | >6.5 | nd | nd | nd |
| p Value | 0.57 | nd | nd | <0.090 | nd | <0.086 | nd | nd | nd |
| 95% CI of | 0.18 | nd | nd | >0.75 | nd | >0.77 | nd | nd | nd |
| OR Quart4 | 22 | nd | nd | na | nd | na | nd | nd | nd |

**Cadherin-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.5 | 2.29 | 14.5 | 9.61 | nd | nd |
| Average | 27.5 | 6.73 | 27.5 | 18.1 | nd | nd |
| Stdev | 30.5 | 7.59 | 30.5 | 25.0 | nd | nd |
| p(t-test) | | 0.073 | | 0.33 | nd | nd |
| Min | 0.690 | 1.03 | 0.690 | 1.72 | nd | nd |
| Max | 212 | 21.7 | 212 | 85.2 | nd | nd |
| n (Samp) | 340 | 7 | 340 | 10 | nd | nd |
| n (Patient) | 195 | 7 | 195 | 10 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 14.5 | 11.4 | nd | nd |
| Average | nd | nd | 28.4 | 20.3 | nd | nd |
| Stdev | nd | nd | 31.8 | 27.9 | nd | nd |
| p(t-test) | nd | nd | | 0.48 | nd | nd |
| Min | nd | nd | 0.690 | 1.72 | nd | nd |
| Max | nd | nd | 212 | 85.2 | nd | nd |
| n (Samp) | nd | nd | 296 | 8 | nd | nd |
| n (Patient) | nd | nd | 163 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.20 | nd | nd | 0.39 | nd | 0.40 | nd | nd | nd |
| SE | 0.10 | nd | nd | 0.096 | nd | 0.11 | nd | nd | nd |
| p | 0.0032 | nd | nd | 0.24 | nd | 0.35 | nd | nd | nd |
| nCohort 1 | 340 | nd | nd | 340 | nd | 296 | nd | nd | nd |
| nCohort 2 | 7 | nd | nd | 10 | nd | 8 | nd | nd | nd |
| Cutoff 1 | 1.96 | nd | nd | 8.79 | nd | 6.00 | nd | nd | nd |
| Sens 1 | 71% | nd | nd | 70% | nd | 75% | nd | nd | nd |
| Spec 1 | 4% | nd | nd | 33% | nd | 23% | nd | nd | nd |
| Cutoff 2 | 1.03 | nd | nd | 6.00 | nd | 1.95 | nd | nd | nd |
| Sens 2 | 86% | nd | nd | 80% | nd | 88% | nd | nd | nd |
| Spec 2 | 1% | nd | nd | 22% | nd | 4% | nd | nd | nd |
| Cutoff 3 | 0.877 | nd | nd | 1.96 | nd | 1.71 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 100% | nd | nd | 90% | nd | 100% | nd | nd | nd |
| Spec 3 | 1% | nd | nd | 4% | nd | 3% | nd | nd | nd |
| Cutoff 4 | 34.1 | nd | nd | 34.1 | nd | 36.2 | nd | nd | nd |
| Sens 4 | 0% | nd | nd | 10% | nd | 12% | nd | nd | nd |
| Spec 4 | 70% | nd | nd | 70% | nd | 70% | nd | nd | nd |
| Cutoff 5 | 47.0 | nd | nd | 47.0 | nd | 48.1 | nd | nd | nd |
| Sens 5 | 0% | nd | nd | 10% | nd | 12% | nd | nd | nd |
| Spec 5 | 80% | nd | nd | 80% | nd | 80% | nd | nd | nd |
| Cutoff 6 | 66.5 | nd | nd | 66.5 | nd | 71.2 | nd | nd | nd |
| Sens 6 | 0% | nd | nd | 10% | nd | 12% | nd | nd | nd |
| Spec 6 | 90% | nd | nd | 90% | nd | 90% | nd | nd | nd |
| OR Quart 2 | >1.0 | nd | nd | 1.0 | nd | 1.0 | nd | nd | nd |
| p Value | <0.99 | nd | nd | 0.99 | nd | 1.0 | nd | nd | nd |
| 95% CI of | >0.062 | nd | nd | 0.062 | nd | 0.061 | nd | nd | nd |
| OR Quart2 | Na | nd | nd | 16 | nd | 16 | nd | nd | nd |
| OR Quart 3 | >2.0 | nd | nd | 5.2 | nd | 3.1 | nd | nd | nd |
| p Value | <0.56 | nd | nd | 0.13 | nd | 0.33 | nd | nd | nd |
| 95% CI of | >0.18 | nd | nd | 0.60 | nd | 0.31 | nd | nd | nd |
| OR Quart3 | Na | nd | nd | 46 | nd | 30 | nd | nd | nd |
| OR Quart 4 | >4.2 | nd | nd | 3.1 | nd | 3.1 | nd | nd | nd |
| p Value | <0.20 | nd | nd | 0.33 | nd | 0.33 | nd | nd | nd |
| 95% CI of | >0.46 | nd | nd | 0.32 | nd | 0.31 | nd | nd | nd |
| OR Quart4 | Na | nd | nd | 30 | nd | 30 | nd | nd | nd |

**Stromelysin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.155 | 1.00E-9 | 0.155 | 1.00E-9 | nd | nd |
| Average | 0.764 | 0.0273 | 0.764 | 0.782 | nd | nd |
| Stdev | 5.17 | 0.0722 | 5.17 | 2.28 | nd | nd |
| p(t-test) | | 0.71 | | 0.99 | nd | nd |
| Min | 1.00E-9 | 1.00E-9 | 1.00E-9 | 1.00E-9 | nd | nd |
| Max | 93.1 | 0.191 | 93.1 | 7.26 | nd | nd |
| n (Samp) | 339 | 7 | 339 | 10 | nd | nd |
| n (Patient) | 194 | 7 | 194 | 10 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.144 | 1.00E-9 | nd | nd |
| Average | nd | nd | 0.829 | 0.938 | nd | nd |
| Stdev | nd | nd | 5.54 | 2.56 | nd | nd |
| p(t-test) | nd | nd | | 0.96 | nd | nd |
| Min | nd | nd | 1.00E-9 | 1.00E-9 | nd | nd |
| Max | nd | nd | 93.1 | 7.26 | nd | nd |
| n (Samp) | nd | nd | 295 | 8 | nd | nd |
| n (Patient) | nd | nd | 162 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.16 | nd | nd | 0.31 | nd | 0.30 | nd | nd | nd |
| SE | 0.095 | nd | nd | 0.094 | nd | 0.11 | nd | nd | nd |
| p | 3.8E-4 | nd | nd | 0.047 | nd | 0.063 | nd | nd | nd |
| nCohort 1 | 339 | nd | nd | 339 | nd | 295 | nd | nd | nd |
| nCohort 2 | 7 | nd | nd | 10 | nd | 8 | nd | nd | nd |
| Cutoff 1 | 0 | nd | nd | 0 | nd | 0 | nd | nd | nd |
| Sens 1 | 100% | nd | nd | 100% | nd | 100% | nd | nd | nd |
| Spec 1 | 0% | nd | nd | 0% | nd | 0% | nd | nd | nd |
| Cutoff 2 | 0 | nd | nd | 0 | nd | 0 | nd | nd | nd |
| Sens 2 | 100% | nd | nd | 100% | nd | 100% | nd | nd | nd |
| Spec 2 | 0% | nd | nd | 0% | nd | 0% | nd | nd | nd |
| Cutoff 3 | 0 | nd | nd | 0 | nd | 0 | nd | nd | nd |
| Sens 3 | 100% | nd | nd | 100% | nd | 100% | nd | nd | nd |
| Spec 3 | 0% | nd | nd | 0% | nd | 0% | nd | nd | nd |
| Cutoff 4 | 0.345 | nd | nd | 0.345 | nd | 0.345 | nd | nd | nd |
| Sens 4 | 0% | nd | nd | 10% | nd | 12% | nd | nd | nd |
| Spec 4 | 70% | nd | nd | 70% | nd | 70% | nd | nd | nd |
| Cutoff 5 | 0.563 | nd | nd | 0.563 | nd | 0.578 | nd | nd | nd |
| Sens 5 | 0% | nd | nd | 10% | nd | 12% | nd | nd | nd |
| Spec 5 | 80% | nd | nd | 80% | nd | 80% | nd | nd | nd |
| Cutoff 6 | 1.09 | nd | nd | 1.09 | nd | 1.11 | nd | nd | nd |
| Sens 6 | 0% | nd | nd | 10% | nd | 12% | nd | nd | nd |
| Spec 6 | 90% | nd | nd | 90% | nd | 90% | nd | nd | nd |
| OR Quart 2 | >1.0 | nd | nd | 2.0 | nd | 1.0 | nd | nd | nd |
| p Value | <0.99 | nd | nd | 0.56 | nd | 1.0 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | >0.063 | nd | nd | 0.18 | nd | 0.061 | nd | nd | nd |
| OR Quart2 | na | nd | nd | 23 | nd | 16 | nd | nd | nd |
| OR Quart 3 | >0 | nd | nd | 1.0 | nd | 1.0 | nd | nd | nd |
| p Value | <na | nd | nd | 0.99 | nd | 1.0 | nd | nd | nd |
| 95% CI of | >na | nd | nd | 0.062 | nd | 0.061 | nd | nd | nd |
| OR Quart3 | na | nd | nd | 16 | nd | 16 | nd | nd | nd |
| OR Quart 4 | >6.5 | nd | nd | 6.4 | nd | 5.4 | nd | nd | nd |
| p Value | <0.086 | nd | nd | 0.088 | nd | 0.13 | nd | nd | nd |
| 95% CI of | >0.77 | nd | nd | 0.76 | nd | 0.61 | nd | nd | nd |
| OR Quart4 | na | nd | nd | 55 | nd | 47 | nd | nd | nd |

**Cathepsin S**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 2.26 | 5.84 | nd | nd |
| Average | nd | nd | 3.00 | 8.97 | nd | nd |
| Stdev | nd | nd | 3.83 | 13.6 | nd | nd |
| p(t-test) | nd | nd |  | 0.0016 | nd | nd |
| Min | nd | nd | 1.00E-9 | 1.12 | nd | nd |
| Max | nd | nd | 37.1 | 39.3 | nd | nd |
| n (Samp) | nd | nd | 122 | 7 | nd | nd |
| n (Patient) | nd | nd | 96 | 7 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.70 | nd | nd | nd | nd | nd |
| SE | nd | nd | nd | 0.11 | nd | nd | nd | nd | nd |
| p | nd | nd | nd | 0.083 | nd | nd | nd | nd | nd |
| nCohort 1 | nd | nd | nd | 122 | nd | nd | nd | nd | nd |
| nCohort 2 | nd | nd | nd | 7 | nd | nd | nd | nd | nd |
| Cutoff 1 | nd | nd | nd | 2.34 | nd | nd | nd | nd | nd |
| Sens 1 | nd | nd | nd | 71% | nd | nd | nd | nd | nd |
| Spec 1 | nd | nd | nd | 55% | nd | nd | nd | nd | nd |
| Cutoff 2 | nd | nd | nd | 1.63 | nd | nd | nd | nd | nd |
| Sens 2 | nd | nd | nd | 86% | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | nd | nd | nd | 40% | nd | nd | nd | nd | nd |
| Cutoff 3 | nd | nd | nd | 1.10 | nd | nd | nd | nd | nd |
| Sens 3 | nd | nd | nd | 100% | nd | nd | nd | nd | nd |
| Spec 3 | nd | nd | nd | 33% | nd | nd | nd | nd | nd |
| Cutoff 4 | nd | nd | nd | 3.58 | nd | nd | nd | nd | nd |
| Sens 4 | nd | nd | nd | 57% | nd | nd | nd | nd | nd |
| Spec 4 | nd | nd | nd | 70% | nd | nd | nd | nd | nd |
| Cutoff 5 | nd | nd | nd | 4.16 | nd | nd | nd | nd | nd |
| Sens 5 | nd | nd | nd | 57% | nd | nd | nd | nd | nd |
| Spec 5 | nd | nd | nd | 80% | nd | nd | nd | nd | nd |
| Cutoff 6 | nd | nd | nd | 6.63 | nd | nd | nd | nd | nd |
| Sens 6 | nd | nd | nd | 14% | nd | nd | nd | nd | nd |
| Spec 6 | nd | nd | nd | 90% | nd | nd | nd | nd | nd |
| OR Quart 2 | nd | nd | nd | >2.1 | nd | nd | nd | nd | nd |
| p Value | nd | nd | nd | <0.54 | nd | nd | nd | nd | nd |
| 95% CI of | nd | nd | nd | >0.18 | nd | nd | nd | nd | nd |
| OR Quart2 | nd | nd | nd | na | nd | nd | nd | nd | nd |
| OR Quart 3 | nd | nd | nd | >1.0 | nd | nd | nd | nd | nd |
| p Value | nd | nd | nd | <0.98 | nd | nd | nd | nd | nd |
| 95% CI of | nd | nd | nd | >0.062 | nd | nd | nd | nd | nd |
| OR Quart3 | nd | nd | nd | na | nd | nd | nd | nd | nd |
| OR Quart 4 | nd | nd | nd | >4.4 | nd | nd | nd | nd | nd |
| p Value | nd | nd | nd | <0.20 | nd | nd | nd | nd | nd |
| 95% CI of | nd | nd | nd | >0.47 | nd | nd | nd | nd | nd |
| OR Quart4 | nd | nd | nd | na | nd | nd | nd | nd | nd |

**Urokinase-type plasminogen activator**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 16.9 | 3.07 | nd | nd |
| Average | nd | nd | 25.7 | 13.9 | nd | nd |
| Stdev | nd | nd | 27.1 | 28.4 | nd | nd |
| p(t-test) | nd | nd | | 0.27 | nd | nd |
| Min | nd | nd | 0.125 | 1.24 | nd | nd |
| Max | nd | nd | 119 | 78.3 | nd | nd |
| n (Samp) | nd | nd | 122 | 7 | nd | nd |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | 96 | 7 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.30 | nd | nd | nd | nd | nd |
| SE | nd | nd | nd | 0.11 | nd | nd | nd | nd | nd |
| p | nd | nd | nd | 0.079 | nd | nd | nd | nd | nd |
| nCohort 1 | nd | nd | nd | 122 | nd | nd | nd | nd | nd |
| nCohort 2 | nd | nd | nd | 7 | nd | nd | nd | nd | nd |
| Cutoff 1 | nd | nd | nd | 1.97 | nd | nd | nd | nd | nd |
| Sens 1 | nd | nd | nd | 71% | nd | nd | nd | nd | nd |
| Spec 1 | nd | nd | nd | 14% | nd | nd | nd | nd | nd |
| Cutoff 2 | nd | nd | nd | 1.87 | nd | nd | nd | nd | nd |
| Sens 2 | nd | nd | nd | 86% | nd | nd | nd | nd | nd |
| Spec 2 | nd | nd | nd | 14% | nd | nd | nd | nd | nd |
| Cutoff 3 | nd | nd | nd | 1.16 | nd | nd | nd | nd | nd |
| Sens 3 | nd | nd | nd | 100% | nd | nd | nd | nd | nd |
| Spec 3 | nd | nd | nd | 7% | nd | nd | nd | nd | nd |
| Cutoff 4 | nd | nd | nd | 31.8 | nd | nd | nd | nd | nd |
| Sens 4 | nd | nd | nd | 14% | nd | nd | nd | nd | nd |
| Spec 4 | nd | nd | nd | 70% | nd | nd | nd | nd | nd |
| Cutoff 5 | nd | nd | nd | 46.4 | nd | nd | nd | nd | nd |
| Sens 5 | nd | nd | nd | 14% | nd | nd | nd | nd | nd |
| Spec 5 | nd | nd | nd | 80% | nd | nd | nd | nd | nd |
| Cutoff 6 | nd | nd | nd | 61.5 | nd | nd | nd | nd | nd |
| Sens 6 | nd | nd | nd | 14% | nd | nd | nd | nd | nd |
| Spec 6 | nd | nd | nd | 90% | nd | nd | nd | nd | nd |
| OR Quart 2 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart2 | nd | nd | nd | na | nd | nd | nd | nd | nd |
| OR Quart 3 | nd | nd | nd | 2.1 | nd | nd | nd | nd | nd |
| p Value | nd | nd | nd | 0.54 | nd | nd | nd | nd | nd |
| 95% CI of | nd | nd | nd | 0.18 | nd | nd | nd | nd | nd |
| OR Quart3 | nd | nd | nd | 25 | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | nd | nd | nd | 4.6 | nd | nd | nd | nd | nd |
| p Value | nd | nd | nd | 0.19 | nd | nd | nd | nd | nd |
| 95% CI of | nd | nd | nd | 0.48 | nd | nd | nd | nd | nd |
| OR Quart4 | nd | nd | nd | 43 | nd | nd | nd | nd | nd |

**C-C motif chemokine 23**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.00309 | 0.0431 | nd | nd |
| Average | nd | nd | 0.120 | 0.292 | nd | nd |
| Stdev | nd | nd | 1.24 | 0.676 | nd | nd |
| p(t-test) | nd | nd |  | 0.72 | nd | nd |
| Min | nd | nd | 1.00E-9 | 0.000202 | nd | nd |
| Max | nd | nd | 13.7 | 1.82 | nd | nd |
| n (Samp) | nd | nd | 122 | 7 | nd | nd |
| n (Patient) | nd | nd | 96 | 7 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.75 | nd | nd | nd | nd | nd |
| SE | nd | nd | nd | 0.11 | nd | nd | nd | nd | nd |
| p | nd | nd | nd | 0.023 | nd | nd | nd | nd | nd |
| nCohort 1 | nd | nd | nd | 122 | nd | nd | nd | nd | nd |
| nCohort 2 | nd | nd | nd | 7 | nd | nd | nd | nd | nd |
| Cutoff 1 | nd | nd | nd | 0.00670 | nd | nd | nd | nd | nd |
| Sens 1 | nd | nd | nd | 71% | nd | nd | nd | nd | nd |
| Spec 1 | nd | nd | nd | 68% | nd | nd | nd | nd | nd |
| Cutoff 2 | nd | nd | nd | 0.00306 | nd | nd | nd | nd | nd |
| Sens 2 | nd | nd | nd | 86% | nd | nd | nd | nd | nd |
| Spec 2 | nd | nd | nd | 50% | nd | nd | nd | nd | nd |
| Cutoff 3 | nd | nd | nd | 0.000195 | nd | nd | nd | nd | nd |
| Sens 3 | nd | nd | nd | 100% | nd | nd | nd | nd | nd |
| Spec 3 | nd | nd | nd | 16% | nd | nd | nd | nd | nd |
| Cutoff 4 | nd | nd | nd | 0.00851 | nd | nd | nd | nd | nd |
| Sens 4 | nd | nd | nd | 57% | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 4 | nd | nd | nd | 70% | nd | nd | nd | nd | nd |
| Cutoff 5 | nd | nd | nd | 0.0142 | nd | nd | nd | nd | nd |
| Sens 5 | nd | nd | nd | 57% | nd | nd | nd | nd | nd |
| Spec 5 | nd | nd | nd | 80% | nd | nd | nd | nd | nd |
| Cutoff 6 | nd | nd | nd | 0.0195 | nd | nd | nd | nd | nd |
| Sens 6 | nd | nd | nd | 57% | nd | nd | nd | nd | nd |
| Spec 6 | nd | nd | nd | 90% | nd | nd | nd | nd | nd |
| OR Quart 2 | nd | nd | nd | 1.0 | nd | nd | nd | nd | nd |
| p Value | nd | nd | nd | 1.0 | nd | nd | nd | nd | nd |
| 95% CI of | nd | nd | nd | 0.060 | nd | nd | nd | nd | nd |
| OR Quart2 | nd | nd | nd | 17 | nd | nd | nd | nd | nd |
| OR Quart 3 | nd | nd | nd | 1.0 | nd | nd | nd | nd | nd |
| p Value | nd | nd | nd | 1.0 | nd | nd | nd | nd | nd |
| 95% CI of | nd | nd | nd | 0.060 | nd | nd | nd | nd | nd |
| OR Quart3 | nd | nd | nd | 17 | nd | nd | nd | nd | nd |
| OR Quart 4 | nd | nd | nd | 4.3 | nd | nd | nd | nd | nd |
| p Value | nd | nd | nd | 0.21 | nd | nd | nd | nd | nd |
| 95% CI of | nd | nd | nd | 0.45 | nd | nd | nd | nd | nd |
| OR Quart4 | nd | nd | nd | 41 | nd | nd | nd | nd | nd |

**Complement C5a**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 1.31 | 1.00E-9 | 3.13 | nd | nd |
| Average | 2.34 | 3.06 | 2.34 | 4.76 | nd | nd |
| Stdev | 6.42 | 4.77 | 6.42 | 5.11 | nd | nd |
| p(t-test) |  | 0.77 |  | 0.29 | nd | nd |
| Min | 1.00E-9 | 1.00E-9 | 1.00E-9 | 1.00E-9 | nd | nd |
| Max | 70.9 | 13.2 | 70.9 | 13.7 | nd | nd |
| n (Samp) | 334 | 7 | 334 | 8 | nd | nd |
| n (Patient) | 195 | 7 | 195 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 1.93 | nd | nd |
| Average | nd | nd | 2.68 | 2.21 | nd | nd |
| Stdev | nd | nd | 6.85 | 2.15 | nd | nd |
| p(t-test) | nd | nd | | 0.87 | nd | nd |
| Min | nd | nd | 1.00E-9 | 1.00E-9 | nd | nd |
| Max | nd | nd | 70.9 | 5.29 | nd | nd |
| n (Samp) | nd | nd | 290 | 6 | nd | nd |
| n (Patient) | nd | nd | 163 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | nd | 0.72 | nd | 0.61 | nd | nd | nd |
| SE | 0.11 | nd | nd | 0.10 | nd | 0.12 | nd | nd | nd |
| p | 0.40 | nd | nd | 0.038 | nd | 0.36 | nd | nd | nd |
| nCohort 1 | 334 | nd | nd | 334 | nd | 290 | nd | nd | nd |
| nCohort 2 | 7 | nd | nd | 8 | nd | 6 | nd | nd | nd |
| Cutoff 1 | 0 | nd | nd | 1.67 | nd | 0 | nd | nd | nd |
| Sens 1 | 100% | nd | nd | 75% | nd | 100% | nd | nd | nd |
| Spec 1 | 0% | nd | nd | 74% | nd | 0% | nd | nd | nd |
| Cutoff 2 | 0 | nd | nd | 0 | nd | 0 | nd | nd | nd |
| Sens 2 | 100% | nd | nd | 100% | nd | 100% | nd | nd | nd |
| Spec 2 | 0% | nd | nd | 0% | nd | 0% | nd | nd | nd |
| Cutoff 3 | 0 | nd | nd | 0 | nd | 0 | nd | nd | nd |
| Sens 3 | 100% | nd | nd | 100% | nd | 100% | nd | nd | nd |
| Spec 3 | 0% | nd | nd | 0% | nd | 0% | nd | nd | nd |
| Cutoff 4 | 1.24 | nd | nd | 1.24 | nd | 1.54 | nd | nd | nd |
| Sens 4 | 57% | nd | nd | 75% | nd | 67% | nd | nd | nd |
| Spec 4 | 70% | nd | nd | 70% | nd | 70% | nd | nd | nd |
| Cutoff 5 | 3.14 | nd | nd | 3.14 | nd | 3.45 | nd | nd | nd |
| Sens 5 | 29% | nd | nd | 50% | nd | 33% | nd | nd | nd |
| Spec 5 | 80% | nd | nd | 80% | nd | 80% | nd | nd | nd |
| Cutoff 6 | 6.21 | nd | nd | 6.21 | nd | 7.24 | nd | nd | nd |
| Sens 6 | 14% | nd | nd | 25% | nd | 0% | nd | nd | nd |
| Spec 6 | 90% | nd | nd | 90% | nd | 90% | nd | nd | nd |
| OR Quart 2 | 0.49 | nd | nd | 0 | nd | >1.0 | nd | nd | nd |
| p Value | 0.57 | nd | nd | na | nd | <0.99 | nd | nd | nd |

EP 2 496 258 B1

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.044 | nd | nd | na | nd | >0.062 | nd | nd | nd |
| OR Quart2 | 5.6 | nd | nd | na | nd | na | nd | nd | nd |
| OR Quart 3 | 0.49 | nd | nd | 0.49 | nd | >3.1 | nd | nd | nd |
| p Value | 0.57 | nd | nd | 0.57 | nd | <0.33 | nd | nd | nd |
| 95% CI of | 0.044 | nd | nd | 0.044 | nd | >0.32 | nd | nd | nd |
| OR Quart3 | 5.6 | nd | nd | 5.6 | nd | na | nd | nd | nd |
| OR Quart 4 | 1.5 | nd | nd | 2.6 | nd | >2.1 | nd | nd | nd |
| p Value | 0.66 | nd | nd | 0.27 | nd | <0.56 | nd | nd | nd |
| 95% CI of | 0.24 | nd | nd | 0.48 | nd | >0.18 | nd | nd | nd |
| OR Quart4 | 9.2 | nd | nd | 14 | nd | na | nd | nd | nd |

**Carcinoembryonic antigen-related cell adhesion molecule 1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6.66 | 5.40 | 6.66 | 9.54 | nd | nd |
| Average | 9.37 | 9.30 | 9.37 | 28.5 | nd | nd |
| Stdev | 9.83 | 8.23 | 9.83 | 46.8 | nd | nd |
| p(t-test) |  | 0.98 |  | 1.8E-6 | nd | nd |
| Min | 0.426 | 1.42 | 0.426 | 1.75 | nd | nd |
| Max | 86.1 | 23.5 | 86.1 | 155 | nd | nd |
| n (Samp) | 340 | 7 | 340 | 10 | nd | nd |
| n (Patient) | 195 | 7 | 195 | 10 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 6.82 | 12.5 | nd | nd |
| Average | nd | nd | 9.27 | 34.8 | nd | nd |
| Stdev | nd | nd | 9.37 | 50.9 | nd | nd |
| p(t-test) | nd | nd |  | 9.6E-9 | nd | nd |
| Min | nd | nd | 0.426 | 4.52 | nd | nd |
| Max | nd | nd | 86.1 | 155 | nd | nd |
| n (Samp) | nd | nd | 296 | 8 | nd | nd |
| n (Patient) | nd | nd | 163 | 8 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.50 | nd | nd | 0.63 | nd | 0.73 | nd | nd | nd |
| SE | 0.11 | nd | nd | 0.096 | nd | 0.10 | nd | nd | nd |
| p | 0.97 | nd | nd | 0.18 | nd | 0.026 | nd | nd | nd |
| nCohort 1 | 340 | nd | nd | 340 | nd | 296 | nd | nd | nd |
| nCohort 2 | 7 | nd | nd | 10 | nd | 8 | nd | nd | nd |
| Cutoff 1 | 5.13 | nd | nd | 5.67 | nd | 7.66 | nd | nd | nd |
| Sens 1 | 71% | nd | nd | 70% | nd | 75% | nd | nd | nd |
| Spec 1 | 38% | nd | nd | 43% | nd | 57% | nd | nd | nd |
| Cutoff 2 | 4.14 | nd | nd | 4.82 | nd | 5.67 | nd | nd | nd |
| Sens 2 | 86% | nd | nd | 80% | nd | 88% | nd | nd | nd |
| Spec 2 | 28% | nd | nd | 35% | nd | 41% | nd | nd | nd |
| Cutoff 3 | 1.38 | nd | nd | 4.51 | nd | 4.51 | nd | nd | nd |
| Sens 3 | 100% | nd | nd | 90% | nd | 100% | nd | nd | nd |
| Spec 3 | 5% | nd | nd | 32% | nd | 31% | nd | nd | nd |
| Cutoff 4 | 10.1 | nd | nd | 10.1 | nd | 10.2 | nd | nd | nd |
| Sens 4 | 29% | nd | nd | 50% | nd | 62% | nd | nd | nd |
| Spec 4 | 70% | nd | nd | 70% | nd | 70% | nd | nd | nd |
| Cutoff 5 | 12.6 | nd | nd | 12.6 | nd | 12.4 | nd | nd | nd |
| Sens 5 | 29% | nd | nd | 40% | nd | 50% | nd | nd | nd |
| Spec 5 | 80% | nd | nd | 80% | nd | 80% | nd | nd | nd |
| Cutoff 6 | 18.8 | nd | nd | 18.8 | nd | 18.8 | nd | nd | nd |
| Sens 6 | 14% | nd | nd | 30% | nd | 38% | nd | nd | nd |
| Spec 6 | 90% | nd | nd | 90% | nd | 90% | nd | nd | nd |
| OR Quart 2 | 0.49 | nd | nd | 3.0 | nd | >2.1 | nd | nd | nd |
| p Value | 0.57 | nd | nd | 0.34 | nd | <0.56 | nd | nd | nd |
| 95% CI of | 0.044 | nd | nd | 0.31 | nd | >0.18 | nd | nd | nd |
| OR Quart2 | 5.6 | nd | nd | 30 | nd | na | nd | nd | nd |
| OR Quart 3 | 1.5 | nd | nd | 2.0 | nd | >2.1 | nd | nd | nd |
| p Value | 0.65 | nd | nd | 0.57 | nd | <0.56 | nd | nd | nd |
| 95% CI of | 0.25 | nd | nd | 0.18 | nd | >0.18 | nd | nd | nd |
| OR Quart3 | 9.3 | nd | nd | 23 | nd | na | nd | nd | nd |
| OR Quart 4 | 0.50 | nd | nd | 4.1 | nd | >4.2 | nd | nd | nd |
| p Value | 0.57 | nd | nd | 0.21 | nd | <0.20 | nd | nd | nd |
| 95% CI of | 0.044 | nd | nd | 0.45 | nd | >0.46 | nd | nd | nd |
| OR Quart4 | 5.6 | nd | nd | 37 | nd | na | nd | nd | nd |

**Platelet basic protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 0.0360 | nd | nd |
| Average | 0.346 | 1.00E-9 | 0.346 | 4.97 | nd | nd |
| Stdev | 3.15 | 0 | 3.15 | 15.5 | nd | nd |
| p(t-test) | | 0.77 | | 3.4E-4 | nd | nd |
| Min | 1.00E-9 | 1.00E-9 | 1.00E-9 | 1.00E-9 | nd | nd |
| Max | 49.9 | 1.00E-9 | 49.9 | 49.1 | nd | nd |
| n (Samp) | 340 | 7 | 340 | 10 | nd | nd |
| n (Patient) | 195 | 7 | 195 | 10 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 1.00E-9 | nd | nd |
| Average | nd | nd | 0.379 | 6.20 | nd | nd |
| Stdev | nd | nd | 3.36 | 17.4 | nd | nd |
| p(t-test) | nd | nd | | 1.6E-4 | nd | nd |
| Min | nd | nd | 1.00E-9 | 1.00E-9 | nd | nd |
| Max | nd | nd | 49.9 | 49.1 | nd | nd |
| n (Samp) | nd | nd | 296 | 8 | nd | nd |
| n (Patient) | nd | nd | 163 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | nd | nd | 0.67 | nd | 0.61 | nd | nd | nd |
| SE | 0.11 | nd | nd | 0.095 | nd | 0.11 | nd | nd | nd |
| p | 0.52 | nd | nd | 0.073 | nd | 0.29 | nd | nd | nd |
| nCohort 1 | 340 | nd | nd | 340 | nd | 296 | nd | nd | nd |
| nCohort 2 | 7 | nd | nd | 10 | nd | 8 | nd | nd | nd |
| Cutoff 1 | 0 | nd | nd | 0 | nd | 0 | nd | nd | nd |
| Sens 1 | 100% | nd | nd | 100% | nd | 100% | nd | nd | nd |
| Spec 1 | 0% | nd | nd | 0% | nd | 0% | nd | nd | nd |
| Cutoff 2 | 0 | nd | nd | 0 | nd | 0 | nd | nd | nd |
| Sens 2 | 100% | nd | nd | 100% | nd | 100% | nd | nd | nd |
| Spec 2 | 0% | nd | nd | 0% | nd | 0% | nd | nd | nd |
| Cutoff 3 | 0 | nd | nd | 0 | nd | 0 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | 100% | nd | nd | 100% | nd | 100% | nd | nd | nd |
| Spec 3 | 0% | nd | nd | 0% | nd | 0% | nd | nd | nd |
| Cutoff 4 | 1.00E-9 | nd | nd | 1.00E-9 | nd | 1.00E-9 | nd | nd | nd |
| Sens 4 | 0% | nd | nd | 50% | nd | 38% | nd | nd | nd |
| Spec 4 | 85% | nd | nd | 85% | nd | 84% | nd | nd | nd |
| Cutoff 5 | 1.00E-9 | nd | nd | 1.00E-9 | nd | 1.00E-9 | nd | nd | nd |
| Sens 5 | 0% | nd | nd | 50% | nd | 38% | nd | nd | nd |
| Spec 5 | 85% | nd | nd | 85% | nd | 84% | nd | nd | nd |
| Cutoff 6 | 0.172 | nd | nd | 0.172 | nd | 0.172 | nd | nd | nd |
| Sens 6 | 0% | nd | nd | 20% | nd | 25% | nd | nd | nd |
| Spec 6 | 90% | nd | nd | 90% | nd | 90% | nd | nd | nd |
| OR Quart 2 | >0 | nd | nd | >5.2 | nd | >5.4 | nd | nd | nd |
| p Value | <na | nd | nd | <0.13 | nd | <0.13 | nd | nd | nd |
| 95% CI of | >na | nd | nd | >0.60 | nd | >0.61 | nd | nd | nd |
| OR Quart2 | na | nd | nd | na | nd | na | nd | nd | nd |
| OR Quart 3 | >7.6 | nd | nd | >0 | nd | >0 | nd | nd | nd |
| p Value | <0.060 | nd | nd | <na | nd | <na | nd | nd | nd |
| 95% CI of | >0.92 | nd | nd | >na | nd | >na | nd | nd | nd |
| OR Quart3 | na | nd | nd | na | nd | na | nd | nd | nd |
| OR Quart 4 | >0 | nd | nd | >5.2 | nd | >3.1 | nd | nd | nd |
| p Value | <na | nd | nd | <0.13 | nd | <0.33 | nd | nd | nd |
| 95% CI of | >na | nd | nd | >0.60 | nd | >0.32 | nd | nd | nd |
| OR Quart4 | na | nd | nd | na | nd | na | nd | nd | nd |

Table 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

**Angiopoietin-2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1600 | 2770 | nd | nd |
| Average | nd | nd | 2000 | 8850 | nd | nd |
| Stdev | nd | nd | 1490 | 15200 | nd | nd |
| p(t-test) | nd | nd |  | 1.7E-9 | nd | nd |
| Min | nd | nd | 1.00E-9 | 1450 | nd | nd |
| Max | nd | nd | 10500 | 39900 | nd | nd |
| n (Samp) | nd | nd | 236 | 6 | nd | nd |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | 153 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.75 | nd | nd | nd | nd | nd |
| SE | nd | nd | nd | 0.12 | nd | nd | nd | nd | nd |
| p | nd | nd | nd | 0.029 | nd | nd | nd | nd | nd |
| nCohort 1 | nd | nd | nd | 236 | nd | nd | nd | nd | nd |
| nCohort 2 | nd | nd | nd | 6 | nd | nd | nd | nd | nd |
| Cutoff 1 | nd | nd | nd | 1530 | nd | nd | nd | nd | nd |
| Sens 1 | nd | nd | nd | 83% | nd | nd | nd | nd | nd |
| Spec 1 | nd | nd | nd | 49% | nd | nd | nd | nd | nd |
| Cutoff 2 | nd | nd | nd | 1530 | nd | nd | nd | nd | nd |
| Sens 2 | nd | nd | nd | 83% | nd | nd | nd | nd | nd |
| Spec 2 | nd | nd | nd | 49% | nd | nd | nd | nd | nd |
| Cutoff 3 | nd | nd | nd | 1450 | nd | nd | nd | nd | nd |
| Sens 3 | nd | nd | nd | 100% | nd | nd | nd | nd | nd |
| Spec 3 | nd | nd | nd | 43% | nd | nd | nd | nd | nd |
| Cutoff 4 | nd | nd | nd | 2070 | nd | nd | nd | nd | nd |
| Sens 4 | nd | nd | nd | 67% | nd | nd | nd | nd | nd |
| Spec 4 | nd | nd | nd | 70% | nd | nd | nd | nd | nd |
| Cutoff 5 | nd | nd | nd | 2460 | nd | nd | nd | nd | nd |
| Sens 5 | nd | nd | nd | 67% | nd | nd | nd | nd | nd |
| Spec 5 | nd | nd | nd | 80% | nd | nd | nd | nd | nd |
| Cutoff 6 | nd | nd | nd | 3480 | nd | nd | nd | nd | nd |
| Sens 6 | nd | nd | nd | 33% | nd | nd | nd | nd | nd |
| Spec 6 | nd | nd | nd | 90% | nd | nd | nd | nd | nd |
| OR Quart 2 | nd | nd | nd | >2.0 | nd | nd | nd | nd | nd |
| p Value | nd | nd | nd | <0.57 | nd | nd | nd | nd | nd |
| 95% CI of | nd | nd | nd | >0.18 | nd | nd | nd | nd | nd |
| OR Quart2 | nd | nd | nd | na | nd | nd | nd | nd | nd |
| OR Quart 3 | nd | nd | nd | >0 | nd | nd | nd | nd | nd |
| p Value | nd | nd | nd | <na | nd | nd | nd | nd | nd |
| 95% CI of | nd | nd | nd | >na | nd | nd | nd | nd | nd |
| OR Quart3 | nd | nd | nd | na | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | nd | nd | nd | >4.2 | nd | nd | nd | nd | nd |
| p Value | nd | nd | nd | <0.20 | nd | nd | nd | nd | nd |
| 95% CI of | nd | nd | nd | >0.46 | nd | nd | nd | nd | nd |
| OR Quart4 | nd | nd | nd | na | nd | nd | nd | nd | nd |

**Brain-derived neurotrophic factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1820 | 1210 | nd | nd |
| Average | nd | nd | 2740 | 1650 | nd | nd |
| Stdev | nd | nd | 3300 | 1300 | nd | nd |
| p(t-test) | nd | nd |  | 0.42 | nd | nd |
| Min | nd | nd | 1.00E-9 | 468 | nd | nd |
| Max | nd | nd | 33100 | 4170 | nd | nd |
| n (Samp) | nd | nd | 247 | 6 | nd | nd |
| n (Patient) | nd | nd | 158 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.42 | nd | nd | nd | nd | nd |
| SE | nd | nd | nd | 0.12 | nd | nd | nd | nd | nd |
| p | nd | nd | nd | 0.52 | nd | nd | nd | nd | nd |
| nCohort 1 | nd | nd | nd | 247 | nd | nd | nd | nd | nd |
| nCohort 2 | nd | nd | nd | 6 | nd | nd | nd | nd | nd |
| Cutoff 1 | nd | nd | nd | 1050 | nd | nd | nd | nd | nd |
| Sens 1 | nd | nd | nd | 83% | nd | nd | nd | nd | nd |
| Spec 1 | nd | nd | nd | 34% | nd | nd | nd | nd | nd |
| Cutoff 2 | nd | nd | nd | 1050 | nd | nd | nd | nd | nd |
| Sens 2 | nd | nd | nd | 83% | nd | nd | nd | nd | nd |
| Spec 2 | nd | nd | nd | 34% | nd | nd | nd | nd | nd |
| Cutoff 3 | nd | nd | nd | 449 | nd | nd | nd | nd | nd |
| Sens 3 | nd | nd | nd | 100% | nd | nd | nd | nd | nd |
| Spec 3 | nd | nd | nd | 15% | nd | nd | nd | nd | nd |
| Cutoff 4 | nd | nd | nd | 3090 | nd | nd | nd | nd | nd |
| Sens 4 | nd | nd | nd | 17% | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 4 | nd | nd | nd | 70% | nd | nd | nd | nd | nd |
| Cutoff 5 | nd | nd | nd | 4150 | nd | nd | nd | nd | nd |
| Sens 5 | nd | nd | nd | 17% | nd | nd | nd | nd | nd |
| Spec 5 | nd | nd | nd | 80% | nd | nd | nd | nd | nd |
| Cutoff 6 | nd | nd | nd | 5890 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | 1.0 | nd | nd | nd | nd | nd |
| p Value | nd | nd | nd | 0.99 | nd | nd | nd | nd | nd |
| 95% CI of | nd | nd | nd | 0.062 | nd | nd | nd | nd | nd |
| OR Quart2 | nd | nd | nd | 17 | nd | nd | nd | nd | nd |
| OR Quart 3 | nd | nd | nd | 3.1 | nd | nd | nd | nd | nd |
| p Value | nd | nd | nd | 0.33 | nd | nd | nd | nd | nd |
| 95% CI of | nd | nd | nd | 0.32 | nd | nd | nd | nd | nd |
| OR Quart3 | nd | nd | nd | 31 | nd | nd | nd | nd | nd |
| OR Quart 4 | nd | nd | nd | 1.0 | nd | nd | nd | nd | nd |
| p Value | nd | nd | nd | 0.99 | nd | nd | nd | nd | nd |
| 95% CI of | nd | nd | nd | 0.062 | nd | nd | nd | nd | nd |
| OR Quart4 | nd | nd | nd | 17 | nd | nd | nd | nd | nd |

**Immunoglobulin E**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 42.1 | 25.6 | 42.1 | 30.8 | nd | nd |
| Average | 129 | 30.9 | 129 | 100 | nd | nd |
| Stdev | 271 | 24.0 | 271 | 150 | nd | nd |
| p(t-test) |  | 0.21 |  | 0.73 | nd | nd |
| Min | 0.140 | 2.16 | 0.140 | 1.37 | nd | nd |
| Max | 1940 | 76.8 | 1940 | 480 | nd | nd |
| n (Samp) | 551 | 12 | 551 | 11 | nd | nd |
| n (Patient) | 213 | 12 | 213 | 11 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 46.2 | 22.3 | 46.2 | 30.8 | nd | nd |
| Average | 125 | 26.9 | 125 | 94.1 | nd | nd |
| Stdev | 250 | 22.4 | 250 | 157 | nd | nd |
| p(t-test) | | 0.27 | | 0.71 | nd | nd |
| Min | 0.140 | 2.16 | 0.140 | 1.37 | nd | nd |
| Max | 1940 | 67.2 | 1940 | 480 | nd | nd |
| n (Samp) | 464 | 8 | 464 | 9 | nd | nd |
| n (Patient) | 173 | 8 | 173 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.36 | nd | 0.32 | 0.45 | nd | 0.41 | nd | nd | nd |
| SE | 0.087 | nd | 0.11 | 0.090 | nd | 0.10 | nd | nd | nd |
| p | 0.11 | nd | 0.081 | 0.55 | nd | 0.39 | nd | nd | nd |
| nCohort 1 | 551 | nd | 464 | 551 | nd | 464 | nd | nd | nd |
| nCohort 2 | 12 | nd | 8 | 11 | nd | 9 | nd | nd | nd |
| Cutoff 1 | 17.2 | nd | 17.2 | 13.6 | nd | 2.39 | nd | nd | nd |
| Sens 1 | 75% | nd | 75% | 73% | nd | 78% | nd | nd | nd |
| Spec 1 | 28% | nd | 26% | 22% | nd | 3% | nd | nd | nd |
| Cutoff 2 | 6.48 | nd | 2.84 | 2.39 | nd | 1.71 | nd | nd | nd |
| Sens 2 | 83% | nd | 88% | 82% | nd | 89% | nd | nd | nd |
| Spec 2 | 9% | nd | 4% | 4% | nd | 2% | nd | nd | nd |
| Cutoff 3 | 2.84 | nd | 2.12 | 1.71 | nd | 1.22 | nd | nd | nd |
| Sens 3 | 92% | nd | 100% | 91% | nd | 100% | nd | nd | nd |
| Spec 3 | 5% | nd | 3% | 3% | nd | 2% | nd | nd | nd |
| Cutoff 4 | 90.3 | nd | 91.9 | 90.3 | nd | 91.9 | nd | nd | nd |
| Sens 4 | 0% | nd | 0% | 27% | nd | 22% | nd | nd | nd |
| Spec 4 | 70% | nd | 70% | 70% | nd | 70% | nd | nd | nd |
| Cutoff 5 | 131 | nd | 135 | 131 | nd | 135 | nd | nd | nd |
| Sens 5 | 0% | nd | 0% | 27% | nd | 22% | nd | nd | nd |
| Spec 5 | 80% | nd | 80% | 80% | nd | 80% | nd | nd | nd |
| Cutoff 6 | 314 | nd | 327 | 314 | nd | 327 | nd | nd | nd |
| Sens 6 | 0% | nd | 0% | 9% | nd | 11% | nd | nd | nd |
| Spec 6 | 90% | nd | 90% | 90% | nd | 90% | nd | nd | nd |
| OR Quart 2 | >4.1 | nd | >2.0 | 0.67 | nd | 1.0 | nd | nd | nd |
| p Value | <0.21 | nd | <0.56 | 0.66 | nd | 0.99 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | >0.45 | nd | >0.18 | 0.11 | nd | 0.14 | nd | nd | nd |
| OR Quart2 | na | nd | na | 4.1 | nd | 7.3 | nd | nd | nd |
| OR Quart 3 | >5.2 | nd | >4.1 | 0.66 | nd | 1.0 | nd | nd | nd |
| p Value | <0.14 | nd | <0.21 | 0.65 | nd | 0.99 | nd | nd | nd |
| 95% CI of | >0.60 | nd | >0.46 | 0.11 | nd | 0.14 | nd | nd | nd |
| OR Quart3 | na | nd | na | 4.0 | nd | 7.3 | nd | nd | nd |
| OR Quart 4 | >3.1 | nd | >2.0 | 1.4 | nd | 1.5 | nd | nd | nd |
| p Value | <0.33 | nd | <0.56 | 0.70 | nd | 0.65 | nd | nd | nd |
| 95% CI of | >0.32 | nd | >0.18 | 0.30 | nd | 0.25 | nd | nd | nd |
| OR Quart4 | na | nd | na | 6.2 | nd | 9.3 | nd | nd | nd |

**Immunoglobulin M**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.534 | 0.731 | 0.534 | 0.896 | nd | nd |
| Average | 0.759 | 0.882 | 0.759 | 1.06 | nd | nd |
| Stdev | 0.688 | 0.705 | 0.688 | 0.897 | nd | nd |
| p(t-test) | | 0.54 | | 0.16 | nd | nd |
| Min | 0.0493 | 0.0919 | 0.0493 | 0.293 | nd | nd |
| Max | 4.82 | 2.85 | 4.82 | 3.51 | nd | nd |
| n (Samp) | 551 | 12 | 551 | 11 | nd | nd |
| n (Patient) | 213 | 12 | 213 | 11 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.517 | 0.815 | 0.517 | 0.817 | nd | nd |
| Average | 0.734 | 0.976 | 0.734 | 1.00 | nd | nd |
| Stdev | 0.670 | 0.853 | 0.670 | 0.986 | nd | nd |
| p(t-test) | | 0.31 | | 0.24 | nd | nd |
| Min | 0.0815 | 0.0919 | 0.0815 | 0.293 | nd | nd |
| Max | 4.82 | 2.85 | 4.82 | 3.51 | nd | nd |
| n (Samp) | 464 | 8 | 464 | 9 | nd | nd |
| n (Patient) | 173 | 8 | 173 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.61 | 0.66 | nd | 0.62 | nd | nd | nd |
| SE | 0.087 | nd | 0.11 | 0.091 | nd | 0.10 | nd | nd | nd |
| p | 0.25 | nd | 0.29 | 0.088 | nd | 0.24 | nd | nd | nd |
| nCohort 1 | 551 | nd | 464 | 551 | nd | 464 | nd | nd | nd |
| nCohort 2 | 12 | nd | 8 | 11 | nd | 9 | nd | nd | nd |
| Cutoff 1 | 0.497 | nd | 0.497 | 0.799 | nd | 0.346 | nd | nd | nd |
| Sens 1 | 75% | nd | 75% | 73% | nd | 78% | nd | nd | nd |
| Spec 1 | 45% | nd | 48% | 70% | nd | 29% | nd | nd | nd |
| Cutoff 2 | 0.488 | nd | 0.370 | 0.428 | nd | 0.313 | nd | nd | nd |
| Sens 2 | 83% | nd | 88% | 82% | nd | 89% | nd | nd | nd |
| Spec 2 | 44% | nd | 32% | 38% | nd | 25% | nd | nd | nd |
| Cutoff 3 | 0.346 | nd | 0.0884 | 0.313 | nd | 0.290 | nd | nd | nd |
| Sens 3 | 92% | nd | 100% | 91% | nd | 100% | nd | nd | nd |
| Spec 3 | 28% | nd | 0% | 25% | nd | 23% | nd | nd | nd |
| Cutoff 4 | 0.794 | nd | 0.746 | 0.794 | nd | 0.746 | nd | nd | nd |
| Sens 4 | 42% | nd | 62% | 73% | nd | 67% | nd | nd | nd |
| Spec 4 | 70% | nd | 70% | 70% | nd | 70% | nd | nd | nd |
| Cutoff 5 | 1.08 | nd | 1.03 | 1.08 | nd | 1.03 | nd | nd | nd |
| Sens 5 | 17% | nd | 25% | 18% | nd | 22% | nd | nd | nd |
| Spec 5 | 80% | nd | 80% | 80% | nd | 80% | nd | nd | nd |
| Cutoff 6 | 1.70 | nd | 1.62 | 1.70 | nd | 1.62 | nd | nd | nd |
| Sens 6 | 8% | nd | 12% | 9% | nd | 11% | nd | nd | nd |
| Spec 6 | 90% | nd | 90% | 90% | nd | 90% | nd | nd | nd |
| OR Quart 2 | 3.0 | nd | 2.0 | 0.49 | nd | 2.0 | nd | nd | nd |
| p Value | 0.34 | nd | 0.57 | 0.57 | nd | 0.57 | nd | nd | nd |
| 95% CI of | 0.31 | nd | 0.18 | 0.044 | nd | 0.18 | nd | nd | nd |
| OR Quart2 | 29 | nd | 23 | 5.5 | nd | 23 | nd | nd | nd |
| OR Quart 3 | 4.1 | nd | 2.0 | 2.0 | nd | 2.0 | nd | nd | nd |
| p Value | 0.21 | nd | 0.57 | 0.42 | nd | 0.57 | nd | nd | nd |
| 95% CI of | 0.45 | nd | 0.18 | 0.37 | nd | 0.18 | nd | nd | nd |
| OR Quart3 | 37 | nd | 23 | 11 | nd | 23 | nd | nd | nd |
| OR Quart 4 | 4.1 | nd | 3.1 | 2.0 | nd | 4.1 | nd | nd | nd |
| p Value | 0.21 | nd | 0.34 | 0.42 | nd | 0.21 | nd | nd | nd |
| 95% CI of | 0.45 | nd | 0.31 | 0.36 | nd | 0.45 | nd | nd | nd |
| OR Quart4 | 37 | nd | 30 | 11 | nd | 37 | nd | nd | nd |

Table 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.
**Angiopoietin-2**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 6.38 | 5.68 | 6.25 | 11.5 | 5.54 | 5.68 |
| Average | 15.1 | 180 | 42.8 | 71.1 | 15.8 | 199 |
| Stdev | 47.5 | 1200 | 509 | 142 | 54.2 | 1260 |
| p(t-test) | | 0.019 | | 0.85 | | 0.035 |
| Min | 1.00E-9 | 0.461 | 1.00E-9 | 0.461 | 1.00E-9 | 0.505 |
| Max | 643 | 9360 | 9360 | 504 | 643 | 9360 |
| n (Samp) | 293 | 61 | 339 | 12 | 212 | 55 |
| n (Patient) | 293 | 61 | 339 | 12 | 212 | 55 |

| | At Enrollment | | |
|---|---|---|---|
| | sCr or UO | sCr only | UO only |
| AUC | 0.49 | 0.62 | 0.51 |
| SE | 0.041 | 0.088 | 0.044 |
| p | 0.77 | 0.18 | 0.82 |
| nCohort 1 | 293 | 339 | 212 |
| nCohort 2 | 61 | 12 | 55 |
| Cutoff 1 | 2.71 | 3.87 | 2.71 |
| Sens 1 | 70% | 75% | 71% |
| Spec 1 | 26% | 37% | 28% |
| Cutoff 2 | 2.02 | 2.09 | 2.00 |
| Sens 2 | 80% | 83% | 80% |
| Spec 2 | 19% | 21% | 19% |
| Cutoff 3 | 1.00 | 0.666 | 1.00 |
| Sens 3 | 90% | 92% | 91% |
| Spec 3 | 7% | 4% | 9% |
| Cutoff 4 | 11.4 | 10.9 | 10.6 |
| Sens 4 | 30% | 50% | 33% |
| Spec 4 | 70% | 70% | 70% |
| Cutoff 5 | 16.7 | 16.4 | 16.2 |
| Sens 5 | 21% | 42% | 25% |
| Spec 5 | 80% | 80% | 80% |
| Cutoff 6 | 27.9 | 29.6 | 27.9 |
| Sens 6 | 16% | 42% | 18% |
| Spec 6 | 90% | 90% | 90% |
| OR Quart 2 | 0.73 | 0.32 | 1.1 |

(continued)

|  | At Enrollment | | |
|---|---|---|---|
|  | sCr or UO | sCr only | UO only |
| p Value | 0.44 | 0.33 | 0.86 |
| 95% CI of | 0.33 | 0.033 | 0.46 |
| OR Quart2 | 1.6 | 3.2 | 2.5 |
| OR Quart 3 | 0.93 | 0.65 | 0.89 |
| p Value | 0.85 | 0.64 | 0.79 |
| 95% CI of | 0.44 | 0.11 | 0.37 |
| OR Quart3 | 2.0 | 4.0 | 2.1 |
| OR Quart 4 | 0.87 | 2.0 | 1.3 |
| p Value | 0.72 | 0.32 | 0.56 |
| 95% CI of | 0.40 | 0.50 | 0.56 |
| OR Quart4 | 1.9 | 8.5 | 2.9 |

**Brain-derived neurotrophic factor**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.308 | 0.654 | 0.359 | 1.45 | 0.359 | 0.654 |
| Average | 3.55 | 28.1 | 7.34 | 21.9 | 1.72 | 31.0 |
| Stdev | 36.7 | 125 | 62.1 | 67.8 | 5.73 | 131 |
| p(t-test) |  | 0.0048 |  | 0.43 |  | 0.0013 |
| Min | 1.00E-9 | 1.00E-9 | 1.00E-9 | 1.00E-9 | 1.00E-9 | 1.00E-9 |
| Max | 624 | 732 | 732 | 237 | 58.0 | 732 |
| n (Samp) | 293 | 61 | 339 | 12 | 212 | 55 |
| n (Patient) | 293 | 61 | 339 | 12 | 212 | 55 |

|  | At Enrollment | | |
|---|---|---|---|
|  | sCr or UO | sCr only | UO only |
| AUC | 0.66 | 0.71 | 0.64 |
| SE | 0.041 | 0.085 | 0.044 |
| p | 1.3E-4 | 0.015 | 0.0011 |
| nCohort 1 | 293 | 339 | 212 |
| nCohort 2 | 61 | 12 | 55 |
| Cutoff 1 | 0.247 | 0.404 | 0.342 |
| Sens 1 | 70% | 75% | 71% |
| Spec 1 | 48% | 54% | 49% |
| Cutoff 2 | 0.233 | 0.234 | 0.233 |
| Sens 2 | 80% | 83% | 82% |

(continued)

|  | At Enrollment | | |
|---|---|---|---|
|  | sCr or UO | sCr only | UO only |
| Spec 2 | 46% | 43% | 43% |
| Cutoff 3 | 0.0466 | 0.0852 | 0.0466 |
| Sens 3 | 90% | 92% | 91% |
| Spec 3 | 32% | 32% | 29% |
| Cutoff 4 | 0.834 | 0.877 | 0.885 |
| Sens 4 | 46% | 67% | 42% |
| Spec 4 | 70% | 71% | 70% |
| Cutoff 5 | 1.13 | 1.38 | 1.20 |
| Sens 5 | 39% | 50% | 38% |
| Spec 5 | 80% | 81% | 80% |
| Cutoff 6 | 2.51 | 3.36 | 3.07 |
| Sens 6 | 25% | 33% | 22% |
| Spec 6 | 90% | 90% | 90% |
| OR Quart 2 | 4.6 | 2.0 | 3.5 |
| p Value | 0.0087 | 0.57 | 0.022 |
| 95% CI of | 1.5 | 0.18 | 1.2 |
| OR Quart2 | 14 | 22 | 10 |
| OR Quart 3 | 4.7 | 2.0 | 3.2 |
| p Value | 0.0081 | 0.57 | 0.035 |
| 95% CI of | 1.5 | 0.18 | 1.1 |
| OR Quart3 | 15 | 22 | 9.5 |
| OR Quart 4 | 8.2 | 7.4 | 5.6 |
| p Value | 1.9E-4 | 0.063 | 0.0013 |
| 95% CI of | 2.7 | 0.89 | 2.0 |
| OR Quart4 | 25 | 62 | 16 |

**Immunoglobulin E**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.0560 | 0.0560 | 0.0560 | 0.352 | 0.0560 | 0.0560 |
| Average | 1.26 | 2.40 | 1.55 | 0.456 | 1.16 | 2.69 |
| Stdev | 2.15 | 9.84 | 4.92 | 0.455 | 2.07 | 10.6 |
| p(t-test) |  | 0.21 |  | 0.53 |  | 0.15 |
| Min | 0.0560 | 0.0560 | 0.0560 | 0.0560 | 0.0560 | 0.0560 |
| Max | 8.64 | 58.4 | 58.4 | 1.18 | 8.64 | 58.4 |
| n (Samp) | 138 | 35 | 164 | 8 | 111 | 30 |

(continued)

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| n (Patient) | 138 | 35 | 164 | 8 | 111 | 30 |

| | At Enrollment | | |
|---|---|---|---|
| | sCr or UO | sCr only | UO only |
| AUC | 0.48 | 0.53 | 0.47 |
| SE | 0.055 | 0.11 | 0.060 |
| p | 0.77 | 0.75 | 0.58 |
| nCohort 1 | 138 | 164 | 111 |
| nCohort 2 | 35 | 8 | 30 |
| Cutoff 1 | 0 | 0 | 0 |
| Sens 1 | 100% | 100% | 100% |
| Spec 1 | 0% | 0% | 0% |
| Cutoff 2 | 0 | 0 | 0 |
| Sens 2 | 100% | 100% | 100% |
| Spec 2 | 0% | 0% | 0% |
| Cutoff 3 | 0 | 0 | 0 |
| Sens 3 | 100% | 100% | 100% |
| Spec 3 | 0% | 0% | 0% |
| Cutoff 4 | 0.772 | 0.772 | 0.626 |
| Sens 4 | 29% | 25% | 27% |
| Spec 4 | 70% | 70% | 70% |
| Cutoff 5 | 2.02 | 2.02 | 1.80 |
| Sens 5 | 14% | 0% | 20% |
| Spec 5 | 80% | 80% | 80% |
| Cutoff 6 | 4.64 | 4.62 | 4.61 |
| Sens 6 | 6% | 0% | 7% |
| Spec 6 | 91% | 90% | 90% |
| OR Quart 2 | 1.4 | 0 | 0.53 |
| p Value | 0.56 | na | 0.36 |
| 95% CI of | 0.48 | na | 0.14 |
| OR Quart2 | 3.9 | na | 2.0 |
| OR Quart 3 | 1.4 | 1.8 | 1.4 |
| p Value | 0.56 | 0.46 | 0.53 |
| 95% CI of | 0.48 | 0.39 | 0.47 |
| OR Quart3 | 3.9 | 7.9 | 4.4 |
| OR Quart 4 | 0.88 | 0 | 1.7 |

(continued)

|  | At Enrollment | | |
|---|---|---|---|
|  | sCr or UO | sCr only | UO only |
| p Value | 0.81 | na | 0.37 |
| 95% CI of | 0.29 | na | 0.55 |
| OR Quart4 | 2.7 | na | 5.0 |

**Immunoglobulin M**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 8.61E-5 | 0.000232 | 0.000104 | 0.000650 | 7.70E-5 | 0.000227 |
| Average | 0.000261 | 0.00181 | 0.000461 | 0.00294 | 0.000219 | 0.00178 |
| Stdev | 0.000471 | 0.00411 | 0.00172 | 0.00449 | 0.000351 | 0.00438 |
| p(t-test) |  | 2.3E-5 |  | 4.7E-4 |  | 2.6E-4 |
| Min | 3.78E-8 | 3.78E-8 | 3.78E-8 | 1.38E-5 | 3.78E-8 | 3.78E-8 |
| Max | 0.00363 | 0.0186 | 0.0186 | 0.0131 | 0.00207 | 0.0186 |
| n (Samp) | 138 | 35 | 164 | 8 | 111 | 30 |
| n (Patient) | 138 | 35 | 164 | 8 | 111 | 30 |

|  | At Enrollment | | |
|---|---|---|---|
|  | sCr or UO | sCr only | UO only |
| AUC | 0.67 | 0.76 | 0.67 |
| SE | 0.054 | 0.10 | 0.059 |
| p | 0.0013 | 0.0083 | 0.0040 |
| nCohort 1 | 138 | 164 | 111 |
| nCohort 2 | 35 | 8 | 30 |
| Cutoff 1 | 0.000154 | 0.000222 | 0.000154 |
| Sens 1 | 71% | 75% | 70% |
| Spec 1 | 65% | 68% | 66% |
| Cutoff 2 | 4.67E-5 | 0.000211 | 4.67E-5 |
| Sens 2 | 80% | 88% | 80% |
| Spec 2 | 37% | 67% | 38% |
| Cutoff 3 | 1.78E-5 | 1.31E-5 | 1.88E-5 |
| Sens 3 | 91% | 100% | 90% |
| Spec 3 | 17% | 13% | 16% |
| Cutoff 4 | 0.000201 | 0.000242 | 0.000201 |
| Sens 4 | 60% | 62% | 57% |
| Spec 4 | 70% | 70% | 71% |
| Cutoff 5 | 0.000362 | 0.000429 | 0.000320 |

(continued)

| | At Enrollment | | |
|---|---|---|---|
| | sCr or UO | sCr only | UO only |
| Sens 5 | 40% | 50% | 43% |
| Spec 5 | 80% | 80% | 80% |
| Cutoff 6 | 0.000753 | 0.000848 | 0.000532 |
| Sens 6 | 31% | 50% | 37% |
| Spec 6 | 91% | 90% | 90% |
| OR Quart 2 | 0.63 | 0 | 0.77 |
| p Value | 0.50 | na | 0.72 |
| 95% CI of | 0.17 | na | 0.19 |
| OR Quart2 | 2.4 | na | 3.2 |
| OR Quart 3 | 1.9 | 3.1 | 1.8 |
| p Value | 0.27 | 0.33 | 0.36 |
| 95% CI of | 0.61 | 0.31 | 0.52 |
| OR Quart3 | 5.7 | 32 | 6.1 |
| OR Quart 4 | 3.2 | 4.3 | 3.4 |
| p Value | 0.033 | 0.20 | 0.040 |
| 95% CI of | 1.1 | 0.46 | 1.1 |
| OR Quart4 | 9.2 | 40 | 11 |

**Matrilysin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 10700 | 28400 | 12800 | 34900 | 12700 | 28400 |
| Average | 19300 | 54100 | 23500 | 75100 | 21600 | 56600 |
| Stdev | 25100 | 93200 | 41700 | 116000 | 27900 | 97500 |
| p(t-test) | | 6.2E-8 | | 1.5E-4 | | 7.2E-6 |
| Min | 1.00E-9 | 1270 | 1.00E-9 | 1300 | 1.00E-9 | 1270 |
| Max | 261000 | 485000 | 485000 | 406000 | 261000 | 485000 |
| n (Samp) | 294 | 61 | 340 | 12 | 213 | 55 |
| n (Patient) | 294 | 61 | 340 | 12 | 213 | 55 |

| | At Enrollment | | |
|---|---|---|---|
| | sCr or UO | sCr only | UO only |
| AUC | 0.68 | 0.68 | 0.67 |
| SE | 0.040 | 0.087 | 0.043 |
| p | 6.4E-6 | 0.040 | 7.5E-5 |
| nCohort 1 | 294 | 340 | 213 |

(continued)

| | At Enrollment | | |
| --- | --- | --- | --- |
| | sCr or UO | sCr only | UO only |
| nCohort 2 | 61 | 12 | 55 |
| Cutoff 1 | 15000 | 19900 | 15100 |
| Sens 1 | 70% | 75% | 71% |
| Spec 1 | 58% | 64% | 54% |
| Cutoff 2 | 9310 | 2610 | 12700 |
| Sens 2 | 80% | 83% | 80% |
| Spec 2 | 46% | 14% | 50% |
| Cutoff 3 | 3460 | 2010 | 6910 |
| Sens 3 | 90% | 92% | 91% |
| Spec 3 | 20% | 11% | 35% |
| Cutoff 4 | 22400 | 24500 | 24300 |
| Sens 4 | 54% | 67% | 55% |
| Spec 4 | 70% | 70% | 70% |
| Cutoff 5 | 30000 | 34000 | 35200 |
| Sens 5 | 48% | 50% | 38% |
| Spec 5 | 80% | 80% | 80% |
| Cutoff 6 | 46100 | 49700 | 51800 |
| Sens 6 | 25% | 42% | 18% |
| Spec 6 | 90% | 90% | 90% |
| OR Quart 2 | 0.85 | 0 | 3.3 |
| p Value | 0.77 | na | 0.032 |
| 95% CI of | 0.30 | na | 1.1 |
| OR Quart2 | 2.5 | na | 9.7 |
| OR Quart 3 | 2.2 | 0.66 | 3.6 |
| p Value | 0.090 | 0.65 | 0.020 |
| 95% CI of | 0.89 | 0.11 | 1.2 |
| OR Quart3 | 5.4 | 4.0 | 11 |
| OR Quart 4 | 5.1 | 2.4 | 5.7 |
| p Value | 1.7E-4 | 0.21 | 0.0012 |
| 95% CI of | 2.2 | 0.61 | 2.0 |
| OR Quart4 | 12 | 9.8 | 16 |

**Heparan Sulfate**

| | sCr or UO | | sCr only | | UO only | |
| --- | --- | --- | --- | --- | --- | --- |
| | Cohort 1 | Cohort 2 | Cohort 1 | Cohort 2 | Cohort 1 | Cohort 2 |
| Median | 3.02 | 6.93 | nd | nd | 2.90 | 7.18 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Average | 4.51 | 7.97 | nd | nd | 4.26 | 8.08 |
| Stdev | 3.90 | 5.33 | nd | nd | 3.75 | 5.61 |
| p(t-test) |  | 0.011 | nd | nd |  | 0.0075 |
| Min | 0.121 | 0.970 | nd | nd | 0.121 | 0.970 |
| Max | 16.3 | 18.3 | nd | nd | 14.5 | 18.3 |
| n (Samp) | 73 | 11 | nd | nd | 59 | 10 |
| n (Patient) | 73 | 11 | nd | nd | 59 | 10 |

|  | At Enrollment | | |
|---|---|---|---|
|  | sCr or UO | sCr only | UO only |
| AUC | 0.70 | nd | 0.71 |
| SE | 0.093 | nd | 0.097 |
| p | 0.034 | nd | 0.031 |
| nCohort 1 | 73 | nd | 59 |
| nCohort 2 | 11 | nd | 10 |
| Cutoff 1 | 5.17 | nd | 5.17 |
| Sens 1 | 73% | nd | 70% |
| Spec 1 | 67% | nd | 69% |
| Cutoff 2 | 3.64 | nd | 3.64 |
| Sens 2 | 82% | nd | 80% |
| Spec 2 | 59% | nd | 63% |
| Cutoff 3 | 1.42 | nd | 1.42 |
| Sens 3 | 91% | nd | 90% |
| Spec 3 | 21% | nd | 24% |
| Cutoff 4 | 5.61 | nd | 5.34 |
| Sens 4 | 64% | nd | 60% |
| Spec 4 | 71% | nd | 71% |
| Cutoff 5 | 7.80 | nd | 7.84 |
| Sens 5 | 36% | nd | 40% |
| Spec 5 | 81% | nd | 81% |
| Cutoff 6 | 10.6 | nd | 10.6 |
| Sens 6 | 36% | nd | 40% |
| Spec 6 | 90% | nd | 92% |
| OR Quart 2 | 0 | nd | 0 |
| p Value | na | nd | na |
| 95% CI of | na | nd | na |

(continued)

|  | At Enrollment | | |
|---|---|---|---|
|  | sCr or UO | sCr only | UO only |
| OR Quart2 | na | nd | na |
| OR Quart 3 | 2.2 | nd | 1.6 |
| p Value | 0.39 | nd | 0.63 |
| 95% CI of | 0.36 | nd | 0.23 |
| OR Quart3 | 14 | nd | 11 |
| OR Quart 4 | 3.0 | nd | 2.9 |
| p Value | 0.23 | nd | 0.25 |
| 95% CI of | 0.51 | nd | 0.48 |
| OR Quart4 | 17 | nd | 17 |

**Transmembrane glycoprotein NMB**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1.65 | 2.42 | nd | nd | 1.64 | 2.66 |
| Average | 1.88 | 3.78 | nd | nd | 1.90 | 3.95 |
| Stdev | 1.21 | 4.87 | nd | nd | 1.23 | 5.06 |
| p(t-test) |  | 3.5E-4 | nd | nd |  | 6.1E-4 |
| Min | 0.340 | 0.706 | nd | nd | 0.340 | 0.706 |
| Max | 8.02 | 25.3 | nd | nd | 8.02 | 25.3 |
| n (Samp) | 111 | 24 | nd | nd | 92 | 22 |
| n (Patient) | 111 | 24 | nd | nd | 92 | 22 |

|  | At Enrollment | | |
|---|---|---|---|
|  | sCr or UO | sCr only | UO only |
| AUC | 0.70 | nd | 0.70 |
| SE | 0.064 | nd | 0.067 |
| p | 0.0016 | nd | 0.0022 |
| nCohort 1 | 111 | nd | 92 |
| nCohort 2 | 24 | nd | 22 |
| Cutoff 1 | 1.74 | nd | 1.74 |
| Sens 1 | 71% | nd | 73% |
| Spec 1 | 55% | nd | 54% |
| Cutoff 2 | 1.44 | nd | 1.43 |
| Sens 2 | 83% | nd | 82% |
| Spec 2 | 42% | nd | 42% |
| Cutoff 3 | 1.04 | nd | 1.04 |

(continued)

|  | At Enrollment | | |
|---|---|---|---|
|  | sCr or UO | sCr only | UO only |
| Sens 3 | 92% | nd | 91% |
| Spec 3 | 27% | nd | 26% |
| Cutoff 4 | 2.24 | nd | 2.27 |
| Sens 4 | 54% | nd | 59% |
| Spec 4 | 70% | nd | 71% |
| Cutoff 5 | 2.43 | nd | 2.61 |
| Sens 5 | 50% | nd | 50% |
| Spec 5 | 80% | nd | 80% |
| Cutoff 6 | 3.46 | nd | 3.46 |
| Sens 6 | 38% | nd | 41% |
| Spec 6 | 90% | nd | 90% |
| OR Quart 2 | 1.3 | nd | 1.3 |
| p Value | 0.72 | nd | 0.72 |
| 95% CI of | 0.27 | nd | 0.27 |
| OR Quart2 | 6.5 | nd | 6.6 |
| OR Quart 3 | 1.7 | nd | 1.4 |
| p Value | 0.48 | nd | 0.69 |
| 95% CI of | 0.38 | nd | 0.28 |
| OR Quart3 | 7.9 | nd | 6.9 |
| OR Quart 4 | 5.5 | nd | 5.1 |
| p Value | 0.016 | nd | 0.024 |
| 95% CI of | 1.4 | nd | 1.2 |
| OR Quart4 | 22 | nd | 21 |

**Cadherin-3**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 12.8 | 6.85 | nd | nd | 11.2 | 6.85 |
| Average | 22.5 | 19.2 | nd | nd | 23.0 | 20.5 |
| Stdev | 24.0 | 35.9 | nd | nd | 25.8 | 37.2 |
| p(t-test) |  | 0.58 | nd | nd |  | 0.71 |
| Min | 1.16 | 1.03 | nd | nd | 1.16 | 1.72 |
| Max | 142 | 164 | nd | nd | 142 | 164 |
| n (Samp) | 111 | 24 | nd | nd | 92 | 22 |
| n (Patient) | 111 | 24 | nd | nd | 92 | 22 |

|  | At Enrollment | | |
|---|---|---|---|
|  | sCr or UO | sCr only | UO only |
| AUC | 0.35 | nd | 0.38 |
| SE | 0.066 | nd | 0.070 |
| p | 0.025 | nd | 0.078 |
| nCohort 1 | 111 | nd | 92 |
| nCohort 2 | 24 | nd | 22 |
| Cutoff 1 | 4.14 | nd | 4.09 |
| Sens 1 | 71% | nd | 73% |
| Spec 1 | 11% | nd | 11% |
| Cutoff 2 | 2.21 | nd | 2.53 |
| Sens 2 | 83% | nd | 82% |
| Spec 2 | 4% | nd | 3% |
| Cutoff 3 | 1.72 | nd | 2.13 |
| Sens 3 | 92% | nd | 91% |
| Spec 3 | 4% | nd | 3% |
| Cutoff 4 | 26.6 | nd | 27.2 |
| Sens 4 | 12% | nd | 14% |
| Spec 4 | 70% | nd | 71% |
| Cutoff 5 | 37.1 | nd | 40.4 |
| Sens 5 | 12% | nd | 14% |
| Spec 5 | 80% | nd | 80% |
| Cutoff 6 | 50.2 | nd | 56.2 |
| Sens 6 | 8% | nd | 9% |
| Spec 6 | 90% | nd | 90% |
| OR Quart 2 | 1.4 | nd | 1.9 |
| p Value | 0.69 | nd | 0.42 |
| 95% CI of | 0.28 | nd | 0.40 |
| OR Quart2 | 6.7 | nd | 8.8 |
| OR Quart 3 | 3.2 | nd | 2.3 |
| p Value | 0.11 | nd | 0.28 |
| 95% CI of | 0.76 | nd | 0.51 |
| OR Quart3 | 13 | nd | 10 |
| OR Quart 4 | 3.9 | nd | 3.5 |
| p Value | 0.060 | nd | 0.093 |
| 95% CI of | 0.94 | nd | 0.81 |
| OR Quart4 | 16 | nd | 15 |

**Stromelysin-2**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.115 | 1.00E-9 | nd | nd | 0.114 | 1.00E-9 |
| Average | 0.291 | 0.710 | nd | nd | 0.320 | 0.761 |
| Stdev | 0.506 | 1.85 | nd | nd | 0.548 | 1.93 |
| p(t-test) | | 0.041 | nd | nd | | 0.060 |
| Min | 1.00E-9 | 1.00E-9 | nd | nd | 1.00E-9 | 1.00E-9 |
| Max | 3.04 | 7.26 | nd | nd | 3.04 | 7.26 |
| n (Samp) | 110 | 24 | nd | nd | 91 | 22 |
| n (Patient) | 110 | 24 | nd | nd | 91 | 22 |

| | At Enrollment | | |
|---|---|---|---|
| | sCr or UO | sCr only | UO only |
| AUC | 0.39 | nd | 0.38 |
| SE | 0.066 | nd | 0.070 |
| p | 0.090 | nd | 0.096 |
| nCohort 1 | 110 | nd | 91 |
| nCohort 2 | 24 | nd | 22 |
| Cutoff 1 | 0 | nd | 0 |
| Sens 1 | 100% | nd | 100% |
| Spec 1 | 0% | nd | 0% |
| Cutoff 2 | 0 | nd | 0 |
| Sens 2 | 100% | nd | 100% |
| Spec 2 | 0% | nd | 0% |
| Cutoff 3 | 0 | nd | 0 |
| Sens 3 | 100% | nd | 100% |
| Spec 3 | 0% | nd | 0% |
| Cutoff 4 | 0.257 | nd | 0.305 |
| Sens 4 | 29% | nd | 27% |
| Spec 4 | 70% | nd | 70% |
| Cutoff 5 | 0.363 | nd | 0.546 |
| Sens 5 | 21% | nd | 23% |
| Spec 5 | 80% | nd | 80% |
| Cutoff 6 | 0.672 | nd | 0.676 |
| Sens 6 | 17% | nd | 18% |
| Spec 6 | 90% | nd | 90% |
| OR Quart 2 | 0.25 | nd | 0.80 |
| p Value | 0.099 | nd | 0.76 |

(continued)

|  | At Enrollment | | |
|---|---|---|---|
|  | sCr or UO | sCr only | UO only |
| 95% CI of | 0.048 | nd | 0.19 |
| OR Quart2 | 1.3 | nd | 3.3 |
| OR Quart 3 | 0.24 | nd | 0.18 |
| p Value | 0.092 | nd | 0.13 |
| 95% CI of | 0.046 | nd | 0.019 |
| OR Quart3 | 1.3 | nd | 1.6 |
| OR Quart 4 | 2.5 | nd | 3.6 |
| p Value | 0.097 | nd | 0.040 |
| 95% CI of | 0.85 | nd | 1.1 |
| OR Quart4 | 7.4 | nd | 12 |

**Cathepsin S**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 2.18 | 4.98 | nd | nd | 2.28 | 4.49 |
| Average | 2.70 | 8.28 | nd | nd | 2.66 | 8.52 |
| Stdev | 2.47 | 11.8 | nd | nd | 2.21 | 12.5 |
| p(t-test) |  | 0.0029 | nd | nd |  | 0.0053 |
| Min | 1.00E-9 | 1.66 | nd | nd | 1.00E-9 | 1.66 |
| Max | 9.78 | 39.3 | nd | nd | 7.93 | 39.3 |
| n (Samp) | 50 | 9 | nd | nd | 42 | 8 |
| n (Patient) | 50 | 9 | nd | nd | 42 | 8 |

|  | At Enrollment | | |
|---|---|---|---|
|  | sCr or UO | sCr only | UO only |
| AUC | 0.76 | nd | 0.75 |
| SE | 0.098 | nd | 0.11 |
| p | 0.0073 | nd | 0.020 |
| nCohort 1 | 50 | nd | 42 |
| nCohort 2 | 9 | nd | 8 |
| Cutoff 1 | 3.88 | nd | 3.88 |
| Sens 1 | 78% | nd | 75% |
| Spec 1 | 80% | nd | 81% |
| Cutoff 2 | 2.09 | nd | 2.09 |
| Sens 2 | 89% | nd | 88% |
| Spec 2 | 50% | nd | 48% |

(continued)

|  | At Enrollment | | |
|---|---|---|---|
|  | sCr or UO | sCr only | UO only |
| Cutoff 3 | 1.63 | nd | 1.63 |
| Sens 3 | 100% | nd | 100% |
| Spec 3 | 42% | nd | 40% |
| Cutoff 4 | 2.83 | nd | 2.88 |
| Sens 4 | 78% | nd | 75% |
| Spec 4 | 70% | nd | 71% |
| Cutoff 5 | 3.88 | nd | 3.88 |
| Sens 5 | 78% | nd | 75% |
| Spec 5 | 80% | nd | 81% |
| Cutoff 6 | 6.63 | nd | 6.56 |
| Sens 6 | 11% | nd | 12% |
| Spec 6 | 90% | nd | 90% |
| OR Quart 2 | >2.2 | nd | >2.2 |
| p Value | <0.55 | nd | <0.55 |
| 95% CI of | >0.17 | nd | >0.17 |
| OR Quart2 | na | nd | na |
| OR Quart 3 | >2.2 | nd | >1.1 |
| p Value | <0.55 | nd | <0.95 |
| 95% CI of | >0.17 | nd | >0.061 |
| OR Quart3 | na | nd | na |
| OR Quart 4 | >7.0 | nd | >7.5 |
| p Value | <0.097 | nd | <0.090 |
| 95% CI of | >0.71 | nd | >0.73 |
| OR Quart4 | na | nd | na |

**Urokinase-type plasminogen activator**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 15.3 | 3.23 | nd | nd | 15.3 | 3.21 |
| Average | 24.9 | 9.94 | nd | nd | 25.8 | 10.6 |
| Stdev | 28.6 | 19.6 | nd | nd | 30.1 | 20.8 |
| p(t-test) |  | 0.14 | nd | nd |  | 0.18 |
| Min | 0.308 | 1.24 | nd | nd | 0.308 | 1.24 |
| Max | 119 | 62.0 | nd | nd | 119 | 62.0 |
| n (Samp) | 50 | 9 | nd | nd | 42 | 8 |
| n (Patient) | 50 | 9 | nd | nd | 42 | 8 |

| | At Enrollment | | |
|---|---|---|---|
| | sCr or UO | sCr only | UO only |
| AUC | 0.31 | nd | 0.31 |
| SE | 0.10 | nd | 0.11 |
| p | 0.067 | nd | 0.081 |
| nCohort 1 | 50 | nd | 42 |
| nCohort 2 | 9 | nd | 8 |
| Cutoff 1 | 2.44 | nd | 1.97 |
| Sens 1 | 78% | nd | 75% |
| Spec 1 | 18% | nd | 17% |
| Cutoff 2 | 1.82 | nd | 1.82 |
| Sens 2 | 89% | nd | 88% |
| Spec 2 | 16% | nd | 17% |
| Cutoff 3 | 0.670 | nd | 0.355 |
| Sens 3 | 100% | nd | 100% |
| Spec 3 | 6% | nd | 5% |
| Cutoff 4 | 29.2 | nd | 31.8 |
| Sens 4 | 11% | nd | 12% |
| Spec 4 | 70% | nd | 71% |
| Cutoff 5 | 42.1 | nd | 44.1 |
| Sens 5 | 11% | nd | 12% |
| Spec 5 | 80% | nd | 81% |
| Cutoff 6 | 55.9 | nd | 55.9 |
| Sens 6 | 11% | nd | 12% |
| Spec 6 | 90% | nd | 90% |
| OR Quart 2 | 0 | nd | 0 |
| p Value | na | nd | na |
| 95% CI of | na | nd | na |
| OR Quart2 | na | nd | na |
| OR Quart 3 | 7.0 | nd | 5.3 |
| p Value | 0.097 | nd | 0.16 |
| 95% CI of | 0.71 | nd | 0.51 |
| OR Quart3 | 69 | nd | 56 |
| OR Quart 4 | 3.8 | nd | 4.0 |
| p Value | 0.27 | nd | 0.26 |
| 95% CI of | 0.35 | nd | 0.35 |
| OR Quart4 | 42 | nd | 45 |

**C-C motif chemokine 23**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.00309 | 0.0431 | nd | nd | 0.00309 | 0.0339 |
| Average | 0.00691 | 0.246 | nd | nd | 0.00889 | 0.262 |
| Stdev | 0.00875 | 0.593 | nd | nd | 0.0186 | 0.632 |
| p(t-test) |  | 0.0043 | nd | nd |  | 0.0092 |
| Min | 1.00E-9 | 0.0155 | nd | nd | 1.00E-9 | 0.0155 |
| Max | 0.0375 | 1.82 | nd | nd | 0.118 | 1.82 |
| n (Samp) | 50 | 9 | nd | nd | 42 | 8 |
| n (Patient) | 50 | 9 | nd | nd | 42 | 8 |

|  | At Enrollment | | |
|---|---|---|---|
|  | sCr or UO | sCr only | UO only |
| AUC | 0.94 | nd | 0.93 |
| SE | 0.054 | nd | 0.065 |
| p | 2.2E-16 | nd | 3.5E-11 |
| nCohort 1 | 50 | nd | 42 |
| nCohort 2 | 9 | nd | 8 |
| Cutoff 1 | 0.0168 | nd | 0.0168 |
| Sens 1 | 78% | nd | 75% |
| Spec 1 | 90% | nd | 90% |
| Cutoff 2 | 0.0160 | nd | 0.0160 |
| Sens 2 | 89% | nd | 88% |
| Spec 2 | 84% | nd | 83% |
| Cutoff 3 | 0.0131 | nd | 0.0131 |
| Sens 3 | 100% | nd | 100% |
| Spec 3 | 82% | nd | 81% |
| Cutoff 4 | 0.00870 | nd | 0.00974 |
| Sens 4 | 100% | nd | 100% |
| Spec 4 | 70% | nd | 71% |
| Cutoff 5 | 0.0112 | nd | 0.0131 |
| Sens 5 | 100% | nd | 100% |
| Spec 5 | 80% | nd | 81% |
| Cutoff 6 | 0.0168 | nd | 0.0168 |
| Sens 6 | 78% | nd | 75% |
| Spec 6 | 90% | nd | 90% |
| OR Quart 2 | >0 | nd | >0 |
| p Value | <na | nd | <na |

(continued)

|  | At Enrollment | | |
|---|---|---|---|
|  | sCr or UO | sCr only | UO only |
| 95% CI of | >na | nd | >na |
| OR Quart2 | na | nd | na |
| OR Quart 3 | >2.2 | nd | >2.4 |
| p Value | <0.55 | nd | <0.50 |
| 95% CI of | >0.17 | nd | >0.19 |
| OR Quart3 | na | nd | na |
| OR Quart 4 | >12 | nd | >10 |
| p Value | <0.030 | nd | <0.048 |
| 95% CI of | >1.3 | nd | >1.0 |
| OR Quart4 | na | nd | na |

**Carcinoembryonic antigen-related cell adhesion molecule 1**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 5.69 | 11.1 | nd | nd | 6.01 | 11.5 |
| Average | 7.27 | 19.8 | nd | nd | 7.42 | 21.1 |
| Stdev | 6.10 | 30.6 | nd | nd | 6.37 | 31.6 |
| p(t-test) |  | 1.1E-4 | nd | nd |  | 1.7E-4 |
| Min | 0.565 | 1.86 | nd | nd | 0.565 | 1.86 |
| Max | 31.5 | 155 | nd | nd | 31.5 | 155 |
| n (Samp) | 111 | 24 | nd | nd | 92 | 22 |
| n (Patient) | 111 | 24 | nd | nd | 92 | 22 |

|  | At Enrollment | | |
|---|---|---|---|
|  | sCr or UO | sCr only | UO only |
| AUC | 0.75 | nd | 0.77 |
| SE | 0.061 | nd | 0.062 |
| p | 6.3E-5 | nd | 1.5E-5 |
| nCohort 1 | 111 | nd | 92 |
| nCohort 2 | 24 | nd | 22 |
| Cutoff 1 | 6.58 | nd | 7.65 |
| Sens 1 | 71% | nd | 73% |
| Spec 1 | 59% | nd | 68% |
| Cutoff 2 | 5.58 | nd | 6.42 |
| Sens 2 | 83% | nd | 82% |
| Spec 2 | 50% | nd | 58% |

(continued)

|  | At Enrollment | | |
|---|---|---|---|
|  | sCr or UO | sCr only | UO only |
| Cutoff 3 | 4.81 | nd | 5.58 |
| Sens 3 | 92% | nd | 91% |
| Spec 3 | 46% | nd | 48% |
| Cutoff 4 | 8.13 | nd | 7.88 |
| Sens 4 | 62% | nd | 68% |
| Spec 4 | 70% | nd | 71% |
| Cutoff 5 | 9.34 | nd | 9.63 |
| Sens 5 | 54% | nd | 59% |
| Spec 5 | 80% | nd | 80% |
| Cutoff 6 | 15.3 | nd | 15.3 |
| Sens 6 | 42% | nd | 45% |
| Spec 6 | 90% | nd | 90% |
| OR Quart 2 | 5.5 | nd | 3.1 |
| p Value | 0.13 | nd | 0.34 |
| 95% CI of | 0.61 | nd | 0.30 |
| OR Quart2 | 50 | nd | 32 |
| OR Quart 3 | 5.5 | nd | 9.0 |
| p Value | 0.13 | nd | 0.047 |
| 95% CI of | 0.61 | nd | 1.0 |
| OR Quart3 | 50 | nd | 79 |
| OR Quart 4 | 20 | nd | 16 |
| p Value | 0.0055 | nd | 0.0100 |
| 95% CI of | 2.4 | nd | 2.0 |
| OR Quart4 | 160 | nd | 140 |

**Insulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1.08 | 0.961 | 1.01 | 1.46 | 1.28 | 0.955 |
| Average | 33.4 | 37.8 | 28.7 | 153 | 50.8 | 4.23 |
| Stdev | 173 | 201 | 159 | 419 | 221 | 10.00 |
| p(t-test) |  | 0.90 |  | 0.055 |  | 0.25 |
| Min | 0.00344 | 0.00344 | 0.00344 | 0.593 | 0.00344 | 0.00344 |
| Max | 1750 | 1190 | 1750 | 1190 | 1750 | 50.1 |
| n (Samp) | 138 | 35 | 164 | 8 | 111 | 30 |
| n (Patient) | 138 | 35 | 164 | 8 | 111 | 30 |

|  | At Enrollment | | |
|---|---|---|---|
|  | sCr or UO | sCr only | UO only |
| AUC | 0.46 | 0.58 | 0.40 |
| SE | 0.055 | 0.11 | 0.060 |
| p | 0.42 | 0.45 | 0.093 |
| nCohort 1 | 138 | 164 | 111 |
| nCohort 2 | 35 | 8 | 30 |
| Cutoff 1 | 0.613 | 0.960 | 0.613 |
| Sens 1 | 71% | 75% | 70% |
| .Spec 1 | 26% | 47% | 21% |
| Cutoff 2 | 0.593 | 0.595 | 0.556 |
| Sens 2 | 80% | 88% | 80% |
| Spec 2 | 24% | 24% | 19% |
| Cutoff 3 | 0.316 | 0.562 | 0.206 |
| Sens 3 | 91% | 100% | 90% |
| Spec 3 | 13% | 23% | 9% |
| Cutoff 4 | 3.65 | 3.39 | 4.53 |
| Sens 4 | 20% | 25% | 17% |
| Spec 4 | 70% | 70% | 70% |
| Cutoff 5 | 12.8 | 8.65 | 18.9 |
| Sens 5 | 9% | 25% | 7% |
| Spec 5 | 80% | 80% | 81% |
| Cutoff 6 | 34.1 | 29.0 | 38.4 |
| Sens 6 | 6% | 12% | 3% |
| Spec 6 | 91% | 90% | 90% |
| OR Quart 2 | 1.9 | 0.49 | 1.3 |
| p Value | 0.25 | 0.56 | 0.71 |
| 95% CI of | 0.63 | 0.043 | 0.35 |
| OR Quart2 | 5.8 | 5.6 | 4.7 |
| OR Quart 3 | 1.9 | 1.5 | 2.1 |
| p Value | 0.25 | 0.65 | 0.22 |
| 95% CI of | 0.63 | 0.24 | 0.64 |
| OR Quart3 | 5.8 | 9.7 | 7.2 |
| OR Quart 4 | 1.7 | 1.0 | 2.5 |
| p Value | 0.37 | 1.0 | 0.14 |
| 95% CI of | 0.54 | 0.13 | 0.75 |
| OR Quart4 | 5.2 | 7.4 | 8.2 |

Table 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

**Angiopoietin-2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1740 | 2400 | nd | nd | 1800 | 2410 |
| Average | 2060 | 4510 | nd | nd | 2100 | 5090 |
| Stdev | 1170 | 8900 | nd | nd | 1200 | 9690 |
| p(t-test) |  | 0.024 | nd | nd |  | 0.021 |
| Min | 1.00E-9 | 708 | nd | nd | 1.00E-9 | 1190 |
| Max | 7300 | 39900 | nd | nd | 7300 | 39900 |
| n (Samp) | 72 | 18 | nd | nd | 60 | 15 |
| n (Patient) | 72 | 18 | nd | nd | 60 | 15 |

|  | At Enrollment | | |
|---|---|---|---|
|  | sCr or UO | sCr only | UO only |
| AUC | 0.64 | nd | 0.68 |
| SE | 0.077 | nd | 0.083 |
| p | 0.071 | nd | 0.032 |
| nCohort 1 | 72 | nd | 60 |
| nCohort 2 | 18 | nd | 15 |
| Cutoff 1 | 1680 | nd | 1880 |
| Sens 1 | 72% | nd | 73% |
| Spec 1 | 47% | nd | 53% |
| Cutoff 2 | 1430 | nd | 1680 |
| Sens 2 | 83% | nd | 80% |
| Spec 2 | 35% | nd | 43% |
| Cutoff 3 | 1150 | nd | 1380 |
| Sens 3 | 94% | nd | 93% |
| Spec 3 | 21% | nd | 35% |
| Cutoff 4 | 2280 | nd | 2430 |
| Sens 4 | 56% | nd | 47% |
| Spec 4 | 71% | nd | 70% |
| Cutoff 5 | 2810 | nd | 2810 |
| Sens 5 | 33% | nd | 40% |
| Spec 5 | 81% | nd | 80% |
| Cutoff 6 | 3480 | nd | 3480 |
| Sens 6 | 22% | nd | 27% |
| Spec 6 | 90% | nd | 90% |
| OR Quart 2 | 2.1 | nd | 4.5 |

(continued)

|  | At Enrollment | | |
|---|---|---|---|
|  | sCr or UO | sCr only | UO only |
| p Value | 0.42 | nd | 0.20 |
| 95% CI of | 0.34 | nd | 0.46 |
| OR Quart2 | 13 | nd | 45 |
| OR Quart 3 | 2.2 | nd | 4.5 |
| p Value | 0.39 | nd | 0.20 |
| 95% CI of | 0.36 | nd | 0.46 |
| OR Quart3 | 14 | nd | 45 |
| OR Quart 4 | 5.3 | nd | 7.8 |
| p Value | 0.052 | nd | 0.071 |
| 95% CI of | 0.99 | nd | 0.84 |
| OR Quart4 | 29 | nd | 73 |

**Brain-derived neurotrophic factor**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1810 | 1230 | nd | nd | 1810 | 1290 |
| Average | 2490 | 1730 | nd | nd | 2410 | 1840 |
| Stdev | 2410 | 1670 | nd | nd | 2310 | 1800 |
| p(t-test) |  | 0.19 | nd | nd |  | 0.35 |
| Min | 23.0 | 118 | nd | nd | 23.0 | 118 |
| Max | 12200 | 6290 | nd | nd | 12200 | 6290 |
| n (Samp) | 76 | 20 | nd | nd | 64 | 17 |
| n (Patient) | 76 | 20 | nd | nd | 64 | 17 |

|  | At Enrollment | | |
|---|---|---|---|
|  | sCr or UO | sCr only | UO only |
| AUC | 0.40 | nd | 0.42 |
| SE | 0.074 | nd | 0.080 |
| p | 0.16 | nd | 0.29 |
| nCohort 1 | 76 | nd | 64 |
| nCohort 2 | 20 | nd | 17 |
| Cutoff 1 | 792 | nd | 712 |
| Sens 1 | 70% | nd | 71% |
| Spec 1 | 26% | nd | 25% |
| Cutoff 2 | 542 | nd | 313 |
| Sens 2 | 80% | nd | 82% |

(continued)

|  | At Enrollment | | |
|---|---|---|---|
|  | sCr or UO | sCr only | UO only |
| Spec 2 | 17% | nd | 11% |
| Cutoff 3 | 298 | nd | 289 |
| Sens 3 | 90% | nd | 94% |
| Spec 3 | 9% | nd | 11% |
| Cutoff 4 | 2950 | nd | 2950 |
| Sens 4 | 15% | nd | 18% |
| Spec 4 | 71% | nd | 70% |
| Cutoff 5 | 3590 | nd | 3460 |
| Sens 5 | 10% | nd | 18% |
| Spec 5 | 80% | nd | 81% |
| Cutoff 6 | 6170 | nd | 6170 |
| Sens 6 | 5% | nd | 6% |
| Spec 6 | 91% | nd | 91% |
| OR Quart 2 | 1.4 | nd | 1.5 |
| p Value | 0.68 | nd | 0.63 |
| 95% CI of | 0.28 | nd | 0.29 |
| OR Quart2 | 7.1 | nd | 7.7 |
| OR Quart 3 | 3.5 | nd | 2.0 |
| p Value | 0.097 | nd | 0.39 |
| 95% CI of | 0.80 | nd | 0.41 |
| OR Quart3 | 15 | nd | 9.8 |
| OR Quart 4 | 1.8 | nd | 2.0 |
| p Value | 0.44 | nd | 0.39 |
| 95% CI of | 0.39 | nd | 0.41 |
| OR Quart4 | 8.8 | nd | 9.8 |

**Immunoglobulin E**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 33.7 | 27.4 | 33.4 | 24.0 | 40.0 | 30.8 |
| Average | 135 | 56.1 | 120 | 55.4 | 136 | 53.1 |
| Stdev | 314 | 85.0 | 291 | 75.5 | 309 | 83.9 |
| p(t-test) |  | 0.14 |  | 0.51 |  | 0.14 |
| Min | 0.140 | 0.586 | 0.140 | 6.65 | 0.140 | 0.586 |
| Max | 1940 | 436 | 1940 | 247 | 1940 | 436 |
| n (Samp) | 139 | 36 | 165 | 9 | 110 | 31 |

(continued)

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| n (Patient) | 139 | 36 | 165 | 9 | 110 | 31 |

| | At Enrollment | | |
|---|---|---|---|
| | sCr or UO | sCr only | UO only |
| AUC | 0.42 | 0.46 | 0.40 |
| SE | 0.055 | 0.10 | 0.060 |
| p | 0.16 | 0.69 | 0.087 |
| nCohort 1 | 139 | 165 | 110 |
| nCohort 2 | 36 | 9 | 31 |
| Cutoff 1 | 14.3 | 13.5 | 15.4 |
| Sens 1 | 72% | 78% | 71% |
| Spec 1 | 23% | 23% | 26% |
| Cutoff 2 | 10.7 | 13.1 | 6.48 |
| Sens 2 | 81% | 89% | 81% |
| Spec 2 | 17% | 21% | 8% |
| Cutoff 3 | 3.23 | 6.48 | 3.23 |
| Sens 3 | 92% | 100% | 90% |
| Spec 3 | 6% | 12% | 5% |
| Cutoff 4 | 81.0 | 72.8 | 89.5 |
| Sens 4 | 17% | 22% | 16% |
| Spec 4 | 71% | 70% | 70% |
| Cutoff 5 | 125 | 119 | 151 |
| Sens 5 | 14% | 11% | 6% |
| Spec 5 | 81% | 80% | 80% |
| Cutoff 6 | 281 | 242 | 281 |
| Sens 6 | 3% | 11% | 3% |
| Spec 6 | 91% | 90% | 90% |
| OR Quart 2 | 2.9 | 3.2 | 3.2 |
| p Value | 0.066 | 0.32 | 0.073 |
| 95% CI of | 0.93 | 0.32 | 0.90 |
| OR Quart2 | 9.2 | 32 | 11 |
| OR Quart 3 | 1.7 | 2.0 | 2.4 |
| p Value | 0.37 | 0.56 | 0.19 |
| 95% CI of | 0.52 | 0.18 | 0.64 |
| OR Quart3 | 5.8 | 23 | 8.7 |
| OR Quart 4 | 2.7 | 3.2 | 2.8 |

(continued)

|  | At Enrollment | | |
|---|---|---|---|
|  | sCr or UO | sCr only | UO only |
| p Value | 0.094 | 0.32 | 0.12 |
| 95% CI of | 0.84 | 0.32 | 0.76 |
| OR Quart4 | 8.5 | 32 | 10 |

**Immunoglobulin M**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.519 | 0.583 | 0.527 | 0.490 | 0.486 | 0.570 |
| Average | 0.736 | 0.752 | 0.747 | 0.624 | 0.686 | 0.746 |
| Stdev | 0.697 | 0.714 | 0.711 | 0.472 | 0.633 | 0.750 |
| p(t-test) |  | 0.90 |  | 0.61 |  | 0.66 |
| Min | 0.0493 | 0.0919 | 0.0493 | 0.0919 | 0.0815 | 0.0919 |
| Max | 4.82 | 3.51 | 4.82 | 1.63 | 4.82 | 3.51 |
| n (Samp) | 139 | 36 | 165 | 9 | 110 | 31 |
| n (Patient) | 139 | 36 | 165 | 9 | 110 | 31 |

|  | At Enrollment | | |
|---|---|---|---|
|  | sCr or UO | sCr only | UO only |
| AUC | 0.52 | 0.47 | 0.52 |
| SE | 0.055 | 0.10 | 0.059 |
| p | 0.69 | 0.77 | 0.71 |
| nCohort 1 | 139 | 165 | 110 |
| nCohort 2 | 36 | 9 | 31 |
| Cutoff 1 | 0.370 | 0.346 | 0.353 |
| Sens 1 | 72% | 78% | 71% |
| Spec 1 | 34% | 28% | 32% |
| Cutoff 2 | 0.299 | 0.215 | 0.293 |
| Sens 2 | 81% | 89% | 81% |
| Spec 2 | 24% | 13% | 24% |
| Cutoff 3 | 0.217 | 0.0815 | 0.217 |
| Sens 3 | 92% | 100% | 90% |
| Spec 3 | 15% | 1% | 15% |
| Cutoff 4 | 0.794 | 0.794 | 0.773 |
| Sens 4 | 28% | 22% | 29% |
| Spec 4 | 71% | 70% | 72% |
| Cutoff 5 | 1.07 | 1.05 | 0.898 |

(continued)

|  | At Enrollment | | |
|---|---|---|---|
|  | sCr or UO | sCr only | UO only |
| Sens 5 | 14% | 11% | 23% |
| Spec 5 | 81% | 80% | 80% |
| Cutoff 6 | 1.62 | 1.53 | 1.49 |
| Sens 6 | 8% | 11% | 6% |
| Spec 6 | 91% | 90% | 91% |
| OR Quart 2 | 1.1 | 1.0 | 0.70 |
| p Value | 0.83 | 0.98 | 0.55 |
| 95% CI of | 0.39 | 0.14 | 0.21 |
| OR Quart2 | 3.3 | 7.6 | 2.3 |
| OR Quart 3 | 1.5 | 1.5 | 1.2 |
| p Value | 0.47 | 0.65 | 0.78 |
| 95% CI of | 0.52 | 0.24 | 0.39 |
| OR Quart3 | 4.1 | 9.7 | 3.5 |
| OR Quart 4 | 0.97 | 1.0 | 0.96 |
| p Value | 0.96 | 0.98 | 0.95 |
| 95% CI of | 0.33 | 0.14 | 0.32 |
| OR Quart4 | 2.9 | 7.6 | 2.9 |

**Transmembrane glycoprotein NMB**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 14.6 | 17.1 | nd | nd | 14.6 | 17.1 |
| Average | 16.1 | 20.2 | nd | nd | 16.4 | 20.2 |
| Stdev | 6.02 | 9.68 | nd | nd | 6.22 | 9.68 |
| p(t-test) |  | 0.11 | nd | nd |  | 0.15 |
| Min | 8.02 | 10.5 | nd | nd | 8.02 | 10.5 |
| Max | 35.8 | 41.2 | nd | nd | 35.8 | 41.2 |
| n (Samp) | 42 | 9 | nd | nd | 36 | 9 |
| n (Patient) | 42 | 9 | nd | nd | 36 | 9 |

|  | At Enrollment | | |
|---|---|---|---|
|  | sCr or UO | sCr only | UO only |
| AUC | 0.64 | nd | 0.63 |
| SE | 0.11 | nd | 0.11 |
| p | 0.19 | nd | 0.24 |
| nCohort 1 | 42 | nd | 36 |

(continued)

| | At Enrollment | | |
|---|---|---|---|
| | sCr or UO | sCr only | UO only |
| nCohort 2 | 9 | nd | 9 |
| Cutoff 1 | 14.7 | nd | 14.7 |
| Sens 1 | 78% | nd | 78% |
| Spec 1 | 52% | nd | 53% |
| Cutoff 2 | 10.9 | nd | 10.9 |
| Sens 2 | 89% | nd | 89% |
| Spec 2 | 17% | nd | 14% |
| Cutoff 3 | 10.3 | nd | 10.3 |
| Sens 3 | 100% | nd | 100% |
| Spec 3 | 14% | nd | 11% |
| Cutoff 4 | 18.3 | nd | 18.9 |
| Sens 4 | 44% | nd | 44% |
| Spec 4 | 71% | nd | 72% |
| Cutoff 5 | 20.4 | nd | 20.4 |
| Sens 5 | 33% | nd | 33% |
| Spec 5 | 81% | nd | 81% |
| Cutoff 6 | 24.8 | nd | 25.2 |
| Sens 6 | 22% | nd | 22% |
| Spec 6 | 90% | nd | 92% |
| OR Quart 2 | 0.42 | nd | 0.45 |
| p Value | 0.50 | nd | 0.54 |
| 95% CI of | 0.033 | nd | 0.035 |
| OR Quart2 | 5.3 | nd | 5.8 |
| OR Quart 3 | 1.5 | nd | 1.7 |
| p Value | 0.69 | nd | 0.61 |
| 95% CI of | 0.20 | nd | 0.22 |
| OR Quart3 | 11 | nd | 13 |
| OR Quart 4 | 1.5 | nd | 1.5 |
| p Value | 0.69 | nd | 0.69 |
| 95% CI of | 0.20 | nd | 0.20 |
| OR Quart4 | 11 | nd | 11 |

**Cadherin-3**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 18.3 | 18.6 | nd | nd | 18.3 | 18.6 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Average | 22.1 | 33.7 | nd | nd | 21.9 | 33.7 |
| Stdev | 14.8 | 39.5 | nd | nd | 15.1 | 39.5 |
| p(t-test) |  | 0.14 | nd | nd |  | 0.15 |
| Min | 1.18 | 6.53 | nd | nd | 1.18 | 6.53 |
| Max | 78.5 | 135 | nd | nd | 78.5 | 135 |
| n (Samp) | 42 | 9 | nd | nd | 36 | 9 |
| n (Patient) | 42 | 9 | nd | nd | 36 | 9 |

|  | At Enrollment | | |
|---|---|---|---|
|  | sCr or UO | sCr only | UO only |
| AUC | 0.57 | nd | 0.58 |
| SE | 0.11 | nd | 0.11 |
| p | 0.51 | nd | 0.47 |
| nCohort 1 | 42 | nd | 36 |
| nCohort 2 | 9 | nd | 9 |
| Cutoff 1 | 14.6 | nd | 14.6 |
| Sens 1 | 78% | nd | 78% |
| Spec 1 | 40% | nd | 42% |
| Cutoff 2 | 13.0 | nd | 13.0 |
| Sens 2 | 89% | nd | 89% |
| Spec 2 | 36% | nd | 36% |
| Cutoff 3 | 6.13 | nd | 6.13 |
| Sens 3 | 100% | nd | 100% |
| Spec 3 | 5% | nd | 6% |
| Cutoff 4 | 26.5 | nd | 26.1 |
| Sens 4 | 33% | nd | 33% |
| Spec 4 | 71% | nd | 72% |
| Cutoff 5 | 31.6 | nd | 31.6 |
| Sens 5 | 33% | nd | 33% |
| Spec 5 | 81% | nd | 81% |
| Cutoff 6 | 44.7 | nd | 44.7 |
| Sens 6 | 11% | nd | 11% |
| Spec 6 | 90% | nd | 92% |
| OR Quart 2 | 3.3 | nd | 3.8 |
| p Value | 0.33 | nd | 0.29 |
| 95% CI of | 0.29 | nd | 0.32 |

(continued)

|  | At Enrollment | | |
|---|---|---|---|
|  | sCr or UO | sCr only | UO only |
| OR Quart2 | 37 | nd | 43 |
| OR Quart 3 | 2.0 | nd | 2.2 |
| p Value | 0.59 | nd | 0.54 |
| 95% CI of | 0.16 | nd | 0.17 |
| OR Quart3 | 25 | nd | 29 |
| OR Quart 4 | 3.3 | nd | 3.3 |
| p Value | 0.33 | nd | 0.33 |
| 95% CI of | 0.29 | nd | 0.29 |
| OR Quart4 | 37 | nd | 38 |

**Cathepsin S**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 7.48 | 7.91 | nd | nd | 7.71 | 7.91 |
| Average | 7.89 | 8.13 | nd | nd | 8.07 | 8.13 |
| Stdev | 2.39 | 1.77 | nd | nd | 2.47 | 1.77 |
| p(t-test) |  | 0.78 | nd | nd |  | 0.95 |
| Min | 4.35 | 6.46 | nd | nd | 4.35 | 6.46 |
| Max | 15.5 | 11.2 | nd | nd | 15.5 | 11.2 |
| n (Samp) | 42 | 9 | nd | nd | 36 | 9 |
| n (Patient) | 42 | 9 | nd | nd | 36 | 9 |

|  | At Enrollment | | |
|---|---|---|---|
|  | sCr or UO | sCr only | UO only |
| AUC | 0.56 | nd | 0.52 |
| SE | 0.11 | nd | 0.11 |
| p | 0.58 | nd | 0.82 |
| nCohort 1 | 42 | nd | 36 |
| nCohort 2 | 9 | nd | 9 |
| Cutoff 1 | 6.57 | nd | 6.57 |
| Sens 1 | 78% | nd | 78% |
| Spec 1 | 36% | nd | 31% |
| Cutoff 2 | 6.56 | nd | 6.51 |
| Sens 2 | 89% | nd | 89% |
| Spec 2 | 36% | nd | 31% |
| Cutoff 3 | 6.41 | nd | 6.41 |

(continued)

|  | At Enrollment | | |
|---|---|---|---|
|  | sCr or UO | sCr only | UO only |
| Sens 3 | 100% | nd | 100% |
| Spec 3 | 31% | nd | 28% |
| Cutoff 4 | 8.56 | nd | 8.80 |
| Sens 4 | 22% | nd | 22% |
| Spec 4 | 71% | nd | 72% |
| Cutoff 5 | 9.21 | nd | 9.32 |
| Sens 5 | 22% | nd | 22% |
| Spec 5 | 81% | nd | 81% |
| Cutoff 6 | 11.7 | nd | 12.1 |
| Sens 6 | 0% | nd | 0% |
| Spec 6 | 90% | nd | 92% |
| OR Quart 2 | >5.3 | nd | 3.8 |
| p Value | <0.16 | nd | 0.29 |
| 95% CI of | >0.51 | nd | 0.32 |
| OR Quart2 | na | nd | 43 |
| OR Quart 3 | >3.6 | nd | 3.8 |
| p Value | <0.30 | nd | 0.29 |
| 95% CI of | >0.32 | nd | 0.32 |
| OR Quart3 | na | nd | 43 |
| OR Quart 4 | >2.2 | nd | 2.0 |
| p Value | <0.55 | nd | 0.59 |
| 95% CI of | >0.17 | nd | 0.16 |
| OR Quart4 | na | nd | 26 |

**Urokinase-type plasminogen activator**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.0630 | 0.0662 | nd | nd | 0.0618 | 0.0662 |
| Average | 0.0740 | 0.120 | nd | nd | 0.0730 | 0.120 |
| Stdev | 0.0455 | 0.129 | nd | nd | 0.0441 | 0.129 |
| p(t-test) |  | 0.067 | nd | nd |  | 0.073 |
| Min | 0.0240 | 0.0248 | nd | nd | 0.0292 | 0.0248 |
| Max | 0.268 | 0.422 | nd | nd | 0.268 | 0.422 |
| n (Samp) | 42 | 9 | nd | nd | 36 | 9 |
| n (Patient) | 42 | 9 | nd | nd | 36 | 9 |

|  | At Enrollment | | |
| --- | --- | --- | --- |
|  | sCr or UO | sCr only | UO only |
| AUC | 0.56 | nd | 0.56 |
| SE | 0.11 | nd | 0.11 |
| p | 0.56 | nd | 0.56 |
| nCohort 1 | 42 | nd | 36 |
| nCohort 2 | 9 | nd | 9 |
| Cutoff 1 | 0.0502 | nd | 0.0502 |
| Sens 1 | 78% | nd | 78% |
| Spec 1 | 33% | nd | 33% |
| Cutoff 2 | 0.0447 | nd | 0.0440 |
| Sens 2 | 89% | nd | 89% |
| Spec 2 | 21% | nd | 19% |
| Cutoff 3 | 0.0240 | nd | 0 |
| Sens 3 | 100% | nd | 100% |
| Spec 3 | 2% | nd | 0% |
| Cutoff 4 | 0.0785 | nd | 0.0785 |
| Sens 4 | 44% | nd | 44% |
| Spec 4 | 71% | nd | 72% |
| Cutoff 5 | 0.0926 | nd | 0.0926 |
| Sens 5 | 22% | nd | 22% |
| Spec 5 | 81% | nd | 81% |
| Cutoff 6 | 0.115 | nd | 0.115 |
| Sens 6 | 22% | nd | 22% |
| Spec 6 | 90% | nd | 92% |
| OR Quart 2 | 0.91 | nd | 0.45 |
| p Value | 0.93 | nd | 0.54 |
| 95% CI of | 0.11 | nd | 0.035 |
| OR Quart2 | 7.7 | nd | 5.8 |
| OR Quart 3 | 0.91 | nd | 1.7 |
| p Value | 0.93 | nd | 0.61 |
| 95% CI of | 0.11 | nd | 0.22 |
| OR Quart3 | 7.7 | nd | 13 |
| OR Quart 4 | 1.5 | nd | 1.5 |
| p Value | 0.69 | nd | 0.69 |
| 95% CI of | 0.20 | nd | 0.20 |
| OR Quart4 | 11 | nd | 11 |

**C-C motif chemokine 23**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1.88 | 3.53 | nd | nd | 2.13 | 3.53 |
| Average | 2.19 | 3.86 | nd | nd | 2.39 | 3.86 |
| Stdev | 1.91 | 2.66 | nd | nd | 1.98 | 2.66 |
| p(t-test) | | 0.030 | nd | nd | | 0.069 |
| Min | 0.00626 | 1.17 | nd | nd | 0.00626 | 1.17 |
| Max | 10.3 | 10.3 | nd | nd | 10.3 | 10.3 |
| n (Samp) | 43 | 9 | nd | nd | 36 | 9 |
| n (Patient) | 43 | 9 | nd | nd | 36 | 9 |

| | At Enrollment | | |
|---|---|---|---|
| | sCr or UO | sCr only | UO only |
| AUC | 0.76 | nd | 0.73 |
| SE | 0.099 | nd | 0.10 |
| p | 0.0086 | nd | 0.022 |
| nCohort 1 | 43 | nd | 36 |
| nCohort 2 | 9 | nd | 9 |
| Cutoff 1 | 2.83 | nd | 2.83 |
| Sens 1 | 78% | nd | 78% |
| Spec 1 | 72% | nd | 69% |
| Cutoff 2 | 1.45 | nd | 1.45 |
| Sens 2 | 89% | nd | 89% |
| Spec 2 | 44% | nd | 42% |
| Cutoff 3 | 1.16 | nd | 1.16 |
| Sens 3 | 100% | nd | 100% |
| Spec 3 | 35% | nd | 31% |
| Cutoff 4 | 2.83 | nd | 2.94 |
| Sens 4 | 78% | nd | 67% |
| Spec 4 | 72% | nd | 72% |
| Cutoff 5 | 3.32 | nd | 3.35 |
| Sens 5 | 56% | nd | 56% |
| Spec 5 | 81% | nd | 81% |
| Cutoff 6 | 4.07 | nd | 4.53 |
| Sens 6 | 22% | nd | 22% |
| Spec 6 | 91% | nd | 92% |
| OR Quart 2 | >2.4 | nd | >2.4 |
| p Value | <0.51 | nd | <0.49 |

(continued)

|  | At Enrollment | | |
|---|---|---|---|
|  | sCr or UO | sCr only | UO only |
| 95% CI of | >0.19 | nd | >0.19 |
| OR Quart2 | na | nd | na |
| OR Quart 3 | >2.4 | nd | >2.4 |
| p Value | <0.51 | nd | <0.49 |
| 95% CI of | >0.19 | nd | >0.19 |
| OR Quart3 | na | nd | na |
| OR Quart 4 | >8.1 | nd | >7.9 |
| p Value | <0.077 | nd | <0.085 |
| 95% CI of | >0.80 | nd | >0.75 |
| OR Quart4 | na | nd | na |

**Insulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.65 | 2.32 | 3.15 | 5.67 | 3.74 | 2.20 |
| Average | 7.94 | 6.26 | 7.36 | 12.8 | 8.95 | 5.00 |
| Stdev | 17.9 | 11.7 | 16.8 | 18.4 | 20.1 | 9.04 |
| p(t-test) |  | 0.59 |  | 0.34 |  | 0.29 |
| Min | 0.00859 | 0.00859 | 0.00859 | 0.00859 | 0.00859 | 0.00859 |
| Max | 162 | 53.1 | 162 | 53.1 | 162 | 39.2 |
| n (Samp) | 139 | 36 | 165 | 9 | 110 | 31 |
| n (Patient) | 139 | 36 | 165 | 9 | 110 | 31 |

|  | At Enrollment | | |
|---|---|---|---|
|  | sCr or UO | sCr only | UO only |
| AUC | 0.39 | 0.58 | 0.37 |
| SE | 0.055 | 0.10 | 0.059 |
| p | 0.048 | 0.45 | 0.023 |
| nCohort 1 | 139 | 165 | 110 |
| nCohort 2 | 36 | 9 | 31 |
| Cutoff 1 | 0.703 | 2.44 | 0.777 |
| Sens 1 | 72% | 78% | 71% |
| Spec 1 | 8% | 42% | 9% |
| Cutoff 2 | 0.565 | 0.593 | 0.565 |
| Sens 2 | 81% | 89% | 81% |
| Spec 2 | 6% | 9% | 6% |

(continued)

| | At Enrollment | | |
|---|---|---|---|
| | sCr or UO | sCr only | UO only |
| 6%Cutoff 3 | 0 | 0 | 0 |
| Sens 3 | 100% | 100% | 100ù |
| Spec 3 | 0% | 0% | 0% |
| Cutoff 4 | 6.24 | 5.66 | 6.72 |
| Sens 4 | 17% | 56% | 13% |
| Spec 4 | 71% | 70% | 70% |
| Cutoff 5 | 9.33 | 8.24 | 106 |
| Sens 5 | 17% | 33% | 13% |
| Spec 5 | 81% | 80% | 80% |
| Cutoff 6 | 15.5 | 14.9 | 15.5 |
| Sens 6 | 8% | 22% | 6% |
| Spec 6 | 91% | 90% | 91% |
| OR Quart 2 | 1.0 | 0.98 | 2.0 |
| p Value | 1.0 | 0.98 | 0.31 |
| 95% CI of | 0.30 | 0.13 | 0.53 |
| OR Quart2 | 3.4 | 7.3 | 7.6 |
| OR Quart 3 | 2.1 | 1.0 | 2.8 |
| p Value | 1.18 | 1.0 | 0.12 |
| 95% CI of | 0.70 | 0.13 | 0.76 |
| OR Quart3 | 6.3 | 7.4 | 10 |
| OR Quart 4 | 2.7 | 1.5 | 3.7 |
| p Value | 0.067 | 0.67 | 0.043 |
| 95% CI of | 0.93 | 0.24 | 1.0 |
| OR Quart4 | 8.1 | 9.5 | 13 |

The examples provided herein are representative of preferred embodiments, and are exemplary.

## Claims

1. A method for evaluating renal status in a subject, comprising:

   performing one or more assays configured to detect one or more biomarkers, the one or more biomarkers comprising C-C motif chemokine 23, on a body fluid sample obtained from the subject to provide an assay result; and
   correlating the assay result(s) to the renal status of the subject.

2. A method according to claim 1, wherein the one or more biomarkers further comprise one or more of soluble Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, soluble Cadherin-3, Complement C5, Platelet factor 4, Platelet basic protein, and Stromelysin-2.

3. A method according to claim 1 or 2, wherein said correlation step comprises correlating the assay result(s) to one or more of risk stratification, diagnosis, staging, prognosis, classifying and monitoring of the renal status of the subject.

4. A method according to claim 1 or 2, wherein said correlating step comprises assigning a likelihood of one or more future changes in renal status to the subject based on the assay result(s).

5. A method according to claim 4, wherein said one or more future changes in renal status comprise

   (a) one or more of a future injury to renal function, future reduced renal function, future improvement in renal function, future acute renal failure (ARF) and future acute kidney injury (AKI); or
   (b) a clinical outcome related to a renal injury suffered by the subject, or wherein the likelihood of one or more future changes in renal status is that an event of interest is more or less likely to occur within 30 days of the time at which the body fluid sample has been obtained from the subject,

   wherein the likelihood of one or more future changes in renal status is preferably that an event of interest is more or less likely to occur within a period selected from the group consisting of 21 days, 14 days, 7 days, 5 days, 96 hours, 72 hours, 48 hours, 36 hours, 24 hours, and 12 hours.

6. A method according to one of claims 1-5, wherein said assay results comprise a measured concentration of C-C motif chemokine 23, and at least 1, 2, 3, or 4 of:

  a measured concentration of soluble Transmembrane glycoprotein NMB,
  a measured concentration of Brain-derived neurotrophic factor,
  a measured concentration of Cathepsgin S,
  a measured concentration of Transforming growth factor beta-2,
  a measured concentration of pro-Transforming growth factor beta-2,
  a measured concentration of Urokinase-type plasminogen activator,
  a measured concentration of Angiopoietin-2,
  a measured concentration of Matrilysin,
  a measured concentration of Carcinoembryonic antigen-related cell adhesion molecule 1,
  a measured concentration of Creatine kinase MB,
  a measured concentration of Insulin,
  a measured concentration of Immunoglobulin M,
  a measured concentration of Immunoglobulin E,
  a measured concentration of Macrophage migration inhibitory factor,
  a measured concentration of Galectin-3,
  a measured concentration of Transforming growth factor beta-3,
  a measured concentration of pro-Transforming growth factor beta-3,
  a measured concentration of Heparan sulfate,
  a measured concentration of soluble Cadherin-3,
  a measured concentration of Complement C5,
  a measured concentration of Complement C5a,
  a measured concentration of Platelet factor 4,
  a measured concentration of Platelet basic protein, and
  a measured concentration of and Stromelysin-2.

7. A method according to one of claims 1-6, wherein a plurality of assay results are combined using a function that converts the plurality of assay results into a single composite result.

8. A method according to one of claims 1-6, wherein the subject is selected for evaluation of renal status based on the pre-existence in the subject of one or more known risk factors for prerenal, intrinsic renal, or postrenal ARF, or wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin, or
wherein said correlating step comprises assigning a diagnosis of the occurrence or nonoccurrence of one or more of an injury to renal function, reduced renal function, or ARF to the subject based on the assay result(s), or
wherein said correlating step comprises assessing whether or not renal function is improving or worsening in a subject who has suffered from an injury to renal function, reduced renal function, or ARF based on the assay result(s), or
wherein said method is a method of diagnosing the occurrence or nonoccurrence of an injury to renal function in said subject, or,
wherein said method is a method of diagnosing the occurrence or nonoccurrence of reduced renal function in said subject, or
wherein said method is a method of diagnosing the occurrence or nonoccurrence of acute renal failure in said subject or
wherein said method is a method of diagnosing the occurrence or nonoccurrence of a need for renal replacement therapy in said subject, or
wherein said method is a method of diagnosing the occurrence or nonoccurrence of a need for renal transplantation in said subject, or
wherein said method is a method of assigning a risk of the future occurrence or nonoccurrence of an injury to renal function in said subject, or

wherein said method is a method of assigning a risk of the future occurrence or nonoccurrence of reduced renal function in said subject, or

wherein said method is a method of assigning a risk of the future occurrence or nonoccurrence of acute renal failure in said subject or

wherein said method is a method of assigning a risk of the future occurrence or nonoccurrence of a need for renal replacement therapy in said subject. or

wherein said method is a method of assigning a risk of the future occurrence or nonoccurrence of a need for renal transplantation in said subject, or

wherein said one or more future changes in renal status comprise one or more of a future injury to renal function, future reduced renal function, future improvement in renal function, and future acute renal failure (ARF) within 72 hours of the time at which the body fluid sample has been obtained, or

wherein said one or more future changes in renal status comprise one or more of a future injury to renal function, future reduced renal function, future improvement in renal function, and future acute renal failure (ARF) within 48 hours of the time at which the body fluid sample has been obtained, or

wherein said one or more future changes in renal status comprise one or more of a future injury to renal function, future reduced renal function, future improvement in renal function, and future acute renal failure (ARF) within 24 hours of the time at which the body fluid sample has been obtained.

9. A method according to one of claims 1-6, wherein the subject is in RIFLE stage 0 or R, wherein the method preferably comprises

a) wherein the subject is in RIFLE stage 0, and said correlating step comprises assigning a likelihood that the subject will reach RIFLE stage R, I or F within 72 hours, more preferably

a.1) wherein said correlating step comprises assigning a likelihood that the subject will reach RIFLE stage I or F within 72 hours, most preferably wherein said correlating step comprises assigning a likelihood that the subject will reach RIFLE stage I or F within 48 hours, or said correlating step comprises assigning a likelihood that the subject will reach RIFLE stage I or F within 24 hours, or

a.2) wherein said correlating step comprises assigning a likelihood that the subject will reach RIFLE stage F within 72 hours, most preferably wherein said correlating step comprises assigning a likelihood that the subject will reach RIFLE stage F within 48 hours or said correlating step comprises assigning a likelihood that the subject will reach RIFLE stage F within 24 hours, or

a.3) wherein said correlating step comprises assigning a likelihood that the subject will reach RIFLE stage R, I or F within 48 hours, or

a.4) wherein said correlating step comprises assigning a likelihood that the subject will reach RIFLE stage R, I or F within 24 hours, or

b) wherein the subject is in RIFLE stage 0 or R, and said correlating step comprises assigning a likelihood that the subject will reach RIFLE stage I or F within 72 hours, more preferably

b.1) wherein said correlating step comprises assigning a likelihood that the subject will reach RIFLE stage F within 72 hours, most preferably wherein said correlating step comprises assigning a likelihood that the subject will reach RIFLE stage F within 48 hours or said correlating step comprises assigning a likelihood that the subject will reach RIFLE stage F within 24 hours, or

b.2) wherein said correlating step comprises assigning a likelihood that the subject will reach RIFLE stage I or F within 48 hours, or

b.3) wherein said correlating step comprises assigning a likelihood that the subject will reach RIFLE stage I or F within 24 hours, or

c) wherein the subject is in RIFLE stage R, and said correlating step comprises assigning a likelihood that the subject will reach RIFLE stage I or F within 72 hours, more preferably

c.1) wherein said correlating step comprises assigning a likelihood that the subject will reach RIFLE stage F within 72 hours, most preferably wherein said correlating step comprises assigning a likelihood that the subject will reach RIFLE stage F within 48 hours or said correlating step comprises assigning a likelihood that the subject will reach RIFLE stage F within 24 hours, or

c.2) wherein said correlating step comprises assigning a likelihood that the subject will reach RIFLE stage I or F within 48 hours, or

c.3) wherein said correlating step comprises assigning a likelihood that the subject will reach RIFLE stage I or F within 24 hours,

or

a method according to one of claims 1-5, wherein the subject is in RIFLE stage 0, R, or I, and said correlating step comprises assigning a likelihood that the subject will reach RIFLE stage F within 72 hours, wherein the method preferably comprises

a) wherein the subject is in RIFLE stage I, and said correlating step comprises assigning a likelihood that the subject will reach RIFLE stage F within 72 hours, more preferably

a.1) wherein said correlating step comprises assigning a likelihood that the subject will reach RIFLE stage F within 48 hours, or

a.2) wherein said correlating step comprises assigning a likelihood that the subject will reach RIFLE stage F within 24 hours, or

b) wherein said correlating step comprises assigning a likelihood that the subject will reach RIFLE stage F within 48 hours, or

c) wherein said correlating step comprises assigning a likelihood that the subject will reach RIFLE stage F within 24 hours.

10. A method according to one of claims 1-6, wherein the subject is not in acute renal failure, or
wherein the subject has not experienced a 1.5-fold or greater increase in serum creatinine over a baseline value determined prior to the time at which the body fluid sample has been obtained, or
wherein the subject has a urine output of at least 0.5 ml/kg/hr over the 6 hours preceding the time at which the body fluid sample has been obtained, or
wherein the subject has not experienced an increase of 0.3 mg/dL or greater in serum creatinine over a baseline value determined prior to the time at which the body fluid sample has been obtained, or
wherein the subject (i) has not experienced a 1.5-fold or greater increase in serum creatinine over a baseline value determined prior to the time at which the body fluid sample has been obtained, (ii) has a urine output of at least 0.5 ml/kg/hr over the 6 hours preceding the time at which the body fluid sample has been obtained, and (iii) has not experienced an increase of 0.3 mg/dL or greater in serum creatinine over a baseline value determined prior to the time at which the body fluid sample has been obtained, or
wherein the subject has not experienced a 1.5-fold or greater increase in serum creatinine over a baseline value determined prior to the time at which the body fluid sample has been obtained, or
wherein the subject has a urine output of at least 0.5 ml/kg/hr over the 6 hours preceding the time at which the body fluid sample has been obtained, or
wherein the subject (i) has not experienced a 1.5-fold or greater increase in serum creatinine over a baseline value determined prior to the time at which the body fluid sample has been obtained, (ii) has a urine output of at least 0.5 ml/kg/hr over the 12 hours preceding the time at which the body fluid sample has been obtained, and (iii) has not experienced an increase of 0.3 mg/dL or greater in serum creatinine over a baseline value determined prior to the time at which the body fluid sample has been obtained, or
wherein said correlating step comprises assigning one or more of: a likelihood that within 72 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine,
wherein said correlating step preferably comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine, or
wherein said correlating step preferably comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine,
wherein said correlating step preferably comprises assigning a likelihood that within 72 hours the subject will experience a 1.5-fold or greater increase in serum creatinine, or wherein said correlating step preferably comprises assigning a likelihood that within 72 hours the subject will have a urine output of less than 0.5 ml/kg/hr over a 6 hour period,
or
wherein said correlating step preferably comprises assigning a likelihood that within 72 hours the subject will experience an increase of 0.3 mg/dL or greater in serum creatinine

wherein said correlating step preferably comprises assigning a likelihood that within 48 hours the subject will experience a 1.5-fold or greater increase in serum creatinine,

wherein said correlating step preferably comprises assigning a likelihood that within 48 hours the subject will have a urine output of less than 0.5 ml/kg/hr over a 6 hour period, or

wherein said correlating step preferably comprises assigning a likelihood that within 48 hours the subject will experience an increase of 0.3 mg/dL or greater in serum creatinine, or

wherein said correlating step preferably comprises assigning a likelihood that within 24 hours the subject will experience a 1.5-fold or greater increase in serum creatinine, or

wherein said correlating step preferably comprises assigning a likelihood that within 24 hours the subject will have a urine output of less than 0.5 ml/kg/hr over a 6 hour period,

wherein said correlating step preferably comprises assigning a likelihood that within 24 hours the subject will experience an increase of 0.3 mg/dL or greater in serum creatinine.

**11.** A method according to one of claims 1-6, wherein the subject has not experienced a 2-fold or greater increase in serum creatinine over a baseline value determined prior to the time at which the body fluid sample has been obtained, or

wherein the subject has a urine output of at least 0.5 ml/kg/hr over the 12 hours preceding the time at which the body fluid sample has been obtained, or

wherein the subject (i) has not experienced a 2-fold or greater increase in serum creatinine over a baseline value determined prior to the time at which the body fluid sample has been obtained, (ii) has a urine output of at least 0.5 ml/kg/hr over the 2 hours preceding the time at which the body fluid sample has been obtained, and (iii) has not experienced an increase of 0.3 mg/dL or greater in serum creatinine over a baseline value determined prior to the time at which the body fluid sample has been obtained, or

wherein the subject has not experienced a 3-fold or greater increase in serum creatinine over a baseline value determined prior to the time at which the body fluid sample has been obtained, or

wherein the subject has a urine output of at least 0.3 ml/kg/hr over the 24 hours preceding the time at which the body fluid sample has been obtained, or anuria over the 12 hours preceding the time at which the body fluid sample has been obtained, or

wherein the subject (i) has not experienced a 3-fold or greater increase in serum creatinine over a baseline value determined prior to the time at which the body fluid sample has been obtained, (ii) has a urine output of at least 0.3 ml/kg/hr over the 24 hours preceding the time at which the body fluid sample has been obtained, or anuria over the 12 hours preceding the time at which the body fluid sample has been obtained, and (iii) has not experienced an increase of 0.3 mg/dL or greater in serum creatinine over a baseline value determined prior to the time at which the body fluid sample has been obtained, or

wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine,

wherein said correlating step preferably comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine,

wherein said correlating step preferably comprises assigning one or more of: a likelihood that within 24 hours the subject will (i) experience a 2-fold or greater increase in serum creatinine, or (ii) have a urine output of less than 0.5 ml/kg/hr over a 6 hour period, or

wherein said correlating step preferably comprises assigning a likelihood that within 72 hours the subject will experience a 2-fold or greater increase in serum creatinine,

wherein said correlating step preferably comprises assigning a likelihood that within 72 hours the subject will have a urine output of less than 0.5 ml/kg/hr over a 6 hour period, wherein said correlating step preferably comprises assigning a likelihood that within 48 hours the subject will experience a 2-fold or greater increase in serum creatinine,

wherein said correlating step preferably comprises assigning a likelihood that within 48 hours the subject will have a urine output of less than 0.5 ml/kg/hr over a 6 hour period,

or

wherein said correlating step preferably comprises assigning a likelihood that within 24 hours the subject will experience a 2-fold or greater increase in serum creatinine, or

wherein said correlating step preferably comprises assigning a likelihood that within 24 hours the subject will have a urine output of less than 0.5 ml/kg/hr over a 6 hour period.

**12.** A method according to one of claims 1-6, wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period,.

wherein said correlating step preferably comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period, or

wherein said correlating step preferably comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period, or

wherein said correlating step preferably comprises assigning a likelihood that within 72 hours the subject will experience a 3-fold or greater increase in serum creatinine, or

wherein said correlating step preferably comprises assigning a likelihood that within 72 hours the subject will have a urine output of less than 0.3 ml/kg/hr over a 24 hour period or anuria over a 12 hour period, or

wherein said correlating step preferably comprises assigning a likelihood that within 48 hours the subject will experience a 3-fold or greater increase in serum creatinine, or

wherein said correlating step preferably comprises assigning a likelihood that within 48 hours the subject will have a urine output of less than 0.3 ml/kg/hr over a 24 hour period or anuria over a 12 hour period, or

wherein said correlating step preferably comprises assigning a likelihood that within 24 hours the subject will experience a 3-fold or greater increase in serum creatinine, or

wherein said correlating step preferably comprises assigning a likelihood that within 24 hours the subject will have a urine output of less than 0.3 ml/kg/hr over a 24 hour period or anuria over a 12 hour period.

13. A method according to one of claims 1-12, wherein the body fluid sample is a urine sample, or

wherein said method comprises performing assays that detect C-C motif chemokine 23 and one, two or more of soluble Transmembrane glycoprotein NMB, Brain-derived neurotrophic factor, Cathepsin S, Transforming growth factor beta-2, Urokinase-type plasminogen activator, Angiopoietin-2, Matrilysin, and Carcinoembryonic antigen-related cell adhesion molecule 1.

14. Use of C-C motif chemokine 23, and optionally one or more biomarkers selected from the group consisting of soluble Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, soluble Cadherin-3, Complement C5, Platelet factor 4, Platelet basic protein, and Stromelysin-2, for the *in vitro* evaluation of renal injury, or use of C-C motif chemokine 23, and optionally one or more biomarkers selected from the group consisting of C-C motif chemokine 23, soluble Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, soluble Cadherin-3, Complement C5, Platelet factor 4, Platelet basic protein, and Stromelysin-2, for the in vitro evaluation of acute renal injury.

15. The use of claim 14, being performed with a kit, the kit comprising:

reagents for performing one or more assays configured to detect C-C motif chemokine 23, and optionally one or more kidney injury markers selected from the group consisting of soluble Transmembrane glycoprotein NMB, Brain-derived neurotrophic factor, Cathepsin S, Transforming growth factor beta-2, Urokinase-type plasminogen activator, Angiopoietin-2, Matrilysin, Carcinoembryonic antigen-related cell adhesion molecule 1, Creatine kinase MB, Insulin, Immunoglobulin M, Immunoglobulin E, Macrophage migration inhibitory factor, Galectin-3, Transforming growth factor beta-3, Heparan sulfate, soluble Cadherin-3, Complement C5, Platelet factor 4, Platelet basic protein, and Stromelysin-2, wherein said reagents preferably comprise one or more binding reagents, each of which specifically binds one of said of kidney injury markers, or

wherein a plurality of binding reagents are preferably contained in a single assay device, or

wherein at least one of said assays is preferably configured as a sandwich binding assay, or

wherein at least one of said assays is preferably configured as a competitive binding assay, or

wherein said one or more assays preferably comprise assays that detect C-C motif chemokine 23, and optionally one, two or three, or more of soluble Transmembrane glycoprotein NMB, Brain-derived neurotrophic factor, Cathepsin S, Transforming growth factor beta-2, Urokinase-type plasminogen activator, Angiopoietin-2, Matrilysin, or Carcinoembryonic antigen-related cell adhesion molecule 1.

**Patentansprüche**

1.  Ein Verfahren zur Evaluierung des Nierenstatus eines Subjektes, umfassend:

    Durchführen einer oder mehrerer Untersuchung(en), die dafür konfiguriert sind, einen oder mehrere Biomarker in einer Körperflüssigkeitsprobe, die von dem Subjekt erhalten wurde, nachzuweisen, um ein Untersuchungsergebnis bereitzustellen, wobei der eine oder die mehreren Biomarker C-C- Motif Chemokin 23 umfasst; und Korrelieren des/der Untersuchungsergebnisse(s) mit dem Nierenstatus des Subjektes.

2.  Ein Verfahren nach Anspruch 1, wobei der eine oder die mehreren Biomarker ferner einen oder mehrere aus löslichem Transmembranglykoprotein NMB, vom Gehirn stammenden neutrophen Faktor, Cathepsin S, transformierenden Wachstumsfaktor beta-2, Urokinase-Typ-Plasminogen-Aktivator, Angiopoietin-2, Matrilysin, carcinoembryonalem Antigen-verwandtes Zelladhäsionsmolekül 1, Kreatinkinase MB, Insulin, Immunglobulin M, Immunglobulin E, Makrophagen-Migrationsinhibierenden Faktor, Galektin-3, transformierenden Wachstumsfaktor beta-3, Heparansulfat, löslichem Cadherin-3, Komplementkomponente C5, Plättchenfaktor 4, basischem Plättchenprotein und Stromelysin-2 umfassen.

3.  Ein Verfahren nach Anspruch 1 oder 2, wobei der Korrelierungsschritt das Korrelieren des/der Untersuchungsergebnis(se) mit einem oder mehreren aus Risikostratifizierung, Diagnose, Einstufen, Prognose, Klassifizieren und Überwachen des Nierenstatus des Subjektes umfasst.

4.  Ein Verfahren nach Anspruch 1 oder 2, wobei der Korrelierungsschritt, basierend auf dem/den Untersuchungsergebnis(sen), das Zuweisen einer Wahrscheinlichkeit einer oder mehrerer zukünftiger Veränderungen im Nierenstatus zu dem Subjekt umfasst.

5.  Ein Verfahren nach Anspruch 4, wobei die eine oder die mehreren zukünftige(n) Veränderungen im Nierenstatus umfassen:

    (a) eine oder mehrere aus einer zukünftigen Verletzung der Nierenfunktion, einer zukünftigen reduzierten Nierenfunktion, einer zukünftigen Verbesserung der Nierenfunktion, eines zukünftigen akuten Nierenversagens (ARF) und einer zukünftigen akuten Nierenverletzung (AKI); oder
    (b) ein klinisches Ergebnis im Zusammenhang mit einer Nierenverletzung, die das Subjekt erlitten hat, oder wobei die Wahrscheinlichkeit von einer oder mehreren zukünftigen Veränderungen im Nierenstatus darin besteht, dass ein Ereignis von Interesse mehr oder weniger wahrscheinlich innerhalb von 30 Tagen nach dem Zeitpunkt auftritt, an dem die Körperflüssigkeitsprobe von dem Subjekt erhalten wurde,

    wobei die Wahrscheinlichkeit einer oder mehrerer zukünftiger Veränderungen im Nierenstatus vorzugsweise darin besteht, dass ein Ereignis von Interesse mehr oder weniger wahrscheinlich innerhalb eines Zeitraumes auftritt, der aus der Gruppe ausgewählt wird, die aus 21, Tagen, 14 Tagen, 7 Tagen, 5 Tagen, 96 Stunden, 72 Stunden, 48 Stunden, 36 Stunden, 24 Stunden und 12 Stunden besteht.

6.  Ein Verfahren nach einem der Ansprüche 1-5, wobei die Untersuchungsergebnisse eine gemessene Konzentration von C-C Motif Chemokin 23 umfassen und mindestens 1, 2, 3 oder 4 aus:

    einer gemessenen Konzentration von löslichem Transmembranglykoprotein NMB,
    einer gemessenen Konzentration von aus dem Gehirn stammenden neutrophen Faktor,
    einer gemessenen Konzentration von Cathepsgin S,
    einer gemessenen Konzentration von transformierendem Wachstumsfaktor beta-2,
    einer gemessenen Konzentration von pro-transformierendem Wachstumsfaktors beta-2,
    einer gemessenen Konzentration von Urokinase-Typ-Plasminogen-Aktivator,
    einer gemessenen Konzentration von Angiopoietin-2,
    einer gemessenen Konzentration von Matrilysin,
    einer gemessenen Konzentration von carcinoembryonalem Antigen-verwandten Zelladhäsionsmolekül 1,
    einer gemessenen Konzentration von Kreatinkinase MB,
    einer gemessenen Konzentration von Insulin,
    einer gemessenen Konzentration von Immunglobulin M,
    einer gemessenen Konzentration von Immunglobulin E,
    einer gemessenen Konzentration von Makrophagen-Migrationsinhibierenden Faktor,

einer gemessenen Konzentration von Galektin-3,
einer gemessenen Konzentration von transformierendem Wachstumsfaktors beta-3,
einer gemessenen Konzentration von pro-transformierendem Wachstumsfaktors beta-3,
einer gemessenen Konzentration von Heparansulfat,
einer gemessenen Konzentration von löslichem Cadherin-3,
einer gemessenen Konzentration von Komplementkomponente C5,
einer gemessenen Konzentration von Komplementkomponente C5a,
einer gemessenen Konzentration von Plättchenfaktor 4,
einer gemessenen Konzentration von basischem Plättchenprotein und
einer gemessenen Konzentration von Stromelysin-2.

7. Ein Verfahren nach einem der Ansprüche 1-6, wobei eine Vielzahl von Untersuchungsergebnissen unter Verwendung einer Funktion kombiniert wird, die die Vielzahl von Untersuchungsergebnissen in ein einziges zusammengesetztes Ergebnis umwandelt.

8. Ein Verfahren nach einem der Ansprüche 1-6, wobei das Subjekt für die Evaluierung des Nierenstatus basierend auf dem vorherigen Bestehen eines oder mehrerer bekannter Risikofaktoren für prärenales, immanent renales oder postrenales ARF in dem Subjekt ausgewählt wird, oder
wobei das Subjekt zur Evaluierung des Nierenstatus, basierend auf einer vorhandenen Diagnose von einem oder mehreren aus kongestiver Herzinsuffizienz, Präeklampsie, Eklampsie, Diabetes mellitus, Hypertonie, koronarer Herzkrankheit, Proteinurie, Niereninsuffizienz, glomerulärer Filtration unterhalb des normalen Bereichs, Zirrhose, Serumkreatinin oberhalb des normalen Bereichs, Sepsis, Verletzung der Nierenfunktion, reduzierter Nierenfunktion oder ARF, oder basierend auf einem laufenden oder vorangegangenen größeren gefäßchirurgischen Eingriffs, Koronararterien-Bypassoperation oder einer anderen Herzoperation, oder basierend auf einer Exposition gegenüber NSAIDs, Cyclosporinen, Tacrolimus, Aminoglykosiden, Foscarnet, Ethylenglykol, Hämoglobin, Myoglobin, Ifosfamid, Schwermetallen, Methotrexat, röntgendichten Kontrastmitteln oder Streptozotocin ausgewählt wird, oder
wobei der Korrelierungsschritt, basierend auf dem/den Untersuchungsergebnis(sen), das Zuweisen einer Diagnose des Auftretens oder Nichtauftretens von einem oder mehreren aus einer Verletzung der Nierenfunktion, reduzierter Nierenfunktion oder ARF zu dem Subjekt umfasst, oder
wobei der Korrelierungsschritt, basierend auf dem/den Untersuchungsergebnis(sen), das Beurteilen umfasst, ob sich die Nierenfunktion bei einem Subjekt, das eine Verletzung der Nierenfunktion, eine reduzierte Nierenfunktion oder ARF erlitten hat, verbessert oder verschlechtert, oder
wobei das Verfahren ein Verfahren zum Diagnostizieren des Auftretens oder Nichtauftretens einer Verletzung der Nierenfunktion bei dem Subjekt ist, oder
wobei das Verfahren ein Verfahren zum Diagnostizieren des Auftretens oder Nichtauftretens von reduzierter Nierenfunktion bei dem Subjekt ist, oder
wobei das Verfahren ein Verfahren zum Diagnostizieren des Auftretens oder Nichtauftretens von akutem Nierenversagen bei dem Subjekt ist, oder
wobei das Verfahren ein Verfahren zum Diagnostizieren des Auftretens oder Nichtauftretens eines Bedarfs für eine Nierenersatztherapie bei dem Subjekt ist, oder
wobei das Verfahren ein Verfahren zum Diagnostizieren des Auftretens oder Nichtauftretens eines Bedarfs für eine Nierentransplantation bei dem Subjekt ist, oder
wobei das Verfahren ein Verfahren des Zuweisens eines Risikos für das zukünftige Auftreten oder Nichtauftreten einer Verletzung der Nierenfunktion bei dem Subjekt ist, oder
wobei das Verfahren ein Verfahren des Zuweisens eines Risikos für das zukünftige Auftreten oder Nichtauftreten einer reduzierten Nierenfunktion bei dem Subjekt ist, oder wobei das Verfahren ein Verfahren des Zuweisens eines Risikos für das zukünftige Auftreten oder Nichtauftreten von akutem Nierenversagen bei dem Subjekt ist, oder
wobei das Verfahren ein Verfahren des Zuweisens eines Risikos für das zukünftige Auftreten oder Nichtauftreten eines Bedarfs einer Nierenersatztherapie bei dem Subjekt ist, oder
wobei das Verfahren ein Verfahren des Zuweisens eines Risikos für das zukünftige Auftreten oder Nichtauftreten eines Bedarfs einer Nierentransplantation bei dem Subjekt ist, oder
wobei die eine oder mehreren zukünftigen Veränderungen im Nierenstatus einen oder mehrere aus einer zukünftigen Verletzung der Nierenfunktion, einer zukünftigen reduzierten Nierenfunktion, einer zukünftigen Verbesserung der Nierenfunktion und zukünftiges akutes Nierenversagen (ARF) innerhalb von 72 Stunden nach dem Zeitpunkt, an dem die Körperflüssigkeitsprobe von dem Subjekt erhalten wurde, umfasst, oder
wobei die eine oder mehreren zukünftigen Veränderungen im Nierenstatus eine oder mehrere aus einer zukünftigen Verletzung der Nierenfunktion, einer zukünftigen reduzierten Nierenfunktion, einer zukünftigen Verbesserung der Nierenfunktion und zukünftiges akutes Nierenversagen (ARF) innerhalb von 48 Stunden nach dem Zeitpunkt, an dem die Körperflüssigkeitsprobe von dem Subjekt erhalten wurde, umfasst, oder

wobei die eine oder mehreren zukünftigen Veränderungen im Nierenstatus eine oder mehrere aus einer zukünftigen Verletzung der Nierenfunktion, einer zukünftigen reduzierten Nierenfunktion, einer zukünftigen Verbesserung der Nierenfunktion und zukünftiges akutes Nierenversagen (ARF) innerhalb von 24 Stunden nach dem Zeitpunkt, an dem die Körperflüssigkeitsprobe von dem Subjekt erhalten wurde, umfasst.

**9.** Ein Verfahren nach einem der Ansprüche 1-6, wobei das Subjekt in RIFLE - Stadium 0 oder R ist, wobei das Verfahren vorzugsweise umfasst:

a) wobei das Subjekt in RIFLE-Stadium 0 ist und der Korrelierungsschritt das Zuweisen einer Wahrscheinlichkeit umfasst, dass das Subjekt RIFLE-Stadium R, I oder F innerhalb von 72 Stunden erreichen wird, weiter bevorzugt

a.1) wobei der Korrelierungsschritt das Zuweisen einer Wahrscheinlichkeit umfasst, dass das Subjekt RIFLE-Stadium I oder F innerhalb von 72 Stunden erreichen wird, am meisten bevorzugt, wobei der Korrelierungsschritt das Zuweisen einer Wahrscheinlichkeit umfasst, dass das Subjekt RIFLE-Stadium I oder F innerhalb von 48 Stunden erreichen wird, oder der Korrelierungsschritt das Zuweisen einer Wahrscheinlichkeit umfasst, dass das Subjekt RIFLE-Stadium I oder F innerhalb von 24 Stunden erreichen wird, oder

a.2) wobei der Korrelierungsschritt das Zuweisen einer Wahrscheinlichkeit umfasst, dass das Subjekt RIFLE-Stadium F innerhalb von 72 Stunden erreichen wird, am meisten bevorzugt, wobei der Korrelierungsschritt das Zuweisen einer Wahrscheinlichkeit umfasst, dass das Subjekt RIFLE-Stadium F innerhalb von 48 Stunden erreichen wird, oder der Korrelierungsschritt das Zuweisen einer Wahrscheinlichkeit umfasst, dass das Subjekt RIFLE-Stadium F innerhalb von 24 Stunden erreichen wird, oder

a.3) wobei der Korrelierungsschritt das Zuweisen einer Wahrscheinlichkeit umfasst, dass das Subjekt RIFLE-Stadium R, I oder F innerhalb von 48 Stunden erreichen wird, oder

a.4) wobei der Korrelierungsschritt das Zuweisen einer Wahrscheinlichkeit umfasst, dass das Subjekt RIFLE-Stadium R, I oder F innerhalb von 24 Stunden erreichen wird, oder

b) wobei das Subjekt in RIFLE-Stadium 0 oder R ist und der Korrelierungsschritt das Zuweisen einer Wahrscheinlichkeit umfasst, dass das Subjekt RIFLE-Stadium I oder F innerhalb von 72 Stunden erreichen wird, weiter bevorzugt

b.1) wobei der Korrelierungsschritt das Zuweisen einer Wahrscheinlichkeit umfasst, dass das Subjekt RIFLE-Stadium F innerhalb von 72 Stunden erreichen wird, am meisten bevorzugt, wobei der Korrelierungsschritt das Zuweisen einer Wahrscheinlichkeit umfasst, dass das Subjekt RIFLE-Stadium F innerhalb von 48 Stunden erreichen wird, oder der Korrelierungsschritt das Zuweisen einer Wahrscheinlichkeit umfasst, dass das Subjekt RIFLE-Stadium F innerhalb von 24 Stunden erreichen wird, oder

b.2) wobei der Korrelierungsschritt das Zuweisen einer Wahrscheinlichkeit umfasst, dass das Subjekt RIFLE-Stadium I oder F innerhalb von 48 Stunden erreichen wird, oder

b.3) wobei der Korrelierungsschritt das Zuweisen einer Wahrscheinlichkeit umfasst, dass das Subjekt RIFLE-Stadium I oder F innerhalb von 24 Stunden erreichen wird, oder

c) wobei das Subjekt in RIFLE-Stadium R ist und der Korrelierungsschritt das Zuweisen einer Wahrscheinlichkeit umfasst, dass das Subjekt RIFLE-Stadium I oder F innerhalb von 72 Stunden erreichen wird, weiter bevorzugt,

c.1) wobei der Korrelierungsschritt das Zuweisen einer Wahrscheinlichkeit umfasst, dass das Subjekt RIFLE-Stadium F innerhalb von 72 Stunden erreichen wird, am meisten bevorzugt, wobei der Korrelierungsschritt das Zuweisen einer Wahrscheinlichkeit umfasst, dass das Subjekt RIFLE-Stadium F innerhalb von 48 Stunden erreichen wird, oder der Korrelierungsschritt das Zuweisen einer Wahrscheinlichkeit umfasst, dass das Subjekt RIFLE-Stadium F innerhalb von 24 Stunden erreichen wird, oder

c.2) wobei der Korrelierungsschritt das Zuweisen einer Wahrscheinlichkeit umfasst, dass das Subjekt RIFLE-Stadium I oder F innerhalb von 48 Stunden erreichen wird, oder

c.3) wobei der Korrelierungsschritt das Zuweisen einer Wahrscheinlichkeit umfasst, dass das Subjekt RIFLE-Stadium I oder F innerhalb von 24 Stunden erreichen wird,

oder

ein Verfahren nach einem der Ansprüche 1-5, wobei das Subjekt in RIFLE-Stadium 0, R oder I ist und der Korrelierungsschritt das Zuweisen einer Wahrscheinlichkeit umfasst, dass das Subjekt RIFLE-Stadium F innerhalb von 72 Stunden erreichen wird, wobei das Verfahren vorzugsweise umfasst

a) wobei das Subjekt in RIFLE-Stadium I ist und der Korrelierungsschritt das Zuweisen einer Wahrscheinlichkeit umfasst, dass das Subjekt RIFLE-Stadium F innerhalb von 72 Stunden erreichen wird, weiter bevorzugt

a.1) wobei der Korrelierungsschritt das Zuweisen einer Wahrscheinlichkeit umfasst, dass das Subjekt RIFLE-Stadium F innerhalb von 48 Stunden erreichen wird, oder

a.2) wobei der Korrelierungsschritt das Zuweisen einer Wahrscheinlichkeit umfasst, dass das Subjekt RIFLE-Stadium F innerhalb von 24 Stunden erreichen wird, oder

b) wobei der Korrelierungsschritt das Zuweisen einer Wahrscheinlichkeit umfasst, dass das Subjekt RIFLE-Stadium F innerhalb von 48 Stunden erreichen wird, oder

c) wobei der Korrelierungsschritt das Zuweisen einer Wahrscheinlichkeit umfasst, dass das Subjekt RIFLE-Stadium F innerhalb von 24 Stunden erreichen wird.

10. Ein Verfahren nach einem der Ansprüche 1-6, wobei das Subjekt kein akutes Nierenversagen hat, oder wobei das Subjekt keine 1,5-fache oder größere Zunahme im Serumkreatinin über einen Basiswert, der vor dem Zeitpunkt, an dem die Körperflüssigkeitsprobe erhalten wurde, bestimmt wurde, erfahren hat, oder wobei das Subjekt eine Urinausscheidung von mindestens 0,5 ml/kg/Std. über 6 Stunden vor dem Zeitpunkt hat, an dem die Körperflüssigkeitsprobe erhalten wurde, oder wobei das Subjekt keine Zunahme von 0,3 mg/dL oder mehr im Serumkreatinin über einen Basiswert, der vor dem Zeitpunkt, an dem die Körperflüssigkeitsprobe erhalten wurde, bestimmt wurde, erfahren hat, oder wobei das Subjekt (i) keine 1,5-fache oder größere Zunahme im Serumkreatinin über einen Basiswert, der vor dem Zeitpunkt, an dem die Körperflüssigkeitsprobe erhalten wurde, bestimmt wurde, erfahren hat, (ii) eine Urinausscheidung von mindestens 0,5 ml/kg/Std. über 6 Stunden vor dem Zeitpunkt hat, an dem die Körperflüssigkeitsprobe erhalten wurde, und (iii) keine Zunahme von 0,3 mg/dL oder mehr im Serumkreatinin über einen Basiswert, der vor dem Zeitpunkt, an dem die Körperflüssigkeitsprobe erhalten wurde, bestimmt wurde, erfahren hat, oder wobei das Subjekt keine 1,5-fache oder größere Zunahme im Serumkreatinin über einen Basiswert, der vor dem Zeitpunkt, an dem die Körperflüssigkeitsprobe erhalten wurde, bestimmt wurde, erfahren hat, oder wobei das Subjekt eine Urinausscheidung von mindestens 0,5 ml/kg/Std. über 6 Stunden vor dem Zeitpunkt hat, an dem die Körperflüssigkeitsprobe erhalten wurde, oder wobei das Subjekt (i) keine 1,5-fache oder größere Zunahme im Serumkreatinin über einen Basiswert, der vor dem Zeitpunkt, an dem die Körperflüssigkeitsprobe erhalten wurde, bestimmt wurde, erfahren hat, (ii) eine Urinausscheidung von mindestens 0,5 ml/kg/Std. über 12 Stunden vor dem Zeitpunkt hat, an dem die Körperflüssigkeitsprobe erhalten wurde, und (iii) keine Zunahme im Serumkreatinin von 0,3 mg/dL oder mehr über einen Basiswert, der vor dem Zeitpunkt, an dem die Körperflüssigkeitsprobe erhalten wurde, bestimmt wurde, erfahren hat, oder wobei der Korrelierungsschritt das Zuweisen von einem oder mehreren aus: einer Wahrscheinlichkeit, dass das Subjekt innerhalb von 72 Stunden (i) eine 1,5-fache oder größere Zunahme im Serumkreatinin erfahren wird (ii) eine Urinausscheidung von weniger als 0,5 ml/kg/Std. über einen Zeitraum von 6 Stunden haben wird, oder (iii) eine Zunahme im Serumkreatinin von 0,3 mg/dL oder mehr erfahren wird, umfasst, wobei der Korrelierungsschritt vorzugsweise das Zuweisen von einem oder mehreren aus: einer Wahrscheinlichkeit, dass das Subjekt innerhalb von 48 Stunden (i) eine 1,5-fache oder größere Zunahme im Serumkreatinin erfahren wird (ii) eine Urinausscheidung von weniger als 0,5 ml/kg/Std. über einen Zeitraum von 6 Stunden haben wird, oder (iii) eine Zunahme im Serumkreatinin von 0,3 mg/dL oder mehr erfahren wird, umfasst, oder wobei der Korrelierungsschritt vorzugsweise das Zuweisen von einem oder mehreren aus: einer Wahrscheinlichkeit, dass das Subjekt innerhalb von 24 Stunden (i) eine 1,5-fache oder größere Zunahme im Serumkreatinin erfahren wird (ii) eine Urinausscheidung von weniger als 0,5 ml/kg/Std. über einen Zeitraum von 6 Stunden haben wird, oder (iii) eine Zunahme im Serumkreatinin von 0,3 mg/dL oder mehr erfahren wird, umfasst, wobei der Korrelierungsschritt vorzugsweise das Zuweisen einer Wahrscheinlichkeit, dass das Subjekt innerhalb von 72 Stunden eine 1,5-fache oder größere Zunahme im Serumkreatinin erfahren wird, umfasst, oder wobei der Korrelierungsschritt vorzugsweise das Zuweisen einer Wahrscheinlichkeit umfasst, dass das Subjekt innerhalb von 72 Stunden eine Urinausscheidung von weniger als 0,5 ml/kg/Std. über einen Zeitraum von 6 Stunden haben wird,
oder
wobei der Korrelierungsschritt vorzugsweise das Zuweisen einer Wahrscheinlichkeit umfasst, dass das Subjekt innerhalb von 72 Stunden eine Zunahme von 0,3 mg/dL oder mehr im Serumkreatinin erfahren wird, wobei der Korrelierungsschritt vorzugsweise das Zuweisen einer Wahrscheinlichkeit umfasst, dass das Subjekt innerhalb von 48 Stunden eine 1,5-fache oder größere Zunahme im Serumkreatinin erfahren wird, wobei der Korrelierungsschritt vorzugsweise das Zuweisen einer Wahrscheinlichkeit umfasst, dass das Subjekt innerhalb von 48 Stunden eine Urinausscheidung von weniger als 0,5 ml/kg/Std. über einen Zeitraum von 6 Stunden haben wird,

oder

wobei der Korrelierungsschritt vorzugsweise das Zuweisen einer Wahrscheinlichkeit umfasst, dass das Subjekt innerhalb von 48 Stunden eine Zunahme von 0,3 mg/dL oder mehr im Serumkreatinin erfahren wird,

oder

wobei der Korrelierungsschritt vorzugsweise das Zuweisen einer Wahrscheinlichkeit umfasst, dass das Subjekt innerhalb von 24 Stunden eine 1,5-fache oder größere Zunahme im Serumkreatinin erfahren wird, oder

wobei der Korrelierungsschritt vorzugsweise das Zuweisen einer Wahrscheinlichkeit umfasst, dass das Subjekt innerhalb von 24 Stunden eine Urinausscheidung von weniger als 0,5 ml/kg/Std. über einen Zeitraum von 6 Stunden haben wird,

wobei der Korrelierungsschritt vorzugsweise das Zuweisen einer Wahrscheinlichkeit umfasst, dass das Subjekt innerhalb von 24 Stunden eine Zunahme von 0,3 mg/dL oder mehr im Serumkreatinin erfahren wird.

11. Ein Verfahren nach einem der Ansprüche 1-6, wobei das Subjekt keine 2-fache oder größere Zunahme im Serumkreatinin über einen Basiswert erfahren hat, der vor dem Zeitpunkt bestimmt wurde, an dem die Körperflüssigkeitsprobe erhalten wurde, oder

wobei das Subjekt eine Urinausscheidung von mindestens 0,5 ml/kg/Std. über 12 Stunden vor dem Zeitpunkt hat, an dem die Körperflüssigkeitsprobe erhalten wurde, oder

wobei das Subjekt (i) keine 2-fache oder größere Zunahme im Serumkreatinin über einen Basiswert erfahren hat, der vor dem Zeitpunkt bestimmt wurde, an dem die Körperflüssigkeitsprobe erhalten wurde, (ii) eine Urinausscheidung von mindestens 0,5 ml/kg/Std. über 2 Stunden vor dem Zeitpunkt hat, an dem die Körperflüssigkeitsprobe erhalten wurde, und (iii) keine Zunahme von 0,3 mg/dL oder mehr im Serumkreatinin über einen Basiswert erfahren hat, der vor dem Zeitpunkt bestimmt wurde, an dem die Körperflüssigkeitsprobe erhalten wurde, oder

wobei das Subjekt keine 3-fache oder größere Zunahme im Serumkreatinin über einen Basiswert erfahren hat, der vor dem Zeitpunkt bestimmt wurde, an dem die Körperflüssigkeitsprobe erhalten wurde, oder

wobei das Subjekt eine Urinausscheidung von mindestens 0,3 ml/kg/Std. über 24 Stunden vor dem Zeitpunkt hat, an dem die Körperflüssigkeitsprobe erhalten wurde, oder Anurie über 12 Stunden vor dem Zeitpunkt, an dem die Körperflüssigkeitsprobe erhalten wurde, oder

wobei das Subjekt (i) keine 3-fache oder größere Zunahme im Serumkreatinin über einen Basiswert erfahren hat, der vor dem Zeitpunkt bestimmt wurde, an dem die Körperflüssigkeitsprobe erhalten wurde, (ii) eine Urinausscheidung von mindestens 0,3 ml/kg/Std. über 24 Stunden vor dem Zeitpunkt hat, an dem die Körperflüssigkeitsprobe erhalten wurde, oder Anurie über die 12 Stunden vor dem Zeitpunkt, an dem die Körperflüssigkeitsprobe erhalten wurde, und (iii) keine Zunahme im Serumkreatinin von 0,3 mg/dL oder mehr über einen Basiswert erfahren hat, der vor dem Zeitpunkt bestimmt wurde, an dem die Körperflüssigkeitsprobe erhalten wurde, oder

wobei der Korrelierungsschritt das Zuweisen von einem oder mehreren aus: einer Wahrscheinlichkeit, dass das Subjekt innerhalb von 72 Stunden (i) eine 2-fache oder größere Zunahme im Serumkreatinin erfahren wird (ii) eine Urinausscheidung von weniger als 0,5 ml/kg/Std. über einen Zeitraum von 12 Stunden haben wird, oder (iii) eine Zunahme von 0,3 mg/dL oder mehr im Serumkreatinin erfahren wird, umfasst,

wobei der Korrelierungsschritt vorzugsweise das Zuweisen von einem oder mehreren aus: einer Wahrscheinlichkeit, dass das Subjekt innerhalb von 48 Stunden (i) eine 2-fache oder größere Zunahme im Serumkreatinin erfahren wird (ii) eine Urinausscheidung von weniger als 0,5 ml/kg/Std. über einen Zeitraum von 6 Stunden haben wird, oder (iii) eine Zunahme von 0,3 mg/dL oder mehr im Serumkreatinin erfahren wird, umfasst,

wobei der Korrelierungsschritt vorzugsweise das Zuweisen von einem oder mehreren aus: einer Wahrscheinlichkeit, dass das Subjekt innerhalb von 24 Stunden (i) eine 2-fache oder größere Zunahme im Serumkreatinin erfahren wird oder (ii) eine Urinausscheidung von weniger als 0,5 ml/kg/Std. über einen Zeitraum von 6 Stunden haben wird, umfasst, oder

wobei der Korrelierungsschritt vorzugsweise das Zuweisen einer Wahrscheinlichkeit umfasst, dass das Subjekt innerhalb von 72 Stunden eine 2-fache oder größere Zunahme im Serumkreatinin erfahren wird,

wobei der Korrelierungsschritt vorzugsweise das Zuweisen einer Wahrscheinlichkeit umfasst, dass das Subjekt innerhalb von 72 Stunden eine Urinausscheidung von weniger als 0,5 ml/kg/Std. über einen Zeitraum von 6 Stunden haben wird,

wobei der Korrelierungsschritt vorzugsweise das Zuweisen einer Wahrscheinlichkeit umfasst, dass das Subjekt innerhalb von 48 Stunden eine 2-fache oder größere Zunahme im Serumkreatinin erfahren wird,

wobei der Korrelierungsschritt vorzugsweise das Zuweisen einer Wahrscheinlichkeit umfasst, dass das Subjekt innerhalb von 48 Stunden eine Urinausscheidung von weniger als 0,5 ml/kg/Std. über einen Zeitraum von 6 Stunden haben wird,

oder

wobei der Korrelierungsschritt vorzugsweise das Zuweisen einer Wahrscheinlichkeit umfasst, dass das Subjekt innerhalb von 24 Stunden eine 2-fache oder größere Zunahme im Serumkreatinin erfahren wird, oder wobei der Korrelierungsschritt vorzugsweise das Zuweisen einer Wahrscheinlichkeit umfasst, dass das Subjekt innerhalb von 24 Stunden eine Urinausscheidung von weniger als 0,5 ml/kg/Std. über einen 6-Stunden-Zeitraum haben wird.

12. Ein Verfahren nach einem der Ansprüche 1-6, wobei der Korrelierungsschritt das Zuweisen von einem oder mehreren aus: einer Wahrscheinlichkeit, dass das Subjekt innerhalb von 72 Stunden (i) eine 3-fache oder größere Zunahme im Serumkreatinin erfahren wird, oder (ii) eine Urinausscheidung von weniger als 0,3 ml/kg/Std. über einen Zeitraum von 24 Stunden oder Anurie über einen Zeitraum von 12 Stunden haben wird, umfasst,

wobei der Korrelierungsschritt vorzugsweise das Zuweisen von einem oder mehreren aus: einer Wahrscheinlichkeit, dass das Subjekt innerhalb von 48 Stunden (i) eine 3-fache oder größere Zunahme im Serumkreatinin erfahren wird, oder (ii) eine Urinausscheidung von weniger als 0,3 ml/kg/Std. über einen Zeitraum von 24 Stunden oder Anurie über einen Zeitraum von 12 Stunden haben wird, umfasst, oder

wobei der Korrelierungsschritt vorzugsweise das Zuweisen von einem oder mehreren aus: einer Wahrscheinlichkeit, dass das Subjekt innerhalb von 24 Stunden (i) eine 3-fache oder größere Zunahme im Serumkreatinin erfahren wird, oder (ii) eine Urinausscheidung von weniger als 0,3 ml/kg/Std. über einen Zeitraum von 24 Stunden oder Anurie über einen Zeitraum von 12 Stunden haben wird, umfasst, oder

wobei der Korrelierungsschritt vorzugsweise das Zuweisen einer Wahrscheinlichkeit umfasst, dass das Subjekt innerhalb von 72 Stunden eine 3-fache oder größere Zunahme im Serumkreatinin erfahren wird, oder

wobei der Korrelierungsschritt vorzugsweise das Zuweisen einer Wahrscheinlichkeit umfasst, dass das Subjekt innerhalb von 72 Stunden eine Urinausscheidung von weniger als 0,3 ml/kg/Std. über einen Zeitraum von 24 Stunden oder Anurie über einen Zeitraum von 12 Stunden haben wird, oder

wobei der Korrelierungsschritt vorzugsweise das Zuweisen einer Wahrscheinlichkeit umfasst, dass das Subjekt innerhalb von 48 Stunden eine 3-fache oder größere Zunahme im Serumkreatinin erfahren wird, oder

wobei der Korrelierungsschritt vorzugsweise das Zuweisen einer Wahrscheinlichkeit umfasst, dass das Subjekt innerhalb von 48 Stunden eine Urinausscheidung von weniger als 0,3 ml/kg/Std. über einen Zeitraum von 24 Stunden oder Anurie über einen Zeitraum von 12 Stunden haben wird, oder

wobei der Korrelierungsschritt vorzugsweise das Zuweisen einer Wahrscheinlichkeit umfasst, dass das Subjekt innerhalb von 24 Stunden eine 3-fache oder größere Zunahme im Serumkreatinin erfahren wird, oder

wobei der Korrelierungsschritt vorzugsweise das Zuweisen einer Wahrscheinlichkeit umfasst, dass das Subjekt innerhalb von 24 Stunden eine Urinausscheidung von weniger als 0,3 ml/kg/Std. über einen Zeitraum von 24 Stunden oder Anurie über einen Zeitraum von 12 Stunden haben wird.

13. Ein Verfahren nach einem der Ansprüche 1-12, wobei die Körperflüssigkeitsprobe eine Urinprobe ist, oder wobei das Verfahren das Durchführen von Untersuchungen umfasst, die C-C Motif Chemokin 23 und ein, zwei oder mehr aus löslichem Transmembranglykoprotein NMB, aus dem Gehirn stammenden neutrophen Faktor, Cathepsin S, transformierenden Wachstumsfaktor beta-2, Urokinase-Typ-Plasminogen-Aktivator, Angiopoietin-2, Matrilysin und carcinoembryonalem Antigen-verwandten Zelladhäsionsmolekül 1 nachweisen.

14. Verwendung von C-C Motif Chemokin 23 und optional einem oder mehreren Biomarkern, die aus der Gruppe ausgewählt werden, die aus löslichem Transmembranglykoprotein NMB, aus dem Gehirn stammenden neutrophen Faktor, Cathepsin S, transformierendem Wachstumsfaktor beta-2, Urokinase-Typ-Plasminogen-Aktivator, Angiopoietin-2, Matrilysin, carcinoembryonalem Antigen-verwandtem Zelladhäsionsmolekül 1, Kreatinkinase MB, Insulin, Immunglobulin M, Immunglobulin E, Makrophagen-Migrationsinhibierender Faktor, Galektin-3, transformierendem Wachstumsfaktor beta-3, Heparansulfat, löslichem Cadherin-3, Komplementkomponente C5, Plättchenfaktor 4, basischem Plättchenprotein und Stromelysin-2 besteht, zur *in vitro* Evaluierung von Nierenverletzung, oder Verwendung von C-C Motif Chemokin 23 und wahlweise einem oder mehreren Biomarkern, die aus der Gruppe ausgewählt werden, die aus löslichem Transmembranglykoprotein NMB, aus dem Gehirn stammenden neutrophen Faktor, Cathepsin S, transformierendem Wachstumsfaktor beta-2, Urokinase-Typ-Plasminogen-Aktivator, Angiopoietin-2, Matrilysin, carcinoembryonalem Antigen-verwandtem Zelladhäsionsmolekül 1, Kreatinkinase MB, Insulin, Immunglobulin M, Immunglobulin E, Makrophagen-Migrationsinhibierender Faktor, Galektin-3, transformierendem Wachstumsfaktor beta-3, Heparansulfat, löslichem Cadherin-3, Komplementkomponente C5, Plättchenfaktor 4, basischem Plättchenprotein und Stromelysin-2 besteht, zur *in vitro* Evaluierung von akutem Nierenversagen.

15. Die Verwendung nach Anspruch 14, die mit einem Kit durchgeführt wird, wobei das Kit umfasst:

Reagenzien zum Durchführen von einer oder mehreren Untersuchungen, die dafür konfiguriert sind C-C Motif Chemokin 23 zu detektieren, und optional einen oder mehrere Nierenverletzungsmarker, die aus der Gruppe ausgewählt werden, die aus löslichem Transmembranglykoprotein NMB, aus dem Gehirn stammenden neutrophem Faktor, Cathepsin S, transformierendem Wachstumsfaktor beta-2, Urokinase-Typ-PlasminogenAktivator, Angiopoietin-2, Matrilysin, carcinoembryonalem Antigen-verwandtem Zelladhäsionsmolekül 1, Kreatinkinase MB, Insulin, Immunglobulin M, Immunglobulin E, Makrophagen-Migrationsinhibierenden Faktor, Galektin-3, transformierendem Wachstumsfaktor beta-3, Heparansulfat, löslichem Cadherin-3, Komplementkomponente C5, Plättchenfaktor 4, basischem Plättchenprotein und Stromelysin-2 besteht, wobei die Reagenzien vorzugsweise ein oder mehrere Bindungsreagenzien umfassen, die jeweils einen der Nierenverletzungsmarker spezifisch binden, oder wobei eine Vielzahl von Bindungsreagenzien vorzugsweise in einer einzelnen Untersuchungsvorrichtung enthalten sind,

oder

wobei mindestens eine der Untersuchungen vorzugsweise als Sandwich-Bindungsassay konfiguriert ist, oder

wobei mindestens eine der Untersuchungen vorzugsweise als kompetitiver Bindungsassay konfiguriert ist, oder wobei die eine oder die mehreren Untersuchungen vorzugsweise Untersuchungen umfassen, die C-C Motif Chemokin 23, und optional ein, zwei, drei oder mehr aus löslichem Transmembranglykoprotein NMB, aus dem Gehirn stammenden neutrophen Faktor, Cathepsin S, transformierendem Wachstumsfaktor beta-2, Urokinase-Typ-Plasminogen-Aktivator, Angiopoietin-2, Matrilysin oder carcinoembryonalem Antigenverwandten Zelladhäsionsmolekül 1 nachweisen.

## Revendications

1. Procédé d'évaluation de l'état rénal chez un sujet, comprenant:

    la réalisation d'un ou de plusieurs analyse(s) configuré(s) pour détecter un ou plusieurs marqueurs biologiques sur un échantillon de fluide corporel obtenu du sujet pour fournir un résultat d'analyse, l'un ou les plusieurs marqueurs biologiques comprenant C-C motif chimiokine 23; et
    la corrélation du/des résultat(s) d'analyse(s) à l'état rénal du sujet.

2. Procédé selon la revendication 1, l'un ou les plusieurs marqueurs biologiques comprenant en outre un ou plusieurs de la glycoprotéine transmembranaire soluble NMB, du facteur neurotrophique dérivé du cerveau, de la cathépsine S, du facteur de croissance transformant bêta-2, de l'activateur du plasminogène de type urokinase, de l'angiopoïéitine-2, de la matrilysine, de la molécule d'adhésion cellulaire 1 liée à l'antigène carcino-embryonnaire, de la créatine kinase MB, de l'insuline, de l'immunoglobuline M, de l'immunoglobuline E, du facteur inhibiteur de la migration des macrophages, de la galectine-3, du facteur de croissance transformant bêta-3, du sulfate d'héparane, de la cadhérine-3 soluble, du complément C5, du facteur plaquettaire 4, de la protéine basique plaquettaire, et de la stromélysine-2.

3. Procédé selon la revendication 1 ou 2, l'étape de corrélation comprenant la corrélation du (des) résultat(s) d'analyse(s) à un ou plusieurs parmi une stratification des risques, un diagnostic, une stadification, un pronostic, une classification et une surveillance de l'état rénal du sujet.

4. Procédé selon la revendication 1 ou 2, l'étape de corrélation comprenant l'assignation d'une probabilité d'un ou de plusieurs changement(s) futur(s) de l'état rénal chez le sujet en fonction du/des résultat(s) d'analyse(s).

5. Procédé selon la revendication 4, l'un ou les plusieurs changement(s) futur(s) de l'état rénal comprenant:

    (a) un ou plusieurs parmi une lésion future de la fonction rénale, une fonction rénale réduite future, une amélioration future de la fonction rénale, une insuffisance rénale aiguë (IRA) future et une lésion rénale aiguë future (LRA); ou
    (b) un résultat clinique lié à une lésion rénale souffert par le sujet, la probabilité d'un ou de plusieurs changement(s) futur(s) de l'état rénal étant qu'un événement d'intérêt est plus ou moins probable dans les 30 jours du moment auquel l'échantillon de fluide corporel a été obtenu du sujet,

    la probabilité d'un ou de plusieurs changement(s) futur(s) de l'état rénal étant de préférence qu'un événement d'intérêt est plus ou moins probable dans un délai sélectionné du groupe comportant 21 jours, 14 jours, 7 jours, 5 jours, 96 heures, 72 heures, 48 heures, 36 heures, 24 heures et 12 heures.

**6.** Procédé selon l'une quelconque des revendications 1-5, lesdits résultats d'analyse comprenant une concentration mesurée de la C-C motif chimiokine 23, et au moins 1, 2, 3 ou 4 d'une:

concentration mesurée de la glycoprotéine transmembranaire soluble NMB,
concentration mesurée du facteur neurotrophique dérivé du cerveau,
concentration mesurée de la cathépsine S,
concentration mesurée du facteur de croissance transformant bêta-2,
concentration mesurée du facteur de croissance pré-transformant bêta-2,
concentration mesurée de l'activateur du plasminogène de type urokinase,
concentration mesurée de l'angiopoïéitine-2,
concentration mesurée de la matrilysine,
concentration mesurée de la molécule d'adhésion cellulaire 1 liée à l'antigène carcino-embryonnaire,
concentration mesurée de la créatine kinase MB,
concentration mesurée de l'insuline,
concentration mesurée de l'immunoglobuline M,
concentration mesurée de l'immunoglobuline E,
concentration mesurée du facteur inhibiteur de la migration des macrophages,
concentration mesurée de la galectine-3,
concentration mesurée du facteur de croissance transformant bêta-3,
concentration mesurée du sulfate d'héparane,
concentration mesurée de la cadhérine-3 soluble,
concentration mesurée du complément C5,
concentration mesurée du complément C5a,
concentration mesurée du facteur plaquettaire 4,
concentration mesurée de la protéine basique plaquettaire, et
concentration mesurée de la stromélysine-2.

**7.** Procédé selon l'une quelconque des revendications 1-6, une pluralité de résultats d'analyse étant combinée en utilisant une fonction qui convertit la pluralité de résultats d'analyse dans un seul résultat composite.

**8.** Procédé selon l'une quelconque des revendications 1-6, le sujet étant sélectionné pour l'évaluation de l'état rénal en fonction de la préexistence chez le sujet d'un ou de plusieurs facteurs de risque connus pour une IRA pré-rénale, rénale intrinsèque, ou post-rénale, ou
le sujet étant sélectionné pour l'évaluation de l'état rénal en fonction d'un diagnostic existant d'un(e) ou de plusieurs parmi une insuffisance cardiaque congestive, une pré-éclampsie, une éclampsie, un diabète sucré, une hypertension, une coronaropathie, une protéinurie, une insuffisance rénale, une filtration glomérulaire inférieure à la plage normale, une cirrhose, de la créatinine sérique supérieure à la plage normale, une sepsie, une lésion de la fonction rénale, une fonction rénale réduite, ou une IRA, ou en fonction du fait de subir ou d'avoir subi une intervention chirurgicale vasculaire majeure, un pontage aorto-coro-narien, ou une autre intervention cardiaque, ou en fonction de l'exposition à des AINS, à des cyclosporines, au tacrolimus, à des aminogly-cosides, au foscarnet, à de l'éthylèneglycol, à l'hémoglobine, à la myo-globine, à l'ifosfamide, à des métaux lourds, au méthotrexate, à des agents de contraste radio-opaques, ou à la streptozotocine, ou
ladite étape de corrélation comprenant l'assignation d'un diagnostic de l'occurrence ou de non-occurrence d'un ou plusieurs parmi une lésion de la fonction rénale, une fonction rénale réduite ou une IRA chez le sujet en fonction du (des) résultat(s) d'analyse, ou
ladite étape de corrélation comprenant l'évaluation si la fonction rénale s'améliore ou s'aggrave ou non chez un sujet qui a souffert d'une lésion de la fonction rénale, d'une fonction rénale réduite ou d'une IRA en fonction du (des) résultat(s) de l'analyse, ou
ledit procédé étant un procédé de diagnostic de l'occurrence ou de la non-occurrence d'une lésion de la fonction rénale chez ledit sujet, ou
ledit procédé étant un procédé de diagnostic de l'occurrence ou de la non-occurrence de la fonction rénal réduite chez ledit sujet, ou
ledit procédé étant un procédé de diagnostic de l'occurrence ou de la non-occurrence de l'insuffisance rénale aiguë chez ledit sujet, ou
ledit procédé étant un procédé de diagnostic de l'occurrence ou de la non-occurrence d'un besoin de traitement rénal substitutif chez ledit sujet, ou
ledit procédé étant un procédé de diagnostic de l'occurrence ou de la non-occurrence d'un besoin de transplantation rénale chez ledit sujet, ou

ledit procédé étant un procédé de l'assignation d'un risque de l'occurrence ou non-occurrence future d'une lésion de la fonction rénale chez ledit sujet, ou

ledit procédé étant un procédé de l'assignation d'un risque de l'occurrence ou non-occurrence future de la fonction rénale réduite chez ledit sujet, ou

ledit procédé étant un procédé de l'assignation d'un risque de l'occurrence ou non-occurrence future de l'insuffisance rénale aiguë chez ledit sujet, ou

ledit procédé étant un procédé de l'assignation d'un risque de l'occurrence ou non-occurrence future d'un besoin de traitement rénal substitutif chez ledit sujet, ou

ledit procédé étant un procédé de l'assignation d'un risque de l'occurrence ou non-occurrence future d'un besoin de transplantation rénale chez ledit sujet, ou

ledit un ou plusieurs changement(s) futur(s) de l'état rénal comprenant une ou plusieurs d'une lésion future de la fonction rénale, d'une fonction rénale réduite future, d'une amélioration future de la fonction rénale, et d'une insuffisance rénale aiguë (IRA) future dans les 72 heures du moment auquel l'échantillon de fluide corporel est obtenu, ou

ledit un ou plusieurs changement(s) futur(s) de l'état rénal comprenant une ou plusieurs d'une lésion future de la fonction rénale, d'une fonction rénale réduite future, d'une amélioration future de la fonction rénale, et d'une insuffisance rénale aiguë (IRA) future dans les 48 heures du moment auquel l'échantillon de fluide corporel est obtenu, ou

ledit un ou plusieurs changement(s) futur(s) de l'état rénal comprenant une ou plusieurs d'une lésion future de la fonction rénale, d'une fonction rénale réduite future, d'une amélioration future de la fonction rénale, et d'une insuffisance rénale aiguë (IRA) future dans les 24 heures du moment auquel l'échantillon de fluide corporel est obtenu.

9. Procédé selon l'une des revendications 1-6, le sujet étant dans la classe 0 ou R de RIFLE, le procédé comprenant de préférence

a) le sujet étant dans la classe 0 de RIFLE, et ladite étape de corrélation comprenant l'assignation d'une probabilité que le sujet atteindra la classe R, I ou F de RIFLE dans les 72 heures, plus préférablement

a.1) ladite étape de corrélation comprenant l'assignation d'une probabilité que le sujet atteindra la classe I ou F de RIFLE dans les 72 heures, le plus préférablement, ladite étape de corrélation comprenant l'assignation d'une probabilité que le sujet atteindra la classe 1 ou F de RIFLE dans les 48 heures, ou ladite étape de corrélation comprenant l'assignation d'une probabilité que le sujet atteindra la classe I ou F de RIFLE dans les 24 heures, ou

a.2) ladite étape de corrélation comprenant l'assignation d'une probabilité que le sujet atteindra la classe F de RIFLE dans les 72 heures, le plus préférablement ladite étape de corrélation comprenant l'assignation d'une probabilité que le sujet atteindra la classe F de RIFLE dans les 48 heures, ou ladite étape de corrélation comprenant l'assignation d'une probabilité que le sujet atteindra la classe F de RIFLE dans les 24 heures, ou

a.3) ladite étape de corrélation comprenant l'assignation d'une probabilité que le sujet atteindra la classe R, I ou F de RIFLE dans les 48 heures, ou

a.4) ladite étape de corrélation comprenant l'assignation d'une probabilité que le sujet atteindra la classe R, I ou F de RIFLE dans les 24 heures, ou

b) le sujet étant dans la classe 0 ou R de RIFLE, et ladite étape de corrélation comprenant l'assignation d'une probabilité que le sujet atteindra la classe I ou F de RIFLE dans les 72 heures, plus préférablement

b.1) ladite étape de corrélation comprenant l'assignation d'une probabilité que le sujet atteindra la classe F de RIFLE dans les 72 heures, le plus préférablement, ladite étape de corrélation comprenant l'assignation d'une probabilité que le sujet atteindra la classe F de RIFLE dans les 48 heures, ou ladite étape de corrélation comprenant l'assignation d'une probabilité que le sujet atteindra la classe F de RIFLE dans les 24 heures, ou

b.2) ladite étape de corrélation comprenant l'assignation d'une probabilité que le sujet atteindra la classe I ou F de RIFLE dans les 48 heures, ou

b.3) ladite étape de corrélation comprenant l'assignation d'une probabilité que le sujet atteindra la classe I ou F de RIFLE dans les 24 heures, ou

c) le sujet étant dans la classe R de RIFLE, et ladite étape de corrélation comprenant l'assignation d'une probabilité que le sujet atteindra la classe I ou F de RIFLE dans les 72 heures, plus préférablement

c.1) ladite étape de corrélation comprenant l'assignation d'une probabilité que le sujet atteindra la classe F de RIFLE dans les 72 heures, le plus préférablement, ladite étape de corrélation comprenant l'assignation d'une probabilité que le sujet atteindra la classe F de RIFLE dans les 48 heures, ou ladite étape de corrélation comprenant l'assignation d'une probabilité que le sujet atteindra la classe F de RIFLE dans les 24 heures, ou

c.2) ladite étape de corrélation comprenant l'assignation d'une probabilité que le sujet atteindra la classe I ou F de RIFLE dans les 48 heures, ou

c.3) ladite étape de corrélation comprenant l'assignation d'une probabilité que le sujet atteindra la classe I ou F de RIFLE dans les 24 heures,

ou

procédé selon l'une des revendications 1-5, le sujet étant dans la classe 0, R ou I de RIFLE, et ladite étape de corrélation comprenant l'assignation d'une probabilité que le sujet atteindra la classe F de RIFLE dans les 72 heures, le procédé comprenant de préférence

a) le sujet étant dans la classe I de RIFLE, et ladite étape de corrélation comprenant l'assignation d'une probabilité que le sujet atteindra la classe F de RIFLE dans les 72 heures, plus préférablement

a.1) ladite étape de corrélation comprenant l'assignation d'une probabilité que le sujet atteindra la classe F de RIFLE dans les 48 heures, ou

a.2) ladite étape de corrélation comprenant l'assignation d'une probabilité que le sujet atteindra la classe F de RIFLE dans les 24 heures, ou

b) ladite étape de corrélation comprenant l'assignation d'une probabilité que le sujet atteindra la classe F de RIFLE dans les 48 heures, ou

c) ladite étape de corrélation comprenant l'assignation d'une probabilité que le sujet atteindra la classe F de RIFLE dans les 24 heures.

10. Procédé selon l'une des revendications 1-6, le sujet n'étant pas dans une insuffisance rénale aiguë, ou le sujet n'ayant pas connu d'augmentation de 1,5 fois ou plus de la créatinine sérique par rapport à une valeur de référence déterminée avant le moment auquel l'échantillon de fluide corporel est obtenu, ou le sujet ayant une production d'urine d'au moins 0,5 ml/kg/heure sur les 6 heures qui précèdent le moment auquel l'échantillon de fluide corporel est obtenu, ou le sujet n'ayant pas connu d'augmentation de 0,3 mg/dL ou plus de la créatinine sérique par rapport à une valeur de référence déterminée avant le moment auquel l'échantillon de fluide corporel est obtenu, ou le sujet (i) n'ayant pas connu d'augmentation de 1,5 fois ou plus de la créatinine sérique par rapport à une valeur de référence déterminée avant le moment auquel l'échantillon de fluide corporel est obtenu, (ii) ayant une production d'urine d'au moins 0,5 ml/kg/heure sur les 6 heures qui précèdent le moment auquel l'échantillon de fluide corporel est obtenu, et (iii) n'ayant pas connu d'augmentation de 0,3 mg/dL ou plus de la créatinine sérique par rapport à une valeur de référence déterminée avant le moment auquel l'échantillon de fluide corporel est obtenu, ou le sujet n'ayant pas connu d'augmentation de 1,5 fois ou plus de la créatinine sérique par rapport à une valeur de référence déterminée avant le moment auquel l'échantillon de fluide corporel est obtenu, ou le sujet ayant une production d'urine d'au moins 0,5 ml/kg/heure sur les 6 heures qui précèdent le moment auquel l'échantillon de fluide corporel est obtenu, ou le sujet (i) n'ayant pas connu d'augmentation de 1,5 fois ou plus de la créatinine sérique par rapport à une valeur de référence déterminée avant le moment auquel l'échantillon de fluide corporel est obtenu, (ii) ayant une production d'urine d'au moins 0,5 ml/kg/heure sur les 12 heures qui précèdent le moment auquel l'échantillon de fluide corporel est obtenu, et (iii) n'ayant pas connu d'augmentation de 0,3 mg/dL ou plus de la créatinine sérique par rapport à une valeur de référence déterminée avant le moment auquel l'échantillon de fluide corporel est obtenu, ou ladite étape de corrélation comprenant l'assignation d'un ou plusieurs parmi: une probabilité que dans les 72 heures le sujet (i) connaîtra d'augmentation de 1,5 fois ou plus de la créatinine sérique (ii) aura une production d'urine de moins de 0,5 ml/kg/heure sur une période de 6 heures, ou (iii) connaîtra d'augmentation de 0,3 mg/dL ou plus de la créatinine sérique, ladite étape de corrélation comprenant de préférence l'assignation d'un ou plusieurs parmi: une probabilité que dans les 48 heures le sujet (i) connaîtra d'augmentation de 1,5 fois ou plus de la créatinine sérique (ii) aura une production d'urine de moins de 0,5 ml/kg/heure sur une période de 6 heures, ou (iii) connaîtra d'augmentation de 0,3 mg/dL ou plus de la créatinine sérique, ou ladite étape de corrélation comprenant de préférence l'assignation d'un ou plusieurs parmi: une probabilité que dans les 24 heures le sujet (i) connaîtra d'augmentation de 1,5 fois ou plus de la créatinine sérique (ii) aura une production d'urine de moins de 0,5 ml/kg/heure sur une période de 6 heures, ou (iii) connaîtra d'augmentation de 0,3 mg/dL ou plus de la créatinine sérique, ladite étape de corrélation comprenant de préférence l'assignation d'une probabilité que dans les 72 heures le sujet connaîtra d'augmentation de 1,5 fois ou plus de la créatinine sérique, ou

ladite étape de corrélation comprenant de préférence l'assignation d'une probabilité que dans les 72 heures le sujet aura une production d'urine de moins de 0,5 ml/kg/heure sur une période de 6 heures, ou

ladite étape de corrélation comprenant de préférence une probabilité que dans les 72 heures le sujet connaîtra d'augmentation de 0,3 mg/dL ou plus de la créatinine sérique

ladite étape de corrélation comprenant de préférence l'assignation d'une probabilité que dans les 48 heures le sujet connaîtra d'augmentation de 1,5 fois ou plus de la créatinine sérique,

ladite étape de corrélation comprenant de préférence l'assignation d'une probabilité que dans les 48 heures le sujet aura une production d'urine de moins de 0,5 ml/kg/heure sur une période de 6 heures, ou

ladite étape de corrélation comprenant de préférence l'assignation d'une probabilité que dans les 48 heures le sujet connaîtra d'augmentation de 0,3 mg/dL ou plus de la créatinine sérique, ou

ladite étape de corrélation comprenant de préférence l'assignation d'une probabilité que dans les 24 heures le sujet connaîtra d'augmentation de 1,5 fois ou plus de la créatinine sérique, ou

ladite étape de corrélation comprenant de préférence l'assignation d'une probabilité que dans les 24 heures le sujet aura une production d'urine de moins de 0,5 ml/kg/heure sur une période de 6 heures,

ladite étape de corrélation comprenant de préférence l'assignation d'une probabilité que dans les 24 heures le sujet connaîtra d'augmentation de 0,3 mg/dL ou plus de la créatinine sérique.

11. Procédé selon l'une des revendications 1-6, le sujet n'ayant pas connu d'augmentation de 2 fois ou plus de la créatinine sérique par rapport à une valeur de référence déterminée avant le moment auquel l'échantillon de fluide corporel est obtenu, ou

le sujet ayant une production d'urine d'au moins 0,5 ml/kg/heure sur les 12 heures qui précèdent le moment auquel l'échantillon de fluide corporel est obtenu, ou

le sujet (i) n'ayant pas connu d'augmentation de 2 fois ou plus de la créatinine sérique par rapport à une valeur de référence déterminée avant le moment auquel l'échantillon de fluide corporel est obtenu, (ii) ayant une production d'urine d'au moins 0,5 ml/kg/heure sur les 2 heures qui précèdent le moment auquel l'échantillon de fluide corporel est obtenu, et (iii) n'ayant pas connu d'augmentation de 0,3 mg/dL ou plus de la créatinine sérique par rapport à une valeur de référence déterminée avant le moment auquel l'échantillon de fluide corporel est obtenu, ou

le sujet n'ayant pas connu d'augmentation de 3 fois ou plus de la créatinine sérique par rapport à une valeur de référence déterminée avant le moment auquel l'échantillon de fluide corporel est obtenu, ou

le sujet ayant une production d'urine d'au moins 0,3 ml/kg/heure sur les 24 heures qui précèdent le moment auquel l'échantillon de fluide corporel est obtenu, ou de l'anurie sur les 12 heures qui précèdent le moment auquel l'échantillon de fluide corporel est obtenu, ou

le sujet (i) n'ayant pas connu d'augmentation de 3 fois ou plus de la créatinine sérique par rapport à une valeur de référence déterminée avant le moment auquel l'échantillon de fluide corporel est obtenu, (ii) ayant une production d'urine d'au moins 0,3 ml/kg/heure sur les 24 heures qui précèdent le moment auquel l'échantillon de fluide corporel est obtenu, ou de l'anurie sur les 12 heures qui précèdent le moment auquel l'échantillon de fluide corporel est obtenu et (iii) n'ayant pas connu d'augmentation de 0,3 mg/dL ou plus de la créatinine sérique par rapport à une valeur de référence déterminée avant le moment auquel l'échantillon de fluide corporel est obtenu, ou

ladite étape de corrélation comprenant l'assignation d'un ou plusieurs parmi: une probabilité que dans les 72 heures le sujet (i) connaîtra d'augmentation de 2 fois ou plus de la créatinine sérique (ii) aura une production d'urine de moins de 0,5 ml/kg/heure sur une période de 12 heures, ou (iii) connaîtra d'augmentation de 0,3 mg/dL ou plus de la créatinine sérique,

ladite étape de corrélation comprenant de préférence l'assignation d'un ou plusieurs parmi: une probabilité que dans les 48 heures le sujet (i) connaîtra d'augmentation de 2 fois ou plus de la créatinine sérique (ii) aura une production d'urine de moins de 0,5 ml/kg/heure sur une période de 6 heures, ou (iii) connaîtra d'augmentation de 0,3 mg/dL ou plus de la créatinine sérique,

ladite étape de corrélation comprenant de préférence l'assignation d'un ou plusieurs parmi: une probabilité que dans les 24 heures le sujet (i) connaîtra d'augmentation de 2 fois ou plus de la créatinine sérique, ou (ii) aura une production d'urine de moins de 0,5 ml/kg/heure sur une période de 6 heures, ou

ladite étape de corrélation comprenant de préférence l'assignation d'une probabilité que dans les 72 heures le sujet connaîtra d'augmentation de 2 fois ou plus de la créatinine sérique,

ladite étape de corrélation comprenant de préférence l'assignation d'une probabilité que dans les 72 heures le sujet aura une production d'urine de moins de 0,5 ml/kg/heure sur une période de 6 heures,

ladite étape de corrélation comprenant de préférence l'assignation d'une probabilité que dans les 48 heures le sujet connaîtra d'augmentation de 2 fois ou plus de la créatinine sérique,
ladite étape de corrélation comprenant de préférence l'assignation d'une probabilité que dans les 48 heures le sujet aura une production d'urine de moins de 0,5 ml/kg/heure sur une période de 6 heures, ou
ladite étape de corrélation comprenant de préférence l'assignation d'une probabilité que dans les 24 heures le sujet connaîtra d'augmentation de 2 fois ou plus de la créatinine sérique, ou
ladite étape de corrélation comprenant de préférence l'assignation d'une probabilité que dans les 24 heures le sujet aura une production d'urine de moins de 0,5 ml/kg/heure sur une période de 6 heures.

12. Procédé selon l'une des revendications 1-6, ladite étape de corrélation nant l'assignation d'un ou plusieurs parmi:
une probabilité que dans les 72 heures le sujet (i) connaîtra d'augmentation de 3 fois ou plus de la créatinine sérique, ou (ii) aura une production d'urine de moins de 0,3 ml/kg/heure sur une période de 24 heures, ou de l'anurie sur une période de 12 heures,
ladite étape de corrélation comprenant de préférence l'assignation d'un ou plusieurs parmi: une probabilité que dans les 48 heures le sujet (i) connaîtra d'augmentation de 3 fois ou plus de la créatinine sérique, ou (ii) aura une production d'urine de moins de 0,3 ml/kg/heure sur une période de 24 heures, ou de l'anurie sur une période de 12 heures, ou
ladite étape de corrélation comprenant de préférence l'assignation d'un ou plusieurs parmi: une probabilité que dans les 24 heures le sujet (i) connaîtra d'augmentation de 3 fois ou plus de la créatinine sérique, ou (ii) aura une production d'urine de moins de 0,3 ml/kg/heure sur une période de 24 heures, ou de l'anurie sur une période de 12 heures, ou
ladite étape de corrélation comprenant de préférence l'assignation d'une probabilité que dans les 72 heures le sujet connaîtra d'augmentation de 3 fois ou plus de la créatinine sérique, ou
ladite étape de corrélation comprenant de préférence l'assignation d'une probabilité que dans les 72 heures le sujet aura une production d'urine de moins de 0,3 ml/kg/heure sur une période de 24 heures, ou de l'anurie sur une période de 12 heures, ou
ladite étape de corrélation comprenant de préférence l'assignation d'une probabilité que dans les 48 heures le sujet connaîtra d'augmentation de 3 fois ou plus de la créatinine sérique, ou
ladite étape de corrélation comprenant de préférence l'assignation d'une probabilité que dans les 48 heures le sujet aura une production d'urine de moins de 0,3 ml/kg/heure sur une période de 24 heures, ou de l'anurie sur une période de 12 heures, ou
ladite étape de corrélation comprenant de préférence l'assignation d'une probabilité que dans les 24 heures le sujet connaîtra d'augmentation de 3 fois ou plus de la créatinine sérique, ou
ladite étape de corrélation comprenant de préférence l'assignation d'une probabilité que dans les 24 heures le sujet aura une production d'urine de moins de 0,3 ml/kg/heure sur une période de 24 heures, ou de l'anurie sur une période de 12 heures.

13. Procédé selon l'une des revendications 1-12, l'échantillon de fluide corporel étant un échantillon d'urine, ou
le procédé comprenant la réalisation des analyses qui détectent de la C-C motif chimiokine 23, et un, deux ou plusieurs de la glycoprotéine transmembranaire soluble NMB, du facteur neurotrophique dérivé du cerveau, de la cathépsine S, du facteur de croissance transformant bêta-2, de l'activateur du plasminogène de type urokinase, de l'angiopoïéitine-2, de la matrilysine, et de la molécule d'adhésion cellulaire 1 liée à l'antigène carcino-embryonnaire.

14. Utilisation de la C-C motif chimiokine 23, et facultativement un ou plusieurs des marqueurs biologiques sélectionnés du groupe comprenant de la glycoprotéine transmembranaire soluble NMB, du facteur neurotrophique dérivé du cerveau, de la cathépsine S, du facteur de croissance transformant bêta-2, de l'activateur du plasminogène de type urokinase, de l'angiopoïéitine-2, de la matrilysine, de la molécule d'adhésion cellulaire 1 liée à l'antigène carcino-embryonnaire, de la créatine kinase MB, de l'insuline, de l'immunoglobuline M, de l'immunoglobuline E, du facteur inhibiteur de la migration des macrophages, de la galectine-3, du facteur de croissance transformant bêta-3, du sulfate d'héparane, de la cadhérine-3 soluble, du complément C5, du facteur plaquettaire 4, de la protéine basique plaquettaire et de la stromélysine-2, pour l'évaluation *in vitro* de la lésion rénale, ou
l'utilisation de la C-C motif chimiokine 23, et facultativement un ou plusieurs des marqueurs biologiques sélectionnés du groupe comprenant de la C-C motif chimiokine 23, de la glycoprotéine transmembranaire soluble NMB, du facteur neurotrophique dérivé du cerveau, de la cathépsine S, du facteur de croissance transformant bêta-2, de l'activateur du plasminogène de type urokinase, de l'angiopoïéitine-2, de la matrilysine, de la molécule d'adhésion cellulaire 1 liée à l'antigène car-cino-embryonnaire, de la créatine kinase MB, de l'insuline, de l'immu-noglobuline M, de l'immunoglobuline E, du facteur inhibiteur de la migration des macrophages, de la galectine-3, du facteur de croissance transformant bêta-3, du sulfate d'héparane, de la cadhérine-3 soluble, du complément C5, du facteur plaquettaire 4, de la protéine basique plaquettaire, et de la stromélysine-2, pour l'évaluation in vitro de la lésion rénale aiguë.

15. Utilisation selon la revendication 14 étant réalisée avec un kit, le kit comprenant:

des réactifs pour la réalisation d'une ou de plusieurs analyse(s) configurée(s) pour détecter la C-C motif chimiokine 23, et facultativement un ou plusieurs marqueurs de la lésion rénale, sélectionnés du groupe comprenant la glycoprotéine transmembranaire soluble NMB, le facteur neurotrophique dérivé du cerveau, la cathépsine S, le facteur de croissance transformant bêta-2, l'activateur du plasminogène de type urokinase, l'angiopoïéitine-2, la matrilysine, la molécule d'adhésion cellulaire 1 liée à l'antigène carcino-embryonnaire, la créatine kinase MB, l'insuline, l'immunoglobuline M, l'immunoglobuline E, le facteur inhibiteur de la migration des macrophages, la galectine-3, le facteur de croissance transformant bêta-3, le sulfate d'héparane, la cadhérine-3 soluble, le complément C5, le facteur plaquettaire 4, la protéine basique plaquettaire, et de la stromélysine-2, lesdits réactifs comprenant de préférence un ou plusieurs réactifs de liaison dont chacun se lie spécifiquement à un desdit marqueurs de la lésion rénale, ou

une pluralité de réactifs de liaison étant contenue de préférence dans un dispositif d'analyse unique, ou

au moins une desdites analyses étant de préférence configurée en tant qu'analyse de liaison «en sandwich», ou

au moins une desdites analyses étant de préférence configurée en tant qu'analyse de liaison compétitive, ou ladite ou lesdites une ou plusieurs analyse(s) comprenant de préférence des analyses qui détectent de la C-C motif chimiokine 23, et facultativement un, deux ou trois ou plus de la glycoprotéine transmembranaire soluble NMB, du facteur neurotrophique dérivé du cerveau, de la cathépsine S, du facteur de croissance transformant bêta-2, de l'activateur du plasminogène de type urokinase, de l'angiopoïéitine-2, de la matrilysine, ou de la molécule d'adhésion cellulaire 1 liée à l'antigène carcino-embryonnaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61259135 A
- US 61259136 A
- US 61259137 A
- US 61259139 A
- US 61259145 A
- US 61259147 A
- US 61259148 A
- US 61259151 A
- US 61259152 A
- US 61259153 A
- US 61259155 A
- US 61259156 A
- US 61259157 A
- US 61259159 A
- US 61259160 A
- US 61259161 A
- US 61259162 A
- US 61285928 A
- US 61285929 A
- US 61320723 A
- US 61346551 A
- US 61346547 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A
- US 2010055721 W
- US 61346551 B
- US 61346547 B
- US 61320723 B
- US 61285929 B
- US 61285928 B
- US 61259162 B
- US 61259161 B
- US 61259160 B
- US 61259159 B
- US 61259157 B
- US 61259156 B
- US 61259155 B
- US 61259153 B
- US 61259152 B
- US 61259151 B
- US 61259148 B
- US 61259147 B
- US 61259145 B
- US 61259139 B
- US 61259137 B
- US 61259136 B
- US 61259135 B

**Non-patent literature cited in the description**

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- **COCA et al.** Biomarkers for the diagnosis and risk sratification of acute kidney injury: A systematic review. *Kidney International,* 2008, vol. 73, 1008-1016
- **DEVARAJAN.** *Novel biomarkers for the early prediction of acute kidney injury,* 2005

- **BERAHOVICH et al.** Proteolytic activation of alternative CCR1 ligands in inflammation. *The Journal of Immunology,* 2005, vol. 174, 7341-7351
- **NISHIYAMA et al.** Up-Regulation of Galectin-3 in acute renal failure in rat. *American Journal of Pathology,* 2000, vol. 157 (3), 815-823
- **BICIK et al.** Role of transforming growth factor β2 in, and a possible transforming growth factor β2 gene polymorphism as a marker of, renal dysfunction in essential hypertension: a study in turish patients. *Current Therapeutic Research,* 2005, vol. 66 (4), 266-278
- **RABENSTEIN.** *Nat. Prod. Rep.,* 2002, vol. 19, 312-331
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant,* 2008, vol. 23, 1203-1210
- Merck Manual of Diagnosis and Therapy. Merck Research Laboratories, 1999
- **WIJEYSUNDERA et al.** *JAMA,* 2007, vol. 297, 1801-9
- **HANLEY, J. A. ; MCNEIL, B.J.** The meaning and use of the area under a receiver operating characteristic (ROC) curve. *Radiology,* 1982, vol. 143, 29-36

SEQUENCE LISTING

<110> ASTUTE MEDICAL, INC.

<120> METHODS AND COMPOSITIONS FOR DIAGNOSIS AND PROGNOSIS OF RENAL INJURY AND RENAL FAILURE

<130> AST-1240-PC

<140> PCT/US2010/055721
<141> 2010-11-05

<150> 61/346,551

<151> 2010-05-20

<150> 61/346,547
<151> 2010-05-20

<150> 61/320,723
<151> 2010-04-03

<150> 61/285,929
<151> 2009-12-11

<150> 61/285,928
<151> 2009-12-11

<150> 61/259,162
<151> 2009-11-07

<150> 61/259,161
<151> 2009-11-07

<150> 61/259,160
<151> 2009-11-07

<150> 61/259,159
<151> 2009-11-07

<150> 61/259,157
<151> 2009-11-07

<150> 61/259,156
<151> 2009-11-07

<150> 61/259,155
<151> 2009-11-07

<150> 61/259,153
<151> 2009-11-07

<150> 61/259,152
<151> 2009-11-07

<150> 61/259,151
<151> 2009-11-07

<150> 61/259,148
<151> 2009-11-07

<150> 61/259,147
<151> 2009-11-07

<150> 61/259,145
<151> 2009-11-07

<150> 61/259,139
<151> 2009-11-07

<150> 61/259,137
<151> 2009-11-07

<150> 61/259,136
<151> 2009-11-07

<150> 61/259,135
<151> 2009-11-07

<160> 24

<170> PatentIn version 3.5

<210> 1
<211> 120
<212> PRT
<213> Homo sapiens

<400> 1

```
Met Lys Val Ser Val Ala Ala Leu Ser Cys Leu Met Leu Val Thr Ala
1               5               10              15

Leu Gly Ser Gln Ala Arg Val Thr Lys Asp Ala Glu Thr Glu Phe Met
            20              25              30

Met Ser Lys Leu Pro Leu Glu Asn Pro Val Leu Leu Asp Arg Phe His
        35              40              45

Ala Thr Ser Ala Asp Cys Cys Ile Ser Tyr Thr Pro Arg Ser Ile Pro
    50              55              60

Cys Ser Leu Leu Glu Ser Tyr Phe Glu Thr Asn Ser Glu Cys Ser Lys
65              70              75              80

Pro Gly Val Ile Phe Leu Thr Lys Lys Gly Arg Arg Phe Cys Ala Asn
                85              90              95

Pro Ser Asp Lys Gln Val Gln Val Cys Val Arg Met Leu Lys Leu Asp
            100             105             110

Thr Arg Ile Lys Thr Arg Lys Asn
            115             120
```

<210> 2
<211> 572
<212> PRT
<213> Homo sapiens

<400> 2

```
Met Glu Cys Leu Tyr Tyr Phe Leu Gly Phe Leu Leu Leu Ala Ala Arg
1               5               10              15

Leu Pro Leu Asp Ala Ala Lys Arg Phe His Asp Val Leu Gly Asn Glu
            20              25              30

Arg Pro Ser Ala Tyr Met Arg Glu His Asn Gln Leu Asn Gly Trp Ser
        35              40              45

Ser Asp Glu Asn Asp Trp Asn Glu Lys Leu Tyr Pro Val Trp Lys Arg
        50              55              60

Gly Asp Met Arg Trp Lys Asn Ser Trp Lys Gly Gly Arg Val Gln Ala
65              70              75              80

Val Leu Thr Ser Asp Ser Pro Ala Leu Val Gly Ser Asn Ile Thr Phe
            85              90              95

Ala Val Asn Leu Ile Phe Pro Arg Cys Gln Lys Glu Asp Ala Asn Gly
        100             105             110

Asn Ile Val Tyr Glu Lys Asn Cys Arg Asn Glu Ala Gly Leu Ser Ala
        115             120             125

Asp Pro Tyr Val Tyr Asn Trp Thr Ala Trp Ser Glu Asp Ser Asp Gly
    130             135             140

Glu Asn Gly Thr Gly Gln Ser His His Asn Val Phe Pro Asp Gly Lys
145             150             155             160

Pro Phe Pro His His Pro Gly Trp Arg Arg Trp Asn Phe Ile Tyr Val
            165             170             175

Phe His Thr Leu Gly Gln Tyr Phe Gln Lys Leu Gly Arg Cys Ser Val
        180             185             190

Arg Val Ser Val Asn Thr Ala Asn Val Thr Leu Gly Pro Gln Leu Met
        195             200             205

Glu Val Thr Val Tyr Arg Arg His Gly Arg Ala Tyr Val Pro Ile Ala
    210             215             220

Gln Val Lys Asp Val Tyr Val Val Thr Asp Gln Ile Pro Val Phe Val
225             230             235             240

Thr Met Phe Gln Lys Asn Asp Arg Asn Ser Ser Asp Glu Thr Phe Leu
            245             250             255
```

285

Lys Asp Leu Pro Ile Met Phe Asp Val Leu Ile His Asp Pro Ser His
        260                 265                 270

Phe Leu Asn Tyr Ser Thr Ile Asn Tyr Lys Trp Ser Phe Gly Asp Asn
        275                 280                 285

Thr Gly Leu Phe Val Ser Thr Asn His Thr Val Asn His Thr Tyr Val
        290                 295                 300

Leu Asn Gly Thr Phe Ser Leu Asn Leu Thr Val Lys Ala Ala Ala Pro
305                 310                 315                 320

Gly Pro Cys Pro Pro Pro Pro Pro Pro Arg Pro Ser Lys Pro Thr
        325                 330                 335

Pro Ser Leu Ala Thr Thr Leu Lys Ser Tyr Asp Ser Asn Thr Pro Gly
        340                 345                 350

Pro Ala Gly Asp Asn Pro Leu Glu Leu Ser Arg Ile Pro Asp Glu Asn
        355                 360                 365

Cys Gln Ile Asn Arg Tyr Gly His Phe Gln Ala Thr Ile Thr Ile Val
        370                 375                 380

Glu Gly Ile Leu Glu Val Asn Ile Ile Gln Met Thr Asp Val Leu Met
385                 390                 395                 400

Pro Val Pro Trp Pro Glu Ser Ser Leu Ile Asp Phe Val Val Thr Cys
        405                 410                 415

Gln Gly Ser Ile Pro Thr Glu Val Cys Thr Ile Ile Ser Asp Pro Thr
        420                 425                 430

Cys Glu Ile Thr Gln Asn Thr Val Cys Ser Pro Val Asp Val Asp Glu
        435                 440                 445

Met Cys Leu Leu Thr Val Arg Arg Thr Phe Asn Gly Ser Gly Thr Tyr
        450                 455                 460

Cys Val Asn Leu Thr Leu Gly Asp Asp Thr Ser Leu Ala Leu Thr Ser
465                 470                 475                 480

Thr Leu Ile Ser Val Pro Asp Arg Asp Pro Ala Ser Pro Leu Arg Met
        485                 490                 495

Ala Asn Ser Ala Leu Ile Ser Val Gly Cys Leu Ala Ile Phe Val Thr
        500                 505                 510

```
Val Ile Ser Leu Leu Val Tyr Lys Lys His Lys Glu Tyr Asn Pro Ile
        515             520             525

Glu Asn Ser Pro Gly Asn Val Val Arg Ser Lys Gly Leu Ser Val Phe
        530             535             540

Leu Asn Arg Ala Lys Ala Val Phe Phe Pro Gly Asn Gln Glu Lys Asp
545             550             555             560

Pro Leu Leu Lys Asn Gln Glu Phe Lys Gly Val Ser
        565             570
```

<210> 3
<211> 247
<212> PRT
<213> Homo sapiens

<400> 3

```
Met Thr Ile Leu Phe Leu Thr Met Val Ile Ser Tyr Phe Gly Cys Met
1               5               10              15

Lys Ala Ala Pro Met Lys Glu Ala Asn Ile Arg Gly Gln Gly Gly Leu
        20              25              30

Ala Tyr Pro Gly Val Arg Thr His Gly Thr Leu Glu Ser Val Asn Gly
        35              40              45

Pro Lys Ala Gly Ser Arg Gly Leu Thr Ser Leu Ala Asp Thr Phe Glu
        50              55              60

His Val Ile Glu Glu Leu Leu Asp Glu Asp Gln Lys Val Arg Pro Asn
65              70              75              80

Glu Glu Asn Asn Lys Asp Ala Asp Leu Tyr Thr Ser Arg Val Met Leu
                85              90              95

Ser Ser Gln Val Pro Leu Glu Pro Pro Leu Leu Phe Leu Leu Glu Glu
        100             105             110

Tyr Lys Asn Tyr Leu Asp Ala Ala Asn Met Ser Met Arg Val Arg Arg
        115             120             125

His Ser Asp Pro Ala Arg Arg Gly Glu Leu Ser Val Cys Asp Ser Ile
        130             135             140

Ser Glu Trp Val Thr Ala Ala Asp Lys Lys Thr Ala Val Asp Met Ser
145             150             155             160
```

287

```
Gly Gly Thr Val Thr Val Leu Glu Lys Val Pro Val Ser Lys Gly Gln
                165             170             175

Leu Lys Gln Tyr Phe Tyr Glu Thr Lys Cys Asn Pro Met Gly Tyr Thr
            180             185             190

Lys Glu Gly Cys Arg Gly Ile Asp Lys Arg His Trp Asn Ser Gln Cys
            195             200             205

Arg Thr Thr Gln Ser Tyr Val Arg Ala Leu Thr Met Asp Ser Lys Lys
    210             215             220

Arg Ile Gly Trp Arg Phe Ile Arg Ile Asp Thr Ser Cys Val Cys Thr
225             230             235             240

Leu Thr Ile Lys Arg Gly Arg
                245
```

<210> 4
<211> 331
<212> PRT
<213> Homo sapiens

<400> 4

```
Met Lys Arg Leu Val Cys Val Leu Leu Val Cys Ser Ser Ala Val Ala
1               5               10              15

Gln Leu His Lys Asp Pro Thr Leu Asp His His Trp His Leu Trp Lys
            20              25              30

Lys Thr Tyr Gly Lys Gln Tyr Lys Glu Lys Asn Glu Glu Ala Val Arg
            35              40              45

Arg Leu Ile Trp Glu Lys Asn Leu Lys Phe Val Met Leu His Asn Leu
    50              55              60

Glu His Ser Met Gly Met His Ser Tyr Asp Leu Gly Met Asn His Leu
65              70              75              80

Gly Asp Met Thr Ser Glu Glu Val Met Ser Leu Met Ser Ser Leu Arg
            85              90              95

Val Pro Ser Gln Trp Gln Arg Asn Ile Thr Tyr Lys Ser Asn Pro Asn
            100             105             110

Arg Ile Leu Pro Asp Ser Val Asp Trp Arg Glu Lys Gly Cys Val Thr
            115             120             125
```

```
Glu Val Lys Tyr Gln Gly Ser Cys Gly Ala Cys Trp Ala Phe Ser Ala
    130             135             140

Val Gly Ala Leu Glu Ala Gln Leu Lys Leu Lys Thr Gly Lys Leu Val
    145             150             155             160

Ser Leu Ser Ala Gln Asn Leu Val Asp Cys Ser Thr Glu Lys Tyr Gly
                165             170             175

Asn Lys Gly Cys Asn Gly Gly Phe Met Thr Thr Ala Phe Gln Tyr Ile
            180             185             190

Ile Asp Asn Lys Gly Ile Asp Ser Asp Ala Ser Tyr Pro Tyr Lys Ala
            195             200             205

Met Asp Gln Lys Cys Gln Tyr Asp Ser Lys Tyr Arg Ala Ala Thr Cys
    210             215             220

Ser Lys Tyr Thr Glu Leu Pro Tyr Gly Arg Glu Asp Val Leu Lys Glu
225             230             235             240

Ala Val Ala Asn Lys Gly Pro Val Ser Val Gly Val Asp Ala Arg His
            245             250             255

Pro Ser Phe Phe Leu Tyr Arg Ser Gly Val Tyr Tyr Glu Pro Ser Cys
            260             265             270

Thr Gln Asn Val Asn His Gly Val Leu Val Val Gly Tyr Gly Asp Leu
            275             280             285

Asn Gly Lys Glu Tyr Trp Leu Val Lys Asn Ser Trp Gly His Asn Phe
    290             295             300

Gly Glu Glu Gly Tyr Ile Arg Met Ala Arg Asn Lys Gly Asn His Cys
305             310             315             320

Gly Ile Ala Ser Phe Pro Ser Tyr Pro Glu Ile
            325             330
```

<210> 5
<211> 414
<212> PRT
<213> Homo sapiens

<400> 5

```
Met His Tyr Cys Val Leu Ser Ala Phe Leu Ile Leu His Leu Val Thr
1               5                   10                  15

Val Ala Leu Ser Leu Ser Thr Cys Ser Thr Leu Asp Met Asp Gln Phe
```

```
                    20                        25                          30

        Met Arg Lys Arg Ile Glu Ala Ile Arg Gly Gln Ile Leu Ser Lys Leu
                35                  40                  45

        Lys Leu Thr Ser Pro Pro Glu Asp Tyr Pro Glu Pro Glu Glu Val Pro
                50                  55                  60

        Pro Glu Val Ile Ser Ile Tyr Asn Ser Thr Arg Asp Leu Leu Gln Glu
        65                  70                  75                      80

        Lys Ala Ser Arg Arg Ala Ala Ala Cys Glu Arg Glu Arg Ser Asp Glu
                        85                  90                  95

        Glu Tyr Tyr Ala Lys Glu Val Tyr Lys Ile Asp Met Pro Pro Phe Phe
                    100                 105                 110

        Pro Ser Glu Asn Ala Ile Pro Pro Thr Phe Tyr Arg Pro Tyr Phe Arg
                115                 120                 125

        Ile Val Arg Phe Asp Val Ser Ala Met Glu Lys Asn Ala Ser Asn Leu
                130                 135                 140

        Val Lys Ala Glu Phe Arg Val Phe Arg Leu Gln Asn Pro Lys Ala Arg
        145                 150                 155                     160

        Val Pro Glu Gln Arg Ile Glu Leu Tyr Gln Ile Leu Lys Ser Lys Asp
                        165                 170                 175

        Leu Thr Ser Pro Thr Gln Arg Tyr Ile Asp Ser Lys Val Val Lys Thr
                    180                 185                 190

        Arg Ala Glu Gly Glu Trp Leu Ser Phe Asp Val Thr Asp Ala Val His
                195                 200                 205

        Glu Trp Leu His His Lys Asp Arg Asn Leu Gly Phe Lys Ile Ser Leu
                210                 215                 220

        His Cys Pro Cys Cys Thr Phe Val Pro Ser Asn Asn Tyr Ile Ile Pro
        225                 230                 235                     240

        Asn Lys Ser Glu Glu Leu Glu Ala Arg Phe Ala Gly Ile Asp Gly Thr
                        245                 250                 255

        Ser Thr Tyr Thr Ser Gly Asp Gln Lys Thr Ile Lys Ser Thr Arg Lys
                    260                 265                 270
```

291

```
Lys Asn Ser Gly Lys Thr Pro His Leu Leu Leu Met Leu Leu Pro Ser
        275             280             285

Tyr Arg Leu Glu Ser Gln Gln Thr Asn Arg Arg Lys Lys Arg Ala Leu
        290             295             300

Asp Ala Ala Tyr Cys Phe Arg Asn Val Gln Asp Asn Cys Cys Leu Arg
305             310             315             320

Pro Leu Tyr Ile Asp Phe Lys Arg Asp Leu Gly Trp Lys Trp Ile His
                325             330             335

Glu Pro Lys Gly Tyr Asn Ala Asn Phe Cys Ala Gly Ala Cys Pro Tyr
        340             345             350

Leu Trp Ser Ser Asp Thr Gln His Ser Arg Val Leu Ser Leu Tyr Asn
        355             360             365

Thr Ile Asn Pro Glu Ala Ser Ala Ser Pro Cys Cys Val Ser Gln Asp
    370             375             380

Leu Glu Pro Leu Thr Ile Leu Tyr Tyr Ile Gly Lys Thr Pro Lys Ile
385             390             395             400

Glu Gln Leu Ser Asn Met Ile Val Lys Ser Cys Lys Cys Ser
                405             410
```

<210> 6
<211> 29
<212> PRT
<213> Homo sapiens

<400> 6

```
Thr Val Cys Pro Val Val Thr Thr Pro Ser Gly Ser Val Gly Ser Leu
1               5               10              15

Cys Ser Arg Gln Ser Gln Val Leu Cys Gly Tyr Leu Asp
        20              25
```

<210> 7
<211> 431
<212> PRT
<213> Homo sapiens

<400> 7

```
Met Arg Ala Leu Leu Ala Arg Leu Leu Leu Cys Val Leu Val Val Ser
1               5               10              15
```

```
Asp Ser Lys Gly Ser Asn Glu Leu His Gln Val Pro Ser Asn Cys Asp
            20              25              30
```

```
Cys Leu Asn Gly Gly Thr Cys Val Ser Asn Lys Tyr Phe Ser Asn Ile
        35              40                  45

His Trp Cys Asn Cys Pro Lys Lys Phe Gly Gly Gln His Cys Glu Ile
        50              55                  60

Asp Lys Ser Lys Thr Cys Tyr Glu Gly Asn Gly His Phe Tyr Arg Gly
65              70                  75                  80

Lys Ala Ser Thr Asp Thr Met Gly Arg Pro Cys Leu Pro Trp Asn Ser
            85                  90                  95

Ala Thr Val Leu Gln Gln Thr Tyr His Ala His Arg Ser Asp Ala Leu
            100                 105                 110

Gln Leu Gly Leu Gly Lys His Asn Tyr Cys Arg Asn Pro Asp Asn Arg
            115                 120                 125

Arg Arg Pro Trp Cys Tyr Val Gln Val Gly Leu Lys Pro Leu Val Gln
    130                 135                 140

Glu Cys Met Val His Asp Cys Ala Asp Gly Lys Lys Pro Ser Ser Pro
145                 150                 155                 160

Pro Glu Glu Leu Lys Phe Gln Cys Gly Gln Lys Thr Leu Arg Pro Arg
                165                 170                 175

Phe Lys Ile Ile Gly Gly Glu Phe Thr Thr Ile Glu Asn Gln Pro Trp
            180                 185                 190

Phe Ala Ala Ile Tyr Arg Arg His Arg Gly Gly Ser Val Thr Tyr Val
        195                 200                 205

Cys Gly Gly Ser Leu Ile Ser Pro Cys Trp Val Ile Ser Ala Thr His
210                 215                 220

Cys Phe Ile Asp Tyr Pro Lys Lys Glu Asp Tyr Ile Val Tyr Leu Gly
225                 230                 235                 240

Arg Ser Arg Leu Asn Ser Asn Thr Gln Gly Glu Met Lys Phe Glu Val
            245                 250                 255

Glu Asn Leu Ile Leu His Lys Asp Tyr Ser Ala Asp Thr Leu Ala His
        260                 265                 270

His Asn Asp Ile Ala Leu Leu Lys Ile Arg Ser Lys Glu Gly Arg Cys
        275                 280                 285
```

294

```
Ala Gln Pro Ser Arg Thr Ile Gln Thr Ile Cys Leu Pro Ser Met Tyr
    290                 295                 300

Asn Asp Pro Gln Phe Gly Thr Ser Cys Glu Ile Thr Gly Phe Gly Lys
305                 310                 315                 320

Glu Asn Ser Thr Asp Tyr Leu Tyr Pro Glu Gln Leu Lys Met Thr Val
                325                 330                 335

Val Lys Leu Ile Ser His Arg Glu Cys Gln Gln Pro His Tyr Tyr Gly
            340                 345                 350

Ser Glu Val Thr Thr Lys Met Leu Cys Ala Ala Asp Pro Gln Trp Lys
            355                 360                 365

Thr Asp Ser Cys Gln Gly Asp Ser Gly Gly Pro Leu Val Cys Ser Leu
    370                 375                 380

Gln Gly Arg Met Thr Leu Thr Gly Ile Val Ser Trp Gly Arg Gly Cys
385                 390                 395                 400

Ala Leu Lys Asp Lys Pro Gly Val Tyr Thr Arg Val Ser His Phe Leu
            405                 410                 415

Pro Trp Ile Arg Ser His Thr Lys Glu Glu Asn Gly Leu Ala Leu
            420                 425                 430
```

<210> 8
<211> 496
<212> PRT
<213> Homo sapiens

<400> 8

```
Met Trp Gln Ile Val Phe Phe Thr Leu Ser Cys Asp Leu Val Leu Ala
1               5                   10                  15

Ala Ala Tyr Asn Asn Phe Arg Lys Ser Met Asp Ser Ile Gly Lys Lys
            20                  25                  30

Gln Tyr Gln Val Gln His Gly Ser Cys Ser Tyr Thr Phe Leu Leu Pro
            35                  40                  45

Glu Met Asp Asn Cys Arg Ser Ser Ser Pro Tyr Val Ser Asn Ala
    50                  55                  60

Val Gln Arg Asp Ala Pro Leu Glu Tyr Asp Asp Ser Val Gln Arg Leu
65                  70                  75                  80
```

```
Gln Val Leu Glu Asn Ile Met Glu Asn Asn Thr Gln Trp Leu Met Lys
              85                90                95


Leu Glu Asn Tyr Ile Gln Asp Asn Met Lys Lys Glu Met Val Glu Ile
             100               105               110


Gln Gln Asn Ala Val Gln Asn Gln Thr Ala Val Met Ile Glu Ile Gly
             115               120               125


Thr Asn Leu Leu Asn Gln Thr Ala Glu Gln Thr Arg Lys Leu Thr Asp
         130               135               140


Val Glu Ala Gln Val Leu Asn Gln Thr Thr Arg Leu Glu Leu Gln Leu
145               150               155               160


Leu Glu His Ser Leu Ser Thr Asn Lys Leu Glu Lys Gln Ile Leu Asp
             165               170               175


Gln Thr Ser Glu Ile Asn Lys Leu Gln Asp Lys Asn Ser Phe Leu Glu
             180               185               190


Lys Lys Val Leu Ala Met Glu Asp Lys His Ile Ile Gln Leu Gln Ser
         195               200               205


Ile Lys Glu Glu Lys Asp Gln Leu Gln Val Leu Val Ser Lys Gln Asn
         210               215               220


Ser Ile Ile Glu Glu Leu Glu Lys Lys Ile Val Thr Ala Thr Val Asn
225               230               235               240


Asn Ser Val Leu Gln Lys Gln Gln His Asp Leu Met Glu Thr Val Asn
             245               250               255


Asn Leu Leu Thr Met Met Ser Thr Ser Asn Ser Ala Lys Asp Pro Thr
             260               265               270


Val Ala Lys Glu Glu Gln Ile Ser Phe Arg Asp Cys Ala Glu Val Phe
         275               280               285


Lys Ser Gly His Thr Thr Asn Gly Ile Tyr Thr Leu Thr Phe Pro Asn
         290               295               300


Ser Thr Glu Glu Ile Lys Ala Tyr Cys Asp Met Glu Ala Gly Gly Gly
305               310               315               320


Gly Trp Thr Ile Ile Gln Arg Arg Glu Asp Gly Ser Val Asp Phe Gln
             325               330               335
```

Arg Thr Trp Lys Glu Tyr Lys Val Gly Phe Gly Asn Pro Ser Gly Glu
                340                     345                 350

Tyr Trp Leu Gly Asn Glu Phe Val Ser Gln Leu Thr Asn Gln Gln Arg
                355                     360                 365

Tyr Val Leu Lys Ile His Leu Lys Asp Trp Glu Gly Asn Glu Ala Tyr
        370                 375                 380

Ser Leu Tyr Glu His Phe Tyr Leu Ser Ser Glu Glu Leu Asn Tyr Arg
385                 390                 395                 400

Ile His Leu Lys Gly Leu Thr Gly Thr Ala Gly Lys Ile Ser Ser Ile
                405                 410                 415

Ser Gln Pro Gly Asn Asp Phe Ser Thr Lys Asp Gly Asp Asn Asp Lys
                420                 425                 430

Cys Ile Cys Lys Cys Ser Gln Met Leu Thr Gly Gly Trp Trp Phe Asp
                435                 440                 445

Ala Cys Gly Pro Ser Asn Leu Asn Gly Met Tyr Tyr Pro Gln Arg Gln
        450                 455                 460

Asn Thr Asn Lys Phe Asn Gly Ile Lys Trp Tyr Tyr Trp Lys Gly Ser
465                 470                 475                 480

Gly Tyr Ser Leu Lys Ala Thr Thr Met Met Ile Arg Pro Ala Asp Phe
                485                 490                 495

<210> 9
<211> 267
<212> PRT
<213> Homo sapiens

<400> 9

Met Arg Leu Thr Val Leu Cys Ala Val Cys Leu Leu Pro Gly Ser Leu
1               5               10                  15

Ala Leu Pro Leu Pro Gln Glu Ala Gly Gly Met Ser Glu Leu Gln Trp
                20                  25                  30

Glu Gln Ala Gln Asp Tyr Leu Lys Arg Phe Tyr Leu Tyr Asp Ser Glu
                35                  40                  45

Thr Lys Asn Ala Asn Ser Leu Glu Ala Lys Leu Lys Glu Met Gln Lys
        50                  55                  60

```
Phe Phe Gly Leu Pro Ile Thr Gly Met Leu Asn Ser Arg Val Ile Glu
65              70          75              80

Ile Met Gln Lys Pro Arg Cys Gly Val Pro Asp Val Ala Glu Tyr Ser
            85              90              95

Leu Phe Pro Asn Ser Pro Lys Trp Thr Ser Lys Val Val Thr Tyr Arg
            100             105             110

Ile Val Ser Tyr Thr Arg Asp Leu Pro His Ile Thr Val Asp Arg Leu
            115             120             125

Val Ser Lys Ala Leu Asn Met Trp Gly Lys Glu Ile Pro Leu His Phe
            130             135             140

Arg Lys Val Val Trp Gly Thr Ala Asp Ile Met Ile Gly Phe Ala Arg
145             150             155             160

Gly Ala His Gly Asp Ser Tyr Pro Phe Asp Gly Pro Gly Asn Thr Leu
            165             170             175

Ala His Ala Phe Ala Pro Gly Thr Gly Leu Gly Gly Asp Ala His Phe
            180             185             190

Asp Glu Asp Glu Arg Trp Thr Asp Gly Ser Ser Leu Gly Ile Asn Phe
            195             200             205

Leu Tyr Ala Ala Thr His Glu Leu Gly His Ser Leu Gly Met Gly His
    210             215             220

Ser Ser Asp Pro Asn Ala Val Met Tyr Pro Thr Tyr Gly Asn Gly Asp
225             230             235             240

Pro Gln Asn Phe Lys Leu Ser Gln Asp Asp Ile Lys Gly Ile Gln Lys
            245             250             255

Leu Tyr Gly Lys Arg Ser Asn Ser Arg Lys Lys
            260             265
```

<210> 10
<211> 526
<212> PRT
<213> Homo sapiens

<400> 10

```
Met Gly His Leu Ser Ala Pro Leu His Arg Val Arg Val Pro Trp Gln
1               5               10              15
```

Gly Leu Leu Leu Thr Ala Ser Leu Leu Thr Phe Trp Asn Pro Pro Thr
20 25 30

Thr Ala Gln Leu Thr Thr Glu Ser Met Pro Phe Asn Val Ala Glu Gly
35 40 45

Lys Glu Val Leu Leu Leu Val His Asn Leu Pro Gln Gln Leu Phe Gly
50 55 60

Tyr Ser Trp Tyr Lys Gly Glu Arg Val Asp Gly Asn Arg Gln Ile Val
65 70 75 80

Gly Tyr Ala Ile Gly Thr Gln Gln Ala Thr Pro Gly Pro Ala Asn Ser
85 90 95

Gly Arg Glu Thr Ile Tyr Pro Asn Ala Ser Leu Leu Ile Gln Asn Val
100 105 110

Thr Gln Asn Asp Thr Gly Phe Tyr Thr Leu Gln Val Ile Lys Ser Asp
115 120 125

Leu Val Asn Glu Glu Ala Thr Gly Gln Phe His Val Tyr Pro Glu Leu
130 135 140

Pro Lys Pro Ser Ile Ser Ser Asn Asn Ser Asn Pro Val Glu Asp Lys
145 150 155 160

Asp Ala Val Ala Phe Thr Cys Glu Pro Glu Thr Gln Asp Thr Thr Tyr
165 170 175

Leu Trp Trp Ile Asn Asn Gln Ser Leu Pro Val Ser Pro Arg Leu Gln
180 185 190

Leu Ser Asn Gly Asn Arg Thr Leu Thr Leu Leu Ser Val Thr Arg Asn
195 200 205

Asp Thr Gly Pro Tyr Glu Cys Glu Ile Gln Asn Pro Val Ser Ala Asn
210 215 220

Arg Ser Asp Pro Val Thr Leu Asn Val Thr Tyr Gly Pro Asp Thr Pro
225 230 235 240

Thr Ile Ser Pro Ser Asp Thr Tyr Tyr Arg Pro Gly Ala Asn Leu Ser
245 250 255

Leu Ser Cys Tyr Ala Ala Ser Asn Pro Pro Ala Gln Tyr Ser Trp Leu
260 265 270

```
Ile Asn Gly Thr Phe Gln Gln Ser Thr Gln Glu Leu Phe Ile Pro Asn
        275                 280                 285

Ile Thr Val Asn Asn Ser Gly Ser Tyr Thr Cys His Ala Asn Asn Ser
        290                 295                 300

Val Thr Gly Cys Asn Arg Thr Thr Val Lys Thr Ile Ile Val Thr Glu
305                 310                 315                 320

Leu Ser Pro Val Val Ala Lys Pro Gln Ile Lys Ala Ser Lys Thr Thr
                325                 330                 335

Val Thr Gly Asp Lys Asp Ser Val Asn Leu Thr Cys Ser Thr Asn Asp
                340                 345                 350

Thr Gly Ile Ser Ile Arg Trp Phe Phe Lys Asn Gln Ser Leu Pro Ser
        355                 360                 365

Ser Glu Arg Met Lys Leu Ser Gln Gly Asn Thr Thr Leu Ser Ile Asn
        370                 375                 380

Pro Val Lys Arg Glu Asp Ala Gly Thr Tyr Trp Cys Glu Val Phe Asn
385                 390                 395                 400

Pro Ile Ser Lys Asn Gln Ser Asp Pro Ile Met Leu Asn Val Asn Tyr
                405                 410                 415

Asn Ala Leu Pro Gln Glu Asn Gly Leu Ser Pro Gly Ala Ile Ala Gly
                420                 425                 430

Ile Val Ile Gly Val Val Ala Leu Val Ala Leu Ile Ala Val Ala Leu
        435                 440                 445

Ala Cys Phe Leu His Phe Gly Lys Thr Gly Arg Ala Ser Asp Gln Arg
        450                 455                 460

Asp Leu Thr Glu His Lys Pro Ser Val Ser Asn His Thr Gln Asp His
465                 470                 475                 480

Ser Asn Asp Pro Pro Asn Lys Met Asn Glu Val Thr Tyr Ser Thr Leu
                485                 490                 495

Asn Phe Glu Ala Gln Gln Pro Thr Gln Pro Thr Ser Ala Ser Pro Ser
                500                 505                 510

Leu Thr Ala Thr Glu Ile Ile Tyr Ser Glu Val Lys Lys Gln
        515                 520                 525
```

<210> 11
<211> 31
<212> PRT
<213> Homo sapiens

<400> 11

Arg Gln Asn Leu Thr Met Leu Pro Arg Leu Asp Ser Asn Ser Trp Ala
1               5                   10                  15

Gln Ala Ile Leu Pro Ser Val Ser Gln Ser Ala Glu Ile Thr Asp
            20                  25                  30

<210> 12
<211> 31
<212> PRT
<213> Homo sapiens

<400> 12

Ser Pro Val Leu Gly Glu Asp Glu Ala Val Pro Gly Gln His His Pro
1               5                   10                  15

Gln His Lys Pro Cys Gln Glu Gly Gly Cys Trp Asp Val Leu Val
            20                  25                  30

<210> 13
<211> 381
<212> PRT
<213> Homo sapiens

<400> 13

```
Met Pro Phe Ser Asn Ser His Asn Ala Leu Lys Leu Arg Phe Pro Ala
1               5               10              15

Glu Asp Glu Phe Pro Asp Leu Ser Ala His Asn Asn His Met Ala Lys
            20              25              30

Val Leu Thr Pro Glu Leu Tyr Ala Glu Leu Arg Ala Lys Ser Thr Pro
            35              40              45

Ser Gly Phe Thr Leu Asp Asp Val Ile Gln Thr Gly Val Asp Asn Pro
    50              55              60

Gly His Pro Tyr Ile Met Thr Val Gly Cys Val Ala Gly Asp Glu Glu
65              70              75              80

Ser Tyr Glu Val Phe Lys Asp Leu Phe Asp Pro Ile Ile Glu Asp Arg
            85              90              95

His Gly Gly Tyr Lys Pro Ser Asp Glu His Lys Thr Asp Leu Asn Pro
            100             105             110
```

```
Asp Asn Leu Gln Gly Gly Asp Asp Leu Asp Pro Asn Tyr Val Leu Ser
        115             120             125

Ser Arg Val Arg Thr Gly Arg Ser Ile Arg Gly Phe Cys Leu Pro Pro
        130             135             140

His Cys Ser Arg Gly Glu Arg Arg Ala Ile Glu Lys Leu Ala Val Glu
145             150             155             160

Ala Leu Ser Ser Leu Asp Gly Asp Leu Ala Gly Arg Tyr Tyr Ala Leu
            165             170             175

Lys Ser Met Thr Glu Ala Glu Gln Gln Gln Leu Ile Asp Asp His Phe
            180             185             190

Leu Phe Asp Lys Pro Val Ser Pro Leu Leu Leu Ala Ser Gly Met Ala
            195             200             205

Arg Asp Trp Pro Asp Ala Arg Gly Ile Trp His Asn Asp Asn Lys Thr
        210             215             220

Phe Leu Val Trp Val Asn Glu Glu Asp His Leu Arg Val Ile Ser Met
225             230             235             240

Gln Lys Gly Gly Asn Met Lys Glu Val Phe Thr Arg Phe Cys Thr Gly
            245             250             255

Leu Thr Gln Ile Glu Thr Leu Phe Lys Ser Lys Asp Tyr Glu Phe Met
            260             265             270

Trp Asn Pro His Leu Gly Tyr Ile Leu Thr Cys Pro Ser Asn Leu Gly
        275             280             285

Thr Gly Leu Arg Ala Gly Val His Ile Lys Leu Pro Asn Leu Gly Lys
        290             295             300

His Glu Lys Phe Ser Glu Val Leu Lys Arg Leu Arg Leu Gln Lys Arg
305             310             315             320

Gly Thr Gly Gly Val Asp Thr Ala Ala Val Gly Gly Val Phe Asp Val
            325             330             335

Ser Asn Ala Asp Arg Leu Gly Phe Ser Glu Val Glu Leu Val Gln Met
        340             345             350

Val Val Asp Gly Val Lys Leu Leu Ile Glu Met Glu Gln Arg Leu Glu
        355             360             365
```

303

```
                Gln Gly Gln Ala Ile Asp Asp Leu Met Pro Ala Gln Lys
                     370                 375             380
```

<210> 14
<211> 381
<212> PRT
<213> Homo sapiens


<400> 14


```
        Met Pro Phe Gly Asn Thr His Asn Lys Phe Lys Leu Asn Tyr Lys Pro
        1               5               10              15

        Glu Glu Glu Tyr Pro Asp Leu Ser Lys His Asn Asn His Met Ala Lys
                     20              25              30

        Val Leu Thr Leu Glu Leu Tyr Lys Lys Leu Arg Asp Lys Glu Thr Pro
                     35              40              45

        Ser Gly Phe Thr Val Asp Asp Val Ile Gln Thr Gly Val Asp Asn Pro
             50              55              60

        Gly His Pro Phe Ile Met Thr Val Gly Cys Val Ala Gly Asp Glu Glu
        65              70              75              80

        Ser Tyr Glu Val Phe Lys Glu Leu Phe Asp Pro Ile Ile Ser Asp Arg
                        85              90              95

        His Gly Gly Tyr Lys Pro Thr Asp Lys His Lys Thr Asp Leu Asn His
                     100             105             110

        Glu Asn Leu Lys Gly Gly Asp Asp Leu Asp Pro Asn Tyr Val Leu Ser
                 115             120             125

        Ser Arg Val Arg Thr Gly Arg Ser Ile Lys Gly Tyr Thr Leu Pro Pro
             130             135             140

        His Cys Ser Arg Gly Glu Arg Arg Ala Val Glu Lys Leu Ser Val Glu
        145             150             155             160

        Ala Leu Asn Ser Leu Thr Gly Glu Phe Lys Gly Lys Tyr Tyr Pro Leu
                     165             170             175

        Lys Ser Met Thr Glu Lys Glu Gln Gln Gln Leu Ile Asp Asp His Phe
                     180             185             190

        Leu Phe Asp Lys Pro Val Ser Pro Leu Leu Leu Ala Ser Gly Met Ala
                     195             200             205
```

```
Arg Asp Trp Pro Asp Ala Arg Gly Ile Trp His Asn Asp Asn Lys Ser
    210             215             220

Phe Leu Val Trp Val Asn Glu Glu Asp His Leu Arg Val Ile Ser Met
225             230             235                 240

Glu Lys Gly Gly Asn Met Lys Glu Val Phe Arg Arg Phe Cys Val Gly
                245             250             255

Leu Gln Lys Ile Glu Glu Ile Phe Lys Lys Ala Gly His Pro Phe Met
            260             265             270

Trp Asn Gln His Leu Gly Tyr Val Leu Thr Cys Pro Ser Asn Leu Gly
        275             280             285

Thr Gly Leu Arg Gly Gly Val His Val Lys Leu Ala His Leu Ser Lys
    290             295             300

His Pro Lys Phe Glu Glu Ile Leu Thr Arg Leu Arg Leu Gln Lys Arg
305             310             315                 320

Gly Thr Gly Gly Val Asp Thr Ala Ala Val Gly Ser Val Phe Asp Val
            325             330             335

Ser Asn Ala Asp Arg Leu Gly Ser Ser Glu Val Glu Gln Val Gln Leu
        340             345             350

Val Val Asp Gly Val Lys Leu Met Val Glu Met Glu Lys Lys Leu Glu
        355             360             365

Lys Gly Gln Ser Ile Asp Asp Met Ile Pro Ala Gln Lys
    370             375             380
```

<210> 15
<211> 110
<212> PRT
<213> Homo sapiens

<400> 15

```
Met Ala Leu Trp Met Arg Leu Leu Pro Leu Leu Ala Leu Leu Ala Leu
1               5               10                  15

Trp Gly Pro Asp Pro Ala Ala Ala Phe Val Asn Gln His Leu Cys Gly
            20              25              30

Ser His Leu Val Glu Ala Leu Tyr Leu Val Cys Gly Glu Arg Gly Phe
        35              40              45
```

```
Phe Tyr Thr Pro Lys Thr Arg Arg Glu Ala Glu Asp Leu Gln Val Gly
    50              55              60

Gln Val Glu Leu Gly Gly Gly Pro Gly Ala Gly Ser Leu Gln Pro Leu
65              70              75              80

Ala Leu Glu Gly Ser Leu Gln Lys Arg Gly Ile Val Glu Gln Cys Cys
                85              90              95

Thr Ser Ile Cys Ser Leu Tyr Gln Leu Glu Asn Tyr Cys Asn
            100             105             110
```

<210> 16
<211> 115
<212> PRT
<213> Homo sapiens

<400> 16

```
Met Pro Met Phe Ile Val Asn Thr Asn Val Pro Arg Ala Ser Val Pro
1               5               10              15

Asp Gly Phe Leu Ser Glu Leu Thr Gln Gln Leu Ala Gln Ala Thr Gly
            20              25              30

Lys Pro Pro Gln Tyr Ile Ala Val His Val Val Pro Asp Gln Leu Met
            35              40              45

Ala Phe Gly Gly Ser Ser Glu Pro Cys Ala Leu Cys Ser Leu His Ser
    50              55              60

Ile Gly Lys Ile Gly Gly Ala Gln Asn Arg Ser Tyr Ser Lys Leu Leu
65              70              75              80

Cys Gly Leu Leu Ala Glu Arg Leu Arg Ile Ser Pro Asp Arg Val Tyr
                85              90              95

Ile Asn Tyr Tyr Asp Met Asn Ala Ala Asn Val Gly Trp Asn Asn Ser
            100             105             110

Thr Phe Ala
        115
```

<210> 17
<211> 250
<212> PRT
<213> Homo sapiens

<400> 17

```
Met Ala Asp Asn Phe Ser Leu His Asp Ala Leu Ser Gly Ser Gly Asn
1               5                   10                  15

Pro Asn Pro Gln Gly Trp Pro Gly Ala Trp Gly Asn Gln Pro Ala Gly
            20              25                  30

Ala Gly Gly Tyr Pro Gly Ala Ser Tyr Pro Gly Ala Tyr Pro Gly Gln
        35                  40                  45

Ala Pro Pro Gly Ala Tyr Pro Gly Gln Ala Pro Pro Gly Ala Tyr Pro
    50                  55                  60

Gly Ala Pro Gly Ala Tyr Pro Gly Ala Pro Ala Pro Gly Val Tyr Pro
65                  70                  75                  80

Gly Pro Pro Ser Gly Pro Gly Ala Tyr Pro Ser Ser Gly Gln Pro Ser
                85                  90                  95

Ala Thr Gly Ala Tyr Pro Ala Thr Gly Pro Tyr Gly Ala Pro Ala Gly
            100                 105                 110

Pro Leu Ile Val Pro Tyr Asn Leu Pro Leu Pro Gly Gly Val Val Pro
        115                 120                 125

Arg Met Leu Ile Thr Ile Leu Gly Thr Val Lys Pro Asn Ala Asn Arg
    130                 135                 140

Ile Ala Leu Asp Phe Gln Arg Gly Asn Asp Val Ala Phe His Phe Asn
145                 150                 155                 160

Pro Arg Phe Asn Glu Asn Asn Arg Arg Val Ile Val Cys Asn Thr Lys
                165                 170                 175

Leu Asp Asn Asn Trp Gly Arg Glu Glu Arg Gln Ser Val Phe Pro Phe
            180                 185                 190

Glu Ser Gly Lys Pro Phe Lys Ile Gln Val Leu Val Glu Pro Asp His
        195                 200                 205

Phe Lys Val Ala Val Asn Asp Ala His Leu Leu Gln Tyr Asn His Arg
    210                 215                 220

Val Lys Lys Leu Asn Glu Ile Ser Lys Leu Gly Ile Ser Gly Asp Ile
225                 230                 235                 240

Asp Leu Thr Ser Ala Ser Tyr Thr Met Ile
            245                 250
```

<210> 18
<211> 412
<212> PRT
<213> Homo sapiens

<400> 18

```
Met Lys Met His Leu Gln Arg Ala Leu Val Val Leu Ala Leu Leu Asn
1               5               10              15

Phe Ala Thr Val Ser Leu Ser Leu Ser Thr Cys Thr Thr Leu Asp Phe
            20              25              30

Gly His Ile Lys Lys Lys Arg Val Glu Ala Ile Arg Gly Gln Ile Leu
            35              40              45

Ser Lys Leu Arg Leu Thr Ser Pro Pro Glu Pro Thr Val Met Thr His
    50              55              60

Val Pro Tyr Gln Val Leu Ala Leu Tyr Asn Ser Thr Arg Glu Leu Leu
65              70              75              80

Glu Glu Met His Gly Glu Arg Glu Glu Gly Cys Thr Gln Glu Asn Thr
            85              90              95

Glu Ser Glu Tyr Tyr Ala Lys Glu Ile His Lys Phe Asp Met Ile Gln
            100             105             110

Gly Leu Ala Glu His Asn Glu Leu Ala Val Cys Pro Lys Gly Ile Thr
        115             120             125

Ser Lys Val Phe Arg Phe Asn Val Ser Ser Val Glu Lys Asn Arg Thr
    130             135             140

Asn Leu Phe Arg Ala Glu Phe Arg Val Leu Arg Val Pro Asn Pro Ser
145             150             155             160

Ser Lys Arg Asn Glu Gln Arg Ile Glu Leu Phe Gln Ile Leu Arg Pro
            165             170             175

Asp Glu His Ile Ala Lys Gln Arg Tyr Ile Gly Gly Lys Asn Leu Pro
        180             185             190

Thr Arg Gly Thr Ala Glu Trp Leu Ser Phe Asp Val Thr Asp Thr Val
        195             200             205

Arg Glu Trp Leu Leu Arg Arg Glu Ser Asn Leu Gly Leu Glu Ile Ser
    210             215             220

Ile His Cys Pro Cys His Thr Phe Gln Pro Asn Gly Asp Ile Leu Glu
225             230             235             240
```

```
Asn Ile His Glu Val Met Glu Ile Lys Phe Lys Gly Val Asp Asn Glu
              245             250             255

Asp Asp His Gly Arg Gly Asp Leu Gly Arg Leu Lys Lys Gln Lys Asp
              260             265             270

His His Asn Pro His Leu Ile Leu Met Met Ile Pro Pro His Arg Leu
              275             280             285

Asp Asn Pro Gly Gln Gly Gly Gln Arg Lys Lys Arg Ala Leu Asp Thr
              290             295             300

Asn Tyr Cys Phe Arg Asn Leu Glu Glu Asn Cys Cys Val Arg Pro Leu
305             310             315             320

Tyr Ile Asp Phe Arg Gln Asp Leu Gly Trp Lys Trp Val His Glu Pro
              325             330             335

Lys Gly Tyr Tyr Ala Asn Phe Cys Ser Gly Pro Cys Pro Tyr Leu Arg
              340             345             350

Ser Ala Asp Thr Thr His Ser Thr Val Leu Gly Leu Tyr Asn Thr Leu
              355             360             365

Asn Pro Glu Ala Ser Ala Ser Pro Cys Cys Val Pro Gln Asp Leu Glu
              370             375             380

Pro Leu Thr Ile Leu Tyr Tyr Val Gly Arg Thr Pro Lys Val Glu Gln
385             390             395             400

Leu Ser Asn Met Val Val Lys Ser Cys Lys Cys Ser
              405             410
```

<210> 19
<211> 829
<212> PRT
<213> Homo sapiens

<400> 19

```
Met Gly Leu Pro Arg Gly Pro Leu Ala Ser Leu Leu Leu Leu Gln Val
1               5               10              15

Cys Trp Leu Gln Cys Ala Ala Ser Glu Pro Cys Arg Ala Val Phe Arg
              20              25              30

Glu Ala Glu Val Thr Leu Glu Ala Gly Gly Ala Glu Gln Glu Pro Gly
              35              40              45
```

```
Gln Ala Leu Gly Lys Val Phe Met Gly Cys Pro Gly Gln Glu Pro Ala
    50                  55                  60

Leu Phe Ser Thr Asp Asn Asp Asp Phe Thr Val Arg Asn Gly Glu Thr
    65                  70                  75                  80

Val Gln Glu Arg Arg Ser Leu Lys Glu Arg Asn Pro Leu Lys Ile Phe
                85                  90                  95

Pro Ser Lys Arg Ile Leu Arg Arg His Lys Arg Asp Trp Val Val Ala
            100                 105                 110

Pro Ile Ser Val Pro Glu Asn Gly Lys Gly Pro Phe Pro Gln Arg Leu
            115                 120                 125

Asn Gln Leu Lys Ser Asn Lys Asp Arg Asp Thr Lys Ile Phe Tyr Ser
            130                 135                 140

Ile Thr Gly Pro Gly Ala Asp Ser Pro Pro Glu Gly Val Phe Ala Val
145                 150                 155                 160

Glu Lys Glu Thr Gly Trp Leu Leu Leu Asn Lys Pro Leu Asp Arg Glu
                165                 170                 175

Glu Ile Ala Lys Tyr Glu Leu Phe Gly His Ala Val Ser Glu Asn Gly
            180                 185                 190

Ala Ser Val Glu Asp Pro Met Asn Ile Ser Ile Ile Val Thr Asp Gln
            195                 200                 205

Asn Asp His Lys Pro Lys Phe Thr Gln Asp Thr Phe Arg Gly Ser Val
    210                 215                 220

Leu Glu Gly Val Leu Pro Gly Thr Ser Val Met Gln Val Thr Ala Thr
225                 230                 235                 240

Asp Glu Asp Asp Ala Ile Tyr Thr Tyr Asn Gly Val Val Ala Tyr Ser
                245                 250                 255

Ile His Ser Gln Glu Pro Lys Asp Pro His Asp Leu Met Phe Thr Ile
            260                 265                 270

His Arg Ser Thr Gly Thr Ile Ser Val Ile Ser Ser Gly Leu Asp Arg
            275                 280                 285

Glu Lys Val Pro Glu Tyr Thr Leu Thr Ile Gln Ala Thr Asp Met Asp
    290                 295                 300
```

```
Gly Asp Gly Ser Thr Thr Thr Ala Val Ala Val Val Glu Ile Leu Asp
305             310         315             320

Ala Asn Asp Asn Ala Pro Met Phe Asp Pro Gln Lys Tyr Glu Ala His
            325         330             335

Val Pro Glu Asn Ala Val Gly His Glu Val Gln Arg Leu Thr Val Thr
            340         345             350

Asp Leu Asp Ala Pro Asn Ser Pro Ala Trp Arg Ala Thr Tyr Leu Ile
            355         360             365

Met Gly Gly Asp Asp Gly Asp His Phe Thr Ile Thr Thr His Pro Glu
            370         375             380

Ser Asn Gln Gly Ile Leu Thr Thr Arg Lys Gly Leu Asp Phe Glu Ala
385             390             395             400

Lys Asn Gln His Thr Leu Tyr Val Glu Val Thr Asn Glu Ala Pro Phe
            405             410             415

Val Leu Lys Leu Pro Thr Ser Thr Ala Thr Ile Val Val His Val Glu
            420             425             430

Asp Val Asn Glu Ala Pro Val Phe Val Pro Pro Ser Lys Val Val Glu
            435         440             445

Val Gln Glu Gly Ile Pro Thr Gly Glu Pro Val Cys Val Tyr Thr Ala
    450             455             460

Glu Asp Pro Asp Lys Glu Asn Gln Lys Ile Ser Tyr Arg Ile Leu Arg
465             470             475             480

Asp Pro Ala Gly Trp Leu Ala Met Asp Pro Asp Ser Gly Gln Val Thr
            485         490             495

Ala Val Gly Thr Leu Asp Arg Glu Asp Glu Gln Phe Val Arg Asn Asn
            500         505             510

Ile Tyr Glu Val Met Val Leu Ala Met Asp Asn Gly Ser Pro Pro Thr
            515         520             525

Thr Gly Thr Gly Thr Leu Leu Leu Thr Leu Ile Asp Val Asn Asp His
            530             535             540

Gly Pro Val Pro Glu Pro Arg Gln Ile Thr Ile Cys Asn Gln Ser Pro
545             550             555             560
```

```
Val Arg Gln Val Leu Asn Ile Thr Asp Lys Asp Leu Ser Pro His Thr
                565             570             575

Ser Pro Phe Gln Ala Gln Leu Thr Asp Asp Ser Asp Ile Tyr Trp Thr
                580             585             590

Ala Glu Val Asn Glu Glu Gly Asp Thr Val Val Leu Ser Leu Lys Lys
                595             600             605

Phe Leu Lys Gln Asp Thr Tyr Asp Val His Leu Ser Leu Ser Asp His
    610             615             620

Gly Asn Lys Glu Gln Leu Thr Val Ile Arg Ala Thr Val Cys Asp Cys
625             630             635             640

His Gly His Val Glu Thr Cys Pro Gly Pro Trp Lys Gly Gly Phe Ile
                645             650             655

Leu Pro Val Leu Gly Ala Val Leu Ala Leu Leu Phe Leu Leu Leu Val
                660             665             670

Leu Leu Leu Leu Val Arg Lys Lys Arg Lys Ile Lys Glu Pro Leu Leu
                675             680             685

Leu Pro Glu Asp Asp Thr Arg Asp Asn Val Phe Tyr Tyr Gly Glu Glu
    690             695             700

Gly Gly Gly Glu Glu Asp Gln Asp Tyr Asp Ile Thr Gln Leu His Arg
705             710             715             720

Gly Leu Glu Ala Arg Pro Glu Val Val Leu Arg Asn Asp Val Ala Pro
                725             730             735

Thr Ile Ile Pro Thr Pro Met Tyr Arg Pro Arg Pro Ala Asn Pro Asp
                740             745             750

Glu Ile Gly Asn Phe Ile Ile Glu Asn Leu Lys Ala Ala Asn Thr Asp
                755             760             765

Pro Thr Ala Pro Pro Tyr Asp Thr Leu Leu Val Phe Asp Tyr Glu Gly
    770             775             780

Ser Gly Ser Asp Ala Ala Ser Leu Ser Ser Leu Thr Ser Ser Ala Ser
785             790             795             800

Asp Gln Asp Gln Asp Tyr Asp Tyr Leu Asn Glu Trp Gly Ser Arg Phe
```

```
                     805                    810                    815


        Lys Lys Leu Ala Asp Met Tyr Gly Gly Gly Glu Asp Asp
                     820                    825
```

<210> 20
<211> 24
<212> PRT
<213> Homo sapiens

<400> 20

```
        Gly Arg Gly Glu Arg Gly Ser Gln Arg Gly Asn Gly Gly Leu Gln Leu
        1                   5                   10                  15


        Ala Arg Gly Arg Thr Arg Arg Ser
                     20
```

<210> 21
<211> 1676
<212> PRT
<213> Homo sapiens

<400> 21

```
Met Gly Leu Leu Gly Ile Leu Cys Phe Leu Ile Phe Leu Gly Lys Thr
1               5               10              15

Trp Gly Gln Glu Gln Thr Tyr Val Ile Ser Ala Pro Lys Ile Phe Arg
            20              25              30

Val Gly Ala Ser Glu Asn Ile Val Ile Gln Val Tyr Gly Tyr Thr Glu
            35              40              45

Ala Phe Asp Ala Thr Ile Ser Ile Lys Ser Tyr Pro Asp Lys Lys Phe
        50              55              60

Ser Tyr Ser Ser Gly His Val His Leu Ser Ser Glu Asn Lys Phe Gln
65              70              75              80

Asn Ser Ala Ile Leu Thr Ile Gln Pro Lys Gln Leu Pro Gly Gly Gln
            85              90              95

Asn Pro Val Ser Tyr Val Tyr Leu Glu Val Val Ser Lys His Phe Ser
            100             105             110

Lys Ser Lys Arg Met Pro Ile Thr Tyr Asp Asn Gly Phe Leu Phe Ile
        115             120             125

His Thr Asp Lys Pro Val Tyr Thr Pro Asp Gln Ser Val Lys Val Arg
        130             135             140
```

```
Val Tyr Ser Leu Asn Asp Asp Leu Lys Pro Ala Lys Arg Glu Thr Val
145             150             155             160

Leu Thr Phe Ile Asp Pro Glu Gly Ser Glu Val Asp Met Val Glu Glu
                165             170             175

Ile Asp His Ile Gly Ile Ile Ser Phe Pro Asp Phe Lys Ile Pro Ser
                180             185             190

Asn Pro Arg Tyr Gly Met Trp Thr Ile Lys Ala Lys Tyr Lys Glu Asp
            195             200             205

Phe Ser Thr Thr Gly Thr Ala Tyr Phe Glu Val Lys Glu Tyr Val Leu
    210             215             220

Pro His Phe Ser Val Ser Ile Glu Pro Glu Tyr Asn Phe Ile Gly Tyr
225             230             235             240

Lys Asn Phe Lys Asn Phe Glu Ile Thr Ile Lys Ala Arg Tyr Phe Tyr
                245             250             255

Asn Lys Val Val Thr Glu Ala Asp Val Tyr Ile Thr Phe Gly Ile Arg
            260             265             270

Glu Asp Leu Lys Asp Asp Gln Lys Glu Met Met Gln Thr Ala Met Gln
    275             280             285

Asn Thr Met Leu Ile Asn Gly Ile Ala Gln Val Thr Phe Asp Ser Glu
    290             295             300

Thr Ala Val Lys Glu Leu Ser Tyr Tyr Ser Leu Glu Asp Leu Asn Asn
305             310             315             320

Lys Tyr Leu Tyr Ile Ala Val Thr Val Ile Glu Ser Thr Gly Gly Phe
                325             330             335

Ser Glu Glu Ala Glu Ile Pro Gly Ile Lys Tyr Val Leu Ser Pro Tyr
            340             345             350

Lys Leu Asn Leu Val Ala Thr Pro Leu Phe Leu Lys Pro Gly Ile Pro
            355             360             365

Tyr Pro Ile Lys Val Gln Val Lys Asp Ser Leu Asp Gln Leu Val Gly
    370             375             380

Gly Val Pro Val Thr Leu Asn Ala Gln Thr Ile Asp Val Asn Gln Glu
385             390             395             400
```

Thr Ser Asp Leu Asp Pro Ser Lys Ser Val Thr Arg Val Asp Asp Gly
405 410 415

Val Ala Ser Phe Val Leu Asn Leu Pro Ser Gly Val Thr Val Leu Glu
420 425 430

Phe Asn Val Lys Thr Asp Ala Pro Asp Leu Pro Glu Glu Asn Gln Ala
435 440 445

Arg Glu Gly Tyr Arg Ala Ile Ala Tyr Ser Ser Leu Ser Gln Ser Tyr
450 455 460

Leu Tyr Ile Asp Trp Thr Asp Asn His Lys Ala Leu Leu Val Gly Glu
465 470 475 480

His Leu Asn Ile Ile Val Thr Pro Lys Ser Pro Tyr Ile Asp Lys Ile
485 490 495

Thr His Tyr Asn Tyr Leu Ile Leu Ser Lys Gly Lys Ile Ile His Phe
500 505 510

Gly Thr Arg Glu Lys Phe Ser Asp Ala Ser Tyr Gln Ser Ile Asn Ile
515 520 525

Pro Val Thr Gln Asn Met Val Pro Ser Ser Arg Leu Leu Val Tyr Tyr
530 535 540

Ile Val Thr Gly Glu Gln Thr Ala Glu Leu Val Ser Asp Ser Val Trp
545 550 555 560

Leu Asn Ile Glu Glu Lys Cys Gly Asn Gln Leu Gln Val His Leu Ser
565 570 575

Pro Asp Ala Asp Ala Tyr Ser Pro Gly Gln Thr Val Ser Leu Asn Met
580 585 590

Ala Thr Gly Met Asp Ser Trp Val Ala Leu Ala Ala Val Asp Ser Ala
595 600 605

Val Tyr Gly Val Gln Arg Gly Ala Lys Lys Pro Leu Glu Arg Val Phe
610 615 620

Gln Phe Leu Glu Lys Ser Asp Leu Gly Cys Gly Ala Gly Gly Gly Leu
625 630 635 640

Asn Asn Ala Asn Val Phe His Leu Ala Gly Leu Thr Phe Leu Thr Asn
645 650 655

```
Ala Asn Ala Asp Asp Ser Gln Glu Asn Asp Glu Pro Cys Lys Glu Ile
            660             665             670

Leu Arg Pro Arg Arg Thr Leu Gln Lys Lys Ile Glu Glu Ile Ala Ala
            675             680             685

Lys Tyr Lys His Ser Val Val Lys Lys Cys Cys Tyr Asp Gly Ala Cys
    690             695             700

Val Asn Asn Asp Glu Thr Cys Glu Gln Arg Ala Ala Arg Ile Ser Leu
705             710             715             720

Gly Pro Arg Cys Ile Lys Ala Phe Thr Glu Cys Cys Val Val Ala Ser
            725             730             735

Gln Leu Arg Ala Asn Ile Ser His Lys Asp Met Gln Leu Gly Arg Leu
            740             745             750

His Met Lys Thr Leu Leu Pro Val Ser Lys Pro Glu Ile Arg Ser Tyr
            755             760             765

Phe Pro Glu Ser Trp Leu Trp Glu Val His Leu Val Pro Arg Arg Lys
    770             775             780

Gln Leu Gln Phe Ala Leu Pro Asp Ser Leu Thr Thr Trp Glu Ile Gln
785             790             795             800

Gly Val Gly Ile Ser Asn Thr Gly Ile Cys Val Ala Asp Thr Val Lys
            805             810             815

Ala Lys Val Phe Lys Asp Val Phe Leu Glu Met Asn Ile Pro Tyr Ser
            820             825             830

Val Val Arg Gly Glu Gln Ile Gln Leu Lys Gly Thr Val Tyr Asn Tyr
            835             840             845

Arg Thr Ser Gly Met Gln Phe Cys Val Lys Met Ser Ala Val Glu Gly
    850             855             860

Ile Cys Thr Ser Glu Ser Pro Val Ile Asp His Gln Gly Thr Lys Ser
865             870             875             880

Ser Lys Cys Val Arg Gln Lys Val Glu Gly Ser Ser Ser His Leu Val
            885             890             895

Thr Phe Thr Val Leu Pro Leu Glu Ile Gly Leu His Asn Ile Asn Phe
```

318

```
                900                      905                      910

      Ser Leu Glu Thr Trp Phe Gly Lys Glu Ile Leu Val Lys Thr Leu Arg
              915                  920                  925


      Val Val Pro Glu Gly Val Lys Arg Glu Ser Tyr Ser Gly Val Thr Leu
              930                  935                  940


      Asp Pro Arg Gly Ile Tyr Gly Thr Ile Ser Arg Arg Lys Glu Phe Pro
      945                      950                  955                  960


      Tyr Arg Ile Pro Leu Asp Leu Val Pro Lys Thr Glu Ile Lys Arg Ile
                      965                  970                      975


      Leu Ser Val Lys Gly Leu Leu Val Gly Glu Ile Leu Ser Ala Val Leu
                      980                  985                  990


      Ser Gln Glu Gly Ile Asn Ile Leu  Thr His Leu Pro Lys  Gly Ser Ala
              995                  1000                 1005


      Glu Ala  Glu Leu Met Ser Val  Val Pro Val Phe Tyr  Val Phe His
          1010                 1015                 1020


      Tyr Leu  Glu Thr Gly Asn His  Trp Asn Ile Phe His  Ser Asp Pro
          1025                 1030                 1035


      Leu Ile  Glu Lys Gln Lys Leu  Lys Lys Lys Leu Lys  Glu Gly Met
          1040                 1045                 1050


      Leu Ser  Ile Met Ser Tyr Arg  Asn Ala Asp Tyr Ser  Tyr Ser Val
          1055                 1060                 1065


      Trp Lys  Gly Gly Ser Ala Ser  Thr Trp Leu Thr Ala  Phe Ala Leu
          1070                 1075                 1080


      Arg Val  Leu Gly Gln Val Asn  Lys Tyr Val Glu Gln  Asn Gln Asn
          1085                 1090                 1095


      Ser Ile  Cys Asn Ser Leu Leu  Trp Leu Val Glu Asn  Tyr Gln Leu
          1100                 1105                 1110


      Asp Asn  Gly Ser Phe Lys Glu  Asn Ser Gln Tyr Gln  Pro Ile Lys
          1115                 1120                 1125


      Leu Gln  Gly Thr Leu Pro Val  Glu Ala Arg Glu Asn  Ser Leu Tyr
          1130                 1135                 1140
```

```
Leu Thr Ala Phe Thr Val Ile  Gly Ile Arg Lys Ala  Phe Asp Ile
    1145                1150              1155

Cys Pro Leu Val Lys Ile Asp  Thr Ala Leu Ile Lys  Ala Asp Asn
    1160                1165              1170

Phe Leu Leu Glu Asn Thr Leu  Pro Ala Gln Ser Thr  Phe Thr Leu
    1175                1180              1185

Ala Ile Ser Ala Tyr Ala Leu  Ser Leu Gly Asp Lys  Thr His Pro
    1190                1195              1200

Gln Phe Arg Ser Ile Val Ser  Ala Leu Lys Arg Glu  Ala Leu Val
    1205                1210              1215

Lys Gly Asn Pro Pro Ile Tyr  Arg Phe Trp Lys Asp  Asn Leu Gln
    1220                1225              1230

His Lys Asp Ser Ser Val Pro  Asn Thr Gly Thr Ala  Arg Met Val
    1235                1240              1245

Glu Thr Thr Ala Tyr Ala Leu  Leu Thr Ser Leu Asn  Leu Lys Asp
    1250                1255              1260

Ile Asn Tyr Val Asn Pro Val  Ile Lys Trp Leu Ser  Glu Glu Gln
    1265                1270              1275

Arg Tyr Gly Gly Gly Phe Tyr  Ser Thr Gln Asp Thr  Ile Asn Ala
    1280                1285              1290

Ile Glu Gly Leu Thr Glu Tyr  Ser Leu Leu Val Lys  Gln Leu Arg
    1295                1300              1305

Leu Ser Met Asp Ile Asp Val  Ser Tyr Lys His Lys  Gly Ala Leu
    1310                1315              1320

His Asn Tyr Lys Met Thr Asp  Lys Asn Phe Leu Gly  Arg Pro Val
    1325                1330              1335

Glu Val Leu Leu Asn Asp Asp  Leu Ile Val Ser Thr  Gly Phe Gly
    1340                1345              1350

Ser Gly Leu Ala Thr Val His  Val Thr Thr Val Val  His Lys Thr
    1355                1360              1365

Ser Thr Ser Glu Glu Val Cys  Ser Phe Tyr Leu Lys  Ile Asp Thr
    1370                1375              1380
```

320

```
Gln Asp  Ile Glu Ala Ser His  Tyr Arg Gly Tyr Gly  Asn Ser Asp
    1385             1390             1395

Tyr Lys  Arg Ile Val Ala Cys  Ala Ser Tyr Lys Pro  Ser Arg Glu
    1400             1405             1410

Glu Ser  Ser Ser Gly Ser Ser  His Ala Val Met Asp  Ile Ser Leu
    1415             1420             1425

Pro Thr  Gly Ile Ser Ala Asn  Glu Glu Asp Leu Lys  Ala Leu Val
    1430             1435             1440

Glu Gly  Val Asp Gln Leu Phe  Thr Asp Tyr Gln Ile  Lys Asp Gly
    1445             1450             1455

His Val  Ile Leu Gln Leu Asn  Ser Ile Pro Ser Ser  Asp Phe Leu
    1460             1465             1470

Cys Val  Arg Phe Arg Ile Phe  Glu Leu Phe Glu Val  Gly Phe Leu
    1475             1480             1485

Ser Pro  Ala Thr Phe Thr Val  Tyr Glu Tyr His Arg  Pro Asp Lys
    1490             1495             1500

Gln Cys  Thr Met Phe Tyr Ser  Thr Ser Asn Ile Lys  Ile Gln Lys
    1505             1510             1515

Val Cys  Glu Gly Ala Ala Cys  Lys Cys Val Glu Ala  Asp Cys Gly
    1520             1525             1530

Gln Met  Gln Glu Glu Leu Asp  Leu Thr Ile Ser Ala  Glu Thr Arg
    1535             1540             1545

Lys Gln  Thr Ala Cys Lys Pro  Glu Ile Ala Tyr Ala  Tyr Lys Val
    1550             1555             1560

Ser Ile  Thr Ser Ile Thr Val  Glu Asn Val Phe Val  Lys Tyr Lys
    1565             1570             1575

Ala Thr  Leu Leu Asp Ile Tyr  Lys Thr Gly Glu Ala  Val Ala Glu
    1580             1585             1590

Lys Asp  Ser Glu Ile Thr Phe  Ile Lys Lys Val Thr  Cys Thr Asn
    1595             1600             1605

Ala Glu  Leu Val Lys Gly Arg  Gln Tyr Leu Ile Met  Gly Lys Glu
    1610             1615             1620
```

321

```
Ala Leu  Gln Ile Lys Tyr Asn  Phe Ser Phe Arg Tyr  Ile Tyr Pro
    1625             1630             1635

Leu Asp  Ser Leu Thr Trp Ile  Glu Tyr Trp Pro Arg  Asp Thr Thr
    1640             1645             1650

Cys Ser  Ser Cys Gln Ala Phe  Leu Ala Asn Leu Asp  Glu Phe Ala
    1655             1660             1665

Glu Asp  Ile Phe Leu Asn Gly  Cys
    1670             1675
```

<210> 22
<211> 101
<212> PRT
<213> Homo sapiens

<400> 22

```
Met Ser Ser Ala Ala Gly Phe Cys Ala Ser Arg Pro Gly Leu Leu Phe
1             5             10             15

Leu Gly Leu Leu Leu Leu Pro Leu Val Val Ala Phe Ala Ser Ala Glu
        20             25             30

Ala Glu Glu Asp Gly Asp Leu Gln Cys Leu Cys Val Lys Thr Thr Ser
        35             40             45

Gln Val Arg Pro Arg His Ile Thr Ser Leu Glu Val Ile Lys Ala Gly
        50             55             60

Pro His Cys Pro Thr Ala Gln Leu Ile Ala Thr Leu Lys Asn Gly Arg
65             70             75             80

Lys Ile Cys Leu Asp Leu Gln Ala Pro Leu Tyr Lys Lys Ile Ile Lys
            85             90             95

Lys Leu Leu Glu Ser
            100
```

<210> 23
<211> 128
<212> PRT
<213> Homo sapiens

<400> 23

```
Met Ser Leu Arg Leu Asp Thr Thr Pro Ser Cys Asn Ser Ala Arg Pro
1            5                 10                15


Leu His Ala Leu Gln Val Leu Leu Leu Leu Ser Leu Leu Leu Thr Ala
            20              25              30


Leu Ala Ser Ser Thr Lys Gly Gln Thr Lys Arg Asn Leu Ala Lys Gly
        35              40              45


Lys Glu Glu Ser Leu Asp Ser Asp Leu Tyr Ala Glu Leu Arg Cys Met
    50              55              60


Cys Ile Lys Thr Thr Ser Gly Ile His Pro Lys Asn Ile Gln Ser Leu
65              70              75              80


Glu Val Ile Gly Lys Gly Thr His Cys Asn Gln Val Glu Val Ile Ala
            85              90              95


Thr Leu Lys Asp Gly Arg Lys Ile Cys Leu Asp Pro Asp Ala Pro Arg
            100             105             110


Ile Lys Lys Ile Val Gln Lys Lys Leu Ala Gly Asp Glu Ser Ala Asp
            115             120             125
```

<210> 24
<211> 476
<212> PRT
<213> Homo sapiens

<400> 24

```
Met Met His Leu Ala Phe Leu Val Leu Leu Cys Leu Pro Val Cys Ser
1             5               10            15

Ala Tyr Pro Leu Ser Gly Ala Ala Lys Glu Glu Asp Ser Asn Lys Asp
            20              25            30

Leu Ala Gln Gln Tyr Leu Glu Lys Tyr Tyr Asn Leu Glu Lys Asp Val
        35              40            45

Lys Gln Phe Arg Arg Lys Asp Ser Asn Leu Ile Val Lys Lys Ile Gln
    50              55            60

Gly Met Gln Lys Phe Leu Gly Leu Glu Val Thr Gly Lys Leu Asp Thr
65              70            75              80

Asp Thr Leu Glu Val Met Arg Lys Pro Arg Cys Gly Val Pro Asp Val
            85              90            95

Gly His Phe Ser Ser Phe Pro Gly Met Pro Lys Trp Arg Lys Thr His
            100             105           110

Leu Thr Tyr Arg Ile Val Asn Tyr Thr Pro Asp Leu Pro Arg Asp Ala
            115             120           125
```

Val Asp Ser Ala Ile Glu Lys Ala Leu Lys Val Trp Glu Glu Val Thr
    130             135             140

Pro Leu Thr Phe Ser Arg Leu Tyr Glu Gly Glu Ala Asp Ile Met Ile
    145             150             155             160

Ser Phe Ala Val Lys Glu His Gly Asp Phe Tyr Ser Phe Asp Gly Pro
                165             170             175

Gly His Ser Leu Ala His Ala Tyr Pro Pro Gly Pro Gly Leu Tyr Gly
                180             185             190

Asp Ile His Phe Asp Asp Asp Glu Lys Trp Thr Glu Asp Ala Ser Gly
                195             200             205

Thr Asn Leu Phe Leu Val Ala Ala His Glu Leu Gly His Ser Leu Gly
    210             215             220

Leu Phe His Ser Ala Asn Thr Glu Ala Leu Met Tyr Pro Leu Tyr Asn
225             230             235             240

Ser Phe Thr Glu Leu Ala Gln Phe Arg Leu Ser Gln Asp Asp Val Asn
                245             250             255

Gly Ile Gln Ser Leu Tyr Gly Pro Pro Pro Ala Ser Thr Glu Glu Pro
                260             265             270

Leu Val Pro Thr Lys Ser Val Pro Ser Gly Ser Glu Met Pro Ala Lys
                275             280             285

Cys Asp Pro Ala Leu Ser Phe Asp Ala Ile Ser Thr Leu Arg Gly Glu
    290             295             300

Tyr Leu Phe Phe Lys Asp Arg Tyr Phe Trp Arg Arg Ser His Trp Asn
305             310             315             320

Pro Glu Pro Glu Phe His Leu Ile Ser Ala Phe Trp Pro Ser Leu Pro
                325             330             335

Ser Tyr Leu Asp Ala Ala Tyr Glu Val Asn Ser Arg Asp Thr Val Phe
                340             345             350

Ile Phe Lys Gly Asn Glu Phe Trp Ala Ile Arg Gly Asn Glu Val Gln
                355             360             365

Ala Gly Tyr Pro Arg Gly Ile His Thr Leu Gly Phe Pro Pro Thr Ile

```
        370                  375                      380

        Arg Lys Ile Asp Ala Ala Val Ser Asp Lys Glu Lys Lys Lys Thr Tyr
        385             390             395                 400

        Phe Phe Ala Ala Asp Lys Tyr Trp Arg Phe Asp Glu Asn Ser Gln Ser
                    405             410                 415

        Met Glu Gln Gly Phe Pro Arg Leu Ile Ala Asp Asp Phe Pro Gly Val
                    420             425                 430

        Glu Pro Lys Val Asp Ala Val Leu Gln Ala Phe Gly Phe Phe Tyr Phe
                435             440                 445

        Phe Ser Gly Ser Ser Gln Phe Glu Phe Asp Pro Asn Ala Arg Met Val
            450             455                 460

        Thr His Ile Leu Lys Ser Asn Ser Trp Leu His Cys
        465             470             475
```